(19) Europäisches Patentamt
European Patent Office
Office européen des brevets

(11) EP 1 518 925 A1

(12) EUROPEAN PATENT APPLICATION
published in accordance with Art. 158(3) EPC

(43) Date of publication:
**30.03.2005 Bulletin 2005/13**

(21) Application number: **03723380.6**

(22) Date of filing: **15.05.2003**

(51) Int Cl.[7]: **C12N 9/12**, C12Q 1/48

(86) International application number:
**PCT/JP2003/006054**

(87) International publication number:
**WO 2003/097824 (27.11.2003 Gazette 2003/48)**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **16.05.2002 JP 2002142232**

(71) Applicant: **BANYU PHARMACEUTICAL CO., LTD.
Chuo-ku, Tokyo 103-8416 (JP)**

(72) Inventors:
- **KAMATA, Kenji, c/o Tsukuba Research Institute
Tsukuba-shi, Ibaraki 300-2611 (JP)**
- **NAGATA, Yasufumi,
c/o Tsukuba Research Institute
Tsukuba-shi, Ibaraki 300-2611 (JP)**
- **IWAMA, Toshiharu,
c/o Tsukuba Research Institute
Tsukuba-shi, Ibaraki 300-2611 (JP)**

(74) Representative: **Mackenzie, Andrew Bryan et al
Marks & Clerk
45 Grosvenor Road
St. Albans, Hertfordshire AL1 3AW (GB)**

# (54) CRYSTAL OF GLUCOKINASE PROTEIN, AND METHOD FOR DRUG DESIGN USING THE CRYSTAL

(57) Glucokinase is crystallized, the three-dimensional structure thereof is analyzed, and then a compound to be bonded to glucokinase is designed on the basis of the coordinate for the resulting three-dimensional structure. Specifically, glucokinase is freed of a part of amino acid residues being on the N-terminal side thereof, to thereby crystallize it, and the three-dimensional structure of the resulting crystal is elucidated through the X-ray crystallographic analysis thereof.

EP 1 518 925 A1

Printed by Jouve, 75001 PARIS (FR)

SEQUENCE LISTING

<110> Banyu Pharmaceutical Co., Ltd.

<120> Crystal of Glucokinase Protein and Drug Desing Method Using Thereof

<130> P03-0064PCT

<140>
<141>

<150> JP2002-142232
<151> 2002-05-16

<160> 10

<170> PatentIn Ver. 2.1

<210> 1
<211> 1401
<212> DNA
<213> Homo sapiens

<400> 1

```
atggcgatgg atgtcacaag gagccaggcc cagacagcct tgactctggt agagcagatc 60
ctggcagagt tccagctgca ggaggaggac ctgaagaagg tgatgagacg gatgcagaag 120
gagatggacc gcggcctgag gctggagacc catgaagagg ccagtgtgaa gatgctgccc 180
acctacgtgc gctccacccc agaaggctca gaagtcgggg acttcctctc cctggacctg 240
ggtggcacta acttcagggt gatgctggtg aaggtgggag aaggtgagga ggggcagtgg 300
agcgtgaaga ccaaacacca gatgtactcc atccccgagg acgccatgac cggcactgct 360
gagatgctct tcgactacat ctctgagtgc atctccgact tcctggacaa gcatcagatg 420
aaacacaaga agctgcccct gggcttcacc ttctcctttc ctgtgaggca cgaagacatc 480
gataagggca tccttctcaa ctggaccaag ggcttcaagg cctcaggagc agaagggaac 540
aatgtcgtgg ggcttctgcg agacgctatc aaacggagag gggactttga aatggatgtg 600
gtggcaatgg tgaatgacac ggtggccacg atgatctcct gctactacga agaccatcag 660
```

tgcgaggtcg gcatgatcgt gggcacgggc tgcaatgcct gctacatgga ggagatgcag 720
aatgtggagc tggtggaggg ggacgagggc cgcatgtgcg tcaataccga gtggggcgcc 780
ttcggggact ccggcgagct ggacgagttc ctgctggagt atgaccgcct ggtggacgag 840
agctctgcaa accccggtca gcagctgtat gagaagctca taggtggcaa gtacatgggc 900
gagctggtgc ggcttgtgct gctcaggctc gtggacgaaa acctgctctt ccacggggag 960
gcctccgagc agctgcgcac acgcggagcc ttcgagacgc gcttcgtgtc gcaggtggag 1020
agcgacacgg gcgaccgcaa gcagatctac aacatcctga gcacgctggg gctgcgaccc 1080
tcgaccaccg actgcgacat cgtgcgccgc gcctgcgaga gcgtgtctac gcgcgctgcg 1140
cacatgtgct cggcggggct ggcgggcgtc atcaaccgca tgcgcgagag ccgcagcgag 1200
gacgtaatgc gcatcactgt gggcgtggat ggctccgtgt acaagctgca ccccagcttc 1260
aaggagcggt tccatgccag cgtgcgcagg ctgacgccca gctgcgagat caccttcatc 1320
gagtcggagg agggcagtgg ccggggcgcg gccctggtct cggcggtggc ctgtaagaag 1380
gcctgtatgc tgggccagtg a 1401

<210> 2
<211> 466
<212> PRT
<213> Homo sapiens

<400> 2
Met Ala Met Asp Val Thr Arg Ser Gln Ala Gln Thr Ala Leu Thr Leu
1 5 10 15

Val Glu Gln Ile Leu Ala Glu Phe Gln Leu Gln Glu Glu Asp Leu Lys
20 25 30

Lys Val Met Arg Arg Met Gln Lys Glu Met Asp Arg Gly Leu Arg Leu
35 40 45

Glu Thr His Glu Glu Ala Ser Val Lys Met Leu Pro Thr Tyr Val Arg
50 55 60

Ser Thr Pro Glu Gly Ser Glu Val Gly Asp Phe Leu Ser Leu Asp Leu
65 70 75 80

Gly Gly Thr Asn Phe Arg Val Met Leu Val Lys Val Gly Glu Gly Glu
85 90 95

Glu Gly Gln Trp Ser Val Lys Thr Lys His Gln Met Tyr Ser Ile Pro
100 105 110

Glu Asp Ala Met Thr Gly Thr Ala Glu Met Leu Phe Asp Tyr Ile Ser
115 120 125

Glu Cys Ile Ser Asp Phe Leu Asp Lys His Gln Met Lys His Lys Lys
130 135 140

Leu Pro Leu Gly Phe Thr Phe Ser Phe Pro Val Arg His Glu Asp Ile
145 150 155 160

Asp Lys Gly Ile Leu Leu Asn Trp Thr Lys Gly Phe Lys Ala Ser Gly
165 170 175

Ala Glu Gly Asn Asn Val Val Gly Leu Leu Arg Asp Ala Ile Lys Arg
180 185 190

Arg Gly Asp Phe Glu Met Asp Val Val Ala Met Val Asn Asp Thr Val
195 200 205

Ala Thr Met Ile Ser Cys Tyr Tyr Glu Asp His Gln Cys Glu Val Gly
210 215 220

Met Ile Val Gly Thr Gly Cys Asn Ala Cys Tyr Met Glu Glu Met Gln
225 230 235 240

Asn Val Glu Leu Val Glu Gly Asp Glu Gly Arg Met Cys Val Asn Thr
245 250 255

Glu Trp Gly Ala Phe Gly Asp Ser Gly Glu Leu Asp Glu Phe Leu Leu
260 265 270

Glu Tyr Asp Arg Leu Val Asp Glu Ser Ser Ala Asn Pro Gly Gln Gln
275 280 285

Leu Tyr Glu Lys Leu Ile Gly Gly Lys Tyr Met Gly Glu Leu Val Arg
290 295 300

Leu Val Leu Leu Arg Leu Val Asp Glu Asn Leu Leu Phe His Gly Glu
305 310 315 320

Ala Ser Glu Gln Leu Arg Thr Arg Gly Ala Phe Glu Thr Arg Phe Val
325 330 335

Ser Gln Val Glu Ser Asp Thr Gly Asp Arg Lys Gln Ile Tyr Asn Ile
340 345 350

Leu Ser Thr Leu Gly Leu Arg Pro Ser Thr Thr Asp Cys Asp Ile Val
355 360 365

Arg Arg Ala Cys Glu Ser Val Ser Thr Arg Ala Ala His Met Cys Ser
370 375 380

Ala Gly Leu Ala Gly Val Ile Asn Arg Met Arg Glu Ser Arg Ser Glu
385 390 395 400

Asp Val Met Arg Ile Thr Val Gly Val Asp Gly Ser Val Tyr Lys Leu
405 410 415

His Pro Ser Phe Lys Glu Arg Phe His Ala Ser Val Arg Arg Leu Thr
420 425 430

Pro Ser Cys Glu Ile Thr Phe Ile Glu Ser Glu Glu Gly Ser Gly Arg
435 440 445

Gly Ala Ala Leu Val Ser Ala Val Ala Cys Lys Lys Ala Cys Met Leu
450 455 460

Gly Gln
465

<210> 3
<211> 465
<212> PRT
<213> Homo sapiens

<400> 3
Met Leu Asp Asp Arg Ala Arg Met Glu Ala Ala Lys Lys Glu Lys Val
1               5                   10                  15

Glu Gln Ile Leu Ala Glu Phe Gln Leu Gln Glu Glu Asp Leu Lys Lys
            20                  25                  30

Val Met Arg Arg Met Gln Lys Glu Met Asp Arg Gly Leu Arg Leu Glu
        35                  40                  45

Thr His Glu Glu Ala Ser Val Lys Met Leu Pro Thr Tyr Val Arg Ser
    50                  55                  60

Thr Pro Glu Gly Ser Glu Val Gly Asp Phe Leu Ser Leu Asp Leu Gly
65                  70                  75                  80

Gly Thr Asn Phe Arg Val Met Leu Val Lys Val Gly Glu Gly Glu Glu
                85                  90                  95

Gly Gln Trp Ser Val Lys Thr Lys His Gln Met Tyr Ser Ile Pro Glu
            100                 105                 110

Asp Ala Met Thr Gly Thr Ala Glu Met Leu Phe Asp Tyr Ile Ser Glu
        115                 120                 125

Cys Ile Ser Asp Phe Leu Asp Lys His Gln Met Lys His Lys Lys Leu
    130                 135                 140

Pro Leu Gly Phe Thr Phe Ser Phe Pro Val Arg His Glu Asp Ile Asp
145 150 155 160

Lys Gly Ile Leu Leu Asn Trp Thr Lys Gly Phe Lys Ala Ser Gly Ala
165 170 175

Glu Gly Asn Asn Val Val Gly Leu Leu Arg Asp Ala Ile Lys Arg Arg
180 185 190

Gly Asp Phe Glu Met Asp Val Val Ala Met Val Asn Asp Thr Val Ala
195 200 205

Thr Met Ile Ser Cys Tyr Tyr Glu Asp His Gln Cys Glu Val Gly Met
210 215 220

Ile Val Gly Thr Gly Cys Asn Ala Cys Tyr Met Glu Glu Met Gln Asn
225 230 235 240

Val Glu Leu Val Glu Gly Asp Glu Gly Arg Met Cys Val Asn Thr Glu
245 250 255

Trp Gly Ala Phe Gly Asp Ser Gly Glu Leu Asp Glu Phe Leu Leu Glu
260 265 270

Tyr Asp Arg Leu Val Asp Glu Ser Ser Ala Asn Pro Gly Gln Gln Leu
275 280 285

Tyr Glu Lys Leu Ile Gly Gly Lys Tyr Met Gly Glu Leu Val Arg Leu
290 295 300

Val Leu Leu Arg Leu Val Asp Glu Asn Leu Leu Phe His Gly Glu Ala
305 310 315 320

Ser Glu Gln Leu Arg Thr Arg Gly Ala Phe Glu Thr Arg Phe Val Ser
325 330 335

Gln Val Glu Ser Asp Thr Gly Asp Arg Lys Gln Ile Tyr Asn Ile Leu
340 345 350

Ser Thr Leu Gly Leu Arg Pro Ser Thr Thr Asp Cys Asp Ile Val Arg
355 360 365

Arg Ala Cys Glu Ser Val Ser Thr Arg Ala Ala His Met Cys Ser Ala
370 375 380

Gly Leu Ala Gly Val Ile Asn Arg Met Arg Glu Ser Arg Ser Glu Asp
385 390 395 400

Val Met Arg Ile Thr Val Gly Val Asp Gly Ser Val Tyr Lys Leu His
405 410 415

Pro Ser Phe Lys Glu Arg Phe His Ala Ser Val Arg Arg Leu Thr Pro
420 425 430

Ser Cys Glu Ile Thr Phe Ile Glu Ser Glu Glu Gly Ser Gly Arg Gly
435 440 445

Ala Ala Leu Val Ser Ala Val Ala Cys Lys Lys Ala Cys Met Leu Gly
450 455 460

Gln
465

<210> 4
<211> 1368
<212> DNA
<213> Homo sapiens

<400> 4
atggccttga ctctggtaga gcagatcctg gcagagttcc agctgcagga ggaggacctg 60

aagaaggtga tgagacggat gcagaaggag atggaccgcg gcctgaggct ggagacccat 120
gaagaggcca gtgtgaagat gctgcccacc tacgtgcgct ccaccccaga aggctcagaa 180
gtcggggact tcctctccct ggacctgggt ggcactaact tcagggtgat gctggtgaag 240
gtgggagaag gtgaggaggg gcagtggagc gtgaagacca aacaccagat gtactccatc 300
cccgaggacg ccatgaccgg cactgctgag atgctcttcg actacatctc tgagtgcatc 360
tccgacttcc tggacaagca tcagatgaaa cacaagaagc tgcccctggg cttcaccttc 420
tcctttcctg tgaggcacga agacatcgat aagggcatcc ttctcaactg gaccaagggc 480
ttcaaggcct caggagcaga agggaacaat gtcgtggggc ttctgcgaga cgctatcaaa 540
cggagagggg actttgaaat ggatgtggtg gcaatggtga atgacacggt ggccacgatg 600
atctcctgct actacgaaga ccatcagtgc gaggtcggca tgatcgtggg cacgggctgc 660
aatgcctgct acatggagga gatgcagaat gtggagctgg tggaggggga cgagggccgc 720
atgtgcgtca ataccgagtg gggcgccttc ggggactccg gcgagctgga cgagttcctg 780
ctggagtatg accgcctggt ggacgagagc tctgcaaacc ccggtcagca gctgtatgag 840
aagctcatag gtggcaagta catgggcgag ctggtgcggc ttgtgctgct caggctcgtg 900
gacgaaaacc tgctcttcca cggggaggcc tccgagcagc tgcgcacacg cggagccttc 960
gagacgcgct tcgtgtcgca ggtggagagc gacacgggcg accgcaagca gatctacaac 1020
atcctgagca cgctggggct gcgaccctcg accaccgact gcgacatcgt gcgccgcgcc 1080
tgcgagagcg tgtctacgcg cgctgcgcac atgtgctcgg cggggctggc gggcgtcatc 1140
aaccgcatgc gcgagagccg cagcgaggac gtaatgcgca tcactgtggg cgtggatggc 1200
tccgtgtaca agctgcaccc cagcttcaag gagcggttcc atgccagcgt gcgcaggctg 1260
acgcccagct gcgagatcac cttcatcgag tcggaggagg gcagtggccg gggcgcggcc 1320
ctggtctcgg cggtggcctg taagaaggcc tgtatgctgg gccagtga 1368

<210> 5
<211> 455
<212> PRT
<213> Homo sapiens

<400> 5
Met Ala Leu Thr Leu Val Glu Gln Ile Leu Ala Glu Phe Gln Leu Gln
1 5 10 15

Glu Glu Asp Leu Lys Lys Val Met Arg Arg Met Gln Lys Glu Met Asp
20 25 30

Arg Gly Leu Arg Leu Glu Thr His Glu Glu Ala Ser Val Lys Met Leu
35 40 45

Pro Thr Tyr Val Arg Ser Thr Pro Glu Gly Ser Glu Val Gly Asp Phe
50 55 60

Leu Ser Leu Asp Leu Gly Gly Thr Asn Phe Arg Val Met Leu Val Lys
65 70 75 80

Val Gly Glu Gly Glu Glu Gly Gln Trp Ser Val Lys Thr Lys His Gln
85 90 95

Met Tyr Ser Ile Pro Glu Asp Ala Met Thr Gly Thr Ala Glu Met Leu
100 105 110

Phe Asp Tyr Ile Ser Glu Cys Ile Ser Asp Phe Leu Asp Lys His Gln
115 120 125

Met Lys His Lys Lys Leu Pro Leu Gly Phe Thr Phe Ser Phe Pro Val
130 135 140

Arg His Glu Asp Ile Asp Lys Gly Ile Leu Leu Asn Trp Thr Lys Gly
145 150 155 160

Phe Lys Ala Ser Gly Ala Glu Gly Asn Asn Val Val Gly Leu Leu Arg
165 170 175

Asp Ala Ile Lys Arg Arg Gly Asp Phe Glu Met Asp Val Val Ala Met
180 185 190

Val Asn Asp Thr Val Ala Thr Met Ile Ser Cys Tyr Tyr Glu Asp His
195 200 205

Gln Cys Glu Val Gly Met Ile Val Gly Thr Gly Cys Asn Ala Cys Tyr
210 215 220

Met Glu Glu Met Gln Asn Val Glu Leu Val Glu Gly Asp Glu Gly Arg
225 230 235 240

Met Cys Val Asn Thr Glu Trp Gly Ala Phe Gly Asp Ser Gly Glu Leu
245 250 255

Asp Glu Phe Leu Leu Glu Tyr Asp Arg Leu Val Asp Glu Ser Ser Ala
260 265 270

Asn Pro Gly Gln Gln Leu Tyr Glu Lys Leu Ile Gly Gly Lys Tyr Met
275 280 285

Gly Glu Leu Val Arg Leu Val Leu Leu Arg Leu Val Asp Glu Asn Leu
290 295 300

Leu Phe His Gly Glu Ala Ser Glu Gln Leu Arg Thr Arg Gly Ala Phe
305 310 315 320

Glu Thr Arg Phe Val Ser Gln Val Glu Ser Asp Thr Gly Asp Arg Lys
325 330 335

Gln Ile Tyr Asn Ile Leu Ser Thr Leu Gly Leu Arg Pro Ser Thr Thr
340 345 350

Asp Cys Asp Ile Val Arg Arg Ala Cys Glu Ser Val Ser Thr Arg Ala
355 360 365

Ala His Met Cys Ser Ala Gly Leu Ala Gly Val Ile Asn Arg Met Arg
370 375 380

Glu Ser Arg Ser Glu Asp Val Met Arg Ile Thr Val Gly Val Asp Gly
385 390 395 400

Ser Val Tyr Lys Leu His Pro Ser Phe Lys Glu Arg Phe His Ala Ser
405 410 415

Val Arg Arg Leu Thr Pro Ser Cys Glu Ile Thr Phe Ile Glu Ser Glu
420 425 430

Glu Gly Ser Gly Arg Gly Ala Ala Leu Val Ser Ala Val Ala Cys Lys
435 440 445

Lys Ala Cys Met Leu Gly Gln
450 455

<210> 6
<211> 39
<212> DNA
<213> Artificial Sequence

<220>
<223> Description of Artificial Sequence:Primer

<400> 6
gtcacaagga gccagaagct tatggcctga ctctggtag 39

<210> 7
<211> 28
<212> DNA
<213> Artificial Sequence

<220>
<223> Description of Artificial Sequence:Primer

<400> 7
gaagccccac gacattgttc ccttctgc 28

<210> 8
<211> 451

<212> PRT
<213> Homo sapiens

<400> 8
Met Val Glu Gln Ile Leu Ala Glu Phe Gln Leu Gln Glu Glu Asp Leu
1 5 10 15

Lys Lys Val Met Arg Arg Met Gln Lys Glu Met Asp Arg Gly Leu Arg
20 25 30

Leu Glu Thr His Glu Glu Ala Ser Val Lys Met Leu Pro Thr Tyr Val
35 40 45

Arg Ser Thr Pro Glu Gly Ser Glu Val Gly Asp Phe Leu Ser Leu Asp
50 55 60

Leu Gly Gly Thr Asn Phe Arg Val Met Leu Val Lys Val Gly Glu Gly
65 70 75 80

Glu Glu Gly Gln Trp Ser Val Lys Thr Lys His Gln Met Tyr Ser Ile
85 90 95

Pro Glu Asp Ala Met Thr Gly Thr Ala Glu Met Leu Phe Asp Tyr Ile
100 105 110

Ser Glu Cys Ile Ser Asp Phe Leu Asp Lys His Gln Met Lys His Lys
115 120 125

Lys Leu Pro Leu Gly Phe Thr Phe Ser Phe Pro Val Arg His Glu Asp
130 135 140

Ile Asp Lys Gly Ile Leu Leu Asn Trp Thr Lys Gly Phe Lys Ala Ser
145 150 155 160

Gly Ala Glu Gly Asn Asn Val Val Gly Leu Leu Arg Asp Ala Ile Lys
165 170 175

Arg Arg Gly Asp Phe Glu Met Asp Val Val Ala Met Val Asn Asp Thr
180 185 190

Val Ala Thr Met Ile Ser Cys Tyr Tyr Glu Asp His Gln Cys Glu Val
195 200 205

Gly Met Ile Val Gly Thr Gly Cys Asn Ala Cys Tyr Met Glu Glu Met
210 215 220

Gln Asn Val Glu Leu Val Glu Gly Asp Glu Gly Arg Met Cys Val Asn
225 230 235 240

Thr Glu Trp Gly Ala Phe Gly Asp Ser Gly Glu Leu Asp Glu Phe Leu
245 250 255

Leu Glu Tyr Asp Arg Leu Val Asp Glu Ser Ser Ala Asn Pro Gly Gln
260 265 270

Gln Leu Tyr Glu Lys Leu Ile Gly Gly Lys Tyr Met Gly Glu Leu Val
275 280 285

Arg Leu Val Leu Leu Arg Leu Val Asp Glu Asn Leu Leu Phe His Gly
290 295 300

Glu Ala Ser Glu Gln Leu Arg Thr Arg Gly Ala Phe Glu Thr Arg Phe
305 310 315 320

Val Ser Gln Val Glu Ser Asp Thr Gly Asp Arg Lys Gln Ile Tyr Asn
325 330 335

Ile Leu Ser Thr Leu Gly Leu Arg Pro Ser Thr Thr Asp Cys Asp Ile
340 345 350

Val Arg Arg Ala Cys Glu Ser Val Ser Thr Arg Ala Ala His Met Cys
355 360 365

Ser Ala Gly Leu Ala Gly Val Ile Asn Arg Met Arg Glu Ser Arg Ser
370 375 380

Glu Asp Val Met Arg Ile Thr Val Gly Val Asp Gly Ser Val Tyr Lys
385 390 395 400

Leu His Pro Ser Phe Lys Glu Arg Phe His Ala Ser Val Arg Arg Leu
405 410 415

Thr Pro Ser Cys Glu Ile Thr Phe Ile Glu Ser Glu Glu Gly Ser Gly
420 425 430

Arg Gly Ala Ala Leu Val Ser Ala Val Ala Cys Lys Lys Ala Cys Met
435 440 445

Leu Gly Gln
450

<210> 9
<211> 38
<212> DNA
<213> Artificial Sequence

<220>
<223> Description of Artificial Sequence:Primer

<400> 9
ccaggcccag acagccaagc ttatggtaga gcagatcc 38

<210> 10
<211> 28
<212> DNA
<213> Artificial Sequence

<220>
<223> Description of Artificial Sequence:Primer

<400> 10
gaagccccac gacattgttc ccttctgc 28

## Description

Technical Field

The present invention relates a novel crystal of a glucokinase protein (hereinafter, referred to as GK protein), a drug design method using a three dimensional structure coordinate obtained by using this crystal, and the like.

Background Art

Glucokinase (ATP: D-hexose 6-phosphotransferaze, EC2. 7. 1. 1) is one (hexokinase IV) of four kinds of hexokinase isozymes of mammals. These isozymes catalyze the same reaction, however, show a difference in Km value for glucose. Namely, the Km values of hexokinases I, II, III are from $10^{-5}$ to $10^{-4}$ M, while the Km value for glucose of hexokinase IV also called glucokinase is by far larger, about $10^{-2}$ M. Hexokinase is an enzyme correlated with the initial stage of the glycolysis system, and catalyzes a reaction from glucose into glucose-6-phosphate.
Manifestation of glucokinase is localized mainly in liver and pancreatic β cells, and by controlling the rate determining stage of glucose metabolism in these cells, glucokinase plays an important role in metabolism of the whole body. Glucokinases of liver and pancreatic β cells differ in a 15-amino acid sequence at the N-terminal due to difference in splicing, however, have the same enzymological nature.
For about 10 years, there is suggested a hypothesis that glucokinase works as a glucose sensor in pancreatic β cells and liver (Garfinkel D, et al: Am J Physiol 247 (3Pt2): R527-537,1984). From recent results of glucokinase gene-manipulated mice, it has been clarified that glucokinase actually plays an important role in glucose homeostasis in the whole body.
A mouse having a glucokinase gene broken dies soon after birth by diabetes mellitus. (Grupe A, et al: Cell 83:69-78. 1995). On the other hand, a mouse having glucokinase expressed excessively shows lower blood glucose level (Ferre T, et al: Proc Natl Acad Sci USA 93: 7225-7230. 1996). When a glucokinase activity increases by increase in the concentration of glucose, the reaction of a pancreatic β cell and the reaction of a liver cell are different, however, both of them act to lower blood glucose level. By this, a pancreatic β cell secretes a larger amount of insulin, and liver incorporates glucose and store this as glycogen, and glucose release lowers simultaneously.
Variation of glucokinase enzymatic activity thus performs an important role in glucose homeostasis of mammals via liver and pancreatic β cells. In a case generating diabetes mellitus in younger ages called MODY2 (maturity-onset diabetes of the young), mutation of a glucokinase gene was found, and decrease in glucokinase activity causes increase in blood glucose value (Vionnet N, et al: Nature 356: 721-722, 1992). On the other hand, a family having mutation increasing a glucokinase activity was also found, and persons in such a family show hypoglycemia (Glaser B, et al: N Engl J Med 338: 226-230, 1998).
As described above, glucokinase acts as a glucose sensor also in humans and performs an important role in glucose homeostasis. On the other hand, since glucokinases of a lot of patients suffering from type II diabetes mellitus have no variation, blood glucose regulation utilizing a glucokinase sensor system is believed possible. A glucokinase activating substance is believed to be useful as a remedy for patients suffering from type II diabetes mellitus since this substance can be expected to have an action to promote insulation secretion of a pancreatic β cell and an action of accentuating incorporation of glucose of liver and suppressing release of glucose.
Recently, it has been clarified that pancreatic β cell type glucokinases are expressed locally in rat brains, among others, particularly ventromedial hypothalamus (VMH) which is a feeding center. About 20% neurocytes of VMH are called glucose response neuron, and conventionally believed to perform an important role in weight control. When glucose is dosed into a rat brain, feeding amount decreases, while when glucose metabolism is suppressed by administration of glucosamine which is a glucose analog into a brain, overeating occurs. It is known from electrophysiological experiments that a glucose responsive neuron is activated in response to physiological glucose concentration change (from 5 to 20 mM), while, when glucose metabolism is suppressed by glucosamine and the like, activity suppression is recognized. In a glucose concentration sensing system of VMH, a mechanism via glucokinase is hypothesized which is the same as that for insulation secretion of pancreatic β cells. Therefore, in addition to liver and pancreatic β cells, substances effecting glucokinase activation of VHM not only have an effect of correcting blood glucose value but also have a possibility of correcting also obesity which is a problem in may patients suffering from type II diabetes mellitus.
On the other hand, DIABETES, vol. 48, 1698-1705, September 1999 describes that the steric structure of glucokinase is predicted from hexokinase I, however, actually, it is nor crystallized and not practical.
From the above-described facts, to clarify the three dimensional steric structure of glucokinase and to enable efficient finding of a compound acting mutually with glucokinase are believed to make large progress in development of, for example, therapeutic agents or preventive preparations of diabetes mellitus; therapeutic agents or preventive preparations of chronic complications of diabetes mellitus such as retinopathy, nephropathy, neuropathy, ischemic heart disease, arterial sclerosis and the like; therapeutic agents or preventive preparations of obesity.

Currently, CARDD (Computer Aided Rational Drug Design) utilizing computers is frequently used at practical level, for analysis of the activity center and forecasting of the reaction mechanism, of a protein.

In a drug finding system by CARDD, the structure of the active portion of a protein is predicted based on the three-dimensional structure analysis data of a target protein. Information regarding the candidate of a compound capable of binding with the structure of its active portion is obtained from the database of the compound. Thereafter, candidates of a compound capable of binding to a target protein are selected, in view of the active portion of a target protein and the three-dimensional structure and physical nature of the candidate compound. These processes are a so-called insilico-screening process.

Whether a compound selected in the insilico-screening process is connected with a target protein to change its activity or not is checked by an actual test (wet experiment). A compound actually changing the activity of a target protein becomes an effective ingredient of a drug. By this, a compound interacting with a target protein can be efficiently found without conducting an operation of confirming an interaction by allowing a large amount of compounds to act respectively on target proteins in a laboratory.

The insilico-screening is judged to be a means effective for development of medical products since it can significantly narrow down candidate compounds which bind to a target protein.

In a drug finding system by CARDD, three dimensional structure analysis data by X-ray structure analysis of a target protein constitute a piece of important information. For three dimensional structure analysis by X-ray structure analysis, a crystal of a target protein is necessary as an analysis sample. Therefore, a crystal of GK is necessary for progressing the development of drug finding correlated with GK based on a drug finding system by CARDD. However, it is difficult to crystallize GK as described above, and it does not impart information necessary for CARDD.

Disclosure of Invention

The present invention has been accomplished in view of the above-mentioned problems of conventional technologies, and has objects to obtain a crystal of glucokinase and to design a compound binding to glucokinase based on information obtained from the crystal.

At least one of the above-mentioned objects is solved by the following inventions.

[1] A glucokinase protein, used for crystallization.

[2] The protein according to [1], consisting of an amino acid sequence as depicted in SEQ ID No. 5.

[3] A crystal of a protein, consisting of an amino acid sequence as depicted in SEQ ID No. 5 or an amino acid sequence which is substantially the same as the amino acid sequence.

[4] The crystal according to [3], wherein the above-mentioned protein is a glucokinase protein.

[5] The crystal according to [3], which is a crystal of a protein having an amino acid sequence as depicted in SEQ ID No. 5.

[6] The crystal according to [3], wherein the lattice constant satisfies the following formulae (1) to (4):

$$a = b = 79.9 \pm 4\ \text{Å} \qquad (1)$$

$$c = 322.2 \pm 15\ \text{Å} \qquad (2)$$

$$\alpha = \beta = 90^\circ \qquad (3)$$

$$\gamma = 120^\circ \qquad (4)$$

[7] The crystal according to [6], wherein the space group is $P6_522$.

[8] A crystal of a protein specified by three dimensional structure coordinate data described in Table 1.

[9] A crystal wherein, in three dimensional structure coordinate data obtained by changing at least one datum in three dimensional structure coordinate data described in Table 1, the average square deviation between an atom (Cα atom) of a main chain of an amino acid shown by three dimensional structure coordinate data described in Table 1 and a Cα atom shown by the above-mentioned changed three dimensional structure coordinate data corresponding to the Cα atom is 0.6 Å or less.

[10] The crystal according to any of [3] to[9], wherein the compound binding portion is constituted of at least one of amino acid residues of tyrosine 61 to serine 69, glutamic acid 96 to glutamine 98, isoleucine 159, methionine

210 to tyrosine 215, histidine 218 to glutamic acid 221, methionine 235, arginine 250 and leucine 451 to lysine 459, in an amino acid sequence as depicted in SEQ ID No. 5.

[11] A crystal comprising a complex of a protein consisting of an amino acid sequence as depicted in SEQ ID No. 5 or an amino acid sequence which is substantially the same as the amino acid sequence with a compound capable of binding to the protein.

[12] The crystal according to [11], wherein the above-mentioned compound is represented by the formula (I):

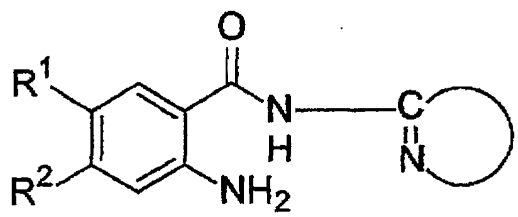

(I)

[wherein, $R^1$ represents a halogen atom, $-S-(O)_p-A$, $-S-(O)_q-B$ or -O-B (wherein, p and q are the same or different and represent an integer of 0 to 2, A represents a straight chain $C_1$ to $C_6$ alkyl group optionally substituted and B represents a 5-membered cyclic or 6-membered cyclic aryl group or heteroaryl group optionally substituted), $R^2$ represents a hydrogen atom or halogen atom, and

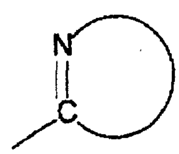

(II)

represents a mono-cyclic or di-cyclic heteroaryl group optionally substituted, having a nitrogen atom adjacent to a carbon atom connected to an amide group.].

[13] The crystal according to [12], wherein the above-mentioned compound is any of compounds of the formulae (IIIa) to (IIIc):

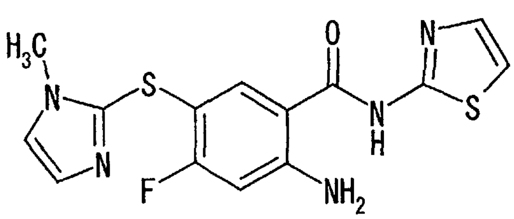

(IIIa)

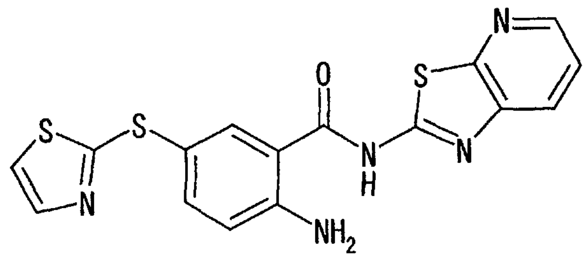

(IIIb)

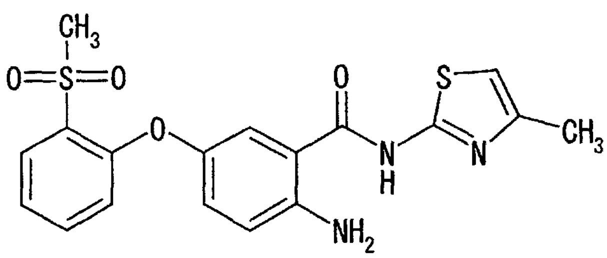

(IIIc)

[14] The protein according to [1], consisting of an amino acid sequence as depicted in SEQ ID No. 8.
[15] A crystal of a protein, consisting of an amino acid sequence as depicted in SEQ ID No. 8 or an amino acid sequence which is substantially the same as the amino acid sequence.
[16] The crystal according to [15], wherein the above-mentioned protein is a glucokinase protein.
[17] The crystal according to [15], which is a crystal of a protein having an amino acid sequence as depicted in SEQ ID No. 8.
[18] The crystal according to [15], wherein the lattice constant satisfies the following formulae:

$$a = b = 103.2 \pm 5 \text{ Å} \quad (5)$$

$$c = 281.0 \pm 7 \text{ Å} \quad (6)$$

$$\alpha = \beta = 90^\circ \quad (7)$$

$$\gamma = 120^\circ \quad (8)$$

[19] The crystal according to [18], wherein the space group is $P6_522$.
[20] A crystal of a protein specified by three dimensional structure coordinate data described in Table 2.
[21] A crystal wherein, in three dimensional structure coordinate data obtained by changing at least one datum in three dimensional structure coordinate data described in Table 2, the average square deviation between an atom (Cα atom) of a main chain of an amino acid shown by three dimensional structure coordinate data described in Table 2 and a Cα atom shown by the above-mentioned changed three dimensional structure coordinate data corresponding to the Cα atom is 0.6 Å or less.
[22] A method of producing a crystal comprising a complex of a protein with a compound binding to the protein, comprising

a protein production step of producing a protein having an amino acid sequence obtained by deleting 1 to

50 amino acid residues from one or both of the N terminal and C terminal of a protein having an amino acid sequence as depicted in SEQ ID No. 2, and

a protein reaction step of reacting a compound binding to a protein obtained in the above-mentioned protein production step with a protein obtained in the above-mentioned protein production step.

[23] A method of producing a crystal of a protein, using a protein containing an amino acid sequence as depicted in SEQ ID No. 5 or an amino acid sequence which is substantially the same as the amino acid sequence, and having a glucokinase activity, and a compound capable of binding to the above-mentioned protein.

[24] The method of producing a crystal of a protein according to [23], wherein the above-mentioned compound capable of binding to the protein is a compound of the formula (I):

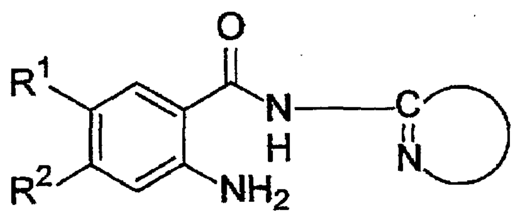

(I)

[wherein, $R^1$ represents a halogen atom, $-S-(O)_p-A$, $-S-(O)_q-B$ or -O-B (wherein, p and q are the same or different and represent an integer of 0 to 2, A represents a straight chain $C_1$ to $C_6$ alkyl group optionally substituted and B represents a 5-membered cyclic or 6-membered cyclic aryl group or heteroaryl group optionally substituted), $R^2$ represents a hydrogen atom or halogen atom, and

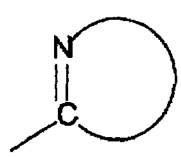

(II)

represents a mono-cyclic or di-cyclic heteroaryl group optionally substituted, having a nitrogen atom adjacent to a carbon atom connected to an amide group.].

[25] The method of producing a crystal according to [23] or [24], according to a co-crystallization method or soaking method.

[26] A drug design method of designing the structure of a compound capable of binding to a protein based on the steric structure information of the protein, wherein the steric structure information of the protein is information obtained by analyzing the crystal according to any of [3] to [13], [15] to [21].

[27] The drug design method according to [26], comprising

a binding portion guessing step of guessing a compound binding portion of the above-mentioned protein based on the above-mentioned steric structure information, and

a selection step of selecting a compound adaptable to a compound binding portion guessed in the above-mentioned binding portion guessing step from a compound library.

[28] The drug design method according to [26], comprising

a binding portion guessing step of guessing a compound binding portion of the above-mentioned protein based on the above-mentioned steric structure information, and

a compound structure fabricating step of fabricating the structure of a compound adaptable to a compound binding portion guessed in the above-mentioned binding portion guessing step.

[29] The drug design method according to [26], comprising

a binding portion guessing step of guessing a compound binding portion of the above-mentioned protein based on the above-mentioned steric structure information, and

a design step of designing visually the structure of a compound so that a compound binding portion guessed in the above-mentioned binding portion guessing step and a compound adaptable to the compound binding portion interact.

[30] The drug design method according to any of [26] to [29], wherein the above-mentioned compound binding portion is constituted of at least one of amino acid residues of tyrosine 61 to serine 69, glutamic acid 96 to glutamine 98, isoleucine 159, methionine 210 to tyrosine 215, histidine 218 to glutamic acid 221, methionine 235, arginine 250 and leucine 451 to lysine 459, in an amino acid sequence as depicted in SEQ ID No. 5.

[31] The drug design method according to any of [26] to [30], further comprising a step of measuring the physiological activity of a candidate compound guessed to be adaptable to the above-mentioned compound binding portion.

[32] The drug design method according to any of [26] to [30], further comprising a binding judgment step of contacting a candidate compound guessed to be adaptable to the above-mentioned compound binding portion with a protein containing an amino acid sequence as depicted in SEQ ID No. 5 or an amino acid sequence which is substantially the same as the amino acid sequence, and judging if the candidate compound binds to the protein or not.

[33] A method of producing a compound array, comprising combining groups of compounds selected by the drug design method according to any of [26] to [30], so as to be a compound array.

Brief Description of Drawings

Fig. 1 is a ribbon view showing the three dimensional structure of glucokinase.

(Fig. 1a is a ribbon view showing the structure of glucokinase (Δ1-11)/glucose/compound 1 (compound of the formula IIIa). A right view is obtained by rotating a left view.)

(Fig. 1b is a ribbon view showing the structure of a glucokinase (Δ1-15) single entity. A right view is obtained by rotating a left view.)

Fig. 2 is a view showing a mode of binding of a compound 1 (compound of the formula IIIa) to the binding portion of glucokinase (Δ1-11).

Fig. 3 is a view showing the structure of the binding portion of glucokinase (Δ1-11).

Best Mode For Carrying Out The Invention

In this specification, an amino acid, peptide and protein are represented by using abbreviations adopted in IUPAC-IUB Committee of Biochemical Nomenclature shown below. Unless otherwise stated, a sequence of an amino acid residue of a peptide and protein is represented so that the left end is the N-terminal and the right end is the C-terminal and the N-terminal is No. 1.

Embodiments of the invention will be described in detail below.

(Glucokinase protein)

First, the present invention provides a glucokinase protein, used for crystallization. A glucokinase protein (GK protein) is involved in an extremely important saccharometabolism in organisms. Therefore, a compound binding to a GK protein (namely, activating agent or inhibitor) can be searched by clarifying the three dimensional structure of a GK protein and analyzing the active portion of a GK protein. That is, it is important to clarify the three dimensional structure of a GK protein.

As the means for clarifying the three dimensional structure of a protein, X-ray crystal structure analysis is well known. Namely, a protein is crystallized, the crystal is irradiated with monochromatized X-ray, and the three dimensional structure of this protein is analyzed based on the resulted X-ray diffraction image (Blundell, T. L. and Johnson, L. N., PROTEIN CRYSTALLOGRAPHY, 1-565, (1976) Academic Press, New York). It is necessary to first crystallize a GK protein, for analyzing the X-ray crystal structure of a GK protein.

Here, "GK protein" of the present invention means a protein containing a liver type glucokinase derived from human having an amino acid sequence as depicted in SEQ ID No. 2 and an amino acid sequence which is substantially the same as that of SEQ ID No. 2. Here, this protein containing substantially the same amino acid sequence is preferably that having a glucokinase activity. Accordingly, in this specification, the GK protein includes not only a liver type glucokinase derived from human but also a pancreas type glucokinase derived from human, and GK proteins derived

from animals other than human such as mouse, rat, monkey and the like. In the present invention, a human liver type glucokinase is preferably used. In human-derived glucokinases, that of liver type and that of pancreas type differ in a 15-amino acid residue at the N-terminal. Here, "glucokinase activity" means an activity catalyzing a reaction from glucose into glucose-6-phosphate.

It is well known that crystallization of a protein is generally difficult, consequently, a GK protein could not be crystallized as it was. The present inventors have conducted various experiments through trial and error, and resultantly succeeded for the first time in crystallization of a GK protein by deleting 11 or 15 amino acids a the N-terminal side of a GK protein. It is believed that the deleted region projects from a spherical GK protein molecule in trying crystallization, as a result, constitutes steric hindrance between a GK protein molecule adjacent in the crystal, to prevent crystallization of a GK protein. Namely, in the present invention, a crystal of a GK protein was obtained by using a GK protein including deletion of 11 amino acid residues at the N-terminal (SEQ ID No. 5) or a GK protein including deletion of 15 amino acid residues at the N-terminal (SEQ ID No. 8), in a glucokinase of which amino acid sequence was known, however, of which crystallization had not been succeeded. However, the number of amino acids to be deleted is not limited providing steric hindrance is not formed between an adjacent crystal. Specifically, amino acid sequences obtained by deleting 1 to 50, preferably 3 to 30, more preferably 5 to 25, further preferably 8 to 18, particularly preferably 11 to 15 amino acid residues at the N-terminal in an amino acid sequence as depicted in SEQ ID No. 2, are used in the present invention. Further, amino acid sequences including deletion of 1 to 8, preferably 1 to 7, more preferably 2 to 6 amino acid residues at the C-terminal are used in the present invention.

(Crystal of glucokinase protein and production method thereof)

Next, in the present invention, a crystal comprising a protein containing an amino acid sequence as depicted in SEQ ID No. 5, SEQ ID No. 8 or an amino acid sequence which is substantially the same as this amino acid sequence is provided.

As described above, as a GK protein to be crystallized, a protein containing an amino acid sequence as depicted in SEQ ID No. 5 and/or SEQ ID No. 8 or an amino acid sequence which is substantially the same as this amino acid sequence, and the like are provided.

A protein containing an amino acid sequence as depicted in SEQ ID No. 5 and/or SEQ ID No. 8 or an amino acid sequence which is substantially the same as this amino acid sequence (hereinafter, abbreviated in some cases as "GK protein", also including a protein having an amino acid sequence as depicted in SEQ ID No. 2 or an amino acid sequence which is substantially the same as this amino acid sequence) may be permissible providing crystallization thereof is possible, and its amino acid sequence is not particularly restricted. Here, a protein containing an amino acid sequence which is substantially the same as an amino acid sequence as depicted in SEQ ID No. 5 and/or SEQ ID No. 8 is not required to have a glucokinase activity, and may also be an inactive variant (for example, a variant obtained by inactivation through a variation at a binding portion of ATP) providing it has a crystal structure capable of providing information necessary for drug design. Here, the protein containing an amino acid sequence which is substantially the same as an amino acid sequence as depicted in SEQ ID No. 2 or 5 includes amino acid sequences having a homology of about 60% or more, preferably about 70% or more, further preferably about 80% or more, particularly preferably about 90% or more, most preferably about 95% or more with the amino acid sequence as depicted in SEQ ID No. 2 or 5. Examples of the protein containing an amino acid sequence which is substantially the same as an amino acid sequence as depicted in SEQ ID No. 2 or 5 include amino acid sequences obtained by substitution, deletion, addition and/or insertion of 1 to 10, preferably 1 to 5, further preferably 1 to 3, further preferably 1 to 2 amino acid residues in an amino acid sequence as depicted in SEQ ID No. 2 or 5.

Analysis of the three dimensional structure of a GK protein is conducted, for example, as described below. First, a protein is purified. Then, a series of processes such as crystallization, X-ray diffraction intensity data collection, determination of phases of diffraction spots, electron density calculation, molecule model making, precise structure formation and the like are conducted. As main equipments for conducting analysis of a protein structure, an incubator for crystallization, binocular microscope, X-ray diffractometer, three dimensional computer graphics apparatus and the like are used. Specifically, an experimental process for producing a crystal of a protein is divided into a stage of purifying a large amount of protein (preferably, several mg or more), a stage of widely searching conditions for obtaining a crystal, and a stage of obtaining a crystal of good quality suitable for X-ray analysis. These processes will be described specifically below.

In crystallization, a GK protein is purified at high purity. As the purification method, known methods can be utilized, and for example, column chromatography, salting out, centrifugal separation and the like are used.

The purified GK protein is crystallized and used as a sample for analysis of an X-ray crystal structure. Crystallization is conducted based on known methods such as a vapor diffusion method, dialysis method and the like. In obtaining a crystal of a protein, it is necessary to investigate a lot of factors such as the purity and concentration of a protein, temperature, pH, the concentration of a precipitating agent used, and the like. Investigation of crystallization

conditions can be conducted in a wider range using a commercially available screening reagent, and it is preferable to conduct searching using 1 to 2 μL of a protein solution having a concentration of 1 to 2% for one condition. When a fine crystal is thus obtained, it is preferable to further refine the condition.

For obtaining a crystal of a GK protein, very many conditions should be searched. Therefore, also for investigating crystallization conditions, it is preferable to fabricate a system of expressing a large amount of protein. In general, a significant proportion of proteins which are crystallized are in mono-dispersion in a solution condition, and those in poly-dispersion are not crystallized in many cases. Then, it may also be permissible that the N-terminals of GK proteins are sequentially cut, a mono-dispersibility of a solution of the resulted protein is judged using a light scattering apparatus, and whether a sample is suitable for crystallization or not is investigated.

Next, X-ray diffraction intensity measurement is conducted using a crystal of the resulted GK protein. Recently, there is also utilized a method in which a crystal is scooped by a ring of narrow string and the like and cooled quickly to a liquid nitrogen temperature, and measurement is conducted as it is at lower temperatures. Measurement of the intensity of diffracted X-ray is usually conducted by a two dimensional detector such as an imaging plate and the like. A lot of diffracted beams generated by rotating a crystal while irradiating with X-ray are recorded on an imaging plate, and the recorded diffraction intensity is read by irradiating with laser.

Next, it is preferable to prepare a heavy atom isomorphic substituted body by a heavy atom soaking method or co-crystallization method. Using this, the phase of a protein crystal can be determined by a multiple isomorphic substitution method (MIR method). Instead of introduction of a heavy atom, there can be also utilized a multi-wavelength abnormal diffusion method (MAD method) determining a phase based on diffraction intensity data by X-ray of complicated wavelength. When a molecule having an analogous structure is already analyzed, its molecule structure can be applied into a crystal to give the initial structure, and there is also known a molecule substitution method (MR method) of drawing a Fourier synthesis diagram based on the resulted initial structure, and analyzing the structure of remaining portions to determine the whole structure.

When a phase is determined by the above-mentioned method, electron density is calculated based on this. This precision depends on the number of reflection (resolution ability) and precision of reflection used. The resolution ability is represented by minimum plane interval of reflection used. Base on this electron density diagram, a molecule model is fabricated. When a molecule model is fabricated, an atomic coordinate is obtained, and by this, a calculation value of structural factors is obtained, and preciseness of atomic parameter is increased by a minimum square method of approximating this value to an observed value. Thus, structure information which is as reasonable as possible is obtained.

The present invention has succeeded in preparing a crystal of a GK protein as depicted in SEQ ID No. 5 (see, examples below). Thus obtained crystal of a GK protein had a lattice constant satisfying the following formulae (1) to (4):

$$a = b = 79.9 \pm 4\ \text{Å} \quad (1)$$

$$c = 322.2 \pm 15\ \text{Å} \quad (2)$$

$$\alpha = \beta = 90^\circ \quad (3)$$

$$\gamma = 120^\circ \quad (4)$$

This crystal was analyzed to have a space group of $P6_522$. Here, the above-mentioned a = b is preferably 79.9 ± 3 Å, more preferably 79.9 ± 2 Å, further preferably 79.9 ± 1 Å. The above-mentioned c is preferably 322.2 ± 10 Å, more preferably 322.2 ± 8 Å, further preferably 322.2 ± 5 Å.

The three dimensional structure coordinate of thus obtained GK protein crystal is shown in Table 1.

[Table 1]

| | | | | | | | | | |
|---|---|---|---|---|---|---|---|---|---|
| ATOM | 1 | CB | THR | 14 | 25.972 | -34.025 | 76.567 | 1.00 | 51.12 |
| ATOM | 2 | OG1 | THR | 14 | 27.398 | -34.012 | 76.715 | 1.00 | 51.49 |
| ATOM | 3 | CG2 | THR | 14 | 25.626 | -34.173 | 75.095 | 1.00 | 49.96 |
| ATOM | 4 | C | THR | 14 | 24.138 | -32.317 | 76.374 | 1.00 | 50.95 |
| ATOM | 5 | O | THR | 14 | 24.246 | -31.685 | 75.330 | 1.00 | 52.42 |
| ATOM | 6 | N | THR | 14 | 25.108 | -32.861 | 78.611 | 1.00 | 51.41 |
| ATOM | 7 | CA | THR | 14 | 25.384 | -32.717 | 77.154 | 1.00 | 50.49 |
| ATOM | 8 | N | LEU | 15 | 22.957 | -32.673 | 76.871 | 1.00 | 49.75 |
| ATOM | 9 | CA | LEU | 15 | 21.733 | -32.307 | 76.167 | 1.00 | 49.25 |
| ATOM | 10 | CB | LEU | 15 | 20.496 | -32.824 | 76.904 | 1.00 | 52.56 |
| ATOM | 11 | CG | LEU | 15 | 20.439 | -34.307 | 77.291 | 1.00 | 55.08 |
| ATOM | 12 | CD1 | LEU | 15 | 21.186 | -34.524 | 78.610 | 1.00 | 53.67 |
| ATOM | 13 | CD2 | LEU | 15 | 18.980 | -34.742 | 77.438 | 1.00 | 54.84 |
| ATOM | 14 | C | LEU | 15 | 21.676 | -30.781 | 76.078 | 1.00 | 48.68 |
| ATOM | 15 | O | LEU | 15 | 21.397 | -30.208 | 75.023 | 1.00 | 47.52 |
| ATOM | 16 | N | VAL | 16 | 21.955 | -30.128 | 77.201 | 1.00 | 47.07 |
| ATOM | 17 | CA | VAL | 16 | 21.950 | -28.677 | 77.265 | 1.00 | 44.96 |
| ATOM | 18 | CB | VAL | 16 | 21.988 | -28.188 | 78.733 | 1.00 | 46.09 |
| ATOM | 19 | CG1 | VAL | 16 | 22.239 | -26.684 | 78.784 | 1.00 | 44.09 |
| ATOM | 20 | CG2 | VAL | 16 | 20.670 | -28.523 | 79.418 | 1.00 | 45.38 |
| ATOM | 21 | C | VAL | 16 | 23.142 | -28.097 | 76.512 | 1.00 | 43.58 |
| ATOM | 22 | O | VAL | 16 | 23.004 | -27.110 | 75.790 | 1.00 | 41.54 |
| ATOM | 23 | N | GLU | 17 | 24.310 | -28.712 | 76.672 | 1.00 | 43.48 |
| ATOM | 24 | CA | GLU | 17 | 25.507 | -28.223 | 75.998 | 1.00 | 45.62 |
| ATOM | 25 | CB | GLU | 17 | 26.759 | -28.931 | 76.532 | 1.00 | 46.30 |
| ATOM | 26 | CG | GLU | 17 | 27.140 | -28.571 | 77.984 | 1.00 | 49.19 |

| | | | | | | | | | |
|---|---|---|---|---|---|---|---|---|---|
| ATOM | 27 | CD | GLU | 17 | 27.467 | -27.087 | 78.191 | 1.00 | 50.74 |
| ATOM | 28 | OE1 | GLU | 17 | 28.238 | -26.520 | 77.386 | 1.00 | 50.39 |
| ATOM | 29 | OE2 | GLU | 17 | 26.966 | -26.488 | 79.170 | 1.00 | 50.85 |
| ATOM | 30 | C | GLU | 17 | 25.417 | -28.378 | 74.479 | 1.00 | 45.93 |
| ATOM | 31 | O | GLU | 17 | 26.097 | -27.666 | 73.735 | 1.00 | 44.10 |
| ATOM | 32 | N | GLN | 18 | 24.577 | -29.303 | 74.020 | 1.00 | 45.41 |
| ATOM | 33 | CA | GLN | 18 | 24.400 | -29.513 | 72.588 | 1.00 | 46.37 |
| ATOM | 34 | CB | GLN | 18 | 23.643 | -30.818 | 72.307 | 1.00 | 49.99 |
| ATOM | 35 | CG | GLN | 18 | 24.488 | -32.086 | 72.423 | 1.00 | 55.59 |
| ATOM | 36 | CD | GLN | 18 | 23.701 | -33.352 | 72.088 | 1.00 | 58.40 |
| ATOM | 37 | OE1 | GLN | 18 | 23.158 | -33.489 | 70.988 | 1.00 | 60.78 |
| ATOM | 38 | NE2 | GLN | 18 | 23.638 | -34.280 | 73.037 | 1.00 | 56.40 |
| ATOM | 39 | C | GLN | 18 | 23.617 | -28.338 | 72.014 | 1.00 | 44.35 |
| ATOM | 40 | O | GLN | 18 | 23.849 | -27.912 | 70.885 | 1.00 | 43.20 |
| ATOM | 41 | N | ILE | 19 | 22.677 | -27.821 | 72.791 | 1.00 | 41.97 |
| ATOM | 42 | CA | ILE | 19 | 21.895 | -26.689 | 72.327 | 1.00 | 40.37 |
| ATOM | 43 | CB | ILE | 19 | 20.631 | -26.500 | 73.193 | 1.00 | 39.71 |
| ATOM | 44 | CG2 | ILE | 19 | 19.976 | -25.166 | 72.894 | 1.00 | 39.42 |
| ATOM | 45 | CG1 | ILE | 19 | 19.653 | -27.653 | 72.915 | 1.00 | 40.83 |
| ATOM | 46 | CD1 | ILE | 19 | 18.356 | -27.599 | 73.719 | 1.00 | 38.38 |
| ATOM | 47 | C | ILE | 19 | 22.764 | -25.431 | 72.344 | 1.00 | 39.01 |
| ATOM | 48 | O | ILE | 19 | 22.746 | -24.644 | 71.394 | 1.00 | 40.12 |
| ATOM | 49 | N | LEU | 20 | 23.550 | -25.267 | 73.404 | 1.00 | 35.38 |
| ATOM | 50 | CA | LEU | 20 | 24.423 | -24.109 | 73.537 | 1.00 | 34.35 |
| ATOM | 51 | CB | LEU | 20 | 25.026 | -24.050 | 74.944 | 1.00 | 32.09 |
| ATOM | 52 | CG | LEU | 20 | 24.050 | -23.887 | 76.106 | 1.00 | 30.92 |
| ATOM | 53 | CD1 | LEU | 20 | 24.813 | -23.722 | 77.420 | 1.00 | 27.61 |
| ATOM | 54 | CD2 | LEU | 20 | 23.171 | -22.689 | 75.843 | 1.00 | 29.31 |
| ATOM | 55 | C | LEU | 20 | 25.555 | -24.135 | 72.518 | 1.00 | 34.62 |
| ATOM | 56 | O | LEU | 20 | 26.066 | -23.087 | 72.112 | 1.00 | 34.19 |
| ATOM | 57 | N | ALA | 21 | 25.946 | -25.336 | 72.116 | 1.00 | 33.16 |
| ATOM | 58 | CA | ALA | 21 | 27.030 | -25.509 | 71.163 | 1.00 | 34.30 |
| ATOM | 59 | CB | ALA | 21 | 27.344 | -26.992 | 70.993 | 1.00 | 34.49 |
| ATOM | 60 | C | ALA | 21 | 26.696 | -24.886 | 69.814 | 1.00 | 35.20 |
| ATOM | 61 | O | ALA | 21 | 27.587 | -24.619 | 69.007 | 1.00 | 35.57 |

| | | | | | | | | | |
|---|---|---|---|---|---|---|---|---|---|
| ATOM | 62 | N | GLU | 22 | 25.412 | -24.652 | 69.578 | 1.00 | 36.75 |
| ATOM | 63 | CA | GLU | 22 | 24.961 | -24.053 | 68.329 | 1.00 | 37.80 |
| ATOM | 64 | CB | GLU | 22 | 23.435 | -24.102 | 68.256 | 1.00 | 41.47 |
| ATOM | 65 | CG | GLU | 22 | 22.878 | -23.851 | 66.867 | 1.00 | 47.91 |
| ATOM | 66 | CD | GLU | 22 | 21.384 | -24.128 | 66.767 | 1.00 | 49.95 |
| ATOM | 67 | OE1 | GLU | 22 | 20.857 | -24.163 | 65.630 | 1.00 | 50.84 |
| ATOM | 68 | OE2 | GLU | 22 | 20.741 | -24.307 | 67.822 | 1.00 | 50.26 |
| ATOM | 69 | C | GLU | 22 | 25.444 | -22.605 | 68.177 | 1.00 | 37.38 |
| ATOM | 70 | O | GLU | 22 | 25.380 | -22.039 | 67.088 | 1.00 | 38.34 |
| ATOM | 71 | N | PHE | 23 | 25.928 | -22.012 | 69.268 | 1.00 | 35.41 |
| ATOM | 72 | CA | PHE | 23 | 26.426 | -20.636 | 69.249 | 1.00 | 33.38 |
| ATOM | 73 | CB | PHE | 23 | 26.224 | -19.962 | 70.614 | 1.00 | 31.59 |
| ATOM | 74 | CG | PHE | 23 | 24.826 | -19.470 | 70.843 | 1.00 | 29.81 |
| ATOM | 75 | CD1 | PHE | 23 | 23.836 | -20.328 | 71.310 | 1.00 | 26.48 |
| ATOM | 76 | CD2 | PHE | 23 | 24.489 | -18.151 | 70.555 | 1.00 | 28.79 |
| ATOM | 77 | CE1 | PHE | 23 | 22.520 | -19.882 | 71.487 | 1.00 | 29.30 |
| ATOM | 78 | CE2 | PHE | 23 | 23.177 | -17.691 | 70.727 | 1.00 | 31.65 |
| ATOM | 79 | CZ | PHE | 23 | 22.189 | -18.563 | 71.195 | 1.00 | 28.91 |
| ATOM | 80 | C | PHE | 23 | 27.899 | -20.542 | 68.877 | 1.00 | 33.33 |
| ATOM | 81 | O | PHE | 23 | 28.396 | -19.467 | 68.549 | 1.00 | 34.12 |
| ATOM | 82 | N | GLN | 24 | 28.596 | -21.670 | 68.940 | 1.00 | 32.75 |
| ATOM | 83 | CA | GLN | 24 | 30.016 | -21.716 | 68.620 | 1.00 | 32.56 |
| ATOM | 84 | CB | GLN | 24 | 30.543 | -23.147 | 68.778 | 1.00 | 35.53 |
| ATOM | 85 | CG | GLN | 24 | 30.817 | -23.603 | 70.210 | 1.00 | 37.84 |
| ATOM | 86 | CD | GLN | 24 | 31.214 | -25.074 | 70.266 | 1.00 | 42.36 |
| ATOM | 87 | OE1 | GLN | 24 | 31.802 | -25.601 | 69.320 | 1.00 | 43.06 |
| ATOM | 88 | NE2 | GLN | 24 | 30.902 | -25.739 | 71.375 | 1.00 | 40.61 |
| ATOM | 89 | C | GLN | 24 | 30.335 | -21.233 | 67.208 | 1.00 | 31.93 |
| ATOM | 90 | O | GLN | 24 | 29.508 | -21.320 | 66.299 | 1.00 | 30.32 |
| ATOM | 91 | N | LEU | 25 | 31.548 | -20.717 | 67.043 | 1.00 | 31.64 |
| ATOM | 92 | CA | LEU | 25 | 32.029 | -20.257 | 65.751 | 1.00 | 31.85 |
| ATOM | 93 | CB | LEU | 25 | 31.876 | -18.742 | 65.615 | 1.00 | 31.24 |
| ATOM | 94 | CG | LEU | 25 | 30.441 | -18.211 | 65.563 | 1.00 | 29.93 |
| ATOM | 95 | CD1 | LEU | 25 | 30.436 | -16.690 | 65.710 | 1.00 | 28.63 |
| ATOM | 96 | CD2 | LEU | 25 | 29.801 | -18.640 | 64.262 | 1.00 | 27.61 |

| | | | | | | | | | |
|---|---|---|---|---|---|---|---|---|---|
| ATOM | 97 | C | LEU | 25 | 33.502 | -20.635 | 65.667 | 1.00 | 33.30 |
| ATOM | 98 | O | LEU | 25 | 34.298 | -20.218 | 66.502 | 1.00 | 33.97 |
| ATOM | 99 | N | GLN | 26 | 33.856 | -21.450 | 64.679 | 1.00 | 34.57 |
| ATOM | 100 | CA | GLN | 26 | 35.244 | -21.860 | 64.496 | 1.00 | 36.87 |
| ATOM | 101 | CB | GLN | 26 | 35.330 | -23.053 | 63.540 | 1.00 | 40.20 |
| ATOM | 102 | CG | GLN | 26 | 35.105 | -24.414 | 64.182 | 1.00 | 46.34 |
| ATOM | 103 | CD | GLN | 26 | 33.863 | -24.462 | 65.041 | 1.00 | 48.48 |
| ATOM | 104 | OE1 | GLN | 26 | 33.918 | -24.229 | 66.253 | 1.00 | 49.27 |
| ATOM | 105 | NE2 | GLN | 26 | 32.725 | -24.757 | 64.417 | 1.00 | 51.72 |
| ATOM | 106 | C | GLN | 26 | 36.024 | -20.688 | 63.910 | 1.00 | 36.49 |
| ATOM | 107 | O | GLN | 26 | 35.430 | -19.735 | 63.403 | 1.00 | 35.76 |
| ATOM | 108 | N | GLU | 27 | 37.347 | -20.761 | 63.981 | 1.00 | 35.17 |
| ATOM | 109 | CA | GLU | 27 | 38.181 | -19.705 | 63.441 | 1.00 | 37.77 |
| ATOM | 110 | CB | GLU | 27 | 39.658 | -20.047 | 63.627 | 1.00 | 40.11 |
| ATOM | 111 | CG | GLU | 27 | 40.596 | -19.156 | 62.831 | 1.00 | 47.14 |
| ATOM | 112 | CD | GLU | 27 | 41.754 | -18.639 | 63.662 | 1.00 | 52.56 |
| ATOM | 113 | OE1 | GLU | 27 | 41.507 | -17.808 | 64.567 | 1.00 | 54.72 |
| ATOM | 114 | OE2 | GLU | 27 | 42.906 | -19.067 | 63.415 | 1.00 | 54.43 |
| ATOM | 115 | C | GLU | 27 | 37.878 | -19.511 | 61.961 | 1.00 | 37.80 |
| ATOM | 116 | O | GLU | 27 | 37.915 | -18.392 | 61.446 | 1.00 | 37.09 |
| ATOM | 117 | N | GLU | 28 | 37.557 | -20.605 | 61.282 | 1.00 | 36.94 |
| ATOM | 118 | CA | GLU | 28 | 37.261 | -20.535 | 59.862 | 1.00 | 36.18 |
| ATOM | 119 | CB | GLU | 28 | 37.175 | -21.939 | 59.267 | 1.00 | 37.83 |
| ATOM | 120 | CG | GLU | 28 | 37.826 | -22.039 | 57.902 | 1.00 | 41.72 |
| ATOM | 121 | CD | GLU | 28 | 39.154 | -21.287 | 57.843 | 1.00 | 44.57 |
| ATOM | 122 | OE1 | GLU | 28 | 40.033 | -21.531 | 58.706 | 1.00 | 46.91 |
| ATOM | 123 | OE2 | GLU | 28 | 39.313 | -20.446 | 56.933 | 1.00 | 44.10 |
| ATOM | 124 | C | GLU | 28 | 35.973 | -19.779 | 59.588 | 1.00 | 34.66 |
| ATOM | 125 | O | GLU | 28 | 35.860 | -19.089 | 58.575 | 1.00 | 33.91 |
| ATOM | 126 | N | ASP | 29 | 34.994 | -19.926 | 60.472 | 1.00 | 32.44 |
| ATOM | 127 | CA | ASP | 29 | 33.738 | -19.219 | 60.301 | 1.00 | 32.41 |
| ATOM | 128 | CB | ASP | 29 | 32.713 | -19.625 | 61.370 | 1.00 | 34.13 |
| ATOM | 129 | CG | ASP | 29 | 32.302 | -21.091 | 61.285 | 1.00 | 34.13 |
| ATOM | 130 | OD1 | ASP | 29 | 32.012 | -21.580 | 60.173 | 1.00 | 34.03 |
| ATOM | 131 | OD2 | ASP | 29 | 32.246 | -21.749 | 62.347 | 1.00 | 35.16 |

| | | | | | | | | | |
|---|---|---|---|---|---|---|---|---|---|
| ATOM | 132 | C | ASP | 29 | 34.054 | -17.728 | 60.456 | 1.00 | 31.21 |
| ATOM | 133 | O | ASP | 29 | 33.542 | -16.895 | 59.717 | 1.00 | 29.93 |
| ATOM | 134 | N | LEU | 30 | 34.912 | -17.403 | 61.419 | 1.00 | 29.60 |
| ATOM | 135 | CA | LEU | 30 | 35.274 | -16.016 | 61.674 | 1.00 | 28.38 |
| ATOM | 136 | CB | LEU | 30 | 36.101 | -15.901 | 62.964 | 1.00 | 23.67 |
| ATOM | 137 | CG | LEU | 30 | 35.435 | -16.298 | 64.289 | 1.00 | 23.54 |
| ATOM | 138 | CD1 | LEU | 30 | 36.314 | -15.823 | 65.433 | 1.00 | 22.55 |
| ATOM | 139 | CD2 | LEU | 30 | 34.038 | -15.674 | 64.418 | 1.00 | 24.55 |
| ATOM | 140 | C | LEU | 30 | 36.032 | -15.390 | 60.499 | 1.00 | 29.80 |
| ATOM | 141 | O | LEU | 30 | 35.775 | -14.242 | 60.139 | 1.00 | 29.56 |
| ATOM | 142 | N | LYS | 31 | 36.963 | -16.131 | 59.906 | 1.00 | 29.13 |
| ATOM | 143 | CA | LYS | 31 | 37.704 | -15.609 | 58.770 | 1.00 | 30.46 |
| ATOM | 144 | CB | LYS | 31 | 38.823 | -16.574 | 58.365 | 1.00 | 32.24 |
| ATOM | 145 | CG | LYS | 31 | 39.970 | -16.653 | 59.374 | 1.00 | 36.80 |
| ATOM | 146 | CD | LYS | 31 | 41.091 | -17.577 | 58.885 | 1.00 | 40.49 |
| ATOM | 147 | CE | LYS | 31 | 42.291 | -17.534 | 59.829 | 1.00 | 44.52 |
| ATOM | 148 | NZ | LYS | 31 | 43.443 | -18.369 | 59.363 | 1.00 | 47.22 |
| ATOM | 149 | C | LYS | 31 | 36.746 | -15.391 | 57.599 | 1.00 | 31.28 |
| ATOM | 150 | O | LYS | 31 | 36.918 | -14.464 | 56.816 | 1.00 | 32.79 |
| ATOM | 151 | N | LYS | 32 | 35.730 | -16.243 | 57.486 | 1.00 | 30.96 |
| ATOM | 152 | CA | LYS | 32 | 34.758 | -16.116 | 56.406 | 1.00 | 32.66 |
| ATOM | 153 | CB | LYS | 32 | 33.868 | -17.364 | 56.324 | 1.00 | 32.27 |
| ATOM | 154 | CG | LYS | 32 | 32.921 | -17.362 | 55.135 | 1.00 | 34.72 |
| ATOM | 155 | CD | LYS | 32 | 32.203 | -18.701 | 54.965 | 1.00 | 39.55 |
| ATOM | 156 | CE | LYS | 32 | 31.272 | -18.678 | 53.745 | 1.00 | 42.65 |
| ATOM | 157 | NZ | LYS | 32 | 30.699 | -20.026 | 53.417 | 1.00 | 42.72 |
| ATOM | 158 | C | LYS | 32 | 33.890 | -14.868 | 56.609 | 1.00 | 32.63 |
| ATOM | 159 | O | LYS | 32 | 33.607 | -14.140 | 55.652 | 1.00 | 32.25 |
| ATOM | 160 | N | VAL | 33 | 33.463 | -14.629 | 57.847 | 1.00 | 30.17 |
| ATOM | 161 | CA | VAL | 33 | 32.654 | -13.451 | 58.149 | 1.00 | 29.03 |
| ATOM | 162 | CB | VAL | 33 | 32.154 | -13.460 | 59.626 | 1.00 | 30.49 |
| ATOM | 163 | CG1 | VAL | 33 | 31.519 | -12.123 | 59.985 | 1.00 | 31.03 |
| ATOM | 164 | CG2 | VAL | 33 | 31.130 | -14.562 | 59.815 | 1.00 | 32.03 |
| ATOM | 165 | C | VAL | 33 | 33.538 | -12.226 | 57.908 | 1.00 | 26.62 |
| ATOM | 166 | O | VAL | 33 | 33.091 | -11.237 | 57.338 | 1.00 | 22.25 |

| | | | | | | | | | |
|---|---|---|---|---|---|---|---|---|---|
| ATOM | 167 | N | MET | 34 | 34.802 | -12.321 | 58.317 | 1.00 | 25.50 |
| ATOM | 168 | CA | MET | 34 | 35.750 | -11.226 | 58.142 | 1.00 | 27.22 |
| ATOM | 169 | CB | MET | 34 | 37.108 | -11.583 | 58.748 | 1.00 | 24.41 |
| ATOM | 170 | CG | MET | 34 | 38.150 | -10.512 | 58.537 | 1.00 | 26.32 |
| ATOM | 171 | SD | MET | 34 | 39.793 | -11.040 | 59.074 | 1.00 | 32.95 |
| ATOM | 172 | CE | MET | 34 | 40.162 | -12.313 | 57.821 | 1.00 | 30.64 |
| ATOM | 173 | C | MET | 34 | 35.927 | -10.879 | 56.665 | 1.00 | 29.30 |
| ATOM | 174 | O | MET | 34 | 35.850 | -9.717 | 56.286 | 1.00 | 29.01 |
| ATOM | 175 | N | ARG | 35 | 36.164 | -11.883 | 55.827 | 1.00 | 30.96 |
| ATOM | 176 | CA | ARG | 35 | 36.340 | -11.621 | 54.403 | 1.00 | 32.99 |
| ATOM | 177 | CB | ARG | 35 | 36.664 | -12.913 | 53.641 | 1.00 | 34.85 |
| ATOM | 178 | CG | ARG | 35 | 37.948 | -13.585 | 54.081 | 1.00 | 38.82 |
| ATOM | 179 | CD | ARG | 35 | 38.377 | -14.682 | 53.126 | 1.00 | 43.22 |
| ATOM | 180 | NE | ARG | 35 | 38.963 | -15.791 | 53.869 | 1.00 | 47.35 |
| ATOM | 181 | CZ | ARG | 35 | 38.260 | -16.801 | 54.366 | 1.00 | 47.12 |
| ATOM | 182 | NH1 | ARG | 35 | 36.946 | -16.850 | 54.186 | 1.00 | 48.27 |
| ATOM | 183 | NH2 | ARG | 35 | 38.868 | -17.746 | 55.064 | 1.00 | 50.91 |
| ATOM | 184 | C | ARG | 35 | 35.090 | -10.997 | 53.797 | 1.00 | 33.31 |
| ATOM | 185 | O | ARG | 35 | 35.178 | -10.089 | 52.966 | 1.00 | 33.49 |
| ATOM | 186 | N | ARG | 36 | 33.926 | -11.493 | 54.206 | 1.00 | 32.00 |
| ATOM | 187 | CA | ARG | 36 | 32.673 | -10.982 | 53.675 | 1.00 | 31.76 |
| ATOM | 188 | CB | ARG | 36 | 31.511 | -11.857 | 54.158 | 1.00 | 29.95 |
| ATOM | 189 | CG | ARG | 36 | 30.191 | -11.607 | 53.441 | 1.00 | 31.90 |
| ATOM | 190 | CD | ARG | 36 | 30.386 | -11.434 | 51.929 | 1.00 | 33.67 |
| ATOM | 191 | NE | ARG | 36 | 29.114 | -11.263 | 51.230 | 1.00 | 38.02 |
| ATOM | 192 | CZ | ARG | 36 | 28.229 | -12.238 | 51.018 | 1.00 | 40.67 |
| ATOM | 193 | NH1 | ARG | 36 | 28.477 | -13.471 | 51.447 | 1.00 | 40.50 |
| ATOM | 194 | NH2 | ARG | 36 | 27.087 | -11.979 | 50.382 | 1.00 | 41.02 |
| ATOM | 195 | C | ARG | 36 | 32.459 | -9.510 | 54.060 | 1.00 | 31.54 |
| ATOM | 196 | O | ARG | 36 | 31.959 | -8.718 | 53.260 | 1.00 | 30.75 |
| ATOM | 197 | N | MET | 37 | 32.856 | -9.147 | 55.276 | 1.00 | 30.98 |
| ATOM | 198 | CA | MET | 37 | 32.720 | -7.774 | 55.742 | 1.00 | 30.21 |
| ATOM | 199 | CB | MET | 37 | 33.134 | -7.663 | 57.208 | 1.00 | 27.60 |
| ATOM | 200 | CG | MET | 37 | 33.102 | -6.240 | 57.761 | 1.00 | 27.98 |
| ATOM | 201 | SD | MET | 37 | 31.418 | -5.613 | 57.981 | 1.00 | 30.18 |

| | | | | | | | | |
|---|---|---|---|---|---|---|---|---|
| ATOM | 202 | CE | MET 37 | 31.115 | -6.153 | 59.683 | 1.00 | 28.30 |
| ATOM | 203 | C | MET 37 | 33.598 | -6.852 | 54.892 | 1.00 | 30.32 |
| ATOM | 204 | O | MET 37 | 33.162 | -5.782 | 54.479 | 1.00 | 31.66 |
| ATOM | 205 | N | GLN 38 | 34.835 | -7.272 | 54.642 | 1.00 | 30.60 |
| ATOM | 206 | CA | GLN 38 | 35.774 | -6.500 | 53.829 | 1.00 | 31.68 |
| ATOM | 207 | CB | GLN 38 | 37.126 | -7.206 | 53.750 | 1.00 | 32.18 |
| ATOM | 208 | CG | GLN 38 | 38.051 | -6.918 | 54.898 | 1.00 | 36.36 |
| ATOM | 209 | CD | GLN 38 | 39.318 | -7.743 | 54.831 | 1.00 | 37.65 |
| ATOM | 210 | OE1 | GLN 38 | 39.352 | -8.890 | 55.275 | 1.00 | 41.25 |
| ATOM | 211 | NE2 | GLN 38 | 40.362 | -7.170 | 54.258 | 1.00 | 39.99 |
| ATOM | 212 | C | GLN 38 | 35.241 | -6.337 | 52.419 | 1.00 | 32.20 |
| ATOM | 213 | O | GLN 38 | 35.471 | -5.318 | 51.769 | 1.00 | 32.83 |
| ATOM | 214 | N | LYS 39 | 34.541 | -7.360 | 51.947 | 1.00 | 31.94 |
| ATOM | 215 | CA | LYS 39 | 33.965 | -7.343 | 50.611 | 1.00 | 33.33 |
| ATOM | 216 | CB | LYS 39 | 33.515 | -8.754 | 50.220 | 1.00 | 34.32 |
| ATOM | 217 | CG | LYS 39 | 33.757 | -9.105 | 48.756 | 1.00 | 41.05 |
| ATOM | 218 | CD | LYS 39 | 32.994 | -8.183 | 47.799 | 1.00 | 43.55 |
| ATOM | 219 | CE | LYS 39 | 33.319 | -8.502 | 46.336 | 1.00 | 47.30 |
| ATOM | 220 | NZ | LYS 39 | 32.587 | -7.625 | 45.363 | 1.00 | 48.42 |
| ATOM | 221 | C | LYS 39 | 32.774 | -6.378 | 50.555 | 1.00 | 32.37 |
| ATOM | 222 | O | LYS 39 | 32.578 | -5.676 | 49.564 | 1.00 | 33.02 |
| ATOM | 223 | N | GLU 40 | 31.975 | -6.342 | 51.613 | 1.00 | 31.82 |
| ATOM | 224 | CA | GLU 40 | 30.831 | -5.442 | 51.632 | 1.00 | 33.50 |
| ATOM | 225 | CB | GLU 40 | 29.845 | -5.831 | 52.737 | 1.00 | 34.39 |
| ATOM | 226 | CG | GLU 40 | 29.159 | -7.167 | 52.507 | 1.00 | 36.32 |
| ATOM | 227 | CD | GLU 40 | 28.562 | -7.293 | 51.112 | 1.00 | 38.53 |
| ATOM | 228 | OE1 | GLU 40 | 27.878 | -6.350 | 50.660 | 1.00 | 39.61 |
| ATOM | 229 | OE2 | GLU 40 | 28.770 | -8.342 | 50.469 | 1.00 | 38.22 |
| ATOM | 230 | C | GLU 40 | 31.309 | -4.009 | 51.833 | 1.00 | 33.20 |
| ATOM | 231 | O | GLU 40 | 30.691 | -3.072 | 51.345 | 1.00 | 33.12 |
| ATOM | 232 | N | MET 41 | 32.409 | -3.844 | 52.556 | 1.00 | 33.18 |
| ATOM | 233 | CA | MET 41 | 32.957 | -2.515 | 52.783 | 1.00 | 34.90 |
| ATOM | 234 | CB | MET 41 | 34.173 | -2.585 | 53.706 | 1.00 | 32.91 |
| ATOM | 235 | CG | MET 41 | 33.838 | -2.927 | 55.154 | 1.00 | 34.83 |
| ATOM | 236 | SD | MET 41 | 35.327 | -2.987 | 56.170 | 1.00 | 34.41 |

| | | | | | | | | | |
|---|---|---|---|---|---|---|---|---|---|
| ATOM | 237 | CE | MET | 41 | 35.747 | -1.216 | 56.267 | 1.00 | 36.69 |
| ATOM | 238 | C | MET | 41 | 33.368 | -1.941 | 51.430 | 1.00 | 36.56 |
| ATOM | 239 | O | MET | 41 | 33.058 | -0.792 | 51.108 | 1.00 | 34.98 |
| ATOM | 240 | N | ASP | 42 | 34.054 | -2.758 | 50.639 | 1.00 | 36.46 |
| ATOM | 241 | CA | ASP | 42 | 34.508 | -2.346 | 49.317 | 1.00 | 38.91 |
| ATOM | 242 | CB | ASP | 42 | 35.318 | -3.470 | 48.674 | 1.00 | 42.09 |
| ATOM | 243 | CG | ASP | 42 | 36.130 | -2.999 | 47.490 | 1.00 | 43.40 |
| ATOM | 244 | OD1 | ASP | 42 | 37.081 | -2.216 | 47.705 | 1.00 | 45.67 |
| ATOM | 245 | OD2 | ASP | 42 | 35.817 | -3.411 | 46.350 | 1.00 | 42.51 |
| ATOM | 246 | C | ASP | 42 | 33.311 | -1.990 | 48.433 | 1.00 | 38.61 |
| ATOM | 247 | O | ASP | 42 | 33.366 | -1.036 | 47.656 | 1.00 | 39.03 |
| ATOM | 248 | N | ARG | 43 | 32.232 | -2.761 | 48.559 | 1.00 | 36.74 |
| ATOM | 249 | CA | ARG | 43 | 31.012 | -2.524 | 47.788 | 1.00 | 33.90 |
| ATOM | 250 | CB | ARG | 43 | 30.037 | -3.688 | 47.967 | 1.00 | 33.80 |
| ATOM | 251 | CG | ARG | 43 | 30.324 | -4.890 | 47.080 | 1.00 | 34.68 |
| ATOM | 252 | CD | ARG | 43 | 29.654 | -6.163 | 47.614 | 1.00 | 34.89 |
| ATOM | 253 | NE | ARG | 43 | 28.232 | -5.997 | 47.906 | 1.00 | 35.11 |
| ATOM | 254 | CZ | ARG | 43 | 27.296 | -5.729 | 46.998 | 1.00 | 37.42 |
| ATOM | 255 | NH1 | ARG | 43 | 27.620 | -5.589 | 45.719 | 1.00 | 39.98 |
| ATOM | 256 | NH2 | ARG | 43 | 26.028 | -5.615 | 47.366 | 1.00 | 36.46 |
| ATOM | 257 | C | ARG | 43 | 30.313 | -1.229 | 48.193 | 1.00 | 34.64 |
| ATOM | 258 | O | ARG | 43 | 29.712 | -0.550 | 47.357 | 1.00 | 35.89 |
| ATOM | 259 | N | GLY | 44 | 30.382 | -0.892 | 49.475 | 1.00 | 31.21 |
| ATOM | 260 | CA | GLY | 44 | 29.744 | 0.318 | 49.940 | 1.00 | 31.87 |
| ATOM | 261 | C | GLY | 44 | 30.463 | 1.579 | 49.490 | 1.00 | 33.29 |
| ATOM | 262 | O | GLY | 44 | 29.854 | 2.645 | 49.397 | 1.00 | 31.49 |
| ATOM | 263 | N | LEU | 45 | 31.756 | 1.455 | 49.200 | 1.00 | 31.44 |
| ATOM | 264 | CA | LEU | 45 | 32.563 | 2.595 | 48.778 | 1.00 | 32.24 |
| ATOM | 265 | CB | LEU | 45 | 34.033 | 2.358 | 49.129 | 1.00 | 27.43 |
| ATOM | 266 | CG | LEU | 45 | 34.415 | 2.487 | 50.601 | 1.00 | 29.59 |
| ATOM | 267 | CD1 | LEU | 45 | 35.832 | 1.992 | 50.827 | 1.00 | 30.31 |
| ATOM | 268 | CD2 | LEU | 45 | 34.281 | 3.941 | 51.022 | 1.00 | 30.45 |
| ATOM | 269 | C | LEU | 45 | 32.455 | 2.933 | 47.294 | 1.00 | 33.00 |
| ATOM | 270 | O | LEU | 45 | 32.537 | 4.098 | 46.924 | 1.00 | 32.78 |
| ATOM | 271 | N | ARG | 46 | 32.277 | 1.911 | 46.460 | 1.00 | 34.18 |

| | | | | | | | | | |
|---|---|---|---|---|---|---|---|---|---|
| ATOM | 272 | CA | ARG | 46 | 32.179 | 2.074 | 45.009 | 1.00 | 34.76 |
| ATOM | 273 | CB | ARG | 46 | 32.320 | 0.714 | 44.312 | 1.00 | 36.33 |
| ATOM | 274 | CG | ARG | 46 | 33.519 | -0.119 | 44.756 | 1.00 | 39.02 |
| ATOM | 275 | CD | ARG | 46 | 34.794 | 0.267 | 44.035 | 1.00 | 43.71 |
| ATOM | 276 | NE | ARG | 46 | 35.913 | -0.593 | 44.431 | 1.00 | 48.60 |
| ATOM | 277 | CZ | ARG | 46 | 37.142 | -0.527 | 43.915 | 1.00 | 49.59 |
| ATOM | 278 | NH1 | ARG | 46 | 37.429 | 0.359 | 42.969 | 1.00 | 49.57 |
| ATOM | 279 | NH2 | ARG | 46 | 38.091 | -1.344 | 44.354 | 1.00 | 50.09 |
| ATOM | 280 | C | ARG | 46 | 30.856 | 2.710 | 44.587 | 1.00 | 34.95 |
| ATOM | 281 | O | ARG | 46 | 29.785 | 2.361 | 45.091 | 1.00 | 32.49 |
| ATOM | 282 | N | LEU | 47 | 30.935 | 3.638 | 43.644 | 1.00 | 34.90 |
| ATOM | 283 | CA | LEU | 47 | 29.741 | 4.311 | 43.162 | 1.00 | 34.40 |
| ATOM | 284 | CB | LEU | 47 | 30.100 | 5.297 | 42.049 | 1.00 | 34.27 |
| ATOM | 285 | CG | LEU | 47 | 28.929 | 6.085 | 41.447 | 1.00 | 33.85 |
| ATOM | 286 | CD1 | LEU | 47 | 28.445 | 7.144 | 42.442 | 1.00 | 31.01 |
| ATOM | 287 | CD2 | LEU | 47 | 29.381 | 6.741 | 40.144 | 1.00 | 31.08 |
| ATOM | 288 | C | LEU | 47 | 28.727 | 3.316 | 42.625 | 1.00 | 34.52 |
| ATOM | 289 | O | LEU | 47 | 27.535 | 3.411 | 42.922 | 1.00 | 32.39 |
| ATOM | 290 | N | GLU | 48 | 29.202 | 2.353 | 41.841 | 1.00 | 34.67 |
| ATOM | 291 | CA | GLU | 48 | 28.301 | 1.378 | 41.242 | 1.00 | 36.59 |
| ATOM | 292 | CB | GLU | 48 | 29.010 | 0.589 | 40.134 | 1.00 | 38.07 |
| ATOM | 293 | CG | GLU | 48 | 30.205 | -0.248 | 40.562 | 1.00 | 39.26 |
| ATOM | 294 | CD | GLU | 48 | 31.499 | 0.534 | 40.580 | 1.00 | 40.85 |
| ATOM | 295 | OE1 | GLU | 48 | 32.571 | -0.106 | 40.497 | 1.00 | 44.46 |
| ATOM | 296 | OE2 | GLU | 48 | 31.454 | 1.779 | 40.682 | 1.00 | 38.21 |
| ATOM | 297 | C | GLU | 48 | 27.600 | 0.406 | 42.188 | 1.00 | 37.46 |
| ATOM | 298 | O | GLU | 48 | 26.654 | -0.268 | 41.778 | 1.00 | 37.82 |
| ATOM | 299 | N | THR | 49 | 28.037 | 0.321 | 43.441 | 1.00 | 36.85 |
| ATOM | 300 | CA | THR | 49 | 27.371 | -0.591 | 44.370 | 1.00 | 36.40 |
| ATOM | 301 | CB | THR | 49 | 28.212 | -1.855 | 44.645 | 1.00 | 34.37 |
| ATOM | 302 | OG1 | THR | 49 | 29.554 | -1.480 | 44.969 | 1.00 | 33.33 |
| ATOM | 303 | CG2 | THR | 49 | 28.215 | -2.770 | 43.437 | 1.00 | 32.44 |
| ATOM | 304 | C | THR | 49 | 27.032 | 0.037 | 45.703 | 1.00 | 38.54 |
| ATOM | 305 | O | THR | 49 | 26.536 | -0.647 | 46.599 | 1.00 | 40.86 |
| ATOM | 306 | N | HIS | 50 | 27.272 | 1.335 | 45.842 | 1.00 | 38.89 |

| | | | | | | | | | |
|---|---|---|---|---|---|---|---|---|---|
| ATOM | 307 | CA | HIS | 50 | 26.994 | 1.990 | 47.115 | 1.00 | 41.74 |
| ATOM | 308 | CB | HIS | 50 | 27.548 | 3.422 | 47.130 | 1.00 | 44.04 |
| ATOM | 309 | CG | HIS | 50 | 26.666 | 4.426 | 46.451 | 1.00 | 46.35 |
| ATOM | 310 | CD2 | HIS | 50 | 25.795 | 5.331 | 46.959 | 1.00 | 48.65 |
| ATOM | 311 | ND1 | HIS | 50 | 26.607 | 4.565 | 45.081 | 1.00 | 47.18 |
| ATOM | 312 | CE1 | HIS | 50 | 25.738 | 5.512 | 44.772 | 1.00 | 48.13 |
| ATOM | 313 | NE2 | HIS | 50 | 25.231 | 5.993 | 45.894 | 1.00 | 49.20 |
| ATOM | 314 | C | HIS | 50 | 25.512 | 2.030 | 47.466 | 1.00 | 42.66 |
| ATOM | 315 | O | HIS | 50 | 25.153 | 2.046 | 48.642 | 1.00 | 42.85 |
| ATOM | 316 | N | GLU | 51 | 24.657 | 2.034 | 46.447 | 1.00 | 43.12 |
| ATOM | 317 | CA | GLU | 51 | 23.213 | 2.120 | 46.645 | 1.00 | 44.07 |
| ATOM | 318 | CB | GLU | 51 | 22.555 | 2.574 | 45.329 | 1.00 | 44.83 |
| ATOM | 319 | CG | GLU | 51 | 21.051 | 2.824 | 45.399 | 1.00 | 46.43 |
| ATOM | 320 | CD | GLU | 51 | 20.531 | 3.691 | 44.243 | 1.00 | 48.89 |
| ATOM | 321 | OE1 | GLU | 51 | 20.822 | 3.385 | 43.064 | 1.00 | 46.31 |
| ATOM | 322 | OE2 | GLU | 51 | 19.821 | 4.683 | 44.522 | 1.00 | 50.83 |
| ATOM | 323 | C | GLU | 51 | 22.543 | 0.848 | 47.179 | 1.00 | 44.27 |
| ATOM | 324 | O | GLU | 51 | 21.630 | 0.925 | 48.000 | 1.00 | 45.14 |
| ATOM | 325 | N | GLU | 52 | 22.991 | -0.317 | 46.723 | 1.00 | 44.47 |
| ATOM | 326 | CA | GLU | 52 | 22.422 | -1.585 | 47.178 | 1.00 | 44.81 |
| ATOM | 327 | CB | GLU | 52 | 22.199 | -2.521 | 45.988 | 1.00 | 47.15 |
| ATOM | 328 | CG | GLU | 52 | 23.485 | -2.920 | 45.264 | 1.00 | 53.66 |
| ATOM | 329 | CD | GLU | 52 | 23.698 | -2.164 | 43.951 | 1.00 | 57.63 |
| ATOM | 330 | OE1 | GLU | 52 | 23.646 | -0.909 | 43.953 | 1.00 | 55.90 |
| ATOM | 331 | OE2 | GLU | 52 | 23.925 | -2.835 | 42.917 | 1.00 | 57.72 |
| ATOM | 332 | C | GLU | 52 | 23.313 | -2.297 | 48.206 | 1.00 | 42.49 |
| ATOM | 333 | O | GLU | 52 | 23.052 | -3.441 | 48.575 | 1.00 | 43.45 |
| ATOM | 334 | N | ALA | 53 | 24.362 | -1.626 | 48.666 | 1.00 | 39.72 |
| ATOM | 335 | CA | ALA | 53 | 25.285 | -2.224 | 49.628 | 1.00 | 37.01 |
| ATOM | 336 | CB | ALA | 53 | 26.589 | -1.438 | 49.645 | 1.00 | 35.23 |
| ATOM | 337 | C | ALA | 53 | 24.700 | -2.291 | 51.038 | 1.00 | 35.27 |
| ATOM | 338 | O | ALA | 53 | 24.125 | -1.321 | 51.528 | 1.00 | 34.63 |
| ATOM | 339 | N | SER | 54 | 24.845 | -3.439 | 51.689 | 1.00 | 32.88 |
| ATOM | 340 | CA | SER | 54 | 24.339 | -3.594 | 53.052 | 1.00 | 32.06 |
| ATOM | 341 | CB | SER | 54 | 24.397 | -5.062 | 53.476 | 1.00 | 30.23 |

| | | | | | | | | | |
|---|---|---|---|---|---|---|---|---|---|
| ATOM | 342 | OG | SER | 54 | 25.694 | -5.576 | 53.261 | 1.00 | 35.67 |
| ATOM | 343 | C | SER | 54 | 25.188 | -2.741 | 53.990 | 1.00 | 28.49 |
| ATOM | 344 | O | SER | 54 | 24.682 | -2.147 | 54.934 | 1.00 | 29.57 |
| ATOM | 345 | N | VAL | 55 | 26.485 | -2.684 | 53.724 | 1.00 | 28.44 |
| ATOM | 346 | CA | VAL | 55 | 27.386 | -1.876 | 54.535 | 1.00 | 28.63 |
| ATOM | 347 | CB | VAL | 55 | 28.737 | -2.594 | 54.726 | 1.00 | 27.89 |
| ATOM | 348 | CG1 | VAL | 55 | 29.660 | -1.766 | 55.599 | 1.00 | 26.89 |
| ATOM | 349 | CG2 | VAL | 55 | 28.497 | -3.957 | 55.365 | 1.00 | 27.94 |
| ATOM | 350 | C | VAL | 55 | 27.559 | -0.551 | 53.788 | 1.00 | 29.80 |
| ATOM | 351 | O | VAL | 55 | 28.367 | -0.430 | 52.868 | 1.00 | 28.14 |
| ATOM | 352 | N | LYS | 56 | 26.787 | 0.446 | 54.205 | 1.00 | 31.68 |
| ATOM | 353 | CA | LYS | 56 | 26.788 | 1.750 | 53.550 | 1.00 | 30.06 |
| ATOM | 354 | CB | LYS | 56 | 25.727 | 2.628 | 54.203 | 1.00 | 29.96 |
| ATOM | 355 | CG | LYS | 56 | 24.312 | 2.124 | 53.933 | 1.00 | 29.47 |
| ATOM | 356 | CD | LYS | 56 | 23.279 | 2.935 | 54.689 | 1.00 | 31.68 |
| ATOM | 357 | CE | LYS | 56 | 23.417 | 2.767 | 56.196 | 1.00 | 30.78 |
| ATOM | 358 | NZ | LYS | 56 | 22.911 | 1.428 | 56.648 | 1.00 | 36.66 |
| ATOM | 359 | C | LYS | 56 | 28.087 | 2.535 | 53.374 | 1.00 | 28.33 |
| ATOM | 360 | O | LYS | 56 | 28.222 | 3.256 | 52.388 | 1.00 | 30.83 |
| ATOM | 361 | N | MET | 57 | 29.044 | 2.410 | 54.287 | 1.00 | 25.97 |
| ATOM | 362 | CA | MET | 57 | 30.299 | 3.149 | 54.137 | 1.00 | 23.92 |
| ATOM | 363 | CB | MET | 57 | 31.098 | 2.577 | 52.964 | 1.00 | 24.05 |
| ATOM | 364 | CG | MET | 57 | 31.383 | 1.078 | 53.075 | 1.00 | 27.54 |
| ATOM | 365 | SD | MET | 57 | 32.303 | 0.659 | 54.580 | 1.00 | 26.48 |
| ATOM | 366 | CE | MET | 57 | 33.991 | 1.127 | 54.113 | 1.00 | 21.76 |
| ATOM | 367 | C | MET | 57 | 30.006 | 4.643 | 53.887 | 1.00 | 26.44 |
| ATOM | 368 | O | MET | 57 | 30.460 | 5.237 | 52.903 | 1.00 | 24.39 |
| ATOM | 369 | N | LEU | 58 | 29.250 | 5.235 | 54.803 | 1.00 | 26.42 |
| ATOM | 370 | CA | LEU | 58 | 28.843 | 6.630 | 54.713 | 1.00 | 26.83 |
| ATOM | 371 | CB | LEU | 58 | 27.684 | 6.884 | 55.677 | 1.00 | 24.27 |
| ATOM | 372 | CG | LEU | 58 | 26.440 | 6.043 | 55.386 | 1.00 | 30.26 |
| ATOM | 373 | CD1 | LEU | 58 | 25.401 | 6.250 | 56.473 | 1.00 | 28.51 |
| ATOM | 374 | CD2 | LEU | 58 | 25.874 | 6.430 | 54.016 | 1.00 | 31.10 |
| ATOM | 375 | C | LEU | 58 | 29.932 | 7.665 | 54.965 | 1.00 | 25.48 |
| ATOM | 376 | O | LEU | 58 | 30.495 | 7.742 | 56.053 | 1.00 | 25.30 |

| | | | | | | | | | |
|---|---|---|---|---|---|---|---|---|---|
| ATOM | 377 | N | PRO | 59 | 30.242 | 8.476 | 53.946 | 1.00 | 24.56 |
| ATOM | 378 | CD | PRO | 59 | 29.764 | 8.341 | 52.557 | 1.00 | 24.76 |
| ATOM | 379 | CA | PRO | 59 | 31.262 | 9.528 | 54.063 | 1.00 | 26.48 |
| ATOM | 380 | CB | PRO | 59 | 31.217 | 10.196 | 52.686 | 1.00 | 26.76 |
| ATOM | 381 | CG | PRO | 59 | 30.865 | 9.036 | 51.769 | 1.00 | 26.41 |
| ATOM | 382 | C | PRO | 59 | 30.820 | 10.478 | 55.190 | 1.00 | 26.49 |
| ATOM | 383 | O | PRO | 59 | 29.656 | 10.863 | 55.239 | 1.00 | 28.20 |
| ATOM | 384 | N | THR | 60 | 31.728 | 10.845 | 56.092 | 1.00 | 27.28 |
| ATOM | 385 | CA | THR | 60 | 31.372 | 11.720 | 57.220 | 1.00 | 27.77 |
| ATOM | 386 | CB | THR | 60 | 31.994 | 11.217 | 58.544 | 1.00 | 24.87 |
| ATOM | 387 | OG1 | THR | 60 | 33.400 | 11.482 | 58.536 | 1.00 | 22.66 |
| ATOM | 388 | CG2 | THR | 60 | 31.767 | 9.713 | 58.726 | 1.00 | 28.80 |
| ATOM | 389 | C | THR | 60 | 31.800 | 13.196 | 57.085 | 1.00 | 30.72 |
| ATOM | 390 | O | THR | 60 | 31.405 | 14.041 | 57.897 | 1.00 | 29.67 |
| ATOM | 391 | N | TYR | 61 | 32.623 | 13.485 | 56.084 | 1.00 | 30.13 |
| ATOM | 392 | CA | TYR | 61 | 33.144 | 14.824 | 55.844 | 1.00 | 33.87 |
| ATOM | 393 | CB | TYR | 61 | 32.005 | 15.837 | 55.684 | 1.00 | 32.96 |
| ATOM | 394 | CG | TYR | 61 | 31.409 | 15.730 | 54.298 | 1.00 | 35.37 |
| ATOM | 395 | CD1 | TYR | 61 | 32.084 | 16.251 | 53.192 | 1.00 | 36.43 |
| ATOM | 396 | CE1 | TYR | 61 | 31.621 | 16.036 | 51.890 | 1.00 | 34.05 |
| ATOM | 397 | CD2 | TYR | 61 | 30.244 | 14.995 | 54.068 | 1.00 | 34.99 |
| ATOM | 398 | CE2 | TYR | 61 | 29.778 | 14.772 | 52.768 | 1.00 | 33.96 |
| ATOM | 399 | CZ | TYR | 61 | 30.475 | 15.294 | 51.689 | 1.00 | 33.72 |
| ATOM | 400 | OH | TYR | 61 | 30.039 | 15.064 | 50.402 | 1.00 | 37.69 |
| ATOM | 401 | C | TYR | 61 | 34.156 | 15.264 | 56.890 | 1.00 | 34.78 |
| ATOM | 402 | O | TYR | 61 | 34.712 | 16.357 | 56.806 | 1.00 | 34.09 |
| ATOM | 403 | N | VAL | 62 | 34.407 | 14.407 | 57.875 | 1.00 | 36.47 |
| ATOM | 404 | CA | VAL | 62 | 35.426 | 14.713 | 58.869 | 1.00 | 37.40 |
| ATOM | 405 | CB | VAL | 62 | 35.283 | 13.825 | 60.116 | 1.00 | 37.42 |
| ATOM | 406 | CG1 | VAL | 62 | 36.410 | 14.107 | 61.089 | 1.00 | 32.97 |
| ATOM | 407 | CG2 | VAL | 62 | 33.937 | 14.073 | 60.774 | 1.00 | 36.34 |
| ATOM | 408 | C | VAL | 62 | 36.695 | 14.335 | 58.104 | 1.00 | 41.04 |
| ATOM | 409 | O | VAL | 62 | 36.944 | 13.153 | 57.865 | 1.00 | 40.85 |
| ATOM | 410 | N | ARG | 63 | 37.475 | 15.331 | 57.692 | 1.00 | 43.48 |
| ATOM | 411 | CA | ARG | 63 | 38.682 | 15.070 | 56.909 | 1.00 | 48.27 |

| | | | | | | | | | |
|---|---|---|---|---|---|---|---|---|---|
| ATOM | 412 | CB | ARG | 63 | 38.843 | 16.126 | 55.814 | 1.00 | 47.25 |
| ATOM | 413 | CG | ARG | 63 | 37.735 | 16.112 | 54.783 | 1.00 | 49.66 |
| ATOM | 414 | CD | ARG | 63 | 37.648 | 17.447 | 54.061 | 1.00 | 50.62 |
| ATOM | 415 | NE | ARG | 63 | 36.482 | 17.523 | 53.185 | 1.00 | 51.28 |
| ATOM | 416 | CZ | ARG | 63 | 36.405 | 16.961 | 51.982 | 1.00 | 50.52 |
| ATOM | 417 | NH1 | ARG | 63 | 37.430 | 16.274 | 51.492 | 1.00 | 48.44 |
| ATOM | 418 | NH2 | ARG | 63 | 35.295 | 17.089 | 51.268 | 1.00 | 49.50 |
| ATOM | 419 | C | ARG | 63 | 39.952 | 15.006 | 57.728 | 1.00 | 50.30 |
| ATOM | 420 | O | ARG | 63 | 39.998 | 15.478 | 58.860 | 1.00 | 49.69 |
| ATOM | 421 | N | SER | 64 | 40.987 | 14.431 | 57.128 | 1.00 | 54.64 |
| ATOM | 422 | CA | SER | 64 | 42.276 | 14.280 | 57.783 | 1.00 | 60.87 |
| ATOM | 423 | CB | SER | 64 | 43.315 | 13.760 | 56.794 | 1.00 | 60.13 |
| ATOM | 424 | OG | SER | 64 | 44.492 | 13.381 | 57.481 | 1.00 | 62.83 |
| ATOM | 425 | C | SER | 64 | 42.760 | 15.583 | 58.398 | 1.00 | 65.69 |
| ATOM | 426 | O | SER | 64 | 42.952 | 16.584 | 57.703 | 1.00 | 65.99 |
| ATOM | 427 | N | THR | 65 | 42.961 | 15.530 | 59.714 | 1.00 | 71.92 |
| ATOM | 428 | CA | THR | 65 | 43.402 | 16.649 | 60.545 | 1.00 | 77.78 |
| ATOM | 429 | CB | THR | 65 | 44.529 | 16.194 | 61.524 | 1.00 | 78.35 |
| ATOM | 430 | OG1 | THR | 65 | 44.959 | 17.309 | 62.317 | 1.00 | 79.07 |
| ATOM | 431 | CG2 | THR | 65 | 45.714 | 15.611 | 60.757 | 1.00 | 79.19 |
| ATOM | 432 | C | THR | 65 | 43.839 | 17.925 | 59.817 | 1.00 | 80.90 |
| ATOM | 433 | O | THR | 65 | 45.033 | 18.188 | 59.654 | 1.00 | 80.93 |
| ATOM | 434 | N | PRO | 66 | 42.863 | 18.732 | 59.364 | 1.00 | 83.72 |
| ATOM | 435 | CD | PRO | 66 | 41.410 | 18.469 | 59.372 | 1.00 | 84.56 |
| ATOM | 436 | CA | PRO | 66 | 43.162 | 19.983 | 58.661 | 1.00 | 85.58 |
| ATOM | 437 | CB | PRO | 66 | 41.871 | 20.254 | 57.897 | 1.00 | 85.53 |
| ATOM | 438 | CG | PRO | 66 | 40.827 | 19.776 | 58.864 | 1.00 | 85.36 |
| ATOM | 439 | C | PRO | 66 | 43.468 | 21.057 | 59.710 | 1.00 | 87.07 |
| ATOM | 440 | O | PRO | 66 | 42.581 | 21.812 | 60.119 | 1.00 | 87.87 |
| ATOM | 441 | N | GLU | 67 | 44.726 | 21.109 | 60.144 | 1.00 | 87.71 |
| ATOM | 442 | CA | GLU | 67 | 45.162 | 22.055 | 61.169 | 1.00 | 87.66 |
| ATOM | 443 | CB | GLU | 67 | 46.683 | 22.238 | 61.110 | 1.00 | 88.42 |
| ATOM | 444 | CG | GLU | 67 | 47.283 | 22.824 | 62.384 | 1.00 | 89.15 |
| ATOM | 445 | CD | GLU | 67 | 46.871 | 22.058 | 63.636 | 1.00 | 89.71 |
| ATOM | 446 | OE1 | GLU | 67 | 45.689 | 22.150 | 64.037 | 1.00 | 89.95 |

| | | | | | | | | |
|---|---|---|---|---|---|---|---|---|
| ATOM | 447 | OE2 | GLU | 67 | 47.728 | 21.359 | 64.217 | 1.00 89.51 |
| ATOM | 448 | C | GLU | 67 | 44.463 | 23.413 | 61.095 | 1.00 86.97 |
| ATOM | 449 | O | GLU | 67 | 44.203 | 23.944 | 60.013 | 1.00 86.95 |
| ATOM | 450 | N | GLY | 68 | 44.160 | 23.962 | 62.266 | 1.00 85.72 |
| ATOM | 451 | CA | GLY | 68 | 43.475 | 25.237 | 62.344 | 1.00 83.56 |
| ATOM | 452 | C | GLY | 68 | 42.274 | 25.073 | 63.251 | 1.00 82.01 |
| ATOM | 453 | O | GLY | 68 | 41.136 | 24.970 | 62.784 | 1.00 82.39 |
| ATOM | 454 | N | SER | 69 | 42.530 | 25.038 | 64.555 | 1.00 79.39 |
| ATOM | 455 | CA | SER | 69 | 41.469 | 24.869 | 65.537 | 1.00 77.31 |
| ATOM | 456 | CB | SER | 69 | 41.855 | 23.784 | 66.542 | 1.00 77.69 |
| ATOM | 457 | OG | SER | 69 | 40.877 | 23.677 | 67.561 | 1.00 78.20 |
| ATOM | 458 | C | SER | 69 | 41.118 | 26.143 | 66.294 | 1.00 75.21 |
| ATOM | 459 | O | SER | 69 | 41.993 | 26.857 | 66.784 | 1.00 74.23 |
| ATOM | 460 | N | GLU | 70 | 39.822 | 26.413 | 66.386 | 1.00 73.26 |
| ATOM | 461 | CA | GLU | 70 | 39.328 | 27.581 | 67.096 | 1.00 71.89 |
| ATOM | 462 | CB | GLU | 70 | 38.004 | 28.042 | 66.482 | 1.00 73.40 |
| ATOM | 463 | CG | GLU | 70 | 37.897 | 29.544 | 66.297 | 1.00 77.84 |
| ATOM | 464 | CD | GLU | 70 | 38.900 | 30.073 | 65.285 | 1.00 80.27 |
| ATOM | 465 | OE1 | GLU | 70 | 38.763 | 29.757 | 64.082 | 1.00 81.41 |
| ATOM | 466 | OE2 | GLU | 70 | 39.830 | 30.801 | 65.692 | 1.00 81.33 |
| ATOM | 467 | C | GLU | 70 | 39.107 | 27.144 | 68.543 | 1.00 69.48 |
| ATOM | 468 | O | GLU | 70 | 38.409 | 26.163 | 68.789 | 1.00 69.73 |
| ATOM | 469 | N | VAL | 71 | 39.701 | 27.853 | 69.499 | 1.00 65.92 |
| ATOM | 470 | CA | VAL | 71 | 39.536 | 27.490 | 70.904 | 1.00 62.64 |
| ATOM | 471 | CB | VAL | 71 | 40.760 | 27.909 | 71.746 | 1.00 61.59 |
| ATOM | 472 | CG1 | VAL | 71 | 41.993 | 27.156 | 71.275 | 1.00 61.91 |
| ATOM | 473 | CG2 | VAL | 71 | 40.979 | 29.406 | 71.642 | 1.00 61.78 |
| ATOM | 474 | C | VAL | 71 | 38.278 | 28.105 | 71.510 | 1.00 61.05 |
| ATOM | 475 | O | VAL | 71 | 37.608 | 28.919 | 70.877 | 1.00 61.02 |
| ATOM | 476 | N | GLY | 72 | 37.952 | 27.700 | 72.734 | 1.00 59.60 |
| ATOM | 477 | CA | GLY | 72 | 36.769 | 28.225 | 73.390 | 1.00 58.10 |
| ATOM | 478 | C | GLY | 72 | 35.841 | 27.169 | 73.967 | 1.00 57.74 |
| ATOM | 479 | O | GLY | 72 | 36.178 | 25.982 | 74.006 | 1.00 58.27 |
| ATOM | 480 | N | ASP | 73 | 34.664 | 27.607 | 74.410 | 1.00 55.55 |
| ATOM | 481 | CA | ASP | 73 | 33.663 | 26.724 | 75.003 | 1.00 54.21 |

| | | | | | | | | | |
|---|---|---|---|---|---|---|---|---|---|
| ATOM | 482 | CB | ASP | 73 | 32.973 | 27.426 | 76.181 | 1.00 | 57.20 |
| ATOM | 483 | CG | ASP | 73 | 33.846 | 27.496 | 77.424 | 1.00 | 59.78 |
| ATOM | 484 | OD1 | ASP | 73 | 35.046 | 27.830 | 77.299 | 1.00 | 61.37 |
| ATOM | 485 | OD2 | ASP | 73 | 33.324 | 27.225 | 78.529 | 1.00 | 60.87 |
| ATOM | 486 | C | ASP | 73 | 32.599 | 26.310 | 73.994 | 1.00 | 52.36 |
| ATOM | 487 | O | ASP | 73 | 31.936 | 27.161 | 73.406 | 1.00 | 52.44 |
| ATOM | 488 | N | PHE | 74 | 32.424 | 25.005 | 73.800 | 1.00 | 49.73 |
| ATOM | 489 | CA | PHE | 74 | 31.412 | 24.519 | 72.866 | 1.00 | 46.98 |
| ATOM | 490 | CB | PHE | 74 | 32.019 | 23.571 | 71.837 | 1.00 | 46.41 |
| ATOM | 491 | CG | PHE | 74 | 33.117 | 24.179 | 71.030 | 1.00 | 47.09 |
| ATOM | 492 | CD1 | PHE | 74 | 34.335 | 24.492 | 71.618 | 1.00 | 47.62 |
| ATOM | 493 | CD2 | PHE | 74 | 32.930 | 24.452 | 69.681 | 1.00 | 47.01 |
| ATOM | 494 | CE1 | PHE | 74 | 35.359 | 25.071 | 70.874 | 1.00 | 49.47 |
| ATOM | 495 | CE2 | PHE | 74 | 33.943 | 25.031 | 68.924 | 1.00 | 48.12 |
| ATOM | 496 | CZ | PHE | 74 | 35.161 | 25.342 | 69.520 | 1.00 | 48.82 |
| ATOM | 497 | C | PHE | 74 | 30.316 | 23.783 | 73.601 | 1.00 | 45.68 |
| ATOM | 498 | O | PHE | 74 | 30.485 | 23.382 | 74.745 | 1.00 | 46.35 |
| ATOM | 499 | N | LEU | 75 | 29.185 | 23.615 | 72.932 | 1.00 | 45.12 |
| ATOM | 500 | CA | LEU | 75 | 28.064 | 22.895 | 73.501 | 1.00 | 44.80 |
| ATOM | 501 | CB | LEU | 75 | 26.769 | 23.686 | 73.333 | 1.00 | 43.29 |
| ATOM | 502 | CG | LEU | 75 | 25.535 | 23.023 | 73.959 | 1.00 | 45.05 |
| ATOM | 503 | CD1 | LEU | 75 | 25.529 | 23.278 | 75.466 | 1.00 | 41.53 |
| ATOM | 504 | CD2 | LEU | 75 | 24.259 | 23.571 | 73.326 | 1.00 | 43.45 |
| ATOM | 505 | C | LEU | 75 | 27.971 | 21.598 | 72.708 | 1.00 | 46.04 |
| ATOM | 506 | O | LEU | 75 | 28.087 | 21.611 | 71.479 | 1.00 | 46.97 |
| ATOM | 507 | N | SER | 76 | 27.770 | 20.484 | 73.405 | 1.00 | 45.48 |
| ATOM | 508 | CA | SER | 76 | 27.664 | 19.189 | 72.744 | 1.00 | 43.73 |
| ATOM | 509 | CB | SER | 76 | 28.837 | 18.295 | 73.143 | 1.00 | 43.52 |
| ATOM | 510 | OG | SER | 76 | 30.040 | 18.741 | 72.551 | 1.00 | 44.64 |
| ATOM | 511 | C | SER | 76 | 26.361 | 18.469 | 73.051 | 1.00 | 41.60 |
| ATOM | 512 | O | SER | 76 | 26.026 | 18.242 | 74.209 | 1.00 | 40.88 |
| ATOM | 513 | N | LEU | 77 | 25.617 | 18.130 | 72.007 | 1.00 | 41.06 |
| ATOM | 514 | CA | LEU | 77 | 24.369 | 17.397 | 72.175 | 1.00 | 43.50 |
| ATOM | 515 | CB | LEU | 77 | 23.281 | 17.918 | 71.225 | 1.00 | 43.84 |
| ATOM | 516 | CG | LEU | 77 | 22.750 | 19.346 | 71.401 | 1.00 | 45.70 |

| | | | | | | | | | | |
|---|---|---|---|---|---|---|---|---|---|---|
| ATOM | 517 | CD1 | LEU | 77 | 21.587 | 19.577 | 70.442 | 1.00 | 45.96 |
| ATOM | 518 | CD2 | LEU | 77 | 22.284 | 19.550 | 72.835 | 1.00 | 46.75 |
| ATOM | 519 | C | LEU | 77 | 24.662 | 15.933 | 71.851 | 1.00 | 43.78 |
| ATOM | 520 | O | LEU | 77 | 25.529 | 15.635 | 71.026 | 1.00 | 43.07 |
| ATOM | 521 | N | ASP | 78 | 23.946 | 15.021 | 72.496 | 1.00 | 44.50 |
| ATOM | 522 | CA | ASP | 78 | 24.151 | 13.604 | 72.244 | 1.00 | 44.82 |
| ATOM | 523 | CB | ASP | 78 | 25.126 | 13.026 | 73.271 | 1.00 | 44.71 |
| ATOM | 524 | CG | ASP | 78 | 25.597 | 11.628 | 72.905 | 1.00 | 45.55 |
| ATOM | 525 | OD1 | ASP | 78 | 24.738 | 10.750 | 72.672 | 1.00 | 41.76 |
| ATOM | 526 | OD2 | ASP | 78 | 26.828 | 11.410 | 72.853 | 1.00 | 45.32 |
| ATOM | 527 | C | ASP | 78 | 22.838 | 12.829 | 72.276 | 1.00 | 44.74 |
| ATOM | 528 | O | ASP | 78 | 22.245 | 12.633 | 73.333 | 1.00 | 45.25 |
| ATOM | 529 | N | LEU | 79 | 22.385 | 12.398 | 71.107 | 1.00 | 45.72 |
| ATOM | 530 | CA | LEU | 79 | 21.154 | 11.630 | 70.994 | 1.00 | 47.25 |
| ATOM | 531 | CB | LEU | 79 | 20.137 | 12.351 | 70.116 | 1.00 | 45.37 |
| ATOM | 532 | CG | LEU | 79 | 18.865 | 11.530 | 69.915 | 1.00 | 43.65 |
| ATOM | 533 | CD1 | LEU | 79 | 18.067 | 11.553 | 71.200 | 1.00 | 46.42 |
| ATOM | 534 | CD2 | LEU | 79 | 18.045 | 12.086 | 68.777 | 1.00 | 43.81 |
| ATOM | 535 | C | LEU | 79 | 21.491 | 10.295 | 70.354 | 1.00 | 49.50 |
| ATOM | 536 | O | LEU | 79 | 22.073 | 10.249 | 69.274 | 1.00 | 49.35 |
| ATOM | 537 | N | GLY | 80 | 21.123 | 9.207 | 71.016 | 1.00 | 52.24 |
| ATOM | 538 | CA | GLY | 80 | 21.421 | 7.902 | 70.466 | 1.00 | 56.31 |
| ATOM | 539 | C | GLY | 80 | 20.965 | 6.833 | 71.420 | 1.00 | 59.13 |
| ATOM | 540 | O | GLY | 80 | 20.278 | 5.896 | 71.027 | 1.00 | 60.86 |
| ATOM | 541 | N | GLY | 81 | 21.360 | 6.966 | 72.679 | 1.00 | 62.30 |
| ATOM | 542 | CA | GLY | 81 | 20.940 | 6.002 | 73.674 | 1.00 | 65.60 |
| ATOM | 543 | C | GLY | 81 | 19.551 | 6.395 | 74.137 | 1.00 | 67.84 |
| ATOM | 544 | O | GLY | 81 | 18.936 | 7.301 | 73.564 | 1.00 | 69.00 |
| ATOM | 545 | N | THR | 82 | 19.047 | 5.722 | 75.165 | 1.00 | 69.33 |
| ATOM | 546 | CA | THR | 82 | 17.726 | 6.037 | 75.695 | 1.00 | 70.36 |
| ATOM | 547 | CB | THR | 82 | 17.110 | 4.824 | 76.418 | 1.00 | 71.43 |
| ATOM | 548 | OG1 | THR | 82 | 18.032 | 4.332 | 77.398 | 1.00 | 71.60 |
| ATOM | 549 | CG2 | THR | 82 | 16.784 | 3.716 | 75.420 | 1.00 | 71.87 |
| ATOM | 550 | C | THR | 82 | 17.846 | 7.196 | 76.679 | 1.00 | 70.10 |
| ATOM | 551 | O | THR | 82 | 16.933 | 7.458 | 77.464 | 1.00 | 71.18 |

| | | | | | | | | | |
|---|---|---|---|---|---|---|---|---|---|
| ATOM | 552 | N | ASN | 83 | 18.981 | 7.887 | 76.625 | 1.00 | 69.08 |
| ATOM | 553 | CA | ASN | 83 | 19.232 | 9.017 | 77.508 | 1.00 | 68.14 |
| ATOM | 554 | CB | ASN | 83 | 20.161 | 8.584 | 78.646 | 1.00 | 69.98 |
| ATOM | 555 | CG | ASN | 83 | 19.862 | 9.300 | 79.948 | 1.00 | 70.80 |
| ATOM | 556 | OD1 | ASN | 83 | 20.627 | 9.213 | 80.909 | 1.00 | 71.46 |
| ATOM | 557 | ND2 | ASN | 83 | 18.739 | 10.004 | 79.990 | 1.00 | 72.56 |
| ATOM | 558 | C | ASN | 83 | 19.866 | 10.177 | 76.738 | 1.00 | 66.16 |
| ATOM | 559 | O | ASN | 83 | 21.050 | 10.136 | 76.407 | 1.00 | 66.52 |
| ATOM | 560 | N | PHE | 84 | 19.073 | 11.203 | 76.447 | 1.00 | 63.41 |
| ATOM | 561 | CA | PHE | 84 | 19.567 | 12.375 | 75.728 | 1.00 | 60.93 |
| ATOM | 562 | CB | PHE | 84 | 18.398 | 13.227 | 75.241 | 1.00 | 61.87 |
| ATOM | 563 | CG | PHE | 84 | 18.817 | 14.477 | 74.528 | 1.00 | 63.55 |
| ATOM | 564 | CD1 | PHE | 84 | 18.419 | 15.724 | 74.993 | 1.00 | 63.38 |
| ATOM | 565 | CD2 | PHE | 84 | 19.599 | 14.409 | 73.381 | 1.00 | 64.28 |
| ATOM | 566 | CE1 | PHE | 84 | 18.793 | 16.888 | 74.325 | 1.00 | 64.07 |
| ATOM | 567 | CE2 | PHE | 84 | 19.979 | 15.568 | 72.705 | 1.00 | 65.31 |
| ATOM | 568 | CZ | PHE | 84 | 19.574 | 16.810 | 73.179 | 1.00 | 64.75 |
| ATOM | 569 | C | PHE | 84 | 20.442 | 13.206 | 76.658 | 1.00 | 59.07 |
| ATOM | 570 | O | PHE | 84 | 20.011 | 13.582 | 77.744 | 1.00 | 59.19 |
| ATOM | 571 | N | ARG | 85 | 21.665 | 13.500 | 76.232 | 1.00 | 57.25 |
| ATOM | 572 | CA | ARG | 85 | 22.583 | 14.272 | 77.064 | 1.00 | 56.05 |
| ATOM | 573 | CB | ARG | 85 | 23.857 | 13.467 | 77.344 | 1.00 | 56.68 |
| ATOM | 574 | CG | ARG | 85 | 23.605 | 12.044 | 77.828 | 1.00 | 58.78 |
| ATOM | 575 | CD | ARG | 85 | 24.896 | 11.367 | 78.267 | 1.00 | 59.39 |
| ATOM | 576 | NE | ARG | 85 | 25.908 | 11.348 | 77.213 | 1.00 | 59.87 |
| ATOM | 577 | CZ | ARG | 85 | 27.068 | 11.994 | 77.282 | 1.00 | 60.09 |
| ATOM | 578 | NH1 | ARG | 85 | 27.366 | 12.713 | 78.357 | 1.00 | 59.50 |
| ATOM | 579 | NH2 | ARG | 85 | 27.931 | 11.920 | 76.277 | 1.00 | 60.92 |
| ATOM | 580 | C | ARG | 85 | 22.966 | 15.602 | 76.433 | 1.00 | 55.07 |
| ATOM | 581 | O | ARG | 85 | 23.038 | 15.725 | 75.209 | 1.00 | 54.93 |
| ATOM | 582 | N | VAL | 86 | 23.211 | 16.593 | 77.288 | 1.00 | 53.13 |
| ATOM | 583 | CA | VAL | 86 | 23.598 | 17.935 | 76.861 | 1.00 | 51.01 |
| ATOM | 584 | CB | VAL | 86 | 22.425 | 18.939 | 77.003 | 1.00 | 51.19 |
| ATOM | 585 | CG1 | VAL | 86 | 22.851 | 20.313 | 76.509 | 1.00 | 51.39 |
| ATOM | 586 | CG2 | VAL | 86 | 21.216 | 18.446 | 76.225 | 1.00 | 50.96 |

| | | | | | | | | | |
|---|---|---|---|---|---|---|---|---|---|
| ATOM | 587 | C | VAL | 86 | 24.734 | 18.381 | 77.767 | 1.00 | 49.34 |
| ATOM | 588 | O | VAL | 86 | 24.613 | 18.316 | 78.989 | 1.00 | 48.07 |
| ATOM | 589 | N | MET | 87 | 25.834 | 18.835 | 77.178 | 1.00 | 49.52 |
| ATOM | 590 | CA | MET | 87 | 26.970 | 19.260 | 77.981 | 1.00 | 50.78 |
| ATOM | 591 | CB | MET | 87 | 27.864 | 18.054 | 78.284 | 1.00 | 52.70 |
| ATOM | 592 | CG | MET | 87 | 28.572 | 17.461 | 77.072 | 1.00 | 54.49 |
| ATOM | 593 | SD | MET | 87 | 29.005 | 15.694 | 77.269 | 1.00 | 53.62 |
| ATOM | 594 | CE | MET | 87 | 27.839 | 14.951 | 76.090 | 1.00 | 51.63 |
| ATOM | 595 | C | MET | 87 | 27.800 | 20.363 | 77.348 | 1.00 | 50.56 |
| ATOM | 596 | O | MET | 87 | 27.715 | 20.616 | 76.149 | 1.00 | 50.18 |
| ATOM | 597 | N | -LEU | 88 | 28.605 | 21.015 | 78.178 | 1.00 | 50.90 |
| ATOM | 598 | CA | LEU | 88 | 29.477 | 22.093 | 77.739 | 1.00 | 52.10 |
| ATOM | 599 | CB | LEU | 88 | 29.278 | 23.325 | 78.631 | 1.00 | 53.23 |
| ATOM | 600 | CG | LEU | 88 | 30.087 | 24.580 | 78.288 | 1.00 | 54.71 |
| ATOM | 601 | CD1 | LEU | 88 | 29.618 | 25.140 | 76.951 | 1.00 | 54.33 |
| ATOM | 602 | CD2 | LEU | 88 | 29.920 | 25.623 | 79.390 | 1.00 | 54.33 |
| ATOM | 603 | C | LEU | 88 | 30.914 | 21.600 | 77.847 | 1.00 | 52.33 |
| ATOM | 604 | O | LEU | 88 | 31.311 | 21.048 | 78.877 | 1.00 | 53.12 |
| ATOM | 605 | N | VAL | 89 | 31.693 | 21.795 | 76.789 | 1.00 | 52.10 |
| ATOM | 606 | CA | VAL | 89 | 33.078 | 21.342 | 76.788 | 1.00 | 52.46 |
| ATOM | 607 | CB | VAL | 89 | 33.241 | 20.072 | 75.882 | 1.00 | 50.52 |
| ATOM | 608 | CG1 | VAL | 89 | 32.289 | 20.147 | 74.710 | 1.00 | 52.35 |
| ATOM | 609 | CG2 | VAL | 89 | 34.674 | 19.939 | 75.388 | 1.00 | 46.86 |
| ATOM | 610 | C | VAL | 89 | 34.049 | 22.433 | 76.357 | 1.00 | 53.35 |
| ATOM | 611 | O | VAL | 89 | 33.858 | 23.081 | 75.336 | 1.00 | 54.69 |
| ATOM | 612 | N | LYS | 90 | 35.096 | 22.625 | 77.151 | 1.00 | 55.22 |
| ATOM | 613 | CA | LYS | 90 | 36.100 | 23.640 | 76.868 | 1.00 | 56.94 |
| ATOM | 614 | CB | LYS | 90 | 36.656 | 24.205 | 78.181 | 1.00 | 57.66 |
| ATOM | 615 | CG | LYS | 90 | 37.642 | 25.360 | 78.005 | 1.00 | 58.70 |
| ATOM | 616 | CD | LYS | 90 | 38.140 | 25.909 | 79.345 | 1.00 | 59.35 |
| ATOM | 617 | CE | LYS | 90 | 36.995 | 26.399 | 80.226 | 1.00 | 60.64 |
| ATOM | 618 | NZ | LYS | 90 | 36.185 | 27.462 | 79.568 | 1.00 | 61.04 |
| ATOM | 619 | C | LYS | 90 | 37.237 | 23.078 | 76.019 | 1.00 | 57.63 |
| ATOM | 620 | O | LYS | 90 | 37.921 | 22.136 | 76.417 | 1.00 | 57.69 |
| ATOM | 621 | N | VAL | 91 | 37.428 | 23.670 | 74.846 | 1.00 | 58.29 |

| | | | | | | | | | |
|---|---|---|---|---|---|---|---|---|---|
| ATOM | 622 | CA | VAL | 91 | 38.473 | 23.254 | 73.919 | 1.00 | 57.11 |
| ATOM | 623 | CB | VAL | 91 | 37.920 | 23.136 | 72.480 | 1.00 | 56.48 |
| ATOM | 624 | CG1 | VAL | 91 | 39.010 | 22.661 | 71.533 | 1.00 | 55.29 |
| ATOM | 625 | CG2 | VAL | 91 | 36.741 | 22.183 | 72.459 | 1.00 | 55.52 |
| ATOM | 626 | C | VAL | 91 | 39.598 | 24.279 | 73.926 | 1.00 | 57.81 |
| ATOM | 627 | O | VAL | 91 | 39.365 | 25.466 | 73.710 | 1.00 | 59.53 |
| ATOM | 628 | N | GLY | 92 | 40.817 | 23.819 | 74.172 | 1.00 | 58.12 |
| ATOM | 629 | CA | GLY | 92 | 41.947 | 24.723 | 74.200 | 1.00 | 59.69 |
| ATOM | 630 | C | GLY | 92 | 43.047 | 24.245 | 73.286 | 1.00 | 61.78 |
| ATOM | 631 | O | GLY | 92 | 42.821 | 23.381 | 72.448 | 1.00 | 61.06 |
| ATOM | 632 | N | GLU | 93 | 44.240 | 24.803 | 73.449 | 1.00 | 65.18 |
| ATOM | 633 | CA | GLU | 93 | 45.373 | 24.426 | 72.619 | 1.00 | 69.00 |
| ATOM | 634 | CB | GLU | 93 | 45.897 | 25.646 | 71.866 | 1.00 | 71.56 |
| ATOM | 635 | CG | GLU | 93 | 47.082 | 25.344 | 70.965 | 1.00 | 75.20 |
| ATOM | 636 | CD | GLU | 93 | 47.659 | 26.591 | 70.325 | 1.00 | 78.28 |
| ATOM | 637 | OE1 | GLU | 93 | 46.893 | 27.326 | 69.659 | 1.00 | 80.05 |
| ATOM | 638 | OE2 | GLU | 93 | 48.877 | 26.834 | 70.485 | 1.00 | 79.21 |
| ATOM | 639 | C | GLU | 93 | 46.505 | 23.822 | 73.437 | 1.00 | 71.00 |
| ATOM | 640 | O | GLU | 93 | 47.118 | 24.500 | 74.263 | 1.00 | 70.74 |
| ATOM | 641 | N | GLY | 94 | 46.784 | 22.544 | 73.195 | 1.00 | 72.97 |
| ATOM | 642 | CA | GLY | 94 | 47.849 | 21.869 | 73.916 | 1.00 | 74.44 |
| ATOM | 643 | C | GLY | 94 | 49.078 | 21.673 | 73.052 | 1.00 | 75.82 |
| ATOM | 644 | O | GLY | 94 | 49.485 | 22.577 | 72.315 | 1.00 | 76.47 |
| ATOM | 645 | N | GLU | 95 | 49.682 | 20.496 | 73.145 | 1.00 | 75.73 |
| ATOM | 646 | CA | GLU | 95 | 50.859 | 20.195 | 72.349 | 1.00 | 76.61 |
| ATOM | 647 | CB | GLU | 95 | 52.023 | 19.792 | 73.249 | 1.00 | 76.93 |
| ATOM | 648 | CG | GLU | 95 | 52.439 | 20.891 | 74.203 | 1.00 | 78.31 |
| ATOM | 649 | CD | GLU | 95 | 53.614 | 20.497 | 75.065 | 1.00 | 78.40 |
| ATOM | 650 | OE1 | GLU | 95 | 54.715 | 20.274 | 74.514 | 1.00 | 78.51 |
| ATOM | 651 | OE2 | GLU | 95 | 53.432 | 20.408 | 76.295 | 1.00 | 78.60 |
| ATOM | 652 | C | GLU | 95 | 50.516 | 19.071 | 71.392 | 1.00 | 76.91 |
| ATOM | 653 | O | GLU | 95 | 49.833 | 18.116 | 71.764 | 1.00 | 76.81 |
| ATOM | 654 | N | GLU | 96 | 50.987 | 19.203 | 70.155 | 1.00 | 77.78 |
| ATOM | 655 | CA | GLU | 96 | 50.733 | 18.220 | 69.105 | 1.00 | 78.07 |
| ATOM | 656 | CB | GLU | 96 | 51.408 | 16.881 | 69.440 | 1.00 | 81.32 |

| | | | | | | | | | |
|---|---|---|---|---|---|---|---|---|---|
| ATOM | 657 | CG | GLU | 96 | 52.943 | 16.930 | 69.454 | 1.00 | 85.11 |
| ATOM | 658 | CD | GLU | 96 | 53.541 | 17.309 | 68.101 | 1.00 | 87.05 |
| ATOM | 659 | OE1 | GLU | 96 | 53.346 | 16.551 | 67.124 | 1.00 | 88.73 |
| ATOM | 660 | OE2 | GLU | 96 | 54.207 | 18.365 | 68.014 | 1.00 | 87.56 |
| ATOM | 661 | C | GLU | 96 | 49.230 | 18.025 | 68.919 | 1.00 | 75.88 |
| ATOM | 662 | O | GLU | 96 | 48.784 | 17.039 | 68.327 | 1.00 | 75.92 |
| ATOM | 663 | N | GLY | 97 | 48.456 | 18.980 | 69.427 | 1.00 | 72.88 |
| ATOM | 664 | CA | GLY | 97 | 47.013 | 18.910 | 69.309 | 1.00 | 69.37 |
| ATOM | 665 | C | GLY | 97 | 46.296 | 19.710 | 70.380 | 1.00 | 67.02 |
| ATOM | 666 | O | GLY | 97 | 46.921 | 20.230 | 71.305 | 1.00 | 67.10 |
| ATOM | 667 | N | GLN | 98 | 44.978 | 19.811 | 70.250 | 1.00 | 64.76 |
| ATOM | 668 | CA | GLN | 98 | 44.166 | 20.543 | 71.211 | 1.00 | 62.45 |
| ATOM | 669 | CB | GLN | 98 | 42.872 | 21.045 | 70.562 | 1.00 | 62.69 |
| ATOM | 670 | CG | GLN | 98 | 43.026 | 21.908 | 69.315 | 1.00 | 64.93 |
| ATOM | 671 | CD | GLN | 98 | 43.191 | 21.095 | 68.046 | 1.00 | 65.89 |
| ATOM | 672 | OE1 | GLN | 98 | 44.299 | 20.684 | 67.696 | 1.00 | 65.96 |
| ATOM | 673 | NE2 | GLN | 98 | 42.079 | 20.847 | 67.353 | 1.00 | 65.22 |
| ATOM | 674 | C | GLN | 98 | 43.781 | 19.630 | 72.369 | 1.00 | 61.23 |
| ATOM | 675 | O | GLN | 98 | 43.880 | 18.403 | 72.269 | 1.00 | 62.18 |
| ATOM | 676 | N | TRP | 99 | 43.356 | 20.233 | 73.473 | 1.00 | 57.45 |
| ATOM | 677 | CA | TRP | 99 | 42.893 | 19.459 | 74.611 | 1.00 | 54.44 |
| ATOM | 678 | CB | TRP | 99 | 43.639 | 19.822 | 75.904 | 1.00 | 55.51 |
| ATOM | 679 | CG | TRP | 99 | 43.770 | 21.291 | 76.211 | 1.00 | 56.94 |
| ATOM | 680 | CD2 | TRP | 99 | 42.763 | 22.151 | 76.756 | 1.00 | 56.03 |
| ATOM | 681 | CE2 | TRP | 99 | 43.345 | 23.426 | 76.922 | 1.00 | 57.25 |
| ATOM | 682 | CE3 | TRP | 99 | 41.422 | 21.969 | 77.121 | 1.00 | 56.67 |
| ATOM | 683 | CD1 | TRP | 99 | 44.892 | 22.062 | 76.068 | 1.00 | 56.29 |
| ATOM | 684 | NE1 | TRP | 99 | 44.647 | 23.342 | 76.495 | 1.00 | 56.55 |
| ATOM | 685 | CZ2 | TRP | 99 | 42.635 | 24.516 | 77.440 | 1.00 | 56.53 |
| ATOM | 686 | CZ3 | TRP | 99 | 40.712 | 23.053 | 77.637 | 1.00 | 56.67 |
| ATOM | 687 | CH2 | TRP | 99 | 41.322 | 24.309 | 77.790 | 1.00 | 56.70 |
| ATOM | 688 | C | TRP | 99 | 41.408 | 19.756 | 74.737 | 1.00 | 52.04 |
| ATOM | 689 | O | TRP | 99 | 40.899 | 20.664 | 74.089 | 1.00 | 50.70 |
| ATOM | 690 | N | SER | 100 | 40.704 | 18.981 | 75.545 | 1.00 | 49.57 |
| ATOM | 691 | CA | SER | 100 | 39.277 | 19.186 | 75.715 | 1.00 | 48.29 |

| | | | | | | | | | |
|---|---|---|---|---|---|---|---|---|---|
| ATOM | 692 | CB | SER | 100 | 38.506 | 18.475 | 74.597 | 1.00 | 49.26 |
| ATOM | 693 | OG | SER | 100 | 39.055 | 17.196 | 74.315 | 1.00 | 47.27 |
| ATOM | 694 | C | SER | 100 | 38.860 | 18.655 | 77.067 | 1.00 | 47.91 |
| ATOM | 695 | O | SER | 100 | 39.569 | 17.845 | 77.662 | 1.00 | 48.73 |
| ATOM | 696 | N | VAL | 101 | 37.718 | 19.120 | 77.558 | 1.00 | 47.53 |
| ATOM | 697 | CA | VAL | 101 | 37.225 | 18.684 | 78.852 | 1.00 | 47.86 |
| ATOM | 698 | CB | VAL | 101 | 38.102 | 19.233 | 79.995 | 1.00 | 47.92 |
| ATOM | 699 | CG1 | VAL | 101 | 38.160 | 20.747 | 79.923 | 1.00 | 49.02 |
| ATOM | 700 | CG2 | VAL | 101 | 37.545 | 18.783 | 81.342 | 1.00 | 47.98 |
| ATOM | 701 | C | VAL | 101 | 35.784 | 19.102 | 79.101 | 1.00 | 48.77 |
| ATOM | 702 | O | VAL | 101 | 35.391 | 20.228 | 78.798 | 1.00 | 49.05 |
| ATOM | 703 | N | LYS | 102 | 35.004 | 18.176 | 79.649 | 1.00 | 49.04 |
| ATOM | 704 | CA | LYS | 102 | 33.607 | 18.422 | 79.969 | 1.00 | 50.31 |
| ATOM | 705 | CB | LYS | 102 | 32.875 | 17.101 | 80.220 | 1.00 | 51.15 |
| ATOM | 706 | CG | LYS | 102 | 31.385 | 17.263 | 80.452 | 1.00 | 52.57 |
| ATOM | 707 | CD | LYS | 102 | 30.835 | 16.229 | 81.425 | 1.00 | 56.56 |
| ATOM | 708 | CE | LYS | 102 | 30.955 | 14.804 | 80.908 | 1.00 | 57.06 |
| ATOM | 709 | NZ | LYS | 102 | 30.275 | 13.804 | 81.787 | 1.00 | 58.08 |
| ATOM | 710 | C | LYS | 102 | 33.587 | 19.254 | 81.243 | 1.00 | 51.12 |
| ATOM | 711 | O | LYS | 102 | 34.220 | 18.888 | 82.234 | 1.00 | 52.47 |
| ATOM | 712 | N | THR | 103 | 32.859 | 20.366 | 81.217 | 1.00 | 51.40 |
| ATOM | 713 | CA | THR | 103 | 32.774 | 21.252 | 82.373 | 1.00 | 50.47 |
| ATOM | 714 | CB | THR | 103 | 33.004 | 22.715 | 81.965 | 1.00 | 50.28 |
| ATOM | 715 | OG1 | THR | 103 | 31.992 | 23.113 | 81.032 | 1.00 | 51.29 |
| ATOM | 716 | CG2 | THR | 103 | 34.368 | 22.879 | 81.324 | 1.00 | 47.52 |
| ATOM | 717 | C | THR | 103 | 31.416 | 21.148 | 83.048 | 1.00 | 50.90 |
| ATOM | 718 | O | THR | 103 | 31.329 | 21.056 | 84.268 | 1.00 | 50.91 |
| ATOM | 719 | N | LYS | 104 | 30.358 | 21.162 | 82.247 | 1.00 | 52.41 |
| ATOM | 720 | CA | LYS | 104 | 29.000 | 21.063 | 82.770 | 1.00 | 54.04 |
| ATOM | 721 | CB | LYS | 104 | 28.310 | 22.436 | 82.714 | 1.00 | 57.21 |
| ATOM | 722 | CG | LYS | 104 | 28.823 | 23.450 | 83.739 | 1.00 | 59.16 |
| ATOM | 723 | CD | LYS | 104 | 28.138 | 24.809 | 83.576 | 1.00 | 62.54 |
| ATOM | 724 | CE | LYS | 104 | 28.398 | 25.734 | 84.766 | 1.00 | 62.99 |
| ATOM | 725 | NZ | LYS | 104 | 27.798 | 25.217 | 86.037 | 1.00 | 64.17 |
| ATOM | 726 | C | LYS | 104 | 28.215 | 20.047 | 81.948 | 1.00 | 53.79 |

| | | | | | | | | | |
|---|---|---|---|---|---|---|---|---|---|
| ATOM | 727 | O | LYS | 104 | 28.411 | 19.941 | 80.740 | 1.00 | 53.53 |
| ATOM | 728 | N | HIS | 105 | 27.330 | 19.299 | 82.600 | 1.00 | 53.65 |
| ATOM | 729 | CA | HIS | 105 | 26.539 | 18.295 | 81.903 | 1.00 | 55.05 |
| ATOM | 730 | CB | HIS | 105 | 27.316 | 16.972 | 81.837 | 1.00 | 55.94 |
| ATOM | 731 | CG | HIS | 105 | 27.668 | 16.397 | 83.176 | 1.00 | 55.84 |
| ATOM | 732 | CD2 | HIS | 105 | 28.793 | 16.501 | 83.924 | 1.00 | 55.19 |
| ATOM | 733 | ND1 | HIS | 105 | 26.803 | 15.602 | 83.897 | 1.00 | 55.83 |
| ATOM | 734 | CE1 | HIS | 105 | 27.380 | 15.241 | 85.030 | 1.00 | 56.35 |
| ATOM | 735 | NE2 | HIS | 105 | 28.589 | 15.773 | 85.071 | 1.00 | 55.64 |
| ATOM | 736 | C | HIS | 105 | 25.169 | 18.074 | 82.534 | 1.00 | 56.32 |
| ATOM | 737 | O | HIS | 105 | 24.903 | 18.535 | 83.640 | 1.00 | 56.55 |
| ATOM | 738 | N | GLN | 106 | 24.302 | 17.365 | 81.817 | 1.00 | 58.21 |
| ATOM | 739 | CA | GLN | 106 | 22.950 | 17.090 | 82.289 | 1.00 | 60.74 |
| ATOM | 740 | CB | GLN | 106 | 22.108 | 18.367 | 82.224 | 1.00 | 61.97 |
| ATOM | 741 | CG | GLN | 106 | 20.775 | 18.285 | 82.945 | 1.00 | 64.86 |
| ATOM | 742 | CD | GLN | 106 | 20.928 | 18.379 | 84.447 | 1.00 | 67.03 |
| ATOM | 743 | OE1 | GLN | 106 | 21.447 | 19.370 | 84.969 | 1.00 | 68.82 |
| ATOM | 744 | NE2 | GLN | 106 | 20.479 | 17.348 | 85.155 | 1.00 | 67.41 |
| ATOM | 745 | C | GLN | 106 | 22.322 | 16.025 | 81.396 | 1.00 | 61.62 |
| ATOM | 746 | O | GLN | 106 | 22.532 | 16.027 | 80.186 | 1.00 | 62.03 |
| ATOM | 747 | N | MET | 107 | 21.550 | 15.121 | 81.990 | 1.00 | 63.03 |
| ATOM | 748 | CA | MET | 107 | 20.900 | 14.058 | 81.232 | 1.00 | 64.74 |
| ATOM | 749 | CB | MET | 107 | 21.322 | 12.688 | 81.769 | 1.00 | 66.23 |
| ATOM | 750 | CG | MET | 107 | 22.821 | 12.456 | 81.786 | 1.00 | 68.74 |
| ATOM | 751 | SD | MET | 107 | 23.248 | 10.812 | 82.388 | 1.00 | 70.84 |
| ATOM | 752 | CE | MET | 107 | 23.427 | 9.926 | 80.853 | 1.00 | 71.13 |
| ATOM | 753 | C | MET | 107 | 19.385 | 14.175 | 81.313 | 1.00 | 65.81 |
| ATOM | 754 | O | MET | 107 | 18.837 | 14.489 | 82.369 | 1.00 | 65.52 |
| ATOM | 755 | N | TYR | 108 | 18.712 | 13.915 | 80.196 | 1.00 | 66.87 |
| ATOM | 756 | CA | TYR | 108 | 17.258 | 13.984 | 80.143 | 1.00 | 68.20 |
| ATOM | 757 | CB | TYR | 108 | 16.800 | 15.167 | 79.286 | 1.00 | 67.20 |
| ATOM | 758 | CG | TYR | 108 | 17.436 | 16.484 | 79.660 | 1.00 | 66.35 |
| ATOM | 759 | CD1 | TYR | 108 | 18.781 | 16.731 | 79.386 | 1.00 | 65.95 |
| ATOM | 760 | CE1 | TYR | 108 | 19.380 | 17.929 | 79.746 | 1.00 | 65.76 |
| ATOM | 761 | CD2 | TYR | 108 | 16.702 | 17.477 | 80.307 | 1.00 | 66.24 |

| | | | | | | | | | |
|---|---|---|---|---|---|---|---|---|---|
| ATOM | 762 | CE2 | TYR | 108 | 17.292 | 18.683 | 80.674 | 1.00 | 65.93 |
| ATOM | 763 | CZ | TYR | 108 | 18.633 | 18.902 | 80.391 | 1.00 | 66.14 |
| ATOM | 764 | OH | TYR | 108 | 19.235 | 20.083 | 80.763 | 1.00 | 64.27 |
| ATOM | 765 | C | TYR | 108 | 16.706 | 12.700 | 79.549 | 1.00 | 70.20 |
| ATOM | 766 | O | TYR | 108 | 16.995 | 12.363 | 78.404 | 1.00 | 70.55 |
| ATOM | 767 | N | SER | 109 | 15.912 | 11.982 | 80.331 | 1.00 | 73.54 |
| ATOM | 768 | CA | SER | 109 | 15.322 | 10.739 | 79.863 | 1.00 | 76.84 |
| ATOM | 769 | CB | SER | 109 | 14.524 | 10.082 | 80.992 | 1.00 | 77.63 |
| ATOM | 770 | OG | SER | 109 | 15.353 | 9.837 | 82.120 | 1.00 | 78.13 |
| ATOM | 771 | C | SER | 109 | 14.419 | 11.020 | 78.664 | 1.00 | 78.98 |
| ATOM | 772 | O | SER | 109 | 13.936 | 12.138 | 78.486 | 1.00 | 78.51 |
| ATOM | 773 | N | ILE | 110 | 14.198 | 10.002 | 77.841 | 1.00 | 82.34 |
| ATOM | 774 | CA | ILE | 110 | 13.369 | 10.143 | 76.651 | 1.00 | 86.07 |
| ATOM | 775 | CB | ILE | 110 | 13.892 | 9.249 | 75.511 | 1.00 | 86.28 |
| ATOM | 776 | CG2 | ILE | 110 | 13.092 | 9.505 | 74.242 | 1.00 | 86.56 |
| ATOM | 777 | CG1 | ILE | 110 | 15.379 | 9.529 | 75.275 | 1.00 | 86.19 |
| ATOM | 778 | CD1 | ILE | 110 | 16.025 | 8.612 | 74.258 | 1.00 | 86.76 |
| ATOM | 779 | C | ILE | 110 | 11.916 | 9.772 | 76.927 | 1.00 | 88.58 |
| ATOM | 780 | O | ILE | 110 | 11.596 | 8.606 | 77.152 | 1.00 | 88.69 |
| ATOM | 781 | N | PRO | 111 | 11.016 | 10.767 | 76.910 | 1.00 | 91.13 |
| ATOM | 782 | CD | PRO | 111 | 11.319 | 12.205 | 76.811 | 1.00 | 91.83 |
| ATOM | 783 | CA | PRO | 111 | 9.585 | 10.562 | 77.157 | 1.00 | 93.32 |
| ATOM | 784 | CB | PRO | 111 | 9.015 | 11.975 | 77.062 | 1.00 | 93.16 |
| ATOM | 785 | CG | PRO | 111 | 10.147 | 12.819 | 77.536 | 1.00 | 92.31 |
| ATOM | 786 | C | PRO | 111 | 8.928 | 9.613 | 76.159 | 1.00 | 95.40 |
| ATOM | 787 | O | PRO | 111 | 9.466 | 9.355 | 75.082 | 1.00 | 95.80 |
| ATOM | 788 | N | GLU | 112 | 7.758 | 9.101 | 76.529 | 1.00 | 97.55 |
| ATOM | 789 | CA | GLU | 112 | 7.006 | 8.185 | 75.679 | 1.00 | 99.50 |
| ATOM | 790 | CB | GLU | 112 | 5.816 | 7.611 | 76.458 | 1.00 | 100.31 |
| ATOM | 791 | CG | GLU | 112 | 4.745 | 6.971 | 75.589 | 1.00 | 101.76 |
| ATOM | 792 | CD | GLU | 112 | 5.316 | 5.989 | 74.587 | 1.00 | 102.84 |
| ATOM | 793 | OE1 | GLU | 112 | 5.967 | 5.012 | 75.014 | 1.00 | 103.66 |
| ATOM | 794 | OE2 | GLU | 112 | 5.113 | 6.196 | 73.372 | 1.00 | 103.00 |
| ATOM | 795 | C | GLU | 112 | 6.508 | 8.884 | 74.418 | 1.00 | 100.37 |
| ATOM | 796 | O | GLU | 112 | 6.914 | 8.545 | 73.304 | 1.00 | 100.17 |

| | | | | | | | | |
|---|---|---|---|---|---|---|---|---|
| ATOM | 797 | N | ASP | 113 | 5.625 | 9.859 | 74.606 | 1.00101.44 |
| ATOM | 798 | CA | ASP | 113 | 5.056 | 10.620 | 73.499 | 1.00102.05 |
| ATOM | 799 | CB | ASP | 113 | 4.087 | 11.680 | 74.038 | 1.00102.23 |
| ATOM | 800 | CG | ASP | 113 | 4.682 | 12.494 | 75.177 | 1.00102.33 |
| ATOM | 801 | OD1 | ASP | 113 | 4.961 | 11.913 | 76.249 | 1.00102.01 |
| ATOM | 802 | OD2 | ASP | 113 | 4.870 | 13.716 | 74.999 | 1.00101.99 |
| ATOM | 803 | C | ASP | 113 | 6.131 | 11.282 | 72.638 | 1.00102.09 |
| ATOM | 804 | O | ASP | 113 | 5.843 | 11.789 | 71.553 | 1.00101.96 |
| ATOM | 805 | N | ALA | 114 | 7.368 | 11.273 | 73.126 | 1.00102.12 |
| ATOM | 806 | CA | ALA | 114 | 8.484 | 11.869 | 72.401 | 1.00102.09 |
| ATOM | 807 | CB | ALA | 114 | 9.590 | 12.256 | 73.377 | 1.00101.76 |
| ATOM | 808 | C | ALA | 114 | 9.022 | 10.895 | 71.358 | 1.00102.06 |
| ATOM | 809 | O | ALA | 114 | 9.763 | 11.282 | 70.455 | 1.00101.89 |
| ATOM | 810 | N | MET | 115 | 8.640 | 9.630 | 71.491 | 1.00102.04 |
| ATOM | 811 | CA | MET | 115 | 9.081 | 8.592 | 70.569 | 1.00102.05 |
| ATOM | 812 | CB | MET | 115 | 9.466 | 7.331 | 71.346 | 1.00102.77 |
| ATOM | 813 | CG | MET | 115 | 10.637 | 7.509 | 72.307 | 1.00103.47 |
| ATOM | 814 | SD | MET | 115 | 12.256 | 7.549 | 71.502 | 1.00104.26 |
| ATOM | 815 | CE | MET | 115 | 12.740 | 5.824 | 71.638 | 1.00103.48 |
| ATOM | 816 | C | MET | 115 | 8.004 | 8.253 | 69.538 | 1.00101.77 |
| ATOM | 817 | O | MET | 115 | 8.268 | 8.275 | 68.337 | 1.00102.14 |
| ATOM | 818 | N | THR | 116 | 6.796 | 7.942 | 70.006 | 1.00101.14 |
| ATOM | 819 | CA | THR | 116 | 5.690 | 7.590 | 69.110 | 1.00100.36 |
| ATOM | 820 | CB | THR | 116 | 4.517 | 6.927 | 69.880 | 1.00100.42 |
| ATOM | 821 | OG1 | THR | 116 | 5.004 | 5.805 | 70.625 | 1.00100.29 |
| ATOM | 822 | CG2 | THR | 116 | 3.441 | 6.441 | 68.911 | 1.00100.05 |
| ATOM | 823 | C | THR | 116 | 5.150 | 8.816 | 68.379 | 1.00 99.62 |
| ATOM | 824 | O | THR | 116 | 4.423 | 8.694 | 67.391 | 1.00 99.72 |
| ATOM | 825 | N | GLY | 117 | 5.510 | 9.996 | 68.870 | 1.00 98.62 |
| ATOM | 826 | CA | GLY | 117 | 5.048 | 11.224 | 68.252 | 1.00 97.42 |
| ATOM | 827 | C | GLY | 117 | 5.619 | 11.447 | 66.866 | 1.00 96.48 |
| ATOM | 828 | O | GLY | 117 | 5.746 | 10.511 | 66.074 | 1.00 96.38 |
| ATOM | 829 | N | THR | 118 | 5.962 | 12.696 | 66.570 | 1.00 95.25 |
| ATOM | 830 | CA | THR | 118 | 6.521 | 13.050 | 65.273 | 1.00 93.78 |
| ATOM | 831 | CB | THR | 118 | 5.679 | 14.133 | 64.578 | 1.00 93.57 |

| | | | | | | | | | |
|---|---|---|---|---|---|---|---|---|---|
| ATOM | 832 | OG1 | THR | 118 | 5.735 | 15.343 | 65.342 | 1.00 | 93.50 |
| ATOM | 833 | CG2 | THR | 118 | 4.234 | 13.685 | 64.457 | 1.00 | 93.65 |
| ATOM | 834 | C | THR | 118 | 7.936 | 13.583 | 65.440 | 1.00 | 92.67 |
| ATOM | 835 | O | THR | 118 | 8.335 | 13.976 | 66.537 | 1.00 | 92.39 |
| ATOM | 836 | N | ALA | 119 | 8.687 | 13.593 | 64.343 | 1.00 | 91.30 |
| ATOM | 837 | CA | ALA | 119 | 10.058 | 14.084 | 64.356 | 1.00 | 90.00 |
| ATOM | 838 | CB | ALA | 119 | 10.643 | 14.031 | 62.956 | 1.00 | 89.81 |
| ATOM | 839 | C | ALA | 119 | 10.066 | 15.513 | 64.867 | 1.00 | 89.21 |
| ATOM | 840 | O | ALA | 119 | 11.045 | 15.972 | 65.455 | 1.00 | 88.98 |
| ATOM | 841 | N | GLU | 120 | 8.959 | 16.210 | 64.636 | 1.00 | 88.61 |
| ATOM | 842 | CA | GLU | 120 | 8.819 | 17.593 | 65.063 | 1.00 | 87.61 |
| ATOM | 843 | CB | GLU | 120 | 7.505 | 18.177 | 64.536 | 1.00 | 87.74 |
| ATOM | 844 | CG | GLU | 120 | 7.138 | 17.763 | 63.112 | 1.00 | 86.31 |
| ATOM | 845 | CD | GLU | 120 | 8.269 | 17.956 | 62.120 | 1.00 | 85.84 |
| ATOM | 846 | OE1 | GLU | 120 | 8.884 | 19.042 | 62.113 | 1.00 | 84.76 |
| ATOM | 847 | OE2 | GLU | 120 | 8.535 | 17.020 | 61.336 | 1.00 | 85.71 |
| ATOM | 848 | C | GLU | 120 | 8.837 | 17.658 | 66.588 | 1.00 | 86.71 |
| ATOM | 849 | O | GLU | 120 | 9.610 | 18.412 | 67.179 | 1.00 | 86.71 |
| ATOM | 850 | N | MET | 121 | 7.980 | 16.859 | 67.216 | 1.00 | 85.74 |
| ATOM | 851 | CA | MET | 121 | 7.895 | 16.817 | 68.671 | 1.00 | 84.85 |
| ATOM | 852 | CB | MET | 121 | 6.798 | 15.842 | 69.111 | 1.00 | 84.04 |
| ATOM | 853 | CG | MET | 121 | 5.390 | 16.273 | 68.740 | 1.00 | 81.88 |
| ATOM | 854 | SD | MET | 121 | 4.152 | 15.078 | 69.268 | 1.00 | 80.83 |
| ATOM | 855 | CE | MET | 121 | 3.772 | 14.283 | 67.730 | 1.00 | 78.55 |
| ATOM | 856 | C | MET | 121 | 9.226 | 16.397 | 69.286 | 1.00 | 84.73 |
| ATOM | 857 | O | MET | 121 | 9.687 | 17.003 | 70.255 | 1.00 | 84.87 |
| ATOM | 858 | N | LEU | 122 | 9.839 | 15.360 | 68.717 | 1.00 | 84.21 |
| ATOM | 859 | CA | LEU | 122 | 11.115 | 14.851 | 69.211 | 1.00 | 83.20 |
| ATOM | 860 | CB | LEU | 122 | 11.711 | 13.847 | 68.221 | 1.00 | 83.29 |
| ATOM | 861 | CG | LEU | 122 | 12.966 | 13.109 | 68.697 | 1.00 | 83.07 |
| ATOM | 862 | CD1 | LEU | 122 | 12.612 | 12.232 | 69.885 | 1.00 | 82.78 |
| ATOM | 863 | CD2 | LEU | 122 | 13.533 | 12.261 | 67.572 | 1.00 | 82.52 |
| ATOM | 864 | C | LEU | 122 | 12.110 | 15.980 | 69.448 | 1.00 | 82.61 |
| ATOM | 865 | O | LEU | 122 | 12.546 | 16.204 | 70.575 | 1.00 | 82.47 |
| ATOM | 866 | N | PHE | 123 | 12.467 | 16.694 | 68.385 | 1.00 | 82.28 |

| | | | | | | | | | |
|---|---|---|---|---|---|---|---|---|---|
| ATOM | 867 | CA | PHE | 123 | 13.414 | 17.794 | 68.512 | 1.00 | 82.09 |
| ATOM | 868 | CB | PHE | 123 | 13.898 | 18.251 | 67.136 | 1.00 | 82.08 |
| ATOM | 869 | CG | PHE | 123 | 14.948 | 17.357 | 66.547 | 1.00 | 81.61 |
| ATOM | 870 | CD1 | PHE | 123 | 14.616 | 16.098 | 66.060 | 1.00 | 81.34 |
| ATOM | 871 | CD2 | PHE | 123 | 16.281 | 17.756 | 66.523 | 1.00 | 81.33 |
| ATOM | 872 | CE1 | PHE | 123 | 15.594 | 15.246 | 65.559 | 1.00 | 80.67 |
| ATOM | 873 | CE2 | PHE | 123 | 17.268 | 16.912 | 66.026 | 1.00 | 81.58 |
| ATOM | 874 | CZ | PHE | 123 | 16.923 | 15.653 | 65.543 | 1.00 | 81.33 |
| ATOM | 875 | C | PHE | 123 | 12.834 | 18.964 | 69.288 | 1.00 | 81.98 |
| ATOM | 876 | O | PHE | 123 | 13.570 | 19.838 | 69.747 | 1.00 | 81.74 |
| ATOM | 877 | N | ASP | 124 | 11.512 | 18.980 | 69.429 | 1.00 | 82.09 |
| ATOM | 878 | CA | ASP | 124 | 10.852 | 20.028 | 70.195 | 1.00 | 82.29 |
| ATOM | 879 | CB | ASP | 124 | 9.329 | 19.909 | 70.073 | 1.00 | 81.96 |
| ATOM | 880 | CG | ASP | 124 | 8.731 | 20.961 | 69.157 | 1.00 | 81.56 |
| ATOM | 881 | OD1 | ASP | 124 | 7.510 | 20.897 | 68.901 | 1.00 | 81.25 |
| ATOM | 882 | OD2 | ASP | 124 | 9.477 | 21.855 | 68.701 | 1.00 | 80.94 |
| ATOM | 883 | C | ASP | 124 | 11.279 | 19.808 | 71.641 | 1.00 | 82.22 |
| ATOM | 884 | O | ASP | 124 | 11.819 | 20.707 | 72.287 | 1.00 | 81.61 |
| ATOM | 885 | N | TYR | 125 | 11.047 | 18.595 | 72.133 | 1.00 | 82.59 |
| ATOM | 886 | CA | TYR | 125 | 11.420 | 18.233 | 73.494 | 1.00 | 83.66 |
| ATOM | 887 | CB | TYR | 125 | 11.048 | 16.771 | 73.767 | 1.00 | 85.84 |
| ATOM | 888 | CG | TYR | 125 | 11.533 | 16.240 | 75.100 | 1.00 | 88.74 |
| ATOM | 889 | CD1 | TYR | 125 | 12.763 | 15.590 | 75.209 | 1.00 | 89.83 |
| ATOM | 890 | CE1 | TYR | 125 | 13.222 | 15.110 | 76.437 | 1.00 | 91.28 |
| ATOM | 891 | CD2 | TYR | 125 | 10.770 | 16.399 | 76.257 | 1.00 | 90.41 |
| ATOM | 892 | CE2 | TYR | 125 | 11.221 | 15.926 | 77.493 | 1.00 | 91.86 |
| ATOM | 893 | CZ | TYR | 125 | 12.448 | 15.281 | 77.574 | 1.00 | 92.09 |
| ATOM | 894 | OH | TYR | 125 | 12.896 | 14.807 | 78.789 | 1.00 | 93.08 |
| ATOM | 895 | C | TYR | 125 | 12.917 | 18.451 | 73.704 | 1.00 | 82.86 |
| ATOM | 896 | O | TYR | 125 | 13.352 | 18.829 | 74.792 | 1.00 | 82.74 |
| ATOM | 897 | N | ILE | 126 | 13.701 | 18.215 | 72.655 | 1.00 | 81.74 |
| ATOM | 898 | CA | ILE | 126 | 15.146 | 18.398 | 72.727 | 1.00 | 80.58 |
| ATOM | 899 | CB | ILE | 126 | 15.824 | 18.005 | 71.397 | 1.00 | 79.32 |
| ATOM | 900 | CG2 | ILE | 126 | 17.277 | 18.443 | 71.398 | 1.00 | 78.57 |
| ATOM | 901 | CG1 | ILE | 126 | 15.719 | 16.494 | 71.194 | 1.00 | 78.47 |

| | | | | | | | | | |
|---|---|---|---|---|---|---|---|---|---|
| ATOM | 902 | CD1 | ILE | 126 | 16.408 | 15.993 | 69.946 | 1.00 | 78.42 |
| ATOM | 903 | C | ILE | 126 | 15.479 | 19.852 | 73.047 | 1.00 | 80.87 |
| ATOM | 904 | O | ILE | 126 | 16.334 | 20.133 | 73.887 | 1.00 | 79.71 |
| ATOM | 905 | N | SER | 127 | 14.799 | 20.772 | 72.370 | 1.00 | 81.80 |
| ATOM | 906 | CA | SER | 127 | 15.018 | 22.196 | 72.594 | 1.00 | 82.44 |
| ATOM | 907 | CB | SER | 127 | 14.160 | 23.021 | 71.636 | 1.00 | 82.62 |
| ATOM | 908 | OG | SER | 127 | 14.559 | 22.807 | 70.294 | 1.00 | 83.20 |
| ATOM | 909 | C | SER | 127 | 14.668 | 22.543 | 74.034 | 1.00 | 82.44 |
| ATOM | 910 | O | SER | 127 | 15.318 | 23.382 | 74.660 | 1.00 | 81.86 |
| ATOM | 911 | N | GLU | 128 | 13.636 | 21.884 | 74.553 | 1.00 | 83.04 |
| ATOM | 912 | CA | GLU | 128 | 13.202 | 22.106 | 75.927 | 1.00 | 83.79 |
| ATOM | 913 | CB | GLU | 128 | 11.944 | 21.289 | 76.232 | 1.00 | 84.79 |
| ATOM | 914 | CG | GLU | 128 | 11.408 | 21.490 | 77.645 | 1.00 | 86.70 |
| ATOM | 915 | CD | GLU | 128 | 10.425 | 20.409 | 78.061 | 1.00 | 88.14 |
| ATOM | 916 | OE1 | GLU | 128 | 9.408 | 20.222 | 77.357 | 1.00 | 88.36 |
| ATOM | 917 | OE2 | GLU | 128 | 10.672 | 19.747 | 79.094 | 1.00 | 88.06 |
| ATOM | 918 | C | GLU | 128 | 14.318 | 21.686 | 76.877 | 1.00 | 83.42 |
| ATOM | 919 | O | GLU | 128 | 14.483 | 22.261 | 77.952 | 1.00 | 84.16 |
| ATOM | 920 | N | CYS | 129 | 15.081 | 20.675 | 76.475 | 1.00 | 82.77 |
| ATOM | 921 | CA | CYS | 129 | 16.177 | 20.179 | 77.295 | 1.00 | 81.21 |
| ATOM | 922 | CB | CYS | 129 | 16.554 | 18.760 | 76.873 | 1.00 | 81.07 |
| ATOM | 923 | SG | CYS | 129 | 15.206 | 17.569 | 77.006 | 1.00 | 80.63 |
| ATOM | 924 | C | CYS | 129 | 17.391 | 21.089 | 77.178 | 1.00 | 80.64 |
| ATOM | 925 | O | CYS | 129 | 18.092 | 21.330 | 78.160 | 1.00 | 79.84 |
| ATOM | 926 | N | ILE | 130 | 17.644 | 21.591 | 75.975 | 1.00 | 80.16 |
| ATOM | 927 | CA | ILE | 130 | 18.782 | 22.475 | 75.775 | 1.00 | 80.33 |
| ATOM | 928 | CB | ILE | 130 | 18.944 | 22.860 | 74.298 | 1.00 | 79.59 |
| ATOM | 929 | CG2 | ILE | 130 | 20.253 | 23.614 | 74.102 | 1.00 | 79.29 |
| ATOM | 930 | CG1 | ILE | 130 | 18.933 | 21.599 | 73.436 | 1.00 | 79.00 |
| ATOM | 931 | CD1 | ILE | 130 | 19.069 | 21.860 | 71.958 | 1.00 | 79.73 |
| ATOM | 932 | C | ILE | 130 | 18.559 | 23.735 | 76.595 | 1.00 | 80.49 |
| ATOM | 933 | O | ILE | 130 | 19.475 | 24.241 | 77.239 | 1.00 | 80.22 |
| ATOM | 934 | N | SER | 131 | 17.326 | 24.229 | 76.574 | 1.00 | 81.09 |
| ATOM | 935 | CA | SER | 131 | 16.970 | 25.428 | 77.320 | 1.00 | 82.28 |
| ATOM | 936 | CB | SER | 131 | 15.525 | 25.826 | 77.006 | 1.00 | 83.15 |

| | | | | | | | | | |
|---|---|---|---|---|---|---|---|---|---|
| ATOM | 937 | OG | SER | 131 | 14.641 | 24.736 | 77.195 | 1.00 | 82.88 |
| ATOM | 938 | C | SER | 131 | 17.136 | 25.195 | 78.820 | 1.00 | 82.33 |
| ATOM | 939 | O | SER | 131 | 17.843 | 25.940 | 79.501 | 1.00 | 82.07 |
| ATOM | 940 | N | ASP | 132 | 16.478 | 24.155 | 79.322 | 1.00 | 82.42 |
| ATOM | 941 | CA | ASP | 132 | 16.540 | 23.792 | 80.735 | 1.00 | 82.24 |
| ATOM | 942 | CB | ASP | 132 | 15.893 | 22.411 | 80.934 | 1.00 | 83.24 |
| ATOM | 943 | CG | ASP | 132 | 15.836 | 21.981 | 82.393 | 1.00 | 83.66 |
| ATOM | 944 | OD1 | ASP | 132 | 15.165 | 20.963 | 82.678 | 1.00 | 83.28 |
| ATOM | 945 | OD2 | ASP | 132 | 16.458 | 22.645 | 83.250 | 1.00 | 83.85 |
| ATOM | 946 | C | ASP | 132 | 17.996 | 23.778 | 81.200 | 1.00 | 81.62 |
| ATOM | 947 | O | ASP | 132 | 18.324 | 24.293 | 82.270 | 1.00 | 82.12 |
| ATOM | 948 | N | PHE | 133 | 18.866 | 23.193 | 80.383 | 1.00 | 80.65 |
| ATOM | 949 | CA | PHE | 133 | 20.286 | 23.118 | 80.698 | 1.00 | 79.47 |
| ATOM | 950 | CB | PHE | 133 | 21.033 | 22.331 | 79.616 | 1.00 | 77.80 |
| ATOM | 951 | CG | PHE | 133 | 22.528 | 22.391 | 79.750 | 1.00 | 75.86 |
| ATOM | 952 | CD1 | PHE | 133 | 23.178 | 21.695 | 80.761 | 1.00 | 75.50 |
| ATOM | 953 | CD2 | PHE | 133 | 23.284 | 23.179 | 78.889 | 1.00 | 75.39 |
| ATOM | 954 | CE1 | PHE | 133 | 24.562 | 21.785 | 80.914 | 1.00 | 74.78 |
| ATOM | 955 | CE2 | PHE | 133 | 24.667 | 23.275 | 79.035 | 1.00 | 74.59 |
| ATOM | 956 | CZ | PHE | 133 | 25.305 | 22.578 | 80.049 | 1.00 | 74.18 |
| ATOM | 957 | C | PHE | 133 | 20.876 | 24.519 | 80.786 | 1.00 | 79.33 |
| ATOM | 958 | O | PHE | 133 | 21.690 | 24.810 | 81.659 | 1.00 | 79.06 |
| ATOM | 959 | N | LEU | 134 | 20.459 | 25.382 | 79.869 | 1.00 | 79.23 |
| ATOM | 960 | CA | LEU | 134 | 20.951 | 26.748 | 79.828 | 1.00 | 79.59 |
| ATOM | 961 | CB | LEU | 134 | 20.482 | 27.412 | 78.534 | 1.00 | 79.43 |
| ATOM | 962 | CG | LEU | 134 | 21.043 | 26.703 | 77.297 | 1.00 | 78.61 |
| ATOM | 963 | CD1 | LEU | 134 | 20.401 | 27.247 | 76.032 | 1.00 | 78.47 |
| ATOM | 964 | CD2 | LEU | 134 | 22.554 | 26.878 | 77.264 | 1.00 | 77.75 |
| ATOM | 965 | C | LEU | 134 | 20.524 | 27.565 | 81.043 | 1.00 | 79.41 |
| ATOM | 966 | O | LEU | 134 | 21.324 | 28.310 | 81.609 | 1.00 | 78.74 |
| ATOM | 967 | N | ASP | 135 | 19.268 | 27.423 | 81.448 | 1.00 | 80.16 |
| ATOM | 968 | CA | ASP | 135 | 18.780 | 28.152 | 82.609 | 1.00 | 80.92 |
| ATOM | 969 | CB | ASP | 135 | 17.271 | 27.966 | 82.777 | 1.00 | 80.81 |
| ATOM | 970 | CG | ASP | 135 | 16.474 | 28.778 | 81.783 | 1.00 | 81.08 |
| ATOM | 971 | OD1 | ASP | 135 | 16.801 | 29.970 | 81.599 | 1.00 | 82.67 |

| | | | | | | | | | |
|---|---|---|---|---|---|---|---|---|---|
| ATOM | 972 | OD2 | ASP | 135 | 15.517 | 28.234 | 81.195 | 1.00 | 81.12 |
| ATOM | 973 | C | ASP | 135 | 19.486 | 27.686 | 83.872 | 1.00 | 81.80 |
| ATOM | 974 | O | ASP | 135 | 20.090 | 28.490 | 84.578 | 1.00 | 82.12 |
| ATOM | 975 | N | LYS | 136 | 19.418 | 26.384 | 84.143 | 1.00 | 82.43 |
| ATOM | 976 | CA | LYS | 136 | 20.041 | 25.811 | 85.333 | 1.00 | 83.25 |
| ATOM | 977 | CB | LYS | 136 | 19.750 | 24.307 | 85.418 | 1.00 | 82.64 |
| ATOM | 978 | CG | LYS | 136 | 18.288 | 23.970 | 85.677 | 1.00 | 82.57 |
| ATOM | 979 | CD | LYS | 136 | 18.095 | 22.487 | 85.952 | 1.00 | 82.49 |
| ATOM | 980 | CE | LYS | 136 | 16.630 | 22.154 | 86.182 | 1.00 | 82.31 |
| ATOM | 981 | NZ | LYS | 136 | 16.053 | 22.914 | 87.323 | 1.00 | 82.43 |
| ATOM | 982 | C | LYS | 136 | 21.548 | 26.044 | 85.429 | 1.00 | 84.12 |
| ATOM | 983 | O | LYS | 136 | 22.185 | 25.610 | 86.390 | 1.00 | 84.51 |
| ATOM | 984 | N | HIS | 137 | 22.119 | 26.727 | 84.442 | 1.00 | 85.08 |
| ATOM | 985 | CA | HIS | 137 | 23.551 | 27.010 | 84.450 | 1.00 | 86.27 |
| ATOM | 986 | CB | HIS | 137 | 24.280 | 26.115 | 83.438 | 1.00 | 86.74 |
| ATOM | 987 | CG | HIS | 137 | 24.169 | 24.649 | 83.730 | 1.00 | 87.04 |
| ATOM | 988 | CD2 | HIS | 137 | 25.112 | 23.729 | 84.047 | 1.00 | 86.44 |
| ATOM | 989 | ND1 | HIS | 137 | 22.968 | 23.971 | 83.708 | 1.00 | 87.51 |
| ATOM | 990 | CE1 | HIS | 137 | 23.176 | 22.699 | 83.999 | 1.00 | 86.59 |
| ATOM | 991 | NE2 | HIS | 137 | 24.468 | 22.526 | 84.209 | 1.00 | 86.35 |
| ATOM | 992 | C | HIS | 137 | 23.820 | 28.476 | 84.123 | 1.00 | 87.11 |
| ATOM | 993 | O | HIS | 137 | 24.943 | 28.842 | 83.776 | 1.00 | 86.73 |
| ATOM | 994 | N | GLN | 138 | 22.784 | 29.307 | 84.249 | 1.00 | 88.41 |
| ATOM | 995 | CA | GLN | 138 | 22.883 | 30.736 | 83.955 | 1.00 | 89.43 |
| ATOM | 996 | CB | GLN | 138 | 23.469 | 31.512 | 85.140 | 1.00 | 90.47 |
| ATOM | 997 | CG | GLN | 138 | 22.654 | 31.451 | 86.419 | 1.00 | 92.10 |
| ATOM | 998 | CD | GLN | 138 | 22.738 | 30.099 | 87.095 | 1.00 | 93.09 |
| ATOM | 999 | OE1 | GLN | 138 | 23.829 | 29.598 | 87.372 | 1.00 | 93.35 |
| ATOM | 1000 | NE2 | GLN | 138 | 21.584 | 29.501 | 87.371 | 1.00 | 93.71 |
| ATOM | 1001 | C | GLN | 138 | 23.779 | 30.931 | 82.747 | 1.00 | 89.90 |
| ATOM | 1002 | O | GLN | 138 | 24.922 | 31.376 | 82.875 | 1.00 | 89.53 |
| ATOM | 1003 | N | MET | 139 | 23.262 | 30.591 | 81.573 | 1.00 | 89.97 |
| ATOM | 1004 | CA | MET | 139 | 24.046 | 30.725 | 80.359 | 1.00 | 90.27 |
| ATOM | 1005 | CB | MET | 139 | 24.995 | 29.529 | 80.235 | 1.00 | 90.82 |
| ATOM | 1006 | CG | MET | 139 | 26.314 | 29.838 | 79.542 | 1.00 | 91.26 |

| | | | | | | | | | |
|---|---|---|---|---|---|---|---|---|---|
| ATOM | 1007 | SD | MET | 139 | 27.526 | 28.508 | 79.736 | 1.00 | 90.73 |
| ATOM | 1008 | CE | MET | 139 | 28.303 | 28.974 | 81.303 | 1.00 | 91.08 |
| ATOM | 1009 | C | MET | 139 | 23.137 | 30.820 | 79.140 | 1.00 | 90.17 |
| ATOM | 1010 | O | MET | 139 | 23.610 | 30.894 | 78.006 | 1.00 | 90.11 |
| ATOM | 1011 | N | LYS | 140 | 21.829 | 30.829 | 79.380 | 1.00 | 89.92 |
| ATOM | 1012 | CA | LYS | 140 | 20.851 | 30.921 | 78.300 | 1.00 | 89.78 |
| ATOM | 1013 | CB | LYS | 140 | 19.434 | 30.922 | 78.874 | 1.00 | 89.37 |
| ATOM | 1014 | CG | LYS | 140 | 18.357 | 31.239 | 77.852 | 1.00 | 89.17 |
| ATOM | 1015 | CD | LYS | 140 | 16.972 | 31.055 | 78.438 | 1.00 | 89.06 |
| ATOM | 1016 | CE | LYS | 140 | 16.688 | 29.588 | 78.675 | 1.00 | 88.66 |
| ATOM | 1017 | NZ | LYS | 140 | 16.797 | 28.822 | 77.406 | 1.00 | 88.73 |
| ATOM | 1018 | C | LYS | 140 | 21.067 | 32.179 | 77.466 | 1.00 | 89.78 |
| ATOM | 1019 | O | LYS | 140 | 20.593 | 32.278 | 76.334 | 1.00 | 89.28 |
| ATOM | 1020 | N | HIS | 141 | 21.794 | 33.133 | 78.037 | 1.00 | 90.38 |
| ATOM | 1021 | CA | HIS | 141 | 22.082 | 34.401 | 77.376 | 1.00 | 90.81 |
| ATOM | 1022 | CB | HIS | 141 | 22.222 | 35.506 | 78.427 | 1.00 | 90.98 |
| ATOM | 1023 | CG | HIS | 141 | 23.294 | 35.243 | 79.443 | 1.00 | 91.18 |
| ATOM | 1024 | CD2 | HIS | 141 | 24.520 | 35.794 | 79.610 | 1.00 | 91.04 |
| ATOM | 1025 | ND1 | HIS | 141 | 23.163 | 34.294 | 80.434 | 1.00 | 91.11 |
| ATOM | 1026 | CE1 | HIS | 141 | 24.262 | 34.273 | 81.168 | 1.00 | 91.45 |
| ATOM | 1027 | NE2 | HIS | 141 | 25.102 | 35.174 | 80.688 | 1.00 | 90.96 |
| ATOM | 1028 | C | HIS | 141 | 23.349 | 34.367 | 76.516 | 1.00 | 90.72 |
| ATOM | 1029 | O | HIS | 141 | 24.048 | 35.374 | 76.399 | 1.00 | 91.00 |
| ATOM | 1030 | N | LYS | 142 | 23.648 | 33.220 | 75.912 | 1.00 | 90.17 |
| ATOM | 1031 | CA | LYS | 142 | 24.845 | 33.109 | 75.082 | 1.00 | 89.12 |
| ATOM | 1032 | CB | LYS | 142 | 26.000 | 32.529 | 75.908 | 1.00 | 89.54 |
| ATOM | 1033 | CG | LYS | 142 | 26.424 | 33.406 | 77.079 | 1.00 | 90.51 |
| ATOM | 1034 | CD | LYS | 142 | 27.490 | 32.730 | 77.926 | 1.00 | 91.91 |
| ATOM | 1035 | CE | LYS | 142 | 27.867 | 33.579 | 79.131 | 1.00 | 92.42 |
| ATOM | 1036 | NZ | LYS | 142 | 28.820 | 32.863 | 80.026 | 1.00 | 92.34 |
| ATOM | 1037 | C | LYS | 142 | 24.643 | 32.276 | 73.815 | 1.00 | 87.58 |
| ATOM | 1038 | O | LYS | 142 | 23.763 | 31.418 | 73.749 | 1.00 | 87.74 |
| ATOM | 1039 | N | LYS | 143 | 25.465 | 32.554 | 72.808 | 1.00 | 85.65 |
| ATOM | 1040 | CA | LYS | 143 | 25.414 | 31.849 | 71.532 | 1.00 | 83.45 |
| ATOM | 1041 | CB | LYS | 143 | 25.052 | 32.819 | 70.402 | 1.00 | 83.10 |

| | | | | | | | | | | |
|---|---|---|---|---|---|---|---|---|---|---|
| ATOM | 1042 | CG | LYS | 143 | 25.199 | 32.262 | 68.988 | 1.00 | 82.55 |
| ATOM | 1043 | CD | LYS | 143 | 24.890 | 33.339 | 67.951 | 1.00 | 82.36 |
| ATOM | 1044 | CE | LYS | 143 | 25.289 | 32.922 | 66.540 | 1.00 | 82.46 |
| ATOM | 1045 | NZ | LYS | 143 | 24.519 | 31.749 | 66.045 | 1.00 | 82.29 |
| ATOM | 1046 | C | LYS | 143 | 26.790 | 31.252 | 71.283 | 1.00 | 82.32 |
| ATOM | 1047 | O | LYS | 143 | 27.751 | 31.974 | 71.002 | 1.00 | 82.33 |
| ATOM | 1048 | N | LEU | 144 | 26.884 | 29.932 | 71.409 | 1.00 | 79.90 |
| ATOM | 1049 | CA | LEU | 144 | 28.146 | 29.233 | 71.198 | 1.00 | 77.12 |
| ATOM | 1050 | CB | LEU | 144 | 28.653 | 28.634 | 72.517 | 1.00 | 78.89 |
| ATOM | 1051 | CG | LEU | 144 | 29.417 | 29.543 | 73.491 | 1.00 | 80.11 |
| ATOM | 1052 | CD1 | LEU | 144 | 28.560 | 30.727 | 73.924 | 1.00 | 81.77 |
| ATOM | 1053 | CD2 | LEU | 144 | 29.836 | 28.721 | 74.698 | 1.00 | 80.96 |
| ATOM | 1054 | C | LEU | 144 | 27.993 | 28.132 | 70.156 | 1.00 | 73.23 |
| ATOM | 1055 | O | LEU | 144 | 26.876 | 27.742 | 69.810 | 1.00 | 72.89 |
| ATOM | 1056 | N | PRO | 145 | 29.119 | 27.628 | 69.628 | 1.00 | 70.01 |
| ATOM | 1057 | CD | PRO | 145 | 30.498 | 28.104 | 69.833 | 1.00 | 68.83 |
| ATOM | 1058 | CA | PRO | 145 | 29.081 | 26.565 | 68.621 | 1.00 | 67.77 |
| ATOM | 1059 | CB | PRO | 145 | 30.555 | 26.356 | 68.285 | 1.00 | 68.79 |
| ATOM | 1060 | CG | PRO | 145 | 31.159 | 27.706 | 68.542 | 1.00 | 69.21 |
| ATOM | 1061 | C | PRO | 145 | 28.434 | 25.299 | 69.181 | 1.00 | 65.49 |
| ATOM | 1062 | O | PRO | 145 | 28.615 | 24.963 | 70.351 | 1.00 | 64.23 |
| ATOM | 1063 | N | LEU | 146 | 27.677 | 24.603 | 68.340 | 1.00 | 63.31 |
| ATOM | 1064 | CA | LEU | 146 | 27.007 | 23.383 | 68.757 | 1.00 | 61.72 |
| ATOM | 1065 | CB | LEU | 146 | 25.492 | 23.532 | 68.602 | 1.00 | 62.15 |
| ATOM | 1066 | CG | LEU | 146 | 24.678 | 22.285 | 68.945 | 1.00 | 62.90 |
| ATOM | 1067 | CD1 | LEU | 146 | 25.011 | 21.842 | 70.353 | 1.00 | 64.57 |
| ATOM | 1068 | CD2 | LEU | 146 | 23.194 | 22.577 | 68.817 | 1.00 | 65.06 |
| ATOM | 1069 | C | LEU | 146 | 27.473 | 22.152 | 67.985 | 1.00 | 59.94 |
| ATOM | 1070 | O | LEU | 146 | 27.342 | 22.086 | 66.763 | 1.00 | 59.04 |
| ATOM | 1071 | N | GLY | 147 | 28.028 | 21.189 | 68.721 | 1.00 | 58.65 |
| ATOM | 1072 | CA | GLY | 147 | 28.492 | 19.939 | 68.136 | 1.00 | 54.15 |
| ATOM | 1073 | C | GLY | 147 | 27.444 | 18.891 | 68.465 | 1.00 | 49.71 |
| ATOM | 1074 | O | GLY | 147 | 27.175 | 18.628 | 69.635 | 1.00 | 50.70 |
| ATOM | 1075 | N | PHE | 148 | 26.854 | 18.287 | 67.440 | 1.00 | 46.12 |
| ATOM | 1076 | CA | PHE | 148 | 25.795 | 17.297 | 67.635 | 1.00 | 42.39 |

| | | | | | | | | | |
|---|---|---|---|---|---|---|---|---|---|
| ATOM | 1077 | CB | PHE | 148 | 24.610 | 17.675 | 66.740 | 1.00 | 39.68 |
| ATOM | 1078 | CG | PHE | 148 | 23.366 | 16.864 | 66.977 | 1.00 | 38.24 |
| ATOM | 1079 | CD1 | PHE | 148 | 22.326 | 16.901 | 66.056 | 1.00 | 36.04 |
| ATOM | 1080 | CD2 | PHE | 148 | 23.212 | 16.102 | 68.132 | 1.00 | 36.13 |
| ATOM | 1081 | CE1 | PHE | 148 | 21.148 | 16.194 | 66.279 | 1.00 | 38.53 |
| ATOM | 1082 | CE2 | PHE | 148 | 22.042 | 15.395 | 68.365 | 1.00 | 35.28 |
| ATOM | 1083 | CZ | PHE | 148 | 21.005 | 15.440 | 67.437 | 1.00 | 37.48 |
| ATOM | 1084 | C | PHE | 148 | 26.197 | 15.840 | 67.354 | 1.00 | 41.67 |
| ATOM | 1085 | O | PHE | 148 | 26.463 | 15.475 | 66.205 | 1.00 | 42.24 |
| ATOM | 1086 | N | THR | 149 | 26.247 | 15.013 | 68.398 | 1.00 | 40.23 |
| ATOM | 1087 | CA | THR | 149 | 26.562 | 13.593 | 68.222 | 1.00 | 36.30 |
| ATOM | 1088 | CB | THR | 149 | 27.281 | 13.001 | 69.442 | 1.00 | 36.36 |
| ATOM | 1089 | OG1 | THR | 149 | 28.580 | 13.597 | 69.560 | 1.00 | 37.54 |
| ATOM | 1090 | CG2 | THR | 149 | 27.444 | 11.492 | 69.286 | 1.00 | 37.01 |
| ATOM | 1091 | C | THR | 149 | 25.212 | 12.909 | 68.039 | 1.00 | 34.65 |
| ATOM | 1092 | O | THR | 149 | 24.412 | 12.836 | 68.967 | 1.00 | 31.13 |
| ATOM | 1093 | N | PHE | 150 | 24.972 | 12.422 | 66.825 | 1.00 | 33.67 |
| ATOM | 1094 | CA | PHE | 150 | 23.714 | 11.782 | 66.456 | 1.00 | 34.60 |
| ATOM | 1095 | CB | PHE | 150 | 23.061 | 12.614 | 65.336 | 1.00 | 32.78 |
| ATOM | 1096 | CG | PHE | 150 | 21.739 | 12.086 | 64.854 | 1.00 | 30.57 |
| ATOM | 1097 | CD1 | PHE | 150 | 21.625 | 11.513 | 63.595 | 1.00 | 30.43 |
| ATOM | 1098 | CD2 | PHE | 150 | 20.598 | 12.213 | 65.637 | 1.00 | 31.90 |
| ATOM | 1099 | CE1 | PHE | 150 | 20.382 | 11.076 | 63.115 | 1.00 | 34.54 |
| ATOM | 1100 | CE2 | PHE | 150 | 19.356 | 11.783 | 65.176 | 1.00 | 30.63 |
| ATOM | 1101 | CZ | PHE | 150 | 19.241 | 11.213 | 63.913 | 1.00 | 32.01 |
| ATOM | 1102 | C | PHE | 150 | 24.011 | 10.358 | 65.991 | 1.00 | 35.95 |
| ATOM | 1103 | O | PHE | 150 | 24.369 | 10.128 | 64.836 | 1.00 | 38.42 |
| ATOM | 1104 | N | SER | 151 | 23.843 | 9.412 | 66.908 | 1.00 | 36.96 |
| ATOM | 1105 | CA | SER | 151 | 24.129 | 7.995 | 66.680 | 1.00 | 34.37 |
| ATOM | 1106 | CB | SER | 151 | 24.186 | 7.271 | 68.025 | 1.00 | 35.80 |
| ATOM | 1107 | OG | SER | 151 | 25.111 | 7.897 | 68.892 | 1.00 | 39.97 |
| ATOM | 1108 | C | SER | 151 | 23.189 | 7.228 | 65.770 | 1.00 | 32.05 |
| ATOM | 1109 | O | SER | 151 | 22.537 | 6.292 | 66.215 | 1.00 | 32.11 |
| ATOM | 1110 | N | PHE | 152 | 23.110 | 7.611 | 64.505 | 1.00 | 31.41 |
| ATOM | 1111 | CA | PHE | 152 | 22.253 | 6.902 | 63.563 | 1.00 | 31.81 |

| | | | | | | | | | |
|---|---|---|---|---|---|---|---|---|---|
| ATOM | 1112 | CB | PHE | 152 | 20.824 | 7.464 | 63.570 | 1.00 | 34.43 |
| ATOM | 1113 | CG | PHE | 152 | 20.149 | 7.372 | 64.904 | 1.00 | 34.95 |
| ATOM | 1114 | CD1 | PHE | 152 | 20.278 | 8.401 | 65.838 | 1.00 | 32.95 |
| ATOM | 1115 | CD2 | PHE | 152 | 19.439 | 6.228 | 65.256 | 1.00 | 35.34 |
| ATOM | 1116 | CE1 | PHE | 152 | 19.713 | 8.291 | 67.108 | 1.00 | 35.00 |
| ATOM | 1117 | CE2 | PHE | 152 | 18.868 | 6.102 | 66.526 | 1.00 | 35.79 |
| ATOM | 1118 | CZ | PHE | 152 | 19.005 | 7.135 | 67.454 | 1.00 | 38.15 |
| ATOM | 1119 | C | PHE | 152 | 22.845 | 7.010 | 62.171 | 1.00 | 31.95 |
| ATOM | 1120 | O | PHE | 152 | 23.727 | 7.831 | 61.921 | 1.00 | 31.72 |
| ATOM | 1121 | N | PRO | 153 | 22.386 | 6.164 | 61.247 | 1.00 | 32.44 |
| ATOM | 1122 | CD | PRO | 153 | 21.374 | 5.098 | 61.343 | 1.00 | 30.73 |
| ATOM | 1123 | CA | PRO | 153 | 22.942 | 6.248 | 59.896 | 1.00 | 34.59 |
| ATOM | 1124 | CB | PRO | 153 | 22.397 | 4.991 | 59.225 | 1.00 | 31.34 |
| ATOM | 1125 | CG | PRO | 153 | 21.072 | 4.812 | 59.884 | 1.00 | 31.98 |
| ATOM | 1126 | C | PRO | 153 | 22.507 | 7.535 | 59.201 | 1.00 | 37.30 |
| ATOM | 1127 | O | PRO | 153 | 21.310 | 7.813 | 59.067 | 1.00 | 39.02 |
| ATOM | 1128 | N | VAL | 154 | 23.483 | 8.325 | 58.770 | 1.00 | 39.02 |
| ATOM | 1129 | CA | VAL | 154 | 23.187 | 9.581 | 58.092 | 1.00 | 40.43 |
| ATOM | 1130 | CB | VAL | 154 | 23.446 | 10.792 | 59.007 | 1.00 | 39.28 |
| ATOM | 1131 | CG1 | VAL | 154 | 23.191 | 12.081 | 58.238 | 1.00 | 41.18 |
| ATOM | 1132 | CG2 | VAL | 154 | 22.557 | 10.727 | 60.221 | 1.00 | 38.37 |
| ATOM | 1133 | C | VAL | 154 | 24.023 | 9.785 | 56.837 | 1.00 | 41.48 |
| ATOM | 1134 | O | VAL | 154 | 25.241 | 9.602 | 56.861 | 1.00 | 41.28 |
| ATOM | 1135 | N | ARG | 155 | 23.365 | 10.162 | 55.743 | 1.00 | 43.31 |
| ATOM | 1136 | CA | ARG | 155 | 24.072 | 10.441 | 54.495 | 1.00 | 46.32 |
| ATOM | 1137 | CB | ARG | 155 | 23.233 | 10.058 | 53.280 | 1.00 | 47.31 |
| ATOM | 1138 | CG | ARG | 155 | 23.809 | 10.586 | 51.968 | 1.00 | 52.20 |
| ATOM | 1139 | CD | ARG | 155 | 23.563 | 9.614 | 50.844 | 1.00 | 55.56 |
| ATOM | 1140 | NE | ARG | 155 | 24.419 | 8.437 | 50.968 | 1.00 | 59.93 |
| ATOM | 1141 | CZ | ARG | 155 | 24.068 | 7.217 | 50.573 | 1.00 | 61.41 |
| ATOM | 1142 | NH1 | ARG | 155 | 22.874 | 7.011 | 50.032 | 1.00 | 63.00 |
| ATOM | 1143 | NH2 | ARG | 155 | 24.910 | 6.203 | 50.717 | 1.00 | 63.35 |
| ATOM | 1144 | C | ARG | 155 | 24.367 | 11.934 | 54.456 | 1.00 | 46.23 |
| ATOM | 1145 | O | ARG | 155 | 23.486 | 12.737 | 54.166 | 1.00 | 47.64 |
| ATOM | 1146 | N | HIS | 156 | 25.613 | 12.291 | 54.754 | 1.00 | 47.03 |

| | | | | | | | | | |
|---|---|---|---|---|---|---|---|---|---|
| ATOM | 1147 | CA | HIS | 156 | 26.046 | 13.682 | 54.791 | 1.00 | 48.05 |
| ATOM | 1148 | CB | HIS | 156 | 27.318 | 13.834 | 55.632 | 1.00 | 49.62 |
| ATOM | 1149 | CG | HIS | 156 | 27.157 | 13.444 | 57.066 | 1.00 | 52.65 |
| ATOM | 1150 | CD2 | HIS | 156 | 26.274 | 12.619 | 57.676 | 1.00 | 53.99 |
| ATOM | 1151 | ND1 | HIS | 156 | 27.990 | 13.916 | 58.057 | 1.00 | 53.35 |
| ATOM | 1152 | CE1 | HIS | 156 | 27.625 | 13.401 | 59.218 | 1.00 | 54.78 |
| ATOM | 1153 | NE2 | HIS | 156 | 26.586 | 12.610 | 59.014 | 1.00 | 54.28 |
| ATOM | 1154 | C | HIS | 156 | 26.334 | 14.317 | 53.440 | 1.00 | 48.30 |
| ATOM | 1155 | O | HIS | 156 | 26.872 | 13.677 | 52.535 | 1.00 | 47.38 |
| ATOM | 1156 | N | GLU | 157 | 25.969 | 15.589 | 53.319 | 1.00 | 47.98 |
| ATOM | 1157 | CA | GLU | 157 | 26.256 | 16.343 | 52.114 | 1.00 | 48.38 |
| ATOM | 1158 | CB | GLU | 157 | 25.113 | 17.296 | 51.749 | 1.00 | 51.05 |
| ATOM | 1159 | CG | GLU | 157 | 25.462 | 18.198 | 50.558 | 1.00 | 57.22 |
| ATOM | 1160 | CD | GLU | 157 | 24.422 | 19.276 | 50.275 | 1.00 | 58.92 |
| ATOM | 1161 | OE1 | GLU | 157 | 23.299 | 18.931 | 49.845 | 1.00 | 60.91 |
| ATOM | 1162 | OE2 | GLU | 157 | 24.734 | 20.471 | 50.485 | 1.00 | 60.69 |
| ATOM | 1163 | C | GLU | 157 | 27.475 | 17.138 | 52.547 | 1.00 | 45.12 |
| ATOM | 1164 | O | GLU | 157 | 28.349 | 17.457 | 51.749 | 1.00 | 43.91 |
| ATOM | 1165 | N | ASP | 158 | 27.529 | 17.427 | 53.843 | 1.00 | 43.85 |
| ATOM | 1166 | CA | ASP | 158 | 28.633 | 18.174 | 54.416 | 1.00 | 43.81 |
| ATOM | 1167 | CB | ASP | 158 | 28.479 | 19.654 | 54.085 | 1.00 | 46.74 |
| ATOM | 1168 | CG | ASP | 158 | 29.743 | 20.445 | 54.349 | 1.00 | 49.54 |
| ATOM | 1169 | OD1 | ASP | 158 | 29.760 | 21.651 | 54.016 | 1.00 | 53.34 |
| ATOM | 1170 | OD2 | ASP | 158 | 30.716 | 19.869 | 54.884 | 1.00 | 49.62 |
| ATOM | 1171 | C | ASP | 158 | 28.671 | 17.972 | 55.928 | 1.00 | 43.93 |
| ATOM | 1172 | O | ASP | 158 | 27.724 | 17.447 | 56.518 | 1.00 | 43.97 |
| ATOM | 1173 | N | ILE | 159 | 29.767 | 18.399 | 56.547 | 1.00 | 43.75 |
| ATOM | 1174 | CA | ILE | 159 | 29.963 | 18.250 | 57.983 | 1.00 | 44.27 |
| ATOM | 1175 | CB | ILE | 159 | 31.248 | 18.971 | 58.452 | 1.00 | 45.07 |
| ATOM | 1176 | CG2 | ILE | 159 | 31.069 | 20.480 | 58.354 | 1.00 | 47.24 |
| ATOM | 1177 | CG1 | ILE | 159 | 31.544 | 18.617 | 59.907 | 1.00 | 45.29 |
| ATOM | 1178 | CD1 | ILE | 159 | 31.733 | 17.140 | 60.152 | 1.00 | 48.99 |
| ATOM | 1179 | C | ILE | 159 | 28.795 | 18.744 | 58.829 | 1.00 | 44.47 |
| ATOM | 1180 | O | ILE | 159 | 28.583 | 18.254 | 59.941 | 1.00 | 44.15 |
| ATOM | 1181 | N | ASP | 160 | 28.037 | 19.709 | 58.317 | 1.00 | 44.10 |

| | | | | | | | | | |
|---|---|---|---|---|---|---|---|---|---|
| ATOM | 1182 | CA | ASP | 160 | 26.904 | 20.239 | 59.072 | 1.00 | 42.92 |
| ATOM | 1183 | CB | ASP | 160 | 27.103 | 21.734 | 59.360 | 1.00 | 44.13 |
| ATOM | 1184 | CG | ASP | 160 | 27.448 | 22.533 | 58.118 | 1.00 | 45.52 |
| ATOM | 1185 | OD1 | ASP | 160 | 28.258 | 23.479 | 58.239 | 1.00 | 47.57 |
| ATOM | 1186 | OD2 | ASP | 160 | 26.912 | 22.228 | 57.031 | 1.00 | 45.51 |
| ATOM | 1187 | C | ASP | 160 | 25.559 | 20.005 | 58.410 | 1.00 | 42.92 |
| ATOM | 1188 | O | ASP | 160 | 24.579 | 20.691 | 58.706 | 1.00 | 44.48 |
| ATOM | 1189 | N | LYS | 161 | 25.509 | 19.026 | 57.518 | 1.00 | 41.57 |
| ATOM | 1190 | CA | LYS | 161 | 24.267 | 18.692 | 56.838 | 1.00 | 41.51 |
| ATOM | 1191 | CB | LYS | 161 | 24.067 | 19.597 | 55.618 | 1.00 | 41.19 |
| ATOM | 1192 | CG | LYS | 161 | 22.783 | 19.306 | 54.863 | 1.00 | 41.39 |
| ATOM | 1193 | CD | LYS | 161 | 22.687 | 20.094 | 53.557 | 1.00 | 43.25 |
| ATOM | 1194 | CE | LYS | 161 | 21.366 | 19.809 | 52.860 | 1.00 | 40.06 |
| ATOM | 1195 | NZ | LYS | 161 | 21.335 | 20.312 | 51.468 | 1.00 | 41.02 |
| ATOM | 1196 | C | LYS | 161 | 24.258 | 17.224 | 56.397 | 1.00 | 41.66 |
| ATOM | 1197 | O | LYS | 161 | 25.239 | 16.725 | 55.838 | 1.00 | 39.36 |
| ATOM | 1198 | N | GLY | 162 | 23.143 | 16.546 | 56.654 | 1.00 | 40.90 |
| ATOM | 1199 | CA | GLY | 162 | 23.005 | 15.152 | 56.276 | 1.00 | 42.70 |
| ATOM | 1200 | C | GLY | 162 | 21.618 | 14.645 | 56.615 | 1.00 | 43.15 |
| ATOM | 1201 | O | GLY | 162 | 21.019 | 15.085 | 57.594 | 1.00 | 43.59 |
| ATOM | 1202 | N | ILE | 163 | 21.096 | 13.722 | 55.816 | 1.00 | 43.93 |
| ATOM | 1203 | CA | ILE | 163 | 19.763 | 13.190 | 56.068 | 1.00 | 45.03 |
| ATOM | 1204 | CB | ILE | 163 | 18.958 | 13.031 | 54.755 | 1.00 | 46.16 |
| ATOM | 1205 | CG2 | ILE | 163 | 18.943 | 14.352 | 53.985 | 1.00 | 45.82 |
| ATOM | 1206 | CG1 | ILE | 163 | 19.585 | 11.938 | 53.889 | 1.00 | 46.11 |
| ATOM | 1207 | CD1 | ILE | 163 | 18.812 | 11.638 | 52.613 | 1.00 | 48.51 |
| ATOM | 1208 | C | ILE | 163 | 19.812 | 11.833 | 56.764 | 1.00 | 46.49 |
| ATOM | 1209 | O | ILE | 163 | 20.771 | 11.074 | 56.609 | 1.00 | 45.36 |
| ATOM | 1210 | N | LEU | 164 | 18.767 | 11.545 | 57.533 | 1.00 | 47.21 |
| ATOM | 1211 | CA | LEU | 164 | 18.649 | 10.286 | 58.253 | 1.00 | 47.53 |
| ATOM | 1212 | CB | LEU | 164 | 17.623 | 10.414 | 59.379 | 1.00 | 47.11 |
| ATOM | 1213 | CG | LEU | 164 | 17.135 | 9.126 | 60.049 | 1.00 | 47.15 |
| ATOM | 1214 | CD1 | LEU | 164 | 18.265 | 8.469 | 60.832 | 1.00 | 45.45 |
| ATOM | 1215 | CD2 | LEU | 164 | 15.981 | 9.465 | 60.977 | 1.00 | 47.00 |
| ATOM | 1216 | C | LEU | 164 | 18.189 | 9.220 | 57.277 | 1.00 | 48.06 |

| | | | | | | | | | |
|---|---|---|---|---|---|---|---|---|---|
| ATOM | 1217 | O | LEU | 164 | 17.137 | 9.352 | 56.657 | 1.00 | 48.49 |
| ATOM | 1218 | N | LEU | 165 | 18.977 | 8.161 | 57.137 | 1.00 | 48.32 |
| ATOM | 1219 | CA | LEU | 165 | 18.614 | 7.093 | 56.224 | 1.00 | 47.47 |
| ATOM | 1220 | CB | LEU | 165 | 19.827 | 6.208 | 55.954 | 1.00 | 45.44 |
| ATOM | 1221 | CG | LEU | 165 | 20.867 | 6.978 | 55.140 | 1.00 | 47.60 |
| ATOM | 1222 | CD1 | LEU | 165 | 22.128 | 6.155 | 54.956 | 1.00 | 47.47 |
| ATOM | 1223 | CD2 | LEU | 165 | 20.261 | 7.342 | 53.786 | 1.00 | 48.48 |
| ATOM | 1224 | C | LEU | 165 | 17.460 | 6.300 | 56.814 | 1.00 | 46.86 |
| ATOM | 1225 | O | LEU | 165 | 16.497 | 5.985 | 56.120 | 1.00 | 46.90 |
| ATOM | 1226 | N | ASN | 166 | 17.562 | 5.992 | 58.101 | 1.00 | 46.60 |
| ATOM | 1227 | CA | ASN | 166 | 16.521 | 5.266 | 58.817 | 1.00 | 47.23 |
| ATOM | 1228 | CB | ASN | 166 | 16.282 | 3.883 | 58.200 | 1.00 | 49.17 |
| ATOM | 1229 | CG | ASN | 166 | 17.542 | 3.053 | 58.118 | 1.00 | 50.36 |
| ATOM | 1230 | OD1 | ASN | 166 | 18.205 | 2.997 | 57.076 | 1.00 | 50.62 |
| ATOM | 1231 | ND2 | ASN | 166 | 17.888 | 2.406 | 59.223 | 1.00 | 50.50 |
| ATOM | 1232 | C | ASN | 166 | 16.913 | 5.123 | 60.279 | 1.00 | 47.60 |
| ATOM | 1233 | O | ASN | 166 | 18.096 | 5.177 | 60.623 | 1.00 | 48.53 |
| ATOM | 1234 | N | TRP | 167 | 15.916 | 4.966 | 61.142 | 1.00 | 46.96 |
| ATOM | 1235 | CA | TRP | 167 | 16.166 | 4.815 | 62.571 | 1.00 | 45.46 |
| ATOM | 1236 | CB | TRP | 167 | 14.890 | 5.085 | 63.376 | 1.00 | 47.63 |
| ATOM | 1237 | CG | TRP | 167 | 14.433 | 6.519 | 63.454 | 1.00 | 49.15 |
| ATOM | 1238 | CD2 | TRP | 167 | 15.093 | 7.602 | 64.126 | 1.00 | 49.07 |
| ATOM | 1239 | CE2 | TRP | 167 | 14.237 | 8.725 | 64.050 | 1.00 | 48.21 |
| ATOM | 1240 | CE3 | TRP | 167 | 16.321 | 7.732 | 64.787 | 1.00 | 49.17 |
| ATOM | 1241 | CD1 | TRP | 167 | 13.242 | 7.022 | 63.003 | 1.00 | 49.03 |
| ATOM | 1242 | NE1 | TRP | 167 | 13.117 | 8.343 | 63.361 | 1.00 | 48.46 |
| ATOM | 1243 | CZ2 | TRP | 167 | 14.569 | 9.962 | 64.614 | 1.00 | 47.68 |
| ATOM | 1244 | CZ3 | TRP | 167 | 16.652 | 8.966 | 65.348 | 1.00 | 49.58 |
| ATOM | 1245 | CH2 | TRP | 167 | 15.777 | 10.064 | 65.256 | 1.00 | 48.80 |
| ATOM | 1246 | C | TRP | 167 | 16.647 | 3.394 | 62.890 | 1.00 | 43.28 |
| ATOM | 1247 | O | TRP | 167 | 16.425 | 2.461 | 62.119 | 1.00 | 42.86 |
| ATOM | 1248 | N | THR | 168 | 17.297 | 3.245 | 64.038 | 1.00 | 41.63 |
| ATOM | 1249 | CA | THR | 168 | 17.796 | 1.953 | 64.501 | 1.00 | 40.13 |
| ATOM | 1250 | CB | THR | 168 | 19.275 | 1.723 | 64.086 | 1.00 | 37.87 |
| ATOM | 1251 | OG1 | THR | 168 | 20.082 | 2.795 | 64.587 | 1.00 | 33.52 |

| | | | | | | | | | |
|---|---|---|---|---|---|---|---|---|---|
| ATOM | 1252 | CG2 | THR | 168 | 19.417 | 1.647 | 62.566 | 1.00 | 34.11 |
| ATOM | 1253 | C | THR | 168 | 17.719 | 1.943 | 66.029 | 1.00 | 41.33 |
| ATOM | 1254 | O | THR | 168 | 17.382 | 2.953 | 66.649 | 1.00 | 41.41 |
| ATOM | 1255 | N | LYS | 169 | 18.025 | 0.799 | 66.631 | 1.00 | 42.06 |
| ATOM | 1256 | CA | LYS | 169 | 18.013 | 0.672 | 68.083 | 1.00 | 42.59 |
| ATOM | 1257 | CB | LYS | 169 | 19.077 | 1.594 | 68.683 | 1.00 | 39.56 |
| ATOM | 1258 | CG | LYS | 169 | 20.497 | 1.209 | 68.287 | 1.00 | 36.24 |
| ATOM | 1259 | CD | LYS | 169 | 21.528 | 2.170 | 68.840 | 1.00 | 33.26 |
| ATOM | 1260 | CE | LYS | 169 | 21.481 | 3.514 | 68.133 | 1.00 | 30.26 |
| ATOM | 1261 | NZ | LYS | 169 | 22.589 | 4.373 | 68.610 | 1.00 | 32.75 |
| ATOM | 1262 | C | LYS | 169 | 16.661 | 0.933 | 68.751 | 1.00 | 45.26 |
| ATOM | 1263 | O | LYS | 169 | 16.598 | 1.191 | 69.955 | 1.00 | 45.85 |
| ATOM | 1264 | N | GLY | 170 | 15.583 | 0.881 | 67.975 | 1.00 | 47.46 |
| ATOM | 1265 | CA | GLY | 170 | 14.267 | 1.083 | 68.555 | 1.00 | 52.44 |
| ATOM | 1266 | C | GLY | 170 | 13.552 | 2.394 | 68.295 | 1.00 | 55.24 |
| ATOM | 1267 | O | GLY | 170 | 12.324 | 2.422 | 68.275 | 1.00 | 56.11 |
| ATOM | 1268 | N | PHE | 171 | 14.293 | 3.482 | 68.118 | 1.00 | 58.10 |
| ATOM | 1269 | CA | PHE | 171 | 13.668 | 4.777 | 67.861 | 1.00 | 61.86 |
| ATOM | 1270 | CB | PHE | 171 | 14.734 | 5.846 | 67.613 | 1.00 | 62.35 |
| ATOM | 1271 | CG | PHE | 171 | 15.449 | 6.285 | 68.856 | 1.00 | 64.08 |
| ATOM | 1272 | CD1 | PHE | 171 | 16.060 | 5.354 | 69.691 | 1.00 | 66.00 |
| ATOM | 1273 | CD2 | PHE | 171 | 15.511 | 7.630 | 69.196 | 1.00 | 64.53 |
| ATOM | 1274 | CE1 | PHE | 171 | 16.721 | 5.760 | 70.851 | 1.00 | 66.96 |
| ATOM | 1275 | CE2 | PHE | 171 | 16.170 | 8.046 | 70.352 | 1.00 | 65.93 |
| ATOM | 1276 | CZ | PHE | 171 | 16.776 | 7.109 | 71.180 | 1.00 | 66.07 |
| ATOM | 1277 | C | PHE | 171 | 12.727 | 4.697 | 66.663 | 1.00 | 63.91 |
| ATOM | 1278 | O | PHE | 171 | 12.994 | 3.975 | 65.702 | 1.00 | 63.50 |
| ATOM | 1279 | N | LYS | 172 | 11.620 | 5.430 | 66.727 | 1.00 | 65.77 |
| ATOM | 1280 | CA | LYS | 172 | 10.657 | 5.424 | 65.633 | 1.00 | 68.37 |
| ATOM | 1281 | CB | LYS | 172 | 9.738 | 4.197 | 65.727 | 1.00 | 70.16 |
| ATOM | 1282 | CG | LYS | 172 | 8.814 | 4.035 | 64.517 | 1.00 | 72.04 |
| ATOM | 1283 | CD | LYS | 172 | 7.867 | 2.842 | 64.647 | 1.00 | 73.43 |
| ATOM | 1284 | CE | LYS | 172 | 6.977 | 2.718 | 63.406 | 1.00 | 74.42 |
| ATOM | 1285 | NZ | LYS | 172 | 5.933 | 1.655 | 63.525 | 1.00 | 73.62 |
| ATOM | 1286 | C | LYS | 172 | 9.808 | 6.688 | 65.606 | 1.00 | 69.18 |

| | | | | | | | | | |
|---|---|---|---|---|---|---|---|---|---|
| ATOM | 1287 | O | LYS | 172 | 8.599 | 6.642 | 65.838 | 1.00 | 70.01 |
| ATOM | 1288 | N | ALA | 173 | 10.445 | 7.820 | 65.332 | 1.00 | 68.98 |
| ATOM | 1289 | CA | ALA | 173 | 9.734 | 9.086 | 65.251 | 1.00 | 69.07 |
| ATOM | 1290 | CB | ALA | 173 | 10.598 | 10.210 | 65.818 | 1.00 | 68.41 |
| ATOM | 1291 | C | ALA | 173 | 9.424 | 9.339 | 63.776 | 1.00 | 69.07 |
| ATOM | 1292 | O | ALA | 173 | 10.336 | 9.471 | 62.962 | 1.00 | 69.61 |
| ATOM | 1293 | N | SER | 174 | 8.139 | 9.394 | 63.432 | 1.00 | 69.06 |
| ATOM | 1294 | CA | SER | 174 | 7.735 | 9.620 | 62.047 | 1.00 | 68.32 |
| ATOM | 1295 | CB | SER | 174 | 6.217 | 9.491 | 61.901 | 1.00 | 69.02 |
| ATOM | 1296 | OG | SER | 174 | 5.546 | 10.503 | 62.632 | 1.00 | 68.18 |
| ATOM | 1297 | C | SER | 174 | 8.173 | 10.996 | 61.568 | 1.00 | 67.71 |
| ATOM | 1298 | O | SER | 174 | 8.410 | 11.897 | 62.370 | 1.00 | 68.23 |
| ATOM | 1299 | N | GLY | 175 | 8.288 | 11.148 | 60.254 | 1.00 | 67.37 |
| ATOM | 1300 | CA | GLY | 175 | 8.688 | 12.424 | 59.690 | 1.00 | 67.08 |
| ATOM | 1301 | C | GLY | 175 | 10.143 | 12.787 | 59.915 | 1.00 | 66.86 |
| ATOM | 1302 | O | GLY | 175 | 10.507 | 13.962 | 59.855 | 1.00 | 67.38 |
| ATOM | 1303 | N | ALA | 176 | 10.979 | 11.786 | 60.172 | 1.00 | 66.42 |
| ATOM | 1304 | CA | ALA | 176 | 12.400 | 12.018 | 60.401 | 1.00 | 64.67 |
| ATOM | 1305 | CB | ALA | 176 | 12.828 | 11.360 | 61.699 | 1.00 | 64.11 |
| ATOM | 1306 | C | ALA | 176 | 13.229 | 11.475 | 59.242 | 1.00 | 64.02 |
| ATOM | 1307 | O | ALA | 176 | 14.053 | 12.183 | 58.667 | 1.00 | 65.00 |
| ATOM | 1308 | N | GLU | 177 | 12.993 | 10.214 | 58.903 | 1.00 | 63.24 |
| ATOM | 1309 | CA | GLU | 177 | 13.710 | 9.544 | 57.825 | 1.00 | 63.08 |
| ATOM | 1310 | CB | GLU | 177 | 13.147 | 8.127 | 57.639 | 1.00 | 62.97 |
| ATOM | 1311 | CG | GLU | 177 | 13.315 | 7.224 | 58.865 | 1.00 | 64.81 |
| ATOM | 1312 | CD | GLU | 177 | 12.712 | 5.837 | 58.682 | 1.00 | 64.99 |
| ATOM | 1313 | OE1 | GLU | 177 | 12.948 | 4.972 | 59.552 | 1.00 | 65.80 |
| ATOM | 1314 | OE2 | GLU | 177 | 12.003 | 5.612 | 57.677 | 1.00 | 64.52 |
| ATOM | 1315 | C | GLU | 177 | 13.669 | 10.293 | 56.491 | 1.00 | 62.92 |
| ATOM | 1316 | O | GLU | 177 | 12.602 | 10.489 | 55.908 | 1.00 | 63.26 |
| ATOM | 1317 | N | GLY | 178 | 14.838 | 10.708 | 56.013 | 1.00 | 62.46 |
| ATOM | 1318 | CA | GLY | 178 | 14.911 | 11.406 | 54.741 | 1.00 | 61.36 |
| ATOM | 1319 | C | GLY | 178 | 15.095 | 12.911 | 54.805 | 1.00 | 60.52 |
| ATOM | 1320 | O | GLY | 178 | 15.337 | 13.539 | 53.777 | 1.00 | 61.73 |
| ATOM | 1321 | N | ASN | 179 | 14.990 | 13.498 | 55.993 | 1.00 | 59.84 |

| | | | | | | | | | |
|---|---|---|---|---|---|---|---|---|---|
| ATOM | 1322 | CA | ASN | 179 | 15.139 | 14.942 | 56.134 | 1.00 | 59.11 |
| ATOM | 1323 | CB | ASN | 179 | 13.985 | 15.512 | 56.959 | 1.00 | 59.72 |
| ATOM | 1324 | CG | ASN | 179 | 12.630 | 15.217 | 56.342 | 1.00 | 61.46 |
| ATOM | 1325 | OD1 | ASN | 179 | 12.423 | 15.416 | 55.143 | 1.00 | 61.86 |
| ATOM | 1326 | ND2 | ASN | 179 | 11.696 | 14.743 | 57.161 | 1.00 | 61.02 |
| ATOM | 1327 | C | ASN | 179 | 16.463 | 15.349 | 56.765 | 1.00 | 58.81 |
| ATOM | 1328 | O | ASN | 179 | 17.108 | 14.553 | 57.441 | 1.00 | 59.10 |
| ATOM | 1329 | N | ASN | 180 | 16.860 | 16.599 | 56.537 | 1.00 | 58.30 |
| ATOM | 1330 | CA | ASN | 180 | 18.107 | 17.130 | 57.079 | 1.00 | 57.96 |
| ATOM | 1331 | CB | ASN | 180 | 18.362 | 18.539 | 56.539 | 1.00 | 58.57 |
| ATOM | 1332 | CG | ASN | 180 | 19.693 | 19.112 | 57.001 | 1.00 | 60.99 |
| ATOM | 1333 | OD1 | ASN | 180 | 20.278 | 18.647 | 57.983 | 1.00 | 60.88 |
| ATOM | 1334 | ND2 | ASN | 180 | 20.171 | 20.139 | 56.302 | 1.00 | 60.74 |
| ATOM | 1335 | C | ASN | 180 | 18.036 | 17.183 | 58.600 | 1.00 | 57.30 |
| ATOM | 1336 | O | ASN | 180 | 17.388 | 18.064 | 59.162 | 1.00 | 57.94 |
| ATOM | 1337 | N | VAL | 181 | 18.709 | 16.245 | 59.261 | 1.00 | 55.49 |
| ATOM | 1338 | CA | VAL | 181 | 18.716 | 16.189 | 60.720 | 1.00 | 54.19 |
| ATOM | 1339 | CB | VAL | 181 | 19.698 | 15.109 | 61.229 | 1.00 | 53.15 |
| ATOM | 1340 | CG1 | VAL | 181 | 19.756 | 15.121 | 62.748 | 1.00 | 50.90 |
| ATOM | 1341 | CG2 | VAL | 181 | 19.258 | 13.742 | 60.731 | 1.00 | 51.33 |
| ATOM | 1342 | C | VAL | 181 | 19.089 | 17.534 | 61.333 | 1.00 | 54.31 |
| ATOM | 1343 | O | VAL | 181 | 18.473 | 17.979 | 62.299 | 1.00 | 53.21 |
| ATOM | 1344 | N | VAL | 182 | 20.110 | 18.174 | 60.777 | 1.00 | 56.27 |
| ATOM | 1345 | CA | VAL | 182 | 20.533 | 19.472 | 61.271 | 1.00 | 58.32 |
| ATOM | 1346 | CB | VAL | 182 | 21.706 | 20.033 | 60.447 | 1.00 | 58.42 |
| ATOM | 1347 | CG1 | VAL | 182 | 22.135 | 21.373 | 61.007 | 1.00 | 58.05 |
| ATOM | 1348 | CG2 | VAL | 182 | 22.867 | 19.054 | 60.460 | 1.00 | 59.02 |
| ATOM | 1349 | C | VAL | 182 | 19.339 | 20.410 | 61.125 | 1.00 | 60.16 |
| ATOM | 1350 | O | VAL | 182 | 19.052 | 21.220 | 62.008 | 1.00 | 59.87 |
| ATOM | 1351 | N | GLY | 183 | 18.640 | 20.275 | 60.003 | 1.00 | 61.05 |
| ATOM | 1352 | CA | GLY | 183 | 17.480 | 21.103 | 59.741 | 1.00 | 63.47 |
| ATOM | 1353 | C | GLY | 183 | 16.412 | 20.967 | 60.805 | 1.00 | 64.68 |
| ATOM | 1354 | O | GLY | 183 | 15.873 | 21.966 | 61.280 | 1.00 | 64.59 |
| ATOM | 1355 | N | LEU | 184 | 16.103 | 19.733 | 61.187 | 1.00 | 65.39 |
| ATOM | 1356 | CA | LEU | 184 | 15.091 | 19.502 | 62.203 | 1.00 | 66.47 |

| | | | | | | | | | |
|---|---|---|---|---|---|---|---|---|---|
| ATOM | 1357 | CB | LEU | 184 | 14.855 | 18.005 | 62.387 | 1.00 | 66.17 |
| ATOM | 1358 | CG | LEU | 184 | 14.407 | 17.254 | 61.132 | 1.00 | 67.51 |
| ATOM | 1359 | CD1 | LEU | 184 | 14.116 | 15.805 | 61.486 | 1.00 | 66.63 |
| ATOM | 1360 | CD2 | LEU | 184 | 13.168 | 17.913 | 60.546 | 1.00 | 68.22 |
| ATOM | 1361 | C | LEU | 184 | 15.502 | 20.130 | 63.528 | 1.00 | 67.67 |
| ATOM | 1362 | O | LEU | 184 | 14.651 | 20.570 | 64.301 | 1.00 | 68.91 |
| ATOM | 1363 | N | LEU | 185 | 16.804 | 20.176 | 63.790 | 1.00 | 68.11 |
| ATOM | 1364 | CA | LEU | 185 | 17.297 | 20.759 | 65.031 | 1.00 | 68.91 |
| ATOM | 1365 | CB | LEU | 185 | 18.797 | 20.501 | 65.194 | 1.00 | 67.32 |
| ATOM | 1366 | CG | LEU | 185 | 19.409 | 21.060 | 66.482 | 1.00 | 66.21 |
| ATOM | 1367 | CD1 | LEU | 185 | 18.776 | 20.375 | 67.676 | 1.00 | 65.16 |
| ATOM | 1368 | CD2 | LEU | 185 | 20.913 | 20.851 | 66.486 | 1.00 | 66.43 |
| ATOM | 1369 | C | LEU | 185 | 17.034 | 22.262 | 65.058 | 1.00 | 70.10 |
| ATOM | 1370 | O | LEU | 185 | 16.422 | 22.776 | 65.991 | 1.00 | 70.26 |
| ATOM | 1371 | N | ARG | 186 | 17.505 | 22.962 | 64.033 | 1.00 | 71.83 |
| ATOM | 1372 | CA | ARG | 186 | 17.314 | 24.403 | 63.948 | 1.00 | 73.78 |
| ATOM | 1373 | CB | ARG | 186 | 18.015 | 24.941 | 62.700 | 1.00 | 73.97 |
| ATOM | 1374 | CG | ARG | 186 | 19.533 | 24.881 | 62.804 | 1.00 | 74.09 |
| ATOM | 1375 | CD | ARG | 186 | 20.206 | 24.984 | 61.448 | 1.00 | 74.37 |
| ATOM | 1376 | NE | ARG | 186 | 21.662 | 24.945 | 61.571 | 1.00 | 75.77 |
| ATOM | 1377 | CZ | ARG | 186 | 22.503 | 24.860 | 60.543 | 1.00 | 75.94 |
| ATOM | 1378 | NH1 | ARG | 186 | 22.036 | 24.800 | 59.303 | 1.00 | 75.97 |
| ATOM | 1379 | NH2 | ARG | 186 | 23.815 | 24.841 | 60.755 | 1.00 | 75.99 |
| ATOM | 1380 | C | ARG | 186 | 15.825 | 24.737 | 63.927 | 1.00 | 74.93 |
| ATOM | 1381 | O | ARG | 186 | 15.365 | 25.609 | 64.665 | 1.00 | 74.59 |
| ATOM | 1382 | N | ASP | 187 | 15.074 | 24.023 | 63.095 | 1.00 | 76.23 |
| ATOM | 1383 | CA | ASP | 187 | 13.632 | 24.225 | 62.981 | 1.00 | 77.59 |
| ATOM | 1384 | CB | ASP | 187 | 13.018 | 23.128 | 62.102 | 1.00 | 75.83 |
| ATOM | 1385 | CG | ASP | 187 | 13.203 | 23.391 | 60.614 | 1.00 | 74.87 |
| ATOM | 1386 | OD1 | ASP | 187 | 14.193 | 24.051 | 60.234 | 1.00 | 73.64 |
| ATOM | 1387 | OD2 | ASP | 187 | 12.359 | 22.924 | 59.820 | 1.00 | 74.33 |
| ATOM | 1388 | C | ASP | 187 | 12.945 | 24.236 | 64.349 | 1.00 | 79.78 |
| ATOM | 1389 | O | ASP | 187 | 11.963 | 24.952 | 64.551 | 1.00 | 80.50 |
| ATOM | 1390 | N | ALA | 188 | 13.461 | 23.445 | 65.286 | 1.00 | 81.21 |
| ATOM | 1391 | CA | ALA | 188 | 12.883 | 23.379 | 66.625 | 1.00 | 82.86 |

| | | | | | | | | | |
|---|---|---|---|---|---|---|---|---|---|
| ATOM | 1392 | CB | ALA | 188 | 13.118 | 22.000 | 67.230 | 1.00 | 83.00 |
| ATOM | 1393 | C | ALA | 188 | 13.477 | 24.456 | 67.525 | 1.00 | 84.14 |
| ATOM | 1394 | O | ALA | 188 | 12.783 | 25.019 | 68.376 | 1.00 | 84.10 |
| ATOM | 1395 | N | ILE | 189 | 14.763 | 24.736 | 67.338 | 1.00 | 85.33 |
| ATOM | 1396 | CA | ILE | 189 | 15.445 | 25.753 | 68.127 | 1.00 | 86.87 |
| ATOM | 1397 | CB | ILE | 189 | 16.947 | 25.819 | 67.776 | 1.00 | 86.40 |
| ATOM | 1398 | CG2 | ILE | 189 | 17.585 | 27.049 | 68.409 | 1.00 | 85.37 |
| ATOM | 1399 | CG1 | ILE | 189 | 17.641 | 24.541 | 68.253 | 1.00 | 86.52 |
| ATOM | 1400 | CD1 | ILE | 189 | 19.136 | 24.516 | 68.004 | 1.00 | 86.36 |
| ATOM | 1401 | C | ILE | 189 | 14.812 | 27.114 | 67.871 | 1.00 | 88.85 |
| ATOM | 1402 | O | ILE | 189 | 14.802 | 27.978 | 68.748 | 1.00 | 89.64 |
| ATOM | 1403 | N | LYS | 190 | 14.278 | 27.295 | 66.666 | 1.00 | 90.36 |
| ATOM | 1404 | CA | LYS | 190 | 13.638 | 28.551 | 66.291 | 1.00 | 91.75 |
| ATOM | 1405 | CB | LYS | 190 | 13.678 | 28.729 | 64.770 | 1.00 | 92.26 |
| ATOM | 1406 | CG | LYS | 190 | 15.032 | 29.205 | 64.234 | 1.00 | 93.56 |
| ATOM | 1407 | CD | LYS | 190 | 16.174 | 28.282 | 64.652 | 1.00 | 94.17 |
| ATOM | 1408 | CE | LYS | 190 | 17.507 | 28.722 | 64.064 | 1.00 | 94.42 |
| ATOM | 1409 | NZ | LYS | 190 | 18.605 | 27.773 | 64.409 | 1.00 | 93.91 |
| ATOM | 1410 | C | LYS | 190 | 12.202 | 28.645 | 66.803 | 1.00 | 92.34 |
| ATOM | 1411 | O | LYS | 190 | 11.612 | 29.723 | 66.817 | 1.00 | 92.82 |
| ATOM | 1412 | N | ARG | 191 | 11.639 | 27.516 | 67.221 | 1.00 | 92.78 |
| ATOM | 1413 | CA | ARG | 191 | 10.286 | 27.502 | 67.763 | 1.00 | 93.41 |
| ATOM | 1414 | CB | ARG | 191 | 9.674 | 26.108 | 67.658 | 1.00 | 93.77 |
| ATOM | 1415 | CG | ARG | 191 | 9.711 | 25.497 | 66.275 | 1.00 | 93.66 |
| ATOM | 1416 | CD | ARG | 191 | 9.530 | 23.993 | 66.378 | 1.00 | 93.81 |
| ATOM | 1417 | NE | ARG | 191 | 9.816 | 23.310 | 65.123 | 1.00 | 93.99 |
| ATOM | 1418 | CZ | ARG | 191 | 10.012 | 22.000 | 65.017 | 1.00 | 94.26 |
| ATOM | 1419 | NH1 | ARG | 191 | 9.954 | 21.231 | 66.095 | 1.00 | 94.65 |
| ATOM | 1420 | NH2 | ARG | 191 | 10.269 | 21.459 | 63.835 | 1.00 | 94.96 |
| ATOM | 1421 | C | ARG | 191 | 10.432 | 27.866 | 69.233 | 1.00 | 94.00 |
| ATOM | 1422 | O | ARG | 191 | 9.526 | 27.654 | 70.036 | 1.00 | 94.16 |
| ATOM | 1423 | N | ARG | 192 | 11.596 | 28.408 | 69.574 | 1.00 | 94.91 |
| ATOM | 1424 | CA | ARG | 192 | 11.897 | 28.795 | 70.943 | 1.00 | 96.15 |
| ATOM | 1425 | CB | ARG | 192 | 13.049 | 27.944 | 71.482 | 1.00 | 96.57 |
| ATOM | 1426 | CG | ARG | 192 | 12.733 | 26.469 | 71.581 | 1.00 | 97.44 |

| | | | | | | | | | |
|---|---|---|---|---|---|---|---|---|---|
| ATOM | 1427 | CD | ARG | 192 | 11.737 | 26.209 | 72.689 | 1.00 | 98.38 |
| ATOM | 1428 | NE | ARG | 192 | 11.339 | 24.808 | 72.745 | 1.00 | 98.97 |
| ATOM | 1429 | CZ | ARG | 192 | 10.624 | 24.278 | 73.730 | 1.00 | 99.63 |
| ATOM | 1430 | NH1 | ARG | 192 | 10.230 | 25.035 | 74.746 | 1.00 | 99.17 |
| ATOM | 1431 | NH2 | ARG | 192 | 10.300 | 22.992 | 73.700 | 1.00 | 99.97 |
| ATOM | 1432 | C | ARG | 192 | 12.273 | 30.265 | 71.062 | 1.00 | 96.50 |
| ATOM | 1433 | O | ARG | 192 | 11.603 | 31.035 | 71.752 | 1.00 | 96.60 |
| ATOM | 1434 | N | GLY | 193 | 13.352 | 30.652 | 70.386 | 1.00 | 96.89 |
| ATOM | 1435 | CA | GLY | 193 | 13.812 | 32.026 | 70.465 | 1.00 | 97.08 |
| ATOM | 1436 | C | GLY | 193 | 14.385 | 32.217 | 71.855 | 1.00 | 97.08 |
| ATOM | 1437 | O | GLY | 193 | 15.060 | 33.204 | 72.147 | 1.00 | 96.46 |
| ATOM | 1438 | N | ASP | 194 | 14.104 | 31.235 | 72.707 | 1.00 | 97.36 |
| ATOM | 1439 | CA | ASP | 194 | 14.552 | 31.205 | 74.092 | 1.00 | 97.35 |
| ATOM | 1440 | CB | ASP | 194 | 13.938 | 29.984 | 74.789 | 1.00 | 98.43 |
| ATOM | 1441 | CG | ASP | 194 | 13.764 | 30.181 | 76.284 | 1.00 | 99.62 |
| ATOM | 1442 | OD1 | ASP | 194 | 13.117 | 31.173 | 76.683 | 1.00 | 100.29 |
| ATOM | 1443 | OD2 | ASP | 194 | 14.262 | 29.338 | 77.059 | 1.00 | 99.87 |
| ATOM | 1444 | C | ASP | 194 | 16.078 | 31.122 | 74.122 | 1.00 | 96.90 |
| ATOM | 1445 | O | ASP | 194 | 16.715 | 31.471 | 75.118 | 1.00 | 97.50 |
| ATOM | 1446 | N | PHE | 195 | 16.657 | 30.655 | 73.018 | 1.00 | 95.62 |
| ATOM | 1447 | CA | PHE | 195 | 18.105 | 30.524 | 72.896 | 1.00 | 94.15 |
| ATOM | 1448 | CB | PHE | 195 | 18.598 | 29.309 | 73.697 | 1.00 | 94.86 |
| ATOM | 1449 | CG | PHE | 195 | 18.043 | 27.988 | 73.224 | 1.00 | 95.10 |
| ATOM | 1450 | CD1 | PHE | 195 | 18.560 | 27.360 | 72.095 | 1.00 | 95.09 |
| ATOM | 1451 | CD2 | PHE | 195 | 17.005 | 27.369 | 73.916 | 1.00 | 95.17 |
| ATOM | 1452 | CE1 | PHE | 195 | 18.053 | 26.136 | 71.663 | 1.00 | 95.14 |
| ATOM | 1453 | CE2 | PHE | 195 | 16.491 | 26.145 | 73.492 | 1.00 | 95.12 |
| ATOM | 1454 | CZ | PHE | 195 | 17.016 | 25.528 | 72.364 | 1.00 | 94.97 |
| ATOM | 1455 | C | PHE | 195 | 18.508 | 30.393 | 71.430 | 1.00 | 92.64 |
| ATOM | 1456 | O | PHE | 195 | 17.667 | 30.131 | 70.569 | 1.00 | 92.22 |
| ATOM | 1457 | N | GLU | 196 | 19.793 | 30.583 | 71.148 | 1.00 | 90.93 |
| ATOM | 1458 | CA | GLU | 196 | 20.292 | 30.486 | 69.779 | 1.00 | 89.04 |
| ATOM | 1459 | CB | GLU | 196 | 20.249 | 31.861 | 69.102 | 1.00 | 89.55 |
| ATOM | 1460 | CG | GLU | 196 | 18.846 | 32.395 | 68.832 | 1.00 | 90.25 |
| ATOM | 1461 | CD | GLU | 196 | 18.859 | 33.771 | 68.187 | 1.00 | 90.61 |

| | | | | | | | | | | |
|---|---|---|---|---|---|---|---|---|---|---|
| ATOM | 1462 | OE1 | GLU | 196 | 19.342 | 34.728 | 68.830 | 1.00 | 90.30 |
| ATOM | 1463 | OE2 | GLU | 196 | 18.390 | 33.895 | 67.035 | 1.00 | 90.79 |
| ATOM | 1464 | C | GLU | 196 | 21.711 | 29.923 | 69.694 | 1.00 | 87.11 |
| ATOM | 1465 | O | GLU | 196 | 22.681 | 30.589 | 70.066 | 1.00 | 86.83 |
| ATOM | 1466 | N | MET | 197 | 21.824 | 28.692 | 69.201 | 1.00 | 84.23 |
| ATOM | 1467 | CA | MET | 197 | 23.121 | 28.043 | 69.045 | 1.00 | 80.79 |
| ATOM | 1468 | CB | MET | 197 | 23.067 | 26.586 | 69.524 | 1.00 | 81.16 |
| ATOM | 1469 | CG | MET | 197 | 22.633 | 26.389 | 70.967 | 1.00 | 80.14 |
| ATOM | 1470 | SD | MET | 197 | 23.597 | 27.356 | 72.135 | 1.00 | 81.93 |
| ATOM | 1471 | CE | MET | 197 | 25.195 | 26.640 | 71.968 | 1.00 | 81.72 |
| ATOM | 1472 | C | MET | 197 | 23.502 | 28.070 | 67.568 | 1.00 | 77.74 |
| ATOM | 1473 | O | MET | 197 | 22.695 | 28.436 | 66.716 | 1.00 | 76.30 |
| ATOM | 1474 | N | ASP | 198 | 24.733 | 27.672 | 67.269 | 1.00 | 75.73 |
| ATOM | 1475 | CA | ASP | 198 | 25.214 | 27.652 | 65.894 | 1.00 | 72.50 |
| ATOM | 1476 | CB | ASP | 198 | 26.297 | 28.723 | 65.720 | 1.00 | 73.47 |
| ATOM | 1477 | CG | ASP | 198 | 26.573 | 29.046 | 64.265 | 1.00 | 75.34 |
| ATOM | 1478 | OD1 | ASP | 198 | 27.407 | 29.941 | 64.005 | 1.00 | 75.72 |
| ATOM | 1479 | OD2 | ASP | 198 | 25.954 | 28.407 | 63.384 | 1.00 | 76.53 |
| ATOM | 1480 | C | ASP | 198 | 25.769 | 26.265 | 65.544 | 1.00 | 69.75 |
| ATOM | 1481 | O | ASP | 198 | 26.962 | 26.005 | 65.703 | 1.00 | 69.48 |
| ATOM | 1482 | N | VAL | 199 | 24.892 | 25.383 | 65.068 | 1.00 | 66.43 |
| ATOM | 1483 | CA | VAL | 199 | 25.266 | 24.018 | 64.697 | 1.00 | 62.47 |
| ATOM | 1484 | CB | VAL | 199 | 24.055 | 23.266 | 64.113 | 1.00 | 62.19 |
| ATOM | 1485 | CG1 | VAL | 199 | 24.426 | 21.823 | 63.827 | 1.00 | 61.88 |
| ATOM | 1486 | CG2 | VAL | 199 | 22.886 | 23.340 | 65.083 | 1.00 | 61.21 |
| ATOM | 1487 | C | VAL | 199 | 26.409 | 23.986 | 63.682 | 1.00 | 60.44 |
| ATOM | 1488 | O | VAL | 199 | 26.192 | 24.135 | 62.479 | 1.00 | 59.37 |
| ATOM | 1489 | N | VAL | 200 | 27.624 | 23.774 | 64.180 | 1.00 | 58.00 |
| ATOM | 1490 | CA | VAL | 200 | 28.820 | 23.741 | 63.341 | 1.00 | 56.24 |
| ATOM | 1491 | CB | VAL | 200 | 30.048 | 24.278 | 64.128 | 1.00 | 57.42 |
| ATOM | 1492 | CG1 | VAL | 200 | 31.326 | 24.071 | 63.331 | 1.00 | 57.94 |
| ATOM | 1493 | CG2 | VAL | 200 | 29.859 | 25.761 | 64.433 | 1.00 | 59.29 |
| ATOM | 1494 | C | VAL | 200 | 29.159 | 22.357 | 62.785 | 1.00 | 54.53 |
| ATOM | 1495 | O | VAL | 200 | 29.759 | 22.242 | 61.715 | 1.00 | 54.29 |
| ATOM | 1496 | N | ALA | 201 | 28.779 | 21.306 | 63.503 | 1.00 | 52.88 |

| | | | | | | | | | | |
|---|---|---|---|---|---|---|---|---|---|---|
| ATOM | 1497 | CA | ALA | 201 | 29.085 | 19.953 | 63.048 | 1.00 | 49.83 |
| ATOM | 1498 | CB | ALA | 201 | 30.541 | 19.627 | 63.349 | 1.00 | 47.49 |
| ATOM | 1499 | C | ALA | 201 | 28.196 | 18.887 | 63.654 | 1.00 | 46.62 |
| ATOM | 1500 | O | ALA | 201 | 27.803 | 18.973 | 64.810 | 1.00 | 47.96 |
| ATOM | 1501 | N | MET | 202 | 27.873 | 17.883 | 62.851 | 1.00 | 44.82 |
| ATOM | 1502 | CA | MET | 202 | 27.065 | 16.762 | 63.309 | 1.00 | 42.12 |
| ATOM | 1503 | CB | MET | 202 | 25.731 | 16.695 | 62.567 | 1.00 | 39.23 |
| ATOM | 1504 | CG | MET | 202 | 24.886 | 15.520 | 63.014 | 1.00 | 38.13 |
| ATOM | 1505 | SD | MET | 202 | 23.425 | 15.193 | 62.026 | 1.00 | 40.98 |
| ATOM | 1506 | CE | MET | 202 | 24.134 | 15.162 | 60.401 | 1.00 | 37.63 |
| ATOM | 1507 | C | MET | 202 | 27.865 | 15.489 | 63.027 | 1.00 | 40.68 |
| ATOM | 1508 | O | MET | 202 | 28.274 | 15.251 | 61.888 | 1.00 | 38.74 |
| ATOM | 1509 | N | VAL | 203 | 28.092 | 14.679 | 64.060 | 1.00 | 39.90 |
| ATOM | 1510 | CA | VAL | 203 | 28.851 | 13.438 | 63.901 | 1.00 | 37.47 |
| ATOM | 1511 | CB | VAL | 203 | 30.264 | 13.549 | 64.517 | 1.00 | 36.73 |
| ATOM | 1512 | CG1 | VAL | 203 | 31.078 | 14.615 | 63.796 | 1.00 | 34.96 |
| ATOM | 1513 | CG2 | VAL | 203 | 30.155 | 13.852 | 65.996 | 1.00 | 37.90 |
| ATOM | 1514 | C | VAL | 203 | 28.190 | 12.199 | 64.505 | 1.00 | 37.09 |
| ATOM | 1515 | O | VAL | 203 | 27.250 | 12.284 | 65.309 | 1.00 | 36.61 |
| ATOM | 1516 | N | ASN | 204 | 28.707 | 11.039 | 64.101 | 1.00 | 36.09 |
| ATOM | 1517 | CA | ASN | 204 | 28.228 | 9.749 | 64.584 | 1.00 | 31.60 |
| ATOM | 1518 | CB | ASN | 204 | 28.461 | 8.695 | 63.497 | 1.00 | 32.07 |
| ATOM | 1519 | CG | ASN | 204 | 27.949 | 7.322 | 63.888 | 1.00 | 31.63 |
| ATOM | 1520 | OD1 | ASN | 204 | 28.729 | 6.443 | 64.250 | 1.00 | 30.91 |
| ATOM | 1521 | ND2 | ASN | 204 | 26.634 | 7.135 | 63.824 | 1.00 | 28.99 |
| ATOM | 1522 | C | ASN | 204 | 29.027 | 9.454 | 65.853 | 1.00 | 28.81 |
| ATOM | 1523 | O | ASN | 204 | 30.122 | 9.990 | 66.019 | 1.00 | 30.24 |
| ATOM | 1524 | N | ASP | 205 | 28.498 | 8.639 | 66.765 | 1.00 | 27.90 |
| ATOM | 1525 | CA | ASP | 205 | 29.240 | 8.361 | 67.995 | 1.00 | 26.70 |
| ATOM | 1526 | CB | ASP | 205 | 28.369 | 7.627 | 69.028 | 1.00 | 27.65 |
| ATOM | 1527 | CG | ASP | 205 | 27.642 | 6.438 | 68.455 | 1.00 | 30.26 |
| ATOM | 1528 | OD1 | ASP | 205 | 27.079 | 5.655 | 69.256 | 1.00 | 28.60 |
| ATOM | 1529 | OD2 | ASP | 205 | 27.623 | 6.289 | 67.213 | 1.00 | 31.99 |
| ATOM | 1530 | C | ASP | 205 | 30.573 | 7.630 | 67.791 | 1.00 | 26.87 |
| ATOM | 1531 | O | ASP | 205 | 31.498 | 7.810 | 68.581 | 1.00 | 27.79 |

| | | | | | | | | | |
|---|---|---|---|---|---|---|---|---|---|
| ATOM | 1532 | N | THR | 206 | 30.686 | 6.816 | 66.740 | 1.00 | 24.79 |
| ATOM | 1533 | CA | THR | 206 | 31.951 | 6.146 | 66.476 | 1.00 | 24.03 |
| ATOM | 1534 | CB | THR | 206 | 31.886 | 5.236 | 65.206 | 1.00 | 25.43 |
| ATOM | 1535 | OG1 | THR | 206 | 31.401 | 5.999 | 64.089 | 1.00 | 26.30 |
| ATOM | 1536 | CG2 | THR | 206 | 30.976 | 4.032 | 65.444 | 1.00 | 16.83 |
| ATOM | 1537 | C | THR | 206 | 32.970 | 7.258 | 66.220 | 1.00 | 24.75 |
| ATOM | 1538 | O | THR | 206 | 34.025 | 7.326 | 66.858 | 1.00 | 25.65 |
| ATOM | 1539 | N | VAL | 207 | 32.632 | 8.136 | 65.285 | 1.00 | 22.49 |
| ATOM | 1540 | CA | VAL | 207 | 33.487 | 9.257 | 64.917 | 1.00 | 23.51 |
| ATOM | 1541 | CB | VAL | 207 | 32.775 | 10.133 | 63.855 | 1.00 | 25.35 |
| ATOM | 1542 | CG1 | VAL | 207 | 33.617 | 11.362 | 63.521 | 1.00 | 24.61 |
| ATOM | 1543 | CG2 | VAL | 207 | 32.509 | 9.299 | 62.609 | 1.00 | 21.66 |
| ATOM | 1544 | C | VAL | 207 | 33.897 | 10.119 | 66.126 | 1.00 | 23.48 |
| ATOM | 1545 | O | VAL | 207 | 35.061 | 10.470 | 66.279 | 1.00 | 26.51 |
| ATOM | 1546 | N | ALA | 208 | 32.948 | 10.452 | 66.989 | 1.00 | 24.53 |
| ATOM | 1547 | CA | ALA | 208 | 33.262 | 11.251 | 68.169 | 1.00 | 26.32 |
| ATOM | 1548 | CB | ALA | 208 | 31.980 | 11.533 | 68.958 | 1.00 | 27.56 |
| ATOM | 1549 | C | ALA | 208 | 34.287 | 10.530 | 69.055 | 1.00 | 28.84 |
| ATOM | 1550 | O | ALA | 208 | 35.247 | 11.138 | 69.549 | 1.00 | 27.69 |
| ATOM | 1551 | N | THR | 209 | 34.084 | 9.228 | 69.258 | 1.00 | 28.76 |
| ATOM | 1552 | CA | THR | 209 | 35.006 | 8.447 | 70.075 | 1.00 | 28.08 |
| ATOM | 1553 | CB | THR | 209 | 34.474 | 7.001 | 70.271 | 1.00 | 31.76 |
| ATOM | 1554 | OG1 | THR | 209 | 33.373 | 7.027 | 71.181 | 1.00 | 33.12 |
| ATOM | 1555 | CG2 | THR | 209 | 35.550 | 6.080 | 70.818 | 1.00 | 30.03 |
| ATOM | 1556 | C | THR | 209 | 36.382 | 8.414 | 69.418 | 1.00 | 26.73 |
| ATOM | 1557 | O | THR | 209 | 37.399 | 8.611 | 70.078 | 1.00 | 28.00 |
| ATOM | 1558 | N | MET | 210 | 36.421 | 8.191 | 68.110 | 1.00 | 28.44 |
| ATOM | 1559 | CA | MET | 210 | 37.703 | 8.143 | 67.419 | 1.00 | 28.08 |
| ATOM | 1560 | CB | MET | 210 | 37.516 | 7.851 | 65.932 | 1.00 | 26.94 |
| ATOM | 1561 | CG | MET | 210 | 38.842 | 7.766 | 65.168 | 1.00 | 28.59 |
| ATOM | 1562 | SD | MET | 210 | 38.643 | 7.734 | 63.374 | 1.00 | 32.14 |
| ATOM | 1563 | CE | MET | 210 | 38.216 | 9.518 | 63.083 | 1.00 | 33.30 |
| ATOM | 1564 | C | MET | 210 | 38.467 | 9.452 | 67.578 | 1.00 | 29.43 |
| ATOM | 1565 | O | MET | 210 | 39.636 | 9.459 | 67.972 | 1.00 | 30.57 |
| ATOM | 1566 | N | ILE | 211 | 37.799 | 10.561 | 67.281 | 1.00 | 31.16 |

| | | | | | | | | | |
|---|---|---|---|---|---|---|---|---|---|
| ATOM | 1567 | CA | ILE | 211 | 38.433 | 11.873 | 67.376 | 1.00 | 30.57 |
| ATOM | 1568 | CB | ILE | 211 | 37.418 | 13.012 | 67.019 | 1.00 | 29.75 |
| ATOM | 1569 | CG2 | ILE | 211 | 38.086 | 14.390 | 67.177 | 1.00 | 28.08 |
| ATOM | 1570 | CG1 | ILE | 211 | 36.928 | 12.837 | 65.578 | 1.00 | 22.83 |
| ATOM | 1571 | CD1 | ILE | 211 | 38.021 | 12.979 | 64.553 | 1.00 | 26.28 |
| ATOM | 1572 | C | ILE | 211 | 39.014 | 12.128 | 68.762 | 1.00 | 30.30 |
| ATOM | 1573 | O | ILE | 211 | 40.185 | 12.489 | 68.897 | 1.00 | 31.89 |
| ATOM | 1574 | N | SER | 212 | 38.203 | 11.914 | 69.792 | 1.00 | 32.78 |
| ATOM | 1575 | CA | SER | 212 | 38.639 | 12.146 | 71.164 | 1.00 | 35.84 |
| ATOM | 1576 | CB | SER | 212 | 37.499 | 11.852 | 72.140 | 1.00 | 35.91 |
| ATOM | 1577 | OG | SER | 212 | 37.317 | 10.455 | 72.307 | 1.00 | 41.55 |
| ATOM | 1578 | C | SER | 212 | 39.864 | 11.334 | 71.566 | 1.00 | 37.74 |
| ATOM | 1579 | O | SER | 212 | 40.684 | 11.803 | 72.354 | 1.00 | 41.44 |
| ATOM | 1580 | N | CYS | 213 | 39.990 | 10.121 | 71.040 | 1.00 | 38.07 |
| ATOM | 1581 | CA | CYS | 213 | 41.132 | 9.273 | 71.374 | 1.00 | 39.83 |
| ATOM | 1582 | CB | CYS | 213 | 40.802 | 7.799 | 71.108 | 1.00 | 38.31 |
| ATOM | 1583 | SG | CYS | 213 | 39.513 | 7.129 | 72.185 | 1.00 | 38.48 |
| ATOM | 1584 | C | CYS | 213 | 42.372 | 9.666 | 70.582 | 1.00 | 41.86 |
| ATOM | 1585 | O | CYS | 213 | 43.503 | 9.426 | 71.012 | 1.00 | 38.47 |
| ATOM | 1586 | N | TYR | 214 | 42.149 | 10.261 | 69.413 | 1.00 | 45.32 |
| ATOM | 1587 | CA | TYR | 214 | 43.243 | 10.701 | 68.554 | 1.00 | 45.02 |
| ATOM | 1588 | CB | TYR | 214 | 42.705 | 11.506 | 67.370 | 1.00 | 45.88 |
| ATOM | 1589 | CG | TYR | 214 | 43.798 | 12.171 | 66.573 | 1.00 | 45.72 |
| ATOM | 1590 | CD1 | TYR | 214 | 44.509 | 11.465 | 65.608 | 1.00 | 46.39 |
| ATOM | 1591 | CE1 | TYR | 214 | 45.556 | 12.061 | 64.913 | 1.00 | 47.16 |
| ATOM | 1592 | CD2 | TYR | 214 | 44.160 | 13.498 | 66.823 | 1.00 | 44.53 |
| ATOM | 1593 | CE2 | TYR | 214 | 45.203 | 14.099 | 66.134 | 1.00 | 45.20 |
| ATOM | 1594 | CZ | TYR | 214 | 45.896 | 13.375 | 65.183 | 1.00 | 46.22 |
| ATOM | 1595 | OH | TYR | 214 | 46.942 | 13.955 | 64.510 | 1.00 | 50.31 |
| ATOM | 1596 | C | TYR | 214 | 44.226 | 11.573 | 69.322 | 1.00 | 44.69 |
| ATOM | 1597 | O | TYR | 214 | 45.420 | 11.296 | 69.363 | 1.00 | 44.40 |
| ATOM | 1598 | N | TYR | 215 | 43.713 | 12.635 | 69.924 | 1.00 | 45.92 |
| ATOM | 1599 | CA | TYR | 215 | 44.556 | 13.552 | 70.667 | 1.00 | 48.38 |
| ATOM | 1600 | CB | TYR | 215 | 43.713 | 14.716 | 71.175 | 1.00 | 51.93 |
| ATOM | 1601 | CG | TYR | 215 | 43.192 | 15.545 | 70.021 | 1.00 | 57.70 |

| | | | | | | | | | |
|---|---|---|---|---|---|---|---|---|---|
| ATOM | 1602 | CD1 | TYR | 215 | 41.918 | 15.330 | 69.484 | 1.00 | 58.41 |
| ATOM | 1603 | CE1 | TYR | 215 | 41.478 | 16.047 | 68.363 | 1.00 | 61.27 |
| ATOM | 1604 | CD2 | TYR | 215 | 44.011 | 16.498 | 69.413 | 1.00 | 59.13 |
| ATOM | 1605 | CE2 | TYR | 215 | 43.586 | 17.214 | 68.300 | 1.00 | 61.22 |
| ATOM | 1606 | CZ | TYR | 215 | 42.325 | 16.991 | 67.780 | 1.00 | 62.20 |
| ATOM | 1607 | OH | TYR | 215 | 41.928 | 17.728 | 66.688 | 1.00 | 61.67 |
| ATOM | 1608 | C | TYR | 215 | 45.304 | 12.871 | 71.792 | 1.00 | 48.87 |
| ATOM | 1609 | O | TYR | 215 | 46.282 | 13.407 | 72.318 | 1.00 | 49.38 |
| ATOM | 1610 | N | GLU | 216 | 44.852 | 11.672 | 72.142 | 1.00 | 47.69 |
| ATOM | 1611 | CA | GLU | 216 | 45.496 | 10.889 | 73.181 | 1.00 | 47.03 |
| ATOM | 1612 | CB | GLU | 216 | 44.474 | 9.979 | 73.863 | 1.00 | 49.83 |
| ATOM | 1613 | CG | GLU | 216 | 44.837 | 9.550 | 75.270 | 1.00 | 55.37 |
| ATOM | 1614 | CD | GLU | 216 | 44.998 | 10.735 | 76.208 | 1.00 | 59.31 |
| ATOM | 1615 | OE1 | GLU | 216 | 44.285 | 11.747 | 76.012 | 1.00 | 59.95 |
| ATOM | 1616 | OE2 | GLU | 216 | 45.824 | 10.649 | 77.146 | 1.00 | 60.13 |
| ATOM | 1617 | C | GLU | 216 | 46.552 | 10.044 | 72.477 | 1.00 | 45.45 |
| ATOM | 1618 | O | GLU | 216 | 47.673 | 9.905 | 72.958 | 1.00 | 45.05 |
| ATOM | 1619 | N | ASP | 217 | 46.183 | 9.495 | 71.321 | 1.00 | 43.73 |
| ATOM | 1620 | CA | ASP | 217 | 47.074 | 8.643 | 70.530 | 1.00 | 41.33 |
| ATOM | 1621 | CB | ASP | 217 | 46.776 | 7.171 | 70.845 | 1.00 | 40.13 |
| ATOM | 1622 | CG | ASP | 217 | 47.780 | 6.208 | 70.226 | 1.00 | 39.76 |
| ATOM | 1623 | OD1 | ASP | 217 | 48.461 | 6.571 | 69.249 | 1.00 | 40.95 |
| ATOM | 1624 | OD2 | ASP | 217 | 47.876 | 5.062 | 70.712 | 1.00 | 42.61 |
| ATOM | 1625 | C | ASP | 217 | 46.852 | 8.921 | 69.033 | 1.00 | 40.53 |
| ATOM | 1626 | O | ASP | 217 | 45.862 | 8.474 | 68.443 | 1.00 | 37.20 |
| ATOM | 1627 | N | HIS | 218 | 47.779 | 9.657 | 68.427 | 1.00 | 41.94 |
| ATOM | 1628 | CA | HIS | 218 | 47.689 | 10.008 | 67.007 | 1.00 | 44.23 |
| ATOM | 1629 | CB | HIS | 218 | 48.912 | 10.828 | 66.603 | 1.00 | 47.00 |
| ATOM | 1630 | CG | HIS | 218 | 48.860 | 12.244 | 67.079 | 1.00 | 51.95 |
| ATOM | 1631 | CD2 | HIS | 218 | 49.230 | 13.402 | 66.483 | 1.00 | 54.47 |
| ATOM | 1632 | ND1 | HIS | 218 | 48.371 | 12.592 | 68.320 | 1.00 | 54.33 |
| ATOM | 1633 | CE1 | HIS | 218 | 48.439 | 13.903 | 68.467 | 1.00 | 55.83 |
| ATOM | 1634 | NE2 | HIS | 218 | 48.957 | 14.419 | 67.367 | 1.00 | 55.95 |
| ATOM | 1635 | C | HIS | 218 | 47.528 | 8.810 | 66.074 | 1.00 | 42.66 |
| ATOM | 1636 | O | HIS | 218 | 47.157 | 8.963 | 64.909 | 1.00 | 42.00 |

| | | | | | | | | | |
|---|---|---|---|---|---|---|---|---|---|
| ATOM | 1637 | N | GLN | 219 | 47.793 | 7.620 | 66.597 | 1.00 | 41.40 |
| ATOM | 1638 | CA | GLN | 219 | 47.667 | 6.394 | 65.820 | 1.00 | 41.15 |
| ATOM | 1639 | CB | GLN | 219 | 48.592 | 5.321 | 66.397 | 1.00 | 45.16 |
| ATOM | 1640 | CG | GLN | 219 | 50.070 | 5.611 | 66.214 | 1.00 | 49.72 |
| ATOM | 1641 | CD | GLN | 219 | 50.566 | 5.230 | 64.832 | 1.00 | 55.92 |
| ATOM | 1642 | OE1 | GLN | 219 | 49.997 | 5.646 | 63.813 | 1.00 | 57.28 |
| ATOM | 1643 | NE2 | GLN | 219 | 51.636 | 4.429 | 64.787 | 1.00 | 57.32 |
| ATOM | 1644 | C | GLN | 219 | 46.228 | 5.869 | 65.792 | 1.00 | 37.41 |
| ATOM | 1645 | O | GLN | 219 | 45.927 | 4.904 | 65.091 | 1.00 | 37.06 |
| ATOM | 1646 | N | CYS | 220 | 45.342 | 6.488 | 66.562 | 1.00 | 34.18 |
| ATOM | 1647 | CA | CYS | 220 | 43.955 | 6.038 | 66.578 | 1.00 | 32.52 |
| ATOM | 1648 | CB | CYS | 220 | 43.199 | 6.597 | 67.783 | 1.00 | 28.93 |
| ATOM | 1649 | SG | CYS | 220 | 41.420 | 6.288 | 67.739 | 1.00 | 31.90 |
| ATOM | 1650 | C | CYS | 220 | 43.272 | 6.474 | 65.303 | 1.00 | 32.01 |
| ATOM | 1651 | O | CYS | 220 | 43.010 | 7.664 | 65.096 | 1.00 | 32.91 |
| ATOM | 1652 | N | GLU | 221 | 42.993 | 5.505 | 64.442 | 1.00 | 29.12 |
| ATOM | 1653 | CA | GLU | 221 | 42.343 | 5.785 | 63.176 | 1.00 | 28.98 |
| ATOM | 1654 | CB | GLU | 221 | 43.273 | 5.437 | 62.009 | 1.00 | 30.00 |
| ATOM | 1655 | CG | GLU | 221 | 44.481 | 6.366 | 61.853 | 1.00 | 35.29 |
| ATOM | 1656 | CD | GLU | 221 | 45.190 | 6.166 | 60.515 | 1.00 | 36.83 |
| ATOM | 1657 | OE1 | GLU | 221 | 44.490 | 6.007 | 59.498 | 1.00 | 38.09 |
| ATOM | 1658 | OE2 | GLU | 221 | 46.436 | 6.176 | 60.465 | 1.00 | 40.80 |
| ATOM | 1659 | C | GLU | 221 | 41.057 | 4.991 | 63.059 | 1.00 | 25.46 |
| ATOM | 1660 | O | GLU | 221 | 40.513 | 4.835 | 61.970 | 1.00 | 22.65 |
| ATOM | 1661 | N | VAL | 222 | 40.569 | 4.491 | 64.185 | 1.00 | 25.43 |
| ATOM | 1662 | CA | VAL | 222 | 39.337 | 3.703 | 64.179 | 1.00 | 25.45 |
| ATOM | 1663 | CB | VAL | 222 | 39.625 | 2.172 | 64.189 | 1.00 | 24.36 |
| ATOM | 1664 | CG1 | VAL | 222 | 38.318 | 1.391 | 64.122 | 1.00 | 21.56 |
| ATOM | 1665 | CG2 | VAL | 222 | 40.533 | 1.795 | 63.029 | 1.00 | 21.70 |
| ATOM | 1666 | C | VAL | 222 | 38.527 | 4.016 | 65.414 | 1.00 | 25.44 |
| ATOM | 1667 | O | VAL | 222 | 39.076 | 4.192 | 66.492 | 1.00 | 25.99 |
| ATOM | 1668 | N | GLY | 223 | 37.217 | 4.090 | 65.240 | 1.00 | 25.97 |
| ATOM | 1669 | CA | GLY | 223 | 36.328 | 4.347 | 66.349 | 1.00 | 25.83 |
| ATOM | 1670 | C | GLY | 223 | 35.337 | 3.201 | 66.340 | 1.00 | 25.37 |
| ATOM | 1671 | O | GLY | 223 | 34.852 | 2.812 | 65.273 | 1.00 | 25.38 |

| | | | | | | | | | |
|---|---|---|---|---|---|---|---|---|---|
| ATOM | 1672 | N | MET | 224 | 35.044 | 2.647 | 67.511 | 1.00 | 24.88 |
| ATOM | 1673 | CA | MET | 224 | 34.114 | 1.527 | 67.587 | 1.00 | 25.47 |
| ATOM | 1674 | CB | MET | 224 | 34.881 | 0.187 | 67.638 | 1.00 | 22.66 |
| ATOM | 1675 | CG | MET | 224 | 33.956 | -1.041 | 67.634 | 1.00 | 25.14 |
| ATOM | 1676 | SD | MET | 224 | 34.806 | -2.680 | 67.748 | 1.00 | 22.18 |
| ATOM | 1677 | CE | MET | 224 | 35.380 | -2.594 | 69.396 | 1.00 | 16.01 |
| ATOM | 1678 | C | MET | 224 | 33.177 | 1.618 | 68.780 | 1.00 | 22.20 |
| ATOM | 1679 | O | MET | 224 | 33.577 | 1.978 | 69.881 | 1.00 | 22.65 |
| ATOM | 1680 | N | ILE | 225 | 31.915 | 1.295 | 68.543 | 1.00 | 21.12 |
| ATOM | 1681 | CA | ILE | 225 | 30.936 | 1.314 | 69.604 | 1.00 | 21.34 |
| ATOM | 1682 | CB | ILE | 225 | 29.757 | 2.295 | 69.293 | 1.00 | 25.85 |
| ATOM | 1683 | CG2 | ILE | 225 | 28.739 | 2.268 | 70.446 | 1.00 | 25.47 |
| ATOM | 1684 | CG1 | ILE | 225 | 30.273 | 3.734 | 69.107 | 1.00 | 25.08 |
| ATOM | 1685 | CD1 | ILE | 225 | 30.838 | 4.355 | 70.382 | 1.00 | 22.09 |
| ATOM | 1686 | C | ILE | 225 | 30.321 | -0.080 | 69.789 | 1.00 | 22.30 |
| ATOM | 1687 | O | ILE | 225 | 29.885 | -0.712 | 68.826 | 1.00 | 24.03 |
| ATOM | 1688 | N | VAL | 226 | 30.313 | -0.563 | 71.025 | 1.00 | 22.67 |
| ATOM | 1689 | CA | VAL | 226 | 29.645 | -1.817 | 71.341 | 1.00 | 21.60 |
| ATOM | 1690 | CB | VAL | 226 | 30.618 | -2.993 | 71.634 | 1.00 | 21.77 |
| ATOM | 1691 | CG1 | VAL | 226 | 29.821 | -4.291 | 71.718 | 1.00 | 21.54 |
| ATOM | 1692 | CG2 | VAL | 226 | 31.663 | -3.113 | 70.541 | 1.00 | 17.23 |
| ATOM | 1693 | C | VAL | 226 | 28.838 | -1.493 | 72.604 | 1.00 | 21.49 |
| ATOM | 1694 | O | VAL | 226 | 29.316 | -1.633 | 73.723 | 1.00 | 18.90 |
| ATOM | 1695 | N | GLY | 227 | 27.615 | -1.016 | 72.402 | 1.00 | 25.39 |
| ATOM | 1696 | CA | GLY | 227 | 26.744 | -0.675 | 73.518 | 1.00 | 26.76 |
| ATOM | 1697 | C | GLY | 227 | 25.353 | -1.140 | 73.150 | 1.00 | 28.03 |
| ATOM | 1698 | O | GLY | 227 | 25.155 | -2.315 | 72.846 | 1.00 | 29.80 |
| ATOM | 1699 | N | THR | 228 | 24.384 | -0.235 | 73.161 | 1.00 | 27.62 |
| ATOM | 1700 | CA | THR | 228 | 23.031 | -0.607 | 72.788 | 1.00 | 27.59 |
| ATOM | 1701 | CB | THR | 228 | 22.083 | 0.601 | 72.911 | 1.00 | 29.15 |
| ATOM | 1702 | OG1 | THR | 228 | 21.937 | 0.932 | 74.294 | 1.00 | 32.52 |
| ATOM | 1703 | CG2 | THR | 228 | 20.719 | 0.291 | 72.339 | 1.00 | 28.08 |
| ATOM | 1704 | C | THR | 228 | 23.094 | -1.080 | 71.345 | 1.00 | 26.98 |
| ATOM | 1705 | O | THR | 228 | 22.460 | -2.065 | 70.960 | 1.00 | 27.95 |
| ATOM | 1706 | N | GLY | 229 | 23.890 | -0.374 | 70.554 | 1.00 | 26.02 |

| | | | | | | | | | |
|---|---|---|---|---|---|---|---|---|---|
| ATOM | 1707 | CA | GLY | 229 | 24.050 | -0.718 | 69.154 | 1.00 | 25.33 |
| ATOM | 1708 | C | GLY | 229 | 25.503 | -1.055 | 68.911 | 1.00 | 24.09 |
| ATOM | 1709 | O | GLY | 229 | 26.312 | -1.004 | 69.838 | 1.00 | 23.25 |
| ATOM | 1710 | N | CYS | 230 | 25.850 | -1.395 | 67.677 | 1.00 | 24.12 |
| ATOM | 1711 | CA | CYS | 230 | 27.235 | -1.750 | 67.376 | 1.00 | 23.83 |
| ATOM | 1712 | CB | CYS | 230 | 27.395 | -3.280 | 67.425 | 1.00 | 20.39 |
| ATOM | 1713 | SG | CYS | 230 | 29.076 | -3.879 | 67.182 | 1.00 | 25.34 |
| ATOM | 1714 | C | CYS | 230 | 27.627 | -1.204 | 66.010 | 1.00 | 20.45 |
| ATOM | 1715 | O | CYS | 230 | 26.919 | -1.406 | 65.035 | 1.00 | 20.28 |
| ATOM | 1716 | N | ASN | 231 | 28.763 | -0.526 | 65.935 | 1.00 | 23.86 |
| ATOM | 1717 | CA | ASN | 231 | 29.196 | 0.076 | 64.669 | 1.00 | 24.35 |
| ATOM | 1718 | CB | ASN | 231 | 28.267 | 1.261 | 64.355 | 1.00 | 25.51 |
| ATOM | 1719 | CG | ASN | 231 | 28.598 | 1.962 | 63.042 | 1.00 | 27.76 |
| ATOM | 1720 | OD1 | ASN | 231 | 28.930 | 1.331 | 62.039 | 1.00 | 24.60 |
| ATOM | 1721 | ND2 | ASN | 231 | 28.472 | 3.288 | 63.043 | 1.00 | 30.91 |
| ATOM | 1722 | C | ASN | 231 | 30.640 | 0.553 | 64.784 | 1.00 | 23.81 |
| ATOM | 1723 | O | ASN | 231 | 31.184 | 0.624 | 65.885 | 1.00 | 23.94 |
| ATOM | 1724 | N | ALA | 232 | 31.249 | 0.885 | 63.651 | 1.00 | 22.70 |
| ATOM | 1725 | CA | ALA | 232 | 32.626 | 1.359 | 63.636 | 1.00 | 25.15 |
| ATOM | 1726 | CB | ALA | 232 | 33.580 | 0.169 | 63.463 | 1.00 | 24.36 |
| ATOM | 1727 | C | ALA | 232 | 32.867 | 2.372 | 62.511 | 1.00 | 26.31 |
| ATOM | 1728 | O | ALA | 232 | 32.127 | 2.416 | 61.530 | 1.00 | 28.47 |
| ATOM | 1729 | N | CYS | 233 | 33.911 | 3.176 | 62.664 | 1.00 | 24.88 |
| ATOM | 1730 | CA | CYS | 233 | 34.291 | 4.160 | 61.653 | 1.00 | 26.51 |
| ATOM | 1731 | CB | CYS | 233 | 33.899 | 5.583 | 62.076 | 1.00 | 24.89 |
| ATOM | 1732 | SG | CYS | 233 | 34.875 | 6.224 | 63.436 | 1.00 | 25.76 |
| ATOM | 1733 | C | CYS | 233 | 35.805 | 4.055 | 61.555 | 1.00 | 25.08 |
| ATOM | 1734 | O | CYS | 233 | 36.450 | 3.564 | 62.480 | 1.00 | 25.19 |
| ATOM | 1735 | N | TYR | 234 | 36.373 | 4.505 | 60.442 | 1.00 | 25.32 |
| ATOM | 1736 | CA | TYR | 234 | 37.820 | 4.427 | 60.245 | 1.00 | 23.93 |
| ATOM | 1737 | CB | TYR | 234 | 38.200 | 3.020 | 59.760 | 1.00 | 20.70 |
| ATOM | 1738 | CG | TYR | 234 | 37.782 | 2.771 | 58.328 | 1.00 | 16.78 |
| ATOM | 1739 | CD1 | TYR | 234 | 38.712 | 2.786 | 57.302 | 1.00 | 18.75 |
| ATOM | 1740 | CE1 | TYR | 234 | 38.326 | 2.668 | 55.975 | 1.00 | 18.89 |
| ATOM | 1741 | CD2 | TYR | 234 | 36.443 | 2.622 | 57.990 | 1.00 | 19.60 |

| | | | | | | | | | |
|---|---|---|---|---|---|---|---|---|---|
| ATOM | 1742 | CE2 | TYR | 234 | 36.043 | 2.506 | 56.666 | 1.00 | 18.40 |
| ATOM | 1743 | CZ | TYR | 234 | 36.990 | 2.535 | 55.665 | 1.00 | 21.55 |
| ATOM | 1744 | OH | TYR | 234 | 36.603 | 2.479 | 54.346 | 1.00 | 23.25 |
| ATOM | 1745 | C | TYR | 234 | 38.254 | 5.452 | 59.194 | 1.00 | 26.41 |
| ATOM | 1746 | O | TYR | 234 | 37.436 | 5.929 | 58.404 | 1.00 | 27.14 |
| ATOM | 1747 | N | MET | 235 | 39.543 | 5.769 | 59.179 | 1.00 | 27.10 |
| ATOM | 1748 | CA | MET | 235 | 40.094 | 6.722 | 58.224 | 1.00 | 28.74 |
| ATOM | 1749 | CB | MET | 235 | 41.383 | 7.331 | 58.789 | 1.00 | 29.38 |
| ATOM | 1750 | CG | MET | 235 | 41.169 | 8.180 | 60.035 | 1.00 | 31.43 |
| ATOM | 1751 | SD | MET | 235 | 39.947 | 9.503 | 59.750 | 1.00 | 32.30 |
| ATOM | 1752 | CE | MET | 235 | 40.866 | 10.535 | 58.591 | 1.00 | 34.11 |
| ATOM | 1753 | C | MET | 235 | 40.374 | 6.066 | 56.869 | 1.00 | 29.42 |
| ATOM | 1754 | O | MET | 235 | 41.170 | 5.134 | 56.767 | 1.00 | 30.49 |
| ATOM | 1755 | N | GLU | 236 | 39.714 | 6.565 | 55.829 | 1.00 | 31.08 |
| ATOM | 1756 | CA | GLU | 236 | 39.867 | 6.040 | 54.476 | 1.00 | 31.04 |
| ATOM | 1757 | CB | GLU | 236 | 38.491 | 5.743 | 53.879 | 1.00 | 31.57 |
| ATOM | 1758 | CG | GLU | 236 | 38.536 | 5.161 | 52.474 | 1.00 | 32.18 |
| ATOM | 1759 | CD | GLU | 236 | 39.330 | 3.875 | 52.427 | 1.00 | 32.52 |
| ATOM | 1760 | OE1 | GLU | 236 | 40.565 | 3.952 | 52.273 | 1.00 | 34.34 |
| ATOM | 1761 | OE2 | GLU | 236 | 38.723 | 2.789 | 52.571 | 1.00 | 30.79 |
| ATOM | 1762 | C | GLU | 236 | 40.598 | 7.030 | 53.574 | 1.00 | 33.43 |
| ATOM | 1763 | O | GLU | 236 | 40.583 | 8.238 | 53.818 | 1.00 | 29.93 |
| ATOM | 1764 | N | GLU | 237 | 41.240 | 6.506 | 52.532 | 1.00 | 35.85 |
| ATOM | 1765 | CA | GLU | 237 | 41.969 | 7.333 | 51.575 | 1.00 | 37.83 |
| ATOM | 1766 | CB | GLU | 237 | 42.934 | 6.462 | 50.764 | 1.00 | 40.16 |
| ATOM | 1767 | CG | GLU | 237 | 43.684 | 5.426 | 51.602 | 1.00 | 43.86 |
| ATOM | 1768 | CD | GLU | 237 | 44.466 | 6.049 | 52.743 | 1.00 | 47.85 |
| ATOM | 1769 | OE1 | GLU | 237 | 44.806 | 5.322 | 53.704 | 1.00 | 51.02 |
| ATOM | 1770 | OE2 | GLU | 237 | 44.747 | 7.264 | 52.681 | 1.00 | 48.78 |
| ATOM | 1771 | C | GLU | 237 | 40.920 | 7.969 | 50.657 | 1.00 | 37.87 |
| ATOM | 1772 | O | GLU | 237 | 40.058 | 7.268 | 50.122 | 1.00 | 38.29 |
| ATOM | 1773 | N | MET | 238 | 40.987 | 9.287 | 50.477 | 1.00 | 37.42 |
| ATOM | 1774 | CA | MET | 238 | 40.009 | 9.987 | 49.644 | 1.00 | 37.50 |
| ATOM | 1775 | CB | MET | 238 | 40.375 | 11.467 | 49.501 | 1.00 | 38.62 |
| ATOM | 1776 | CG | MET | 238 | 39.772 | 12.355 | 50.587 | 1.00 | 40.32 |

| | | | | | | | | | |
|---|---|---|---|---|---|---|---|---|---|
| ATOM | 1777 | SD | MET | 238 | 37.956 | 12.144 | 50.764 | 1.00 | 42.83 |
| ATOM | 1778 | CE | MET | 238 | 37.308 | 13.116 | 49.410 | 1.00 | 44.06 |
| ATOM | 1779 | C | MET | 238 | 39.796 | 9.374 | 48.270 | 1.00 | 36.21 |
| ATOM | 1780 | O | MET | 238 | 38.685 | 9.413 | 47.740 | 1.00 | 33.93 |
| ATOM | 1781 | N | GLN | 239 | 40.848 | 8.803 | 47.690 | 1.00 | 35.50 |
| ATOM | 1782 | CA | GLN | 239 | 40.714 | 8.184 | 46.378 | 1.00 | 36.82 |
| ATOM | 1783 | CB | GLN | 239 | 42.078 | 7.732 | 45.846 | 1.00 | 39.35 |
| ATOM | 1784 | CG | GLN | 239 | 42.839 | 6.804 | 46.774 | 1.00 | 44.12 |
| ATOM | 1785 | CD | GLN | 239 | 43.900 | 7.534 | 47.584 | 1.00 | 49.18 |
| ATOM | 1786 | OE1 | GLN | 239 | 43.635 | 8.580 | 48.192 | 1.00 | 49.88 |
| ATOM | 1787 | NE2 | GLN | 239 | 45.111 | 6.981 | 47.600 | 1.00 | 49.95 |
| ATOM | 1788 | C | GLN | 239 | 39.762 | 6.986 | 46.395 | 1.00 | 35.72 |
| ATOM | 1789 | O | GLN | 239 | 39.276 | 6.568 | 45.348 | 1.00 | 37.20 |
| ATOM | 1790 | N | ASN | 240 | 39.503 | 6.419 | 47.570 | 1.00 | 34.56 |
| ATOM | 1791 | CA | ASN | 240 | 38.604 | 5.272 | 47.648 | 1.00 | 33.20 |
| ATOM | 1792 | CB | ASN | 240 | 39.118 | 4.239 | 48.658 | 1.00 | 33.68 |
| ATOM | 1793 | CG | ASN | 240 | 40.548 | 3.802 | 48.369 | 1.00 | 34.24 |
| ATOM | 1794 | OD1 | ASN | 240 | 40.963 | 3.710 | 47.210 | 1.00 | 33.87 |
| ATOM | 1795 | ND2 | ASN | 240 | 41.306 | 3.523 | 49.424 | 1.00 | 34.32 |
| ATOM | 1796 | C | ASN | 240 | 37.190 | 5.690 | 48.011 | 1.00 | 33.25 |
| ATOM | 1797 | O | ASN | 240 | 36.259 | 4.886 | 47.936 | 1.00 | 33.86 |
| ATOM | 1798 | N | VAL | 241 | 37.024 | 6.946 | 48.414 | 1.00 | 32.52 |
| ATOM | 1799 | CA | VAL | 241 | 35.702 | 7.441 | 48.753 | 1.00 | 31.62 |
| ATOM | 1800 | CB | VAL | 241 | 35.755 | 8.559 | 49.811 | 1.00 | 29.14 |
| ATOM | 1801 | CG1 | VAL | 241 | 34.339 | 8.948 | 50.204 | 1.00 | 31.00 |
| ATOM | 1802 | CG2 | VAL | 241 | 36.530 | 8.107 | 51.021 | 1.00 | 26.87 |
| ATOM | 1803 | C | VAL | 241 | 35.102 | 8.010 | 47.474 | 1.00 | 33.73 |
| ATOM | 1804 | O | VAL | 241 | 35.048 | 9.224 | 47.286 | 1.00 | 35.18 |
| ATOM | 1805 | N | GLU | 242 | 34.643 | 7.132 | 46.595 | 1.00 | 33.33 |
| ATOM | 1806 | CA | GLU | 242 | 34.075 | 7.572 | 45.324 | 1.00 | 33.69 |
| ATOM | 1807 | CB | GLU | 242 | 33.788 | 6.364 | 44.431 | 1.00 | 31.05 |
| ATOM | 1808 | CG | GLU | 242 | 34.983 | 5.457 | 44.222 | 1.00 | 33.00 |
| ATOM | 1809 | CD | GLU | 242 | 34.767 | 4.451 | 43.115 | 1.00 | 33.45 |
| ATOM | 1810 | OE1 | GLU | 242 | 33.595 | 4.162 | 42.776 | 1.00 | 33.74 |
| ATOM | 1811 | OE2 | GLU | 242 | 35.778 | 3.940 | 42.592 | 1.00 | 35.96 |

| | | | | | | | | | |
|---|---|---|---|---|---|---|---|---|---|
| ATOM | 1812 | C | GLU | 242 | 32.812 | 8.437 | 45.427 | 1.00 | 34.45 |
| ATOM | 1813 | O | GLU | 242 | 32.406 | 9.061 | 44.442 | 1.00 | 32.92 |
| ATOM | 1814 | N | LEU | 243 | 32.192 | 8.471 | 46.602 | 1.00 | 33.82 |
| ATOM | 1815 | CA | LEU | 243 | 30.982 | 9.262 | 46.799 | 1.00 | 36.13 |
| ATOM | 1816 | CB | LEU | 243 | 30.080 | 8.598 | 47.844 | 1.00 | 33.99 |
| ATOM | 1817 | CG | LEU | 243 | 29.168 | 7.490 | 47.297 | 1.00 | 37.04 |
| ATOM | 1818 | CD1 | LEU | 243 | 27.999 | 8.096 | 46.545 | 1.00 | 36.01 |
| ATOM | 1819 | CD2 | LEU | 243 | 29.969 | 6.560 | 46.384 | 1.00 | 36.49 |
| ATOM | 1820 | C | LEU | 243 | 31.290 | 10.700 | 47.199 | 1.00 | 35.69 |
| ATOM | 1821 | O | LEU | 243 | 30.406 | 11.458 | 47.585 | 1.00 | 37.51 |
| ATOM | 1822 | N | VAL | 244 | 32.560 | 11.062 | 47.117 | 1.00 | 37.53 |
| ATOM | 1823 | CA | VAL | 244 | 32.992 | 12.411 | 47.426 | 1.00 | 37.50 |
| ATOM | 1824 | CB | VAL | 244 | 33.537 | 12.547 | 48.861 | 1.00 | 36.75 |
| ATOM | 1825 | CG1 | VAL | 244 | 33.967 | 13.990 | 49.109 | 1.00 | 36.55 |
| ATOM | 1826 | CG2 | VAL | 244 | 32.465 | 12.160 | 49.870 | 1.00 | 37.02 |
| ATOM | 1827 | C | VAL | 244 | 34.099 | 12.727 | 46.446 | 1.00 | 39.75 |
| ATOM | 1828 | O | VAL | 244 | 35.090 | 12.003 | 46.361 | 1.00 | 39.55 |
| ATOM | 1829 | N | GLU | 245 | 33.909 | 13.802 | 45.688 | 1.00 | 42.16 |
| ATOM | 1830 | CA | GLU | 245 | 34.880 | 14.232 | 44.695 | 1.00 | 42.30 |
| ATOM | 1831 | CB | GLU | 245 | 34.372 | 15.487 | 43.989 | 1.00 | 45.34 |
| ATOM | 1832 | CG | GLU | 245 | 34.886 | 15.636 | 42.576 | 1.00 | 48.54 |
| ATOM | 1833 | CD | GLU | 245 | 34.377 | 16.893 | 41.901 | 1.00 | 50.12 |
| ATOM | 1834 | OE1 | GLU | 245 | 33.192 | 17.249 | 42.107 | 1.00 | 49.37 |
| ATOM | 1835 | OE2 | GLU | 245 | 35.164 | 17.511 | 41.152 | 1.00 | 52.40 |
| ATOM | 1836 | C | GLU | 245 | 36.203 | 14.532 | 45.378 | 1.00 | 41.00 |
| ATOM | 1837 | O | GLU | 245 | 36.230 | 15.132 | 46.446 | 1.00 | 42.20 |
| ATOM | 1838 | N | GLY | 246 | 37.297 | 14.107 | 44.761 | 1.00 | 41.28 |
| ATOM | 1839 | CA | GLY | 246 | 38.606 | 14.349 | 45.336 | 1.00 | 42.88 |
| ATOM | 1840 | C | GLY | 246 | 39.362 | 13.066 | 45.618 | 1.00 | 45.38 |
| ATOM | 1841 | O | GLY | 246 | 38.774 | 12.056 | 45.997 | 1.00 | 45.50 |
| ATOM | 1842 | N | ASP | 247 | 40.675 | 13.105 | 45.443 | 1.00 | 47.31 |
| ATOM | 1843 | CA | ASP | 247 | 41.509 | 11.940 | 45.687 | 1.00 | 49.13 |
| ATOM | 1844 | CB | ASP | 247 | 42.139 | 11.454 | 44.384 | 1.00 | 51.65 |
| ATOM | 1845 | CG | ASP | 247 | 41.131 | 10.836 | 43.449 | 1.00 | 56.09 |
| ATOM | 1846 | OD1 | ASP | 247 | 41.534 | 10.410 | 42.345 | 1.00 | 58.83 |

| | | | | | | | | | |
|---|---|---|---|---|---|---|---|---|---|
| ATOM | 1847 | OD2 | ASP | 247 | 39.936 | 10.770 | 43.819 | 1.00 | 59.44 |
| ATOM | 1848 | C | ASP | 247 | 42.611 | 12.274 | 46.667 | 1.00 | 49.51 |
| ATOM | 1849 | O | ASP | 247 | 43.406 | 11.415 | 47.039 | 1.00 | 49.57 |
| ATOM | 1850 | N | GLU | 248 | 42.661 | 13.531 | 47.086 | 1.00 | 50.49 |
| ATOM | 1851 | CA | GLU | 248 | 43.696 | 13.957 | 48.011 | 1.00 | 50.97 |
| ATOM | 1852 | CB | GLU | 248 | 44.198 | 15.351 | 47.634 | 1.00 | 54.71 |
| ATOM | 1853 | CG | GLU | 248 | 45.670 | 15.391 | 47.259 | 1.00 | 62.15 |
| ATOM | 1854 | CD | GLU | 248 | 46.067 | 14.259 | 46.317 | 1.00 | 66.63 |
| ATOM | 1855 | OE1 | GLU | 248 | 46.196 | 13.105 | 46.788 | 1.00 | 68.58 |
| ATOM | 1856 | OE2 | GLU | 248 | 46.241 | 14.520 | 45.105 | 1.00 | 68.21 |
| ATOM | 1857 | C | GLU | 248 | 43.222 | 13.955 | 49.446 | 1.00 | 47.90 |
| ATOM | 1858 | O | GLU | 248 | 42.063 | 14.250 | 49.726 | 1.00 | 46.55 |
| ATOM | 1859 | N | GLY | 249 | 44.133 | 13.614 | 50.351 | 1.00 | 45.49 |
| ATOM | 1860 | CA | GLY | 249 | 43.799 | 13.590 | 51.759 | 1.00 | 44.30 |
| ATOM | 1861 | C | GLY | 249 | 43.138 | 12.301 | 52.205 | 1.00 | 42.85 |
| ATOM | 1862 | O | GLY | 249 | 43.257 | 11.259 | 51.552 | 1.00 | 42.97 |
| ATOM | 1863 | N | ARG | 250 | 42.444 | 12.380 | 53.335 | 1.00 | 41.43 |
| ATOM | 1864 | CA | ARG | 250 | 41.747 | 11.232 | 53.897 | 1.00 | 39.63 |
| ATOM | 1865 | CB | ARG | 250 | 42.625 | 10.532 | 54.931 | 1.00 | 40.69 |
| ATOM | 1866 | CG | ARG | 250 | 44.092 | 10.454 | 54.559 | 1.00 | 43.91 |
| ATOM | 1867 | CD | ARG | 250 | 44.903 | 9.902 | 55.714 | 1.00 | 45.22 |
| ATOM | 1868 | NE | ARG | 250 | 44.630 | 8.487 | 55.940 | 1.00 | 45.43 |
| ATOM | 1869 | CZ | ARG | 250 | 45.040 | 7.813 | 57.007 | 1.00 | 44.67 |
| ATOM | 1870 | NH1 | ARG | 250 | 45.738 | 8.426 | 57.954 | 1.00 | 46.95 |
| ATOM | 1871 | NH2 | ARG | 250 | 44.761 | 6.524 | 57.121 | 1.00 | 46.99 |
| ATOM | 1872 | C | ARG | 250 | 40.486 | 11.726 | 54.580 | 1.00 | 37.70 |
| ATOM | 1873 | O | ARG | 250 | 40.430 | 12.865 | 55.042 | 1.00 | 37.51 |
| ATOM | 1874 | N | MET | 251 | 39.473 | 10.867 | 54.630 | 1.00 | 35.10 |
| ATOM | 1875 | CA | MET | 251 | 38.216 | 11.197 | 55.277 | 1.00 | 32.34 |
| ATOM | 1876 | CB | MET | 251 | 37.137 | 11.517 | 54.242 | 1.00 | 33.00 |
| ATOM | 1877 | CG | MET | 251 | 35.803 | 11.907 | 54.868 | 1.00 | 31.56 |
| ATOM | 1878 | SD | MET | 251 | 34.474 | 12.160 | 53.677 | 1.00 | 37.84 |
| ATOM | 1879 | CE | MET | 251 | 35.067 | 13.715 | 52.885 | 1.00 | 32.92 |
| ATOM | 1880 | C | MET | 251 | 37.764 | 10.007 | 56.121 | 1.00 | 32.47 |
| ATOM | 1881 | O | MET | 251 | 38.024 | 8.852 | 55.777 | 1.00 | 31.05 |

| | | | | | | | | | |
|---|---|---|---|---|---|---|---|---|---|
| ATOM | 1882 | N | CYS | 252 | 37.088 | 10.292 | 57.229 | 1.00 | 30.16 |
| ATOM | 1883 | CA | CYS | 252 | 36.595 | 9.236 | 58.092 | 1.00 | 30.32 |
| ATOM | 1884 | CB | CYS | 252 | 36.364 | 9.762 | 59.517 | 1.00 | 30.54 |
| ATOM | 1885 | SG | CYS | 252 | 35.601 | 8.557 | 60.676 | 1.00 | 28.61 |
| ATOM | 1886 | C | CYS | 252 | 35.292 | 8.717 | 57.511 | 1.00 | 29.86 |
| ATOM | 1887 | O | CYS | 252 | 34.422 | 9.495 | 57.114 | 1.00 | 29.84 |
| ATOM | 1888 | N | VAL | 253 | 35.170 | 7.397 | 57.438 | 1.00 | 28.79 |
| ATOM | 1889 | CA | VAL | 253 | 33.960 | 6.776 | 56.921 | 1.00 | 27.69 |
| ATOM | 1890 | CB | VAL | 253 | 34.291 | 5.761 | 55.816 | 1.00 | 28.07 |
| ATOM | 1891 | CG1 | VAL | 253 | 33.033 | 5.005 | 55.405 | 1.00 | 26.98 |
| ATOM | 1892 | CG2 | VAL | 253 | 34.898 | 6.484 | 54.624 | 1.00 | 24.14 |
| ATOM | 1893 | C | VAL | 253 | 33.200 | 6.069 | 58.038 | 1.00 | 28.79 |
| ATOM | 1894 | O | VAL | 253 | 33.801 | 5.448 | 58.922 | 1.00 | 31.23 |
| ATOM | 1895 | N | ASN | 254 | 31.879 | 6.188 | 58.000 | 1.00 | 28.38 |
| ATOM | 1896 | CA | ASN | 254 | 31.003 | 5.557 | 58.976 | 1.00 | 27.73 |
| ATOM | 1897 | CB | ASN | 254 | 29.834 | 6.473 | 59.328 | 1.00 | 27.41 |
| ATOM | 1898 | CG | ASN | 254 | 28.803 | 5.779 | 60.181 | 1.00 | 31.67 |
| ATOM | 1899 | OD1 | ASN | 254 | 29.048 | 4.675 | 60.677 | 1.00 | 32.14 |
| ATOM | 1900 | ND2 | ASN | 254 | 27.643 | 6.415 | 60.367 | 1.00 | 29.17 |
| ATOM | 1901 | C | ASN | 254 | 30.480 | 4.295 | 58.299 | 1.00 | 27.41 |
| ATOM | 1902 | O | ASN | 254 | 29.575 | 4.372 | 57.467 | 1.00 | 25.53 |
| ATOM | 1903 | N | THR | 255 | 31.049 | 3.142 | 58.654 | 1.00 | 24.66 |
| ATOM | 1904 | CA | THR | 255 | 30.662 | 1.883 | 58.016 | 1.00 | 24.86 |
| ATOM | 1905 | CB | THR | 255 | 31.501 | 0.665 | 58.527 | 1.00 | 23.42 |
| ATOM | 1906 | OG1 | THR | 255 | 31.071 | 0.310 | 59.849 | 1.00 | 23.50 |
| ATOM | 1907 | CG2 | THR | 255 | 32.973 | 0.982 | 58.558 | 1.00 | 23.88 |
| ATOM | 1908 | C | THR | 255 | 29.207 | 1.488 | 58.195 | 1.00 | 23.00 |
| ATOM | 1909 | O | THR | 255 | 28.589 | 0.984 | 57.259 | 1.00 | 24.38 |
| ATOM | 1910 | N | GLU | 256 | 28.673 | 1.710 | 59.394 | 1.00 | 23.70 |
| ATOM | 1911 | CA | GLU | 256 | 27.306 | 1.305 | 59.721 | 1.00 | 26.37 |
| ATOM | 1912 | CB | GLU | 256 | 26.271 | 2.017 | 58.838 | 1.00 | 26.22 |
| ATOM | 1913 | CG | GLU | 256 | 25.974 | 3.471 | 59.204 | 1.00 | 29.32 |
| ATOM | 1914 | CD | GLU | 256 | 25.284 | 3.644 | 60.558 | 1.00 | 31.10 |
| ATOM | 1915 | OE1 | GLU | 256 | 24.489 | 2.764 | 60.953 | 1.00 | 31.47 |
| ATOM | 1916 | OE2 | GLU | 256 | 25.523 | 4.682 | 61.218 | 1.00 | 30.04 |

| | | | | | | | | | |
|---|---|---|---|---|---|---|---|---|---|
| ATOM | 1917 | C | GLU | 256 | 27.269 | -0.203 | 59.458 | 1.00 | 27.40 |
| ATOM | 1918 | 0 | GLU | 256 | 26.369 | -0.713 | 58.782 | 1.00 | 26.71 |
| ATOM | 1919 | N | TRP | 257 | 28.269 | -0.912 | 59.982 | 1.00 | 25.98 |
| ATOM | 1920 | CA | TRP | 257 | 28.335 | -2.356 | 59.774 | 1.00 | 24.56 |
| ATOM | 1921 | CB | TRP | 257 | 29.714 | -2.928 | 60.180 | 1.00 | 21.05 |
| ATOM | 1922 | CG | TRP | 257 | 30.100 | -2.891 | 61.653 | 1.00 | 17.51 |
| ATOM | 1923 | CD2 | TRP | 257 | 31.429 | -3.026 | 62.182 | 1.00 | 16.19 |
| ATOM | 1924 | CE2 | TRP | 257 | 31.320 | -3.077 | 63.588 | 1.00 | 14.42 |
| ATOM | 1925 | CE3 | TRP | 257 | 32.705 | -3.112 | 61.597 | 1.00 | 16.23 |
| ATOM | 1926 | CD1 | TRP | 257 | 29.264 | -2.862 | 62.733 | 1.00 | 18.14 |
| ATOM | 1927 | NE1 | TRP | 257 | 29.990 | -2.977 | 63.902 | 1.00 | 19.95 |
| ATOM | 1928 | CZ2 | TRP | 257 | 32.435 | -3.214 | 64.421 | 1.00 | 17.46 |
| ATOM | 1929 | CZ3 | TRP | 257 | 33.815 | -3.246 | 62.424 | 1.00 | 13.91 |
| ATOM | 1930 | CH2 | TRP | 257 | 33.672 | -3.294 | 63.822 | 1.00 | 14.28 |
| ATOM | 1931 | C | TRP | 257 | 27.218 | -3.091 | 60.500 | 1.00 | 24.58 |
| ATOM | 1932 | 0 | TRP | 257 | 27.067 | -4.305 | 60.352 | 1.00 | 24.81 |
| ATOM | 1933 | N | GLY | 258 | 26.427 | -2.354 | 61.273 | 1.00 | 23.21 |
| ATOM | 1934 | CA | GLY | 258 | 25.328 | -2.981 | 61.982 | 1.00 | 23.11 |
| ATOM | 1935 | C | GLY | 258 | 24.385 | -3.640 | 60.991 | 1.00 | 25.72 |
| ATOM | 1936 | 0 | GLY | 258 | 23.758 | -4.660 | 61.285 | 1.00 | 28.37 |
| ATOM | 1937 | N | ALA | 259 | 24.288 | -3.067 | 59.796 | 1.00 | 24.64 |
| ATOM | 1938 | CA | ALA | 259 | 23.406 | -3.630 | 58.789 | 1.00 | 25.53 |
| ATOM | 1939 | CB | ALA | 259 | 22.866 | -2.519 | 57.874 | 1.00 | 25.11 |
| ATOM | 1940 | C | ALA | 259 | 24.084 | -4.724 | 57.961 | 1.00 | 25.44 |
| ATOM | 1941 | 0 | ALA | 259 | 23.515 | -5.205 | 56.985 | 1.00 | 24.68 |
| ATOM | 1942 | N | PHE | 260 | 25.306 | -5.101 | 58.329 | 1.00 | 26.96 |
| ATOM | 1943 | CA | PHE | 260 | 25.995 | -6.175 | 57.614 | 1.00 | 28.11 |
| ATOM | 1944 | CB | PHE | 260 | 27.359 | -6.440 | 58.254 | 1.00 | 30.88 |
| ATOM | 1945 | CG | PHE | 260 | 28.127 | -7.569 | 57.625 | 1.00 | 33.87 |
| ATOM | 1946 | CD1 | PHE | 260 | 28.496 | -7.525 | 56.286 | 1.00 | 33.60 |
| ATOM | 1947 | CD2 | PHE | 260 | 28.499 | -8.675 | 58.380 | 1.00 | 37.30 |
| ATOM | 1948 | CE1 | PHE | 260 | 29.220 | -8.564 | 55.716 | 1.00 | 33.58 |
| ATOM | 1949 | CE2 | PHE | 260 | 29.229 | -9.720 | 57.808 | 1.00 | 35.65 |
| ATOM | 1950 | CZ | PHE | 260 | 29.586 | -9.660 | 56.478 | 1.00 | 34.41 |
| ATOM | 1951 | C | PHE | 260 | 25.080 | -7.388 | 57.783 | 1.00 | 28.87 |

| | | | | | | | | | |
|---|---|---|---|---|---|---|---|---|---|
| ATOM | 1952 | O | PHE | 260 | 24.487 | -7.576 | 58.849 | 1.00 | 27.08 |
| ATOM | 1953 | N | GLY | 261 | 24.941 | -8.193 | 56.737 | 1.00 | 28.88 |
| ATOM | 1954 | CA | GLY | 261 | 24.074 | -9.357 | 56.826 | 1.00 | 30.83 |
| ATOM | 1955 | C | GLY | 261 | 22.664 | -9.092 | 56.317 | 1.00 | 32.15 |
| ATOM | 1956 | O | GLY | 261 | 21.905 | -10.021 | 56.043 | 1.00 | 34.22 |
| ATOM | 1957 | N | ASP | 262 | 22.307 | -7.822 | 56.175 | 1.00 | 33.45 |
| ATOM | 1958 | CA | ASP | 262 | 20.975 | -7.456 | 55.701 | 1.00 | 35.91 |
| ATOM | 1959 | CB | ASP | 262 | 20.761 | -5.948 | 55.868 | 1.00 | 35.78 |
| ATOM | 1960 | CG | ASP | 262 | 20.674 | -5.541 | 57.323 | 1.00 | 35.93 |
| ATOM | 1961 | OD1 | ASP | 262 | 20.903 | -6.415 | 58.182 | 1.00 | 37.70 |
| ATOM | 1962 | OD2 | ASP | 262 | 20.382 | -4.364 | 57.615 | 1.00 | 35.14 |
| ATOM | 1963 | C | ASP | 262 | 20.676 | -7.884 | 54.262 | 1.00 | 36.35 |
| ATOM | 1964 | O | ASP | 262 | 19.546 | -7.758 | 53.799 | 1.00 | 37.40 |
| ATOM | 1965 | N | SER | 263 | 21.685 | -8.380 | 53.554 | 1.00 | 37.07 |
| ATOM | 1966 | CA | SER | 263 | 21.488 | -8.863 | 52.189 | 1.00 | 37.53 |
| ATOM | 1967 | CB | SER | 263 | 22.420 | -8.155 | 51.200 | 1.00 | 37.00 |
| ATOM | 1968 | OG | SER | 263 | 22.028 | -6.815 | 50.991 | 1.00 | 38.85 |
| ATOM | 1969 | C | SER | 263 | 21.770 | -10.359 | 52.161 | 1.00 | 37.06 |
| ATOM | 1970 | O | SER | 263 | 22.062 | -10.923 | 51.107 | 1.00 | 36.90 |
| ATOM | 1971 | N | GLY | 264 | 21.697 | -10.988 | 53.331 | 1.00 | 36.97 |
| ATOM | 1972 | CA | GLY | 264 | 21.934 | -12.418 | 53.428 | 1.00 | 37.50 |
| ATOM | 1973 | C | GLY | 264 | 23.370 | -12.857 | 53.663 | 1.00 | 38.59 |
| ATOM | 1974 | O | GLY | 264 | 23.666 | -14.050 | 53.573 | 1.00 | 40.28 |
| ATOM | 1975 | N | GLU | 265 | 24.263 | -11.915 | 53.961 | 1.00 | 37.52 |
| ATOM | 1976 | CA | GLU | 265 | 25.671 | -12.237 | 54.199 | 1.00 | 36.34 |
| ATOM | 1977 | CB | GLU | 265 | 26.488 | -10.965 | 54.438 | 1.00 | 35.82 |
| ATOM | 1978 | CG | GLU | 265 | 26.535 | -9.976 | 53.289 | 1.00 | 38.57 |
| ATOM | 1979 | CD | GLU | 265 | 25.270 | -9.148 | 53.158 | 1.00 | 39.55 |
| ATOM | 1980 | OE1 | GLU | 265 | 24.600 | -8.901 | 54.173 | 1.00 | 38.51 |
| ATOM | 1981 | OE2 | GLU | 265 | 24.953 | -8.722 | 52.031 | 1.00 | 43.82 |
| ATOM | 1982 | C | GLU | 265 | 25.906 | -13.171 | 55.391 | 1.00 | 36.38 |
| ATOM | 1983 | O | GLU | 265 | 26.899 | -13.906 | 55.425 | 1.00 | 35.35 |
| ATOM | 1984 | N | LEU | 266 | 24.996 | -13.140 | 56.362 | 1.00 | 34.63 |
| ATOM | 1985 | CA | LEU | 266 | 25.130 | -13.955 | 57.567 | 1.00 | 35.02 |
| ATOM | 1986 | CB | LEU | 266 | 25.008 | -13.054 | 58.803 | 1.00 | 31.68 |

| | | | | | | | | | |
|---|---|---|---|---|---|---|---|---|---|
| ATOM | 1987 | CG | LEU | 266 | 26.017 | -11.914 | 58.973 | 1.00 | 33.35 |
| ATOM | 1988 | CD1 | LEU | 266 | 25.555 | -10.975 | 60.077 | 1.00 | 32.52 |
| ATOM | 1989 | CD2 | LEU | 266 | 27.383 | -12.480 | 59.294 | 1.00 | 32.43 |
| ATOM | 1990 | C | LEU | 266 | 24.108 | -15.092 | 57.674 | 1.00 | 35.37 |
| ATOM | 1991 | O | LEU | 266 | 24.047 | -15.779 | 58.696 | 1.00 | 35.21 |
| ATOM | 1992 | N | ASP | 267 | 23.321 | -15.300 | 56.627 | 1.00 | 36.35 |
| ATOM | 1993 | CA | ASP | 267 | 22.286 | -16.332 | 56.643 | 1.00 | 39.50 |
| ATOM | 1994 | CB | ASP | 267 | 21.664 | -16.480 | 55.248 | 1.00 | 42.21 |
| ATOM | 1995 | CG | ASP | 267 | 20.666 | -15.369 | 54.921 | 1.00 | 45.43 |
| ATOM | 1996 | OD1 | ASP | 267 | 20.205 | -15.320 | 53.759 | 1.00 | 48.41 |
| ATOM | 1997 | OD2 | ASP | 267 | 20.332 | -14.554 | 55.813 | 1.00 | 45.57 |
| ATOM | 1998 | C | ASP | 267 | 22.676 | -17.715 | 57.171 | 1.00 | 38.87 |
| ATOM | 1999 | O | ASP | 267 | 21.888 | -18.353 | 57.867 | 1.00 | 39.64 |
| ATOM | 2000 | N | GLU | 268 | 23.879 | -18.179 | 56.860 | 1.00 | 38.72 |
| ATOM | 2001 | CA | GLU | 268 | 24.301 | -19.502 | 57.313 | 1.00 | 39.91 |
| ATOM | 2002 | CB | GLU | 268 | 25.510 | -19.971 | 56.495 | 1.00 | 40.60 |
| ATOM | 2003 | CG | GLU | 268 | 26.847 | -19.444 | 56.976 | 1.00 | 43.85 |
| ATOM | 2004 | CD | GLU | 268 | 27.969 | -19.710 | 55.981 | 1.00 | 47.64 |
| ATOM | 2005 | OE1 | GLU | 268 | 28.013 | -19.017 | 54.941 | 1.00 | 49.73 |
| ATOM | 2006 | OE2 | GLU | 268 | 28.802 | -20.612 | 56.232 | 1.00 | 48.40 |
| ATOM | 2007 | C | GLU | 268 | 24.633 | -19.577 | 58.807 | 1.00 | 40.59 |
| ATOM | 2008 | O | GLU | 268 | 24.790 | -20.667 | 59.360 | 1.00 | 41.43 |
| ATOM | 2009 | N | PHE | 269 | 24.734 | -18.427 | 59.462 | 1.00 | 39.17 |
| ATOM | 2010 | CA | PHE | 269 | 25.070 | -18.402 | 60.882 | 1.00 | 37.75 |
| ATOM | 2011 | CB | PHE | 269 | 26.182 | -17.385 | 61.127 | 1.00 | 34.69 |
| ATOM | 2012 | CG | PHE | 269 | 27.435 | -17.675 | 60.369 | 1.00 | 35.74 |
| ATOM | 2013 | CD1 | PHE | 269 | 28.144 | -18.853 | 60.599 | 1.00 | 35.94 |
| ATOM | 2014 | CD2 | PHE | 269 | 27.910 | -16.781 | 59.416 | 1.00 | 34.75 |
| ATOM | 2015 | CE1 | PHE | 269 | 29.306 | -19.136 | 59.891 | 1.00 | 34.71 |
| ATOM | 2016 | CE2 | PHE | 269 | 29.068 | -17.050 | 58.701 | 1.00 | 34.58 |
| ATOM | 2017 | CZ | PHE | 269 | 29.770 | -18.233 | 58.939 | 1.00 | 35.80 |
| ATOM | 2018 | C | PHE | 269 | 23.898 | -18.085 | 61.793 | 1.00 | 36.73 |
| ATOM | 2019 | O | PHE | 269 | 23.932 | -18.384 | 62.984 | 1.00 | 36.59 |
| ATOM | 2020 | N | LEU | 270 | 22.861 | -17.480 | 61.231 | 1.00 | 37.18 |
| ATOM | 2021 | CA | LEU | 270 | 21.696 | -17.107 | 62.012 | 1.00 | 37.71 |

| | | | | | | | | | |
|---|---|---|---|---|---|---|---|---|---|
| ATOM | 2022 | CB | LEU | 270 | 20.712 | -16.332 | 61.135 | 1.00 | 36.52 |
| ATOM | 2023 | CG | LEU | 270 | 21.264 | -15.036 | 60.521 | 1.00 | 37.18 |
| ATOM | 2024 | CD1 | LEU | 270 | 20.299 | -14.516 | 59.466 | 1.00 | 38.72 |
| ATOM | 2025 | CD2 | LEU | 270 | 21.488 | -13.990 | 61.604 | 1.00 | 34.72 |
| ATOM | 2026 | C | LEU | 270 | 21.010 | -18.312 | 62.644 | 1.00 | 38.27 |
| ATOM | 2027 | O | LEU | 270 | 20.794 | -19.333 | 61.995 | 1.00 | 39.49 |
| ATOM | 2028 | N | LEU | 271 | 20.685 | -18.176 | 63.924 | 1.00 | 37.92 |
| ATOM | 2029 | CA | LEU | 271 | 20.010 | -19.212 | 64.693 | 1.00 | 38.22 |
| ATOM | 2030 | CB | LEU | 271 | 20.657 | -19.339 | 66.078 | 1.00 | 37.71 |
| ATOM | 2031 | CG | LEU | 271 | 21.897 | -20.220 | 66.261 | 1.00 | 38.14 |
| ATOM | 2032 | CD1 | LEU | 271 | 22.827 | -20.111 | 65.075 | 1.00 | 39.09 |
| ATOM | 2033 | CD2 | LEU | 271 | 22.596 | -19.830 | 67.549 | 1.00 | 35.73 |
| ATOM | 2034 | C | LEU | 271 | 18.536 | -18.845 | 64.855 | 1.00 | 39.78 |
| ATOM | 2035 | O | LEU | 271 | 18.125 | -17.721 | 64.538 | 1.00 | 38.05 |
| ATOM | 2036 | N | GLU | 272 | 17.751 | -19.794 | 65.358 | 1.00 | 39.69 |
| ATOM | 2037 | CA | GLU | 272 | 16.322 | -19.590 | 65.575 | 1.00 | 41.03 |
| ATOM | 2038 | CB | GLU | 272 | 15.697 | -20.842 | 66.219 | 1.00 | 43.64 |
| ATOM | 2039 | CG | GLU | 272 | 16.221 | -21.179 | 67.627 | 1.00 | 47.44 |
| ATOM | 2040 | CD | GLU | 272 | 15.685 | -22.509 | 68.182 | 1.00 | 49.81 |
| ATOM | 2041 | OE1 | GLU | 272 | 16.081 | -23.580 | 67.666 | 1.00 | 51.29 |
| ATOM | 2042 | OE2 | GLU | 272 | 14.869 | -22.484 | 69.134 | 1.00 | 47.60 |
| ATOM | 2043 | C | GLU | 272 | 16.084 | -18.377 | 66.466 | 1.00 | 39.89 |
| ATOM | 2044 | O | GLU | 272 | 15.151 | -17.602 | 66.250 | 1.00 | 40.35 |
| ATOM | 2045 | N | TYR | 273 | 16.944 | -18.208 | 67.465 | 1.00 | 38.65 |
| ATOM | 2046 | CA | TYR | 273 | 16.813 | -17.095 | 68.393 | 1.00 | 35.97 |
| ATOM | 2047 | CB | TYR | 273 | 17.829 | -17.238 | 69.530 | 1.00 | 35.50 |
| ATOM | 2048 | CG | TYR | 273 | 18.008 | -18.658 | 70.009 | 1.00 | 34.45 |
| ATOM | 2049 | CD1 | TYR | 273 | 19.109 | -19.416 | 69.611 | 1.00 | 32.53 |
| ATOM | 2050 | CE1 | TYR | 273 | 19.252 | -20.740 | 70.017 | 1.00 | 35.58 |
| ATOM | 2051 | CD2 | TYR | 273 | 17.053 | -19.258 | 70.830 | 1.00 | 34.35 |
| ATOM | 2052 | CE2 | TYR | 273 | 17.185 | -20.580 | 71.241 | 1.00 | 34.82 |
| ATOM | 2053 | CZ | TYR | 273 | 18.281 | -21.314 | 70.830 | 1.00 | 35.96 |
| ATOM | 2054 | OH | TYR | 273 | 18.381 | -22.626 | 71.208 | 1.00 | 38.31 |
| ATOM | 2055 | C | TYR | 273 | 17.021 | -15.763 | 67.680 | 1.00 | 35.11 |
| ATOM | 2056 | O | TYR | 273 | 16.404 | -14.752 | 68.031 | 1.00 | 34.85 |

| | | | | | | | | | |
|---|---|---|---|---|---|---|---|---|---|
| ATOM | 2057 | N | ASP | 274 | 17.888 | -15.763 | 66.676 | 1.00 | 36.47 |
| ATOM | 2058 | CA | ASP | 274 | 18.164 | -14.541 | 65.933 | 1.00 | 36.65 |
| ATOM | 2059 | CB | ASP | 274 | 19.405 | -14.718 | 65.059 | 1.00 | 32.36 |
| ATOM | 2060 | CG | ASP | 274 | 20.627 | -15.072 | 65.869 | 1.00 | 32.89 |
| ATOM | 2061 | OD1 | ASP | 274 | 20.949 | -14.315 | 66.810 | 1.00 | 30.53 |
| ATOM | 2062 | OD2 | ASP | 274 | 21.265 | -16.104 | 65.569 | 1.00 | 32.08 |
| ATOM | 2063 | C | ASP | 274 | 16.968 | -14.165 | 65.081 | 1.00 | 37.27 |
| ATOM | 2064 | O | ASP | 274 | 16.571 | -13.001 | 65.040 | 1.00 | 37.20 |
| ATOM | 2065 | N | ARG | 275 | 16.380 | -15.148 | 64.410 | 1.00 | 39.32 |
| ATOM | 2066 | CA | ARG | 275 | 15.222 | -14.866 | 63.574 | 1.00 | 41.70 |
| ATOM | 2067 | CB | ARG | 275 | 14.803 | -16.121 | 62.809 | 1.00 | 44.47 |
| ATOM | 2068 | CG | ARG | 275 | 15.908 | -16.666 | 61.914 | 1.00 | 49.05 |
| ATOM | 2069 | CD | ARG | 275 | 15.516 | -18.002 | 61.303 | 1.00 | 53.46 |
| ATOM | 2070 | NE | ARG | 275 | 16.668 | -18.740 | 60.779 | 1.00 | 57.36 |
| ATOM | 2071 | CZ | ARG | 275 | 17.352 | -18.408 | 59.685 | 1.00 | 58.81 |
| ATOM | 2072 | NH1 | ARG | 275 | 18.383 | -19.148 | 59.296 | 1.00 | 60.43 |
| ATOM | 2073 | NH2 | ARG | 275 | 17.005 | -17.341 | 58.976 | 1.00 | 61.75 |
| ATOM | 2074 | C | ARG | 275 | 14.079 | -14.353 | 64.446 | 1.00 | 41.43 |
| ATOM | 2075 | O | ARG | 275 | 13.350 | -13.444 | 64.059 | 1.00 | 40.04 |
| ATOM | 2076 | N | LEU | 276 | 13.939 | -14.927 | 65.637 | 1.00 | 40.97 |
| ATOM | 2077 | CA | LEU | 276 | 12.888 | -14.507 | 66.556 | 1.00 | 42.14 |
| ATOM | 2078 | CB | LEU | 276 | 12.831 | -15.450 | 67.761 | 1.00 | 44.12 |
| ATOM | 2079 | CG | LEU | 276 | 12.315 | -16.862 | 67.468 | 1.00 | 47.86 |
| ATOM | 2080 | CD1 | LEU | 276 | 12.662 | -17.800 | 68.618 | 1.00 | 48.62 |
| ATOM | 2081 | CD2 | LEU | 276 | 10.808 | -16.808 | 67.236 | 1.00 | 47.43 |
| ATOM | 2082 | C | LEU | 276 | 13.094 | -13.072 | 67.034 | 1.00 | 40.87 |
| ATOM | 2083 | O | LEU | 276 | 12.152 | -12.281 | 67.072 | 1.00 | 41.20 |
| ATOM | 2084 | N | VAL | 277 | 14.322 | -12.740 | 67.412 | 1.00 | 39.68 |
| ATOM | 2085 | CA | VAL | 277 | 14.617 | -11.390 | 67.876 | 1.00 | 40.86 |
| ATOM | 2086 | CB | VAL | 277 | 16.084 | -11.263 | 68.331 | 1.00 | 41.86 |
| ATOM | 2087 | CG1 | VAL | 277 | 16.447 | -9.802 | 68.497 | 1.00 | 43.25 |
| ATOM | 2088 | CG2 | VAL | 277 | 16.290 | -12.012 | 69.647 | 1.00 | 41.47 |
| ATOM | 2089 | C | VAL | 277 | 14.363 | -10.381 | 66.761 | 1.00 | 40.10 |
| ATOM | 2090 | O | VAL | 277 | 13.813 | -9.305 | 66.993 | 1.00 | 41.12 |
| ATOM | 2091 | N | ASP | 278 | 14.767 | -10.738 | 65.550 | 1.00 | 39.42 |

| | | | | | | | | | |
|---|---|---|---|---|---|---|---|---|---|
| ATOM | 2092 | CA | ASP | 278 | 14.592 | -9.867 | 64.398 | 1.00 | 40.24 |
| ATOM | 2093 | CB | ASP | 278 | 15.356 | -10.434 | 63.195 | 1.00 | 38.24 |
| ATOM | 2094 | CG | ASP | 278 | 15.179 | -9.598 | 61.943 | 1.00 | 40.23 |
| ATOM | 2095 | OD1 | ASP | 278 | 15.260 | -8.351 | 62.043 | 1.00 | 39.72 |
| ATOM | 2096 | OD2 | ASP | 278 | 14.969 | -10.187 | 60.860 | 1.00 | 38.10 |
| ATOM | 2097 | C | ASP | 278 | 13.120 | -9.669 | 64.043 | 1.00 | 41.19 |
| ATOM | 2098 | O | ASP | 278 | 12.693 | -8.545 | 63.791 | 1.00 | 40.82 |
| ATOM | 2099 | N | GLU | 279 | 12.347 | -10.754 | 64.035 | 1.00 | 43.34 |
| ATOM | 2100 | CA | GLU | 279 | 10.922 | -10.688 | 63.696 | 1.00 | 46.81 |
| ATOM | 2101 | CB | GLU | 279 | 10.321 | -12.097 | 63.627 | 1.00 | 50.53 |
| ATOM | 2102 | CG | GLU | 279 | 10.870 | -12.965 | 62.496 | 1.00 | 56.10 |
| ATOM | 2103 | CD | GLU | 279 | 10.320 | -14.382 | 62.523 | 1.00 | 59.07 |
| ATOM | 2104 | OE1 | GLU | 279 | 10.336 | -15.006 | 63.607 | 1.00 | 60.28 |
| ATOM | 2105 | OE2 | GLU | 279 | 9.880 | -14.876 | 61.461 | 1.00 | 60.79 |
| ATOM | 2106 | C | GLU | 279 | 10.086 | -9.840 | 64.652 | 1.00 | 47.25 |
| ATOM | 2107 | O | GLU | 279 | 9.048 | -9.303 | 64.260 | 1.00 | 46.34 |
| ATOM | 2108 | N | SER | 280 | 10.535 | -9.722 | 65.899 | 1.00 | 46.87 |
| ATOM | 2109 | CA | SER | 280 | 9.809 | -8.948 | 66.900 | 1.00 | 47.53 |
| ATOM | 2110 | CB | SER | 280 | 9.769 | -9.708 | 68.228 | 1.00 | 49.98 |
| ATOM | 2111 | OG | SER | 280 | 9.043 | -10.919 | 68.093 | 1.00 | 52.36 |
| ATOM | 2112 | C | SER | 280 | 10.415 | -7.575 | 67.129 | 1.00 | 47.33 |
| ATOM | 2113 | O | SER | 280 | 9.909 | -6.788 | 67.936 | 1.00 | 45.86 |
| ATOM | 2114 | N | SER | 281 | 11.499 | -7.289 | 66.416 | 1.00 | 46.95 |
| ATOM | 2115 | CA | SER | 281 | 12.172 | -6.004 | 66.552 | 1.00 | 46.75 |
| ATOM | 2116 | CB | SER | 281 | 13.581 | -6.081 | 65.971 | 1.00 | 47.24 |
| ATOM | 2117 | OG | SER | 281 | 13.524 | -6.172 | 64.559 | 1.00 | 47.80 |
| ATOM | 2118 | C | SER | 281 | 11.391 | -4.915 | 65.824 | 1.00 | 45.65 |
| ATOM | 2119 | O | SER | 281 | 10.514 | -5.199 | 65.013 | 1.00 | 45.10 |
| ATOM | 2120 | N | ALA | 282 | 11.723 | -3.667 | 66.123 | 1.00 | 45.75 |
| ATOM | 2121 | CA | ALA | 282 | 11.066 | -2.530 | 65.500 | 1.00 | 45.70 |
| ATOM | 2122 | CB | ALA | 282 | 11.257 | -1.289 | 66.354 | 1.00 | 45.60 |
| ATOM | 2123 | C | ALA | 282 | 11.617 | -2.286 | 64.100 | 1.00 | 46.48 |
| ATOM | 2124 | O | ALA | 282 | 11.252 | -1.303 | 63.449 | 1.00 | 48.61 |
| ATOM | 2125 | N | ASN | 283 | 12.493 | -3.172 | 63.633 | 1.00 | 43.90 |
| ATOM | 2126 | CA | ASN | 283 | 13.076 | -3.015 | 62.306 | 1.00 | 41.45 |

| | | | | | | | | | |
|---|---|---|---|---|---|---|---|---|---|
| ATOM | 2127 | CB | ASN | 283 | 14.300 | -2.092 | 62.384 | 1.00 | 40.08 |
| ATOM | 2128 | CG | ASN | 283 | 15.398 | -2.631 | 63.289 | 1.00 | 39.25 |
| ATOM | 2129 | OD1 | ASN | 283 | 15.136 | -3.308 | 64.289 | 1.00 | 37.65 |
| ATOM | 2130 | ND2 | ASN | 283 | 16.641 | -2.310 | 62.950 | 1.00 | 37.96 |
| ATOM | 2131 | C | ASN | 283 | 13.433 | -4.350 | 61.655 | 1.00 | 41.06 |
| ATOM | 2132 | O | ASN | 283 | 14.585 | -4.606 | 61.318 | 1.00 | 40.48 |
| ATOM | 2133 | N | PRO | 284 | 12.423 | -5.211 | 61.455 | 1.00 | 40.23 |
| ATOM | 2134 | CD | PRO | 284 | 11.013 | -4.898 | 61.751 | 1.00 | 40.75 |
| ATOM | 2135 | CA | PRO | 284 | 12.534 | -6.540 | 60.851 | 1.00 | 40.08 |
| ATOM | 2136 | CB | PRO | 284 | 11.080 | -6.914 | 60.581 | 1.00 | 40.52 |
| ATOM | 2137 | CG | PRO | 284 | 10.364 | -6.260 | 61.712 | 1.00 | 41.21 |
| ATOM | 2138 | C | PRO | 284 | 13.366 | -6.565 | 59.579 | 1.00 | 39.55 |
| ATOM | 2139 | O | PRO | 284 | 13.054 | -5.868 | 58.617 | 1.00 | 40.95 |
| ATOM | 2140 | N | GLY | 285 | 14.416 | -7.382 | 59.576 | 1.00 | 38.56 |
| ATOM | 2141 | CA | GLY | 285 | 15.266 | -7.491 | 58.407 | 1.00 | 35.73 |
| ATOM | 2142 | C | GLY | 285 | 16.428 | -6.516 | 58.371 | 1.00 | 35.10 |
| ATOM | 2143 | O | GLY | 285 | 17.288 | -6.624 | 57.500 | 1.00 | 36.22 |
| ATOM | 2144 | N | GLN | 286 | 16.468 | -5.573 | 59.308 | 1.00 | 34.06 |
| ATOM | 2145 | CA | GLN | 286 | 17.547 | -4.584 | 59.348 | 1.00 | 34.96 |
| ATOM | 2146 | CB | GLN | 286 | 16.974 | -3.166 | 59.321 | 1.00 | 39.16 |
| ATOM | 2147 | CG | GLN | 286 | 16.189 | -2.825 | 58.067 | 1.00 | 45.72 |
| ATOM | 2148 | CD | GLN | 286 | 15.698 | -1.384 | 58.074 | 1.00 | 51.15 |
| ATOM | 2149 | OE1 | GLN | 286 | 14.816 | -1.018 | 58.860 | 1.00 | 52.21 |
| ATOM | 2150 | NE2 | GLN | 286 | 16.276 | -0.555 | 57.203 | 1.00 | 50.85 |
| ATOM | 2151 | C | GLN | 286 | 18.439 | -4.719 | 60.573 | 1.00 | 33.59 |
| ATOM | 2152 | O | GLN | 286 | 17.993 | -5.157 | 61.637 | 1.00 | 33.18 |
| ATOM | 2153 | N | GLN | 287 | 19.701 | -4.334 | 60.408 | 1.00 | 32.85 |
| ATOM | 2154 | CA | GLN | 287 | 20.691 | -4.375 | 61.484 | 1.00 | 32.45 |
| ATOM | 2155 | CB | GLN | 287 | 20.248 | -3.456 | 62.636 | 1.00 | 33.34 |
| ATOM | 2156 | CG | GLN | 287 | 19.955 | -1.999 | 62.251 | 1.00 | 31.48 |
| ATOM | 2157 | CD | GLN | 287 | 21.188 | -1.259 | 61.743 | 1.00 | 31.78 |
| ATOM | 2158 | OE1 | GLN | 287 | 21.330 | -1.010 | 60.544 | 1.00 | 33.25 |
| ATOM | 2159 | NE2 | GLN | 287 | 22.090 | -0.921 | 62.652 | 1.00 | 27.51 |
| ATOM | 2160 | C | GLN | 287 | 20.924 | -5.788 | 62.032 | 1.00 | 30.79 |
| ATOM | 2161 | O | GLN | 287 | 21.120 | -5.957 | 63.229 | 1.00 | 29.31 |

| | | | | | | | | | |
|---|---|---|---|---|---|---|---|---|---|
| ATOM | 2162 | N | LEU | 288 | 20.921 | -6.791 | 61.158 | 1.00 | 29.33 |
| ATOM | 2163 | CA | LEU | 288 | 21.101 | -8.181 | 61.585 | 1.00 | 27.53 |
| ATOM | 2164 | CB | LEU | 288 | 20.940 | -9.129 | 60.393 | 1.00 | 28.13 |
| ATOM | 2165 | CG | LEU | 288 | 19.599 | -9.090 | 59.647 | 1.00 | 29.14 |
| ATOM | 2166 | CD1 | LEU | 288 | 19.390 | -10.418 | 58.922 | 1.00 | 27.60 |
| ATOM | 2167 | CD2 | LEU | 288 | 18.453 | -8.844 | 60.621 | 1.00 | 27.42 |
| ATOM | 2168 | C | LEU | 288 | 22.418 | -8.476 | 62.297 | 1.00 | 27.92 |
| ATOM | 2169 | O | LEU | 288 | 22.438 | -9.184 | 63.303 | 1.00 | 28.24 |
| ATOM | 2170 | N | TYR | 289 | 23.520 | -7.946 | 61.776 | 1.00 | 27.17 |
| ATOM | 2171 | CA | TYR | 289 | 24.819 | -8.153 | 62.399 | 1.00 | 24.83 |
| ATOM | 2172 | CB | TYR | 289 | 25.899 | -7.458 | 61.583 | 1.00 | 24.32 |
| ATOM | 2173 | CG | TYR | 289 | 27.303 | -7.575 | 62.137 | 1.00 | 21.26 |
| ATOM | 2174 | CD1 | TYR | 289 | 27.951 | -8.814 | 62.208 | 1.00 | 20.00 |
| ATOM | 2175 | CE1 | TYR | 289 | 29.281 | -8.909 | 62.616 | 1.00 | 18.43 |
| ATOM | 2176 | CD2 | TYR | 289 | 28.013 | -6.441 | 62.503 | 1.00 | 18.12 |
| ATOM | 2177 | CE2 | TYR | 289 | 29.338 | -6.520 | 62.918 | 1.00 | 20.65 |
| ATOM | 2178 | CZ | TYR | 289 | 29.976 | -7.762 | 62.966 | 1.00 | 21.27 |
| ATOM | 2179 | OH | TYR | 289 | 31.314 | -7.833 | 63.326 | 1.00 | 19.02 |
| ATOM | 2180 | C | TYR | 289 | 24.771 | -7.566 | 63.799 | 1.00 | 26.94 |
| ATOM | 2181 | O | TYR | 289 | 25.221 | -8.175 | 64.776 | 1.00 | 27.95 |
| ATOM | 2182 | N | GLU | 290 | 24.198 | -6.374 | 63.892 | 1.00 | 27.68 |
| ATOM | 2183 | CA | GLU | 290 | 24.078 | -5.686 | 65.165 | 1.00 | 26.41 |
| ATOM | 2184 | CB | GLU | 290 | 23.484 | -4.309 | 64.927 | 1.00 | 26.55 |
| ATOM | 2185 | CG | GLU | 290 | 23.059 | -3.595 | 66.180 | 1.00 | 27.05 |
| ATOM | 2186 | CD | GLU | 290 | 22.815 | -2.142 | 65.913 | 1.00 | 25.47 |
| ATOM | 2187 | OE1 | GLU | 290 | 23.716 | -1.336 | 66.204 | 1.00 | 27.17 |
| ATOM | 2188 | OE2 | GLU | 290 | 21.731 | -1.815 | 65.398 | 1.00 | 29.09 |
| ATOM | 2189 | C | GLU | 290 | 23.218 | -6.463 | 66.159 | 1.00 | 26.59 |
| ATOM | 2190 | O | GLU | 290 | 23.458 | -6.430 | 67.371 | 1.00 | 25.62 |
| ATOM | 2191 | N | LYS | 291 | 22.216 | -7.166 | 65.646 | 1.00 | 26.31 |
| ATOM | 2192 | CA | LYS | 291 | 21.343 | -7.942 | 66.509 | 1.00 | 27.77 |
| ATOM | 2193 | CB | LYS | 291 | 20.110 | -8.394 | 65.722 | 1.00 | 28.30 |
| ATOM | 2194 | CG | LYS | 291 | 19.096 | -7.263 | 65.585 | 1.00 | 33.35 |
| ATOM | 2195 | CD | LYS | 291 | 18.005 | -7.529 | 64.555 | 1.00 | 33.56 |
| ATOM | 2196 | CE | LYS | 291 | 17.038 | -6.330 | 64.522 | 1.00 | 36.46 |

| | | | | | | | | | |
|---|---|---|---|---|---|---|---|---|---|
| ATOM | 2197 | NZ | LYS | 291 | 16.150 | -6.319 | 63.327 | 1.00 | 36.55 |
| ATOM | 2198 | C | LYS | 291 | 22.073 | -9.123 | 67.138 | 1.00 | 26.53 |
| ATOM | 2199 | O | LYS | 291 | 21.584 | -9.736 | 68.084 | 1.00 | 27.81 |
| ATOM | 2200 | N | LEU | 292 | 23.261 | -9.426 | 66.628 | 1.00 | 26.02 |
| ATOM | 2201 | CA | LEU | 292 | 24.043 | -10.523 | 67.168 | 1.00 | 25.35 |
| ATOM | 2202 | CB | LEU | 292 | 24.922 | -11.140 | 66.079 | 1.00 | 25.16 |
| ATOM | 2203 | CG | LEU | 292 | 24.229 | -11.746 | 64.856 | 1.00 | 26.25 |
| ATOM | 2204 | CD1 | LEU | 292 | 25.274 | -12.190 | 63.827 | 1.00 | 23.09 |
| ATOM | 2205 | CD2 | LEU | 292 | 23.359 | -12.912 | 65.297 | 1.00 | 24.40 |
| ATOM | 2206 | C | LEU | 292 | 24.942 | -10.030 | 68.283 | 1.00 | 25.18 |
| ATOM | 2207 | O | LEU | 292 | 25.392 | -10.808 | 69.120 | 1.00 | 23.84 |
| ATOM | 2208 | N | ILE | 293 | 25.179 | -8.723 | 68.308 | 1.00 | 24.94 |
| ATOM | 2209 | CA | ILE | 293 | 26.107 | -8.140 | 69.267 | 1.00 | 23.59 |
| ATOM | 2210 | CB | ILE | 293 | 27.259 | -7.468 | 68.476 | 1.00 | 24.66 |
| ATOM | 2211 | CG2 | ILE | 293 | 28.233 | -6.762 | 69.409 | 1.00 | 21.05 |
| ATOM | 2212 | CG1 | ILE | 293 | 27.952 | -8.527 | 67.618 | 1.00 | 24.42 |
| ATOM | 2213 | CD1 | ILE | 293 | 28.715 | -7.965 | 66.441 | 1.00 | 25.64 |
| ATOM | 2214 | C | ILE | 293 | 25.560 | -7.148 | 70.278 | 1.00 | 25.10 |
| ATOM | 2215 | O | ILE | 293 | 25.797 | -7.289 | 71.474 | 1.00 | 23.79 |
| ATOM | 2216 | N | GLY | 294 | 24.845 | -6.136 | 69.781 | 1.00 | 28.83 |
| ATOM | 2217 | CA | GLY | 294 | 24.302 | -5.071 | 70.615 | 1.00 | 26.73 |
| ATOM | 2218 | C | GLY | 294 | 23.551 | -5.379 | 71.898 | 1.00 | 29.79 |
| ATOM | 2219 | O | GLY | 294 | 22.757 | -6.318 | 71.964 | 1.00 | 27.85 |
| ATOM | 2220 | N | GLY | 295 | 23.794 | -4.553 | 72.918 | 1.00 | 30.56 |
| ATOM | 2221 | CA | GLY | 295 | 23.136 | -4.722 | 74.204 | 1.00 | 33.01 |
| ATOM | 2222 | C | GLY | 295 | 21.628 | -4.539 | 74.144 | 1.00 | 34.05 |
| ATOM | 2223 | O | GLY | 295 | 20.927 | -4.810 | 75.107 | 1.00 | 34.93 |
| ATOM | 2224 | N | LYS | 296 | 21.124 | -4.058 | 73.016 | 1.00 | 35.19 |
| ATOM | 2225 | CA | LYS | 296 | 19.690 | -3.868 | 72.851 | 1.00 | 36.24 |
| ATOM | 2226 | CB | LYS | 296 | 19.419 | -2.988 | 71.626 | 1.00 | 38.05 |
| ATOM | 2227 | CG | LYS | 296 | 17.961 | -2.910 | 71.181 | 1.00 | 40.26 |
| ATOM | 2228 | CD | LYS | 296 | 17.122 | -2.093 | 72.141 | 1.00 | 43.32 |
| ATOM | 2229 | CE | LYS | 296 | 15.730 | -1.862 | 71.579 | 1.00 | 44.42 |
| ATOM | 2230 | NZ | LYS | 296 | 14.842 | -1.175 | 72.562 | 1.00 | 44.77 |
| ATOM | 2231 | C | LYS | 296 | 19.045 | -5.235 | 72.654 | 1.00 | 36.63 |

| | | | | | | | | | |
|---|---|---|---|---|---|---|---|---|---|
| ATOM | 2232 | O | LYS | 296 | 17.867 | -5.420 | 72.963 | 1.00 | 38.56 |
| ATOM | 2233 | N | TYR | 297 | 19.836 | -6.193 | 72.168 | 1.00 | 34.63 |
| ATOM | 2234 | CA | TYR | 297 | 19.346 | -7.539 | 71.890 | 1.00 | 33.22 |
| ATOM | 2235 | CB | TYR | 297 | 19.487 | -7.810 | 70.389 | 1.00 | 34.65 |
| ATOM | 2236 | CG | TYR | 297 | 19.073 | -6.631 | 69.535 | 1.00 | 36.28 |
| ATOM | 2237 | CD1 | TYR | 297 | 20.010 | -5.677 | 69.125 | 1.00 | 34.21 |
| ATOM | 2238 | CE1 | TYR | 297 | 19.622 | -4.548 | 68.404 | 1.00 | 36.22 |
| ATOM | 2239 | CD2 | TYR | 297 | 17.732 | -6.431 | 69.195 | 1.00 | 34.24 |
| ATOM | 2240 | CE2 | TYR | 297 | 17.330 | -5.305 | 68.476 | 1.00 | 35.71 |
| ATOM | 2241 | CZ | TYR | 297 | 18.280 | -4.368 | 68.082 | 1.00 | 37.38 |
| ATOM | 2242 | OH | TYR | 297 | 17.887 | -3.258 | 67.375 | 1.00 | 35.33 |
| ATOM | 2243 | C | TYR | 297 | 19.968 | -8.713 | 72.670 | 1.00 | 33.21 |
| ATOM | 2244 | O | TYR | 297 | 19.392 | -9.800 | 72.716 | 1.00 | 33.78 |
| ATOM | 2245 | N | MET | 298 | 21.126 | -8.504 | 73.283 | 1.00 | 31.19 |
| ATOM | 2246 | CA | MET | 298 | 21.803 | -9.576 | 74.005 | 1.00 | 30.16 |
| ATOM | 2247 | CB | MET | 298 | 23.075 | -9.038 | 74.644 | 1.00 | 30.05 |
| ATOM | 2248 | CG | MET | 298 | 23.957 | -10.104 | 75.231 | 1.00 | 26.86 |
| ATOM | 2249 | SD | MET | 298 | 25.486 | -9.405 | 75.850 | 1.00 | 32.83 |
| ATOM | 2250 | CE | MET | 298 | 26.409 | -9.201 | 74.338 | 1.00 | 29.59 |
| ATOM | 2251 | C | MET | 298 | 20.963 | -10.296 | 75.066 | 1.00 | 31.27 |
| ATOM | 2252 | O | MET | 298 | 20.882 | -11.529 | 75.077 | 1.00 | 29.78 |
| ATOM | 2253 | N | GLY | 299 | 20.353 | -9.530 | 75.963 | 1.00 | 30.40 |
| ATOM | 2254 | CA | GLY | 299 | 19.534 | -10.132 | 76.998 | 1.00 | 31.32 |
| ATOM | 2255 | C | GLY | 299 | 18.354 | -10.869 | 76.393 | 1.00 | 33.32 |
| ATOM | 2256 | O | GLY | 299 | 17.988 | -11.962 | 76.831 | 1.00 | 33.97 |
| ATOM | 2257 | N | GLU | 300 | 17.752 | -10.265 | 75.377 | 1.00 | 31.78 |
| ATOM | 2258 | CA | GLU | 300 | 16.617 | -10.874 | 74.707 | 1.00 | 31.93 |
| ATOM | 2259 | CB | GLU | 300 | 16.080 | -9.937 | 73.621 | 1.00 | 29.00 |
| ATOM | 2260 | CG | GLU | 300 | 14.877 | -10.486 | 72.881 | 1.00 | 32.60 |
| ATOM | 2261 | CD | GLU | 300 | 13.655 | -10.655 | 73.769 | 1.00 | 31.13 |
| ATOM | 2262 | OE1 | GLU | 300 | 12.629 | -11.144 | 73.265 | 1.00 | 34.55 |
| ATOM | 2263 | OE2 | GLU | 300 | 13.714 | -10.299 | 74.963 | 1.00 | 33.16 |
| ATOM | 2264 | C | GLU | 300 | 17.013 | -12.215 | 74.092 | 1.00 | 30.90 |
| ATOM | 2265 | O | GLU | 300 | 16.225 | -13.156 | 74.090 | 1.00 | 32.89 |
| ATOM | 2266 | N | LEU | 301 | 18.234 | -12.301 | 73.570 | 1.00 | 31.16 |

| | | | | | | | | | |
|---|---|---|---|---|---|---|---|---|---|
| ATOM | 2267 | CA | LEU | 301 | 18.714 | -13.546 | 72.973 | 1.00 | 28.93 |
| ATOM | 2268 | CB | LEU | 301 | 20.085 | -13.339 | 72.325 | 1.00 | 24.69 |
| ATOM | 2269 | CG | LEU | 301 | 20.152 | -12.667 | 70.952 | 1.00 | 24.17 |
| ATOM | 2270 | CD1 | LEU | 301 | 21.607 | -12.326 | 70.628 | 1.00 | 23.70 |
| ATOM | 2271 | CD2 | LEU | 301 | 19.560 | -13.598 | 69.886 | 1.00 | 23.13 |
| ATOM | 2272 | C | LEU | 301 | 18.814 | -14.616 | 74.056 | 1.00 | 29.42 |
| ATOM | 2273 | O | LEU | 301 | 18.408 | -15.761 | 73.853 | 1.00 | 32.03 |
| ATOM | 2274 | N | VAL | 302 | 19.365 | -14.239 | 75.204 | 1.00 | 28.73 |
| ATOM | 2275 | CA | VAL | 302 | 19.505 | -15.164 | 76.317 | 1.00 | 29.42 |
| ATOM | 2276 | CB | VAL | 302 | 20.265 | -14.510 | 77.497 | 1.00 | 26.51 |
| ATOM | 2277 | CG1 | VAL | 302 | 20.172 | -15.395 | 78.740 | 1.00 | 25.63 |
| ATOM | 2278 | CG2 | VAL | 302 | 21.731 | -14.301 | 77.117 | 1.00 | 25.98 |
| ATOM | 2279 | C | VAL | 302 | 18.127 | -15.624 | 76.795 | 1.00 | 31.88 |
| ATOM | 2280 | O | VAL | 302 | 17.934 | -16.795 | 77.112 | 1.00 | 32.71 |
| ATOM | 2281 | N | ARG | 303 | 17.171 | -14.703 | 76.835 | 1.00 | 32.91 |
| ATOM | 2282 | CA | ARG | 303 | 15.818 | -15.039 | 77.270 | 1.00 | 36.08 |
| ATOM | 2283 | CB | ARG | 303 | 14.910 | -13.802 | 77.250 | 1.00 | 35.86 |
| ATOM | 2284 | CG | ARG | 303 | 13.524 | -14.055 | 77.847 | 1.00 | 36.97 |
| ATOM | 2285 | CD | ARG | 303 | 12.660 | -12.802 | 77.833 | 1.00 | 39.15 |
| ATOM | 2286 | NE | ARG | 303 | 12.105 | -12.529 | 76.511 | 1.00 | 41.95 |
| ATOM | 2287 | CZ | ARG | 303 | 11.090 | -13.197 | 75.968 | 1.00 | 43.84 |
| ATOM | 2288 | NH1 | ARG | 303 | 10.502 | -14.182 | 76.631 | 1.00 | 42.47 |
| ATOM | 2289 | NH2 | ARG | 303 | 10.666 | -12.885 | 74.750 | 1.00 | 43.86 |
| ATOM | 2290 | C | ARG | 303 | 15.215 | -16.110 | 76.373 | 1.00 | 36.97 |
| ATOM | 2291 | O | ARG | 303 | 14.554 | -17.032 | 76.851 | 1.00 | 37.22 |
| ATOM | 2292 | N | LEU | 304 | 15.432 | -15.970 | 75.068 | 1.00 | 37.86 |
| ATOM | 2293 | CA | LEU | 304 | 14.914 | -16.924 | 74.103 | 1.00 | 37.63 |
| ATOM | 2294 | CB | LEU | 304 | 15.113 | -16.387 | 72.687 | 1.00 | 38.69 |
| ATOM | 2295 | CG | LEU | 304 | 13.944 | -15.590 | 72.104 | 1.00 | 40.35 |
| ATOM | 2296 | CD1 | LEU | 304 | 13.486 | -14.516 | 73.062 | 1.00 | 40.85 |
| ATOM | 2297 | CD2 | LEU | 304 | 14.378 | -14.986 | 70.785 | 1.00 | 42.07 |
| ATOM | 2298 | C | LEU | 304 | 15.602 | -18.272 | 74.262 | 1.00 | 37.69 |
| ATOM | 2299 | O | LEU | 304 | 14.978 | -19.324 | 74.120 | 1.00 | 38.84 |
| ATOM | 2300 | N | VAL | 305 | 16.893 | -18.238 | 74.558 | 1.00 | 36.28 |
| ATOM | 2301 | CA | VAL | 305 | 17.647 | -19.466 | 74.753 | 1.00 | 34.31 |

| | | | | | | | | | |
|---|---|---|---|---|---|---|---|---|---|
| ATOM | 2302 | CB | VAL | 305 | 19.148 | -19.184 | 74.908 | 1.00 | 32.24 |
| ATOM | 2303 | CG1 | VAL | 305 | 19.868 | -20.438 | 75.390 | 1.00 | 28.85 |
| ATOM | 2304 | CG2 | VAL | 305 | 19.717 | -18.713 | 73.578 | 1.00 | 29.80 |
| ATOM | 2305 | C | VAL | 305 | 17.153 | -20.158 | 76.012 | 1.00 | 35.48 |
| ATOM | 2306 | O | VAL | 305 | 17.079 | -21.389 | 76.070 | 1.00 | 34.47 |
| ATOM | 2307 | N | LEU | 306 | 16.820 | -19.362 | 77.023 | 1.00 | 34.14 |
| ATOM | 2308 | CA | LEU | 306 | 16.328 | -19.921 | 78.273 | 1.00 | 35.52 |
| ATOM | 2309 | CB | LEU | 306 | 16.257 | -18.841 | 79.353 | 1.00 | 32.11 |
| ATOM | 2310 | CG | LEU | 306 | 17.601 | -18.289 | 79.829 | 1.00 | 32.53 |
| ATOM | 2311 | CD1 | LEU | 306 | 17.359 | -17.326 | 80.964 | 1.00 | 33.54 |
| ATOM | 2312 | CD2 | LEU | 306 | 18.515 | -19.420 | 80.287 | 1.00 | 30.60 |
| ATOM | 2313 | C | LEU | 306 | 14.948 | -20.532 | 78.049 | 1.00 | 37.53 |
| ATOM | 2314 | O | LEU | 306 | 14.637 | -21.608 | 78.566 | 1.00 | 33.87 |
| ATOM | 2315 | N | LEU | 307 | 14.129 | -19.850 | 77.257 | 1.00 | 39.39 |
| ATOM | 2316 | CA | LEU | 307 | 12.787 | -20.336 | 76.971 | 1.00 | 41.43 |
| ATOM | 2317 | CB | LEU | 307 | 12.011 | -19.296 | 76.165 | 1.00 | 40.84 |
| ATOM | 2318 | CG | LEU | 307 | 10.932 | -18.527 | 76.935 | 1.00 | 43.43 |
| ATOM | 2319 | CD1 | LEU | 307 | 11.389 | -18.243 | 78.356 | 1.00 | 43.36 |
| ATOM | 2320 | CD2 | LEU | 307 | 10.610 | -17.233 | 76.197 | 1.00 | 41.75 |
| ATOM | 2321 | C | LEU | 307 | 12.802 | -21.674 | 76.239 | 1.00 | 42.39 |
| ATOM | 2322 | O | LEU | 307 | 11.974 | -22.537 | 76.514 | 1.00 | 42.90 |
| ATOM | 2323 | N | ARG | 308 | 13.729 | -21.860 | 75.306 | 1.00 | 42.02 |
| ATOM | 2324 | CA | ARG | 308 | 13.771 | -23.132 | 74.605 | 1.00 | 42.88 |
| ATOM | 2325 | CB | ARG | 308 | 14.765 | -23.125 | 73.445 | 1.00 | 43.55 |
| ATOM | 2326 | CG | ARG | 308 | 14.891 | -24.514 | 72.837 | 1.00 | 47.00 |
| ATOM | 2327 | CD | ARG | 308 | 15.908 | -24.626 | 71.729 | 1.00 | 49.25 |
| ATOM | 2328 | NE | ARG | 308 | 16.079 | -26.026 | 71.349 | 1.00 | 52.10 |
| ATOM | 2329 | CZ | ARG | 308 | 16.915 | -26.456 | 70.410 | 1.00 | 52.45 |
| ATOM | 2330 | NH1 | ARG | 308 | 17.663 | -25.591 | 69.739 | 1.00 | 54.77 |
| ATOM | 2331 | NH2 | ARG | 308 | 17.016 | -27.756 | 70.154 | 1.00 | 51.73 |
| ATOM | 2332 | C | ARG | 308 | 14.181 | -24.222 | 75.582 | 1.00 | 43.27 |
| ATOM | 2333 | O | ARG | 308 | 13.654 | -25.333 | 75.540 | 1.00 | 42.09 |
| ATOM | 2334 | N | LEU | 309 | 15.135 | -23.895 | 76.452 | 1.00 | 42.54 |
| ATOM | 2335 | CA | LEU | 309 | 15.627 | -24.837 | 77.447 | 1.00 | 42.29 |
| ATOM | 2336 | CB | LEU | 309 | 16.771 | -24.207 | 78.248 | 1.00 | 40.55 |

| | | | | | | | | | |
|---|---|---|---|---|---|---|---|---|---|
| ATOM | 2337 | CG | LEU | 309 | 18.193 | -24.656 | 77.886 | 1.00 | 39.65 |
| ATOM | 2338 | CD1 | LEU | 309 | 18.313 | -24.973 | 76.416 | 1.00 | 38.56 |
| ATOM | 2339 | CD2 | LEU | 309 | 19.171 | -23.569 | 78.284 | 1.00 | 37.67 |
| ATOM | 2340 | C | LEU | 309 | 14.515 | -25.302 | 78.379 | 1.00 | 42.66 |
| ATOM | 2341 | O | LEU | 309 | 14.509 | -26.450 | 78.818 | 1.00 | 41.33 |
| ATOM | 2342 | N | VAL | 310 | 13.570 | -24.416 | 78.676 | 1.00 | 44.27 |
| ATOM | 2343 | CA | VAL | 310 | 12.464 | -24.789 | 79.543 | 1.00 | 46.40 |
| ATOM | 2344 | CB | VAL | 310 | 11.711 | -23.546 | 80.111 | 1.00 | 46.06 |
| ATOM | 2345 | CG1 | VAL | 310 | 12.682 | -22.613 | 80.807 | 1.00 | 45.43 |
| ATOM | 2346 | CG2 | VAL | 310 | 10.976 | -22.825 | 79.014 | 1.00 | 48.29 |
| ATOM | 2347 | C | VAL | 310 | 11.479 | -25.666 | 78.769 | 1.00 | 48.00 |
| ATOM | 2348 | O | VAL | 310 | 10.952 | -26.638 | 79.311 | 1.00 | 47.71 |
| ATOM | 2349 | N | ASP | 311 | 11.242 | -25.333 | 77.501 | 1.00 | 49.58 |
| ATOM | 2350 | CA | ASP | 311 | 10.313 | -26.104 | 76.683 | 1.00 | 52.37 |
| ATOM | 2351 | CB | ASP | 311 | 9.978 | -25.365 | 75.382 | 1.00 | 54.70 |
| ATOM | 2352 | CG | ASP | 311 | 9.318 | -24.014 | 75.626 | 1.00 | 58.89 |
| ATOM | 2353 | OD1 | ASP | 311 | 8.742 | -23.808 | 76.719 | 1.00 | 60.74 |
| ATOM | 2354 | OD2 | ASP | 311 | 9.364 | -23.158 | 74.713 | 1.00 | 60.54 |
| ATOM | 2355 | C | ASP | 311 | 10.872 | -27.485 | 76.365 | 1.00 | 52.35 |
| ATOM | 2356 | O | ASP | 311 | 10.131 | -28.388 | 75.982 | 1.00 | 55.07 |
| ATOM | 2357 | N | GLU | 312 | 12.180 | -27.642 | 76.515 | 1.00 | 51.23 |
| ATOM | 2358 | CA | GLU | 312 | 12.828 | -28.926 | 76.279 | 1.00 | 51.12 |
| ATOM | 2359 | CB | GLU | 312 | 14.277 | -28.729 | 75.834 | 1.00 | 52.62 |
| ATOM | 2360 | CG | GLU | 312 | 14.445 | -28.141 | 74.448 | 1.00 | 57.13 |
| ATOM | 2361 | CD | GLU | 312 | 14.187 | -29.153 | 73.358 | 1.00 | 58.40 |
| ATOM | 2362 | OE1 | GLU | 312 | 14.831 | -30.222 | 73.385 | 1.00 | 59.31 |
| ATOM | 2363 | OE2 | GLU | 312 | 13.346 | -28.879 | 72.476 | 1.00 | 60.41 |
| ATOM | 2364 | C | GLU | 312 | 12.810 | -29.660 | 77.611 | 1.00 | 50.76 |
| ATOM | 2365 | O | GLU | 312 | 13.292 | -30.787 | 77.720 | 1.00 | 50.64 |
| ATOM | 2366 | N | ASN | 313 | 12.265 | -28.989 | 78.624 | 1.00 | 50.08 |
| ATOM | 2367 | CA | ASN | 313 | 12.154 | -29.533 | 79.974 | 1.00 | 51.37 |
| ATOM | 2368 | CB | ASN | 313 | 11.428 | -30.886 | 79.932 | 1.00 | 53.51 |
| ATOM | 2369 | CG | ASN | 313 | 10.846 | -31.275 | 81.271 | 1.00 | 55.73 |
| ATOM | 2370 | OD1 | ASN | 313 | 10.011 | -30.560 | 81.824 | 1.00 | 58.95 |
| ATOM | 2371 | ND2 | ASN | 313 | 11.281 | -32.415 | 81.803 | 1.00 | 59.16 |

| | | | | | | | | | |
|---|---|---|---|---|---|---|---|---|---|
| ATOM | 2372 | C | ASN | 313 | 13.524 | -29.693 | 80.635 | 1.00 | 50.00 |
| ATOM | 2373 | O | ASN | 313 | 13.733 | -30.595 | 81.447 | 1.00 | 50.40 |
| ATOM | 2374 | N | LEU | 314 | 14.449 | -28.799 | 80.296 | 1.00 | 48.35 |
| ATOM | 2375 | CA | LEU | 314 | 15.805 | -28.843 | 80.835 | 1.00 | 45.12 |
| ATOM | 2376 | CB | LEU | 314 | 16.819 | -28.785 | 79.688 | 1.00 | 44.25 |
| ATOM | 2377 | CG | LEU | 314 | 16.759 | -29.872 | 78.611 | 1.00 | 45.98 |
| ATOM | 2378 | CD1 | LEU | 314 | 17.619 | -29.465 | 77.416 | 1.00 | 43.63 |
| ATOM | 2379 | CD2 | LEU | 314 | 17.232 | -31.201 | 79.196 | 1.00 | 45.09 |
| ATOM | 2380 | C | LEU | 314 | 16.119 | -27.724 | 81.829 | 1.00 | 43.38 |
| ATOM | 2381 | O | LEU | 314 | 17.180 | -27.732 | 82.449 | 1.00 | 41.90 |
| ATOM | 2382 | N | LEU | 315 | 15.211 | -26.765 | 81.982 | 1.00 | 41.74 |
| ATOM | 2383 | CA | LEU | 315 | 15.446 | -25.645 | 82.899 | 1.00 | 42.39 |
| ATOM | 2384 | CB | LEU | 315 | 15.907 | -24.407 | 82.116 | 1.00 | 40.17 |
| ATOM | 2385 | CG | LEU | 315 | 17.243 | -23.721 | 82.428 | 1.00 | 39.81 |
| ATOM | 2386 | CD1 | LEU | 315 | 17.262 | -22.383 | 81.689 | 1.00 | 41.89 |
| ATOM | 2387 | CD2 | LEU | 315 | 17.421 | -23.482 | 83.920 | 1.00 | 37.58 |
| ATOM | 2388 | C | LEU | 315 | 14.198 | -25.278 | 83.694 | 1.00 | 42.28 |
| ATOM | 2389 | O | LEU | 315 | 13.103 | -25.214 | 83.144 | 1.00 | 40.83 |
| ATOM | 2390 | N | PHE | 316 | 14.377 | -25.021 | 84.986 | 1.00 | 43.70 |
| ATOM | 2391 | CA | PHE | 316 | 13.271 | -24.648 | 85.863 | 1.00 | 46.70 |
| ATOM | 2392 | CB | PHE | 316 | 12.717 | -23.278 | 85.459 | 1.00 | 47.06 |
| ATOM | 2393 | CG | PHE | 316 | 13.776 | -22.247 | 85.187 | 1.00 | 47.07 |
| ATOM | 2394 | CD1 | PHE | 316 | 14.824 | -22.051 | 86.082 | 1.00 | 47.24 |
| ATOM | 2395 | CD2 | PHE | 316 | 13.722 | -21.467 | 84.037 | 1.00 | 47.25 |
| ATOM | 2396 | CE1 | PHE | 316 | 15.803 | -21.094 | 85.835 | 1.00 | 46.12 |
| ATOM | 2397 | CE2 | PHE | 316 | 14.695 | -20.507 | 83.782 | 1.00 | 47.70 |
| ATOM | 2398 | CZ | PHE | 316 | 15.738 | -20.321 | 84.683 | 1.00 | 47.68 |
| ATOM | 2399 | C | PHE | 316 | 12.131 | -25.672 | 85.857 | 1.00 | 48.45 |
| ATOM | 2400 | O | PHE | 316 | 10.960 | -25.306 | 85.967 | 1.00 | 48.86 |
| ATOM | 2401 | N | HIS | 317 | 12.473 | -26.950 | 85.725 | 1.00 | 50.80 |
| ATOM | 2402 | CA | HIS | 317 | 11.469 | -28.009 | 85.712 | 1.00 | 53.83 |
| ATOM | 2403 | CB | HIS | 317 | 10.655 | -27.986 | 87.010 | 1.00 | 57.67 |
| ATOM | 2404 | CG | HIS | 317 | 11.496 | -27.985 | 88.246 | 1.00 | 61.10 |
| ATOM | 2405 | CD2 | HIS | 317 | 11.558 | -27.116 | 89.282 | 1.00 | 63.07 |
| ATOM | 2406 | ND1 | HIS | 317 | 12.430 | -28.965 | 88.509 | 1.00 | 62.35 |

| | | | | | | | | | |
|---|---|---|---|---|---|---|---|---|---|
| ATOM | 2407 | CE1 | HIS | 317 | 13.032 | -28.699 | 89.655 | 1.00 | 64.77 |
| ATOM | 2408 | NE2 | HIS | 317 | 12.521 | -27.582 | 90.144 | 1.00 | 65.99 |
| ATOM | 2409 | C | HIS | 317 | 10.521 | -27.859 | 84.534 | 1.00 | 53.57 |
| ATOM | 2410 | O | HIS | 317 | 9.429 | -28.425 | 84.537 | 1.00 | 53.60 |
| ATOM | 2411 | N | GLY | 318 | 10.939 | -27.090 | 83.534 | 1.00 | 52.50 |
| ATOM | 2412 | CA | GLY | 318 | 10.113 | -26.881 | 82.358 | 1.00 | 51.83 |
| ATOM | 2413 | C | GLY | 318 | 8.940 | -25.958 | 82.615 | 1.00 | 51.72 |
| ATOM | 2414 | O | GLY | 318 | 7.939 | -25.999 | 81.904 | 1.00 | 50.88 |
| ATOM | 2415 | N | GLU | 319 | 9.073 | -25.110 | 83.627 | 1.00 | 53.43 |
| ATOM | 2416 | CA | GLU | 319 | 8.014 | -24.182 | 83.996 | 1.00 | 55.73 |
| ATOM | 2417 | CB | GLU | 319 | 7.510 | -24.544 | 85.392 | 1.00 | 58.85 |
| ATOM | 2418 | CG | GLU | 319 | 6.145 | -23.998 | 85.761 | 1.00 | 63.60 |
| ATOM | 2419 | CD | GLU | 319 | 5.590 | -24.664 | 87.016 | 1.00 | 66.32 |
| ATOM | 2420 | OE1 | GLU | 319 | 6.206 | -24.527 | 88.100 | 1.00 | 65.47 |
| ATOM | 2421 | OE2 | GLU | 319 | 4.540 | -25.335 | 86.913 | 1.00 | 67.45 |
| ATOM | 2422 | C | GLU | 319 | 8.538 | -22.748 | 83.966 | 1.00 | 55.18 |
| ATOM | 2423 | O | GLU | 319 | 9.278 | -22.324 | 84.851 | 1.00 | 55.23 |
| ATOM | 2424 | N | ALA | 320 | 8.145 | -22.006 | 82.938 | 1.00 | 55.14 |
| ATOM | 2425 | CA | ALA | 320 | 8.585 | -20.630 | 82.780 | 1.00 | 55.95 |
| ATOM | 2426 | CB | ALA | 320 | 8.609 | -20.265 | 81.304 | 1.00 | 55.13 |
| ATOM | 2427 | C | ALA | 320 | 7.708 | -19.649 | 83.544 | 1.00 | 56.88 |
| ATOM | 2428 | O | ALA | 320 | 6.487 | -19.789 | 83.584 | 1.00 | 58.58 |
| ATOM | 2429 | N | SER | 321 | 8.344 | -18.648 | 84.141 | 1.00 | 57.00 |
| ATOM | 2430 | CA | SER | 321 | 7.644 | -17.625 | 84.902 | 1.00 | 56.57 |
| ATOM | 2431 | CB | SER | 321 | 8.649 | -16.808 | 85.705 | 1.00 | 56.74 |
| ATOM | 2432 | OG | SER | 321 | 8.013 | -15.725 | 86.349 | 1.00 | 57.41 |
| ATOM | 2433 | C | SER | 321 | 6.853 | -16.689 | 83.995 | 1.00 | 58.61 |
| ATOM | 2434 | O | SER | 321 | 7.054 | -16.665 | 82.783 | 1.00 | 58.41 |
| ATOM | 2435 | N | GLU | 322 | 5.955 | -15.914 | 84.595 | 1.00 | 60.41 |
| ATOM | 2436 | CA | GLU | 322 | 5.133 | -14.960 | 83.858 | 1.00 | 62.09 |
| ATOM | 2437 | CB | GLU | 322 | 4.171 | -14.254 | 84.819 | 1.00 | 65.34 |
| ATOM | 2438 | CG | GLU | 322 | 3.185 | -13.299 | 84.165 | 1.00 | 69.70 |
| ATOM | 2439 | CD | GLU | 322 | 2.075 | -14.020 | 83.418 | 1.00 | 73.68 |
| ATOM | 2440 | OE1 | GLU | 322 | 1.379 | -14.851 | 84.046 | 1.00 | 74.78 |
| ATOM | 2441 | OE2 | GLU | 322 | 1.896 | -13.751 | 82.208 | 1.00 | 75.02 |

| | | | | | | | | | |
|---|---|---|---|---|---|---|---|---|---|
| ATOM | 2442 | C | GLU | 322 | 6.047 | -13.929 | 83.204 | 1.00 | 61.24 |
| ATOM | 2443 | O | GLU | 322 | 5.913 | -13.612 | 82.022 | 1.00 | 60.81 |
| ATOM | 2444 | N | GLN | 323 | 6.987 | -13.420 | 83.991 | 1.00 | 60.42 |
| ATOM | 2445 | CA | GLN | 323 | 7.935 | -12.422 | 83.521 | 1.00 | 58.63 |
| ATOM | 2446 | CB | GLN | 323 | 8.729 | -11.863 | 84.700 | 1.00 | 59.77 |
| ATOM | 2447 | CG | GLN | 323 | 7.902 | -11.039 | 85.658 | 1.00 | 61.20 |
| ATOM | 2448 | CD | GLN | 323 | 8.690 | -10.608 | 86.873 | 1.00 | 63.03 |
| ATOM | 2449 | OE1 | GLN | 323 | 9.672 | -9.866 | 86.767 | 1.00 | 63.70 |
| ATOM | 2450 | NE2 | GLN | 323 | 8.266 | -11.074 | 88.044 | 1.00 | 64.05 |
| ATOM | 2451 | C | GLN | 323 | 8.904 | -12.955 | 82.478 | 1.00 | 56.96 |
| ATOM | 2452 | O | GLN | 323 | 9.244 | -12.255 | 81.526 | 1.00 | 56.89 |
| ATOM | 2453 | N | LEU | 324 | 9.351 | -14.190 | 82.652 | 1.00 | 53.93 |
| ATOM | 2454 | CA | LEU | 324 | 10.298 | -14.763 | 81.713 | 1.00 | 52.62 |
| ATOM | 2455 | CB | LEU | 324 | 10.745 | -16.151 | 82.180 | 1.00 | 51.22 |
| ATOM | 2456 | CG | LEU | 324 | 11.830 | -16.826 | 81.334 | 1.00 | 50.58 |
| ATOM | 2457 | CD1 | LEU | 324 | 13.076 | -15.952 | 81.299 | 1.00 | 49.50 |
| ATOM | 2458 | CD2 | LEU | 324 | 12.160 | -18.192 | 81.909 | 1.00 | 49.35 |
| ATOM | 2459 | C | LEU | 324 | 9.730 | -14.855 | 80.306 | 1.00 | 52.38 |
| ATOM | 2460 | O | LEU | 324 | 10.485 | -14.870 | 79.337 | 1.00 | 51.83 |
| ATOM | 2461 | N | ARG | 325 | 8.405 | -14.902 | 80.193 | 1.00 | 52.63 |
| ATOM | 2462 | CA | ARG | 325 | 7.759 | -15.015 | 78.887 | 1.00 | 53.00 |
| ATOM | 2463 | CB | ARG | 325 | 6.477 | -15.848 | 79.000 | 1.00 | 54.77 |
| ATOM | 2464 | CG | ARG | 325 | 6.585 | -17.005 | 79.985 | 1.00 | 58.57 |
| ATOM | 2465 | CD | ARG | 325 | 6.013 | -18.330 | 79.458 | 1.00 | 60.34 |
| ATOM | 2466 | NE | ARG | 325 | 6.881 | -18.961 | 78.464 | 1.00 | 62.28 |
| ATOM | 2467 | CZ | ARG | 325 | 6.953 | -20.273 | 78.249 | 1.00 | 62.81 |
| ATOM | 2468 | NH1 | ARG | 325 | 6.208 | -21.109 | 78.963 | 1.00 | 62.98 |
| ATOM | 2469 | NH2 | ARG | 325 | 7.769 | -20.752 | 77.317 | 1.00 | 62.50 |
| ATOM | 2470 | C | ARG | 325 | 7.430 | -13.663 | 78.266 | 1.00 | 52.20 |
| ATOM | 2471 | O | ARG | 325 | 6.835 | -13.595 | 77.194 | 1.00 | 51.65 |
| ATOM | 2472 | N | THR | 326 | 7.820 | -12.589 | 78.940 | 1.00 | 51.52 |
| ATOM | 2473 | CA | THR | 326 | 7.562 | -11.248 | 78.438 | 1.00 | 53.54 |
| ATOM | 2474 | CB | THR | 326 | 7.031 | -10.343 | 79.570 | 1.00 | 54.40 |
| ATOM | 2475 | OG1 | THR | 326 | 8.068 | -10.120 | 80.534 | 1.00 | 56.68 |
| ATOM | 2476 | CG2 | THR | 326 | 5.858 | -11.012 | 80.274 | 1.00 | 53.00 |

| | | | | | | | | | |
|---|---|---|---|---|---|---|---|---|---|
| ATOM | 2477 | C | THR | 326 | 8.853 | -10.655 | 77.850 | 1.00 | 54.00 |
| ATOM | 2478 | O | THR | 326 | 9.891 | -10.626 | 78.515 | 1.00 | 53.48 |
| ATOM | 2479 | N | ARG | 327 | 8.782 | -10.191 | 76.604 | 1.00 | 54.30 |
| ATOM | 2480 | CA | ARG | 327 | 9.948 | -9.628 | 75.923 | 1.00 | 55.25 |
| ATOM | 2481 | CB | ARG | 327 | 9.568 | -9.074 | 74.550 | 1.00 | 58.73 |
| ATOM | 2482 | CG | ARG | 327 | 9.050 | -10.101 | 73.572 | 1.00 | 62.94 |
| ATOM | 2483 | CD | ARG | 327 | 9.189 | -9.599 | 72.143 | 1.00 | 66.63 |
| ATOM | 2484 | NE | ARG | 327 | 8.462 | -10.454 | 71.213 | 1.00 | 70.25 |
| ATOM | 2485 | CZ | ARG | 327 | 7.136 | -10.522 | 71.154 | 1.00 | 72.29 |
| ATOM | 2486 | NH1 | ARG | 327 | 6.399 | -9.778 | 71.969 | 1.00 | 72.86 |
| ATOM | 2487 | NH2 | ARG | 327 | 6.546 | -11.338 | 70.288 | 1.00 | 73.24 |
| ATOM | 2488 | C | ARG | 327 | 10.660 | -8.529 | 76.688 | 1.00 | 53.79 |
| ATOM | 2489 | O | ARG | 327 | 10.027 | -7.690 | 77.326 | 1.00 | 55.10 |
| ATOM | 2490 | N | GLY | 328 | 11.986 | -8.535 | 76.604 | 1.00 | 50.97 |
| ATOM | 2491 | CA | GLY | 328 | 12.773 | -7.520 | 77.276 | 1.00 | 50.03 |
| ATOM | 2492 | C | GLY | 328 | 12.922 | -7.715 | 78.770 | 1.00 | 49.36 |
| ATOM | 2493 | O | GLY | 328 | 13.622 | -6.942 | 79.426 | 1.00 | 49.68 |
| ATOM | 2494 | N | ALA | 329 | 12.274 | -8.740 | 79.315 | 1.00 | 47.47 |
| ATOM | 2495 | CA | ALA | 329 | 12.354 | -9.007 | 80.749 | 1.00 | 46.93 |
| ATOM | 2496 | CB | ALA | 329 | 11.468 | -10.184 | 81.115 | 1.00 | 48.23 |
| ATOM | 2497 | C | ALA | 329 | 13.786 | -9.287 | 81.173 | 1.00 | 45.48 |
| ATOM | 2498 | O | ALA | 329 | 14.247 | -8.794 | 82.203 | 1.00 | 44.91 |
| ATOM | 2499 | N | PHE | 330 | 14.490 | -10.088 | 80.383 | 1.00 | 43.75 |
| ATOM | 2500 | CA | PHE | 330 | 15.870 | -10.392 | 80.710 | 1.00 | 42.95 |
| ATOM | 2501 | CB | PHE | 330 | 16.271 | -11.760 | 80.156 | 1.00 | 39.40 |
| ATOM | 2502 | CG | PHE | 330 | 17.478 | -12.350 | 80.829 | 1.00 | 36.90 |
| ATOM | 2503 | CD1 | PHE | 330 | 18.761 | -11.985 | 80.436 | 1.00 | 35.73 |
| ATOM | 2504 | CD2 | PHE | 330 | 17.330 | -13.241 | 81.893 | 1.00 | 35.23 |
| ATOM | 2505 | CE1 | PHE | 330 | 19.878 | -12.496 | 81.093 | 1.00 | 33.48 |
| ATOM | 2506 | CE2 | PHE | 330 | 18.443 | -13.759 | 82.558 | 1.00 | 31.61 |
| ATOM | 2507 | CZ | PHE | 330 | 19.716 | -13.387 | 82.160 | 1.00 | 33.39 |
| ATOM | 2508 | C | PHE | 330 | 16.752 | -9.292 | 80.130 | 1.00 | 43.51 |
| ATOM | 2509 | O | PHE | 330 | 17.202 | -9.373 | 78.986 | 1.00 | 44.11 |
| ATOM | 2510 | N | GLU | 331 | 16.962 | -8.254 | 80.935 | 1.00 | 43.95 |
| ATOM | 2511 | CA | GLU | 331 | 17.777 | -7.099 | 80.569 | 1.00 | 43.11 |

| | | | | | | | | | |
|---|---|---|---|---|---|---|---|---|---|
| ATOM | 2512 | CB | GLU | 331 | 17.767 | -6.068 | 81.697 | 1.00 | 46.19 |
| ATOM | 2513 | CG | GLU | 331 | 16.393 | -5.551 | 82.092 | 1.00 | 50.13 |
| ATOM | 2514 | CD | GLU | 331 | 16.458 | -4.651 | 83.316 | 1.00 | 53.54 |
| ATOM | 2515 | OE1 | GLU | 331 | 17.324 | -3.745 | 83.343 | 1.00 | 55.03 |
| ATOM | 2516 | OE2 | GLU | 331 | 15.646 | -4.846 | 84.247 | 1.00 | 53.56 |
| ATOM | 2517 | C | GLU | 331 | 19.216 | -7.511 | 80.310 | 1.00 | 42.02 |
| ATOM | 2518 | O | GLU | 331 | 19.742 | -8.411 | 80.968 | 1.00 | 42.05 |
| ATOM | 2519 | N | THR | 332 | 19.855 | -6.830 | 79.365 | 1.00 | 39.23 |
| ATOM | 2520 | CA | THR | 332 | 21.235 | -7.122 | 79.017 | 1.00 | 36.08 |
| ATOM | 2521 | CB | THR | 332 | 21.713 | -6.200 | 77.869 | 1.00 | 36.47 |
| ATOM | 2522 | OG1 | THR | 332 | 21.297 | -6.762 | 76.618 | 1.00 | 33.61 |
| ATOM | 2523 | CG2 | THR | 332 | 23.235 | -6.030 | 77.884 | 1.00 | 31.36 |
| ATOM | 2524 | C | THR | 332 | 22.159 | -6.987 | 80.219 | 1.00 | 35.73 |
| ATOM | 2525 | O | THR | 332 | 23.209 | -7.634 | 80.280 | 1.00 | 35.30 |
| ATOM | 2526 | N | ARG | 333 | 21.782 | -6.151 | 81.180 | 1.00 | 34.21 |
| ATOM | 2527 | CA | ARG | 333 | 22.632 | -6.003 | 82.353 | 1.00 | 34.18 |
| ATOM | 2528 | CB | ARG | 333 | 22.211 | -4.786 | 83.193 | 1.00 | 36.60 |
| ATOM | 2529 | CG | ARG | 333 | 20.830 | -4.854 | 83.835 | 1.00 | 39.58 |
| ATOM | 2530 | CD | ARG | 333 | 20.488 | -3.518 | 84.520 | 1.00 | 42.78 |
| ATOM | 2531 | NE | ARG | 333 | 19.264 | -3.590 | 85.316 | 1.00 | 45.29 |
| ATOM | 2532 | CZ | ARG | 333 | 19.205 | -4.039 | 86.567 | 1.00 | 47.32 |
| ATOM | 2533 | NH1 | ARG | 333 | 20.305 | -4.455 | 87.182 | 1.00 | 49.55 |
| ATOM | 2534 | NH2 | ARG | 333 | 18.042 | -4.080 | 87.205 | 1.00 | 48.70 |
| ATOM | 2535 | C | ARG | 333 | 22.609 | -7.298 | 83.181 | 1.00 | 31.65 |
| ATOM | 2536 | O | ARG | 333 | 23.584 | -7.625 | 83.863 | 1.00 | 31.61 |
| ATOM | 2537 | N | PHE | 334 | 21.513 | -8.049 | 83.105 | 1.00 | 31.01 |
| ATOM | 2538 | CA | PHE | 334 | 21.431 | -9.317 | 83.835 | 1.00 | 30.67 |
| ATOM | 2539 | CB | PHE | 334 | 20.048 | -9.967 | 83.678 | 1.00 | 30.39 |
| ATOM | 2540 | CG | PHE | 334 | 18.923 | -9.210 | 84.330 | 1.00 | 30.58 |
| ATOM | 2541 | CD1 | PHE | 334 | 19.170 | -8.214 | 85.269 | 1.00 | 29.37 |
| ATOM | 2542 | CD2 | PHE | 334 | 17.600 | -9.522 | 84.019 | 1.00 | 31.94 |
| ATOM | 2543 | CE1 | PHE | 334 | 18.113 | -7.539 | 85.891 | 1.00 | 31.67 |
| ATOM | 2544 | CE2 | PHE | 334 | 16.535 | -8.851 | 84.636 | 1.00 | 32.25 |
| ATOM | 2545 | CZ | PHE | 334 | 16.796 | -7.857 | 85.575 | 1.00 | 28.89 |
| ATOM | 2546 | C | PHE | 334 | 22.496 | -10.287 | 83.295 | 1.00 | 30.73 |

| | | | | | | | | | |
|---|---|---|---|---|---|---|---|---|---|
| ATOM | 2547 | O | PHE | 334 | 23.136 | -11.016 | 84.064 | 1.00 | 30.77 |
| ATOM | 2548 | N | VAL | 335 | 22.685 | -10.290 | 81.973 | 1.00 | 29.44 |
| ATOM | 2549 | CA | VAL | 335 | 23.672 | -11.165 | 81.350 | 1.00 | 30.61 |
| ATOM | 2550 | CB | VAL | 335 | 23.777 | -10.921 | 79.831 | 1.00 | 30.75 |
| ATOM | 2551 | CG1 | VAL | 335 | 24.774 | -11.898 | 79.216 | 1.00 | 32.48 |
| ATOM | 2552 | CG2 | VAL | 335 | 22.424 | -11.078 | 79.181 | 1.00 | 29.80 |
| ATOM | 2553 | C | VAL | 335 | 25.041 | -10.904 | 81.964 | 1.00 | 31.64 |
| ATOM | 2554 | O | VAL | 335 | 25.759 | -11.830 | 82.356 | 1.00 | 31.87 |
| ATOM | 2555 | N | SER | 336 | 25.382 | -9.623 | 82.048 | 1.00 | 33.23 |
| ATOM | 2556 | CA | SER | 336 | 26.655 | -9.173 | 82.593 | 1.00 | 32.42 |
| ATOM | 2557 | CB | SER | 336 | 26.778 | -7.660 | 82.384 | 1.00 | 33.94 |
| ATOM | 2558 | OG | SER | 336 | 28.080 | -7.204 | 82.682 | 1.00 | 38.27 |
| ATOM | 2559 | C | SER | 336 | 26.793 | -9.524 | 84.078 | 1.00 | 32.82 |
| ATOM | 2560 | O | SER | 336 | 27.863 | -9.917 | 84.529 | 1.00 | 33.76 |
| ATOM | 2561 | N | GLN | 337 | 25.711 | -9.389 | 84.839 | 1.00 | 32.64 |
| ATOM | 2562 | CA | GLN | 337 | 25.753 | -9.715 | 86.260 | 1.00 | 34.83 |
| ATOM | 2563 | CB | GLN | 337 | 24.480 | -9.233 | 86.958 | 1.00 | 37.43 |
| ATOM | 2564 | CG | GLN | 337 | 24.339 | -7.721 | 86.972 | 1.00 | 42.29 |
| ATOM | 2565 | CD | GLN | 337 | 22.984 | -7.260 | 87.471 | 1.00 | 44.59 |
| ATOM | 2566 | OE1 | GLN | 337 | 22.710 | -6.062 | 87.525 | 1.00 | 46.49 |
| ATOM | 2567 | NE2 | GLN | 337 | 22.128 | -8.209 | 87.835 | 1.00 | 43.79 |
| ATOM | 2568 | C | GLN | 337 | 25.899 | -11.217 | 86.447 | 1.00 | 33.66 |
| ATOM | 2569 | O | GLN | 337 | 26.663 | -11.674 | 87.297 | 1.00 | 35.28 |
| ATOM | 2570 | N | VAL | 338 | 25.159 | -11.983 | 85.655 | 1.00 | 31.29 |
| ATOM | 2571 | CA | VAL | 338 | 25.236 | -13.432 | 85.743 | 1.00 | 29.21 |
| ATOM | 2572 | CB | VAL | 338 | 24.326 | -14.102 | 84.690 | 1.00 | 28.27 |
| ATOM | 2573 | CG1 | VAL | 338 | 24.687 | -15.571 | 84.525 | 1.00 | 27.17 |
| ATOM | 2574 | CG2 | VAL | 338 | 22.877 | -13.984 | 85.129 | 1.00 | 26.99 |
| ATOM | 2575 | C | VAL | 338 | 26.678 | -13.877 | 85.547 | 1.00 | 27.35 |
| ATOM | 2576 | O | VAL | 338 | 27.176 | -14.722 | 86.284 | 1.00 | 26.69 |
| ATOM | 2577 | N | GLU | 339 | 27.361 | -13.283 | 84.576 | 1.00 | 27.29 |
| ATOM | 2578 | CA | GLU | 339 | 28.747 | -13.657 | 84.314 | 1.00 | 27.15 |
| ATOM | 2579 | CB | GLU | 339 | 29.136 | -13.303 | 82.871 | 1.00 | 27.02 |
| ATOM | 2580 | CG | GLU | 339 | 28.404 | -14.185 | 81.843 | 1.00 | 30.73 |
| ATOM | 2581 | CD | GLU | 339 | 28.942 | -14.063 | 80.425 | 1.00 | 30.33 |

| | | | | | | | | | |
|---|---|---|---|---|---|---|---|---|---|
| ATOM | 2582 | OE1 | GLU | 339 | 30.121 | -14.414 | 80.185 | 1.00 | 34.73 |
| ATOM | 2583 | OE2 | GLU | 339 | 28.179 | -13.619 | 79.548 | 1.00 | 29.50 |
| ATOM | 2584 | C | GLU | 339 | 29.749 | -13.085 | 85.311 | 1.00 | 26.93 |
| ATOM | 2585 | O | GLU | 339 | 30.940 | -13.345 | 85.209 | 1.00 | 27.69 |
| ATOM | 2586 | N | SER | 340 | 29.264 | -12.320 | 86.285 | 1.00 | 27.55 |
| ATOM | 2587 | CA | SER | 340 | 30.140 | -11.763 | 87.318 | 1.00 | 28.61 |
| ATOM | 2588 | CB | SER | 340 | 29.741 | -10.323 | 87.667 | 1.00 | 29.40 |
| ATOM | 2589 | OG | SER | 340 | 29.800 | -9.485 | 86.528 | 1.00 | 35.97 |
| ATOM | 2590 | C | SER | 340 | 30.029 | -12.615 | 88.583 | 1.00 | 27.94 |
| ATOM | 2591 | O | SER | 340 | 30.811 | -12.448 | 89.526 | 1.00 | 24.04 |
| ATOM | 2592 | N | ASP | 341 | 29.042 | -13.511 | 88.600 | 1.00 | 28.02 |
| ATOM | 2593 | CA | ASP | 341 | 28.812 | -14.387 | 89.748 | 1.00 | 29.66 |
| ATOM | 2594 | CB | ASP | 341 | 27.808 | -15.490 | 89.393 | 1.00 | 30.94 |
| ATOM | 2595 | CG | ASP | 341 | 27.296 | -16.227 | 90.620 | 1.00 | 33.11 |
| ATOM | 2596 | OD1 | ASP | 341 | 26.289 | -15.778 | 91.217 | 1.00 | 28.78 |
| ATOM | 2597 | OD2 | ASP | 341 | 27.918 | -17.247 | 90.991 | 1.00 | 32.82 |
| ATOM | 2598 | C | ASP | 341 | 30.137 | -15.003 | 90.163 | 1.00 | 30.38 |
| ATOM | 2599 | O | ASP | 341 | 30.853 | -15.564 | 89.342 | 1.00 | 30.59 |
| ATOM | 2600 | N | THR | 342 | 30.466 | -14.886 | 91.443 | 1.00 | 33.59 |
| ATOM | 2601 | CA | THR | 342 | 31.729 | -15.405 | 91.953 | 1.00 | 37.01 |
| ATOM | 2602 | CB | THR | 342 | 32.013 | -14.836 | 93.350 | 1.00 | 38.81 |
| ATOM | 2603 | OG1 | THR | 342 | 31.012 | -15.304 | 94.265 | 1.00 | 43.90 |
| ATOM | 2604 | CG2 | THR | 342 | 31.972 | -13.316 | 93.317 | 1.00 | 35.79 |
| ATOM | 2605 | C | THR | 342 | 31.780 | -16.929 | 92.027 | 1.00 | 37.87 |
| ATOM | 2606 | O | THR | 342 | 32.853 | -17.514 | 92.191 | 1.00 | 39.64 |
| ATOM | 2607 | N | GLY | 343 | 30.625 | -17.568 | 91.894 | 1.00 | 36.81 |
| ATOM | 2608 | CA | GLY | 343 | 30.578 | -19.018 | 91.970 | 1.00 | 39.26 |
| ATOM | 2609 | C | GLY | 343 | 29.631 | -19.515 | 93.053 | 1.00 | 38.98 |
| ATOM | 2610 | O | GLY | 343 | 29.293 | -20.695 | 93.090 | 1.00 | 39.46 |
| ATOM | 2611 | N | ASP | 344 | 29.204 | -18.615 | 93.935 | 1.00 | 38.20 |
| ATOM | 2612 | CA | ASP | 344 | 28.287 | -18.980 | 95.005 | 1.00 | 39.74 |
| ATOM | 2613 | CB | ASP | 344 | 28.480 | -18.071 | 96.231 | 1.00 | 39.14 |
| ATOM | 2614 | CG | ASP | 344 | 28.267 | -16.595 | 95.928 | 1.00 | 41.19 |
| ATOM | 2615 | OD1 | ASP | 344 | 27.733 | -16.256 | 94.848 | 1.00 | 39.57 |
| ATOM | 2616 | OD2 | ASP | 344 | 28.627 | -15.767 | 96.794 | 1.00 | 42.27 |

| | | | | | | | | | |
|---|---|---|---|---|---|---|---|---|---|
| ATOM | 2617 | C | ASP | 344 | 26.842 | -18.926 | 94.516 | 1.00 | 40.25 |
| ATOM | 2618 | O | ASP | 344 | 25.904 | -19.235 | 95.257 | 1.00 | 39.36 |
| ATOM | 2619 | N | ARG | 345 | 26.680 | -18.525 | 93.259 | 1.00 | 38.45 |
| ATOM | 2620 | CA | ARG | 345 | 25.374 | -18.449 | 92.618 | 1.00 | 37.30 |
| ATOM | 2621 | CB | ARG | 345 | 24.738 | -19.847 | 92.587 | 1.00 | 37.49 |
| ATOM | 2622 | CG | ARG | 345 | 25.657 | -20.935 | 92.044 | 1.00 | 38.81 |
| ATOM | 2623 | CD | ARG | 345 | 24.976 | -22.301 | 92.046 | 1.00 | 40.19 |
| ATOM | 2624 | NE | ARG | 345 | 25.790 | -23.327 | 91.397 | 1.00 | 42.18 |
| ATOM | 2625 | CZ | ARG | 345 | 26.730 | -24.051 | 91.999 | 1.00 | 43.19 |
| ATOM | 2626 | NH1 | ARG | 345 | 26.990 | -23.880 | 93.288 | 1.00 | 43.31 |
| ATOM | 2627 | NH2 | ARG | 345 | 27.421 | -24.947 | 91.302 | 1.00 | 40.56 |
| ATOM | 2628 | C | ARG | 345 | 24.397 | -17.456 | 93.246 | 1.00 | 37.06 |
| ATOM | 2629 | O | ARG | 345 | 23.231 | -17.395 | 92.837 | 1.00 | 35.44 |
| ATOM | 2630 | N | LYS | 346 | 24.855 | -16.681 | 94.228 | 1.00 | 37.09 |
| ATOM | 2631 | CA | LYS | 346 | 23.977 | -15.704 | 94.876 | 1.00 | 39.61 |
| ATOM | 2632 | CB | LYS | 346 | 24.710 | -14.964 | 96.005 | 1.00 | 43.18 |
| ATOM | 2633 | CG | LYS | 346 | 25.084 | -15.826 | 97.214 | 1.00 | 47.92 |
| ATOM | 2634 | CD | LYS | 346 | 25.835 | -15.009 | 98.285 | 1.00 | 50.48 |
| ATOM | 2635 | CE | LYS | 346 | 26.274 | -15.887 | 99.466 | 1.00 | 53.20 |
| ATOM | 2636 | NZ | LYS | 346 | 27.039 | -15.136 | 100.520 | 1.00 | 54.15 |
| ATOM | 2637 | C | LYS | 346 | 23.467 | -14.690 | 93.858 | 1.00 | 39.25 |
| ATOM | 2638 | O | LYS | 346 | 22.271 | -14.400 | 93.795 | 1.00 | 38.51 |
| ATOM | 2639 | N | GLN | 347 | 24.384 | -14.158 | 93.055 | 1.00 | 40.01 |
| ATOM | 2640 | CA | GLN | 347 | 24.036 | -13.169 | 92.037 | 1.00 | 39.62 |
| ATOM | 2641 | CB | GLN | 347 | 25.301 | -12.725 | 91.290 | 1.00 | 44.30 |
| ATOM | 2642 | CG | GLN | 347 | 25.117 | -11.507 | 90.403 | 1.00 | 50.12 |
| ATOM | 2643 | CD | GLN | 347 | 24.996 | -10.214 | 91.196 | 1.00 | 54.40 |
| ATOM | 2644 | OE1 | GLN | 347 | 24.699 | -9.153 | 90.637 | 1.00 | 57.36 |
| ATOM | 2645 | NE2 | GLN | 347 | 25.234 | -10.295 | 92.501 | 1.00 | 55.02 |
| ATOM | 2646 | C | GLN | 347 | 23.015 | -13.735 | 91.046 | 1.00 | 36.71 |
| ATOM | 2647 | O | GLN | 347 | 22.012 | -13.087 | 90.732 | 1.00 | 35.38 |
| ATOM | 2648 | N | ILE | 348 | 23.264 | -14.949 | 90.563 | 1.00 | 33.61 |
| ATOM | 2649 | CA | ILE | 348 | 22.360 | -15.579 | 89.610 | 1.00 | 30.26 |
| ATOM | 2650 | CB | ILE | 348 | 22.946 | -16.906 | 89.103 | 1.00 | 31.09 |
| ATOM | 2651 | CG2 | ILE | 348 | 21.983 | -17.561 | 88.102 | 1.00 | 24.14 |

| | | | | | | | | | |
|---|---|---|---|---|---|---|---|---|---|
| ATOM | 2652 | CG1 | ILE | 348 | 24.315 | -16.641 | 88.467 | 1.00 | 24.89 |
| ATOM | 2653 | CD1 | ILE | 348 | 25.016 | -17.870 | 87.989 | 1.00 | 26.20 |
| ATOM | 2654 | C | ILE | 348 | 20.990 | -15.836 | 90.231 | 1.00 | 32.47 |
| ATOM | 2655 | O | ILE | 348 | 19.946 | -15.578 | 89.607 | 1.00 | 28.48 |
| ATOM | 2656 | N | TYR | 349 | 20.996 | -16.330 | 91.468 | 1.00 | 33.64 |
| ATOM | 2657 | CA | TYR | 349 | 19.757 | -16.622 | 92.173 | 1.00 | 33.94 |
| ATOM | 2658 | CB | TYR | 349 | 20.023 | -17.189 | 93.566 | 1.00 | 35.19 |
| ATOM | 2659 | CG | TYR | 349 | 18.728 | -17.513 | 94.273 | 1.00 | 35.54 |
| ATOM | 2660 | CD1 | TYR | 349 | 18.085 | -18.737 | 94.064 | 1.00 | 35.44 |
| ATOM | 2661 | CE1 | TYR | 349 | 16.847 | -19.009 | 94.647 | 1.00 | 35.96 |
| ATOM | 2662 | CD2 | TYR | 349 | 18.100 | -16.569 | 95.083 | 1.00 | 34.28 |
| ATOM | 2663 | CE2 | TYR | 349 | 16.860 | -16.833 | 95.665 | 1.00 | 34.50 |
| ATOM | 2664 | CZ | TYR | 349 | 16.242 | -18.053 | 95.441 | 1.00 | 34.82 |
| ATOM | 2665 | OH | TYR | 349 | 15.007 | -18.305 | 95.990 | 1.00 | 39.44 |
| ATOM | 2666 | C | TYR | 349 | 18.888 | -15.390 | 92.339 | 1.00 | 35.45 |
| ATOM | 2667 | O | TYR | 349 | 17.698 | -15.419 | 92.042 | 1.00 | 37.11 |
| ATOM | 2668 | N | ASN | 350 | 19.475 | -14.312 | 92.846 | 1.00 | 37.18 |
| ATOM | 2669 | CA | ASN | 350 | 18.722 | -13.082 | 93.049 | 1.00 | 38.47 |
| ATOM | 2670 | CB | ASN | 350 | 19.617 | -11.985 | 93.630 | 1.00 | 40.65 |
| ATOM | 2671 | CG | ASN | 350 | 20.014 | -12.263 | 95.065 | 1.00 | 45.75 |
| ATOM | 2672 | OD1 | ASN | 350 | 19.176 | -12.638 | 95.893 | 1.00 | 45.11 |
| ATOM | 2673 | ND2 | ASN | 350 | 21.298 | -12.075 | 95.373 | 1.00 | 46.81 |
| ATOM | 2674 | C | ASN | 350 | 18.085 | -12.585 | 91.768 | 1.00 | 37.56 |
| ATOM | 2675 | O | ASN | 350 | 16.924 | -12.186 | 91.769 | 1.00 | 40.92 |
| ATOM | 2676 | N | ILE | 351 | 18.839 | -12.601 | 90.673 | 1.00 | 37.62 |
| ATOM | 2677 | CA | ILE | 351 | 18.310 | -12.139 | 89.395 | 1.00 | 37.09 |
| ATOM | 2678 | CB | ILE | 351 | 19.401 | -12.130 | 88.308 | 1.00 | 38.11 |
| ATOM | 2679 | CG2 | ILE | 351 | 18.771 | -11.955 | 86.938 | 1.00 | 37.56 |
| ATOM | 2680 | CG1 | ILE | 351 | 20.400 | -11.004 | 88.588 | 1.00 | 38.11 |
| ATOM | 2681 | CD1 | ILE | 351 | 21.726 | -11.178 | 87.879 | 1.00 | 36.24 |
| ATOM | 2682 | C | ILE | 351 | 17.144 | -12.997 | 88.921 | 1.00 | 36.57 |
| ATOM | 2683 | O | ILE | 351 | 16.120 | -12.474 | 88.479 | 1.00 | 38.22 |
| ATOM | 2684 | N | LEU | 352 | 17.291 | -14.314 | 89.012 | 1.00 | 35.96 |
| ATOM | 2685 | CA | LEU | 352 | 16.219 | -15.206 | 88.577 | 1.00 | 36.28 |
| ATOM | 2686 | CB | LEU | 352 | 16.740 | -16.640 | 88.443 | 1.00 | 32.41 |

| | | | | | | | | | |
|---|---|---|---|---|---|---|---|---|---|
| ATOM | 2687 | CG | LEU | 352 | 17.845 | -16.828 | 87.395 | 1.00 | 30.66 |
| ATOM | 2688 | CD1 | LEU | 352 | 18.465 | -18.226 | 87.496 | 1.00 | 25.83 |
| ATOM | 2689 | CD2 | LEU | 352 | 17.262 | -16.597 | 86.025 | 1.00 | 27.66 |
| ATOM | 2690 | C | LEU | 352 | 15.039 | -15.156 | 89.547 | 1.00 | 37.27 |
| ATOM | 2691 | O | LEU | 352 | 13.896 | -15.356 | 89.145 | 1.00 | 38.32 |
| ATOM | 2692 | N | SER | 353 | 15.322 | -14.888 | 90.819 | 1.00 | 39.41 |
| ATOM | 2693 | CA | SER | 353 | 14.279 | -14.794 | 91.838 | 1.00 | 42.13 |
| ATOM | 2694 | CB | SER | 353 | 14.893 | -14.708 | 93.237 | 1.00 | 43.72 |
| ATOM | 2695 | OG | SER | 353 | 13.883 | -14.546 | 94.224 | 1.00 | 48.17 |
| ATOM | 2696 | C | SER | 353 | 13.431 | -13.557 | 91.590 | 1.00 | 43.61 |
| ATOM | 2697 | O | SER | 353 | 12.229 | -13.552 | 91.858 | 1.00 | 42.99 |
| ATOM | 2698 | N | THR | 354 | 14.066 | -12.506 | 91.081 | 1.00 | 44.80 |
| ATOM | 2699 | CA | THR | 354 | 13.363 | -11.267 | 90.785 | 1.00 | 46.06 |
| ATOM | 2700 | CB | THR | 354 | 14.356 | -10.122 | 90.497 | 1.00 | 47.48 |
| ATOM | 2701 | OG1 | THR | 354 | 15.100 | -9.820 | 91.687 | 1.00 | 47.39 |
| ATOM | 2702 | CG2 | THR | 354 | 13.615 | -8.877 | 90.034 | 1.00 | 47.87 |
| ATOM | 2703 | C | THR | 354 | 12.446 | -11.455 | 89.579 | 1.00 | 46.06 |
| ATOM | 2704 | O | THR | 354 | 11.443 | -10.757 | 89.436 | 1.00 | 47.23 |
| ATOM | 2705 | N | LEU | 355 | 12.788 | -12.406 | 88.717 | 1.00 | 46.03 |
| ATOM | 2706 | CA | LEU | 355 | 11.983 | -12.679 | 87.533 | 1.00 | 46.26 |
| ATOM | 2707 | CB | LEU | 355 | 12.875 | -13.157 | 86.390 | 1.00 | 46.43 |
| ATOM | 2708 | CG | LEU | 355 | 14.030 | -12.210 | 86.063 | 1.00 | 46.85 |
| ATOM | 2709 | CD1 | LEU | 355 | 14.861 | -12.813 | 84.950 | 1.00 | 47.00 |
| ATOM | 2710 | CD2 | LEU | 355 | 13.497 | -10.844 | 85.660 | 1.00 | 45.99 |
| ATOM | 2711 | C | LEU | 355 | 10.908 | -13.722 | 87.821 | 1.00 | 46.88 |
| ATOM | 2712 | O | LEU | 355 | 10.370 | -14.346 | 86.902 | 1.00 | 47.28 |
| ATOM | 2713 | N | GLY | 356 | 10.609 | -13.912 | 89.105 | 1.00 | 47.29 |
| ATOM | 2714 | CA | GLY | 356 | 9.586 | -14.858 | 89.511 | 1.00 | 44.74 |
| ATOM | 2715 | C | GLY | 356 | 9.959 | -16.321 | 89.396 | 1.00 | 44.45 |
| ATOM | 2716 | O | GLY | 356 | 9.097 | -17.163 | 89.146 | 1.00 | 45.09 |
| ATOM | 2717 | N | LEU | 357 | 11.235 | -16.635 | 89.575 | 1.00 | 43.26 |
| ATOM | 2718 | CA | LEU | 357 | 11.681 | -18.018 | 89.485 | 1.00 | 41.29 |
| ATOM | 2719 | CB | LEU | 357 | 12.653 | -18.187 | 88.310 | 1.00 | 42.15 |
| ATOM | 2720 | CG | LEU | 357 | 12.171 | -17.833 | 86.896 | 1.00 | 41.21 |
| ATOM | 2721 | CD1 | LEU | 357 | 13.366 | -17.781 | 85.972 | 1.00 | 39.61 |

| | | | | | | | | | |
|---|---|---|---|---|---|---|---|---|---|
| ATOM | 2722 | CD2 | LEU | 357 | 11.153 | -18.849 | 86.393 | 1.00 | 39.50 |
| ATOM | 2723 | C | LEU | 357 | 12.361 | -18.455 | 90.780 | 1.00 | 40.57 |
| ATOM | 2724 | O | LEU | 357 | 12.780 | -17.627 | 91.590 | 1.00 | 38.53 |
| ATOM | 2725 | N | ARG | 358 | 12.448 | -19.766 | 90.970 | 1.00 | 39.68 |
| ATOM | 2726 | CA | ARG | 358 | 13.092 | -20.355 | 92.139 | 1.00 | 40.04 |
| ATOM | 2727 | CB | ARG | 358 | 12.048 | -20.916 | 93.112 | 1.00 | 42.61 |
| ATOM | 2728 | CG | ARG | 358 | 11.172 | -19.845 | 93.760 | 1.00 | 46.08 |
| ATOM | 2729 | CD | ARG | 358 | 12.019 | -18.871 | 94.560 | 1.00 | 49.74 |
| ATOM | 2730 | NE | ARG | 358 | 11.355 | -17.588 | 94.772 | 1.00 | 55.41 |
| ATOM | 2731 | CZ | ARG | 358 | 10.588 | -17.293 | 95.816 | 1.00 | 58.08 |
| ATOM | 2732 | NH1 | ARG | 358 | 10.376 | -18.195 | 96.771 | 1.00 | 59.09 |
| ATOM | 2733 | NH2 | ARG | 358 | 10.035 | -16.087 | 95.906 | 1.00 | 58.98 |
| ATOM | 2734 | C | ARG | 358 | 13.954 | -21.471 | 91.576 | 1.00 | 38.39 |
| ATOM | 2735 | O | ARG | 358 | 13.569 | -22.641 | 91.586 | 1.00 | 37.47 |
| ATOM | 2736 | N | PRO | 359 | 15.140 | -21.109 | 91.065 | 1.00 | 36.51 |
| ATOM | 2737 | CD | PRO | 359 | 15.664 | -19.728 | 91.087 | 1.00 | 36.88 |
| ATOM | 2738 | CA | PRO | 359 | 16.123 | -22.006 | 90.461 | 1.00 | 34.17 |
| ATOM | 2739 | CB | PRO | 359 | 17.035 | -21.039 | 89.722 | 1.00 | 35.29 |
| ATOM | 2740 | CG | PRO | 359 | 17.135 | -19.925 | 90.703 | 1.00 | 34.03 |
| ATOM | 2741 | C | PRO | 359 | 16.915 | -22.872 | 91.416 | 1.00 | 33.10 |
| ATOM | 2742 | O | PRO | 359 | 17.140 | -22.520 | 92.566 | 1.00 | 31.20 |
| ATOM | 2743 | N | SER | 360 | 17.365 | -24.004 | 90.899 | 1.00 | 33.97 |
| ATOM | 2744 | CA | SER | 360 | 18.183 | -24.931 | 91.658 | 1.00 | 34.21 |
| ATOM | 2745 | CB | SER | 360 | 17.912 | -26.363 | 91.210 | 1.00 | 34.53 |
| ATOM | 2746 | OG | SER | 360 | 18.287 | -26.530 | 89.851 | 1.00 | 33.54 |
| ATOM | 2747 | C | SER | 360 | 19.618 | -24.568 | 91.307 | 1.00 | 34.99 |
| ATOM | 2748 | O | SER | 360 | 19.855 | -23.673 | 90.495 | 1.00 | 35.49 |
| ATOM | 2749 | N | THR | 361 | 20.564 | -25.267 | 91.920 | 1.00 | 34.70 |
| ATOM | 2750 | CA | THR | 361 | 21.977 | -25.048 | 91.673 | 1.00 | 36.89 |
| ATOM | 2751 | CB | THR | 361 | 22.838 | -26.003 | 92.535 | 1.00 | 36.99 |
| ATOM | 2752 | OG1 | THR | 361 | 22.828 | -25.558 | 93.898 | 1.00 | 38.93 |
| ATOM | 2753 | CG2 | THR | 361 | 24.260 | -26.041 | 92.033 | 1.00 | 38.24 |
| ATOM | 2754 | C | THR | 361 | 22.303 | -25.291 | 90.201 | 1.00 | 37.14 |
| ATOM | 2755 | O | THR | 361 | 23.142 | -24.606 | 89.616 | 1.00 | 37.81 |
| ATOM | 2756 | N | THR | 362 | 21.635 | -26.273 | 89.612 | 1.00 | 35.92 |

| | | | | | | | | | |
|---|---|---|---|---|---|---|---|---|---|
| ATOM | 2757 | CA | THR | 362 | 21.865 | -26.614 | 88.223 | 1.00 | 34.91 |
| ATOM | 2758 | CB | THR | 362 | 21.369 | -28.037 | 87.914 | 1.00 | 36.12 |
| ATOM | 2759 | OG1 | THR | 362 | 19.969 | -28.117 | 88.199 | 1.00 | 40.45 |
| ATOM | 2760 | CG2 | THR | 362 | 22.113 | -29.063 | 88.771 | 1.00 | 34.62 |
| ATOM | 2761 | C | THR | 362 | 21.181 | -25.626 | 87.292 | 1.00 | 33.53 |
| ATOM | 2762 | O | THR | 362 | 21.684 | -25.360 | 86.205 | 1.00 | 33.46 |
| ATOM | 2763 | N | ASP | 363 | 20.034 | -25.091 | 87.698 | 1.00 | 31.06 |
| ATOM | 2764 | CA | ASP | 363 | 19.355 | -24.115 | 86.860 | 1.00 | 32.46 |
| ATOM | 2765 | CB | ASP | 363 | 18.018 | -23.690 | 87.468 | 1.00 | 34.45 |
| ATOM | 2766 | CG | ASP | 363 | 16.964 | -24.783 | 87.409 | 1.00 | 37.91 |
| ATOM | 2767 | OD1 | ASP | 363 | 16.889 | -25.504 | 86.388 | 1.00 | 38.99 |
| ATOM | 2768 | OD2 | ASP | 363 | 16.194 | -24.907 | 88.385 | 1.00 | 38.23 |
| ATOM | 2769 | C | ASP | 363 | 20.254 | -22.878 | 86.718 | 1.00 | 32.88 |
| ATOM | 2770 | O | ASP | 363 | 20.419 | -22.331 | 85.629 | 1.00 | 30.65 |
| ATOM | 2771 | N | CYS | 364 | 20.833 | -22.451 | 87.836 | 1.00 | 33.86 |
| ATOM | 2772 | CA | CYS | 364 | 21.712 | -21.292 | 87.860 | 1.00 | 32.22 |
| ATOM | 2773 | CB | CYS | 364 | 22.186 | -21.015 | 89.289 | 1.00 | 31.35 |
| ATOM | 2774 | SG | CYS | 364 | 20.915 | -20.338 | 90.389 | 1.00 | 31.77 |
| ATOM | 2775 | C | CYS | 364 | 22.914 | -21.493 | 86.950 | 1.00 | 30.91 |
| ATOM | 2776 | O | CYS | 364 | 23.207 | -20.645 | 86.119 | 1.00 | 30.71 |
| ATOM | 2777 | N | ASP | 365 | 23.608 | -22.614 | 87.107 | 1.00 | 31.25 |
| ATOM | 2778 | CA | ASP | 365 | 24.774 | -22.894 | 86.280 | 1.00 | 32.00 |
| ATOM | 2779 | CB | ASP | 365 | 25.389 | -24.243 | 86.659 | 1.00 | 32.78 |
| ATOM | 2780 | CG | ASP | 365 | 26.037 | -24.211 | 88.023 | 1.00 | 35.48 |
| ATOM | 2781 | OD1 | ASP | 365 | 26.017 | -23.127 | 88.650 | 1.00 | 37.01 |
| ATOM | 2782 | OD2 | ASP | 365 | 26.564 | -25.251 | 88.466 | 1.00 | 35.21 |
| ATOM | 2783 | C | ASP | 365 | 24.405 | -22.886 | 84.810 | 1.00 | 30.41 |
| ATOM | 2784 | O | ASP | 365 | 25.166 | -22.407 | 83.966 | 1.00 | 31.73 |
| ATOM | 2785 | N | ILE | 366 | 23.225 | -23.408 | 84.514 | 1.00 | 29.17 |
| ATOM | 2786 | CA | ILE | 366 | 22.739 | -23.462 | 83.148 | 1.00 | 30.58 |
| ATOM | 2787 | CB | ILE | 366 | 21.456 | -24.318 | 83.058 | 1.00 | 30.61 |
| ATOM | 2788 | CG2 | ILE | 366 | 20.779 | -24.118 | 81.712 | 1.00 | 28.15 |
| ATOM | 2789 | CG1 | ILE | 366 | 21.808 | -25.797 | 83.261 | 1.00 | 33.09 |
| ATOM | 2790 | CD1 | ILE | 366 | 20.577 | -26.702 | 83.405 | 1.00 | 32.69 |
| ATOM | 2791 | C | ILE | 366 | 22.462 | -22.066 | 82.576 | 1.00 | 29.08 |

| | | | | | | | | | |
|---|---|---|---|---|---|---|---|---|---|
| ATOM | 2792 | O | ILE | 366 | 22.729 | -21.815 | 81.405 | 1.00 | 28.78 |
| ATOM | 2793 | N | VAL | 367 | 21.906 | -21.170 | 83.386 | 1.00 | 27.52 |
| ATOM | 2794 | CA | VAL | 367 | 21.632 | -19.817 | 82.910 | 1.00 | 27.71 |
| ATOM | 2795 | CB | VAL | 367 | 20.803 | -19.021 | 83.943 | 1.00 | 26.66 |
| ATOM | 2796 | CG1 | VAL | 367 | 20.812 | -17.531 | 83.609 | 1.00 | 24.57 |
| ATOM | 2797 | CG2 | VAL | 367 | 19.373 | -19.535 | 83.928 | 1.00 | 26.09 |
| ATOM | 2798 | C | VAL | 367 | 22.979 | -19.143 | 82.643 | 1.00 | 28.05 |
| ATOM | 2799 | O | VAL | 367 | 23.144 | -18.409 | 81.670 | 1.00 | 28.53 |
| ATOM | 2800 | N | ARG | 368 | 23.940 | -19.436 | 83.508 | 1.00 | 27.74 |
| ATOM | 2801 | CA | ARG | 368 | 25.300 | -18.927 | 83.386 | 1.00 | 30.76 |
| ATOM | 2802 | CB | ARG | 368 | 26.172 | -19.575 | 84.458 | 1.00 | 31.66 |
| ATOM | 2803 | CG | ARG | 368 | 27.023 | -18.648 | 85.269 | 1.00 | 38.26 |
| ATOM | 2804 | CD | ARG | 368 | 28.312 | -18.282 | 84.579 | 1.00 | 41.00 |
| ATOM | 2805 | NE | ARG | 368 | 29.272 | -17.763 | 85.547 | 1.00 | 43.72 |
| ATOM | 2806 | CZ | ARG | 368 | 30.397 | -17.135 | 85.226 | 1.00 | 46.75 |
| ATOM | 2807 | NH1 | ARG | 368 | 30.710 | -16.938 | 83.954 | 1.00 | 48.06 |
| ATOM | 2808 | NH2 | ARG | 368 | 31.212 | -16.708 | 86.179 | 1.00 | 47.96 |
| ATOM | 2809 | C | ARG | 368 | 25.841 | -19.317 | 82.003 | 1.00 | 30.63 |
| ATOM | 2810 | O | ARG | 368 | 26.343 | -18.469 | 81.256 | 1.00 | 27.84 |
| ATOM | 2811 | N | ARG | 369 | 25.735 | -20.606 | 81.677 | 1.00 | 27.70 |
| ATOM | 2812 | CA | ARG | 369 | 26.228 | -21.115 | 80.399 | 1.00 | 28.24 |
| ATOM | 2813 | CB | ARG | 369 | 26.077 | -22.645 | 80.327 | 1.00 | 26.69 |
| ATOM | 2814 | CG | ARG | 369 | 27.044 | -23.429 | 81.224 | 1.00 | 29.04 |
| ATOM | 2815 | CD | ARG | 369 | 28.506 | -23.228 | 80.815 | 1.00 | 31.91 |
| ATOM | 2816 | NE | ARG | 369 | 28.752 | -23.683 | 79.445 | 1.00 | 35.74 |
| ATOM | 2817 | CZ | ARG | 369 | 29.117 | -22.892 | 78.439 | 1.00 | 36.75 |
| ATOM | 2818 | NH1 | ARG | 369 | 29.291 | -21.590 | 78.638 | 1.00 | 36.65 |
| ATOM | 2819 | NH2 | ARG | 369 | 29.291 | -23.400 | 77.225 | 1.00 | 36.11 |
| ATOM | 2820 | C | ARG | 369 | 25.528 | -20.472 | 79.208 | 1.00 | 27.14 |
| ATOM | 2821 | O | ARG | 369 | 26.160 | -20.188 | 78.189 | 1.00 | 28.06 |
| ATOM | 2822 | N | ALA | 370 | 24.224 | -20.252 | 79.327 | 1.00 | 25.64 |
| ATOM | 2823 | CA | ALA | 370 | 23.480 | -19.634 | 78.238 | 1.00 | 25.08 |
| ATOM | 2824 | CB | ALA | 370 | 21.991 | -19.587 | 78.574 | 1.00 | 25.47 |
| ATOM | 2825 | C | ALA | 370 | 24.015 | -18.218 | 78.006 | 1.00 | 25.14 |
| ATOM | 2826 | O | ALA | 370 | 24.196 | -17.793 | 76.870 | 1.00 | 25.23 |

| | | | | | | | | | |
|---|---|---|---|---|---|---|---|---|---|
| ATOM | 2827 | N | CYS | 371 | 24.268 | -17.491 | 79.087 | 1.00 | 24.15 |
| ATOM | 2828 | CA | CYS | 371 | 24.785 | -16.135 | 78.965 | 1.00 | 25.09 |
| ATOM | 2829 | CB | CYS | 371 | 24.855 | -15.467 | 80.338 | 1.00 | 22.74 |
| ATOM | 2830 | SG | CYS | 371 | 23.239 | -15.076 | 81.033 | 1.00 | 25.40 |
| ATOM | 2831 | C | CYS | 371 | 26.161 | -16.127 | 78.300 | 1.00 | 24.93 |
| ATOM | 2832 | O | CYS | 371 | 26.392 | -15.358 | 77.367 | 1.00 | 25.49 |
| ATOM | 2833 | N | GLU | 372 | 27.062 | -16.991 | 78.765 | 1.00 | 24.70 |
| ATOM | 2834 | CA | GLU | 372 | 28.411 | -17.073 | 78.207 | 1.00 | 26.69 |
| ATOM | 2835 | CB | GLU | 372 | 29.247 | -18.105 | 78.975 | 1.00 | 27.07 |
| ATOM | 2836 | CG | GLU | 372 | 29.232 | -17.890 | 80.481 | 1.00 | 32.77 |
| ATOM | 2837 | CD | GLU | 372 | 30.016 | -18.945 | 81.243 | 1.00 | 33.87 |
| ATOM | 2838 | OE1 | GLU | 372 | 29.905 | -20.139 | 80.892 | 1.00 | 36.95 |
| ATOM | 2839 | OE2 | GLU | 372 | 30.733 | -18.583 | 82.200 | 1.00 | 35.18 |
| ATOM | 2840 | C | GLU | 372 | 28.418 | -17.420 | 76.718 | 1.00 | 27.23 |
| ATOM | 2841 | O | GLU | 372 | 29.259 | -16.922 | 75.966 | 1.00 | 29.09 |
| ATOM | 2842 | N | SER | 373 | 27.489 | -18.273 | 76.296 | 1.00 | 25.93 |
| ATOM | 2843 | CA | SER | 373 | 27.403 | -18.664 | 74.894 | 1.00 | 27.07 |
| ATOM | 2844 | CB | SER | 373 | 26.393 | -19.803 | 74.718 | 1.00 | 25.93 |
| ATOM | 2845 | OG | SER | 373 | 26.784 | -20.951 | 75.457 | 1.00 | 32.56 |
| ATOM | 2846 | C | SER | 373 | 26.988 | -17.471 | 74.034 | 1.00 | 25.31 |
| ATOM | 2847 | O | SER | 373 | 27.585 | -17.207 | 72.998 | 1.00 | 24.49 |
| ATOM | 2848 | N | VAL | 374 | 25.962 | -16.754 | 74.475 | 1.00 | 25.87 |
| ATOM | 2849 | CA | VAL | 374 | 25.473 | -15.596 | 73.743 | 1.00 | 25.12 |
| ATOM | 2850 | CB | VAL | 374 | 24.139 | -15.103 | 74.319 | 1.00 | 26.07 |
| ATOM | 2851 | CG1 | VAL | 374 | 23.754 | -13.766 | 73.682 | 1.00 | 29.29 |
| ATOM | 2852 | CG2 | VAL | 374 | 23.055 | -16.127 | 74.061 | 1.00 | 25.56 |
| ATOM | 2853 | C | VAL | 374 | 26.465 | -14.429 | 73.742 | 1.00 | 24.54 |
| ATOM | 2854 | O | VAL | 374 | 26.657 | -13.792 | 72.714 | 1.00 | 25.64 |
| ATOM | 2855 | N | SER | 375 | 27.094 | -14.144 | 74.878 | 1.00 | 21.70 |
| ATOM | 2856 | CA | SER | 375 | 28.029 | -13.034 | 74.922 | 1.00 | 23.89 |
| ATOM | 2857 | CB | SER | 375 | 28.298 | -12.585 | 76.365 | 1.00 | 23.28 |
| ATOM | 2858 | OG | SER | 375 | 28.986 | -13.565 | 77.120 | 1.00 | 29.71 |
| ATOM | 2859 | C | SER | 375 | 29.324 | -13.391 | 74.210 | 1.00 | 24.77 |
| ATOM | 2860 | O | SER | 375 | 29.873 | -12.560 | 73.490 | 1.00 | 23.61 |
| ATOM | 2861 | N | THR | 376 | 29.805 | -14.623 | 74.386 | 1.00 | 23.54 |

| | | | | | | | | | |
|---|---|---|---|---|---|---|---|---|---|
| ATOM | 2862 | CA | THR | 376 | 31.029 | -15.052 | 73.707 | 1.00 | 23.38 |
| ATOM | 2863 | CB | THR | 376 | 31.444 | -16.501 | 74.096 | 1.00 | 23.76 |
| ATOM | 2864 | OG1 | THR | 376 | 31.874 | -16.527 | 75.458 | 1.00 | 26.36 |
| ATOM | 2865 | CG2 | THR | 376 | 32.594 | -16.987 | 73.222 | 1.00 | 21.48 |
| ATOM | 2866 | C | THR | 376 | 30.859 | -14.996 | 72.189 | 1.00 | 22.33 |
| ATOM | 2867 | O | THR | 376 | 31.810 | -14.694 | 71.465 | 1.00 | 23.88 |
| ATOM | 2868 | N | ARG | 377 | 29.660 | -15.293 | 71.695 | 1.00 | 20.80 |
| ATOM | 2869 | CA | ARG | 377 | 29.452 | -15.239 | 70.253 | 1.00 | 21.46 |
| ATOM | 2870 | CB | ARG | 377 | 28.141 | -15.918 | 69.839 | 1.00 | 22.21 |
| ATOM | 2871 | CG | ARG | 377 | 27.958 | -15.875 | 68.312 | 1.00 | 25.01 |
| ATOM | 2872 | CD | ARG | 377 | 26.601 | -16.377 | 67.827 | 1.00 | 27.70 |
| ATOM | 2873 | NE | ARG | 377 | 25.491 | -15.558 | 68.302 | 1.00 | 25.17 |
| ATOM | 2874 | CZ | ARG | 377 | 24.255 | -15.637 | 67.825 | 1.00 | 26.42 |
| ATOM | 2875 | NH1 | ARG | 377 | 23.973 | -16.492 | 66.850 | 1.00 | 25.23 |
| ATOM | 2876 | NH2 | ARG | 377 | 23.294 | -14.877 | 68.339 | 1.00 | 26.96 |
| ATOM | 2877 | C | ARG | 377 | 29.439 | -13.773 | 69.787 | 1.00 | 21.55 |
| ATOM | 2878 | O | ARG | 377 | 29.856 | -13.462 | 68.670 | 1.00 | 20.80 |
| ATOM | 2879 | N | ALA | 378 | 28.951 | -12.879 | 70.639 | 1.00 | 19.46 |
| ATOM | 2880 | CA | ALA | 378 | 28.927 | -11.463 | 70.302 | 1.00 | 21.17 |
| ATOM | 2881 | CB | ALA | 378 | 28.239 | -10.653 | 71.412 | 1.00 | 20.68 |
| ATOM | 2882 | C | ALA | 378 | 30.374 | -11.015 | 70.151 | 1.00 | 20.18 |
| ATOM | 2883 | O | ALA | 378 | 30.747 | -10.420 | 69.145 | 1.00 | 20.36 |
| ATOM | 2884 | N | ALA | 379 | 31.191 | -11.326 | 71.153 | 1.00 | 19.41 |
| ATOM | 2885 | CA | ALA | 379 | 32.600 | -10.950 | 71.138 | 1.00 | 20.64 |
| ATOM | 2886 | CB | ALA | 379 | 33.296 | -11.515 | 72.371 | 1.00 | 20.04 |
| ATOM | 2887 | C | ALA | 379 | 33.332 | -11.405 | 69.869 | 1.00 | 22.79 |
| ATOM | 2888 | O | ALA | 379 | 34.054 | -10.620 | 69.234 | 1.00 | 21.82 |
| ATOM | 2889 | N | HIS | 380 | 33.139 | -12.666 | 69.489 | 1.00 | 22.45 |
| ATOM | 2890 | CA | HIS | 380 | 33.803 | -13.208 | 68.305 | 1.00 | 22.78 |
| ATOM | 2891 | CB | HIS | 380 | 33.726 | -14.745 | 68.314 | 1.00 | 22.80 |
| ATOM | 2892 | CG | HIS | 380 | 34.584 | -15.384 | 69.364 | 1.00 | 26.52 |
| ATOM | 2893 | CD2 | HIS | 380 | 35.557 | -14.870 | 70.152 | 1.00 | 27.81 |
| ATOM | 2894 | ND1 | HIS | 380 | 34.499 | -16.720 | 69.687 | 1.00 | 28.99 |
| ATOM | 2895 | CE1 | HIS | 380 | 35.383 | -17.002 | 70.627 | 1.00 | 28.15 |
| ATOM | 2896 | NE2 | HIS | 380 | 36.039 | -15.895 | 70.927 | 1.00 | 28.70 |

| | | | | | | | | | |
|---|---|---|---|---|---|---|---|---|---|
| ATOM | 2897 | C | HIS | 380 | 33.242 | -12.657 | 66.994 | 1.00 | 22.38 |
| ATOM | 2898 | O | HIS | 380 | 33.988 | -12.368 | 66.073 | 1.00 | 20.71 |
| ATOM | 2899 | N | MET | 381 | 31.926 | -12.524 | 66.915 | 1.00 | 23.83 |
| ATOM | 2900 | CA | MET | 381 | 31.285 | -12.018 | 65.713 | 1.00 | 26.66 |
| ATOM | 2901 | CB | MET | 381 | 29.760 | -12.086 | 65.899 | 1.00 | 29.06 |
| ATOM | 2902 | CG | MET | 381 | 28.926 | -12.031 | 64.622 | 1.00 | 34.34 |
| ATOM | 2903 | SD | MET | 381 | 29.456 | -13.157 | 63.312 | 1.00 | 33.69 |
| ATOM | 2904 | CE | MET | 381 | 28.228 | -14.472 | 63.429 | 1.00 | 34.64 |
| ATOM | 2905 | C | MET | 381 | 31.781 | -10.580 | 65.509 | 1.00 | 27.50 |
| ATOM | 2906 | O | MET | 381 | 32.153 | -10.188 | 64.406 | 1.00 | 26.70 |
| ATOM | 2907 | N | CYS | 382 | 31.830 | -9.813 | 66.595 | 1.00 | 26.32 |
| ATOM | 2908 | CA | CYS | 382 | 32.302 | -8.441 | 66.536 | 1.00 | 24.87 |
| ATOM | 2909 | CB | CYS | 382 | 32.102 | -7.769 | 67.896 | 1.00 | 26.05 |
| ATOM | 2910 | SG | CYS | 382 | 32.389 | -5.962 | 67.931 | 1.00 | 26.70 |
| ATOM | 2911 | C | CYS | 382 | 33.785 | -8.355 | 66.122 | 1.00 | 24.60 |
| ATOM | 2912 | O | CYS | 382 | 34.187 | -7.457 | 65.360 | 1.00 | 19.92 |
| ATOM | 2913 | N | SER | 383 | 34.590 | -9.288 | 66.623 | 1.00 | 22.62 |
| ATOM | 2914 | CA | SER | 383 | 36.017 | -9.302 | 66.327 | 1.00 | 22.35 |
| ATOM | 2915 | CB | SER | 383 | 36.716 | -10.439 | 67.096 | 1.00 | 23.03 |
| ATOM | 2916 | OG | SER | 383 | 36.361 | -11.712 | 66.571 | 1.00 | 24.25 |
| ATOM | 2917 | C | SER | 383 | 36.272 | -9.463 | 64.834 | 1.00 | 23.77 |
| ATOM | 2918 | O | SER | 383 | 37.202 | -8.875 | 64.288 | 1.00 | 24.79 |
| ATOM | 2919 | N | ALA | 384 | 35.448 | -10.269 | 64.173 | 1.00 | 24.03 |
| ATOM | 2920 | CA | ALA | 384 | 35.612 | -10.480 | 62.743 | 1.00 | 25.52 |
| ATOM | 2921 | CB | ALA | 384 | 34.649 | -11.552 | 62.256 | 1.00 | 22.05 |
| ATOM | 2922 | C | ALA | 384 | 35.369 | -9.182 | 61.980 | 1.00 | 25.61 |
| ATOM | 2923 | O | ALA | 384 | 35.990 | -8.942 | 60.947 | 1.00 | 25.37 |
| ATOM | 2924 | N | GLY | 385 | 34.450 | -8.360 | 62.490 | 1.00 | 25.67 |
| ATOM | 2925 | CA | GLY | 385 | 34.134 | -7.098 | 61.842 | 1.00 | 23.86 |
| ATOM | 2926 | C | GLY | 385 | 35.289 | -6.128 | 61.944 | 1.00 | 20.99 |
| ATOM | 2927 | O | GLY | 385 | 35.702 | -5.531 | 60.960 | 1.00 | 22.47 |
| ATOM | 2928 | N | LEU | 386 | 35.811 | -5.962 | 63.148 | 1.00 | 22.82 |
| ATOM | 2929 | CA | LEU | 386 | 36.937 | -5.065 | 63.364 | 1.00 | 25.33 |
| ATOM | 2930 | CB | LEU | 386 | 37.259 | -4.971 | 64.850 | 1.00 | 23.48 |
| ATOM | 2931 | CG | LEU | 386 | 37.800 | -3.658 | 65.425 | 1.00 | 27.75 |

| | | | | | | | | | |
|---|---|---|---|---|---|---|---|---|---|
| ATOM | 2932 | CD1 | LEU | 386 | 38.641 | -4.007 | 66.641 | 1.00 | 26.18 |
| ATOM | 2933 | CD2 | LEU | 386 | 38.621 | -2.865 | 64.428 | 1.00 | 25.52 |
| ATOM | 2934 | C | LEU | 386 | 38.172 | -5.584 | 62.616 | 1.00 | 26.01 |
| ATOM | 2935 | O | LEU | 386 | 38.953 | -4.794 | 62.067 | 1.00 | 26.60 |
| ATOM | 2936 | N | ALA | 387 | 38.356 | -6.904 | 62.601 | 1.00 | 23.95 |
| ATOM | 2937 | CA | ALA | 387 | 39.509 | -7.482 | 61.902 | 1.00 | 24.13 |
| ATOM | 2938 | CB | ALA | 387 | 39.585 | -8.989 | 62.135 | 1.00 | 20.59 |
| ATOM | 2939 | C | ALA | 387 | 39.405 | -7.181 | 60.411 | 1.00 | 24.07 |
| ATOM | 2940 | O | ALA | 387 | 40.419 | -6.990 | 59.730 | 1.00 | 22.59 |
| ATOM | 2941 | N | GLY | 388 | 38.175 | -7.141 | 59.904 | 1.00 | 24.30 |
| ATOM | 2942 | CA | GLY | 388 | 37.975 | -6.838 | 58.497 | 1.00 | 24.40 |
| ATOM | 2943 | C | GLY | 388 | 38.380 | -5.398 | 58.203 | 1.00 | 25.62 |
| ATOM | 2944 | O | GLY | 388 | 39.048 | -5.114 | 57.205 | 1.00 | 25.24 |
| ATOM | 2945 | N | VAL | 389 | 37.974 | -4.488 | 59.084 | 1.00 | 25.15 |
| ATOM | 2946 | CA | VAL | 389 | 38.294 | -3.072 | 58.950 | 1.00 | 23.08 |
| ATOM | 2947 | CB | VAL | 389 | 37.581 | -2.259 | 60.057 | 1.00 | 21.38 |
| ATOM | 2948 | CG1 | VAL | 389 | 38.083 | -0.820 | 60.076 | 1.00 | 21.90 |
| ATOM | 2949 | CG2 | VAL | 389 | 36.078 | -2.303 | 59.819 | 1.00 | 20.64 |
| ATOM | 2950 | C | VAL | 389 | 39.802 | -2.858 | 59.034 | 1.00 | 24.13 |
| ATOM | 2951 | O | VAL | 389 | 40.402 | -2.198 | 58.178 | 1.00 | 25.99 |
| ATOM | 2952 | N | ILE | 390 | 40.424 | -3.429 | 60.054 | 1.00 | 24.21 |
| ATOM | 2953 | CA | ILE | 390 | 41.866 | -3.289 | 60.209 | 1.00 | 25.31 |
| ATOM | 2954 | CB | ILE | 390 | 42.317 | -3.883 | 61.576 | 1.00 | 25.21 |
| ATOM | 2955 | CG2 | ILE | 390 | 43.831 | -3.962 | 61.661 | 1.00 | 27.92 |
| ATOM | 2956 | CG1 | ILE | 390 | 41.778 | -2.993 | 62.708 | 1.00 | 26.03 |
| ATOM | 2957 | CD1 | ILE | 390 | 42.091 | -3.476 | 64.094 | 1.00 | 27.41 |
| ATOM | 2958 | C | ILE | 390 | 42.668 | -3.899 | 59.040 | 1.00 | 26.27 |
| ATOM | 2959 | O | ILE | 390 | 43.622 | -3.287 | 58.563 | 1.00 | 25.08 |
| ATOM | 2960 | N | ASN | 391 | 42.286 | -5.082 | 58.561 | 1.00 | 27.72 |
| ATOM | 2961 | CA | ASN | 391 | 43.026 | -5.689 | 57.448 | 1.00 | 29.87 |
| ATOM | 2962 | CB | ASN | 391 | 42.649 | -7.162 | 57.250 | 1.00 | 27.74 |
| ATOM | 2963 | CG | ASN | 391 | 43.147 | -8.044 | 58.375 | 1.00 | 29.54 |
| ATOM | 2964 | OD1 | ASN | 391 | 44.216 | -7.804 | 58.939 | 1.00 | 28.68 |
| ATOM | 2965 | ND2 | ASN | 391 | 42.383 | -9.079 | 58.699 | 1.00 | 26.84 |
| ATOM | 2966 | C | ASN | 391 | 42.805 | -4.930 | 56.144 | 1.00 | 31.14 |

| | | | | | | | | | | |
|---|---|---|---|---|---|---|---|---|---|---|
| ATOM | 2967 | O | ASN | 391 | 43.688 | -4.903 | 55.281 | 1.00 | 29.49 |
| ATOM | 2968 | N | ARG | 392 | 41.627 | -4.331 | 55.991 | 1.00 | 31.07 |
| ATOM | 2969 | CA | ARG | 392 | 41.358 | -3.553 | 54.795 | 1.00 | 33.43 |
| ATOM | 2970 | CB | ARG | 392 | 39.921 | -3.018 | 54.780 | 1.00 | 35.04 |
| ATOM | 2971 | CG | ARG | 392 | 39.597 | -2.307 | 53.483 | 1.00 | 35.84 |
| ATOM | 2972 | CD | ARG | 392 | 38.614 | -1.173 | 53.650 | 1.00 | 37.18 |
| ATOM | 2973 | NE | ARG | 392 | 38.804 | -0.186 | 52.589 | 1.00 | 35.89 |
| ATOM | 2974 | CZ | ARG | 392 | 38.518 | -0.390 | 51.309 | 1.00 | 36.67 |
| ATOM | 2975 | NH1 | ARG | 392 | 38.006 | -1.550 | 50.911 | 1.00 | 38.42 |
| ATOM | 2976 | NH2 | ARG | 392 | 38.788 | 0.553 | 50.417 | 1.00 | 37.33 |
| ATOM | 2977 | C | ARG | 392 | 42.335 | -2.377 | 54.831 | 1.00 | 33.73 |
| ATOM | 2978 | O | ARG | 392 | 43.028 | -2.107 | 53.858 | 1.00 | 34.52 |
| ATOM | 2979 | N | MET | 393 | 42.396 | -1.691 | 55.967 | 1.00 | 34.05 |
| ATOM | 2980 | CA | MET | 393 | 43.298 | -0.554 | 56.126 | 1.00 | 35.93 |
| ATOM | 2981 | CB | MET | 393 | 43.119 | 0.073 | 57.517 | 1.00 | 32.21 |
| ATOM | 2982 | CG | MET | 393 | 41.801 | 0.834 | 57.692 | 1.00 | 28.72 |
| ATOM | 2983 | SD | MET | 393 | 41.530 | 1.348 | 59.400 | 1.00 | 27.28 |
| ATOM | 2984 | CE | MET | 393 | 42.652 | 2.753 | 59.533 | 1.00 | 24.26 |
| ATOM | 2985 | C | MET | 393 | 44.751 | -0.979 | 55.947 | 1.00 | 39.48 |
| ATOM | 2986 | O | MET | 393 | 45.579 | -0.216 | 55.448 | 1.00 | 39.63 |
| ATOM | 2987 | N | ARG | 394 | 45.049 | -2.205 | 56.364 | 1.00 | 43.20 |
| ATOM | 2988 | CA | ARG | 394 | 46.391 | -2.766 | 56.277 | 1.00 | 45.79 |
| ATOM | 2989 | CB | ARG | 394 | 46.381 | -4.180 | 56.870 | 1.00 | 49.86 |
| ATOM | 2990 | CG | ARG | 394 | 47.670 | -4.595 | 57.551 | 1.00 | 53.76 |
| ATOM | 2991 | CD | ARG | 394 | 48.587 | -5.335 | 56.612 | 1.00 | 56.09 |
| ATOM | 2992 | NE | ARG | 394 | 49.896 | -5.554 | 57.217 | 1.00 | 60.36 |
| ATOM | 2993 | CZ | ARG | 394 | 50.797 | -4.596 | 57.411 | 1.00 | 60.35 |
| ATOM | 2994 | NH1 | ARG | 394 | 50.528 | -3.353 | 57.042 | 1.00 | 61.48 |
| ATOM | 2995 | NH2 | ARG | 394 | 51.964 | -4.878 | 57.978 | 1.00 | 60.51 |
| ATOM | 2996 | C | ARG | 394 | 46.912 | -2.792 | 54.835 | 1.00 | 46.90 |
| ATOM | 2997 | O | ARG | 394 | 48.117 | -2.697 | 54.606 | 1.00 | 44.95 |
| ATOM | 2998 | N | GLU | 395 | 46.005 | -2.906 | 53.869 | 1.00 | 48.68 |
| ATOM | 2999 | CA | GLU | 395 | 46.387 | -2.943 | 52.459 | 1.00 | 52.84 |
| ATOM | 3000 | CB | GLU | 395 | 45.165 | -3.275 | 51.590 | 1.00 | 54.51 |
| ATOM | 3001 | CG | GLU | 395 | 44.388 | -4.508 | 52.051 | 1.00 | 60.85 |

| | | | | | | | | | |
|---|---|---|---|---|---|---|---|---|---|
| ATOM | 3002 | CD | GLU | 395 | 43.310 | -4.952 | 51.061 | 1.00 | 64.84 |
| ATOM | 3003 | OE1 | GLU | 395 | 42.485 | -4.105 | 50.642 | 1.00 | 65.83 |
| ATOM | 3004 | OE2 | GLU | 395 | 43.286 | -6.155 | 50.708 | 1.00 | 66.43 |
| ATOM | 3005 | C | GLU | 395 | 47.008 | -1.621 | 51.991 | 1.00 | 54.64 |
| ATOM | 3006 | O | GLU | 395 | 47.791 | -1.594 | 51.039 | 1.00 | 53.71 |
| ATOM | 3007 | N | SER | 396 | 46.660 | -0.528 | 52.666 | 1.00 | 56.54 |
| ATOM | 3008 | CA | SER | 396 | 47.179 | 0.794 | 52.313 | 1.00 | 58.22 |
| ATOM | 3009 | CB | SER | 396 | 46.037 | 1.808 | 52.266 | 1.00 | 57.21 |
| ATOM | 3010 | OG | SER | 396 | 44.980 | 1.340 | 51.448 | 1.00 | 59.52 |
| ATOM | 3011 | C | SER | 396 | 48.221 | 1.268 | 53.318 | 1.00 | 60.22 |
| ATOM | 3012 | O | SER | 396 | 48.394 | 2.468 | 53.527 | 1.00 | 60.38 |
| ATOM | 3013 | N | ARG | 397 | 48.915 | 0.324 | 53.941 | 1.00 | 62.22 |
| ATOM | 3014 | CA | ARG | 397 | 49.924 | 0.663 | 54.933 | 1.00 | 64.67 |
| ATOM | 3015 | CB | ARG | 397 | 49.430 | 0.260 | 56.324 | 1.00 | 65.24 |
| ATOM | 3016 | CG | ARG | 397 | 49.798 | 1.218 | 57.444 | 1.00 | 67.16 |
| ATOM | 3017 | CD | ARG | 397 | 49.178 | 2.596 | 57.244 | 1.00 | 68.03 |
| ATOM | 3018 | NE | ARG | 397 | 48.803 | 3.208 | 58.516 | 1.00 | 69.13 |
| ATOM | 3019 | CZ | ARG | 397 | 47.681 | 2.933 | 59.178 | 1.00 | 70.58 |
| ATOM | 3020 | NH1 | ARG | 397 | 46.813 | 2.059 | 58.687 | 1.00 | 71.37 |
| ATOM | 3021 | NH2 | ARG | 397 | 47.429 | 3.521 | 60.340 | 1.00 | 70.29 |
| ATOM | 3022 | C | ARG | 397 | 51.222 | -0.063 | 54.611 | 1.00 | 65.54 |
| ATOM | 3023 | O | ARG | 397 | 51.416 | -1.215 | 54.998 | 1.00 | 66.75 |
| ATOM | 3024 | N | SER | 398 | 52.106 | 0.621 | 53.894 | 1.00 | 66.86 |
| ATOM | 3025 | CA | SER | 398 | 53.388 | 0.052 | 53.508 | 1.00 | 67.48 |
| ATOM | 3026 | CB | SER | 398 | 53.980 | 0.832 | 52.331 | 1.00 | 67.48 |
| ATOM | 3027 | OG | SER | 398 | 53.155 | 0.725 | 51.181 | 1.00 | 66.93 |
| ATOM | 3028 | C | SER | 398 | 54.358 | 0.063 | 54.679 | 1.00 | 68.36 |
| ATOM | 3029 | O | SER | 398 | 55.036 | 1.063 | 54.934 | 1.00 | 69.35 |
| ATOM | 3030 | N | GLU | 399 | 54.413 | -1.059 | 55.388 | 1.00 | 67.90 |
| ATOM | 3031 | CA | GLU | 399 | 55.297 | -1.206 | 56.533 | 1.00 | 68.16 |
| ATOM | 3032 | CB | GLU | 399 | 55.002 | -0.126 | 57.564 | 1.00 | 68.95 |
| ATOM | 3033 | CG | GLU | 399 | 53.540 | 0.020 | 57.889 | 1.00 | 71.05 |
| ATOM | 3034 | CD | GLU | 399 | 53.261 | 1.318 | 58.598 | 1.00 | 71.37 |
| ATOM | 3035 | OE1 | GLU | 399 | 53.871 | 1.545 | 59.662 | 1.00 | 72.25 |
| ATOM | 3036 | OE2 | GLU | 399 | 52.443 | 2.111 | 58.089 | 1.00 | 71.32 |

| | | | | | | | | | |
|---|---|---|---|---|---|---|---|---|---|
| ATOM | 3037 | C | GLU | 399 | 55.167 | -2.581 | 57.168 | 1.00 | 67.57 |
| ATOM | 3038 | O | GLU | 399 | 54.078 | -3.155 | 57.232 | 1.00 | 67.34 |
| ATOM | 3039 | N | ASP | 400 | 56.301 | -3.091 | 57.635 | 1.00 | 66.86 |
| ATOM | 3040 | CA | ASP | 400 | 56.397 | -4.400 | 58.265 | 1.00 | 65.75 |
| ATOM | 3041 | CB | ASP | 400 | 57.739 | -4.507 | 58.989 | 1.00 | 68.55 |
| ATOM | 3042 | CG | ASP | 400 | 58.892 | -3.961 | 58.157 | 1.00 | 71.49 |
| ATOM | 3043 | OD1 | ASP | 400 | 59.015 | -4.356 | 56.976 | 1.00 | 72.29 |
| ATOM | 3044 | OD2 | ASP | 400 | 59.675 | -3.136 | 58.682 | 1.00 | 72.38 |
| ATOM | 3045 | C | ASP | 400 | 55.247 | -4.676 | 59.233 | 1.00 | 63.41 |
| ATOM | 3046 | O | ASP | 400 | 54.385 | -5.514 | 58.962 | 1.00 | 63.27 |
| ATOM | 3047 | N | VAL | 401 | 55.241 | -3.973 | 60.361 | 1.00 | 59.50 |
| ATOM | 3048 | CA | VAL | 401 | 54.193 | -4.138 | 61.360 | 1.00 | 55.59 |
| ATOM | 3049 | CB | VAL | 401 | 54.789 | -4.439 | 62.757 | 1.00 | 55.81 |
| ATOM | 3050 | CG1 | VAL | 401 | 53.698 | -4.375 | 63.818 | 1.00 | 54.69 |
| ATOM | 3051 | CG2 | VAL | 401 | 55.442 | -5.817 | 62.757 | 1.00 | 54.18 |
| ATOM | 3052 | C | VAL | 401 | 53.345 | -2.876 | 61.454 | 1.00 | 53.78 |
| ATOM | 3053 | O | VAL | 401 | 53.841 | -1.807 | 61.820 | 1.00 | 53.39 |
| ATOM | 3054 | N | MET | 402 | 52.065 | -2.991 | 61.114 | 1.00 | 50.91 |
| ATOM | 3055 | CA | MET | 402 | 51.190 | -1.834 | 61.194 | 1.00 | 47.59 |
| ATOM | 3056 | CB | MET | 402 | 49.992 | -1.958 | 60.250 | 1.00 | 46.98 |
| ATOM | 3057 | CG | MET | 402 | 49.043 | -0.768 | 60.387 | 1.00 | 47.22 |
| ATOM | 3058 | SD | MET | 402 | 47.505 | -0.874 | 59.461 | 1.00 | 48.69 |
| ATOM | 3059 | CE | MET | 402 | 46.622 | -2.099 | 60.439 | 1.00 | 48.15 |
| ATOM | 3060 | C | MET | 402 | 50.670 | -1.643 | 62.605 | 1.00 | 44.98 |
| ATOM | 3061 | O | MET | 402 | 49.945 | -2.483 | 63.134 | 1.00 | 43.92 |
| ATOM | 3062 | N | ARG | 403 | 51.054 | -0.533 | 63.219 | 1.00 | 43.27 |
| ATOM | 3063 | CA | ARG | 403 | 50.587 | -0.229 | 64.556 | 1.00 | 41.71 |
| ATOM | 3064 | CB | ARG | 403 | 51.673 | 0.484 | 65.350 | 1.00 | 45.65 |
| ATOM | 3065 | CG | ARG | 403 | 52.903 | -0.356 | 65.596 | 1.00 | 52.20 |
| ATOM | 3066 | CD | ARG | 403 | 53.973 | 0.474 | 66.262 | 1.00 | 57.99 |
| ATOM | 3067 | NE | ARG | 403 | 55.137 | -0.324 | 66.630 | 1.00 | 65.47 |
| ATOM | 3068 | CZ | ARG | 403 | 56.251 | 0.184 | 67.149 | 1.00 | 68.76 |
| ATOM | 3069 | NH1 | ARG | 403 | 56.349 | 1.493 | 67.357 | 1.00 | 69.34 |
| ATOM | 3070 | NH2 | ARG | 403 | 57.265 | -0.615 | 67.468 | 1.00 | 69.59 |
| ATOM | 3071 | C | ARG | 403 | 49.388 | 0.685 | 64.372 | 1.00 | 37.99 |

| | | | | | | | | | |
|---|---|---|---|---|---|---|---|---|---|
| ATOM | 3072 | O | ARG | 403 | 49.471 | 1.692 | 63.679 | 1.00 | 37.13 |
| ATOM | 3073 | N | ILE | 404 | 48.267 | 0.322 | 64.975 | 1.00 | 34.39 |
| ATOM | 3074 | CA | ILE | 404 | 47.069 | 1.129 | 64.854 | 1.00 | 31.53 |
| ATOM | 3075 | CB | ILE | 404 | 46.161 | 0.577 | 63.735 | 1.00 | 33.38 |
| ATOM | 3076 | CG2 | ILE | 404 | 45.681 | -0.829 | 64.096 | 1.00 | 32.57 |
| ATOM | 3077 | CG1 | ILE | 404 | 44.987 | 1.524 | 63.500 | 1.00 | 35.77 |
| ATOM | 3078 | CD1 | ILE | 404 | 44.144 | 1.153 | 62.300 | 1.00 | 38.45 |
| ATOM | 3079 | C | ILE | 404 | 46.322 | 1.152 | 66.179 | 1.00 | 28.96 |
| ATOM | 3080 | O | ILE | 404 | 46.393 | 0.204 | 66.956 | 1.00 | 29.35 |
| ATOM | 3081 | N | THR | 405 | 45.632 | 2.250 | 66.453 | 1.00 | 28.84 |
| ATOM | 3082 | CA | THR | 405 | 44.874 | 2.359 | 67.693 | 1.00 | 27.84 |
| ATOM | 3083 | CB | THR | 405 | 45.323 | 3.558 | 68.535 | 1.00 | 26.65 |
| ATOM | 3084 | OG1 | THR | 405 | 46.663 | 3.335 | 68.990 | 1.00 | 30.48 |
| ATOM | 3085 | CG2 | THR | 405 | 44.428 | 3.715 | 69.749 | 1.00 | 27.32 |
| ATOM | 3086 | C | THR | 405 | 43.387 | 2.460 | 67.408 | 1.00 | 27.13 |
| ATOM | 3087 | O | THR | 405 | 42.964 | 3.127 | 66.462 | 1.00 | 24.36 |
| ATOM | 3088 | N | VAL | 406 | 42.604 | 1.786 | 68.245 | 1.00 | 25.61 |
| ATOM | 3089 | CA | VAL | 406 | 41.160 | 1.737 | 68.107 | 1.00 | 23.67 |
| ATOM | 3090 | CB | VAL | 406 | 40.705 | 0.244 | 67.973 | 1.00 | 23.64 |
| ATOM | 3091 | CG1 | VAL | 406 | 39.189 | 0.138 | 67.798 | 1.00 | 24.19 |
| ATOM | 3092 | CG2 | VAL | 406 | 41.405 | -0.399 | 66.783 | 1.00 | 21.36 |
| ATOM | 3093 | C | VAL | 406 | 40.493 | 2.392 | 69.320 | 1.00 | 26.21 |
| ATOM | 3094 | O | VAL | 406 | 40.763 | 2.018 | 70.469 | 1.00 | 26.86 |
| ATOM | 3095 | N | GLY | 407 | 39.644 | 3.389 | 69.072 | 1.00 | 25.61 |
| ATOM | 3096 | CA | GLY | 407 | 38.943 | 4.044 | 70.168 | 1.00 | 23.09 |
| ATOM | 3097 | C | GLY | 407 | 37.645 | 3.285 | 70.387 | 1.00 | 21.77 |
| ATOM | 3098 | O | GLY | 407 | 36.919 | 3.011 | 69.426 | 1.00 | 23.17 |
| ATOM | 3099 | N | VAL | 408 | 37.334 | 2.943 | 71.632 | 1.00 | 20.52 |
| ATOM | 3100 | CA | VAL | 408 | 36.128 | 2.167 | 71.907 | 1.00 | 21.51 |
| ATOM | 3101 | CB | VAL | 408 | 36.500 | 0.684 | 72.252 | 1.00 | 23.04 |
| ATOM | 3102 | CG1 | VAL | 408 | 35.237 | -0.176 | 72.351 | 1.00 | 19.52 |
| ATOM | 3103 | CG2 | VAL | 408 | 37.436 | 0.121 | 71.201 | 1.00 | 20.49 |
| ATOM | 3104 | C | VAL | 408 | 35.282 | 2.704 | 73.060 | 1.00 | 23.66 |
| ATOM | 3105 | O | VAL | 408 | 35.814 | 3.223 | 74.045 | 1.00 | 23.60 |
| ATOM | 3106 | N | ASP | 409 | 33.963 | 2.580 | 72.923 | 1.00 | 24.58 |

| | | | | | | | | | |
|---|---|---|---|---|---|---|---|---|---|
| ATOM | 3107 | CA | ASP | 409 | 33.040 | 2.992 | 73.975 | 1.00 | 26.70 |
| ATOM | 3108 | CB | ASP | 409 | 32.612 | 4.455 | 73.803 | 1.00 | 30.78 |
| ATOM | 3109 | CG | ASP | 409 | 31.909 | 4.998 | 75.041 | 1.00 | 31.51 |
| ATOM | 3110 | OD1 | ASP | 409 | 32.322 | 4.625 | 76.156 | 1.00 | 31.70 |
| ATOM | 3111 | OD2 | ASP | 409 | 30.955 | 5.794 | 74.910 | 1.00 | 35.70 |
| ATOM | 3112 | C | ASP | 409 | 31.824 | 2.083 | 73.898 | 1.00 | 25.68 |
| ATOM | 3113 | O | ASP | 409 | 31.639 | 1.396 | 72.901 | 1.00 | 27.99 |
| ATOM | 3114 | N | GLY | 410 | 30.999 | 2.079 | 74.943 | 1.00 | 28.67 |
| ATOM | 3115 | CA | GLY | 410 | 29.807 | 1.233 | 74.964 | 1.00 | 29.54 |
| ATOM | 3116 | C | GLY | 410 | 29.755 | 0.355 | 76.212 | 1.00 | 30.09 |
| ATOM | 3117 | O | GLY | 410 | 30.787 | -0.138 | 76.657 | 1.00 | 28.57 |
| ATOM | 3118 | N | SER | 411 | 28.560 | 0.150 | 76.767 | 1.00 | 30.89 |
| ATOM | 3119 | CA | SER | 411 | 28.392 | -0.649 | 77.983 | 1.00 | 32.71 |
| ATOM | 3120 | CB | SER | 411 | 26.941 | -0.554 | 78.490 | 1.00 | 32.88 |
| ATOM | 3121 | OG | SER | 411 | 26.011 | -0.884 | 77.473 | 1.00 | 36.82 |
| ATOM | 3122 | C | SER | 411 | 28.804 | -2.121 | 77.840 | 1.00 | 31.25 |
| ATOM | 3123 | O | SER | 411 | 29.480 | -2.661 | 78.712 | 1.00 | 29.96 |
| ATOM | 3124 | N | VAL | 412 | 28.398 | -2.768 | 76.754 | 1.00 | 29.78 |
| ATOM | 3125 | CA | VAL | 412 | 28.780 | -4.158 | 76.535 | 1.00 | 28.59 |
| ATOM | 3126 | CB | VAL | 412 | 28.264 | -4.665 | 75.174 | 1.00 | 29.68 |
| ATOM | 3127 | CG1 | VAL | 412 | 28.772 | -6.088 | 74.908 | 1.00 | 27.25 |
| ATOM | 3128 | CG2 | VAL | 412 | 26.739 | -4.642 | 75.173 | 1.00 | 29.93 |
| ATOM | 3129 | C | VAL | 412 | 30.307 | -4.320 | 76.584 | 1.00 | 29.24 |
| ATOM | 3130 | O | VAL | 412 | 30.831 | -5.145 | 77.340 | 1.00 | 28.78 |
| ATOM | 3131 | N | TYR | 413 | 31.023 | -3.522 | 75.796 | 1.00 | 27.57 |
| ATOM | 3132 | CA | TYR | 413 | 32.482 | -3.602 | 75.763 | 1.00 | 24.60 |
| ATOM | 3133 | CB | TYR | 413 | 33.049 | -2.730 | 74.645 | 1.00 | 19.87 |
| ATOM | 3134 | CG | TYR | 413 | 34.568 | -2.710 | 74.587 | 1.00 | 20.22 |
| ATOM | 3135 | CD1 | TYR | 413 | 35.270 | -3.566 | 73.735 | 1.00 | 21.52 |
| ATOM | 3136 | CE1 | TYR | 413 | 36.667 | -3.519 | 73.655 | 1.00 | 19.93 |
| ATOM | 3137 | CD2 | TYR | 413 | 35.300 | -1.819 | 75.363 | 1.00 | 14.63 |
| ATOM | 3138 | CE2 | TYR | 413 | 36.690 | -1.770 | 75.294 | 1.00 | 17.31 |
| ATOM | 3139 | CZ | TYR | 413 | 37.364 | -2.616 | 74.439 | 1.00 | 19.92 |
| ATOM | 3140 | OH | TYR | 413 | 38.737 | -2.547 | 74.362 | 1.00 | 23.08 |
| ATOM | 3141 | C | TYR | 413 | 33.151 | -3.193 | 77.072 | 1.00 | 26.48 |

| | | | | | | | | | |
|---|---|---|---|---|---|---|---|---|---|
| ATOM | 3142 | O | TYR | 413 | 34.085 | -3.849 | 77.534 | 1.00 | 26.86 |
| ATOM | 3143 | N | LYS | 414 | 32.690 | -2.108 | 77.669 | 1.00 | 26.13 |
| ATOM | 3144 | CA | LYS | 414 | 33.309 | -1.640 | 78.902 | 1.00 | 29.80 |
| ATOM | 3145 | CB | LYS | 414 | 33.001 | -0.147 | 79.117 | 1.00 | 29.42 |
| ATOM | 3146 | CG | LYS | 414 | 33.882 | 0.802 | 78.302 | 1.00 | 32.94 |
| ATOM | 3147 | CD | LYS | 414 | 33.558 | 2.275 | 78.559 | 1.00 | 34.12 |
| ATOM | 3148 | CE | LYS | 414 | 34.553 | 3.179 | 77.833 | 1.00 | 36.09 |
| ATOM | 3149 | NZ | LYS | 414 | 34.170 | 4.626 | 77.859 | 1.00 | 35.28 |
| ATOM | 3150 | C | LYS | 414 | 32.966 | -2.400 | 80.181 | 1.00 | 29.74 |
| ATOM | 3151 | O | LYS | 414 | 33.850 | -2.677 | 80.988 | 1.00 | 28.77 |
| ATOM | 3152 | N | LEU | 415 | 31.696 | -2.749 | 80.357 | 1.00 | 30.99 |
| ATOM | 3153 | CA | LEU | 415 | 31.255 | -3.395 | 81.591 | 1.00 | 34.39 |
| ATOM | 3154 | CB | LEU | 415 | 29.942 | -2.738 | 82.041 | 1.00 | 34.94 |
| ATOM | 3155 | CG | LEU | 415 | 29.964 | -1.195 | 82.012 | 1.00 | 38.17 |
| ATOM | 3156 | CD1 | LEU | 415 | 28.610 | -0.647 | 82.469 | 1.00 | 38.59 |
| ATOM | 3157 | CD2 | LEU | 415 | 31.080 | -0.660 | 82.901 | 1.00 | 34.42 |
| ATOM | 3158 | C | LEU | 415 | 31.113 | -4.923 | 81.657 | 1.00 | 34.46 |
| ATOM | 3159 | O | LEU | 415 | 31.202 | -5.493 | 82.741 | 1.00 | 33.89 |
| ATOM | 3160 | N | HIS | 416 | 30.886 | -5.586 | 80.531 | 1.00 | 34.56 |
| ATOM | 3161 | CA | HIS | 416 | 30.746 | -7.041 | 80.561 | 1.00 | 36.94 |
| ATOM | 3162 | CB | HIS | 416 | 30.394 | -7.572 | 79.175 | 1.00 | 39.81 |
| ATOM | 3163 | CG | HIS | 416 | 29.811 | -8.949 | 79.192 | 1.00 | 44.04 |
| ATOM | 3164 | CD2 | HIS | 416 | 28.536 | -9.375 | 79.038 | 1.00 | 43.02 |
| ATOM | 3165 | ND1 | HIS | 416 | 30.573 | -10.080 | 79.402 | 1.00 | 44.57 |
| ATOM | 3166 | CE1 | HIS | 416 | 29.791 | -11.144 | 79.374 | 1.00 | 44.76 |
| ATOM | 3167 | NE2 | HIS | 416 | 28.550 | -10.744 | 79.156 | 1.00 | 46.14 |
| ATOM | 3168 | C | HIS | 416 | 32.046 | -7.673 | 81.060 | 1.00 | 35.57 |
| ATOM | 3169 | O | HIS | 416 | 33.103 | -7.483 | 80.471 | 1.00 | 37.06 |
| ATOM | 3170 | N | PRO | 417 | 31.973 | -8.445 | 82.153 | 1.00 | 33.55 |
| ATOM | 3171 | CD | PRO | 417 | 30.727 | -8.999 | 82.700 | 1.00 | 32.04 |
| ATOM | 3172 | CA | PRO | 417 | 33.134 | -9.109 | 82.757 | 1.00 | 33.47 |
| ATOM | 3173 | CB | PRO | 417 | 32.504 | -10.219 | 83.614 | 1.00 | 31.67 |
| ATOM | 3174 | CG | PRO | 417 | 31.142 | -10.410 | 83.016 | 1.00 | 32.88 |
| ATOM | 3175 | C | PRO | 417 | 34.252 | -9.628 | 81.849 | 1.00 | 32.70 |
| ATOM | 3176 | O | PRO | 417 | 35.428 | -9.411 | 82.146 | 1.00 | 36.28 |

| | | | | | | | | | |
|---|---|---|---|---|---|---|---|---|---|
| ATOM | 3177 | N | SER | 418 | 33.929 | -10.302 | 80.752 | 1.00 | 29.70 |
| ATOM | 3178 | CA | SER | 418 | 35.015 | -10.808 | 79.915 | 1.00 | 28.37 |
| ATOM | 3179 | CB | SER | 418 | 35.215 | -12.314 | 80.163 | 1.00 | 30.65 |
| ATOM | 3180 | OG | SER | 418 | 35.798 | -12.555 | 81.439 | 1.00 | 35.61 |
| ATOM | 3181 | C | SER | 418 | 34.895 | -10.560 | 78.418 | 1.00 | 25.27 |
| ATOM | 3182 | O | SER | 418 | 35.730 | -11.028 | 77.648 | 1.00 | 23.69 |
| ATOM | 3183 | N | PHE | 419 | 33.856 | -9.846 | 78.004 | 1.00 | 21.86 |
| ATOM | 3184 | CA | PHE | 419 | 33.673 | -9.543 | 76.587 | 1.00 | 24.13 |
| ATOM | 3185 | CB | PHE | 419 | 32.551 | -8.522 | 76.407 | 1.00 | 22.03 |
| ATOM | 3186 | CG | PHE | 419 | 32.270 | -8.187 | 74.978 | 1.00 | 24.42 |
| ATOM | 3187 | CD1 | PHE | 419 | 31.273 | -8.860 | 74.276 | 1.00 | 23.32 |
| ATOM | 3188 | CD2 | PHE | 419 | 33.033 | -7.231 | 74.312 | 1.00 | 22.16 |
| ATOM | 3189 | CE1 | PHE | 419 | 31.038 | -8.593 | 72.932 | 1.00 | 23.49 |
| ATOM | 3190 | CE2 | PHE | 419 | 32.808 | -6.961 | 72.967 | 1.00 | 25.91 |
| ATOM | 3191 | CZ | PHE | 419 | 31.806 | -7.645 | 72.275 | 1.00 | 24.70 |
| ATOM | 3192 | C | PHE | 419 | 34.961 | -8.965 | 76.000 | 1.00 | 24.09 |
| ATOM | 3193 | O | PHE | 419 | 35.491 | -9.455 | 75.009 | 1.00 | 26.51 |
| ATOM | 3194 | N | LYS | 420 | 35.432 | -7.899 | 76.628 | 1.00 | 25.00 |
| ATOM | 3195 | CA | LYS | 420 | 36.641 | -7.179 | 76.238 | 1.00 | 26.79 |
| ATOM | 3196 | CB | LYS | 420 | 36.984 | -6.207 | 77.370 | 1.00 | 28.35 |
| ATOM | 3197 | CG | LYS | 420 | 38.241 | -5.396 | 77.229 | 1.00 | 30.04 |
| ATOM | 3198 | CD | LYS | 420 | 38.433 | -4.537 | 78.497 | 1.00 | 33.98 |
| ATOM | 3199 | CE | LYS | 420 | 37.170 | -3.740 | 78.832 | 1.00 | 31.09 |
| ATOM | 3200 | NZ | LYS | 420 | 37.322 | -2.923 | 80.067 | 1.00 | 36.69 |
| ATOM | 3201 | C | LYS | 420 | 37.819 | -8.118 | 75.968 | 1.00 | 25.76 |
| ATOM | 3202 | O | LYS | 420 | 38.446 | -8.064 | 74.911 | 1.00 | 25.94 |
| ATOM | 3203 | N | GLU | 421 | 38.111 | -8.961 | 76.951 | 1.00 | 24.13 |
| ATOM | 3204 | CA | GLU | 421 | 39.195 | -9.929 | 76.887 | 1.00 | 26.26 |
| ATOM | 3205 | CB | GLU | 421 | 39.204 | -10.781 | 78.155 | 1.00 | 32.38 |
| ATOM | 3206 | CG | GLU | 421 | 39.547 | -10.043 | 79.417 | 1.00 | 38.45 |
| ATOM | 3207 | CD | GLU | 421 | 38.700 | -8.798 | 79.664 | 1.00 | 41.54 |
| ATOM | 3208 | OE1 | GLU | 421 | 37.458 | -8.844 | 79.501 | 1.00 | 42.17 |
| ATOM | 3209 | OE2 | GLU | 421 | 39.300 | -7.767 | 80.053 | 1.00 | 42.62 |
| ATOM | 3210 | C | GLU | 421 | 39.075 | -10.864 | 75.699 | 1.00 | 24.57 |
| ATOM | 3211 | O | GLU | 421 | 40.017 | -11.023 | 74.930 | 1.00 | 25.86 |

| | | | | | | | | | |
|---|---|---|---|---|---|---|---|---|---|
| ATOM | 3212 | N | ARG | 422 | 37.921 | -11.509 | 75.576 | 1.00 | 24.00 |
| ATOM | 3213 | CA | ARG | 422 | 37.682 | -12.439 | 74.480 | 1.00 | 26.01 |
| ATOM | 3214 | CB | ARG | 422 | 36.284 | -13.063 | 74.610 | 1.00 | 27.36 |
| ATOM | 3215 | CG | ARG | 422 | 36.076 | -13.878 | 75.887 | 1.00 | 31.58 |
| ATOM | 3216 | CD | ARG | 422 | 34.600 | -14.053 | 76.188 | 1.00 | 35.39 |
| ATOM | 3217 | NE | ARG | 422 | 34.390 | -14.834 | 77.397 | 1.00 | 40.58 |
| ATOM | 3218 | CZ | ARG | 422 | 33.232 | -14.911 | 78.046 | 1.00 | 44.53 |
| ATOM | 3219 | NH1 | ARG | 422 | 32.171 | -14.243 | 77.596 | 1.00 | 41.79 |
| ATOM | 3220 | NH2 | ARG | 422 | 33.141 | -15.651 | 79.150 | 1.00 | 41.67 |
| ATOM | 3221 | C | ARG | 422 | 37.794 | -11.691 | 73.160 | 1.00 | 24.48 |
| ATOM | 3222 | O | ARG | 422 | 38.439 | -12.148 | 72.221 | 1.00 | 22.97 |
| ATOM | 3223 | N | PHE | 423 | 37.153 | -10.531 | 73.094 | 1.00 | 24.48 |
| ATOM | 3224 | CA | PHE | 423 | 37.189 | -9.737 | 71.879 | 1.00 | 22.97 |
| ATOM | 3225 | CB | PHE | 423 | 36.403 | -8.442 | 72.089 | 1.00 | 24.98 |
| ATOM | 3226 | CG | PHE | 423 | 36.494 | -7.484 | 70.939 | 1.00 | 25.21 |
| ATOM | 3227 | CD1 | PHE | 423 | 37.468 | -6.490 | 70.926 | 1.00 | 25.04 |
| ATOM | 3228 | CD2 | PHE | 423 | 35.618 | -7.584 | 69.861 | 1.00 | 23.47 |
| ATOM | 3229 | CE1 | PHE | 423 | 37.568 | -5.607 | 69.857 | 1.00 | 24.77 |
| ATOM | 3230 | CE2 | PHE | 423 | 35.710 | -6.708 | 68.784 | 1.00 | 25.48 |
| ATOM | 3231 | CZ | PHE | 423 | 36.684 | -5.715 | 68.780 | 1.00 | 24.31 |
| ATOM | 3232 | C | PHE | 423 | 38.629 | -9.442 | 71.456 | 1.00 | 21.03 |
| ATOM | 3233 | O | PHE | 423 | 38.989 | -9.680 | 70.308 | 1.00 | 19.38 |
| ATOM | 3234 | N | HIS | 424 | 39.454 | -8.952 | 72.381 | 1.00 | 20.46 |
| ATOM | 3235 | CA | HIS | 424 | 40.846 | -8.631 | 72.054 | 1.00 | 23.40 |
| ATOM | 3236 | CB | HIS | 424 | 41.602 | -8.128 | 73.293 | 1.00 | 24.89 |
| ATOM | 3237 | CG | HIS | 424 | 41.133 | -6.803 | 73.808 | 1.00 | 25.28 |
| ATOM | 3238 | CD2 | HIS | 424 | 40.391 | -5.828 | 73.230 | 1.00 | 24.67 |
| ATOM | 3239 | ND1 | HIS | 424 | 41.419 | -6.361 | 75.083 | 1.00 | 25.18 |
| ATOM | 3240 | CE1 | HIS | 424 | 40.869 | -5.174 | 75.269 | 1.00 | 22.64 |
| ATOM | 3241 | NE2 | HIS | 424 | 40.239 | -4.829 | 74.161 | 1.00 | 24.12 |
| ATOM | 3242 | C | HIS | 424 | 41.604 | -9.834 | 71.486 | 1.00 | 24.51 |
| ATOM | 3243 | O | HIS | 424 | 42.239 | -9.741 | 70.432 | 1.00 | 23.58 |
| ATOM | 3244 | N | ALA | 425 | 41.540 | -10.962 | 72.191 | 1.00 | 24.51 |
| ATOM | 3245 | CA | ALA | 425 | 42.242 | -12.164 | 71.746 | 1.00 | 26.94 |
| ATOM | 3246 | CB | ALA | 425 | 42.068 | -13.306 | 72.774 | 1.00 | 27.10 |

| | | | | | | | | | |
|---|---|---|---|---|---|---|---|---|---|
| ATOM | 3247 | C | ALA | 425 | 41.759 | -12.605 | 70.370 | 1.00 | 25.71 |
| ATOM | 3248 | O | ALA | 425 | 42.559 | -12.937 | 69.505 | 1.00 | 27.02 |
| ATOM | 3249 | N | SER | 426 | 40.453 | -12.600 | 70.151 | 1.00 | 24.30 |
| ATOM | 3250 | CA | SER | 426 | 39.967 | -13.003 | 68.850 | 1.00 | 23.93 |
| ATOM | 3251 | CB | SER | 426 | 38.450 | -13.142 | 68.863 | 1.00 | 20.85 |
| ATOM | 3252 | OG | SER | 426 | 38.007 | -13.582 | 67.596 | 1.00 | 21.86 |
| ATOM | 3253 | C | SER | 426 | 40.394 | -12.039 | 67.743 | 1.00 | 25.72 |
| ATOM | 3254 | O | SER | 426 | 40.760 | -12.483 | 66.660 | 1.00 | 25.40 |
| ATOM | 3255 | N | VAL | 427 | 40.363 | -10.727 | 68.007 | 1.00 | 27.03 |
| ATOM | 3256 | CA | VAL | 427 | 40.761 | -9.750 | 66.983 | 1.00 | 27.43 |
| ATOM | 3257 | CB | VAL | 427 | 40.591 | -8.269 | 67.450 | 1.00 | 28.91 |
| ATOM | 3258 | CG1 | VAL | 427 | 40.999 | -7.323 | 66.314 | 1.00 | 29.57 |
| ATOM | 3259 | CG2 | VAL | 427 | 39.150 | -7.990 | 67.852 | 1.00 | 27.73 |
| ATOM | 3260 | C | VAL | 427 | 42.226 | -9.919 | 66.601 | 1.00 | 28.67 |
| ATOM | 3261 | O | VAL | 427 | 42.582 | -9.858 | 65.424 | 1.00 | 27.30 |
| ATOM | 3262 | N | ARG | 428 | 43.076 | -10.119 | 67.603 | 1.00 | 28.43 |
| ATOM | 3263 | CA | ARG | 428 | 44.498 | -10.281 | 67.350 | 1.00 | 31.91 |
| ATOM | 3264 | CB | ARG | 428 | 45.273 | -10.231 | 68.670 | 1.00 | 31.80 |
| ATOM | 3265 | CG | ARG | 428 | 45.449 | -8.793 | 69.130 | 1.00 | 31.90 |
| ATOM | 3266 | CD | ARG | 428 | 45.662 | -8.639 | 70.617 | 1.00 | 34.40 |
| ATOM | 3267 | NE | ARG | 428 | 45.867 | -7.231 | 70.971 | 1.00 | 34.78 |
| ATOM | 3268 | CZ | ARG | 428 | 45.668 | -6.728 | 72.186 | 1.00 | 38.69 |
| ATOM | 3269 | NH1 | ARG | 428 | 45.251 | -7.516 | 73.172 | 1.00 | 38.56 |
| ATOM | 3270 | NH2 | ARG | 428 | 45.901 | -5.442 | 72.424 | 1.00 | 39.08 |
| ATOM | 3271 | C | ARG | 428 | 44.797 | -11.548 | 66.572 | 1.00 | 33.56 |
| ATOM | 3272 | O | ARG | 428 | 45.694 | -11.558 | 65.731 | 1.00 | 32.81 |
| ATOM | 3273 | N | ARG | 429 | 44.037 | -12.609 | 66.837 | 1.00 | 34.25 |
| ATOM | 3274 | CA | ARG | 429 | 44.224 | -13.859 | 66.115 | 1.00 | 33.42 |
| ATOM | 3275 | CB | ARG | 429 | 43.252 | -14.941 | 66.601 | 1.00 | 36.36 |
| ATOM | 3276 | CG | ARG | 429 | 43.756 | -15.769 | 67.760 | 1.00 | 43.73 |
| ATOM | 3277 | CD | ARG | 429 | 42.930 | -17.038 | 67.939 | 1.00 | 47.67 |
| ATOM | 3278 | NE | ARG | 429 | 41.561 | -16.789 | 68.398 | 1.00 | 51.58 |
| ATOM | 3279 | CZ | ARG | 429 | 41.222 | -16.467 | 69.646 | 1.00 | 51.70 |
| ATOM | 3280 | NH1 | ARG | 429 | 42.154 | -16.345 | 70.585 | 1.00 | 50.85 |
| ATOM | 3281 | NH2 | ARG | 429 | 39.945 | -16.288 | 69.962 | 1.00 | 49.95 |

| | | | | | | | | | |
|---|---|---|---|---|---|---|---|---|---|
| ATOM | 3282 | C | ARG | 429 | 43.960 | -13.618 | 64.639 | 1.00 | 32.81 |
| ATOM | 3283 | O | ARG | 429 | 44.610 | -14.215 | 63.783 | 1.00 | 32.29 |
| ATOM | 3284 | N | LEU | 430 | 43.001 | -12.741 | 64.345 | 1.00 | 30.18 |
| ATOM | 3285 | CA | LEU | 430 | 42.623 | -12.455 | 62.965 | 1.00 | 29.19 |
| ATOM | 3286 | CB | LEU | 430 | 41.132 | -12.109 | 62.904 | 1.00 | 29.15 |
| ATOM | 3287 | CG | LEU | 430 | 40.173 | -13.164 | 63.453 | 1.00 | 31.83 |
| ATOM | 3288 | CD1 | LEU | 430 | 38.746 | -12.629 | 63.437 | 1.00 | 28.32 |
| ATOM | 3289 | CD2 | LEU | 430 | 40.281 | -14.441 | 62.613 | 1.00 | 32.03 |
| ATOM | 3290 | C | LEU | 430 | 43.407 | -11.355 | 62.251 | 1.00 | 27.64 |
| ATOM | 3291 | O | LEU | 430 | 43.244 | -11.151 | 61.048 | 1.00 | 28.08 |
| ATOM | 3292 | N | THR | 431 | 44.261 | -10.645 | 62.966 | 1.00 | 28.04 |
| ATOM | 3293 | CA | THR | 431 | 44.988 | -9.567 | 62.326 | 1.00 | 31.15 |
| ATOM | 3294 | CB | THR | 431 | 44.569 | -8.201 | 62.934 | 1.00 | 30.03 |
| ATOM | 3295 | OG1 | THR | 431 | 44.666 | -8.254 | 64.363 | 1.00 | 31.84 |
| ATOM | 3296 | CG2 | THR | 431 | 43.137 | -7.879 | 62.561 | 1.00 | 26.93 |
| ATOM | 3297 | C | THR | 431 | 46.507 | -9.719 | 62.367 | 1.00 | 34.65 |
| ATOM | 3298 | O | THR | 431 | 47.190 | -9.015 | 63.101 | 1.00 | 34.08 |
| ATOM | 3299 | N | PRO | 432 | 47.049 | -10.655 | 61.566 | 1.00 | 37.36 |
| ATOM | 3300 | CD | PRO | 432 | 46.296 | -11.603 | 60.726 | 1.00 | 37.91 |
| ATOM | 3301 | CA | PRO | 432 | 48.489 | -10.923 | 61.484 | 1.00 | 38.59 |
| ATOM | 3302 | CB | PRO | 432 | 48.572 | -12.080 | 60.487 | 1.00 | 38.98 |
| ATOM | 3303 | CG | PRO | 432 | 47.245 | -12.758 | 60.630 | 1.00 | 40.10 |
| ATOM | 3304 | C | PRO | 432 | 49.224 | -9.689 | 60.969 | 1.00 | 39.65 |
| ATOM | 3305 | O | PRO | 432 | 48.712 | -8.968 | 60.113 | 1.00 | 39.80 |
| ATOM | 3306 | N | SER | 433 | 50.420 | -9.461 | 61.495 | 1.00 | 39.94 |
| ATOM | 3307 | CA | SER | 433 | 51.254 | -8.326 | 61.112 | 1.00 | 42.47 |
| ATOM | 3308 | CB | SER | 433 | 51.467 | -8.280 | 59.586 | 1.00 | 44.12 |
| ATOM | 3309 | OG | SER | 433 | 50.363 | -7.707 | 58.898 | 1.00 | 48.10 |
| ATOM | 3310 | C | SER | 433 | 50.687 | -6.996 | 61.598 | 1.00 | 42.26 |
| ATOM | 3311 | O | SER | 433 | 51.085 | -5.932 | 61.121 | 1.00 | 42.50 |
| ATOM | 3312 | N | CYS | 434 | 49.756 | -7.053 | 62.544 | 1.00 | 40.68 |
| ATOM | 3313 | CA | CYS | 434 | 49.184 | -5.831 | 63.092 | 1.00 | 40.64 |
| ATOM | 3314 | CB | CYS | 434 | 47.679 | -5.735 | 62.826 | 1.00 | 39.36 |
| ATOM | 3315 | SG | CYS | 434 | 47.196 | -5.674 | 61.111 | 1.00 | 39.36 |
| ATOM | 3316 | C | CYS | 434 | 49.398 | -5.789 | 64.590 | 1.00 | 40.17 |

| | | | | | | | | | |
|---|---|---|---|---|---|---|---|---|---|
| ATOM | 3317 | O | CYS | 434 | 49.258 | -6.801 | 65.281 | 1.00 | 40.51 |
| ATOM | 3318 | N | GLU | 435 | 49.743 | -4.609 | 65.081 | 1.00 | 38.91 |
| ATOM | 3319 | CA | GLU | 435 | 49.945 | -4.388 | 66.504 | 1.00 | 39.30 |
| ATOM | 3320 | CB | GLU | 435 | 51.302 | -3.733 | 66.738 | 1.00 | 42.29 |
| ATOM | 3321 | CG | GLU | 435 | 51.779 | -3.766 | 68.162 | 1.00 | 49.24 |
| ATOM | 3322 | CD | GLU | 435 | 53.072 | -2.993 | 68.340 | 1.00 | 53.77 |
| ATOM | 3323 | OE1 | GLU | 435 | 54.106 | -3.421 | 67.781 | 1.00 | 56.24 |
| ATOM | 3324 | OE2 | GLU | 435 | 53.047 | -1.950 | 69.032 | 1.00 | 54.88 |
| ATOM | 3325 | C | GLU | 435 | 48.801 | -3.430 | 66.839 | 1.00 | 37.06 |
| ATOM | 3326 | O | GLU | 435 | 48.866 | -2.241 | 66.532 | 1.00 | 34.30 |
| ATOM | 3327 | N | ILE | 436 | 47.749 | -3.971 | 67.449 | 1.00 | 36.39 |
| ATOM | 3328 | CA | ILE | 436 | 46.552 | -3.203 | 67.786 | 1.00 | 34.47 |
| ATOM | 3329 | CB | ILE | 436 | 45.280 | -4.040 | 67.508 | 1.00 | 34.15 |
| ATOM | 3330 | CG2 | ILE | 436 | 44.024 | -3.166 | 67.639 | 1.00 | 33.64 |
| ATOM | 3331 | CG1 | ILE | 436 | 45.357 | -4.633 | 66.100 | 1.00 | 36.06 |
| ATOM | 3332 | CD1 | ILE | 436 | 44.166 | -5.487 | 65.719 | 1.00 | 36.35 |
| ATOM | 3333 | C | ILE | 436 | 46.492 | -2.717 | 69.228 | 1.00 | 34.50 |
| ATOM | 3334 | O | ILE | 436 | 46.612 | -3.506 | 70.164 | 1.00 | 35.88 |
| ATOM | 3335 | N | THR | 437 | 46.308 | -1.411 | 69.405 | 1.00 | 32.66 |
| ATOM | 3336 | CA | THR | 437 | 46.196 | -0.837 | 70.741 | 1.00 | 30.32 |
| ATOM | 3337 | CB | THR | 437 | 47.134 | 0.370 | 70.930 | 1.00 | 29.83 |
| ATOM | 3338 | OG1 | THR | 437 | 48.496 | -0.060 | 70.833 | 1.00 | 33.74 |
| ATOM | 3339 | CG2 | THR | 437 | 46.925 | 0.996 | 72.294 | 1.00 | 28.96 |
| ATOM | 3340 | C | THR | 437 | 44.759 | -0.377 | 70.949 | 1.00 | 29.92 |
| ATOM | 3341 | O | THR | 437 | 44.177 | 0.293 | 70.090 | 1.00 | 28.24 |
| ATOM | 3342 | N | PHE | 438 | 44.179 | -0.750 | 72.083 | 1.00 | 29.43 |
| ATOM | 3343 | CA | PHE | 438 | 42.807 | -0.359 | 72.390 | 1.00 | 29.35 |
| ATOM | 3344 | CB | PHE | 438 | 41.991 | -1.567 | 72.853 | 1.00 | 27.92 |
| ATOM | 3345 | CG | PHE | 438 | 41.794 | -2.614 | 71.789 | 1.00 | 27.95 |
| ATOM | 3346 | CD1 | PHE | 438 | 42.695 | -3.661 | 71.648 | 1.00 | 27.90 |
| ATOM | 3347 | CD2 | PHE | 438 | 40.703 | -2.549 | 70.930 | 1.00 | 24.76 |
| ATOM | 3348 | CE1 | PHE | 438 | 42.505 | -4.634 | 70.662 | 1.00 | 29.21 |
| ATOM | 3349 | CE2 | PHE | 438 | 40.506 | -3.505 | 69.950 | 1.00 | 28.87 |
| ATOM | 3350 | CZ | PHE | 438 | 41.408 | -4.554 | 69.814 | 1.00 | 28.70 |
| ATOM | 3351 | C | PHE | 438 | 42.772 | 0.712 | 73.467 | 1.00 | 30.41 |

| | | | | | | | | | |
|---|---|---|---|---|---|---|---|---|---|
| ATOM | 3352 | O | PHE | 438 | 43.469 | 0.601 | 74.474 | 1.00 | 30.53 |
| ATOM | 3353 | N | ILE | 439 | 41.968 | 1.752 | 73.250 | 1.00 | 30.35 |
| ATOM | 3354 | CA | ILE | 439 | 41.839 | 2.832 | 74.220 | 1.00 | 31.89 |
| ATOM | 3355 | CB | ILE | 439 | 42.544 | 4.124 | 73.751 | 1.00 | 33.03 |
| ATOM | 3356 | CG2 | ILE | 439 | 42.233 | 5.269 | 74.721 | 1.00 | 36.00 |
| ATOM | 3357 | CG1 | ILE | 439 | 44.053 | 3.916 | 73.704 | 1.00 | 33.82 |
| ATOM | 3358 | CD1 | ILE | 439 | 44.818 | 5.165 | 73.296 | 1.00 | 36.93 |
| ATOM | 3359 | C | ILE | 439 | 40.373 | 3.158 | 74.420 | 1.00 | 32.85 |
| ATOM | 3360 | O | ILE | 439 | 39.603 | 3.157 | 73.467 | 1.00 | 33.09 |
| ATOM | 3361 | N | GLU | 440 | 39.991 | 3.442 | 75.659 | 1.00 | 35.09 |
| ATOM | 3362 | CA | GLU | 440 | 38.608 | 3.789 | 75.956 | 1.00 | 39.34 |
| ATOM | 3363 | CB | GLU | 440 | 38.133 | 3.041 | 77.199 | 1.00 | 37.95 |
| ATOM | 3364 | CG | GLU | 440 | 38.213 | 1.526 | 77.038 | 1.00 | 39.96 |
| ATOM | 3365 | CD | GLU | 440 | 37.837 | 0.773 | 78.298 | 1.00 | 40.82 |
| ATOM | 3366 | OE1 | GLU | 440 | 38.058 | -0.456 | 78.340 | 1.00 | 41.23 |
| ATOM | 3367 | OE2 | GLU | 440 | 37.318 | 1.403 | 79.245 | 1.00 | 41.08 |
| ATOM | 3368 | C | GLU | 440 | 38.495 | 5.298 | 76.156 | 1.00 | 41.28 |
| ATOM | 3369 | O | GLU | 440 | 39.356 | 5.918 | 76.769 | 1.00 | 42.10 |
| ATOM | 3370 | N | SER | 441 | 37.431 | 5.886 | 75.627 | 1.00 | 43.99 |
| ATOM | 3371 | CA | SER | 441 | 37.231 | 7.327 | 75.738 | 1.00 | 48.53 |
| ATOM | 3372 | CB | SER | 441 | 36.390 | 7.823 | 74.550 | 1.00 | 47.96 |
| ATOM | 3373 | OG | SER | 441 | 35.196 | 7.066 | 74.390 | 1.00 | 48.42 |
| ATOM | 3374 | C | SER | 441 | 36.577 | 7.752 | 77.051 | 1.00 | 50.20 |
| ATOM | 3375 | O | SER | 441 | 35.654 | 7.087 | 77.531 | 1.00 | 51.01 |
| ATOM | 3376 | N | GLU | 442 | 37.060 | 8.852 | 77.634 | 1.00 | 53.24 |
| ATOM | 3377 | CA | GLU | 442 | 36.490 | 9.359 | 78.885 | 1.00 | 55.51 |
| ATOM | 3378 | CB | GLU | 442 | 37.362 | 10.454 | 79.507 | 1.00 | 60.16 |
| ATOM | 3379 | CG | GLU | 442 | 36.822 | 10.936 | 80.859 | 1.00 | 65.44 |
| ATOM | 3380 | CD | GLU | 442 | 37.596 | 12.107 | 81.450 | 1.00 | 69.63 |
| ATOM | 3381 | OE1 | GLU | 442 | 38.824 | 11.984 | 81.667 | 1.00 | 71.27 |
| ATOM | 3382 | OE2 | GLU | 442 | 36.965 | 13.155 | 81.709 | 1.00 | 72.91 |
| ATOM | 3383 | C | GLU | 442 | 35.118 | 9.938 | 78.579 | 1.00 | 54.68 |
| ATOM | 3384 | O | GLU | 442 | 34.104 | 9.495 | 79.126 | 1.00 | 56.30 |
| ATOM | 3385 | N | GLU | 443 | 35.094 | 10.942 | 77.714 | 1.00 | 51.73 |
| ATOM | 3386 | CA | GLU | 443 | 33.840 | 11.555 | 77.307 | 1.00 | 51.12 |

| | | | | | | | | | |
|---|---|---|---|---|---|---|---|---|---|
| ATOM | 3387 | CB | GLU | 443 | 33.706 | 12.960 | 77.888 | 1.00 | 51.77 |
| ATOM | 3388 | CG | GLU | 443 | 32.561 | 13.086 | 78.869 | 1.00 | 49.05 |
| ATOM | 3389 | CD | GLU | 443 | 31.202 | 12.812 | 78.239 | 1.00 | 48.41 |
| ATOM | 3390 | OE1 | GLU | 443 | 30.245 | 12.572 | 79.006 | 1.00 | 48.06 |
| ATOM | 3391 | OE2 | GLU | 443 | 31.084 | 12.842 | 76.990 | 1.00 | 44.46 |
| ATOM | 3392 | C | GLU | 443 | 33.851 | 11.614 | 75.793 | 1.00 | 50.48 |
| ATOM | 3393 | O | GLU | 443 | 33.624 | 12.662 | 75.191 | 1.00 | 50.61 |
| ATOM | 3394 | N | GLY | 444 | 34.131 | 10.458 | 75.199 | 1.00 | 49.69 |
| ATOM | 3395 | CA | GLY | 444 | 34.213 | 10.321 | 73.760 | 1.00 | 46.29 |
| ATOM | 3396 | C | GLY | 444 | 33.300 | 11.190 | 72.928 | 1.00 | 45.39 |
| ATOM | 3397 | O | GLY | 444 | 33.786 | 12.031 | 72.181 | 1.00 | 44.10 |
| ATOM | 3398 | N | SER | 445 | 31.990 | 10.996 | 73.052 | 1.00 | 44.40 |
| ATOM | 3399 | CA | SER | 445 | 31.035 | 11.765 | 72.263 | 1.00 | 45.82 |
| ATOM | 3400 | CB | SER | 445 | 29.614 | 11.258 | 72.505 | 1.00 | 43.70 |
| ATOM | 3401 | OG | SER | 445 | 29.248 | 11.396 | 73.860 | 1.00 | 51.13 |
| ATOM | 3402 | C | SER | 445 | 31.108 | 13.265 | 72.523 | 1.00 | 45.79 |
| ATOM | 3403 | O | SER | 445 | 31.381 | 14.043 | 71.607 | 1.00 | 46.62 |
| ATOM | 3404 | N | GLY | 446 | 30.867 | 13.666 | 73.766 | 1.00 | 45.46 |
| ATOM | 3405 | CA | GLY | 446 | 30.924 | 15.075 | 74.112 | 1.00 | 44.61 |
| ATOM | 3406 | C | GLY | 446 | 32.176 | 15.778 | 73.615 | 1.00 | 44.65 |
| ATOM | 3407 | O | GLY | 446 | 32.085 | 16.754 | 72.872 | 1.00 | 45.17 |
| ATOM | 3408 | N | ARG | 447 | 33.344 | 15.286 | 74.024 | 1.00 | 44.10 |
| ATOM | 3409 | CA | ARG | 447 | 34.615 | 15.878 | 73.615 | 1.00 | 44.23 |
| ATOM | 3410 | CB | ARG | 447 | 35.765 | 15.244 | 74.396 | 1.00 | 44.71 |
| ATOM | 3411 | CG | ARG | 447 | 36.079 | 15.917 | 75.720 | 1.00 | 46.63 |
| ATOM | 3412 | CD | ARG | 447 | 36.405 | 14.896 | 76.794 | 1.00 | 48.87 |
| ATOM | 3413 | NE | ARG | 447 | 37.226 | 13.804 | 76.286 | 1.00 | 53.97 |
| ATOM | 3414 | CZ | ARG | 447 | 38.507 | 13.915 | 75.956 | 1.00 | 55.65 |
| ATOM | 3415 | NH1 | ARG | 447 | 39.130 | 15.076 | 76.085 | 1.00 | 56.71 |
| ATOM | 3416 | NH2 | ARG | 447 | 39.161 | 12.862 | 75.486 | 1.00 | 58.00 |
| ATOM | 3417 | C | ARG | 447 | 34.891 | 15.739 | 72.122 | 1.00 | 45.53 |
| ATOM | 3418 | O | ARG | 447 | 35.506 | 16.617 | 71.508 | 1.00 | 45.95 |
| ATOM | 3419 | N | GLY | 448 | 34.444 | 14.630 | 71.543 | 1.00 | 45.12 |
| ATOM | 3420 | CA | GLY | 448 | 34.667 | 14.395 | 70.129 | 1.00 | 43.75 |
| ATOM | 3421 | C | GLY | 448 | 33.915 | 15.390 | 69.275 | 1.00 | 44.42 |

| | | | | | | | | | |
|---|---|---|---|---|---|---|---|---|---|
| ATOM | 3422 | O | GLY | 448 | 34.497 | 16.033 | 68.401 | 1.00 | 43.25 |
| ATOM | 3423 | N | ALA | 449 | 32.617 | 15.508 | 69.530 | 1.00 | 44.33 |
| ATOM | 3424 | CA | ALA | 449 | 31.764 | 16.435 | 68.798 | 1.00 | 46.02 |
| ATOM | 3425 | CB | ALA | 449 | 30.349 | 16.393 | 69.362 | 1.00 | 44.02 |
| ATOM | 3426 | C | ALA | 449 | 32.334 | 17.852 | 68.901 | 1.00 | 47.27 |
| ATOM | 3427 | O | ALA | 449 | 32.388 | 18.585 | 67.910 | 1.00 | 46.99 |
| ATOM | 3428 | N | ALA | 450 | 32.771 | 18.226 | 70.100 | 1.00 | 47.32 |
| ATOM | 3429 | CA | ALA | 450 | 33.337 | 19.549 | 70.320 | 1.00 | 48.93 |
| ATOM | 3430 | CB | ALA | 450 | 33.590 | 19.771 | 71.803 | 1.00 | 48.70 |
| ATOM | 3431 | C | ALA | 450 | 34.630 | 19.752 | 69.537 | 1.00 | 49.10 |
| ATOM | 3432 | O | ALA | 450 | 34.795 | 20.770 | 68.864 | 1.00 | 51.55 |
| ATOM | 3433 | N | LEU | 451 | 35.546 | 18.792 | 69.625 | 1.00 | 47.13 |
| ATOM | 3434 | CA | LEU | 451 | 36.828 | 18.889 | 68.923 | 1.00 | 46.08 |
| ATOM | 3435 | CB | LEU | 451 | 37.693 | 17.661 | 69.226 | 1.00 | 43.72 |
| ATOM | 3436 | CG | LEU | 451 | 38.376 | 17.636 | 70.598 | 1.00 | 44.07 |
| ATOM | 3437 | CD1 | LEU | 451 | 38.798 | 16.218 | 70.955 | 1.00 | 41.74 |
| ATOM | 3438 | CD2 | LEU | 451 | 39.577 | 18.574 | 70.574 | 1.00 | 40.23 |
| ATOM | 3439 | C | LEU | 451 | 36.672 | 19.055 | 67.410 | 1.00 | 45.46 |
| ATOM | 3440 | O | LEU | 451 | 37.495 | 19.708 | 66.760 | 1.00 | 46.36 |
| ATOM | 3441 | N | VAL | 452 | 35.618 | 18.465 | 66.857 | 1.00 | 43.95 |
| ATOM | 3442 | CA | VAL | 452 | 35.348 | 18.552 | 65.428 | 1.00 | 44.38 |
| ATOM | 3443 | CB | VAL | 452 | 34.426 | 17.376 | 64.959 | 1.00 | 43.85 |
| ATOM | 3444 | CG1 | VAL | 452 | 33.998 | 17.576 | 63.513 | 1.00 | 41.59 |
| ATOM | 3445 | CG2 | VAL | 452 | 35.169 | 16.040 | 65.087 | 1.00 | 40.86 |
| ATOM | 3446 | C | VAL | 452 | 34.687 | 19.905 | 65.125 | 1.00 | 45.31 |
| ATOM | 3447 | O | VAL | 452 | 34.881 | 20.482 | 64.056 | 1.00 | 42.97 |
| ATOM | 3448 | N | SER | 453 | 33.912 | 20.411 | 66.077 | 1.00 | 46.60 |
| ATOM | 3449 | CA | SER | 453 | 33.253 | 21.693 | 65.900 | 1.00 | 49.07 |
| ATOM | 3450 | CB | SER | 453 | 32.204 | 21.902 | 66.986 | 1.00 | 47.21 |
| ATOM | 3451 | OG | SER | 453 | 31.146 | 20.972 | 66.845 | 1.00 | 44.37 |
| ATOM | 3452 | C | SER | 453 | 34.293 | 22.806 | 65.951 | 1.00 | 51.53 |
| ATOM | 3453 | O | SER | 453 | 34.150 | 23.820 | 65.281 | 1.00 | 52.56 |
| ATOM | 3454 | N | ALA | 454 | 35.352 | 22.593 | 66.728 | 1.00 | 54.40 |
| ATOM | 3455 | CA | ALA | 454 | 36.430 | 23.567 | 66.881 | 1.00 | 56.39 |
| ATOM | 3456 | CB | ALA | 454 | 37.336 | 23.158 | 68.031 | 1.00 | 55.74 |

| | | | | | | | | | |
|---|---|---|---|---|---|---|---|---|---|
| ATOM | 3457 | C | ALA | 454 | 37.259 | 23.751 | 65.614 | 1.00 | 58.75 |
| ATOM | 3458 | O | ALA | 454 | 37.863 | 24.807 | 65.408 | 1.00 | 59.45 |
| ATOM | 3459 | N | VAL | 455 | 37.310 | 22.719 | 64.779 | 1.00 | 60.29 |
| ATOM | 3460 | CA | VAL | 455 | 38.063 | 22.796 | 63.535 | 1.00 | 61.78 |
| ATOM | 3461 | CB | VAL | 455 | 38.603 | 21.416 | 63.112 | 1.00 | 61.44 |
| ATOM | 3462 | CG1 | VAL | 455 | 39.090 | 21.464 | 61.672 | 1.00 | 60.81 |
| ATOM | 3463 | CG2 | VAL | 455 | 39.737 | 21.005 | 64.031 | 1.00 | 60.68 |
| ATOM | 3464 | C | VAL | 455 | 37.152 | 23.330 | 62.442 | 1.00 | 63.56 |
| ATOM | 3465 | O | VAL | 455 | 37.550 | 24.176 | 61.643 | 1.00 | 63.25 |
| ATOM | 3466 | N | ALA | 456 | 35.921 | 22.835 | 62.416 | 1.00 | 65.38 |
| ATOM | 3467 | CA | ALA | 456 | 34.959 | 23.275 | 61.422 | 1.00 | 69.39 |
| ATOM | 3468 | CB | ALA | 456 | 33.751 | 22.354 | 61.423 | 1.00 | 68.17 |
| ATOM | 3469 | C | ALA | 456 | 34.522 | 24.709 | 61.710 | 1.00 | 73.10 |
| ATOM | 3470 | O | ALA | 456 | 33.975 | 25.382 | 60.837 | 1.00 | 73.04 |
| ATOM | 3471 | N | CYS | 457 | 34.771 | 25.170 | 62.935 | 1.00 | 77.06 |
| ATOM | 3472 | CA | CYS | 457 | 34.390 | 26.521 | 63.341 | 1.00 | 81.01 |
| ATOM | 3473 | CB | CYS | 457 | 34.192 | 26.599 | 64.856 | 1.00 | 80.51 |
| ATOM | 3474 | SG | CYS | 457 | 33.478 | 28.151 | 65.432 | 1.00 | 81.75 |
| ATOM | 3475 | C | CYS | 457 | 35.420 | 27.554 | 62.916 | 1.00 | 83.65 |
| ATOM | 3476 | O | CYS | 457 | 35.312 | 28.726 | 63.275 | 1.00 | 85.11 |
| ATOM | 3477 | N | LYS | 458 | 36.430 | 27.118 | 62.172 | 1.00 | 86.29 |
| ATOM | 3478 | CA | LYS | 458 | 37.441 | 28.041 | 61.683 | 1.00 | 89.14 |
| ATOM | 3479 | CB | LYS | 458 | 38.843 | 27.441 | 61.803 | 1.00 | 88.60 |
| ATOM | 3480 | CG | LYS | 458 | 39.932 | 28.486 | 61.632 | 1.00 | 89.51 |
| ATOM | 3481 | CD | LYS | 458 | 41.276 | 27.992 | 62.130 | 1.00 | 89.70 |
| ATOM | 3482 | CE | LYS | 458 | 42.257 | 29.146 | 62.269 | 1.00 | 89.22 |
| ATOM | 3483 | NZ | LYS | 458 | 41.718 | 30.194 | 63.180 | 1.00 | 88.81 |
| ATOM | 3484 | C | LYS | 458 | 37.096 | 28.310 | 60.232 | 1.00 | 91.26 |
| ATOM | 3485 | O | LYS | 458 | 37.936 | 28.733 | 59.438 | 1.00 | 91.56 |
| ATOM | 3486 | N | LYS | 459 | 35.834 | 28.043 | 59.901 | 1.00 | 93.94 |
| ATOM | 3487 | CA | LYS | 459 | 35.302 | 28.240 | 58.548 | 1.00 | 96.28 |
| ATOM | 3488 | CB | LYS | 459 | 35.323 | 26.923 | 57.765 | 1.00 | 96.25 |
| ATOM | 3489 | CG | LYS | 459 | 36.719 | 26.409 | 57.421 | 1.00 | 96.30 |
| ATOM | 3490 | CD | LYS | 459 | 37.458 | 27.348 | 56.475 | 1.00 | 96.73 |
| ATOM | 3491 | CE | LYS | 459 | 38.833 | 26.801 | 56.111 | 1.00 | 97.12 |

| | | | | | | | | | |
|---|---|---|---|---|---|---|---|---|---|
| ATOM | 3492 | NZ | LYS | 459 | 39.577 | 27.717 | 55.197 | 1.00 | 97.75 |
| ATOM | 3493 | C | LYS | 459 | 33.863 | 28.759 | 58.624 | 1.00 | 97.78 |
| ATOM | 3494 | O | LYS | 459 | 33.417 | 29.516 | 57.758 | 1.00 | 98.11 |
| ATOM | 3495 | N | ALA | 460 | 33.153 | 28.327 | 59.666 | 1.00 | 99.29 |
| ATOM | 3496 | CA | ALA | 460 | 31.778 | 28.738 | 59.916 | 1.00 | 100.54 |
| ATOM | 3497 | CB | ALA | 460 | 31.028 | 27.644 | 60.681 | 1.00 | 100.58 |
| ATOM | 3498 | C | ALA | 460 | 31.765 | 30.042 | 60.719 | 1.00 | 101.56 |
| ATOM | 3499 | O | ALA | 460 | 30.755 | 30.750 | 60.777 | 1.00 | 101.79 |
| ATOM | 3500 | N | CYS | 461 | 32.899 | 30.360 | 61.338 | 1.00 | 102.59 |
| ATOM | 3501 | CA | CYS | 461 | 33.033 | 31.572 | 62.156 | 1.00 | 103.00 |
| ATOM | 3502 | CB | CYS | 461 | 33.145 | 31.169 | 63.624 | 1.00 | 103.05 |
| ATOM | 3503 | SG | CYS | 461 | 33.354 | 32.536 | 64.774 | 1.00 | 103.32 |
| ATOM | 3504 | C | CYS | 461 | 34.265 | 32.367 | 61.753 | 1.00 | 103.21 |
| ATOM | 3505 | O | CYS | 461 | 34.788 | 33.098 | 62.620 | 1.00 | 103.54 |
| ATOM | 3506 | OXT | CYS | 461 | 34.665 | 32.248 | 60.578 | 1.00 | 103.24 |
| TER 3507 | | | CYS | 461 | | | | | |
| ATOM | 3508 | C1 | GLC | 500 | 23.469 | 1.767 | 65.521 | 1.00 | 30.82 |
| ATOM | 3509 | C2 | GLC | 500 | 23.418 | 3.122 | 64.706 | 1.00 | 29.40 |
| ATOM | 3510 | C3 | GLC | 500 | 24.837 | 3.619 | 64.445 | 1.00 | 29.78 |
| ATOM | 3511 | C4 | GLC | 500 | 25.496 | 3.860 | 65.778 | 1.00 | 28.77 |
| ATOM | 3512 | C5 | GLC | 500 | 25.529 | 2.514 | 66.593 | 1.00 | 27.72 |
| ATOM | 3513 | C6 | GLC | 500 | 26.162 | 2.717 | 67.936 | 1.00 | 26.98 |
| ATOM | 3514 | O1 | GLC | 500 | 24.127 | 0.765 | 64.857 | 1.00 | 36.62 |
| ATOM | 3515 | O2 | GLC | 500 | 22.756 | 2.872 | 63.483 | 1.00 | 32.75 |
| ATOM | 3516 | O3 | GLC | 500 | 24.786 | 4.837 | 63.698 | 1.00 | 29.31 |
| ATOM | 3517 | O4 | GLC | 500 | 26.853 | 4.253 | 65.639 | 1.00 | 29.10 |
| ATOM | 3518 | O5 | GLC | 500 | 24.152 | 2.040 | 66.770 | 1.00 | 29.59 |
| ATOM | 3519 | O6 | GLC | 500 | 25.517 | 3.687 | 68.814 | 1.00 | 30.98 |
| TER 3520 | | GLC | | 500 | | | | | |
| ATOM | 3521 | S1 | CP1 | 501 | 36.312 | 19.051 | 60.824 | 1.00 | 50.83 |
| ATOM | 3522 | C2 | CP1 | 501 | 35.720 | 19.405 | 59.240 | 1.00 | 49.96 |
| ATOM | 3523 | C3 | CP1 | 501 | 36.398 | 18.662 | 58.318 | 1.00 | 49.96 |
| ATOM | 3524 | N4 | CP1 | 501 | 37.363 | 17.829 | 58.827 | 1.00 | 49.99 |
| ATOM | 3525 | C5 | CP1 | 501 | 37.429 | 17.932 | 60.162 | 1.00 | 49.39 |
| ATOM | 3526 | N6 | CP1 | 501 | 38.317 | 17.183 | 60.878 | 1.00 | 48.07 |

| | | | | | | | | | | |
|---|---|---|---|---|---|---|---|---|---|---|
| ATOM | 3527 | C7 | CP1 | 501 | 38.575 | 17.220 | 62.294 | 1.00 | 46.71 |
| ATOM | 3528 | 08 | CP1 | 501 | 37.968 | 18.001 | 63.039 | 1.00 | 47.48 |
| ATOM | 3529 | C9 | CP1 | 501 | 40.386 | 16.405 | 64.107 | 1.00 | 46.71 |
| ATOM | 3530 | C10 | CP1 | 501 | 39.620 | 16.253 | 62.884 | 1.00 | 47.34 |
| ATOM | 3531 | C11 | CP1 | 501 | 39.831 | 15.053 | 62.110 | 1.00 | 46.39 |
| ATOM | 3532 | C12 | CP1 | 501 | 40.749 | 14.066 | 62.520 | 1.00 | 46.34 |
| ATOM | 3533 | C13 | CP1 | 501 | 41.496 | 14.237 | 63.722 | 1.00 | 47.57 |
| ATOM | 3534 | F | CP1 | 501 | 42.392 | 13.310 | 64.155 | 1.00 | 48.24 |
| ATOM | 3535 | C15 | CP1 | 501 | 41.306 | 15.404 | 64.502 | 1.00 | 46.98 |
| ATOM | 3536 | S16 | CP1 | 501 | 40.907 | 12.638 | 61.485 | 1.00 | 44.61 |
| ATOM | 3537 | N17 | CP1 | 501 | 42.782 | 10.864 | 62.327 | 1.00 | 40.11 |
| ATOM | 3538 | C18 | CP1 | 501 | 42.525 | 11.942 | 61.488 | 1.00 | 41.49 |
| ATOM | 3539 | N19 | CP1 | 501 | 43.528 | 12.436 | 60.686 | 1.00 | 42.95 |
| ATOM | 3540 | C20 | CP1 | 501 | 44.549 | 11.571 | 61.054 | 1.00 | 43.00 |
| ATOM | 3541 | C21 | CP1 | 501 | 44.116 | 10.651 | 62.014 | 1.00 | 39.24 |
| ATOM | 3542 | C22 | CP1 | 501 | 41.894 | 10.152 | 63.276 | 1.00 | 32.83 |
| ATOM | 3543 | N23 | CP1 | 501 | 40.279 | 17.465 | 64.913 | 1.00 | 46.10 |
| TER 3544 | | CP1 | 501 | | JJJJ | | | | |
| ATOM | 3545 | NA+1 | NA1 | 600 | 36.903 | 10.609 | 46.484 | 1.00 | 48.71 |
| ATOM | 3546 | 0 | HOH | 601 | 20.332 | -23.624 | 70.208 | 1.00 | 45.57 |
| ATOM | 3547 | 0 | HOH | 602 | 18.766 | -22.456 | 65.630 | 1.00 | 41.87 |
| ATOM | 3548 | 0 | HOH | 603 | 13.471 | -20.599 | 70.297 | 1.00 | 45.83 |
| ATOM | 3549 | 0 | HOH | 604 | 11.104 | -30.408 | 72.307 | 1.00 | 48.61 |
| ATOM | 3550 | 0 | HOH | 605 | 6.606 | -26.352 | 79.319 | 1.00 | 59.47 |
| ATOM | 3551 | 0 | HOH | 606 | 15.315 | -28.400 | 85.522 | 1.00 | 48.85 |
| ATOM | 3552 | 0 | HOH | 607 | 18.765 | -29.705 | 82.807 | 1.00 | 55.60 |
| ATOM | 3553 | 0 | HOH | 608 | 27.649 | -22.465 | 84.914 | 1.00 | 39.29 |
| ATOM | 3554 | 0 | HOH | 609 | 28.890 | -18.936 | 88.942 | 1.00 | 38.24 |
| ATOM | 3555 | 0 | HOH | 610 | 31.397 | -19.437 | 88.300 | 1.00 | 44.33 |
| ATOM | 3556 | 0 | HOH | 611 | 33.495 | -12.487 | 88.943 | 1.00 | 40.63 |
| ATOM | 3557 | 0 | HOH | 612 | 28.110 | -14.193 | 93.119 | 1.00 | 37.41 |
| ATOM | 3558 | 0 | HOH | 613 | 22.501 | -9.921 | 93.883 | 1.00 | 55.62 |
| ATOM | 3559 | 0 | HOH | 614 | 18.084 | -9.259 | 91.966 | 1.00 | 48.69 |
| ATOM | 3560 | 0 | HOH | 615 | 19.985 | -7.585 | 89.518 | 1.00 | 54.30 |
| ATOM | 3561 | 0 | HOH | 616 | 18.162 | -4.982 | 77.583 | 1.00 | 42.44 |

| | | | | | | | | | |
|---|---|---|---|---|---|---|---|---|---|
| ATOM | 3562 | 0 | HOH | 617 | 15.728 | -5.792 | 77.752 | 1.00 | 49.61 |
| ATOM | 3563 | 0 | HOH | 618 | 17.869 | -7.338 | 75.263 | 1.00 | 52.43 |
| ATOM | 3564 | 0 | HOH | 619 | 14.631 | -9.827 | 77.339 | 1.00 | 27.38 |
| ATOM | 3565 | 0 | HOH | 620 | 14.305 | -5.926 | 69.446 | 1.00 | 38.14 |
| ATOM | 3566 | 0 | HOH | 621 | 13.616 | -3.087 | 68.452 | 1.00 | 51.29 |
| ATOM | 3567 | 0 | HOH | 622 | 15.537 | -2.602 | 66.865 | 1.00 | 35.42 |
| ATOM | 3568 | 0 | HOH | 623 | 18.821 | -1.831 | 65.405 | 1.00 | 31.67 |
| ATOM | 3569 | 0 | HOH | 624 | 17.261 | 0.174 | 60.996 | 1.00 | 34.87 |
| ATOM | 3570 | 0 | HOH | 625 | 18.895 | -0.653 | 58.995 | 1.00 | 41.82 |
| ATOM | 3571 | 0 | HOH | 626 | 20.053 | -2.478 | 55.373 | 1.00 | 35.91 |
| ATOM | 3572 | 0 | HOH | 627 | 22.217 | -1.019 | 55.062 | 1.00 | 36.64 |
| ATOM | 3573 | 0 | HOH | 628 | 25.137 | -0.153 | 56.470 | 1.00 | 24.69 |
| ATOM | 3574 | 0 | HOH | 629 | 22.562 | 1.498 | 59.774 | 1.00 | 31.68 |
| ATOM | 3575 | 0 | HOH | 630 | 24.912 | 0.122 | 62.135 | 1.00 | 25.12 |
| ATOM | 3576 | 0 | HOH | 631 | 25.071 | 2.179 | 71.129 | 1.00 | 26.49 |
| ATOM | 3577 | 0 | HOH | 632 | 27.157 | 5.888 | 71.903 | 1.00 | 41.05 |
| ATOM | 3578 | 0 | HOH | 633 | 29.481 | 7.227 | 73.290 | 1.00 | 47.52 |
| ATOM | 3579 | 0 | HOH | 634 | 31.223 | 8.383 | 71.417 | 1.00 | 44.33 |
| ATOM | 3580 | 0 | HOH | 635 | 32.517 | 7.788 | 77.983 | 1.00 | 44.30 |
| ATOM | 3581 | 0 | HOH | 636 | 35.945 | 15.748 | 80.298 | 1.00 | 32.85 |
| ATOM | 3582 | 0 | HOH | 637 | 41.395 | 13.522 | 74.250 | 1.00 | 52.40 |
| ATOM | 3583 | 0 | HOH | 638 | 41.454 | 16.603 | 73.492 | 1.00 | 35.38 |
| ATOM | 3584 | 0 | HOH | 639 | 44.238 | 18.657 | 64.621 | 1.00 | 57.41 |
| ATOM | 3585 | 0 | HOH | 640 | 48.524 | 12.679 | 62.857 | 1.00 | 55.80 |
| ATOM | 3586 | 0 | HOH | 641 | 50.088 | 10.035 | 69.707 | 1.00 | 37.86 |
| ATOM | 3587 | 0 | HOH | 642 | 47.834 | 4.897 | 73.654 | 1.00 | 43.91 |
| ATOM | 3588 | 0 | HOH | 643 | 47.658 | 2.456 | 75.515 | 1.00 | 46.89 |
| ATOM | 3589 | 0 | HOH | 644 | 45.862 | 0.872 | 75.793 | 1.00 | 36.22 |
| ATOM | 3590 | 0 | HOH | 645 | 42.167 | -0.401 | 77.407 | 1.00 | 46.09 |
| ATOM | 3591 | 0 | HOH | 646 | 39.939 | -1.664 | 76.818 | 1.00 | 28.80 |
| ATOM | 3592 | 0 | HOH | 647 | 41.804 | 2.590 | 77.672 | 1.00 | 30.06 |
| ATOM | 3593 | 0 | HOH | 648 | 35.946 | -0.230 | 81.704 | 1.00 | 44.47 |
| ATOM | 3594 | 0 | HOH | 649 | 35.692 | -3.832 | 84.533 | 1.00 | 48.68 |
| ATOM | 3595 | 0 | HOH | 650 | 35.503 | -5.648 | 82.602 | 1.00 | 39.36 |
| ATOM | 3596 | 0 | HOH | 651 | 34.249 | -6.282 | 78.743 | 1.00 | 28.80 |

| | | | | | | | | | |
|---|---|---|---|---|---|---|---|---|---|
| ATOM | 3597 | O | HOH | 652 | 41.570 | -6.014 | 79.114 | 1.00 | 41.31 |
| ATOM | 3598 | O | HOH | 653 | 42.725 | -8.259 | 76.851 | 1.00 | 34.12 |
| ATOM | 3599 | O | HOH | 654 | 42.400 | -10.619 | 75.649 | 1.00 | 32.12 |
| ATOM | 3600 | O | HOH | 655 | 44.745 | -10.112 | 73.414 | 1.00 | 30.95 |
| ATOM | 3601 | O | HOH | 656 | 44.977 | -6.287 | 75.709 | 1.00 | 54.82 |
| ATOM | 3602 | O | HOH | 657 | 49.536 | -3.896 | 71.639 | 1.00 | 46.68 |
| ATOM | 3603 | O | HOH | 658 | 47.500 | -6.424 | 68.659 | 1.00 | 37.00 |
| ATOM | 3604 | O | HOH | 659 | 46.887 | -8.289 | 65.948 | 1.00 | 35.73 |
| ATOM | 3605 | O | HOH | 660 | 45.007 | -14.004 | 70.403 | 1.00 | 31.53 |
| ATOM | 3606 | O | HOH | 661 | 44.785 | -16.666 | 70.958 | 1.00 | 39.67 |
| ATOM | 3607 | O | HOH | 662 | 39.546 | -15.899 | 74.666 | 1.00 | 38.86 |
| ATOM | 3608 | O | HOH | 663 | 38.539 | -14.985 | 72.232 | 1.00 | 34.80 |
| ATOM | 3609 | O | HOH | 664 | 38.252 | -17.032 | 68.208 | 1.00 | 47.76 |
| ATOM | 3610 | O | HOH | 665 | 39.836 | -15.454 | 66.437 | 1.00 | 38.55 |
| ATOM | 3611 | O | HOH | 666 | 36.975 | -19.549 | 67.636 | 1.00 | 43.12 |
| ATOM | 3612 | O | HOH | 667 | 37.200 | -20.262 | 70.388 | 1.00 | 51.64 |
| ATOM | 3613 | O | HOH | 668 | 33.328 | -20.695 | 70.543 | 1.00 | 49.91 |
| ATOM | 3614 | O | HOH | 669 | 32.877 | -18.716 | 69.209 | 1.00 | 30.69 |
| ATOM | 3615 | O | HOH | 670 | 30.463 | -18.228 | 69.770 | 1.00 | 29.35 |
| ATOM | 3616 | O | HOH | 671 | 29.403 | -18.862 | 72.028 | 1.00 | 29.94 |
| ATOM | 3617 | O | HOH | 672 | 31.677 | -19.876 | 75.929 | 1.00 | 57.83 |
| ATOM | 3618 | O | HOH | 673 | 32.105 | -15.120 | 81.811 | 1.00 | 56.36 |
| ATOM | 3619 | O | HOH | 674 | 25.408 | -13.262 | 70.399 | 1.00 | 19.73 |
| ATOM | 3620 | O | HOH | 675 | 20.199 | -11.770 | 66.567 | 1.00 | 31.95 |
| ATOM | 3621 | O | HOH | 676 | 20.589 | -11.169 | 63.684 | 1.00 | 28.18 |
| ATOM | 3622 | O | HOH | 677 | 18.416 | -12.169 | 62.695 | 1.00 | 34.73 |
| ATOM | 3623 | O | HOH | 678 | 18.037 | -12.657 | 56.097 | 1.00 | 62.31 |
| ATOM | 3624 | O | HOH | 679 | 15.700 | -10.616 | 55.942 | 1.00 | 49.61 |
| ATOM | 3625 | O | HOH | 680 | 17.485 | -8.240 | 55.372 | 1.00 | 37.91 |
| ATOM | 3626 | O | HOH | 681 | 22.370 | -12.555 | 56.733 | 1.00 | 27.53 |
| ATOM | 3627 | O | HOH | 682 | 21.048 | -16.039 | 51.265 | 1.00 | 53.09 |
| ATOM | 3628 | O | HOH | 683 | 25.649 | -8.890 | 49.620 | 1.00 | 43.30 |
| ATOM | 3629 | O | HOH | 684 | 25.472 | -5.908 | 50.031 | 1.00 | 43.23 |
| ATOM | 3630 | O | HOH | 685 | 27.841 | -3.633 | 51.119 | 1.00 | 34.64 |
| ATOM | 3631 | O | HOH | 686 | 23.209 | 1.359 | 50.792 | 1.00 | 44.06 |

| | | | | | | | | | |
|---|---|---|---|---|---|---|---|---|---|
| ATOM | 3632 | 0 | HOH | 687 | 26.198 | 3.711 | 50.151 | 1.00 | 38.65 |
| ATOM | 3633 | 0 | HOH | 688 | 27.728 | 6.416 | 50.494 | 1.00 | 39.66 |
| ATOM | 3634 | 0 | HOH | 689 | 30.171 | 5.238 | 50.152 | 1.00 | 36.90 |
| ATOM | 3635 | 0 | HOH | 690 | 32.248 | 6.334 | 48.750 | 1.00 | 33.36 |
| ATOM | 3636 | 0 | HOH | 691 | 36.665 | 2.495 | 46.196 | 1.00 | 32.68 |
| ATOM | 3637 | 0 | HOH | 692 | 37.821 | 0.573 | 47.634 | 1.00 | 47.42 |
| ATOM | 3638 | 0 | HOH | 693 | 42.794 | 0.201 | 52.097 | 1.00 | 44.65 |
| ATOM | 3639 | 0 | HOH | 694 | 41.559 | 1.725 | 53.810 | 1.00 | 38.52 |
| ATOM | 3640 | 0 | HOH | 695 | 43.105 | 3.662 | 55.242 | 1.00 | 34.89 |
| ATOM | 3641 | 0 | HOH | 696 | 45.510 | 2.836 | 56.086 | 1.00 | 40.92 |
| ATOM | 3642 | 0 | HOH | 697 | 50.206 | 2.510 | 60.598 | 1.00 | 45.86 |
| ATOM | 3643 | 0 | HOH | 698 | 52.258 | 1.308 | 61.720 | 1.00 | 45.43 |
| ATOM | 3644 | 0 | HOH | 699 | 48.954 | 1.961 | 67.618 | 1.00 | 35.43 |
| ATOM | 3645 | 0 | HOH | 700 | 49.694 | -0.399 | 68.442 | 1.00 | 39.38 |
| ATOM | 3646 | 0 | HOH | 701 | 40.015 | -5.106 | 51.960 | 1.00 | 36.49 |
| ATOM | 3647 | 0 | HOH | 702 | 34.048 | -12.903 | 50.839 | 1.00 | 37.87 |
| ATOM | 3648 | 0 | HOH | 703 | 33.190 | -14.541 | 52.882 | 1.00 | 51.09 |
| ATOM | 3649 | 0 | HOH | 704 | 34.961 | -16.254 | 52.067 | 1.00 | 35.42 |
| ATOM | 3650 | 0 | HOH | 705 | 30.397 | -15.105 | 52.902 | 1.00 | 39.69 |
| ATOM | 3651 | 0 | HOH | 706 | 31.770 | -20.985 | 57.467 | 1.00 | 48.16 |
| ATOM | 3652 | 0 | HOH | 707 | 37.192 | -19.637 | 55.866 | 1.00 | 46.43 |
| ATOM | 3653 | 0 | HOH | 708 | 38.187 | -23.567 | 61.924 | 1.00 | 40.92 |
| ATOM | 3654 | 0 | HOH | 709 | 38.470 | -23.126 | 65.456 | 1.00 | 45.43 |
| ATOM | 3655 | 0 | HOH | 710 | 30.533 | -23.844 | 62.578 | 1.00 | 37.90 |
| ATOM | 3656 | 0 | HOH | 711 | 26.515 | -21.678 | 62.544 | 1.00 | 39.08 |
| ATOM | 3657 | 0 | HOH | 712 | 27.242 | -20.400 | 65.671 | 1.00 | 33.60 |
| ATOM | 3658 | 0 | HOH | 713 | 25.907 | -18.116 | 65.171 | 1.00 | 24.64 |
| ATOM | 3659 | 0 | HOH | 714 | 28.226 | -26.567 | 74.622 | 1.00 | 44.93 |
| ATOM | 3660 | 0 | HOH | 715 | 31.091 | -28.151 | 73.632 | 1.00 | 39.43 |
| ATOM | 3661 | 0 | HOH | 716 | 28.020 | -32.685 | 74.512 | 1.00 | 48.35 |
| ATOM | 3662 | 0 | HOH | 717 | 28.401 | -36.363 | 77.956 | 1.00 | 47.24 |
| ATOM | 3663 | 0 | HOH | 718 | 26.796 | -22.733 | 95.375 | 1.00 | 34.50 |
| ATOM | 3664 | 0 | HOH | 719 | 23.506 | -18.729 | 96.532 | 1.00 | 46.50 |
| ATOM | 3665 | 0 | HOH | 720 | 7.193 | -13.392 | 87.134 | 1.00 | 48.33 |
| ATOM | 3666 | 0 | HOH | 721 | 23.769 | -2.393 | 77.130 | 1.00 | 39.79 |

| | | | | | | | | | |
|---|---|---|---|---|---|---|---|---|---|
| ATOM | 3667 | 0 | HOH | 722 | 21.538 | 6.141 | 76.432 | 1.00 | 52.58 |
| ATOM | 3668 | 0 | HOH | 723 | 26.038 | 13.552 | 80.579 | 1.00 | 47.60 |
| ATOM | 3669 | 0 | HOH | 724 | 25.460 | 9.823 | 62.329 | 1.00 | 33.10 |
| ATOM | 3670 | 0 | HOH | 725 | 27.321 | 10.443 | 60.403 | 1.00 | 39.23 |
| ATOM | 3671 | 0 | HOH | 726 | 26.658 | 8.602 | 58.871 | 1.00 | 32.16 |
| ATOM | 3672 | 0 | HOH | 727 | 29.670 | 11.059 | 61.417 | 1.00 | 24.95 |
| ATOM | 3673 | 0 | HOH | 728 | 30.585 | 13.937 | 60.932 | 1.00 | 41.90 |
| ATOM | 3674 | 0 | HOH | 729 | 34.591 | 18.790 | 55.094 | 1.00 | 40.47 |
| ATOM | 3675 | 0 | HOH | 730 | 34.117 | 19.353 | 52.182 | 1.00 | 54.62 |
| ATOM | 3676 | 0 | HOH | 731 | 31.428 | 16.535 | 48.224 | 1.00 | 37.06 |
| ATOM | 3677 | 0 | HOH | 732 | 31.432 | 15.488 | 46.047 | 1.00 | 33.85 |
| ATOM | 3678 | 0 | HOH | 733 | 27.660 | 11.291 | 51.289 | 1.00 | 40.74 |
| ATOM | 3679 | 0 | HOH | 734 | 27.629 | 10.029 | 53.857 | 1.00 | 30.56 |
| ATOM | 3680 | 0 | HOH | 735 | 22.996 | 7.311 | 45.724 | 1.00 | 57.65 |
| ATOM | 3681 | 0 | HOH | 736 | 25.532 | 2.038 | 43.263 | 1.00 | 34.43 |
| ATOM | 3682 | 0 | HOH | 737 | 33.508 | 3.221 | 40.211 | 1.00 | 45.05 |
| ATOM | 3683 | 0 | HOH | 738 | 35.525 | 1.426 | 41.242 | 1.00 | 44.71 |
| ATOM | 3684 | 0 | HOH | 739 | 37.227 | 9.576 | 44.352 | 1.00 | 31.96 |
| ATOM | 3685 | 0 | HOH | 740 | 39.858 | 15.804 | 52.237 | 1.00 | 43.41 |
| ATOM | 3686 | 0 | HOH | 741 | 42.053 | 15.415 | 53.940 | 1.00 | 47.39 |
| ATOM | 3687 | 0 | HOH | 742 | 32.200 | 24.148 | 58.683 | 1.00 | 45.42 |
| ATOM | 3688 | 0 | HOH | 743 | 28.016 | 21.804 | 51.201 | 1.00 | 44.12 |
| ATOM | 3689 | 0 | HOH | 744 | 22.797 | 26.498 | 63.763 | 1.00 | 53.69 |
| ATOM | 3690 | 0 | HOH | 745 | 10.552 | 26.073 | 62.119 | 1.00 | 43.13 |
| ATOM | 3691 | 0 | HOH | 746 | 11.190 | 7.673 | 68.338 | 1.00 | 57.06 |
| ATOM | 3692 | 0 | HOH | 747 | 20.818 | -3.881 | 51.225 | 1.00 | 56.55 |
| ATOM | 3693 | 0 | HOH | 748 | 29.885 | -6.633 | 43.981 | 1.00 | 46.17 |
| ATOM | 3694 | 0 | HOH | 749 | 40.811 | 30.945 | 68.309 | 1.00 | 45.88 |
| TER | 3695 | | HOH | | | | | | |

Table 1 is made according to a description method of Protein Data Bank conventionally used by those skilled in the art. In Table 1, GLC represents a glucose molecule, CP1 represents a compound of the formula IIIa, and HOH represents a water molecule.

The present invention has succeeded in preparing a crystal of a GK protein as depicted in SEQ ID No. 8 (see, examples below). Thus obtained crystal of a GK protein had a lattice constant satisfying the following formulae (5) to (8):

$$a = b = 103.2 \pm 5\text{Å} \quad (5)$$

$$c = 281.0 \pm 7\text{Å} \quad (6)$$

$$\alpha = \beta = 90° \quad (7)$$

$$\gamma = 120° \quad (8)$$

This crystal was analyzed to have a space group of $P6_522$. Here, the above-mentioned a = b is preferably 103.2 ± 3 Å, more preferably 103.2 ± 2 Å, further preferably 103.2 ± 1 Å. The above-mentioned c is preferably 281.0 ± 6 Å, more preferably 281.0 ± 4 Å, further preferably 281.0 ± 2 Å.

The three dimensional structure coordinate of thus obtained GK protein crystal is shown in Table 2.

[Table 2]

| | | | | | | | |
|---|---|---|---|---|---|---|---|
| ATOM 1 | CB | MET15 | 54.150 | 5.972 | 67.103 | 1.00 | 55.10 |
| ATOM 2 | CG | MET15 | 55.594 | 5.943 | 67.591 | 1.00 | 55.46 |
| ATOM 3 | SD | MET15 | 56.013 | 4.505 | 68.603 | 1.00 | 52.92 |
| ATOM 4 | CE | MET15 | 56.517 | 5.326 | 70.108 | 1.00 | 51.73 |
| ATOM 5 | C | MET15 | 52.357 | 4.955 | 65.669 | 1.00 | 56.87 |
| ATOM 6 | O | MET15 | 52.057 | 4.609 | 64.524 | 1.00 | 57.60 |
| ATOM 7 | N | MET15 | 54.770 | 4.766 | 65.028 | 1.00 | 55.00 |
| ATOM 8 | CA | MET15 | 53.800 | 4.813 | 66.167 | 1.00 | 56.04 |
| ATOM 9 | N | VAL16 | 51.468 | 5.456 | 66.525 | 1.00 | 55.58 |
| ATOM 10 | CA | VAL16 | 50.065 | 5.625 | 66.154 | 1.00 | 52.87 |
| ATOM 11 | CB | VAL16 | 49.141 | 4.862 | 67.129 | 1.00 | 49.32 |
| ATOM 12 | CG1 | VAL16 | 47.696 | 5.016 | 66.716 | 1.00 | 48.26 |
| ATOM 13 | CG2 | VAL16 | 49.508 | 3.394 | 67.126 | 1.00 | 47.28 |
| ATOM 14 | C | VAL16 | 49.666 | 7.097 | 66.085 | 1.00 | 53.26 |
| ATOM 15 | O | VAL16 | 49.218 | 7.563 | 65.040 | 1.00 | 52.32 |
| ATOM 16 | N | GLU17 | 49.845 | 7.828 | 67.182 | 1.00 | 56.12 |
| ATOM 17 | CA | GLU17 | 49.511 | 9.253 | 67.210 | 1.00 | 59.41 |
| ATOM 18 | CB | GLU17 | 50.102 | 9.921 | 68.456 | 1.00 | 63.35 |
| ATOM 19 | CG | GLU17 | 49.063 | 10.373 | 69.484 | 1.00 | 68.69 |
| ATOM 20 | CD | GLU17 | 48.174 | 11.525 | 69.004 | 1.00 | 72.00 |
| ATOM 21 | OE1 | GLU17 | 47.314 | 11.964 | 69.805 | 1.00 | 74.22 |
| ATOM 22 | OE2 | GLU17 | 48.328 | 11.992 | 67.847 | 1.00 | 72.36 |
| ATOM 23 | C | GLU17 | 50.035 | 9.963 | 65.967 | 1.00 | 59.05 |
| ATOM 24 | O | GLU17 | 49.521 | 11.011 | 65.566 | 1.00 | 57.70 |
| ATOM 25 | N | GLN18 | 51.070 | 9.389 | 65.367 | 1.00 | 60.75 |
| ATOM 26 | CA | GLN18 | 51.661 | 9.960 | 64.170 | 1.00 | 61.70 |
| ATOM 27 | CB | GLN18 | 53.038 | 9.329 | 63.895 | 1.00 | 66.55 |
| ATOM 28 | CG | GLN18 | 54.001 | 9.219 | 65.110 | 1.00 | 72.22 |
| ATOM 29 | CD | GLN18 | 54.509 | 10.566 | 65.654 | 1.00 | 75.87 |
| ATOM 30 | OE1 | GLN18 | 55.317 | 10.605 | 66.595 | 1.00 | 75.55 |
| ATOM 31 | NE2 | GLN18 | 54.037 | 11.669 | 65.067 | 1.00 | 77.63 |
| ATOM 32 | C | GLN18 | 50.709 | 9.682 | 63.004 | 1.00 | 59.33 |
| ATOM 33 | O | GLN18 | 50.322 | 10.601 | 62.287 | 1.00 | 59.09 |
| ATOM 34 | N | ILE19 | 50.321 | 8.418 | 62.832 | 1.00 | 55.64 |
| ATOM 35 | CA | ILE19 | 49.416 | 8.029 | 61.747 | 1.00 | 53.41 |

[Table 2] (continued)

| | | | | | | | |
|---|---|---|---|---|---|---|---|
| ATOM 36 | CB | ILE19 | 49.113 | 6.529 | 61.778 | 1.00 | 52.34 |
| ATOM 37 | CG2 | ILE19 | 47.964 | 6.211 | 60.832 | 1.00 | 50.69 |
| ATOM 38 | CG1 | ILE19 | 50.374 | 5.754 | 61.389 | 1.00 | 52.73 |
| ATOM 39 | CD1 | ILE19 | 50.186 | 4.256 | 61.274 | 1.00 | 53.73 |
| ATOM 40 | C | ILE19 | 48.088 | 8.774 | 61.741 | 1.00 | 53.03 |
| ATOM 41 | O | ILE19 | 47.791 | 9.528 | 60.812 | 1.00 | 52.86 |
| ATOM 42 | N | LEU20 | 47.279 | 8.548 | 62.766 | 1.00 | 52.38 |
| ATOM 43 | CA | LEU20 | 45.997 | 9.228 | 62.861 | 1.00 | 51.95 |
| ATOM 44 | CB | LEU20 | 45.336 | 8.937 | 64.195 | 1.00 | 50.70 |
| ATOM 45 | CG | LEU20 | 44.563 | 7.632 | 64.212 | 1.00 | 51.65 |
| ATOM 46 | CD1 | LEU20 | 45.450 | 6.454 | 63.803 | 1.00 | 51.77 |
| ATOM 47 | CD2 | LEU20 | 44.010 | 7.463 | 65.599 | 1.00 | 51.02 |
| ATOM 48 | C | LEU20 | 46.158 | 10.723 | 62.727 | 1.00 | 52.33 |
| ATOM 49 | O | LEU20 | 45.204 | 11.427 | 62.401 | 1.00 | 54.11 |
| ATOM 50 | N | ALA21 | 47.366 | 11.207 | 62.990 | 1.00 | 51.49 |
| ATOM 51 | CA | ALA21 | 47.643 | 12.628 | 62.907 | 1.00 | 49.87 |
| ATOM 52 | CB | ALA21 | 49.066 | 12.899 | 63.342 | 1.00 | 50.58 |
| ATOM 53 | C | ALA21 | 47.414 | 13.133 | 61.491 | 1.00 | 48.63 |
| ATOM 54 | O | ALA21 | 47.090 | 14.301 | 61.286 | 1.00 | 47.74 |
| ATOM 55 | N | GLU22 | 47.571 | 12.243 | 60.517 | 1.00 | 47.60 |
| ATOM 56 | CA | GLU22 | 47.383 | 12.605 | 59.121 | 1.00 | 48.69 |
| ATOM 57 | CB | GLU22 | 47.818 | 11.457 | 58.215 | 1.00 | 51.49 |
| ATOM 58 | CG | GLU22 | 49.282 | 11.520 | 57.838 | 1.00 | 59.47 |
| ATOM 59 | CD | GLU22 | 49.738 | 10.335 | 57.003 | 1.00 | 64.78 |
| ATOM 60 | OE1 | GLU22 | 50.896 | 10.369 | 56.519 | 1.00 | 66.47 |
| ATOM 61 | OE2 | GLU22 | 48.948 | 9.373 | 56.839 | 1.00 | 68.05 |
| ATOM 62 | C | GLU22 | 45.954 | 12.999 | 58.794 | 1.00 | 48.26 |
| ATOM 63 | O | GLU22 | 45.683 | 13.538 | 57.721 | 1.00 | 48.86 |
| ATOM 64 | N | PHE23 | 45.036 | 12. 733 | 59.715 | 1.00 | 47.14 |
| ATOM 65 | CA | PHE23 | 43.641 | 13.076 | 59.490 | 1.00 | 45.51 |
| ATOM 66 | CB | PHE23 | 42.722 | 12.045 | 60.147 | 1.00 | 41.36 |
| ATOM 67 | CG | PHE23 | 42.544 | 10.783 | 59.347 | 1.00 | 37.96 |
| ATOM 68 | CD1 | PHE23 | 43.208 | 9.613 | 59.697 | 1.00 | 35.23 |
| ATOM 69 | CD2 | PHE23 | 41.687 | 10.758 | 58.255 | 1.00 | 37.67 |
| ATOM 70 | CE1 | PHE23 | 43.016 | 8.435 | 58.968 | 1.00 | 32.67 |
| ATOM 71 | CE2 | PHE23 | 41.492 | 9.583 | 57.523 | 1.00 | 37.15 |
| ATOM 72 | CZ | PHE23 | 42.158 | 8.423 | 57.883 | 1.00 | 33.48 |
| ATOM 73 | C | PHE23 | 43.310 | 14.468 | 60.013 | 1.00 | 47.24 |
| ATOM 74 | O | PHE23 | 42.227 | 14.993 | 59.767 | 1.00 | 46.34 |
| ATOM 75 | N | GLN24 | 44.245 | 15.068 | 60.735 | 1.00 | 50.44 |
| ATOM 76 | CA | GLN24 | 44.028 | 16.400 | 61.279 | 1.00 | 55.06 |
| ATOM 77 | CB | GLN24 | 45.306 | 16.882 | 61.979 | 1.00 | 59.10 |
| ATOM 78 | CG | GLN24 | 45.715 | 16.023 | 63.168 | 1.00 | 62.03 |
| ATOM 79 | CD | GLN24 | 44.686 | 16.075 | 64.277 | 1.00 | 65.56 |
| ATOM 80 | OE1 | GLN24 | 44.653 | 15.207 | 65.156 | 1.00 | 66.95 |
| ATOM 81 | NE2 | GLN24 | 43.834 | 17.103 | 64.245 | 1.00 | 65.89 |
| ATOM 82 | C | GLN24 | 43.644 | 17.359 | 60.149 | 1.00 | 56.09 |
| ATOM 83 | O | GLN24 | 43.892 | 17.073 | 58.979 | 1.00 | 57.63 |
| ATOM 84 | N | LEU25 | 43.016 | 18.476 | 60.504 | 1.00 | 55.99 |
| ATOM 85 | CA | LEU25 | 42.616 | 19.501 | 59.540 | 1.00 | 55.27 |
| ATOM 86 | CB | LEU25 | 41.303 | 19.128 | 58.841 | 1.00 | 54.71 |

[Table 2] (continued)

| | | | | | | | |
|---|---|---|---|---|---|---|---|
| ATOM 87 | CG | LEU25 | 41.325 | 17.896 | 57.922 | 1.00 | 53.30 |
| ATOM 88 | CD1 | LEU25 | 39.928 | 17.618 | 57.419 | 1.00 | 53.18 |
| ATOM 89 | CD2 | LEU25 | 42.264 | 18.113 | 56.755 | 1.00 | 51.55 |
| ATOM 90 | C | LEU25 | 42.444 | 20.786 | 60.336 | 1.00 | 56.31 |
| ATOM 91 | O | LEU25 | 41.377 | 21.061 | 60.889 | 1.00 | 55.85 |
| ATOM 92 | N | GLN26 | 43.519 | 21.563 | 60.399 | 1.00 | 58.22 |
| ATOM 93 | CA | GLN26 | 43.527 | 22.807 | 61.153 | 1.00 | 58.31 |
| ATOM 94 | CB | GLN26 | 44.980 | 23.280 | 61.361 | 1.00 | 63.03 |
| ATOM 95 | CG | GLN26 | 45.118 | 24.480 | 62.313 | 1.00 | 69.87 |
| ATOM 96 | CD | GLN26 | 46.490 | 25.161 | 62.245 | 1.00 | 73.70 |
| ATOM 97 | OE1 | GLN26 | 47.009 | 25.446 | 61.158 | 1.00 | 74.68 |
| ATOM 98 | NE2 | GLN26 | 47.067 | 25.446 | 63.411 | 1.00 | 74.99 |
| ATOM 99 | C | GLN26 | 42.702 | 23.903 | 60.485 | 1.00 | 55.29 |
| ATOM 100 | O | GLN26 | 42.358 | 23.811 | 59.308 | 1.00 | 51.30 |
| ATOM 101 | N | GLU27 | 42.389 | 24.931 | 61.267 | 1.00 | 55.08 |
| ATOM 102 | CA | GLU27 | 41.617 | 26.083 | 60.824 | 1.00 | 55.66 |
| ATOM 103 | CB | GLU27 | 41.940 | 27.280 | 61.709 | 1.00 | 57.13 |
| ATOM 104 | CG | GLU27 | 41.029 | 28.469 | 61.523 | 1.00 | 59.64 |
| ATOM 105 | CD | GLU27 | 39.694 | 28.272 | 62.208 | 1.00 | 62.00 |
| ATOM 106 | OE1 | GLU27 | 39.685 | 27.840 | 63.382 | 1.00 | 62.44 |
| ATOM 107 | OE2 | GLU27 | 38.653 | 28.559 | 61.581 | 1.00 | 64.27 |
| ATOM 108 | C | GLU27 | 41.905 | 26.454 | 59.380 | 1.00 | 55.70 |
| ATOM 109 | O | GLU27 | 41.025 | 26.416 | 58.531 | 1.00 | 56.30 |
| ATOM 110 | N | GLU28 | 43.147 | 26.828 | 59.113 | 1.00 | 56.74 |
| ATOM 111 | CA | GLU28 | 43.571 | 27.208 | 57.770 | 1.00 | 58.34 |
| ATOM 112 | CB | GLU28 | 45.102 | 27.226 | 57.714 | 1.00 | 63.94 |
| ATOM 113 | CG | GLU28 | 45.704 | 28.026 | 56.573 | 1.00 | 70.36 |
| ATOM 114 | CD | GLU28 | 45.615 | 29.524 | 56.806 | 1.00 | 74.74 |
| ATOM 115 | OE1 | GLU28 | 46.245 | 30.289 | 56.040 | 1.00 | 77.18 |
| ATOM 116 | OE2 | GLU28 | 44.912 | 29.938 | 57.755 | 1.00 | 77.44 |
| ATOM 117 | C | GLU28 | 43.032 | 26.231 | 56.721 | 1.00 | 56.56 |
| ATOM 118 | O | GLU28 | 42.375 | 26.641 | 55.764 | 1.00 | 54.38 |
| ATOM 119 | N | ASP29 | 43.316 | 24.942 | 56.921 | 1.00 | 55.20 |
| ATOM 120 | CA | ASP29 | 42.893 | 23.869 | 56.015 | 1.00 | 53.13 |
| ATOM 121 | CB | ASP29 | 43.106 | 22.499 | 56.667 | 1.00 | 56.36 |
| ATOM 122 | CG | ASP29 | 44.570 | 22.116 | 56.758 | 1.00 | 59.69 |
| ATOM 123 | OD1 | ASP29 | 45.263 | 22.198 | 55.717 | 1.00 | 61.07 |
| ATOM 124 | OD2 | ASP29 | 45.021 | 21.727 | 57.863 | 1.00 | 60.92 |
| ATOM 125 | C | ASP29 | 41.439 | 23.995 | 55.607 | 1.00 | 49.74 |
| ATOM 126 | O | ASP29 | 41.100 | 23.924 | 54.424 | 1.00 | 47.81 |
| ATOM 127 | N | LEU30 | 40.579 | 24.156 | 56.603 | 1.00 | 46.04 |
| ATOM 128 | CA | LEU30 | 39.167 | 24.309 | 56.344 | 1.00 | 43.06 |
| ATOM 129 | CB | LEU30 | 38.393 | 24.491 | 57.649 | 1.00 | 39.08 |
| ATOM 130 | CG | LEU30 | 38.026 | 23.218 | 58.404 | 1.00 | 36.61 |
| ATOM 131 | CD1 | LEU30 | 39.280 | 22.441 | 58.756 | 1.00 | 37.28 |
| ATOM 132 | CD2 | LEU30 | 37.233 | 23.576 | 59.642 | 1.00 | 35.29 |
| ATOM 133 | C | LEU30 | 38.948 | 25.516 | 55.452 | 1.00 | 44.18 |
| ATOM 134 | O | LEU30 | 38.410 | 25.388 | 54.354 | 1.00 | 45.60 |
| ATOM 135 | N | LYS31 | 39.381 | 26.685 | 55.920 | 1.00 | 44.63 |
| ATOM 136 | CA | LYS31 | 39.206 | 27.927 | 55.170 | 1.00 | 43.67 |
| ATOM 137 | CB | LYS31 | 40.136 | 29.020 | 55.695 | 1.00 | 45.23 |

[Table 2] (continued)

| | | | | | | | |
|---|---|---|---|---|---|---|---|
| ATOM 138 | CG | LYS31 | 39.968 | 29.361 | 57.165 | 1.00 | 46.98 |
| ATOM 139 | CD | LYS31 | 38.743 | 30.221 | 57.440 | 1.00 | 45.54 |
| ATOM 140 | CE | LYS31 | 38.695 | 30.675 | 58.915 | 1.00 | 45.82 |
| ATOM 141 | NZ | LYS31 | 39.836 | 31.545 | 59.387 | 1.00 | 42.73 |
| ATOM 142 | C | LYS31 | 39.483 | 27.725 | 53.697 | 1.00 | 42.23 |
| ATOM 143 | O | LYS31 | 38.759 | 28.241 | 52.855 | 1.00 | 41.29 |
| ATOM 144 | N | LYS32 | 40.535 | 26.976 | 53.385 | 1.00 | 41.79 |
| ATOM 145 | CA | LYS32 | 40.877 | 26.737 | 51.994 | 1.00 | 43.47 |
| ATOM 146 | CB | LYS32 | 42.171 | 25.928 | 51.888 | 1.00 | 45.16 |
| ATOM 147 | CG | LYS32 | 42.811 | 25.974 | 50.499 | 1.00 | 50.49 |
| ATOM 148 | CD | LYS32 | 44.302 | 25.565 | 50.510 | 1.00 | 54.48 |
| ATOM 149 | CE | LYS32 | 44.505 | 24.086 | 50.900 | 1.00 | 57.45 |
| ATOM 150 | NZ | LYS32 | 45.934 | 23.610 | 51.002 | 1.00 | 56.65 |
| ATOM 151 | C | LYS32 | 39.740 | 25.995 | 51.308 | 1.00 | 43.99 |
| ATOM 152 | O | LYS32 | 39.260 | 26.407 | 50.246 | 1.00 | 43.34 |
| ATOM 153 | N | VAL33 | 39.306 | 24.901 | 51.925 | 1.00 | 43.47 |
| ATOM 154 | CA | VAL33 | 38.218 | 24.100 | 51.382 | 1.00 | 40.87 |
| ATOM 155 | CB | VAL33 | 37.895 | 22.927 | 52.310 | 1.00 | 40.53 |
| ATOM 156 | CG1 | VAL33 | 36.977 | 21.939 | 51.604 | 1.00 | 40.20 |
| ATOM 157 | CG2 | VAL33 | 39.183 | 22.248 | 52.729 | 1.00 | 40.29 |
| ATOM 158 | C | VAL33 | 36.994 | 24.981 | 51.226 | 1.00 | 39.39 |
| ATOM 159 | O | VAL33 | 36.370 | 25.011 | 50.165 | 1.00 | 37.22 |
| ATOM 160 | N | MET34 | 36.675 | 25.707 | 52.290 | 1.00 | 39.46 |
| ATOM 161 | CA | MET34 | 35.539 | 26.609 | 52.288 | 1.00 | 42.17 |
| ATOM 162 | CB | MET34 | 35.515 | 27.460 | 53.555 | 1.00 | 43.81 |
| ATOM 163 | CG | MET34 | 34.259 | 28.305 | 53.656 | 1.00 | 48.81 |
| ATOM 164 | SD | MET34 | 34.302 | 29.606 | 54.908 | 1.00 | 56.60 |
| ATOM 165 | CE | MET34 | 34.576 | 31.074 | 53.859 | 1.00 | 55.54 |
| ATOM 166 | C | MET34 | 35.612 | 27.535 | 51.086 | 1.00 | 43.35 |
| ATOM 167 | O | MET34 | 34.626 | 27.735 | 50.383 | 1.00 | 43.86 |
| ATOM 168 | N | ARG35 | 36.785 | 28.104 | 50.847 | 1.00 | 44.90 |
| ATOM 169 | CA | ARG35 | 36.938 | 29.015 | 49.729 | 1.00 | 45.60 |
| ATOM 170 | CB | ARG35 | 38.286 | 29.727 | 49.815 | 1.00 | 49.40 |
| ATOM 171 | CG | ARG35 | 38.459 | 30.563 | 51.075 | 1.00 | 53.81 |
| ATOM 172 | CD | ARG35 | 38.231 | 32.052 | 50.851 | 1.00 | 57.78 |
| ATOM 173 | NE | ARG35 | 38.483 | 32.807 | 52.077 | 1.00 | 63.20 |
| ATOM 174 | CZ | ARG35 | 39.587 | 32.696 | 52.820 | 1.00 | 65.30 |
| ATOM 175 | NH1 | ARG35 | 40.557 | 31.854 | 52.466 | 1.00 | 64.80 |
| ATOM 176 | NH2 | ARG35 | 39.720 | 33.425 | 53.925 | 1.00 | 66.89 |
| ATOM 177 | C | ARG35 | 36.814 | 28.262 | 48.418 | 1.00 | 44.08 |
| ATOM 178 | O | ARG35 | 35.977 | 28.605 | 47.586 | 1.00 | 43.75 |
| ATOM 179 | N | ARG36 | 37.633 | 27.227 | 48.245 | 1.00 | 43.43 |
| ATOM 180 | CA | ARG36 | 37.612 | 26.418 | 47.026 | 1.00 | 43.94 |
| ATOM 181 | CB | ARG36 | 38.547 | 25.212 | 47.174 | 1.00 | 44.76 |
| ATOM 182 | CG | ARG36 | 40.020 | 25.580 | 47.244 | 1.00 | 44.66 |
| ATOM 183 | CD | ARG36 | 40.898 | 24.392 | 47.617 | 1.00 | 44.20 |
| ATOM 184 | NE | ARG36 | 41.728 | 23.919 | 46.512 | 1.00 | 44.66 |
| ATOM 185 | CZ | ARG36 | 42.890 | 23.292 | 46.678 | 1.00 | 45.10 |
| ATOM 186 | NH1 | ARG36 | 43.350 | 23.075 | 47.900 | 1.00 | 44.34 |
| ATOM 187 | NH2 | ARG36 | 43.590 | 22.870 | 45.631 | 1.00 | 45.47 |
| ATOM 188 | C | ARG36 | 36.202 | 25.941 | 46.660 | 1.00 | 43.73 |

[Table 2] (continued)

| | | | | | | | |
|---|---|---|---|---|---|---|---|
| ATOM 189 | O | ARG36 | 35.921 | 25.645 | 45.497 | 1.00 | 43.31 |
| ATOM 190 | N | MET37 | 35.324 | 25.851 | 47.656 | 1.00 | 42.87 |
| ATOM 191 | CA | MET37 | 33.946 | 25.440 | 47.413 | 1.00 | 41.30 |
| ATOM 192 | CB | MET37 | 33.222 | 25.136 | 48.726 | 1.00 | 43.30 |
| ATOM 193 | CG | MET37 | 31.782 | 24.636 | 48.556 | 1.00 | 45.16 |
| ATOM 194 | SD | MET37 | 31.646 | 22.826 | 48.280 | 1.00 | 52.61 |
| ATOM 195 | CE | MET37 | 31.892 | 22.708 | 46.492 | 1.00 | 46.47 |
| ATOM 196 | C | MET37 | 33.249 | 26.603 | 46.723 | 1.00 | 39.52 |
| ATOM 197 | O | MET37 | 32.702 | 26.458 | 45.635 | 1.00 | 39.06 |
| ATOM 198 | N | GLN38 | 33.275 | 27.767 | 47.359 | 1.00 | 37.22 |
| ATOM 199 | CA | GLN38 | 32.637 | 28.927 | 46.776 | 1.00 | 35.67 |
| ATOM 200 | CB | GLN38 | 32.874 | 30.155 | 47.643 | 1.00 | 36.29 |
| ATOM 201 | CG | GLN38 | 32.128 | 30.122 | 48.950 | 1.00 | 37.44 |
| ATOM 202 | CD | GLN38 | 32.689 | 31.108 | 49.950 | 1.00 | 41.99 |
| ATOM 203 | OE1 | GLN38 | 33.841 | 30.992 | 50.376 | 1.00 | 44.33 |
| ATOM 204 | NE2 | GLN38 | 31.880 | 32.091 | 50.331 | 1.00 | 44.58 |
| ATOM 205 | C | GLN38 | 33.184 | 29.155 | 45.382 | 1.00 | 35.21 |
| ATOM 206 | O | GLN38 | 32.454 | 29.557 | 44.486 | 1.00 | 34.82 |
| ATOM 207 | N | LYS39 | 34.467 | 28.884 | 45.188 | 1.00 | 36.41 |
| ATOM 208 | CA | LYS39 | 35.069 | 29.081 | 43.875 | 1.00 | 38.60 |
| ATOM 209 | CB | LYS39 | 36.560 | 28.708 | 43.888 | 1.00 | 42.47 |
| ATOM 210 | CG | LYS39 | 37.395 | 29.263 | 42.714 | 1.00 | 45.02 |
| ATOM 211 | CD | LYS39 | 37.638 | 30.775 | 42.861 | 1.00 | 49.54 |
| ATOM 212 | CE | LYS39 | 38.523 | 31.365 | 41.752 | 1.00 | 51.65 |
| ATOM 213 | NZ | LYS39 | 38.621 | 32.865 | 41.821 | 1.00 | 53.58 |
| ATOM 214 | C | LYS39 | 34.339 | 28.196 | 42.884 | 1.00 | 38.31 |
| ATOM 215 | O | LYS39 | 34.229 | 28.534 | 41.710 | 1.00 | 40.28 |
| ATOM 216 | N | GLU40 | 33.827 | 27.066 | 43.369 | 1.00 | 37.21 |
| ATOM 217 | CA | GLU40 | 33.117 | 26.107 | 42.525 | 1.00 | 34.69 |
| ATOM 218 | CB | GLU40 | 33.329 | 24.705 | 43.072 | 1.00 | 32.80 |
| ATOM 219 | CG | GLU40 | 34.742 | 24.245 | 42.900 | 1.00 | 33.53 |
| ATOM 220 | CD | GLU40 | 35.164 | 24.348 | 41.459 | 1.00 | 36.48 |
| ATOM 221 | OE1 | GLU40 | 34.318 | 24.044 | 40.589 | 1.00 | 39.36 |
| ATOM 222 | OE2 | GLU40 | 36.326 | 24.720 | 41.187 | 1.00 | 37.18 |
| ATOM 223 | C | GLU40 | 31.632 | 26.387 | 42.375 | 1.00 | 34.48 |
| ATOM 224 | O | GLU40 | 31.040 | 26.110 | 41.332 | 1.00 | 32.30 |
| ATOM 225 | N | MET41 | 31.030 | 26.928 | 43.425 | 1.00 | 35.61 |
| ATOM 226 | CA | MET41 | 29.621 | 27.256 | 43.373 | 1.00 | 39.30 |
| ATOM 227 | CB | MET41 | 29.155 | 27.852 | 44.692 | 1.00 | 39.16 |
| ATOM 228 | CG | MET41 | 29.146 | 26.910 | 45.867 | 1.00 | 40.71 |
| ATOM 229 | SD | MET41 | 27.930 | 27. 569 | 47.040 | 1.00 | 46.34 |
| ATOM 230 | CE | MET41 | 28.978 | 28. 338 | 48.243 | 1.00 | 46.54 |
| ATOM 231 | C | MET41 | 29.336 | 28.258 | 42.251 | 1.00 | 42.24 |
| ATOM 232 | O | MET41 | 28.358 | 28.113 | 41.517 | 1.00 | 44.97 |
| ATOM 233 | N | ASP42 | 30.173 | 29.284 | 42.118 | 1.00 | 43.47 |
| ATOM 234 | CA | ASP42 | 29.952 | 30.274 | 41.069 | 1.00 | 42.69 |
| ATOM 235 | CB | ASP42 | 30.848 | 31.497 | 41.249 | 1.00 | 44.70 |
| ATOM 236 | CG | ASP42 | 30.548 | 32.254 | 42.523 | 1.00 | 49.63 |
| ATOM 237 | OD1 | ASP42 | 31.352 | 32.128 | 43.477 | 1.00 | 52.14 |
| ATOM 238 | OD2 | ASP42 | 29.510 | 32.968 | 42.572 | 1.00 | 49.66 |
| ATOM 239 | C | ASP42 | 30.248 | 29.641 | 39.739 | 1.00 | 41.40 |

[Table 2] (continued)

| | | | | | | | |
|---|---|---|---|---|---|---|---|
| ATOM 240 | O | ASP42 | 29.550 | 29.880 | 38.759 | 1.00 | 41.06 |
| ATOM 241 | N | ARG43 | 31.289 | 28.826 | 39.707 | 1.00 | 39.70 |
| ATOM 242 | CA | ARG43 | 31.668 | 28.171 | 38.477 | 1.00 | 39.99 |
| ATOM 243 | CB | ARG43 | 32.835 | 27.227 | 38.739 | 1.00 | 43.98 |
| ATOM 244 | CG | ARG43 | 33.329 | 26.482 | 37.516 | 1.00 | 49.72 |
| ATOM 245 | CD | ARG43 | 34.636 | 25.777 | 37.831 | 1.00 | 55.67 |
| ATOM 246 | NE | ARG43 | 34.962 | 24.746 | 36.854 | 1.00 | 62.98 |
| ATOM 247 | CZ | ARG43 | 36.062 | 24.002 | 36.899 | 1.00 | 67.95 |
| ATOM 248 | NH1 | ARG43 | 36.950 | 24.178 | 37.877 | 1.00 | 69.41 |
| ATOM 249 | NH2 | ARG43 | 36.269 | 23.075 | 35.969 | 1.00 | 70.32 |
| ATOM 250 | C | ARG43 | 30.488 | 27.417 | 37.881 | 1.00 | 38.35 |
| ATOM 251 | O | ARG43 | 30.253 | 27.493 | 36.677 | 1.00 | 38.07 |
| ATOM 252 | N | GLY44 | 29.739 | 26.709 | 38.728 | 1.00 | 36.44 |
| ATOM 253 | CA | GLY44 | 28.592 | 25.938 | 38.262 | 1.00 | 32.80 |
| ATOM 254 | C | GLY44 | 27.344 | 26.772 | 38.062 | 1.00 | 31.71 |
| ATOM 255 | O | GLY44 | 26.483 | 26.448 | 37.251 | 1.00 | 30.43 |
| ATOM 256 | N | LEU45 | 27.258 | 27.854 | 38.820 | 1.00 | 31.23 |
| ATOM 257 | CA | LEU45 | 26.144 | 28.774 | 38.761 | 1.00 | 31.72 |
| ATOM 258 | CB | LEU45 | 26.168 | 29.638 | 40.010 | 1.00 | 30.96 |
| ATOM 259 | CG | LEU45 | 25.063 | 29.363 | 41.013 | 1.00 | 34.38 |
| ATOM 260 | CD1 | LEU45 | 25.346 | 30.066 | 42.334 | 1.00 | 34.74 |
| ATOM 261 | CD2 | LEU45 | 23.750 | 29.849 | 40.413 | 1.00 | 37.12 |
| ATOM 262 | C | LEU45 | 26.204 | 29.666 | 37.517 | 1.00 | 33.39 |
| ATOM 263 | O | LEU45 | 25.184 | 30.211 | 37.086 | 1.00 | 34.01 |
| ATOM 264 | N | ARG46 | 27.402 | 29.813 | 36.955 | 1.00 | 34.39 |
| ATOM 265 | CA | ARG46 | 27.628 | 30.651 | 35.774 | 1.00 | 37.39 |
| ATOM 266 | CB | ARG46 | 29.092 | 31.140 | 35.744 | 1.00 | 42.80 |
| ATOM 267 | CG | ARG46 | 29.463 | 32.067 | 34.562 | 1.00 | 48.17 |
| ATOM 268 | CD | ARG46 | 30.951 | 32.487 | 34.546 | 1.00 | 49.35 |
| ATOM 269 | NE | ARG46 | 31.250 | 33.400 | 33.441 | 1.00 | 54.04 |
| ATOM 270 | CZ | ARG46 | 30.599 | 34.542 | 33.216 | 1.00 | 57.98 |
| ATOM 271 | NH1 | ARG46 | 29.608 | 34.915 | 34.019 | 1.00 | 56.34 |
| ATOM 272 | NH2 | ARG46 | 30.936 | 35.316 | 32.187 | 1.00 | 59.91 |
| ATOM 273 | C | ARG46 | 27.301 | 29.920 | 34.477 | 1.00 | 37.53 |
| ATOM 274 | O | ARG46 | 27.773 | 28.804 | 34.243 | 1.00 | 38.11 |
| ATOM 275 | N | LEU47 | 26.515 | 30.573 | 33.623 | 1.00 | 36.42 |
| ATOM 276 | CA | LEU47 | 26.089 | 29.993 | 32.350 | 1.00 | 35.82 |
| ATOM 277 | CB | LEU47 | 25.151 | 30.957 | 31.617 | 1.00 | 31.45 |
| ATOM 278 | CG | LEU47 | 24.771 | 30.548 | 30.196 | 1.00 | 29.68 |
| ATOM 279 | CD1 | LEU47 | 24.031 | 29.240 | 30.230 | 1.00 | 28.93 |
| ATOM 280 | CD2 | LEU47 | 23.929 | 31.622 | 29.559 | 1.00 | 28.83 |
| ATOM 281 | C | LEU47 | 27.223 | 29.578 | 31.418 | 1.00 | 37.14 |
| ATOM 282 | O | LEU47 | 27.152 | 28.534 | 30.764 | 1.00 | 36.41 |
| ATOM 283 | N | GLU48 | 28.272 | 30.383 | 31.347 | 1.00 | 39.28 |
| ATOM 284 | CA | GLU48 | 29.371 | 30.034 | 30.462 | 1.00 | 42.38 |
| ATOM 285 | CB | GLU48 | 30.448 | 31.126 | 30.473 | 1.00 | 43.91 |
| ATOM 286 | CG | GLU48 | 30.126 | 32.354 | 29.631 | 1.00 | 46.02 |
| ATOM 287 | CD | GLU48 | 29.022 | 33.215 | 30.221 | 1.00 | 48.71 |
| ATOM 288 | OE1 | GLU48 | 28.581 | 34.157 | 29.524 | 1.00 | 48.10 |
| ATOM 289 | OE2 | GLU48 | 28.600 | 32.959 | 31.375 | 1.00 | 49.31 |
| ATOM 290 | C | GLU48 | 30.005 | 28.691 | 30.809 | 1.00 | 43.42 |

[Table 2] (continued)

| | | | | | | | |
|---|---|---|---|---|---|---|---|
| ATOM 291 | O | GLU48 | 30.593 | 28.045 | 29.939 | 1.00 | 43.61 |
| ATOM 292 | N | THR49 | 29.873 | 28.262 | 32.066 | 1.00 | 44.28 |
| ATOM 293 | CA | THR49 | 30.484 | 26.999 | 32.508 | 1.00 | 46.81 |
| ATOM 294 | CB | THR49 | 31.761 | 27.267 | 33.366 | 1.00 | 47.70 |
| ATOM 295 | OG1 | THR49 | 31.477 | 28.265 | 34.356 | 1.00 | 45.18 |
| ATOM 296 | CG2 | THR49 | 32.921 | 27.739 | 32.486 | 1.00 | 48.17 |
| ATOM 297 | C | THR49 | 29.595 | 26.024 | 33.293 | 1.00 | 46.50 |
| ATOM 298 | O | THR49 | 30.043 | 24.932 | 33.683 | 1.00 | 45.72 |
| ATOM 299 | N | HIS50 | 28.340 | 26.405 | 33.508 | 1.00 | 44.18 |
| ATOM 300 | CA | HIS50 | 27.416 | 25.565 | 34.262 | 1.00 | 41.93 |
| ATOM 301 | CB | HIS50 | 25.980 | 26.129 | 34.190 | 1.00 | 38.83 |
| ATOM 302 | CG | HIS50 | 25.217 | 25.754 | 32.953 | 1.00 | 35.50 |
| ATOM 303 | CD2 | HIS50 | 23.950 | 25.304 | 32.795 | 1.00 | 33.70 |
| ATOM 304 | ND1 | HIS50 | 25.730 | 25.894 | 31.682 | 1.00 | 36.24 |
| ATOM 305 | CE1 | HIS50 | 24.812 | 25.550 | 30.796 | 1.00 | 33.56 |
| ATOM 306 | NE2 | HIS50 | 23.722 | 25.189 | 31.446 | 1.00 | 32.06 |
| ATOM 307 | C | HIS50 | 27.447 | 24.117 | 33.804 | 1.00 | 41.73 |
| ATOM 308 | O | HIS50 | 27.144 | 23.212 | 34.572 | 1.00 | 41.14 |
| ATOM 309 | N | GLU51 | 27.848 | 23.883 | 32.566 | 1.00 | 42.00 |
| ATOM 310 | CA | GLU51 | 27.863 | 22.519 | 32.103 | 1.00 | 45.79 |
| ATOM 311 | CB | GLU51 | 27.573 | 22.463 | 30.617 | 1.00 | 46.76 |
| ATOM 312 | CG | GLU51 | 27.523 | 21.048 | 30.100 | 1.00 | 50.98 |
| ATOM 313 | CD | GLU51 | 26.521 | 20.885 | 28.989 | 1.00 | 53.94 |
| ATOM 314 | OE1 | GLU51 | 25.313 | 21.082 | 29.253 | 1.00 | 55.61 |
| ATOM 315 | OE2 | GLU51 | 26.940 | 20.560 | 27.857 | 1.00 | 55.48 |
| ATOM 316 | C | GLU51 | 29.139 | 21.757 | 32.402 | 1.00 | 48.17 |
| ATOM 317 | O | GLU51 | 29.094 | 20.657 | 32.953 | 1.00 | 49.35 |
| ATOM 318 | N | GLU52 | 30.276 | 22.331 | 32.034 | 1.00 | 50.75 |
| ATOM 319 | CA | GLU52 | 31.565 | 21.681 | 32.264 | 1.00 | 52.07 |
| ATOM 320 | CB | GLU52 | 32.633 | 22.321 | 31.352 | 1.00 | 56.66 |
| ATOM 321 | CG | GLU52 | 32.768 | 23.854 | 31.476 | 1.00 | 63.81 |
| ATOM 322 | CD | GLU52 | 33.420 | 24.528 | 30.253 | 1.00 | 67.84 |
| ATOM 323 | OE1 | GLU52 | 33.601 | 25.770 | 30.278 | 1.00 | 68.83 |
| ATOM 324 | OE2 | GLU52 | 33.742 | 23.826 | 29.266 | 1.00 | 70.00 |
| ATOM 325 | C | GLU52 | 31.982 | 21.760 | 33.738 | 1.00 | 49.95 |
| ATOM 326 | O | GLU52 | 33.013 | 21.215 | 34.132 | 1.00 | 47.47 |
| ATOM 327 | N | ALA53 | 31.162 | 22.429 | 34.548 | 1.00 | 48.46 |
| ATOM 328 | CA | ALA53 | 31.449 | 22.594 | 35.972 | 1.00 | 47.88 |
| ATOM 329 | CB | ALA53 | 30.418 | 23.510 | 36.615 | 1.00 | 47.30 |
| ATOM 330 | C | ALA53 | 31.510 | 21.278 | 36.731 | 1.00 | 46.84 |
| ATOM 331 | O | ALA53 | 31.287 | 20.206 | 36.172 | 1.00 | 48.51 |
| ATOM 332 | N | SER54 | 31.816 | 21.353 | 38.016 | 1.00 | 44.67 |
| ATOM 333 | CA | SER54 | 31.895 | 20.133 | 38.792 | 1.00 | 42.38 |
| ATOM 334 | CB | SER54 | 33.201 | 20.090 | 39.581 | 1.00 | 44.26 |
| ATOM 335 | OG | SER54 | 33.290 | 18.883 | 40.316 | 1.00 | 45.49 |
| ATOM 336 | C | SER54 | 30.712 | 20.059 | 39.734 | 1.00 | 39.72 |
| ATOM 337 | O | SER54 | 30.058 | 19.028 | 39.841 | 1.00 | 41.09 |
| ATOM 338 | N | VAL55 | 30.440 | 21.165 | 40.411 | 1.00 | 34.77 |
| ATOM 339 | CA | VAL55 | 29.326 | 21.239 | 41.343 | 1.00 | 30.58 |
| ATOM 340 | CB | VAL55 | 29.682 | 22.186 | 42.498 | 1.00 | 28.73 |
| ATOM 341 | CG1 | VAL55 | 28.480 | 22.433 | 43.383 | 1.00 | 30.75 |

[Table 2] (continued)

| | | | | | | | |
|---|---|---|---|---|---|---|---|
| ATOM 342 | CG2 | VAL55 | 30.814 | 21.596 | 43.297 | 1.00 | 25.80 |
| ATOM 343 | C | VAL55 | 28.094 | 21.760 | 40.597 | 1.00 | 30.28 |
| ATOM 344 | O | VAL55 | 27.704 | 22.920 | 40.745 | 1.00 | 32.16 |
| ATOM 345 | N | LYS56 | 27.482 | 20.887 | 39.803 | 1.00 | 26.82 |
| ATOM 346 | CA | LYS56 | 26.323 | 21.235 | 38.986 | 1.00 | 21.66 |
| ATOM 347 | CB | LYS56 | 25.362 | 20.046 | 38.891 | 1.00 | 26.53 |
| ATOM 348 | CG | LYS56 | 25.936 | 18.737 | 38.337 | 1.00 | 29.32 |
| ATOM 349 | CD | LYS56 | 26.311 | 18.836 | 36.875 | 1.00 | 29.86 |
| ATOM 350 | CE | LYS56 | 27.609 | 19.592 | 36.698 | 1.00 | 29.73 |
| ATOM 351 | NZ | LYS56 | 27.932 | 19.759 | 35.259 | 1.00 | 32.80 |
| ATOM 352 | C | LYS56 | 25.520 | 22.470 | 39.374 | 1.00 | 17.56 |
| ATOM 353 | O | LYS56 | 25.133 | 23.236 | 38.498 | 1.00 | 15.95 |
| ATOM 354 | N | MET57 | 25.257 | 22.660 | 40.665 | 1.00 | 14.30 |
| ATOM 355 | CA | MET 57 | 24.462 | 23.803 | 41.128 | 1.00 | 12.73 |
| ATOM 356 | CB | MET57 | 25.277 | 25.089 | 41.059 | 1.00 | 9.92 |
| ATOM 357 | CG | MET57 | 26.515 | 25.090 | 41.930 | 1.00 | 6.47 |
| ATOM 358 | SD | MET57 | 26.219 | 25.164 | 43.694 | 1.00 | 8.00 |
| ATOM 359 | CE | MET57 | 25.523 | 26.842 | 43.905 | 1.00 | 1.00 |
| ATOM 360 | C | MET57 | 23.207 | 23.953 | 40.270 | 1.00 | 14.05 |
| ATOM 361 | O | MET57 | 23.000 | 24.972 | 39.610 | 1.00 | 12.36 |
| ATOM 362 | N | LEU58 | 22.371 | 22.923 | 40.290 | 1.00 | 17.80 |
| ATOM 363 | CA | LEU58 | 21.154 | 22.914 | 39.498 | 1.00 | 19.02 |
| ATOM 364 | CB | LEU58 | 20.710 | 21.466 | 39.245 | 1.00 | 18.03 |
| ATOM 365 | CG | LEU58 | 21.726 | 20.444 | 38.720 | 1.00 | 16.28 |
| ATOM 366 | CD1 | LEU58 | 21.193 | 19.068 | 39.021 | 1.00 | 20.44 |
| ATOM 367 | CD2 | LEU58 | 21.999 | 20.608 | 37.233 | 1.00 | 15.03 |
| ATOM 368 | C | LEU58 | 20.005 | 23.696 | 40.134 | 1.00 | 20.20 |
| ATOM 369 | O | LEU58 | 19.752 | 23.602 | 41.340 | 1.00 | 19.91 |
| ATOM 370 | N | PRO59 | 19.316 | 24.507 | 39.320 | 1.00 | 20.57 |
| ATOM 371 | CD | PR059 | 19.856 | 24.939 | 38.022 | 1.00 | 20.39 |
| ATOM 372 | CA | PR059 | 18.171 | 25.342 | 39.694 | 1.00 | 22.50 |
| ATOM 373 | CB | PR059 | 17.939 | 26.168 | 38.437 | 1.00 | 22.07 |
| ATOM 374 | CG | PR059 | 19.306 | 26.329 | 37.906 | 1.00 | 21.92 |
| ATOM 375 | C | PR059 | 16.975 | 24.437 | 40.010 | 1.00 | 23.49 |
| ATOM 376 | O | PR059 | 16.698 | 23.504 | 39.264 | 1.00 | 25.36 |
| ATOM 377 | N | THR60 | 16.258 | 24.714 | 41.092 | 1.00 | 22.35 |
| ATOM 378 | CA | THR60 | 15.133 | 23.871 | 41.469 | 1.00 | 20.99 |
| ATOM 379 | CB | THR60 | 15.097 | 23.607 | 42.964 | 1.00 | 22.35 |
| ATOM 380 | OG1 | THR60 | 14.823 | 24.837 | 43.647 | 1.00 | 24.53 |
| ATOM 381 | CG2 | THR60 | 16.408 | 23.049 | 43.441 | 1.00 | 24.88 |
| ATOM 382 | C | THR60 | 13.815 | 24.516 | 41.160 | 1.00 | 20.21 |
| ATOM 383 | O | THR60 | 12.793 | 23.848 | 41.119 | 1.00 | 24.18 |
| ATOM 384 | N | TYR61 | 13.839 | 25.822 | 40.973 | 1.00 | 19.09 |
| ATOM 385 | CA | TYR61 | 12.628 | 26.595 | 40.715 | 1.00 | 20.03 |
| ATOM 386 | CB | TYR61 | 11.955 | 26.172 | 39.427 | 1.00 | 13.50 |
| ATOM 387 | CG | TYR61 | 12.581 | 26.830 | 38.234 | 1.00 | 13.18 |
| ATOM 388 | CD1 | TYR61 | 12.028 | 27.983 | 37.666 | 1.00 | 8.00 |
| ATOM 389 | CE1 | TYR61 | 12.596 | 28.551 | 36.536 | 1.00 | 4.24 |
| ATOM 390 | CD2 | TYR61 | 13.725 | 26.281 | 37.647 | 1.00 | 14.04 |
| ATOM 391 | CE2 | TYR61 | 14.296 | 26.843 | 36.529 | 1.00 | 10.05 |
| ATOM 392 | CZ | TYR61 | 13.730 | 27.963 | 35.976 | 1.00 | 5.80 |

[Table 2] (continued)

| | | | | | | | |
|---|---|---|---|---|---|---|---|
| ATOM 393 | OH | TYR61 | 14.307 | 28.423 | 34.828 | 1.00 | 4.54 |
| ATOM 394 | C | TYR61 | 11.620 | 26.572 | 41.833 | 1.00 | 21.95 |
| ATOM 395 | O | TYR61 | 10.437 | 26.816 | 41.609 | 1.00 | 22.47 |
| ATOM 396 | N | VAL62 | 12.102 | 26.293 | 43.037 | 1.00 | 24.47 |
| ATOM 397 | CA | VAL62 | 11.265 | 26.288 | 44.218 | 1.00 | 29.86 |
| ATOM 398 | CB | VAL62 | 11.750 | 25.231 | 45.207 | 1.00 | 28.92 |
| ATOM 399 | CG1 | VAL62 | 10.780 | 25.091 | 46.370 | 1.00 | 28.30 |
| ATOM 400 | CG2 | VAL62 | 11.909 | 23.926 | 44.480 | 1.00 | 28.58 |
| ATOM 401 | C | VAL62 | 11.494 | 27.680 | 44.786 | 1.00 | 34.67 |
| ATOM 402 | O | VAL62 | 11.584 | 27.879 | 45.993 | 1.00 | 39.01 |
| ATOM 403 | N | ARG63 | 11.589 | 28.638 | 43.874 | 1.00 | 38.40 |
| ATOM 404 | CA | ARG63 | 11.847 | 30.038 | 44.182 | 1.00 | 41.10 |
| ATOM 405 | CB | ARG63 | 12.041 | 30.804 | 42.874 | 1.00 | 42.02 |
| ATOM 406 | CG | ARG63 | 10.794 | 30.798 | 41.996 | 1.00 | 44.76 |
| ATOM 407 | CD | ARG63 | 11.072 | 31.197 | 40.550 | 1.00 | 46.61 |
| ATOM 408 | NE | ARG63 | 9.827 | 31.366 | 39.804 | 1.00 | 48.56 |
| ATOM 409 | CZ | ARG63 | 8.972 | 30.381 | 39.541 | 1.00 | 50.39 |
| ATOM 410 | NH1 | ARG63 | 9.225 | 29.145 | 39.955 | 1.00 | 50.83 |
| ATOM 411 | NH2 | ARG63 | 7.854 | 30.635 | 38.875 | 1.00 | 51.11 |
| ATOM 412 | C | ARG63 | 10.788 | 30.751 | 45.004 | 1.00 | 42.71 |
| ATOM 413 | O | ARG63 | 9.790 | 30.167 | 45.424 | 1.00 | 41.58 |
| ATOM 414 | N | SER64 | 11.047 | 32.036 | 45.224 | 1.00 | 46.12 |
| ATOM 415 | CA | SER64 | 10.155 | 32.922 | 45.954 | 1.00 | 49.96 |
| ATOM 416 | CB | SER64 | 10.400 | 32.826 | 47.454 | 1.00 | 50.57 |
| ATOM 417 | OG | SER64 | 9.374 | 33.507 | 48.157 | 1.00 | 53.70 |
| ATOM 418 | C | SER64 | 10.435 | 34.340 | 45.458 | 1.00 | 51.04 |
| ATOM 419 | O | SER64 | 11.300 | 35.047 | 45.985 | 1.00 | 50.38 |
| ATOM 420 | N | THR65 | 9.690 | 34.728 | 44.425 | 1.00 | 53.23 |
| ATOM 421 | CA | THR65 | 9.827 | 36.031 | 43.791 | 1.00 | 54.89 |
| ATOM 422 | CB | THR65 | 10.151 | 35.871 | 42.281 | 1.00 | 56.21 |
| ATOM 423 | OG1 | THR65 | 9.094 | 35.158 | 41.622 | 1.00 | 55.23 |
| ATOM 424 | CG2 | THR65 | 11.461 | 35.112 | 42.103 | 1.00 | 56.71 |
| ATOM 425 | C | THR65 | 8.582 | 36.911 | 43.939 | 1.00 | 56.01 |
| ATOM 426 | O | THR65 | 7.503 | 36.430 | 44.291 | 1.00 | 56.26 |
| ATOM 427 | N | PR066 | 8.728 | 38.222 | 43.676 | 1.00 | 56.49 |
| ATOM 428 | CD | PR066 | 10.019 | 38.866 | 43.377 | 1.00 | 56.96 |
| ATOM 429 | CA | PR066 | 7.666 | 39.228 | 43.758 | 1.00 | 56.28 |
| ATOM 430 | CB | PR066 | 8.369 | 40.502 | 43.313 | 1.00 | 57.08 |
| ATOM 431 | CG | PR066 | 9.759 | 40.287 | 43.786 | 1.00 | 58.08 |
| ATOM 432 | C | PR066 | 6.487 | 38.901 | 42.864 | 1.00 | 56.75 |
| ATOM 433 | O | PR066 | 5.477 | 39.604 | 42.874 | 1.00 | 57.23 |
| ATOM 434 | N | GLU67 | 6.631 | 37.849 | 42.072 | 1.00 | 56.42 |
| ATOM 435 | CA | GLU67 | 5.540 | 37.445 | 41.193 | 1.00 | 56.82 |
| ATOM 436 | CB | GLU67 | 6.048 | 36.487 | 40.115 | 1.00 | 61.19 |
| ATOM 437 | CG | GLU67 | 6.421 | 35.108 | 40.637 | 1.00 | 66.99 |
| ATOM 438 | CD | GLU67 | 7.123 | 34.261 | 39.594 | 1.00 | 69.61 |
| ATOM 439 | OE1 | GLU67 | 8.253 | 34.618 | 39.201 | 1.00 | 70.19 |
| ATOM 440 | OE2 | GLU67 | 6.541 | 33.241 | 39.168 | 1.00 | 70.18 |
| ATOM 441 | C | GLU67 | 4.406 | 36.803 | 41.984 | 1.00 | 54.30 |
| ATOM 442 | O | GLU67 | 3.241 | 36.940 | 41.633 | 1.00 | 54.25 |
| ATOM 443 | N | GLY68 | 4.753 | 36.116 | 43.076 | 1.00 | 50.50 |

[Table 2] (continued)

| | | | | | | | |
|---|---|---|---|---|---|---|---|
| ATOM 444 | CA | GLY68 | 3.741 | 35.478 | 43.901 | 1.00 | 45.77 |
| ATOM 445 | C | GLY68 | 4.166 | 34.087 | 44.316 | 1.00 | 43.04 |
| ATOM 446 | O | GLY68 | 3.626 | 33.503 | 45.259 | 1.00 | 40.69 |
| ATOM 447 | N | SER69 | 5.154 | 33.564 | 43.599 | 1.00 | 42.30 |
| ATOM 448 | CA | SER69 | 5.690 | 32.230 | 43.845 | 1.00 | 41.02 |
| ATOM 449 | CB | SER69 | 6.769 | 31.902 | 42.804 | 1.00 | 41.03 |
| ATOM 450 | OG | SER69 | 6.438 | 32.404 | 41.517 | 1.00 | 42.34 |
| ATOM 451 | C | SER69 | 6.301 | 32.126 | 45.240 | 1.00 | 39.68 |
| ATOM 452 | O | SER69 | 7.163 | 32.920 | 45.607 | 1.00 | 38.89 |
| ATOM 453 | N | GLU70 | 5.857 | 31.143 | 46.014 | 1.00 | 39.96 |
| ATOM 454 | CA | GLU70 | 6.388 | 30.942 | 47.355 | 1.00 | 40.53 |
| ATOM 455 | CB | GLU70 | 5.265 | 31.074 | 48.391 | 1.00 | 44.80 |
| ATOM 456 | CG | GLU70 | 4.675 | 32.483 | 48.492 | 1.00 | 52.74 |
| ATOM 457 | CD | GLU70 | 5.705 | 33.554 | 48.900 | 1.00 | 58.55 |
| ATOM 458 | OE1 | GLU70 | 5.362 | 34.763 | 48.866 | 1.00 | 59.55 |
| ATOM 459 | OE2 | GLU70 | 6.852 | 33.192 | 49.258 | 1.00 | 60.30 |
| ATOM 460 | C | GLU70 | 7.075 | 29.583 | 47.483 | 1.00 | 38.65 |
| ATOM 461 | O | GLU70 | 6.807 | 28.660 | 46.704 | 1.00 | 37.89 |
| ATOM 462 | N | VAL71 | 7.962 | 29.459 | 48.466 | 1.00 | 35.96 |
| ATOM 463 | CA | VAL71 | 8.670 | 28.207 | 48.653 | 1.00 | 34.46 |
| ATOM 464 | CB | VAL71 | 9.723 | 28.319 | 49.755 | 1.00 | 33.00 |
| ATOM 465 | CG1 | VAL71 | 10.236 | 26.949 | 50.120 | 1.00 | 33.91 |
| ATOM 466 | CG2 | VAL71 | 10.885 | 29.152 | 49.249 | 1.00 | 32.56 |
| ATOM 467 | C | VAL71 | 7.730 | 27.042 | 48.931 | 1.00 | 34.75 |
| ATOM 468 | O | VAL71 | 7.851 | 25.985 | 48.310 | 1.00 | 37.23 |
| ATOM 469 | N | GLY72 | 6.783 | 27.219 | 49.841 | 1.00 | 33.37 |
| ATOM 470 | CA | GLY72 | 5.842 | 26.139 | 50.105 | 1.00 | 32.39 |
| ATOM 471 | C | GLY72 | 5.066 | 25.644 | 48.879 | 1.00 | 31.10 |
| ATOM 472 | O | GLY72 | 4.631 | 24.493 | 48.859 | 1.00 | 28.98 |
| ATOM 473 | N | ASP73 | 4.878 | 26.503 | 47.870 | 1.00 | 31.05 |
| ATOM 474 | CA | ASP73 | 4.156 | 26.129 | 46.650 | 1.00 | 31.14 |
| ATOM 475 | CB | ASP73 | 4.389 | 27.147 | 45.532 | 1.00 | 34.00 |
| ATOM 476 | CG | ASP73 | 3.759 | 28.491 | 45.817 | 1.00 | 38.43 |
| ATOM 477 | OD1 | ASP73 | 3.758 | 29.355 | 44.907 | 1.00 | 41.88 |
| ATOM 478 | OD2 | ASP73 | 3.262 | 28.690 | 46.945 | 1.00 | 41.23 |
| ATOM 479 | C | ASP73 | 4.675 | 24.785 | 46.189 | 1.00 | 30.89 |
| ATOM 480 | O | ASP73 | 5.875 | 24.544 | 46.256 | 1.00 | 32.81 |
| ATOM 481 | N | PHE74 | 3.796 | 23.921 | 45.694 | 1.00 | 28.84 |
| ATOM 482 | CA | PHE74 | 4.233 | 22.595 | 45.271 | 1.00 | 27.21 |
| ATOM 483 | CB | PHE74 | 4.728 | 21.834 | 46.502 | 1.00 | 26.13 |
| ATOM 484 | CG | PHE74 | 5.407 | 20.551 | 46.185 | 1.00 | 25.61 |
| ATOM 485 | CD1 | PHE74 | 6.641 | 20.546 | 45.547 | 1.00 | 29.29 |
| ATOM 486 | CD2 | PHE74 | 4.805 | 19.344 | 46.496 | 1.00 | 24.94 |
| ATOM 487 | CE1 | PHE74 | 7.259 | 19.354 | 45.213 | 1.00 | 31.36 |
| ATOM 488 | CE2 | PHE74 | 5.408 | 18.149 | 46.168 | 1.00 | 27.38 |
| ATOM 489 | CZ | PHE74 | 6.640 | 18.149 | 45.527 | 1.00 | 30.18 |
| ATOM 490 | C | PHE74 | 3.080 | 21.837 | 44.604 | 1.00 | 27.31 |
| ATOM 491 | O | PHE74 | 1.912 | 22.034 | 44.951 | 1.00 | 28.04 |
| ATOM 492 | N | LEU75 | 3.402 | 20.965 | 43.654 | 1.00 | 23.99 |
| ATOM 493 | CA | LEU75 | 2.370 | 20.214 | 42.958 | 1.00 | 20.00 |
| ATOM 494 | CB | LEU75 | 2.222 | 20.725 | 41.534 | 1.00 | 19.88 |

[Table 2] (continued)

| | | | | | | | |
|---|---|---|---|---|---|---|---|
| ATOM 495 | CG | LEU75 | 0.868 | 20.487 | 40.865 | 1.00 | 21.27 |
| ATOM 496 | CD1 | LEU75 | 1.083 | 20.282 | 39.354 | 1.00 | 19.58 |
| ATOM 497 | CD2 | LEU75 | 0.190 | 19.279 | 41.474 | 1.00 | 18.85 |
| ATOM 498 | C | LEU75 | 2.755 | 18.758 | 42.911 | 1.00 | 18.82 |
| ATOM 499 | O | LEU75 | 3.587 | 18.369 | 42.102 | 1.00 | 19.49 |
| ATOM 500 | N | SER76 | 2.143 | 17.957 | 43.774 | 1.00 | 21.08 |
| ATOM 501 | CA | SER76 | 2.434 | 16.530 | 43.834 | 1.00 | 22.49 |
| ATOM 502 | CB | SER76 | 2.333 | 16.001 | 45.261 | 1.00 | 22.74 |
| ATOM 503 | OG | SER76 | 2.591 | 14.612 | 45.292 | 1.00 | 20.37 |
| ATOM 504 | C | SER76 | 1.507 | 15.720 | 42.967 | 1.00 | 23.58 |
| ATOM 505 | O | SER76 | 0.309 | 15.980 | 42.866 | 1.00 | 23.06 |
| ATOM 506 | N | LEU77 | 2.064 | 14.686 | 42.378 | 1.00 | 25.35 |
| ATOM 507 | CA | LEU77 | 1.280 | 13.862 | 41.509 | 1.00 | 27.55 |
| ATOM 508 | CB | LEU77 | 1.758 | 14.122 | 40.089 | 1.00 | 29.38 |
| ATOM 509 | CG | LEU77 | 1.176 | 13.275 | 38.980 | 1.00 | 32.75 |
| ATOM 510 | CD1 | LEU77 | -0.334 | 13.434 | 38.974 | 1.00 | 34.55 |
| ATOM 511 | CD2 | LEU77 | 1.796 | 13.695 | 37.661 | 1.00 | 32.83 |
| ATOM 512 | C | LEU77 | 1.445 | 12.402 | 41.913 | 1.00 | 28.86 |
| ATOM 513 | O | LEU77 | 2.527 | 11.826 | 41.760 | 1.00 | 26.84 |
| ATOM 514 | N | ASP78 | 0.386 | 11.811 | 42.465 | 1.00 | 29.41 |
| ATOM 515 | CA | ASP78 | 0.457 | 10.407 | 42.865 | 1.00 | 30.41 |
| ATOM 516 | CB | ASP78 | -0.150 | 10.186 | 44.255 | 1.00 | 31.87 |
| ATOM 517 | CG | ASP78 | -0.286 | 8.702 | 44.606 | 1.00 | 33.99 |
| ATOM 518 | OD1 | ASP78 | -1.025 | 7.993 | 43.894 | 1.00 | 35.38 |
| ATOM 519 | OD2 | ASP78 | 0.338 | 8.241 | 45.586 | 1.00 | 33.31 |
| ATOM 520 | C | ASP78 | -0.270 | 9.530 | 41.860 | 1.00 | 29.41 |
| ATOM 521 | O | ASP78 | -1.484 | 9.587 | 41.732 | 1.00 | 29.74 |
| ATOM 522 | N | LEU79 | 0.472 | 8.710 | 41.143 | 1.00 | 27.93 |
| ATOM 523 | CA | LEU79 | -0.169 | 7.858 | 40.184 | 1.00 | 28.08 |
| ATOM 524 | CB | LEU79 | 0.323 | 8.173 | 38.781 | 1.00 | 25.78 |
| ATOM 525 | CG | LEU79 | 1.676 | 7.627 | 38.371 | 1.00 | 24.57 |
| ATOM 526 | CD1 | LEU79 | 1.845 | 7.871 | 36.904 | 1.00 | 25.82 |
| ATOM 527 | CD2 | LEU79 | 2.779 | 8.274 | 39.166 | 1.00 | 26.37 |
| ATOM 528 | C | LEU79 | 0.114 | 6.420 | 40.548 | 1.00 | 31.25 |
| ATOM 529 | O | LEU79 | 1.265 | 6.017 | 40.712 | 1.00 | 32.14 |
| ATOM 530 | N | GLY80 | -0.955 | 5.652 | 40.699 | 1.00 | 34.99 |
| ATOM 531 | CA | GLY80 | -0.812 | 4.259 | 41.056 | 1.00 | 38.29 |
| ATOM 532 | C | GLY80 | -2.088 | 3.499 | 40.776 | 1.00 | 40.81 |
| ATOM 533 | O | GLY80 | -3.100 | 3.686 | 41.452 | 1.00 | 40.77 |
| ATOM 534 | N | GLY81 | -2.038 | 2.642 | 39.765 | 1.00 | 43.19 |
| ATOM 535 | CA | GLY81 | -3.197 | 1.850 | 39.422 | 1.00 | 45.84 |
| ATOM 536 | C | GLY81 | -3.936 | 2.428 | 38.244 | 1.00 | 49.22 |
| ATOM 537 | O | GLY81 | -3.328 | 2.825 | 37.241 | 1.00 | 49.20 |
| ATOM 538 | N | THR82 | -5.260 | 2.465 | 38.365 | 1.00 | 51.93 |
| ATOM 539 | CA | THR82 | -6.117 | 3.003 | 37.312 | 1.00 | 54.41 |
| ATOM 540 | CB | THR82 | -7.344 | 2.090 | 37.060 | 1.00 | 56.74 |
| ATOM 541 | OG1 | THR82 | -6.908 | 0.727 | 36.952 | 1.00 | 60.43 |
| ATOM 542 | CG2 | THR82 | -8.043 | 2.473 | 35.752 | 1.00 | 58.23 |
| ATOM 543 | C | THR82 | -6.584 | 4.382 | 37.759 | 1.00 | 52.48 |
| ATOM 544 | O | THR82 | -7.308 | 5.077 | 37.046 | 1.00 | 52.21 |
| ATOM 545 | N | ASN83 | -6.148 | 4.778 | 38.946 | 1.00 | 50.63 |

[Table 2] (continued)

| | | | | | | | |
|---|---|---|---|---|---|---|---|
| ATOM 546 | CA | ASN83 | -6.523 | 6.071 | 39.466 | 1.00 | 50.52 |
| ATOM 547 | CB | ASN83 | -7.574 | 5.911 | 40.568 | 1.00 | 53.97 |
| ATOM 548 | CG | ASN83 | -8.955 | 5.560 | 40.020 | 1.00 | 58.88 |
| ATOM 549 | OD1 | ASN83 | -9.508 | 6.290 | 39.190 | 1.00 | 60.51 |
| ATOM 550 | ND2 | ASN83 | -9.521 | 4.444 | 40.489 | 1.00 | 60.30 |
| ATOM 551 | C | ASN83 | -5.338 | 6.861 | 39.997 | 1.00 | 48.79 |
| ATOM 552 | O | ASN83 | -4.682 | 6.442 | 40.956 | 1.00 | 48.09 |
| ATOM 553 | N | PHE84 | -5.068 | 8.003 | 39.356 | 1.00 | 45.51 |
| ATOM 554 | CA | PHE84 | -3.995 | 8.907 | 39.772 | 1.00 | 40.32 |
| ATOM 555 | CB | PHE84 | -2.998 | 9.145 | 38.644 | 1.00 | 39.20 |
| ATOM 556 | CG | PHE84 | -3.436 | 10.175 | 37.652 | 1.00 | 39.52 |
| ATOM 557 | CD1 | PHE84 | -4.096 | 9.802 | 36.494 | 1.00 | 40.87 |
| ATOM 558 | CD2 | PHE84 | -3.159 | 11.524 | 37.860 | 1.00 | 39.69 |
| ATOM 559 | CE1 | PHE84 | -4.479 | 10.758 | 35.549 | 1.00 | 41.79 |
| ATOM 560 | CE2 | PHE84 | -3.540 | 12.490 | 36.922 | 1.00 | 40.16 |
| ATOM 561 | CZ | PHE84 | -4.198 | 12.105 | 35.762 | 1.00 | 40.38 |
| ATOM 562 | C | PHE84 | -4.604 | 10.246 | 40.176 | 1.00 | 37.84 |
| ATOM 563 | O | PHE84 | -5.405 | 10.806 | 39.439 | 1.00 | 37.11 |
| ATOM 564 | N | ARG85 | -4.216 | 10.762 | 41.338 | 1.00 | 36.37 |
| ATOM 565 | CA | ARG85 | -4.738 | 12.032 | 41.840 | 1.00 | 35.14 |
| ATOM 566 | CB | ARG85 | -5.496 | 11.779 | 43.136 | 1.00 | 39.80 |
| ATOM 567 | CG | ARG85 | -4.888 | 10.677 | 43.970 | 1.00 | 47.71 |
| ATOM 568 | CD | ARG85 | -5.948 | 9.964 | 44.805 | 1.00 | 55.73 |
| ATOM 569 | NE | ARG85 | -5.391 | 8.801 | 45.493 | 1.00 | 62.76 |
| ATOM 570 | CZ | ARG85 | -4.799 | 7.772 | 44.883 | 1.00 | 65.65 |
| ATOM 571 | NH1 | ARG85 | -4.684 | 7.749 | 43.557 | 1.00 | 63.79 |
| ATOM 572 | NH2 | ARG85 | -4.314 | 6.765 | 45.605 | 1.00 | 66.67 |
| ATOM 573 | C | ARG85 | -3.664 | 13.088 | 42.075 | 1.00 | 32.14 |
| ATOM 574 | O | ARG85 | -2.561 | 12.772 | 42.522 | 1.00 | 32.77 |
| ATOM 575 | N | VAL86 | -3.977 | 14.345 | 41.778 | 1.00 | 27.45 |
| ATOM 576 | CA | VAL86 | -2.997 | 15.405 | 41.983 | 1.00 | 26.49 |
| ATOM 577 | CB | VAL86 | -2.975 | 16.400 | 40.821 | 1.00 | 24.77 |
| ATOM 578 | CG1 | VAL86 | -3.033 | 15.655 | 39.510 | 1.00 | 26.70 |
| ATOM 579 | CG2 | VAL86 | -4.109 | 17.373 | 40.948 | 1.00 | 24.73 |
| ATOM 580 | C | VAL86 | -3.292 | 16.177 | 43.257 | 1.00 | 26.66 |
| ATOM 581 | O | VAL86 | -4.401 | 16.121 | 43.779 | 1.00 | 28.06 |
| ATOM 582 | N | MET87 | -2.289 | 16.888 | 43.757 | 1.00 | 26.93 |
| ATOM 583 | CA | MET87 | -2.427 | 17.677 | 44.973 | 1.00 | 25.08 |
| ATOM 584 | CB | MET87 | -1.748 | 16.979 | 46.138 | 1.00 | 25.05 |
| ATOM 585 | CG | MET87 | -1.674 | 17.833 | 47.375 | 1.00 | 24.83 |
| ATOM 586 | SD | MET87 | -0.509 | 17.090 | 48.503 | 1.00 | 30.68 |
| ATOM 587 | CE | MET87 | -1.544 | 16.749 | 49.894 | 1.00 | 29.41 |
| ATOM 588 | C | MET87 | -1.768 | 19.021 | 44.774 | 1.00 | 24.52 |
| ATOM 589 | O | MET87 | -0.638 | 19.097 | 44.298 | 1.00 | 27.12 |
| ATOM 590 | N | LEU88 | -2.455 | 20.087 | 45.146 | 1.00 | 22.16 |
| ATOM 591 | CA | LEU88 | -1.872 | 21.398 | 44.975 | 1.00 | 20.70 |
| ATOM 592 | CB | LEU88 | -2.825 | 22.309 | 44.230 | 1.00 | 20.34 |
| ATOM 593 | CG | LEU88 | -2.178 | 23.663 | 43.991 | 1.00 | 23.49 |
| ATOM 594 | CD1 | LEU88 | -0.806 | 23.470 | 43.354 | 1.00 | 24.39 |
| ATOM 595 | CD2 | LEU88 | -3.078 | 24.493 | 43.094 | 1.00 | 25.91 |
| ATOM 596 | C | LEU88 | -1.535 | 22.021 | 46.301 | 1.00 | 19.94 |

[Table 2] (continued)

| | | | | | | | |
|---|---|---|---|---|---|---|---|
| ATOM 597 | O | LEU88 | -2.225 | 21.794 | 47.282 | 1.00 | 21.18 |
| ATOM 598 | N | VAL89 | -0.463 | 22.799 | 46.343 | 1.00 | 20.16 |
| ATOM 599 | CA | VAL89 | -0.082 | 23.462 | 47.580 | 1.00 | 21.15 |
| ATOM 600 | CB | VAL89 | 0.984 | 22.676 | 48.357 | 1.00 | 14.95 |
| ATOM 601 | CG1 | VAL89 | 1.292 | 23.385 | 49.657 | 1.00 | 7.73 |
| ATOM 602 | CG2 | VAL89 | 0.515 | 21.268 | 48.609 | 1.00 | 10.59 |
| ATOM 603 | C | VAL89 | 0.491 | 24.829 | 47.254 | 1.00 | 27.10 |
| ATOM 604 | O | VAL89 | 1.410 | 24.939 | 46.442 | 1.00 | 27.22 |
| ATOM 605 | N | LYS90 | -0.066 | 25.866 | 47.875 | 1.00 | 33.21 |
| ATOM 606 | CA | LYS90 | 0.401 | 27.235 | 47.671 | 1.00 | 40.01 |
| ATOM 607 | CB | LYS90 | -0.443 | 27.962 | 46.604 | 1.00 | 41.03 |
| ATOM 608 | CG | LYS90 | -1.941 | 27.979 | 46.850 | 1.00 | 47.19 |
| ATOM 609 | CD | LYS90 | -2.749 | 28.454 | 45.622 | 1.00 | 52.33 |
| ATOM 610 | CE | LYS90 | -4.274 | 28.393 | 45.899 | 1.00 | 55.73 |
| ATOM 611 | NZ | LYS90 | -5.161 | 28.724 | 44.731 | 1.00 | 56.02 |
| ATOM 612 | C | LYS90 | 0.384 | 28.009 | 48.981 | 1.00 | 43.61 |
| ATOM 613 | O | LYS90 | -0.577 | 27.943 | 49.747 | 1.00 | 44.04 |
| ATOM 614 | N | VAL91 | 1.469 | 28.728 | 49.241 | 1.00 | 47.88 |
| ATOM 615 | CA | VAL91 | 1.587 | 29.513 | 50.458 | 1.00 | 51.82 |
| ATOM 616 | CB | VAL91 | 3.059 | 29.780 | 50.788 | 1.00 | 51.29 |
| ATOM 617 | CG1 | VAL91 | 3.160 | 30.748 | 51.947 | 1.00 | 54.88 |
| ATOM 618 | CG2 | VAL91 | 3.749 | 28.479 | 51.137 | 1.00 | 48.18 |
| ATOM 619 | C | VAL91 | 0.849 | 30.846 | 50.355 | 1.00 | 55.01 |
| ATOM 620 | O | VAL91 | 0.994 | 31.569 | 49.369 | 1.00 | 54.57 |
| ATOM 621 | N | GLY92 | 0.060 | 31.157 | 51.382 | 1.00 | 59.16 |
| ATOM 622 | CA | GLY92 | -0.696 | 32.396 | 51.401 | 1.00 | 64.58 |
| ATOM 623 | C | GLY92 | -0.305 | 33.297 | 52.558 | 1.00 | 68.39 |
| ATOM 624 | O | GLY92 | 0.637 | 32.992 | 53.295 | 1.00 | 66.92 |
| ATOM 625 | N | GLU93 | -1.025 | 34.410 | 52.712 | 1.00 | 73.13 |
| ATOM 626 | CA | GLU93 | -0.751 | 35.351 | 53.792 | 1.00 | 78.27 |
| ATOM 627 | CB | GLU93 | -0.623 | 36.780 | 53.248 | 1.00 | 79.11 |
| ATOM 628 | CG | GLU93 | 0.334 | 37.635 | 54.077 | 1.00 | 82.44 |
| ATOM 629 | CD | GLU93 | 0.218 | 39.120 | 53.795 | 1.00 | 84.34 |
| ATOM 630 | OE1 | GLU93 | -0.877 | 39.688 | 54.018 | 1.00 | 84.71 |
| ATOM 631 | OE2 | GLU93 | 1.228 | 39.718 | 53.359 | 1.00 | 85.45 |
| ATOM 632 | C | GLU93 | -1.813 | 35.309 | 54.904 | 1.00 | 80.72 |
| ATOM 633 | O | GLU93 | -1.469 | 35.340 | 56.086 | 1.00 | 81.42 |
| ATOM 634 | N | GLY94 | -3.093 | 35.240 | 54.536 | 1.00 | 83.03 |
| ATOM 635 | CA | GLY94 | -4.153 | 35.182 | 55.538 | 1.00 | 85.37 |
| ATOM 636 | C | GLY94 | -4.867 | 36.502 | 55.792 | 1.00 | 87.51 |
| ATOM 637 | O | GLY94 | -4.356 | 37.562 | 55.430 | 1.00 | 88.65 |
| ATOM 638 | N | GLU95 | -6.041 | 36.447 | 56.427 | 1.00 | 88.43 |
| ATOM 639 | CA | GLU95 | -6.831 | 37.653 | 56.716 | 1.00 | 88.66 |
| ATOM 640 | CB | GLU95 | -8.192 | 37.281 | 57.328 | 1.00 | 89.61 |
| ATOM 641 | CG | GLU95 | -9.077 | 36.406 | 56.448 | 1.00 | 90.41 |
| ATOM 642 | CD | GLU95 | -8.620 | 34.958 | 56.408 | 1.00 | 91.01 |
| ATOM 643 | OE1 | GLU95 | -9.089 | 34.211 | 55.523 | 1.00 | 90.26 |
| ATOM 644 | OE2 | GLU95 | -7.800 | 34.565 | 57.266 | 1.00 | 91.81 |
| ATOM 645 | C | GLU95 | -6.115 | 38.625 | 57.652 | 1.00 | 88.62 |
| ATOM 646 | O | GLU95 | -6.576 | 39.748 | 57.868 | 1.00 | 88.29 |
| ATOM 647 | N | GLU96 | -4.991 | 38.182 | 58.208 | 1.00 | 89.03 |

[Table 2] (continued)

| | | | | | | | |
|---|---|---|---|---|---|---|---|
| ATOM 648 | CA | GLU96 | -4. 200 | 38.995 | 59.124 | 1.00 | 88.80 |
| ATOM 649 | CB | GLU96 | -4.065 | 38.282 | 60.476 | 1.00 | 88.55 |
| ATOM 650 | CG | GLU96 | -5.368 | 38.155 | 61.268 | 1.00 | 89.59 |
| ATOM 651 | CD | GLU96 | -6.400 | 37.262 | 60.593 | 1.00 | 90.56 |
| ATOM 652 | OE1 | GLU96 | -6.163 | 36.040 | 60.481 | 1.00 | 90.53 |
| ATOM 653 | OE2 | GLU96 | -7.452 | 37.785 | 60.172 | 1.00 | 90.67 |
| ATOM 654 | C | GLU96 | -2.810 | 39.327 | 58.519 | 1.00 | 88.40 |
| ATOM 655 | O | GLU96 | -2.097 | 40.166 | 59.052 | 1.00 | 89.12 |
| ATOM 656 | N | GLY97 | -2.431 | 38.700 | 57.404 | 1.00 | 86.87 |
| ATOM 657 | CA | GLY97 | -1.133 | 38.917 | 56.789 | 1.00 | 85.05 |
| ATOM 658 | C | GLY97 | -0.161 | 37.976 | 57.494 | 1.00 | 84.17 |
| ATOM 659 | O | GLY97 | 1.044 | 38.179 | 57.605 | 1.00 | 83.49 |
| ATOM 660 | N | GLN98 | -0.820 | 36.901 | 57.977 | 1.00 | 83.07 |
| ATOM 661 | CA | GLN98 | -0.253 | 35.810 | 58.769 | 1.00 | 82.28 |
| ATOM 662 | CB | GLN98 | -1.346 | 34.825 | 59.250 | 1.00 | 82.41 |
| ATOM 663 | CG | GLN98 | -2.647 | 35.462 | 59.699 | 1.00 | 83.61 |
| ATOM 664 | CD | GLN98 | -3.740 | 34.427 | 60.007 | 1.00 | 84.16 |
| ATOM 665 | OE1 | GLN98 | -3.606 | 33.239 | 59.714 | 1.00 | 84.01 |
| ATOM 666 | NE2 | GLN98 | -4.905 | 34.685 | 60.592 | 1.00 | 84.46 |
| ATOM 667 | C | GLN98 | 0.735 | 34.981 | 58.011 | 1.00 | 81.85 |
| ATOM 668 | O | GLN98 | 1.955 | 35.200 | 57.956 | 1.00 | 83.51 |
| ATOM 669 | N | TRP99 | 0.118 | 33.962 | 57.470 | 1.00 | 79.05 |
| ATOM 670 | CA | TRP99 | 0.703 | 32.914 | 56.706 | 1.00 | 75.85 |
| ATOM 671 | CB | TRP99 | 1.993 | 32.398 | 57.308 | 1.00 | 73.88 |
| ATOM 672 | CG | TRP99 | 2.968 | 31.780 | 56.325 | 1.00 | 71.82 |
| ATOM 673 | CD2 | TRP99 | 3.211 | 30.386 | 56.075 | 1.00 | 70.49 |
| ATOM 674 | CE2 | TRP99 | 4.222 | 30.308 | 55.123 | 1.00 | 69.72 |
| ATOM 675 | CE3 | TRP99 | 2.671 | 29.200 | 56.550 | 1.00 | 69.52 |
| ATOM 676 | CD1 | TRP99 | 3.832 | 32.464 | 55.525 | 1.00 | 71.99 |
| ATOM 677 | NE1 | TRP99 | 4.598 | 31.589 | 54.790 | 1.00 | 71.07 |
| ATOM 678 | CZ2 | TRP99 | 4.692 | 29.089 | 54.624 | 1.00 | 67.81 |
| ATOM 679 | CZ3 | TRP99 | 3.136 | 27.984 | 56.080 | 1.00 | 67.31 |
| ATOM 680 | CH2 | TRP99 | 4.151 | 27.945 | 55.111 | 1.00 | 67.77 |
| ATOM 681 | C | TRP99 | -0.247 | 31.793 | 56.673 | 1.00 | 74.58 |
| ATOM 682 | O | TRP99 | -1.060 | 31.567 | 57.556 | 1.00 | 75.00 |
| ATOM 683 | N | SER100 | -0.090 | 31.087 | 55.647 | 1.00 | 72.11 |
| ATOM 684 | CA | SER100 | -0.948 | 29.999 | 55.517 | 1.00 | 68.48 |
| ATOM 685 | CB | SER100 | -2.376 | 30.466 | 55.232 | 1.00 | 68. 40 |
| ATOM 686 | OG | SER100 | -2.467 | 31.128 | 53.985 | 1.00 | 68.76 |
| ATOM 687 | C | SER100 | -0.522 | 29.152 | 54.382 | 1.00 | 66.28 |
| ATOM 688 | O | SER100 | 0.405 | 29.473 | 53.632 | 1.00 | 65.13 |
| ATOM 689 | N | VAL101 | -1.225 | 28.028 | 54.291 | 1.00 | 64.27 |
| ATOM 690 | CA | VAL101 | -0.982 | 27.030 | 53.262 | 1.00 | 62.66 |
| ATOM 691 | CB | VAL101 | 0.090 | 26.023 | 53.715 | 1.00 | 62.98 |
| ATOM 692 | CG1 | VAL101 | 1.493 | 26.554 | 53.459 | 1.00 | 66.77 |
| ATOM 693 | CG2 | VAL101 | -0.075 | 25.688 | 55.198 | 1.00 | 63.17 |
| ATOM 694 | C | VAL101 | -2.219 | 26.243 | 52.878 | 1.00 | 60.88 |
| ATOM 695 | O | VAL101 | -2.561 | 25.258 | 53.530 | 1.00 | 60.62 |
| ATOM 696 | N | LYS102 | -2.880 | 26.671 | 51.810 | 1.00 | 58.24 |
| ATOM 697 | CA | LYS102 | -4.066 | 25.981 | 51.337 | 1.00 | 56.12 |
| ATOM 698 | CB | LYS102 | -4.887 | 26.880 | 50.410 | 1.00 | 57.06 |

[Table 2] (continued)

| | | | | | | | |
|---|---|---|---|---|---|---|---|
| ATOM 699 | CG | LYS102 | -5.884 | 27.806 | 51.111 | 1.00 | 60.55 |
| ATOM 700 | CD | LYS102 | -7.056 | 27.038 | 51.748 | 1.00 | 63.17 |
| ATOM 701 | CE | LYS102 | -8.282 | 27.944 | 52.036 | 1.00 | 64.70 |
| ATOM 702 | NZ | LYS 102 | -8.021 | 29.150 | 52.899 | 1.00 | 63.52 |
| ATOM 703 | C | LYS102 | -3.677 | 24.710 | 50.596 | 1.00 | 54.04 |
| ATOM 704 | O | LYS102 | -2.599 | 24.609 | 50.007 | 1.00 | 52.35 |
| ATOM 705 | N | THR103 | -4.576 | 23.738 | 50.631 | 1.00 | 52.24 |
| ATOM 706 | CA | THR103 | -4.345 | 22.474 | 49.972 | 1.00 | 49.72 |
| ATOM 707 | CB | THR103 | -4.139 | 21.385 | 51.010 | 1.00 | 49.49 |
| ATOM 708 | OG1 | THR103 | -3.399 | 20.316 | 50.422 | 1.00 | 53.11 |
| ATOM 709 | CG2 | THR103 | -5.475 | 20.861 | 51.517 | 1.00 | 48.32 |
| ATOM 710 | C | THR103 | -5.563 | 22.158 | 49.106 | 1.00 | 49.61 |
| ATOM 711 | O | THR103 | -6.693 | 22.435 | 49.507 | 1.00 | 50.24 |
| ATOM 712 | N | LYS104 | -5.330 | 21.587 | 47.924 | 1.00 | 48.56 |
| ATOM 713 | CA | LYS104 | -6.404 | 21.251 | 46.983 | 1.00 | 48.50 |
| ATOM 714 | CB | LYS104 | -6.469 | 22.298 | 45.864 | 1.00 | 49.98 |
| ATOM 715 | CG | LYS104 | -6.753 | 23.737 | 46.313 | 1.00 | 56.05 |
| ATOM 716 | CD | LYS104 | -8.195 | 23.932 | 46.814 | 1.00 | 60.38 |
| ATOM 717 | CE | LYS104 | -8.456 | 25.383 | 47.254 | 1.00 | 62.32 |
| ATOM 718 | NZ | LYS104 | -9.845 | 25.649 | 47.761 | 1.00 | 61.31 |
| ATOM 719 | C | LYS104 | -6.224 | 19.878 | 46.332 | 1.00 | 48.13 |
| ATOM 720 | O | LYS104 | -5.286 | 19.685 | 45.563 | 1.00 | 49.60 |
| ATOM 721 | N | HIS105 | -7.127 | 18.936 | 46.606 | 1.00 | 47.57 |
| ATOM 722 | CA | HIS105 | -7.023 | 17.601 | 46.010 | 1.00 | 47.23 |
| ATOM 723 | CB | HIS105 | -7.165 | 16.529 | 47.074 | 1.00 | 47.40 |
| ATOM 724 | CG | HIS105 | -6.241 | 16.709 | 48.228 | 1.00 | 49.37 |
| ATOM 725 | CD2 | HIS105 | -5.098 | 16.066 | 48.563 | 1.00 | 49.55 |
| ATOM 726 | ND1 | HIS105 | -6.459 | 17.648 | 49.212 | 1.00 | 50.43 |
| ATOM 727 | CE1 | HIS105 | -5.493 | 17.571 | 50.110 | 1.00 | 51.38 |
| ATOM 728 | NE2 | HIS105 | -4.655 | 16.619 | 49.740 | 1.00 | 50.58 |
| ATOM 729 | C | HIS105 | -8.030 | 17.304 | 44.907 | 1.00 | 46.39 |
| ATOM 730 | O | HIS105 | -9.195 | 17.692 | 44.985 | 1.00 | 49.62 |
| ATOM 731 | N | GLN106 | -7.575 | 16.580 | 43.894 | 1.00 | 42.98 |
| ATOM 732 | CA | GLN106 | -8.419 | 16.226 | 42.771 | 1.00 | 40.44 |
| ATOM 733 | CB | GLN106 | -8.284 | 17.285 | 41.685 | 1.00 | 40.41 |
| ATOM 734 | CG | GLN106 | -9.546 | 17.548 | 40.908 | 1.00 | 40.59 |
| ATOM 735 | CD | GLN106 | -10.428 | 16.324 | 40.813 | 1.00 | 40.54 |
| ATOM 736 | OE1 | GLN106 | -11.061 | 15.927 | 41.795 | 1.00 | 39.16 |
| ATOM 737 | NE2 | GLN106 | -10.475 | 15.712 | 39.631 | 1.00 | 40.06 |
| ATOM 738 | C | GLN106 | -7.940 | 14.878 | 42.249 | 1.00 | 40.70 |
| ATOM 739 | 0 | GLN106 | -6.745 | 14.699 | 42.012 | 1.00 | 41.69 |
| ATOM 740 | N | MET107 | -8.867 | 13.937 | 42.066 | 1.00 | 41.01 |
| ATOM 741 | CA | MET107 | -8.532 | 12.588 | 41.599 | 1.00 | 40.17 |
| ATOM 742 | CB | MET107 | -9.083 | 11.551 | 42.588 | 1.00 | 42.07 |
| ATOM 743 | CG | MET107 | -8.772 | 10.094 | 42.249 | 1.00 | 44.67 |
| ATOM 744 | SD | MET107 | -10.185 | 9.202 | 41.551 | 1.00 | 50.71 |
| ATOM 745 | CE | MET107 | -10.688 | 8.056 | 42.927 | 1.00 | 43.37 |
| ATOM 746 | C | MET107 | -9.059 | 12.294 | 40.204 | 1.00 | 38.93 |
| ATOM 747 | O | MET107 | -10.264 | 12.285 | 39.979 | 1.00 | 41.30 |
| ATOM 748 | N | TYR108 | -8.161 | 12.044 | 39.264 | 1.00 | 37.96 |
| ATOM 749 | CA | TYR108 | -8.588 | 11.750 | 37.907 | 1.00 | 38.48 |

[Table 2] (continued)

| | | | | | | | |
|---|---|---|---|---|---|---|---|
| ATOM 750 | CB | TYR108 | -7.670 | 12.454 | 36.900 | 1.00 | 35.63 |
| ATOM 751 | CG | TYR108 | -7.732 | 13.972 | 36.977 | 1.00 | 35.18 |
| ATOM 752 | CD1 | TYR108 | -7.492 | 14.645 | 38.180 | 1.00 | 37.21 |
| ATOM 753 | CE1 | TYR108 | -7.550 | 16.047 | 38.268 | 1.00 | 34.81 |
| ATOM 754 | CD2 | TYR108 | -8.031 | 14.735 | 35.857 | 1.00 | 34.14 |
| ATOM 755 | CE2 | TYR108 | -8.092 | 16.134 | 35.931 | 1.00 | 35.09 |
| ATOM 756 | CZ | TYR108 | -7.852 | 16.783 | 37.139 | 1.00 | 35.25 |
| ATOM 757 | OH | TYR108 | -7.937 | 18.158 | 37.211 | 1.00 | 33.27 |
| ATOM 758 | C | TYR108 | -8.583 | 10.241 | 37.689 | 1.00 | 40.17 |
| ATOM 759 | O | TYR108 | -7.817 | 9.514 | 38.325 | 1.00 | 38.04 |
| ATOM 760 | N | SER109 | -9.469 | 9.765 | 36.818 | 1.00 | 42.63 |
| ATOM 761 | CA | SER109 | -9.524 | 8.341 | 36.530 | 1.00 | 44.60 |
| ATOM 762 | CB | SER109 | -10.929 | 7.787 | 36.736 | 1.00 | 43.05 |
| ATOM 763 | OG | SER109 | -10.926 | 6.385 | 36.522 | 1.00 | 41.66 |
| ATOM 764 | C | SER109 | -9.090 | 8.106 | 35.097 | 1.00 | 46.74 |
| ATOM 765 | O | SER109 | -9.531 | 8.799 | 34.182 | 1.00 | 44.65 |
| ATOM 766 | N | ILE110 | -8.217 | 7.120 | 34.918 | 1.00 | 50.31 |
| ATOM 767 | CA | ILE110 | -7.686 | 6.782 | 33.608 | 1.00 | 55.29 |
| ATOM 768 | CB | ILE110 | -6.326 | 6.060 | 33.731 | 1.00 | 54.32 |
| ATOM 769 | CG2 | ILE110 | -5.690 | 5.932 | 32.364 | 1.00 | 56.16 |
| ATOM 770 | CG1 | ILE110 | -5.373 | 6.844 | 34.626 | 1.00 | 53.30 |
| ATOM 771 | CD1 | ILE110 | -4.067 | 6.117 | 34.869 | 1.00 | 51.57 |
| ATOM 772 | C | ILE110 | -8.621 | 5.882 | 32.799 | 1.00 | 59.70 |
| ATOM 773 | O | ILE110 | -8.906 | 4.749 | 33.199 | 1.00 | 58.82 |
| ATOM 774 | N | PRO111 | -9.114 | 6.381 | 31.650 | 1.00 | 64.10 |
| ATOM 775 | CD | PR0111 | -8.972 | 7.759 | 31.142 | 1.00 | 64.05 |
| ATOM 776 | CA | PRO111 | -10.012 | 5.608 | 30.788 | 1.00 | 68.40 |
| ATOM 777 | CB | PRO111 | -10.118 | 6.484 | 29.547 | 1.00 | 67.29 |
| ATOM 778 | CG | PRO111 | -10.105 | 7.860 | 30.144 | 1.00 | 63.88 |
| ATOM 779 | C | PRO111 | -9.416 | 4.231 | 30.494 | 1.00 | 72.88 |
| ATOM 780 | O | PRO111 | -8.195 | 4.065 | 30.506 | 1.00 | 73.72 |
| ATOM 781 | N | GLU112 | -10.280 | 3.250 | 30.239 | 1.00 | 77.60 |
| ATOM 782 | CA | GLU112 | -9.845 | 1.879 | 29.958 | 1.00 | 80.79 |
| ATOM 783 | CB | GLU112 | -11.072 | 0.968 | 29.798 | 1.00 | 82.29 |
| ATOM 784 | CG | GLU112 | -10.748 | -0.498 | 29.524 | 1.00 | 83.62 |
| ATOM 785 | CD | GLU112 | -11.896 | -1.247 | 28.851 | 1.00 | 85.04 |
| ATOM 786 | OE1 | GLU112 | -11.697 | -2.423 | 28.470 | 1.00 | 85.60 |
| ATOM 787 | OE2 | GLU112 | -12.995 | -0.665 | 28.700 | 1.00 | 85.42 |
| ATOM 788 | C | GLU112 | -8.971 | 1.806 | 28.702 | 1.00 | 82.21 |
| ATOM 789 | O | GLU112 | -7.936 | 1.137 | 28.693 | 1.00 | 82.17 |
| ATOM 790 | N | ASP113 | -9.394 | 2.501 | 27.649 | 1.00 | 83.97 |
| ATOM 791 | CA | ASP113 | -8.660 | 2.522 | 26.385 | 1.00 | 85.79 |
| ATOM 792 | CB | ASP113 | -9.506 | 3.221 | 25.302 | 1.00 | 86.45 |
| ATOM 793 | CG | ASP113 | -9.961 | 4.624 | 25.712 | 1.00 | 87.32 |
| ATOM 794 | OD1 | ASP113 | -10.655 | 4.756 | 26.748 | 1.00 | 86.75 |
| ATOM 795 | OD2 | ASP113 | -9.629 | 5.595 | 24.991 | 1.00 | 87.18 |
| ATOM 796 | C | ASP113 | -7.297 | 3.215 | 26.533 | 1.00 | 86.44 |
| ATOM 797 | O | ASP113 | -6.467 | 3.195 | 25.617 | 1.00 | 86.35 |
| ATOM 798 | N | ALA114 | -7.075 | 3.813 | 27.701 | 1.00 | 86.34 |
| ATOM 799 | CA | ALA114 | -5.837 | 4.533 | 28.000 | 1.00 | 85.22 |
| ATOM 800 | CB | ALA114 | -6.174 | 5.904 | 28.585 | 1.00 | 84.46 |

[Table 2] (continued)

| | | | | | | | |
|---|---|---|---|---|---|---|---|
| ATOM 801 | C | ALA114 | -4.928 | 3.768 | 28.963 | 1.00 | 83.67 |
| ATOM 802 | O | ALA114 | -3.716 | 3.692 | 28.762 | 1.00 | 83.48 |
| ATOM 803 | N | MET115 | -5.528 | 3.212 | 30.012 | 1.00 | 81.79 |
| ATOM 804 | CA | MET115 | -4.802 | 2.457 | 31.023 | 1.00 | 78.70 |
| ATOM 805 | CB | MET115 | -5.776 | 2.050 | 32.135 | 1.00 | 81.16 |
| ATOM 806 | CG | MET115 | -5.148 | 1.863 | 33.503 | 1.00 | 84.52 |
| ATOM 807 | SD | MET115 | -3.978 | 0.492 | 33.553 | 1.00 | 90.44 |
| ATOM 808 | CE | MET115 | -5.060 | -0.891 | 34.119 | 1.00 | 88.49 |
| ATOM 809 | C | MET115 | -4.145 | 1.224 | 30.391 | 1.00 | 76.27 |
| ATOM 810 | O | MET115 | -3.066 | 0.809 | 30.813 | 1.00 | 74.47 |
| ATOM 811 | N | THR116 | -4.796 | 0.658 | 29.372 | 1.00 | 74.50 |
| ATOM 812 | CA | THR116 | -4.282 | -0.518 | 28.666 | 1.00 | 72.46 |
| ATOM 813 | CB | THR116 | -5.399 | -1.524 | 28.309 | 1.00 | 72.22 |
| ATOM 814 | OG1 | THR116 | -6.200 | -0.993 | 27.244 | 1.00 | 71.17 |
| ATOM 815 | CG2 | THR116 | -6.275 | -1.805 | 29.516 | 1.00 | 71.94 |
| ATOM 816 | C | THR116 | -3.621 | -0.110 | 27.356 | 1.00 | 71.75 |
| ATOM 817 | O | THR116 | -3.562 | -0.899 | 26.412 | 1.00 | 71.39 |
| ATOM 818 | N | GLY117 | -3.142 | 1.131 | 27.301 | 1.00 | 71.09 |
| ATOM 819 | CA | GLY117 | -2.477 | 1.639 | 26.110 | 1.00 | 68.62 |
| ATOM 820 | C | GLY117 | -0.961 | 1.651 | 26.260 | 1.00 | 66.70 |
| ATOM 821 | O | GLY117 | -0.384 | 0.702 | 26.798 | 1.00 | 67.20 |
| ATOM 822 | N | THR118 | -0.313 | 2.716 | 25.783 | 1.00 | 63.05 |
| ATOM 823 | CA | THR118 | 1.142 | 2.844 | 25.876 | 1.00 | 59.92 |
| ATOM 824 | CB | THR 118 | 1.796 | 3.020 | 24.502 | 1.00 | 59.06 |
| ATOM 825 | OG1 | THR118 | 1.013 | 3.926 | 23.718 | 1.00 | 57.88 |
| ATOM 826 | CG2 | THR118 | 1.917 | 1.688 | 23.794 | 1.00 | 59.21 |
| ATOM 827 | C | THR118 | 1.548 | 4.038 | 26.721 | 1.00 | 58.97 |
| ATOM 828 | O | THR118 | 0.764 | 4.971 | 26.912 | 1.00 | 58.11 |
| ATOM 829 | N | ALA119 | 2.782 | 4.001 | 27.218 | 1.00 | 56.72 |
| ATOM 830 | CA | ALA119 | 3.313 | 5.071 | 28.052 | 1.00 | 52.86 |
| ATOM 831 | CB | ALA119 | 4.807 | 4.938 | 28.177 | 1.00 | 51.30 |
| ATOM 832 | C | ALA119 | 2.972 | 6.399 | 27.421 | 1.00 | 51.58 |
| ATOM 833 | O | ALA119 | 2.456 | 7.301 | 28.080 | 1.00 | 52.70 |
| ATOM 834 | N | GLU120 | 3.260 | 6.502 | 26.131 | 1.00 | 48.02 |
| ATOM 835 | CA | GLU120 | 2.994 | 7.716 | 25.386 | 1.00 | 46.07 |
| ATOM 836 | CB | GLU120 | 3.194 | 7.471 | 23.894 | 1.00 | 49.10 |
| ATOM 837 | CG | GLU120 | 4.210 | 6.381 | 23.550 | 1.00 | 52.89 |
| ATOM 838 | CD | GLU120 | 5.630 | 6.736 | 23.945 | 1.00 | 53.64 |
| ATOM 839 | OE1 | GLU120 | 5.962 | 6.621 | 25.141 | 1.00 | 55.30 |
| ATOM 840 | OE2 | GLU120 | 6.411 | 7.139 | 23.057 | 1.00 | 52.83 |
| ATOM 841 | C | GLU120 | 1.557 | 8.140 | 25.630 | 1.00 | 44.27 |
| ATOM 842 | O | GLU120 | 1.295 | 9.257 | 26.070 | 1.00 | 44.84 |
| ATOM 843 | N | MET121 | 0.627 | 7.235 | 25.351 | 1.00 | 41.37 |
| ATOM 844 | CA | MET121 | -0.791 | 7.525 | 25.513 | 1.00 | 38.57 |
| ATOM 845 | CB | MET121 | -1.626 | 6.358 | 24.990 | 1.00 | 41.30 |
| ATOM 846 | CG | MET121 | -1.721 | 6.328 | 23.479 | 1.00 | 46.24 |
| ATOM 847 | SD | MET121 | -2.483 | 4.835 | 22.838 | 1.00 | 50.88 |
| ATOM 848 | CE | MET121 | -3.908 | 4.669 | 23.961 | 1.00 | 50.02 |
| ATOM 849 | C | MET121 | -1.190 | 7.820 | 26.937 | 1.00 | 34.60 |
| ATOM 850 | O | MET121 | -1.910 | 8.780 | 27.204 | 1.00 | 31.69 |
| ATOM 851 | N | LEU122 | -0.719 | 6.985 | 27.852 | 1.00 | 32.63 |

[Table 2] (continued)

| | | | | | | | |
|---|---|---|---|---|---|---|---|
| ATOM 852 | CA | LEU122 | -1.051 | 7.141 | 29.263 | 1.00 | 30.24 |
| ATOM 853 | CB | LEU122 | -0.256 | 6.140 | 30.108 | 1.00 | 27.33 |
| ATOM 854 | CG | LEU122 | -0.778 | 5.923 | 31.533 | 1.00 | 21.99 |
| ATOM 855 | CD1 | LEU122 | -0.279 | 4.601 | 32.031 | 1.00 | 22.53 |
| ATOM 856 | CD2 | LEU122 | -0.366 | 7.034 | 32.456 | 1.00 | 17.78 |
| ATOM 857 | C | LEU122 | -0.759 | 8.551 | 29.746 | 1.00 | 28.67 |
| ATOM 858 | O | LEU122 | -1.619 | 9.228 | 30.326 | 1.00 | 25.21 |
| ATOM 859 | N | PHE123 | 0.469 | 8.987 | 29.502 | 1.00 | 26.83 |
| ATOM 860 | CA | PHE123 | 0.871 | 10.306 | 29.929 | 1.00 | 25.29 |
| ATOM 861 | CB | PHE123 | 2.387 | 10.398 | 29.908 | 1.00 | 20.22 |
| ATOM 862 | CG | PHE123 | 3.015 | 9.772 | 31.112 | 1.00 | 15.51 |
| ATOM 863 | CD1 | PHE123 | 3.538 | 8.494 | 31.064 | 1.00 | 12.96 |
| ATOM 864 | CD2 | PHE123 | 3.028 | 10.457 | 32.328 | 1.00 | 13.35 |
| ATOM 865 | CE1 | PHE123 | 4.067 | 7.910 | 32.217 | 1.00 | 12.87 |
| ATOM 866 | CE2 | PHE123 | 3.552 | 9.879 | 33.484 | 1.00 | 9.69 |
| ATOM 867 | CZ | PHE123 | 4.072 | 8.609 | 33.432 | 1.00 | 9.56 |
| ATOM 868 | C | PHE123 | 0.202 | 11.432 | 29.157 | 1.00 | 26.20 |
| ATOM 869 | O | PHE123 | -0.102 | 12.489 | 29.722 | 1.00 | 26.61 |
| ATOM 870 | N | ASP124 | -0.053 | 11.207 | 27.875 | 1.00 | 24.47 |
| ATOM 871 | CA | ASP124 | -0.750 | 12.210 | 27.090 | 1.00 | 23.14 |
| ATOM 872 | CB | ASP124 | -1.228 | 11.614 | 25.785 | 1.00 | 24.52 |
| ATOM 873 | CG | ASP124 | -0.178 | 11.628 | 24.747 | 1.00 | 27.01 |
| ATOM 874 | OD1 | ASP124 | -0.376 | 10.955 | 23.715 | 1.00 | 26.39 |
| ATOM 875 | OD2 | ASP124 | 0.839 | 12.325 | 24.968 | 1.00 | 29.23 |
| ATOM 876 | C | ASP124 | -1.967 | 12.650 | 27.875 | 1.00 | 21.89 |
| ATOM 877 | O | ASP124 | -2.361 | 13.815 | 27.841 | 1.00 | 20.01 |
| ATOM 878 | N | TYR125 | -2.562 | 11.688 | 28.574 | 1.00 | 20.84 |
| ATOM 879 | CA | TYR125 | -3.749 | 11.943 | 29.371 | 1.00 | 20.51 |
| ATOM 880 | CB | TYR125 | -4.414 | 10.619 | 29.792 | 1.00 | 20.43 |
| ATOM 881 | CG | TYR125 | -5.796 | 10.794 | 30.394 | 1.00 | 22.84 |
| ATOM 882 | CD1 | TYR125 | -6.083 | 10.358 | 31.692 | 1.00 | 23.51 |
| ATOM 883 | CE1 | TYR125 | -7.345 | 10.584 | 32.268 | 1.00 | 31.08 |
| ATOM 884 | CD2 | TYR125 | -6.803 | 11.451 | 29.678 | 1.00 | 26.43 |
| ATOM 885 | CE2 | TYR125 | -8.064 | 11.685 | 30.232 | 1.00 | 31.61 |
| ATOM 886 | CZ | TYR125 | -8.336 | 11.255 | 31.528 | 1.00 | 34.64 |
| ATOM 887 | OH | TYR125 | -9.585 | 11.520 | 32.073 | 1.00 | 38.10 |
| ATOM 888 | C | TYR125 | -3.382 | 12.752 | 30.605 | 1.00 | 19.11 |
| ATOM 889 | O | TYR125 | -3.904 | 13.848 | 30.824 | 1.00 | 16.08 |
| ATOM 890 | N | ILE126 | -2.465 | 12.212 | 31.399 | 1.00 | 17.91 |
| ATOM 891 | CA | ILE126 | -2.049 | 12.879 | 32.615 | 1.00 | 17.82 |
| ATOM 892 | CB | ILE126 | -0.819 | 12.236 | 33.203 | 1.00 | 19.82 |
| ATOM 893 | CG2 | ILE126 | -0.489 | 12.905 | 34.538 | 1.00 | 18.77 |
| ATOM 894 | CG1 | ILE126 | -1.055 | 10.732 | 33.331 | 1.00 | 21.27 |
| ATOM 895 | CD1 | ILE126 | 0.045 | 9.984 | 34.062 | 1.00 | 23.92 |
| ATOM 896 | C | ILE126 | -1.717 | 14.313 | 32.325 | 1.00 | 18.09 |
| ATOM 897 | O | ILE126 | -1.991 | 15.205 | 33.123 | 1.00 | 16.68 |
| ATOM 898 | N | SER127 | -1.108 | 14.532 | 31.172 | 1.00 | 19.12 |
| ATOM 899 | CA | SER127 | -0.747 | 15.877 | 30.789 | 1.00 | 20.96 |
| ATOM 900 | CB | SER127 | -0.057 | 15.857 | 29.432 | 1.00 | 19.89 |
| ATOM 901 | OG | SER127 | 0.569 | 17.100 | 29.190 | 1.00 | 22.20 |
| ATOM 902 | C | SER127 | -2.011 | 16.742 | 30.746 | 1.00 | 21.92 |

[Table 2] (continued)

| | | | | | | | |
|---|---|---|---|---|---|---|---|
| ATOM 903 | O | SER127 | -2.177 | 17.658 | 31.551 | 1.00 | 20.25 |
| ATOM 904 | N | GLU128 | -2.902 | 16.431 | 29.813 | 1.00 | 23.87 |
| ATOM 905 | CA | GLU128 | -4.152 | 17.161 | 29.670 | 1.00 | 26.98 |
| ATOM 906 | CB | GLU128 | -5.111 | 16.353 | 28.802 | 1.00 | 33.10 |
| ATOM 907 | CG | GLU128 | -6.471 | 16.990 | 28.544 | 1.00 | 39.51 |
| ATOM 908 | CD | GLU128 | -7.280 | 16.175 | 27.544 | 1.00 | 44.52 |
| ATOM 909 | OE1 | GLU128 | -7.211 | 16.481 | 26.327 | 1.00 | 46.11 |
| ATOM 910 | OE2 | GLU128 | -7.963 | 15.218 | 27.980 | 1.00 | 43.93 |
| ATOM 911 | C | GLU128 | -4.797 | 17.431 | 31.020 | 1.00 | 26.55 |
| ATOM 912 | O | GLU128 | -5.177 | 18.561 | 31.334 | 1.00 | 26.16 |
| ATOM 913 | N | CYS129 | -4.929 | 16.384 | 31.820 | 1.00 | 26.36 |
| ATOM 914 | CA | CYS129 | -5.532 | 16.535 | 33.130 | 1.00 | 26.47 |
| ATOM 915 | CB | CYS129 | -5.452 | 15.219 | 33.893 | 1.00 | 28.39 |
| ATOM 916 | SG | CYS129 | -6.450 | 13.922 | 33.126 | 1.00 | 37.58 |
| ATOM 917 | C | CYS129 | -4.853 | 17.636 | 33.914 | 1.00 | 25.00 |
| ATOM 918 | O | CYS129 | -5.515 | 18.561 | 34.372 | 1.00 | 24.97 |
| ATOM 919 | N | ILE130 | -3.532 | 17.536 | 34.059 | 1.00 | 24.74 |
| ATOM 920 | CA | ILE130 | -2.763 | 18.536 | 34.793 | 1.00 | 21.55 |
| ATOM 921 | CB | ILE130 | -1.245 | 18.255 | 34.709 | 1.00 | 17.55 |
| ATOM 922 | CG2 | ILE130 | -0.458 | 19.404 | 35.304 | 1.00 | 15.00 |
| ATOM 923 | CG1 | ILE130 | -0.915 | 16.984 | 35.490 | 1.00 | 16.42 |
| ATOM 924 | CD1 | ILE130 | 0.574 | 16.713 | 35.623 | 1.00 | 18.34 |
| ATOM 925 | C | ILE130 | -3.070 | 19.910 | 34.219 | 1.00 | 23.54 |
| ATOM 926 | O | ILE130 | -3.572 | 20.780 | 34.926 | 1.00 | 21.27 |
| ATOM 927 | N | SER131 | -2.785 | 20.091 | 32.933 | 1.00 | 26.25 |
| ATOM 928 | CA | SER131 | -3.048 | 21.353 | 32.270 | 1.00 | 28.50 |
| ATOM 929 | CB | SER131 | -3.011 | 21.186 | 30.764 | 1.00 | 28.76 |
| ATOM 930 | OG | SER131 | -3.856 | 22.154 | 30.164 | 1.00 | 32.87 |
| ATOM 931 | C | SER131 | -4.417 | 21.851 | 32.661 | 1.00 | 31.48 |
| ATOM 932 | O | SER131 | -4.586 | 23.002 | 33.057 | 1.00 | 33.67 |
| ATOM 933 | N | ASP132 | -5.411 | 20.986 | 32.546 | 1.00 | 34.56 |
| ATOM 934 | CA | ASP132 | -6.753 | 21.397 | 32.908 | 1.00 | 39.04 |
| ATOM 935 | CB | ASP132 | -7.735 | 20.248 | 32.694 | 1.00 | 44.84 |
| ATOM 936 | CG | ASP132 | -9.165 | 20.650 | 32.987 | 1.00 | 50.51 |
| ATOM 937 | OD1 | ASP132 | -9.764 | 21.347 | 32.131 | 1.00 | 53.56 |
| ATOM 938 | OD2 | ASP132 | -9.674 | 20.283 | 34.078 | 1.00 | 52.37 |
| ATOM 939 | C | ASP132 | -6.790 | 21.843 | 34.376 | 1.00 | 38.23 |
| ATOM 940 | O | ASP132 | -7.160 | 22.982 | 34.677 | 1.00 | 36.81 |
| ATOM 941 | N | PHE133 | -6.394 | 20.932 | 35.270 | 1.00 | 36.88 |
| ATOM 942 | CA | PHE133 | -6.372 | 21.170 | 36.713 | 1.00 | 34.85 |
| ATOM 943 | CB | PHE133 | -5.604 | 20.060 | 37.433 | 1.00 | 33.59 |
| ATOM 944 | CG | PHE133 | -5.343 | 20.362 | 38.878 | 1.00 | 34.77 |
| ATOM 945 | CD1 | PHE133 | -6.396 | 20.547 | 39.760 | 1.00 | 35.58 |
| ATOM 946 | CD2 | PHE133 | -4.043 | 20.523 | 39.348 | 1.00 | 37.81 |
| ATOM 947 | CE1 | PHE133 | -6.159 | 20.896 | 41.091 | 1.00 | 37.66 |
| ATOM 948 | CE2 | PHE133 | -3.792 | 20.872 | 40.678 | 1.00 | 38.00 |
| ATOM 949 | CZ | PHE133 | -4.850 | 21.059 | 41.548 | 1.00 | 38.85 |
| ATOM 950 | C | PHE133 | -5.755 | 22.503 | 37.094 | 1.00 | 34.28 |
| ATOM 951 | O | PHE133 | -6.274 | 23.226 | 37.947 | 1.00 | 33.97 |
| ATOM 952 | N | LEU134 | -4.622 | 22.813 | 36.482 | 1.00 | 33.97 |
| ATOM 953 | CA | LEU134 | -3.958 | 24.070 | 36.766 | 1.00 | 31.79 |

[Table 2] (continued)

| | | | | | | | |
|---|---|---|---|---|---|---|---|
| ATOM 954 | CB | LEU134 | -2.590 | 24.109 | 36.089 | 1.00 | 24.12 |
| ATOM 955 | CG | LEU134 | -1.618 | 23.026 | 36.545 | 1.00 | 16.64 |
| ATOM 956 | CD1 | LEU134 | -0.368 | 23.101 | 35.705 | 1.00 | 15.98 |
| ATOM 957 | CD2 | LEU134 | -1.305 | 23.184 | 38.014 | 1.00 | 10.77 |
| ATOM 958 | C | LEU134 | -4.855 | 25.176 | 36.234 | 1.00 | 34.44 |
| ATOM 959 | O | LEU134 | -5.111 | 26.163 | 36.920 | 1.00 | 34.41 |
| ATOM 960 | N | ASP135 | -5.365 | 24.999 | 35.022 | 1.00 | 37.26 |
| ATOM 961 | CA | ASP135 | -6.230 | 26.014 | 34.454 | 1.00 | 42.65 |
| ATOM 962 | CB | ASP135 | -6.815 | 25.565 | 33.121 | 1.00 | 46.76 |
| ATOM 963 | CG | ASP135 | -7.707 | 26.629 | 32.509 | 1.00 | 52.18 |
| ATOM 964 | OD1 | ASP135 | -8.659 | 26.271 | 31.772 | 1.00 | 53.75 |
| ATOM 965 | OD2 | ASP135 | -7.443 | 27.829 | 32.772 | 1.00 | 52.70 |
| ATOM 966 | C | ASP135 | -7.386 | 26.381 | 35.383 | 1.00 | 43.96 |
| ATOM 967 | O | ASP135 | -7.643 | 27.563 | 35.619 | 1.00 | 44.98 |
| ATOM 968 | N | LYS136 | -8.084 | 25.368 | 35.894 | 1.00 | 44.30 |
| ATOM 969 | CA | LYS136 | -9.225 | 25.578 | 36.780 | 1.00 | 44.56 |
| ATOM 970 | CB | LYS136 | -9.889 | 24.237 | 37.124 | 1.00 | 46.76 |
| ATOM 971 | CG | LYS136 | -11.195 | 24.350 | 37.941 | 1.00 | 52.67 |
| ATOM 972 | CD | LYS136 | -11.910 | 22.981 | 38.128 | 1.00 | 55.98 |
| ATOM 973 | CE | LYS136 | -13.367 | 23.120 | 38.628 | 1.00 | 55.25 |
| ATOM 974 | NZ | LYS136 | -14.106 | 21.817 | 38.719 | 1.00 | 51.28 |
| ATOM 975 | C | LYS136 | -8.862 | 26.306 | 38. 069 | 1.00 | 44.85 |
| ATOM 976 | O | LYS136 | -9.730 | 26.894 | 38.717 | 1.00 | 45.87 |
| ATOM 977 | N | HIS137 | -7.586 | 26.273 | 38.444 | 1.00 | 44.25 |
| ATOM 978 | CA | HIS137 | -7.149 | 26.937 | 39.670 | 1.00 | 43.21 |
| ATOM 979 | CB | HIS137 | -6.434 | 25.937 | 40.585 | 1.00 | 44.13 |
| ATOM 980 | CG | HIS137 | -7.344 | 24.915 | 41.199 | 1.00 | 45.24 |
| ATOM 981 | CD2 | HIS137 | -7.676 | 24.680 | 42.492 | 1.00 | 45.35 |
| ATOM 982 | ND1 | HIS137 | -8.042 | 23.991 | 40.452 | 1.00 | 45.45 |
| ATOM 983 | CE1 | HIS137 | -8.764 | 23.231 | 41.257 | 1.00 | 45.40 |
| ATOM 984 | NE2 | HIS137 | -8.560 | 23.629 | 42.500 | 1.00 | 44.34 |
| ATOM 985 | C | HIS137 | -6.242 | 28.132 | 39.400 | 1.00 | 41.96 |
| ATOM 986 | O | HIS137 | -5.592 | 28.649 | 40.307 | 1.00 | 40.24 |
| ATOM 987 | N | GLN138 | -6.217 | 28.577 | 38.151 | 1.00 | 42.87 |
| ATOM 988 | CA | GLN138 | -5.390 | 29.706 | 37.766 | 1.00 | 44.93 |
| ATOM 989 | CB | GLN138 | -5.949 | 30.993 | 38.373 | 1.00 | 47.58 |
| ATOM 990 | CG | GLN138 | -7.258 | 31.448 | 37.749 | 1.00 | 51.96 |
| ATOM 991 | CD | GLN138 | -7.416 | 32.966 | 37.766 | 1.00 | 55.20 |
| ATOM 992 | OE1 | GLN138 | -6.680 | 33.698 | 37.088 | 1.00 | 56.05 |
| ATOM 993 | NE2 | GLN138 | -8.375 | 33.445 | 38.546 | 1.00 | 55.44 |
| ATOM 994 | C | GLN138 | -3.921 | 29.537 | 38.162 | 1.00 | 44.67 |
| ATOM 995 | O | GLN138 | -3.316 | 30.437 | 38.747 | 1.00 | 45.78 |
| ATOM 996 | N | MET139 | -3.350 | 28.383 | 37.836 | 1.00 | 41.86 |
| ATOM 997 | CA | MET139 | -1.951 | 28.109 | 38.138 | 1.00 | 38.60 |
| ATOM 998 | CB | MET139 | -1.846 | 27.062 | 39.236 | 1.00 | 39.19 |
| ATOM 999 | CG | MET139 | -2.048 | 27.660 | 40.604 | 1.00 | 41.24 |
| ATOM 1000 | SD | MET139 | -0.859 | 28.992 | 40.852 | 1.00 | 47.65 |
| ATOM 1001 | CE | MET139 | 0.308 | 28.217 | 42.007 | 1.00 | 44.32 |
| ATOM 1002 | C | MET139 | -1.232 | 27.653 | 36.881 | 1.00 | 36.60 |
| ATOM 1003 | O | MET139 | -0.316 | 26.823 | 36.910 | 1.00 | 35.29 |
| ATOM 1004 | N | LYS140 | -1.659 | 28.237 | 35.771 | 1.00 | 34.23 |

[Table 2] (continued)

| | | | | | | | |
|---|---|---|---|---|---|---|---|
| ATOM 1005 | CA | LYS140 | -1.101 | 27.921 | 34.477 | 1.00 | 32.15 |
| ATOM 1006 | CB | LYS140 | -2.198 | 28.062 | 33.417 | 1.00 | 31.04 |
| ATOM 1007 | CG | LYS140 | -1.970 | 27.293 | 32.116 | 1.00 | 31.48 |
| ATOM 1008 | CD | LYS140 | -2.184 | 25.780 | 32.275 | 1.00 | 32.43 |
| ATOM 1009 | CE | LYS140 | -2.112 | 25.015 | 30.925 | 1.00 | 30.89 |
| ATOM 1010 | NZ | LYS140 | -0.811 | 25.130 | 30.168 | 1.00 | 29.56 |
| ATOM 1011 | C | LYS140 | 0.085 | 28.834 | 34.161 | 1.00 | 31.02 |
| ATOM 1012 | O | LYS140 | 0.047 | 30.045 | 34.412 | 1.00 | 29.99 |
| ATOM 1013 | N | HIS141 | 1.143 | 28.228 | 33.627 | 1.00 | 31.35 |
| ATOM 1014 | CA | HIS141 | 2.353 | 28.940 | 33.244 | 1.00 | 30.03 |
| ATOM 1015 | CB | HIS141 | 1.989 | 30.145 | 32.385 | 1.00 | 30.05 |
| ATOM 1016 | CG | HIS141 | 1.001 | 29.836 | 31.305 | 1.00 | 31.15 |
| ATOM 1017 | CD2 | HIS141 | -0.132 | 30.473 | 30.927 | 1.00 | 30.91 |
| ATOM 1018 | ND1 | HIS141 | 1.148 | 28.769 | 30.448 | 1.00 | 33.49 |
| ATOM 1019 | CE1 | HIS141 | 0.147 | 28.763 | 29.584 | 1.00 | 35.03 |
| ATOM 1020 | NE2 | HIS141 | -0.643 | 29.787 | 29.853 | 1.00 | 32.67 |
| ATOM 1021 | C | HIS141 | 3.138 | 29.396 | 34.460 | 1.00 | 29.17 |
| ATOM 1022 | O | HIS141 | 4.211 | 29.983 | 34.341 | 1.00 | 28.17 |
| ATOM 1023 | N | LYS142 | 2.601 | 29.108 | 35.635 | 1.00 | 28.81 |
| ATOM 1024 | CA | LYS142 | 3.248 | 29.505 | 36.869 | 1.00 | 29.17 |
| ATOM 1025 | CB | LYS142 | 2.317 | 29.240 | 38.065 | 1.00 | 33.65 |
| ATOM 1026 | CG | LYS142 | 0.986 | 30.042 | 38.072 | 1.00 | 39.35 |
| ATOM 1027 | CD | LYS142 | 1.194 | 31.561 | 38.214 | 1.00 | 42.74 |
| ATOM 1028 | CE | LYS142 | -0.122 | 32.360 | 38.170 | 1.00 | 45.49 |
| ATOM 1029 | NZ | LYS142 | 0.110 | 33.844 | 38.325 | 1.00 | 46.19 |
| ATOM 1030 | C | LYS142 | 4.575 | 28.785 | 37.075 | 1.00 | 26.49 |
| ATOM 1031 | O | LYS142 | 5.340 | 29.138 | 37.966 | 1.00 | 26.10 |
| ATOM 1032 | N | LYS143 | 4.862 | 27.784 | 36.254 | 1.00 | 24.58 |
| ATOM 1033 | CA | LYS143 | 6.106 | 27.042 | 36.416 | 1.00 | 22.67 |
| ATOM 1034 | CB | LYS143 | 7.258 | 27.847 | 35.836 | 1.00 | 21.51 |
| ATOM 1035 | CG | LYS143 | 8.533 | 27.071 | 35.737 | 1.00 | 22.59 |
| ATOM 1036 | CD | LYS143 | 9.319 | 27.510 | 34.516 | 1.00 | 25.81 |
| ATOM 1037 | CE | LYS143 | 10.455 | 26.542 | 34.240 | 1.00 | 28.01 |
| ATOM 1038 | NZ | LYS143 | 11.140 | 26.828 | 32.959 | 1.00 | 27.25 |
| ATOM 1039 | C | LYS143 | 6.383 | 26.732 | 37.896 | 1.00 | 22.14 |
| ATOM 1040 | O | LYS143 | 7.133 | 27.459 | 38.556 | 1.00 | 21.99 |
| ATOM 1041 | N | LEU144 | 5.766 | 25.655 | 38.401 | 1.00 | 20.81 |
| ATOM 1042 | CA | LEU144 | 5.910 | 25.214 | 39.797 | 1.00 | 16.90 |
| ATOM 1043 | CB | LEU144 | 4.577 | 25.351 | 40.567 | 1.00 | 16.78 |
| ATOM 1044 | CG | LEU144 | 3.208 | 24.956 | 39.983 | 1.00 | 18.43 |
| ATOM 1045 | CD1 | LEU144 | 2.148 | 24.915 | 41.074 | 1.00 | 17.60 |
| ATOM1046 | CD2 | LEU144 | 2.795 | 25.960 | 38.929 | 1.00 | 19.20 |
| ATOM 1047 | C | LEU144 | 6.432 | 23.781 | 39.933 | 1.00 | 15.80 |
| ATOM 1048 | O | LEU144 | 6.265 | 22.958 | 39.032 | 1.00 | 12.24 |
| ATOM 1049 | N | PRO145 | 7.078 | 23.478 | 41.076 | 1.00 | 16.26 |
| ATOM 1050 | CD | PRO145 | 7.227 | 24.446 | 42.172 | 1.00 | 15.64 |
| ATOM 1051 | CA | PRO145 | 7.678 | 22.196 | 41.467 | 1.00 | 14.17 |
| ATOM 1052 | CB | PRO145 | 8.079 | 22.427 | 42.923 | 1.00 | 18.10 |
| ATOM 1053 | CG | PRO145 | 8.378 | 23.860 | 42.963 | 1.00 | 17.14 |
| ATOM 1054 | C | PRO145 | 6.707 | 21.050 | 41.357 | 1.00 | 12.75 |
| ATOM 1055 | O | PRO145 | 5.580 | 21.141 | 41.852 | 1.00 | 12.27 |

[Table 2] (continued)

| | | | | | | | |
|---|---|---|---|---|---|---|---|
| ATOM 1056 | N | LEU146 | 7.160 | 19.957 | 40.758 | 1.00 | 10.29 |
| ATOM 1057 | CA | LEU146 | 6.290 | 18.804 | 40.560 | 1.00 | 11.21 |
| ATOM 1058 | CB | LEU146 | 6.156 | 18.539 | 39.075 | 1.00 | 7.24 |
| ATOM 1059 | CG | LEU146 | 5.160 | 17.439 | 38.824 | 1.00 | 3.01 |
| ATOM 1060 | CD1 | LEU146 | 3.817 | 17.832 | 39.389 | 1.00 | 1.00 |
| ATOM 1061 | CD2 | LEU146 | 5.083 | 17.215 | 37.342 | 1.00 | 3.06 |
| ATOM 1062 | C | LEU146 | 6.696 | 17.502 | 41.233 | 1.00 | 12.36 |
| ATOM 1063 | O | LEU146 | 7.629 | 16.851 | 40.790 | 1.00 | 15.11 |
| ATOM 1064 | N | GLY147 | 5.972 | 17.086 | 42.262 | 1.00 | 14.72 |
| ATOM 1065 | CA | GLY147 | 6.333 | 15.851 | 42.937 | 1.00 | 17.81 |
| ATOM 1066 | C | GLY147 | 5.716 | 14.586 | 42.371 | 1.00 | 18.51 |
| ATOM 1067 | O | GLY147 | 4.689 | 14.644 | 41.704 | 1.00 | 20.85 |
| ATOM 1068 | N | PHE148 | 6.342 | 13.440 | 42.631 | 1.00 | 19.28 |
| ATOM 1069 | CA | PHE148 | 5.825 | 12.167 | 42.142 | 1.00 | 20.55 |
| ATOM 1070 | CB | PHE148 | 6.707 | 11.635 | 41.023 | 1.00 | 16.36 |
| ATOM 1071 | CG | PHE148 | 6.593 | 12.409 | 39.759 | 1.00 | 17.72 |
| ATOM 1072 | CD1 | PHE148 | 6.792 | 13.779 | 39.753 | 1.00 | 17.99 |
| ATOM 1073 | CD2 | PHE148 | 6.298 | 11.769 | 38.560 | 1.00 | 21.10 |
| ATOM 1074 | CE1 | PHE148 | 6.695 | 14.509 | 38.570 | 1.00 | 22.37 |
| ATOM 1075 | CE2 | PHE148 | 6.198 | 12.494 | 37.362 | 1.00 | 22.82 |
| ATOM 1076 | CZ | PHE148 | 6.398 | 13.864 | 37.366 | 1.00 | 21.67 |
| ATOM 1077 | C | PHE148 | 5.712 | 11.104 | 43.222 | 1.00 | 22.75 |
| ATOM 1078 | O | PHE148 | 6.691 | 10.783 | 43.885 | 1.00 | 24.66 |
| ATOM 1079 | N | THR149 | 4.513 | 10.562 | 43.403 | 1.00 | 24.45 |
| ATOM 1080 | CA | THR149 | 4.312 | 9.514 | 44.387 | 1.00 | 24.75 |
| ATOM 1081 | CB | THR149 | 3.365 | 9.917 | 45.497 | 1.00 | 23.76 |
| ATOM 1082 | OG1 | THR149 | 2.757 | 11.175 | 45.192 | 1.00 | 25.51 |
| ATOM 1083 | CG2 | THR149 | 4.107 | 9.989 | 46.786 | 1.00 | 22.63 |
| ATOM 1084 | C | THR149 | 3.705 | 8.306 | 43.715 | 1.00 | 27.38 |
| ATOM 1085 | O | THR149 | 3.093 | 8.405 | 42.647 | 1.00 | 24.58 |
| ATOM 1086 | N | PHE150 | 3.857 | 7.160 | 44.361 | 1.00 | 30.07 |
| ATOM 1087 | CA | PHE150 | 3.327 | 5.936 | 43.811 | 1.00 | 32.54 |
| ATOM 1088 | CB | PHE150 | 4.455 | 5.120 | 43.215 | 1.00 | 29.97 |
| ATOM 1089 | CG | PHE150 | 5.172 | 5.820 | 42.119 | 1.00 | 27.55 |
| ATOM 1090 | CD1 | PHE150 | 6.134 | 6.770 | 42.397 | 1.00 | 27.41 |
| ATOM 1091 | CD2 | PHE150 | 4.850 | 5.561 | 40.798 | 1.00 | 27.56 |
| ATOM 1092 | CE1 | PHE150 | 6.770 | 7.447 | 41.366 | 1.00 | 28.61 |
| ATOM 1093 | CE2 | PHE150 | 5.481 | 6.231 | 39.762 | 1.00 | 26.86 |
| ATOM 1094 | CZ | PHE150 | 6.437 | 7.177 | 40.045 | 1.00 | 27.37 |
| ATOM 1095 | C | PHE150 | 2.561 | 5.093 | 44.808 | 1.00 | 35.78 |
| ATOM 1096 | O | PHE150 | 3.095 | 4.695 | 45.845 | 1.00 | 36.93 |
| ATOM 1097 | N | SER151 | 1.305 | 4.813 | 44.467 | 1.00 | 38.60 |
| ATOM 1098 | CA | SER151 | 0.420 | 4.006 | 45.295 | 1.00 | 40.51 |
| ATOM 1099 | CB | SER151 | -0.830 | 4.802 | 45.641 | 1.00 | 41.51 |
| ATOM 1100 | OG | SER151 | -1.507 | 5.159 | 44.453 | 1.00 | 47.40 |
| ATOM 1101 | C | SER151 | 0.038 | 2.736 | 44.533 | 1.00 | 41.75 |
| ATOM 1102 | O | SER151 | 0.069 | 2.696 | 43.301 | 1.00 | 40.78 |
| ATOM 1103 | N | PHE152 | -0.336 | 1.704 | 45.278 | 1.00 | 43.86 |
| ATOM 1104 | CA | PHE152 | -0.684 | 0.421 | 44.687 | 1.00 | 45.76 |
| ATOM 1105 | CB | PHE152 | 0.465 | -0.557 | 44.965 | 1.00 | 51.67 |
| ATOM 1106 | CG | PHE152 | 0.429 | -1.808 | 44.133 | 1.00 | 57.82 |

[Table 2] (continued)

| | | | | | | | |
|---|---|---|---|---|---|---|---|
| ATOM 1107 | CD1 | PHE152 | 0.597 | -1.749 | 42.751 | 1.00 | 59.39 |
| ATOM 1108 | CD2 | PHE152 | 0.256 | -3.056 | 44.739 | 1.00 | 60.37 |
| ATOM 1109 | CE1 | PHE152 | 0.598 | -2.915 | 41.979 | 1.00 | 61.23 |
| ATOM 1110 | CE2 | PHE152 | 0.254 | -4.232 | 43.978 | 1.00 | 61.76 |
| ATOM 1111 | CZ | PHE152 | 0.426 | -4.161 | 42.593 | 1.00 | 61.46 |
| ATOM 1112 | C | PHE152 | -2.007 | -0.134 | 45.238 | 1.00 | 43.74 |
| ATOM 1113 | O | PHE152 | -2.137 | -0.382 | 46.437 | 1.00 | 43.01 |
| ATOM 1114 | N | PRO153 | -3.005 | -0.322 | 44.359 | 1.00 | 40.65 |
| ATOM 1115 | CD | PRO153 | -2.993 | 0.179 | 42.979 | 1.00 | 39.35 |
| ATOM 1116 | CA | PRO153 | -4.330 | -0.844 | 44.685 | 1.00 | 38.88 |
| ATOM 1117 | CB | PRO153 | -5.045 | -0.803 | 43.352 | 1.00 | 36.16 |
| ATOM 1118 | CG | PRO153 | -4.454 | 0.359 | 42.711 | 1.00 | 37.38 |
| ATOM 1119 | C | PRO153 | -4.235 | -2.255 | 45.192 | 1.00 | 41.30 |
| ATOM 1120 | O | PRO153 | -3.481 | -3.057 | 44.657 | 1.00 | 42.17 |
| ATOM 1121 | N | VAL154 | -5.013 | -2.565 | 46.215 | 1.00 | 45.30 |
| ATOM 1122 | CA | VAL154 | -5.016 | -3.905 | 46.767 | 1.00 | 49.50 |
| ATOM 1123 | CB | VAL154 | -4.124 | -3.989 | 47.990 | 1.00 | 45.75 |
| ATOM 1124 | CG1 | VAL154 | -4.297 | -5.331 | 48.638 | 1.00 | 45.79 |
| ATOM 1125 | CG2 | VAL154 | -2.684 | -3.772 | 47.594 | 1.00 | 44.88 |
| ATOM 1126 | C | VAL154 | -6.432 | -4.268 | 47.181 | 1.00 | 55.51 |
| ATOM 1127 | O | VAL154 | -6.963 | -3.683 | 48.119 | 1.00 | 58.30 |
| ATOM 1128 | N | ARG155 | -7.042 | -5.232 | 46.495 | 1.00 | 61.06 |
| ATOM 1129 | CA | ARG155 | -8.413 | -5.643 | 46.812 | 1.00 | 67.71 |
| ATOM 1130 | CB | ARG 155 | -8.812 | -6.847 | 45.956 | 1.00 | 71.43 |
| ATOM 1131 | CG | ARG155 | -9.033 | -6.501 | 44.501 | 1.00 | 76.11 |
| ATOM 1132 | CD | ARG155 | -9.094 | -7.736 | 43.621 | 1.00 | 78.73 |
| ATOM 1133 | NE | ARG155 | -9.292 | -7.352 | 42.226 | 1.00 | 81.59 |
| ATOM 1134 | CZ | ARG155 | -9.138 | -8.168 | 41.190 | 1.00 | 82.83 |
| ATOM 1135 | NH1 | ARG155 | -8.778 | -9.432 | 41.386 | 1.00 | 83.55 |
| ATOM 1136 | NH2 | ARG155 | -9.340 | -7.717 | 39. 956 | 1.00 | 82.01 |
| ATOM 1137 | C | ARG155 | -8.639 | -5.965 | 48.291 | 1.00 | 70.15 |
| ATOM 1138 | O | ARG155 | -7.689 | -6.255 | 49.022 | 1.00 | 71.24 |
| ATOM 1139 | N | HIS156 | -9.903 | -5.923 | 48.720 | 1.00 | 71.23 |
| ATOM 1140 | CA | HIS156 | -10.265 | -6.184 | 50.117 | 1.00 | 72.30 |
| ATOM 1141 | CB | HIS156 | -11.724 | -5.769 | 50.365 | 1.00 | 73.82 |
| ATOM 1142 | CG | HIS156 | -12.049 | -5.506 | 51.808 | 1.00 | 76.32 |
| ATOM 1143 | CD2 | HIS156 | -11.335 | -5.722 | 52.941 | 1.00 | 76.70 |
| ATOM 1144 | ND1 | HIS156 | -13.243 | -4.944 | 52.211 | 1.00 | 76.54 |
| ATOM 1145 | CE1 | HIS156 | -13.251 | -4.823 | 53.527 | 1.00 | 76.16 |
| ATOM 1146 | NE2 | HIS156 | -12.106 | -5.288 | 53.994 | 1.00 | 77.55 |
| ATOM 1147 | C | HIS156 | -10.063 | -7.645 | 50.522 | 1.00 | 72.42 |
| ATOM 1148 | O | HIS156 | -9.196 | -7.957 | 51.345 | 1.00 | 71.15 |
| ATOM 1149 | N | ASN180 | 11.816 | 6.551 | 32.482 | 1.00 | 43.22 |
| ATOM 1150 | CA | ASN180 | 11.492 | 7.278 | 33.706 | 1.00 | 42.73 |
| ATOM 1151 | CB | ASN180 | 12.677 | 8.168 | 34.155 | 1.00 | 46.67 |
| ATOM 1152 | CG | ASN180 | 13.189 | 9.094 | 33.052 | 1.00 | 50.13 |
| ATOM 1153 | OD1 | ASN180 | 14.152 | 8.777 | 32.336 | 1.00 | 51.24 |
| ATOM 1154 | ND2 | ASN180 | 12.547 | 10.250 | 32.915 | 1.00 | 51.73 |
| ATOM 1155 | C | ASN180 | 10.228 | 8.110 | 33.523 | 1.00 | 38.44 |
| ATOM 1156 | O | ASN180 | 9.941 | 8.600 | 32.431 | 1.00 | 36.40 |
| ATOM 1157 | N | VAL181 | 9.473 | 8.257 | 34.603 | 1.00 | 34.02 |

[Table 2] (continued)

| | | | | | | | |
|---|---|---|---|---|---|---|---|
| ATOM 1158 | CA | VAL181 | 8.218 | 8.995 | 34.577 | 1.00 | 31.37 |
| ATOM 1159 | CB | VAL181 | 7.498 | 8.874 | 35.957 | 1.00 | 34.84 |
| ATOM 1160 | CG1 | VAL181 | 6.091 | 9.484 | 35.909 | 1.00 | 32.59 |
| ATOM 1161 | CG2 | VAL181 | 7.414 | 7.405 | 36.353 | 1.00 | 38.00 |
| ATOM 1162 | C | VAL181 | 8.426 | 10.458 | 34.221 | 1.00 | 26.36 |
| ATOM 1163 | O | VAL181 | 7.882 | 10.964 | 33.237 | 1.00 | 23.28 |
| ATOM 1164 | N | VAL182 | 9.228 | 11.131 | 35.030 | 1.00 | 23.56 |
| ATOM 1165 | CA | VAL182 | 9.518 | 12.538 | 34.826 | 1.00 | 18.23 |
| ATOM 1166 | CB | VAL182 | 10.702 | 12.958 | 35.716 | 1.00 | 14.26 |
| ATOM 1167 | CG1 | VAL182 | 11.905 | 12.084 | 35.426 | 1.00 | 14.73 |
| ATOM 1168 | CG2 | VAL182 | 11.001 | 14.403 | 35.508 | 1.00 | 11.08 |
| ATOM 1169 | C | VAL182 | 9.773 | 12.882 | 33.352 | 1.00 | 15.36 |
| ATOM 1170 | O | VAL182 | 9.330 | 13.924 | 32.875 | 1.00 | 15.32 |
| ATOM 1171 | N | GLY183 | 10.467 | 12.009 | 32.632 | 1.00 | 13.34 |
| ATOM 1172 | CA | GLY183 | 10.713 | 12.267 | 31.228 | 1.00 | 12.56 |
| ATOM 1173 | C | GLY183 | 9.458 | 12.098 | 30.382 | 1.00 | 13.06 |
| ATOM 1174 | O | GLY183 | 9.104 | 12.978 | 29.601 | 1.00 | 12.05 |
| ATOM 1175 | N | LEU184 | 8.772 | 10.971 | 30.540 | 1.00 | 15.78 |
| ATOM 1176 | CA | LEU184 | 7.549 | 10.708 | 29.777 | 1.00 | 15.21 |
| ATOM 1177 | CB | LEU184 | 6.858 | 9.435 | 30.295 | 1.00 | 16.78 |
| ATOM 1178 | CG | LEU184 | 7.613 | 8.108 | 30.075 | 1.00 | 15.45 |
| ATOM 1179 | CD1 | LEU184 | 7.037 | 7.023 | 30.951 | 1.00 | 10.71 |
| ATOM 1180 | CD2 | LEU184 | 7.548 | 7.708 | 28.608 | 1.00 | 16.62 |
| ATOM 1181 | C | LEU184 | 6.601 | 11.894 | 29.863 | 1.00 | 13.07 |
| ATOM 1182 | O | LEU184 | 6.041 | 12.311 | 28.855 | 1.00 | 13.90 |
| ATOM 1183 | N | LEU185 | 6.430 | 12.436 | 31.064 | 1.00 | 11.99 |
| ATOM 1184 | CA | LEU185 | 5.571 | 13.600 | 31.250 | 1.00 | 12.43 |
| ATOM 1185 | CB | LEU185 | 5.524 | 13.997 | 32.729 | 1.00 | 13.27 |
| ATOM 1186 | CG | LEU185 | 4.630 | 15.191 | 33.080 | 1.00 | 11.52 |
| ATOM 1187 | CD1 | LEU185 | 3.256 | 14.936 | 32.515 | 1.00 | 10.60 |
| ATOM 1188 | CD2 | LEU185 | 4.553 | 15.395 | 34.600 | 1.00 | 12.16 |
| ATOM 1189 | C | LEU185 | 6.077 | 14.788 | 30.419 | 1.00 | 12.48 |
| ATOM 1190 | O | LEU185 | 5.289 | 15.488 | 29.784 | 1.00 | 9.22 |
| ATOM 1191 | N | ARG186 | 7.388 | 15.020 | 30.428 | 1.00 | 13.24 |
| ATOM 1192 | CA | ARG186 | 7.946 | 16.123 | 29.661 | 1.00 | 14.83 |
| ATOM 1193 | CB | ARG186 | 9.478 | 16.135 | 29.727 | 1.00 | 14.69 |
| ATOM 1194 | CG | ARG186 | 10.112 | 17.274 | 30.526 | 1.00 | 18.47 |
| ATOM 1195 | CD | ARG186 | 11.633 | 17.063 | 30.663 | 1.00 | 25.71 |
| ATOM 1196 | NE | ARG186 | 12.325 | 18.069 | 31.484 | 1.00 | 37.62 |
| ATOM 1197 | CZ | ARG186 | 12.048 | 18.357 | 32.764 | 1.00 | 42.54 |
| ATOM 1198 | NH1 | ARG186 | 11.070 | 17.721 | 33.407 | 1.00 | 43.86 |
| ATOM 1199 | NH2 | ARG186 | 12.762 | 19.277 | 33.414 | 1.00 | 39.97 |
| ATOM 1200 | C | ARG186 | 7.510 | 15.968 | 28.220 | 1.00 | 16.38 |
| ATOM 1201 | O | ARG186 | 6.857 | 16.851 | 27.673 | 1.00 | 17.00 |
| ATOM 1202 | N | ASP187 | 7.850 | 14.832 | 27.616 | 1.00 | 19.34 |
| ATOM 1203 | CA | ASP187 | 7.519 | 14.579 | 26.214 | 1.00 | 24.04 |
| ATOM 1204 | CB | ASP187 | 7.799 | 13.123 | 25.822 | 1.00 | 30.35 |
| ATOM 1205 | CG | ASP187 | 9.226 | 12.696 | 26.123 | 1.00 | 37.33 |
| ATOM 1206 | OD1 | ASP187 | 9.479 | 12.216 | 27.251 | 1.00 | 40.99 |
| ATOM 1207 | OD2 | ASP187 | 10.096 | 12.845 | 25.234 | 1.00 | 40.65 |
| ATOM 1208 | C | ASP187 | 6.069 | 14.889 | 25.912 | 1.00 | 23.78 |

[Table 2] (continued)

| | | | | | | | |
|---|---|---|---|---|---|---|---|
| ATOM 1209 | O | ASP187 | 5.756 | 15.541 | 24.909 | 1.00 | 25.37 |
| ATOM 1210 | N | ALA188 | 5.185 | 14.413 | 26.780 | 1.00 | 20.98 |
| ATOM 1211 | CA | ALA188 | 3.761 | 14.634 | 26.603 | 1.00 | 17.11 |
| ATOM 1212 | CB | ALA188 | 2.996 | 13.943 | 27.722 | 1.00 | 19.70 |
| ATOM 1213 | C | ALA188 | 3.475 | 16.130 | 26.600 | 1.00 | 14.48 |
| ATOM 1214 | O | ALA188 | 2.911 | 16.660 | 25.646 | 1.00 | 11.69 |
| ATOM 1215 | N | ILE189 | 3.873 | 16.801 | 27.677 | 1.00 | 13.32 |
| ATOM 1216 | CA | ILE189 | 3.682 | 18.239 | 27.817 | 1.00 | 13.84 |
| ATOM 1217 | CB | ILE189 | 4.422 | 18.754 | 29.056 | 1.00 | 12.34 |
| ATOM 1218 | CG2 | ILE189 | 4.368 | 20.266 | 29.118 | 1.00 | 13.98 |
| ATOM 1219 | CG1 | ILE189 | 3.776 | 18.153 | 30.302 | 1.00 | 14.10 |
| ATOM 1220 | CD1 | ILE189 | 4.455 | 18.530 | 31.595 | 1.00 | 14.04 |
| ATOM 1221 | C | ILE189 | 4.223 | 18.928 | 26.575 | 1.00 | 15.60 |
| ATOM 1222 | O | ILE189 | 3.634 | 19.888 | 26.058 | 1.00 | 14.87 |
| ATOM 1223 | N | LYS190 | 5.351 | 18.408 | 26.103 | 1.00 | 16.13 |
| ATOM 1224 | CA | LYS190 | 6.010 | 18.913 | 24.918 | 1.00 | 16.34 |
| ATOM 1225 | CB | LYS190 | 7.361 | 18.211 | 24.737 | 1.00 | 18.43 |
| ATOM 1226 | CG | LYS190 | 8.503 | 19.081 | 24.175 | 1.00 | 24.32 |
| ATOM 1227 | CD | LYS190 | 8.539 | 19.154 | 22.631 | 1.00 | 28.76 |
| ATOM 1228 | CE | LYS190 | 9.830 | 19.841 | 22.125 | 1.00 | 30.07 |
| ATOM 1229 | NZ | LYS190 | 10.060 | 19.788 | 20.642 | 1.00 | 27.01 |
| ATOM 1230 | C | LYS190 | 5.101 | 18.652 | 23.718 | 1.00 | 16.41 |
| ATOM 1231 | O | LYS190 | 4.786 | 19.575 | 22.981 | 1.00 | 17.80 |
| ATOM 1232 | N | ARG191 | 4.656 | 17.413 | 23.529 | 1.00 | 14.92 |
| ATOM 1233 | CA | ARG191 | 3.798 | 17.107 | 22.386 | 1.00 | 15.62 |
| ATOM 1234 | CB | ARG191 | 3.241 | 15.684 | 22.491 | 1.00 | 19.10 |
| ATOM 1235 | CG | ARG191 | 4.071 | 14.622 | 21.775 | 1.00 | 20.57 |
| ATOM 1236 | CD | ARG191 | 3.634 | 13.221 | 22.156 | 1.00 | 19.26 |
| ATOM 1237 | NE | ARG191 | 3.950 | 12.925 | 23.547 | 1.00 | 23.45 |
| ATOM 1238 | CZ | ARG191 | 3.732 | 11.747 | 24.119 | 1.00 | 28.59 |
| ATOM 1239 | NH1 | ARG191 | 3.194 | 10.767 | 23.406 | 1.00 | 32.19 |
| ATOM 1240 | NH2 | ARG191 | 4.062 | 11.537 | 25.391 | 1.00 | 29.84 |
| ATOM 1241 | C | ARG191 | 2.652 | 18.086 | 22.207 | 1.00 | 15.44 |
| ATOM 1242 | O | ARG191 | 2.383 | 18.513 | 21.098 | 1.00 | 15.17 |
| ATOM 1243 | N | ARG192 | 1.980 | 18.441 | 23.295 | 1.00 | 17.09 |
| ATOM 1244 | CA | ARG192 | 0.853 | 19.372 | 23.253 | 1.00 | 19.02 |
| ATOM 1245 | CB | ARG192 | 0.588 | 19.885 | 24.647 | 1.00 | 17.94 |
| ATOM 1246 | CG | ARG192 | 0.579 | 18.785 | 25.635 | 1.00 | 20.35 |
| ATOM 1247 | CD | ARG192 | -0.812 | 18.328 | 25.855 | 1.00 | 22.03 |
| ATOM 1248 | NE | ARG192 | -1.565 | 19.332 | 26.586 | 1.00 | 27.30 |
| ATOM 1249 | CZ | ARG192 | -2.824 | 19.164 | 26.954 | 1.00 | 32.13 |
| ATOM 1250 | NH1 | ARG192 | -3.437 | 18.028 | 26.639 | 1.00 | 34.51 |
| ATOM 1251 | NH2 | ARG192 | -3.465 | 20.115 | 27.631 | 1.00 | 33.64 |
| ATOM 1252 | C | ARG192 | 1.010 | 20.572 | 22.321 | 1.00 | 22.21 |
| ATOM 1253 | O | ARG192 | 0.017 | 21.184 | 21.937 | 1.00 | 24.03 |
| ATOM 1254 | N | GLY193 | 2.245 | 20.923 | 21.975 | 1.00 | 24.28 |
| ATOM 1255 | CA | GLY193 | 2.472 | 22.052 | 21.088 | 1.00 | 25.59 |
| ATOM 1256 | C | GLY193 | 2.351 | 23.417 | 21.750 | 1.00 | 27.55 |
| ATOM 1257 | O | GLY193 | 2.734 | 24.437 | 21.163 | 1.00 | 26.53 |
| ATOM 1258 | N | ASP194 | 1.836 | 23.434 | 22.981 | 1.00 | 28.09 |
| ATOM 1259 | CA | ASP194 | 1.634 | 24.678 | 23.725 | 1.00 | 28.74 |

[Table 2] (continued)

| | | | | | | | |
|---|---|---|---|---|---|---|---|
| ATOM 1260 | CB | ASP194 | 0.349 | 24.597 | 24.548 | 1.00 | 32.11 |
| ATOM 1261 | CG | ASP 194 | -0.873 | 24.329 | 23.692 | 1.00 | 36.60 |
| ATOM 1262 | OD1 | ASP194 | -1.053 | 25.025 | 22.668 | 1.00 | 38.48 |
| ATOM 1263 | OD2 | ASP194 | -1.659 | 23.424 | 24.046 | 1.00 | 40.23 |
| ATOM 1264 | C | ASP194 | 2.774 | 25.089 | 24.641 | 1.00 | 27.04 |
| ATOM 1265 | O | ASP194 | 3.815 | 24.439 | 24.689 | 1.00 | 26.55 |
| ATOM 1266 | N | PHE195 | 2.565 | 26.181 | 25.370 | 1.00 | 25.47 |
| ATOM 1267 | CA | PHE195 | 3.582 | 26.691 | 26.274 | 1.00 | 25.41 |
| ATOM 1268 | CB | PHE195 | 3.083 | 27.932 | 27.016 | 1.00 | 27.05 |
| ATOM 1269 | CG | PHE195 | 3.156 | 29.192 | 26.201 | 1.00 | 28.43 |
| ATOM 1270 | CD1 | PHE195 | 2.032 | 29.686 | 25.550 | 1.00 | 31.56 |
| ATOM 1271 | CD2 | PHE195 | 4.353 | 29.880 | 26.067 | 1.00 | 29.11 |
| ATOM 1272 | CE1 | PHE195 | 2.097 | 30.852 | 24.771 | 1.00 | 30.45 |
| ATOM 1273 | CE2 | PHE195 | 4.426 | 31.046 | 25.290 | 1.00 | 30.62 |
| ATOM 1274 | CZ | PHE195 | 3.294 | 31.528 | 24.644 | 1.00 | 29.47 |
| ATOM 1275 | C | PHE195 | 4.024 | 25.642 | 27.267 | 1.00 | 24.15 |
| ATOM 1276 | O | PHE195 | 3.214 | 25.083 | 28.000 | 1.00 | 25.61 |
| ATOM 1277 | N | GLU196 | 5.324 | 25.385 | 27.280 | 1.00 | 22.49 |
| ATOM 1278 | CA | GLU196 | 5.897 | 24.394 | 28.166 | 1.00 | 23.12 |
| ATOM 1279 | CB | GLU196 | 7.117 | 23.754 | 27.499 | 1.00 | 21.72 |
| ATOM 1280 | CG | GLU196 | 6.942 | 23.418 | 26.020 | 1.00 | 22.22 |
| ATOM 1281 | CD | GLU196 | 8.121 | 22.629 | 25.477 | 1.00 | 24.60 |
| ATOM 1282 | OE1 | GLU196 | 8.336 | 22.601 | 24.241 | 1.00 | 23.54 |
| ATOM 1283 | OE2 | GLU196 | 8.839 | 22.026 | 26.301 | 1.00 | 26.49 |
| ATOM 1284 | C | GLU196 | 6.314 | 25.066 | 29.466 | 1.00 | 24.25 |
| ATOM 1285 | O | GLU196 | 7.467 | 24.966 | 29.882 | 1.00 | 26.05 |
| ATOM 1286 | N | MET197 | 5.376 | 25.729 | 30.126 | 1.00 | 25.12 |
| ATOM 1287 | CA | MET197 | 5.711 | 26.444 | 31.352 | 1.00 | 27.52 |
| ATOM 1288 | CB | MET197 | 5.546 | 27.942 | 31.096 | 1.00 | 29.51 |
| ATOM 1289 | CG | MET197 | 6.758 | 28.782 | 31.466 | 1.00 | 33.61 |
| ATOM 1290 | SD | MET197 | 7.208 | 29.992 | 30.181 | 1.00 | 35.72 |
| ATOM 1291 | CE | MET197 | 5.967 | 31.256 | 30.466 | 1.00 | 37.45 |
| ATOM 1292 | C | MET197 | 4.906 | 26.045 | 32.583 | 1.00 | 27.47 |
| ATOM 1293 | O | MET197 | 4.921 | 26.749 | 33.597 | 1.00 | 25.63 |
| ATOM 1294 | N | ASP198 | 4.230 | 24.903 | 32.502 | 1.00 | 27.57 |
| ATOM 1295 | CA | ASP198 | 3.384 | 24.430 | 33.598 | 1.00 | 26.12 |
| ATOM 1296 | CB | ASP198 | 2.462 | 23.298 | 33.110 | 1.00 | 29.89 |
| ATOM 1297 | CG | ASP198 | 1.326 | 23.796 | 32.232 | 1.00 | 31.76 |
| ATOM 1298 | OD1 | ASP198 | 0.736 | 24.840 | 32.590 | 1.00 | 30.59 |
| ATOM 1299 | OD2 | ASP198 | 1.023 | 23.135 | 31.203 | 1.00 | 32.71 |
| ATOM 1300 | C | ASP198 | 4.110 | 23.959 | 34.853 | 1.00 | 22.26 |
| ATOM 1301 | O | ASP198 | 3.960 | 24.551 | 35.923 | 1.00 | 18.00 |
| ATOM 1302 | N | VAL199 | 4.873 | 22.878 | 34.717 | 1.00 | 19.81 |
| ATOM 1303 | CA | VAL199 | 5.605 | 22.301 | 35.841 | 1.00 | 18.78 |
| ATOM 1304 | CB | VAL199 | 5.133 | 20.852 | 36.115 | 1.00 | 16.48 |
| ATOM 1305 | CG1 | VAL199 | 3.736 | 20.859 | 36.696 | 1.00 | 19.07 |
| ATOM 1306 | CG2 | VAL199 | 5.150 | 20.042 | 34.823 | 1.00 | 10.86 |
| ATOM 1307 | C | VAL199 | 7.121 | 22.267 | 35.648 | 1.00 | 20.19 |
| ATOM 1308 | O | VAL199 | 7.665 | 22.752 | 34.655 | 1.00 | 21.16 |
| ATOM 1309 | N | VAL200 | 7.798 | 21.695 | 36.629 | 1.00 | 20.40 |
| ATOM 1310 | CA | VAL200 | 9.237 | 21.547 | 36.594 | 1.00 | 22.39 |

[Table 2] (continued)

| | | | | | | | |
|---|---|---|---|---|---|---|---|
| ATOM 1311 | CB | VAL200 | 9.975 | 22.834 | 37.007 | 1.00 | 24.84 |
| ATOM 1312 | CG1 | VAL200 | 9.331 | 23.406 | 38.255 | 1.00 | 31.58 |
| ATOM 1313 | CG2 | VAL200 | 11.465 | 22.539 | 37.266 | 1.00 | 21.54 |
| ATOM 1314 | C | VAL200 | 9.502 | 20.457 | 37.598 | 1.00 | 23.06 |
| ATOM 1315 | O | VAL200 | 9.039 | 20.501 | 38.755 | 1.00 | 22.26 |
| ATOM 1316 | N | ALA201 | 10.229 | 19.460 | 37.120 | 1.00 | 23.03 |
| ATOM 1317 | CA | ALA201 | 10.569 | 18.300 | 37.907 | 1.00 | 22.74 |
| ATOM 1318 | CB | ALA201 | 11.460 | 17.418 | 37.112 | 1.00 | 23.66 |
| ATOM 1319 | C | ALA201 | 11.236 | 18.646 | 39.209 | 1.00 | 24.47 |
| ATOM 1320 | O | ALA201 | 12.045 | 19.564 | 39.285 | 1.00 | 27.55 |
| ATOM 1321 | N | MET202 | 10.872 | 17.914 | 40.244 | 1.00 | 25.96 |
| ATOM 1322 | CA | MET202 | 11.479 | 18.106 | 41.547 | 1.00 | 27.52 |
| ATOM 1323 | CB | MET202 | 10.720 | 19.124 | 42.386 | 1.00 | 27.45 |
| ATOM 1324 | CG | MET202 | 11.516 | 19.580 | 43.597 | 1.00 | 27.56 |
| ATOM 1325 | SD | MET202 | 11.967 | 18.244 | 44.740 | 1.00 | 28.85 |
| ATOM 1326 | CE | MET202 | 10.732 | 18.486 | 46.045 | 1.00 | 23.74 |
| ATOM 1327 | C | MET202 | 11.436 | 16.752 | 42.219 | 1.00 | 28.79 |
| ATOM 1328 | O | MET202 | 10.377 | 16.290 | 42.653 | 1.00 | 25.51 |
| ATOM 1329 | N | VAL203 | 12.600 | 16.118 | 42.293 | 1.00 | 29.76 |
| ATOM 1330 | CA | VAL203 | 12.695 | 14.802 | 42.883 | 1.00 | 28.97 |
| ATOM 1331 | CB | VAL203 | 12.943 | 13.727 | 41.813 | 1.00 | 25.86 |
| ATOM 1332 | CG1 | VAL203 | 11.936 | 13.870 | 40.681 | 1.00 | 22.02 |
| ATOM 1333 | CG2 | VAL203 | 14.361 | 13.831 | 41.310 | 1.00 | 23.30 |
| ATOM 1334 | C | VAL203 | 13.815 | 14.713 | 43.890 | 1.00 | 31.36 |
| ATOM 1335 | O | VAL203 | 13.934 | 13.713 | 44.585 | 1.00 | 34.93 |
| ATOM 1336 | N | ASN204 | 14.638 | 15.745 | 43.987 | 1.00 | 32.12 |
| ATOM 1337 | CA | ASN204 | 15.741 | 15.674 | 44.929 | 1.00 | 33.37 |
| ATOM 1338 | CB | ASN204 | 16.667 | 16.867 | 44.736 | 1.00 | 36.19 |
| ATOM 1339 | CG | ASN204 | 18.052 | 16.601 | 45.260 | 1.00 | 39.20 |
| ATOM 1340 | OD1 | ASN204 | 18.847 | 15.905 | 44.621 | 1.00 | 41.71 |
| ATOM 1341 | ND2 | ASN204 | 18.349 | 17.133 | 46.440 | 1.00 | 39.72 |
| ATOM 1342 | C | ASN204 | 15.220 | 15.625 | 46.363 | 1.00 | 32.02 |
| ATOM 1343 | O | ASN204 | 14.382 | 16.439 | 46.751 | 1.00 | 28.87 |
| ATOM 1344 | N | ASP205 | 15.705 | 14.665 | 47.149 | 1.00 | 31.97 |
| ATOM 1345 | CA | ASP205 | 15.245 | 14.538 | 48.541 | 1.00 | 33.94 |
| ATOM 1346 | CB | ASP205 | 15.792 | 13.266 | 49.197 | 1.00 | 32.38 |
| ATOM 1347 | CG | ASP205 | 15.163 | 12.017 | 48.642 | 1.00 | 31.18 |
| ATOM 1348 | OD1 | ASP205 | 15.386 | 10.935 | 49.217 | 1.00 | 31.15 |
| ATOM 1349 | OD2 | ASP205 | 14.450 | 12.118 | 47.625 | 1.00 | 28.42 |
| ATOM 1350 | C | ASP205 | 15.626 | 15.722 | 49.414 | 1.00 | 33.64 |
| ATOM 1351 | O | ASP205 | 14.909 | 16.080 | 50.356 | 1.00 | 33.83 |
| ATOM 1352 | N | THR206 | 16.770 | 16.313 | 49.092 | 1.00 | 31.15 |
| ATOM 1353 | CA | THR206 | 17.290 | 17.449 | 49.826 | 1.00 | 25.09 |
| ATOM 1354 | CB | THR206 | 18.646 | 17.825 | 49.278 | 1.00 | 25.45 |
| ATOM 1355 | OG1 | THR206 | 19.423 | 16.630 | 49.123 | 1.00 | 24.8 |
| ATOM 1356 | CG2 | THR206 | 19.350 | 18.769 | 50.232 | 1.00 | 26.26 |
| ATOM 1357 | C | THR206 | 16.347 | 18.634 | 49.734 | 1.00 | 20.16 |
| ATOM 1358 | O | THR206 | 15.923 | 19.184 | 50.755 | 1.00 | 17.86 |
| ATOM 1359 | N | VAL207 | 16.009 | 19.016 | 48.510 | 1.00 | 12.86 |
| ATOM 1360 | CA | VAL207 | 15.106 | 20.133 | 48.308 | 1.00 | 9.27 |
| ATOM 1361 | CB | VAL207 | 14.582 | 20.164 | 46.867 | 1.00 | 5.21 |

[Table 2] (continued)

| | | | | | | | |
|---|---|---|---|---|---|---|---|
| ATOM 1362 | CG1 | VAL207 | 13.555 | 21.243 | 46.720 | 1.00 | 1.26 |
| ATOM 1363 | CG2 | VAL207 | 15.714 | 20.397 | 45.910 | 1.00 | 4.57 |
| ATOM 1364 | C | VAL207 | 13.917 | 19.992 | 49.255 | 1.00 | 11.72 |
| ATOM 1365 | O | VAL207 | 13.584 | 20.909 | 50.016 | 1.00 | 9.00 |
| ATOM 1366 | N | ALA208 | 13.291 | 18.819 | 49.212 | 1.00 | 14.04 |
| ATOM 1367 | CA | ALA208 | 12.122 | 18.523 | 50.041 | 1.00 | 14.67 |
| ATOM 1368 | CB | ALA208 | 11.598 | 17.148 | 49.702 | 1.00 | 14.60 |
| ATOM 1369 | C | ALA208 | 12.422 | 18.615 | 51.537 | 1.00 | 15.41 |
| ATOM 1370 | O | ALA208 | 11.514 | 18.770 | 52.362 | 1.00 | 14.28 |
| ATOM 1371 | N | THR209 | 13.699 | 18.498 | 51.879 | 1.00 | 13.94 |
| ATOM 1372 | CA | THR209 | 14.123 | 18.591 | 53.261 | 1.00 | 13.05 |
| ATOM 1373 | CB | THR209 | 15.567 | 18.237 | 53.423 | 1.00 | 11.66 |
| ATOM 1374 | OG1 | THR209 | 15.887 | 17.177 | 52.525 | 1.00 | 12.70 |
| ATOM 1375 | CG2 | THR209 | 15.833 | 17.807 | 54.846 | 1.00 | 7.92 |
| ATOM 1376 | C | THR209 | 14.007 | 20.041 | 53.626 | 1.00 | 14.97 |
| ATOM 1377 | O | THR209 | 13.554 | 20.401 | 54.714 | 1.00 | 14.80 |
| ATOM 1378 | N | MET210 | 14.447 | 20.885 | 52.707 | 1.00 | 15.34 |
| ATOM 1379 | CA | MET210 | 14.363 | 22.298 | 52.965 | 1.00 | 16.36 |
| ATOM 1380 | CB | MET210 | 15.043 | 23.091 | 51.845 | 1.00 | 19.89 |
| ATOM 1381 | CG | MET210 | 15.119 | 24.592 | 52.103 | 1.00 | 23.82 |
| ATOM 1382 | SD | MET210 | 15.258 | 25.542 | 50.561 | 1.00 | 29.33 |
| ATOM 1383 | CE | MET210 | 13.547 | 25.995 | 50.325 | 1.00 | 27.80 |
| ATOM 1384 | C | MET210 | 12.864 | 22.592 | 53.031 | 1.00 | 14.33 |
| ATOM 1385 | O | MET210 | 12.332 | 22.896 | 54.102 | 1.00 | 15.04 |
| ATOM 1386 | N | ILE211 | 12.180 | 22.452 | 51.898 | 1.00 | 11.15 |
| ATOM 1387 | CA | ILE211 | 10.743 | 22.708 | 51.831 | 1.00 | 9.09 |
| ATOM 1388 | CB | ILE211 | 10.157 | 22.122 | 50.566 | 1.00 | 5.39 |
| ATOM 1389 | CG2 | ILE211 | 8.748 | 22.693 | 50.337 | 1.00 | 3.22 |
| ATOM 1390 | CG1 | ILE211 | 11.111 | 22.412 | 49.412 | 1.00 | 2.02 |
| ATOM 1391 | CD1 | ILE211 | 10.580 | 22.065 | 48.067 | 1.00 | 1.00 |
| ATOM 1392 | C | ILE211 | 9.987 | 22.129 | 53.022 | 1.00 | 10.92 |
| ATOM 1393 | O | ILE211 | 9.117 | 22.781 | 53.605 | 1.00 | 7.92 |
| ATOM 1394 | N | SER212 | 10.319 | 20.891 | 53.364 | 1.00 | 12.74 |
| ATOM 1395 | CA | SER212 | 9.701 | 20.254 | 54.489 | 1.00 | 15.18 |
| ATOM 1396 | CB | SER212 | 10.300 | 18.880 | 54.704 | 1.00 | 12.84 |
| ATOM 1397 | OG | SER212 | 10.216 | 18.533 | 56.078 | 1.00 | 19.56 |
| ATOM 1398 | C | SER212 | 9.918 | 21.101 | 55.736 | 1.00 | 19.90 |
| ATOM 1399 | O | SER212 | 8.969 | 21.432 | 56.435 | 1.00 | 21.30 |
| ATOM 1400 | N | CYS213 | 11.161 | 21.476 | 56.016 | 1.00 | 24.22 |
| ATOM 1401 | CA | CYS213 | 11.432 | 22.259 | 57.219 | 1.00 | 28.52 |
| ATOM 1402 | CB | CYS213 | 12.934 | 22.367 | 57.464 | 1.00 | 30.65 |
| ATOM 1403 | SG | CYS213 | 13.713 | 20.766 | 57.805 | 1.00 | 39.09 |
| ATOM 1404 | C | CYS213 | 10.822 | 23.637 | 57.168 | 1.00 | 29.40 |
| ATOM 1405 | O | CYS213 | 10.366 | 24.150 | 58.186 | 1.00 | 30.64 |
| ATOM 1406 | N | TYR214 | 10.816 | 24.229 | 55.981 | 1.00 | 29.50 |
| ATOM 1407 | CA | TYR214 | 10.243 | 25.548 | 55.788 | 1.00 | 29.27 |
| ATOM 1408 | CB | TYR214 | 10.168 | 25.846 | 54.292 | 1.00 | 31.33 |
| ATOM 1409 | CG | TYR214 | 9.637 | 27.212 | 53.985 | 1.00 | 33.15 |
| ATOM 1410 | CD1 | TYR214 | 10.182 | 28.328 | 54.594 | 1.00 | 36.28 |
| ATOM 1411 | CE1 | TYR214 | 9.694 | 29.592 | 54.341 | 1.00 | 39.73 |
| ATOM 1412 | CD2 | TYR214 | 8.582 | 27.390 | 53.100 | 1.00 | 35.23 |

[Table 2] (continued)

| | | | | | | | |
|---|---|---|---|---|---|---|---|
| ATOM 1413 | CE2 | TYR214 | 8.080 | 28.656 | 52.833 | 1.00 | 39.38 |
| ATOM 1414 | CZ | TYR214 | 8.644 | 29.758 | 53.463 | 1.00 | 41.11 |
| ATOM 1415 | OH | TYR214 | 8.168 | 31.034 | 53.241 | 1.00 | 43.33 |
| ATOM 1416 | C | TYR214 | 8.848 | 25.649 | 56.429 | 1.00 | 28.57 |
| ATOM 1417 | O | TYR214 | 8.561 | 26.578 | 57.185 | 1.00 | 27.99 |
| ATOM 1418 | N | TYR215 | 7.986 | 24.685 | 56.136 | 1.00 | 27.91 |
| ATOM 1419 | CA | TYR215 | 6.642 | 24.685 | 56.691 | 1.00 | 27.12 |
| ATOM 1420 | CB | TYR215 | 5.922 | 23.403 | 56.309 | 1.00 | 21.95 |
| ATOM 1421 | CG | TYR215 | 5.723 | 23.235 | 54.829 | 1.00 | 18.06 |
| ATOM 1422 | CD1 | TYR215 | 6.064 | 22.048 | 54.197 | 1.00 | 17.25 |
| ATOM 1423 | CE1 | TYR215 | 5.835 | 21.867 | 52.841 | 1.00 | 17.47 |
| ATOM 1424 | CD2 | TYR215 | 5.152 | 24.246 | 54.065 | 1.00 | 16.51 |
| ATOM 1425 | CE2 | TYR215 | 4.917 | 24.075 | 52.711 | 1.00 | 15.51 |
| ATOM 1426 | CZ | TYR215 | 5.257 | 22.882 | 52.109 | 1.00 | 17.82 |
| ATOM 1427 | OH | TYR215 | 4.979 | 22.681 | 50.785 | 1.00 | 20.98 |
| ATOM 1428 | C | TYR215 | 6.658 | 24.810 | 58.201 | 1.00 | 30.19 |
| ATOM 1429 | O | TYR215 | 5.780 | 25.438 | 58.778 | 1.00 | 31.10 |
| ATOM 1430 | N | GLU216 | 7.640 | 24.197 | 58.850 | 1.00 | 35.15 |
| ATOM 1431 | CA | GLU216 | 7.725 | 24.278 | 60.306 | 1.00 | 41.19 |
| ATOM 1432 | CB | GLU216 | 8.560 | 23.132 | 60.876 | 1.00 | 44.10 |
| ATOM 1433 | CG | GLU216 | 7.877 | 21.767 | 60.887 | 1.00 | 52.19 |
| ATOM 1434 | CD | GLU216 | 6.579 | 21.749 | 61.685 | 1.00 | 54.93 |
| ATOM 1435 | OE1 | GLU216 | 6.491 | 22.481 | 62.702 | 1.00 | 55.44 |
| ATOM 1436 | OE2 | GLU216 | 5.658 | 20.988 | 61.296 | 1.00 | 56.26 |
| ATOM 1437 | C | GLU216 | 8.369 | 25.591 | 60.707 | 1.00 | 43.33 |
| ATOM 1438 | O | GLU216 | 7.787 | 26.385 | 61.449 | 1.00 | 44.64 |
| ATOM 1439 | N | ASP217 | 9.583 | 25.802 | 60.209 | 1.00 | 44.35 |
| ATOM 1440 | CA | ASP217 | 10.357 | 27.007 | 60.489 | 1.00 | 44.65 |
| ATOM 1441 | CB | ASP217 | 11.734 | 26.623 | 61.033 | 1.00 | 47.71 |
| ATOM 1442 | CG | ASP217 | 12.667 | 27.806 | 61.136 | 1.00 | 50.46 |
| ATOM 1443 | OD1 | ASP217 | 13.252 | 28.205 | 60.106 | 1.00 | 51.95 |
| ATOM 1444 | OD2 | ASP217 | 12.804 | 28.346 | 62.252 | 1.00 | 54.06 |
| ATOM 1445 | C | ASP217 | 10.514 | 27.820 | 59.215 | 1.00 | 43.04 |
| ATOM 1446 | O | ASP217 | 11.372 | 27.527 | 58.385 | 1.00 | 44.60 |
| ATOM 1447 | N | HIS218 | 9.691 | 28.848 | 59.059 | 1.00 | 41.00 |
| ATOM 1448 | CA | HIS218 | 9.750 | 29.671 | 57.862 | 1.00 | 39.42 |
| ATOM 1449 | CB | HIS218 | 8.569 | 30.630 | 57.826 | 1.00 | 40.46 |
| ATOM 1450 | CG | HIS218 | 7.261 | 29.960 | 58.083 | 1.00 | 44.54 |
| ATOM 1451 | CD2 | HIS218 | 6.652 | 28.930 | 57.450 | 1.00 | 45.30 |
| ATOM 1452 | ND1 | HIS218 | 6.449 | 30.290 | 59.147 | 1.00 | 47.09 |
| ATOM 1453 | CE1 | HIS218 | 5.397 | 29.492 | 59.161 | 1.00 | 45.61 |
| ATOM 1454 | NE2 | HIS218 | 5.497 | 28.657 | 58.142 | 1.00 | 46.44 |
| ATOM 1455 | C | HIS218 | 11.036 | 30.452 | 57.759 | 1.00 | 37.69 |
| ATOM 1456 | O | HIS218 | 11.120 | 31.381 | 56.974 | 1.00 | 37.21 |
| ATOM 1457 | N | GLN219 | 12.041 | 30.076 | 58.537 | 1.00 | 37.38 |
| ATOM 1458 | CA | GLN219 | 13.312 | 30.779 | 58.494 | 1.00 | 38.18 |
| ATOM 1459 | CB | GLN219 | 13.727 | 31.186 | 59.910 | 1.00 | 41.72 |
| ATOM 1460 | CG | GLN219 | 14.577 | 32.451 | 60.011 | 1.00 | 48.69 |
| ATOM 1461 | CD | GLN219 | 13.836 | 33.718 | 59.546 | 1.00 | 55.14 |
| ATOM 1462 | OE1 | GLN219 | 12.665 | 33.945 | 59.908 | 1.00 | 55.89 |
| ATOM 1463 | NE2 | GLN219 | 14.523 | 34.555 | 58.751 | 1.00 | 55.41 |

[Table 2] (continued)

| | | | | | | | |
|---|---|---|---|---|---|---|---|
| ATOM 1464 | C | GLN219 | 14.348 | 29.846 | 57.886 | 1.00 | 36.85 |
| ATOM 1465 | O | GLN219 | 15.508 | 30.200 | 57.735 | 1.00 | 37.28 |
| ATOM 1466 | N | CYS220 | 13.912 | 28.647 | 57.535 | 1.00 | 36.02 |
| ATOM 1467 | CA | CYS220 | 14.790 | 27.646 | 56.950 | 1.00 | 37.10 |
| ATOM 1468 | CB | CYS220 | 14.103 | 26.286 | 57.043 | 1.00 | 38.40 |
| ATOM 1469 | SG | CYS220 | 15.067 | 24.916 | 56.396 | 1.00 | 44.24 |
| ATOM 1470 | C | CYS220 | 15.106 | 27.970 | 55.486 | 1.00 | 37.48 |
| ATOM 1471 | O | CYS220 | 14.193 | 28.081 | 54.672 | 1.00 | 40.52 |
| ATOM 1472 | N | GLU221 | 16.382 | 28.123 | 55.137 | 1.00 | 36.17 |
| ATOM 1473 | CA | GLU221 | 16.742 | 28.428 | 53.746 | 1.00 | 35.58 |
| ATOM 1474 | CB | GLU221 | 17.116 | 29.911 | 53.591 | 1.00 | 38.60 |
| ATOM 1475 | CG | GLU221 | 15.921 | 30.878 | 53.645 | 1.00 | 42.48 |
| ATOM 1476 | CD | GLU221 | 16.325 | 32.347 | 53.760 | 1.00 | 42.62 |
| ATOM 1477 | OE1 | GLU221 | 17.120 | 32.815 | 52.909 | 1.00 | 42.89 |
| ATOM 1478 | OE2 | GLU221 | 15.835 | 33.024 | 54.700 | 1.00 | 40.36 |
| ATOM 1479 | C | GLU221 | 17.896 | 27.566 | 53.260 | 1.00 | 33.89 |
| ATOM 1480 | O | GLU221 | 18.498 | 27.826 | 52.217 | 1.00 | 32.29 |
| ATOM 1481 | N | VAL222 | 18.199 | 26.525 | 54.018 | 1.00 | 32.57 |
| ATOM 1482 | CA | VAL222 | 19.286 | 25.654 | 53.645 | 1.00 | 31.01 |
| ATOM 1483 | CB | VAL222 | 20.548 | 26.041 | 54.376 | 1.00 | 29.59 |
| ATOM 1484 | CG1 | VAL222 | 21.673 | 25.102 | 53.995 | 1.00 | 29.07 |
| ATOM 1485 | CG2 | VAL222 | 20.895 | 27.465 | 54.043 | 1.00 | 30.00 |
| ATOM 1486 | C | VAL222 | 18.983 | 24.214 | 53.966 | 1.00 | 31.75 |
| ATOM 1487 | O | VAL222 | 18.872 | 23.846 | 55.132 | 1.00 | 33.50 |
| ATOM 1488 | N | GLY223 | 18.858 | 23.400 | 52.925 | 1.00 | 31.02 |
| ATOM 1489 | CA | GLY223 | 18.575 | 21.994 | 53.119 | 1.00 | 28.49 |
| ATOM 1490 | C | GLY223 | 19.847 | 21.184 | 53.026 | 1.00 | 26.21 |
| ATOM 1491 | O | GLY223 | 20.757 | 21.528 | 52.267 | 1.00 | 25.39 |
| ATOM 1492 | N | MET224 | 19.911 | 20.098 | 53.786 | 1.00 | 24.93 |
| ATOM 1493 | CA | MET224 | 21.101 | 19.267 | 53.774 | 1.00 | 24.66 |
| ATOM 1494 | CB | MET224 | 22.164 | 19.958 | 54.623 | 1.00 | 26.07 |
| ATOM 1495 | CG | MET224 | 23.584 | 19.535 | 54.358 | 1.00 | 26.25 |
| ATOM 1496 | SD | MET224 | 24.664 | 20.375 | 55.525 | 1.00 | 28.76 |
| ATOM 1497 | CE | MET224 | 24.493 | 19.328 | 56.939 | 1.00 | 27.46 |
| ATOM 1498 | C | MET224 | 20.867 | 17.819 | 54.253 | 1.00 | 23.62 |
| ATOM 1499 | O | MET224 | 20.243 | 17.581 | 55.294 | 1.00 | 21.62 |
| ATOM 1500 | N | ILE225 | 21.389 | 16.867 | 53.478 | 1.00 | 21.96 |
| ATOM 1501 | CA | ILE225 | 21.265 | 15.434 | 53.764 | 1.00 | 21.80 |
| ATOM 1502 | CB | ILE225 | 20.514 | 14.706 | 52.662 | 1.00 | 23.26 |
| ATOM 1503 | CG2 | ILE225 | 20.389 | 13.242 | 53.026 | 1.00 | 22.57 |
| ATOM 1504 | CG1 | ILE225 | 19.142 | 15.332 | 52.463 | 1.00 | 26.22 |
| ATOM 1505 | CD1 | ILE225 | 18.270 | 15.229 | 53.688 | 1.00 | 30.06 |
| ATOM 1506 | C | ILE225 | 22.595 | 14.702 | 53.904 | 1.00 | 21.76 |
| ATOM 1507 | O | ILE225 | 23.204 | 14.299 | 52.909 | 1.00 | 20.84 |
| ATOM 1508 | N | VAL226 | 23.008 | 14.492 | 55.146 | 1.00 | 22.14 |
| ATOM 1509 | CA | VAL226 | 24.263 | 13.824 | 55.454 | 1.00 | 22.07 |
| ATOM 1510 | CB | VAL226 | 25.031 | 14.613 | 56.514 | 1.00 | 22.20 |
| ATOM 1511 | CG1 | VAL226 | 26.321 | 13.905 | 56.872 | 1.00 | 20.57 |
| ATOM 1512 | CG2 | VAL226 | 25.283 | 16.016 | 56.005 | 1.00 | 22.66 |
| ATOM 1513 | C | VAL226 | 24.060 | 12.411 | 55.972 | 1.00 | 22.96 |
| ATOM 1514 | O | VAL226 | 24.032 | 12.172 | 57.183 | 1.00 | 23.79 |

[Table 2] (continued)

| | | | | | | | |
|---|---|---|---|---|---|---|---|
| ATOM 1515 | N | GLY227 | 23.924 | 11.470 | 55.054 | 1.00 | 23.08 |
| ATOM 1516 | CA | GLY227 | 23.738 | 10.094 | 55.459 | 1.00 | 25.20 |
| ATOM 1517 | C | GLY227 | 24.623 | 9.207 | 54.621 | 1.00 | 25.79 |
| ATOM 1518 | O | GLY227 | 25.820 | 9.447 | 54.501 | 1.00 | 26.18 |
| ATOM 1519 | N | THR228 | 24.039 | 8.181 | 54.026 | 1.00 | 27.28 |
| ATOM 1520 | CA | THR228 | 24.822 | 7.291 | 53.200 | 1.00 | 29.44 |
| ATOM 1521 | CB | THR228 | 23.900 | 6.356 | 52.413 | 1.00 | 28.91 |
| ATOM 1522 | OG1 | THR228 | 24.691 | 5.441 | 51.650 | 1.00 | 27.64 |
| ATOM 1523 | CG2 | THR228 | 22.983 | 7.159 | 51.496 | 1.00 | 30.69 |
| ATOM 1524 | C | THR228 | 25.705 | 8.139 | 52.267 | 1.00 | 30.87 |
| ATOM 1525 | O | THR228 | 26.878 | 7.834 | 52.072 | 1.00 | 32.00 |
| ATOM 1526 | N | GLY229 | 25.140 | 9.216 | 51.723 | 1.00 | 31.23 |
| ATOM 1527 | CA | GLY229 | 25.888 | 10.111 | 50.855 | 1.00 | 30.25 |
| ATOM 1528 | C | GLY229 | 25.716 | 11.501 | 51.434 | 1.00 | 32.12 |
| ATOM 1529 | O | GLY229 | 25.139 | 11.632 | 52.518 | 1.00 | 33.23 |
| ATOM 1530 | N | CYS230 | 26.208 | 12.535 | 50.749 | 1.00 | 31.95 |
| ATOM 1531 | CA | CYS230 | 26.057 | 13.909 | 51.247 | 1.00 | 31.05 |
| ATOM 1532 | CB | CYS230 | 27.344 | 14.417 | 51.891 | 1.00 | 31.11 |
| ATOM 1533 | SG | CYS230 | 27.145 | 16.090 | 52.562 | 1.00 | 40.64 |
| ATOM 1534 | C | CYS230 | 25.650 | 14.909 | 50.183 | 1.00 | 29.04 |
| ATOM1535 | O | CYS230 | 26.202 | 14.913 | 49.087 | 1.00 | 30.85 |
| ATOM 1536 | N | ASN231 | 24.701 | 15.775 | 50.513 | 1.00 | 26.12 |
| ATOM 1537 | CA | ASN231 | 24.267 | 16.773 | 49.554 | 1.00 | 26.17 |
| ATOM 1538 | CB | ASN231 | 23.380 | 16.130 | 48.505 | 1.00 | 24.13 |
| ATOM 1539 | CG | ASN231 | 23.146 | 17.030 | 47.341 | 1.00 | 24.98 |
| ATOM 1540 | OD1 | ASN231 | 22.505 | 18.064 | 47.463 | 1.00 | 24.73 |
| ATOM 1541 | ND2 | ASN231 | 23.684 | 16.656 | 46.196 | 1.00 | 29.51 |
| ATOM 1542 | C | ASN231 | 23.529 | 17.927 | 50.213 | 1.00 | 27.77 |
| ATOM 1543 | O | ASN231 | 22.929 | 17.757 | 51.275 | 1.00 | 28.70 |
| ATOM 1544 | N | ALA232 | 23.569 | 19.103 | 49.587 | 1.00 | 27.44 |
| ATOM 1545 | CA | ALA232 | 22.890 | 20.258 | 50.158 | 1.00 | 26.70 |
| ATOM 1546 | CB | ALA232 | 23.806 | 20.963 | 51.113 | 1.00 | 26.89 |
| ATOM 1547 | C | ALA232 | 22.366 | 21.245 | 49.144 | 1.00 | 26.61 |
| ATOM 1548 | O | ALA232 | 22.693 | 21.184 | 47.963 | 1.00 | 26.44 |
| ATOM 1549 | N | CYS233 | 21.537 | 22.161 | 49.617 | 1.00 | 27.04 |
| ATOM 1550 | CA | CYS233 | 20.976 | 23.172 | 48.743 | 1.00 | 31.21 |
| ATOM 1551 | CB | CYS233 | 19.676 | 22.666 | 48.127 | 1.00 | 31.60 |
| ATOM 1552 | SG | CYS233 | 18.376 | 22.446 | 49.348 | 1.00 | 35.31 |
| ATOM 1553 | C | CYS233 | 20.708 | 24.408 | 49.589 | 1.00 | 31.98 |
| ATOM 1554 | O | CYS233 | 20.596 | 24.303 | 50.809 | 1.00 | 32.62 |
| ATOM 1555 | N | TYR234 | 20.621 | 25.572 | 48.949 | 1.00 | 30.70 |
| ATOM 1556 | CA | TYR234 | 20.366 | 26.822 | 49.660 | 1.00 | 30.60 |
| ATOM 1557 | CB | TYR234 | 21.684 | 27.524 | 50.026 | 1.00 | 29.53 |
| ATOM 1558 | CG | TYR234 | 22.464 | 28.011 | 48.829 | 1.00 | 27.41 |
| ATOM 1559 | CD1 | TYR234 | 22.363 | 29.327 | 48.393 | 1.00 | 25.56 |
| ATOM 1560 | CE1 | TYR234 | 22.981 | 29.739 | 47.217 | 1.00 | 25.47 |
| ATOM 1561 | CD2 | TYR234 | 23.218 | 27.121 | 48.061 | 1.00 | 28.10 |
| ATOM 1562 | CE2 | TYR234 | 23.838 | 27.524 | 46.882 | 1.00 | 26.39 |
| ATOM 1563 | CZ | TYR234 | 23.707 | 28.830 | 46.462 | 1.00 | 25.77 |
| ATOM 1564 | OH | TYR234 | 24.240 | 29.201 | 45.253 | 1.00 | 27.36 |
| ATOM 1565 | C | TYR234 | 19.531 | 27.742 | 48.797 | 1.00 | 32.10 |

[Table 2] (continued)

| | | | | | | | |
|---|---|---|---|---|---|---|---|
| ATOM 1566 | O | TYR234 | 19.211 | 27.411 | 47.657 | 1.00 | 32.79 |
| ATOM 1567 | N | MET235 | 19.184 | 28.897 | 49.357 | 1.00 | 34.08 |
| ATOM 1568 | CA | MET235 | 18.380 | 29.908 | 48.679 | 1.00 | 34.57 |
| ATOM 1569 | CB | MET235 | 17.492 | 30.617 | 49.697 | 1.00 | 34.74 |
| ATOM 1570 | CG | MET235 | 16.489 | 29.699 | 50.305 | 1.00 | 34.74 |
| ATOM 1571 | SD | MET235 | 15.575 | 28.985 | 48.959 | 1.00 | 35.81 |
| ATOM 1572 | CE | MET235 | 14.171 | 30.092 | 48.917 | 1.00 | 34.50 |
| ATOM 1573 | C | MET235 | 19.270 | 30.933 | 48.009 | 1.00 | 35.41 |
| ATOM 1574 | O | MET235 | 19.631 | 31.930 | 48.625 | 1.00 | 37.55 |
| ATOM 1575 | N | GLU236 | 19.626 | 30.702 | 46.753 | 1.00 | 35.58 |
| ATOM 1576 | CA | GLU236 | 20.487 | 31.643 | 46.049 | 1.00 | 36.59 |
| ATOM 1577 | CB | GLU236 | 21.168 | 30.949 | 44.869 | 1.00 | 38.16 |
| ATOM 1578 | CG | GLU236 | 22.051 | 31.861 | 44.051 | 1.00 | 39.44 |
| ATOM 1579 | CD | GLU236 | 23.107 | 32.542 | 44.890 | 1.00 | 41.44 |
| ATOM 1580 | OE1 | GLU236 | 24.116 | 31.891 | 45.240 | 1.00 | 40.65 |
| ATOM 1581 | OE2 | GLU236 | 22.918 | 33.735 | 45.208 | 1.00 | 42.03 |
| ATOM 1582 | C | GLU236 | 19.679 | 32.838 | 45.564 | 1.00 | 37.02 |
| ATOM 1583 | O | GLU236 | 18.452 | 32.810 | 45.580 | 1.00 | 38.00 |
| ATOM 1584 | N | GLU237 | 20.354 | 33.898 | 45.149 | 1.00 | 38.75 |
| ATOM 1585 | CA | GLU237 | 19.634 | 35.062 | 44.668 | 1.00 | 41.18 |
| ATOM 1586 | CB | GLU237 | 20.482 | 36.317 | 44.830 | 1.00 | 39.63 |
| ATOM 1587 | CG | GLU237 | 20.912 | 36.579 | 46.258 | 1.00 | 36.10 |
| ATOM 1588 | CD | GLU237 | 19.764 | 37.022 | 47.131 | 1.00 | 35.20 |
| ATOM 1589 | OE1 | GLU237 | 19.056 | 37.971 | 46.726 | 1.00 | 34.49 |
| ATOM 1590 | OE2 | GLU237 | 19.574 | 36.434 | 48.221 | 1.00 | 33.72 |
| ATOM 1591 | C | GLU237 | 19.307 | 34.836 | 43.206 | 1.00 | 43.71 |
| ATOM 1592 | O | GLU237 | 20.143 | 34.351 | 42.437 | 1.00 | 43.65 |
| ATOM 1593 | N | MET238 | 18.078 | 35.172 | 42.832 | 1.00 | 45.47 |
| ATOM 1594 | CA | MET238 | 17.625 | 35.013 | 41.457 | 1.00 | 47.13 |
| ATOM 1595 | CB | MET238 | 16.275 | 35.705 | 41.275 | 1.00 | 47.10 |
| ATOM 1596 | CG | MET238 | 15.094 | 34.875 | 41.721 | 1.00 | 46.82 |
| ATOM 1597 | SD | MET238 | 14.773 | 33.548 | 40.554 | 1.00 | 45.37 |
| ATOM 1598 | CE | MET238 | 13.564 | 34.332 | 39.412 | 1.00 | 46.47 |
| ATOM 1599 | C | MET238 | 18.629 | 35.589 | 40.466 | 1.00 | 48.34 |
| ATOM 1600 | O | MET238 | 18.814 | 35.061 | 39.371 | 1.00 | 49.97 |
| ATOM 1601 | N | GLN239 | 19.280 | 36.672 | 40.868 | 1.00 | 48.44 |
| ATOM 1602 | CA | GLN239 | 20.252 | 37.344 | 40.026 | 1.00 | 49.76 |
| ATOM 1603 | CB | GLN239 | 20.398 | 38.794 | 40.491 | 1.00 | 54.00 |
| ATOM 1604 | CG | GLN239 | 20.375 | 38.963 | 42.007 | 1.00 | 58.66 |
| ATOM 1605 | CD | GLN239 | 20.056 | 40.394 | 42.447 | 1.00 | 63.23 |
| ATOM 1606 | OE1 | GLN239 | 19.660 | 40.624 | 43.593 | 1.00 | 65.75 |
| ATOM 1607 | NE2 | GLN239 | 20.233 | 41.359 | 41.540 | 1.00 | 63.23 |
| ATOM 1608 | C | GLN239 | 21.612 | 36.665 | 40.011 | 1.00 | 48.87 |
| ATOM 1609 | O | GLN239 | 22.611 | 37.295 | 39.687 | 1.00 | 49. 50 |
| ATOM 1610 | N | ASN240 | 21.656 | 35.384 | 40.354 | 1.00 | 47.67 |
| ATOM 1611 | CA | ASN240 | 22.926 | 34.660 | 40.379 | 1.00 | 47.01 |
| ATOM 1612 | CB | ASN240 | 23.301 | 34.278 | 41.809 | 1.00 | 47.66 |
| ATOM 1613 | CG | ASN240 | 24.101 | 35.347 | 42.518 | 1.00 | 45.71 |
| ATOM 1614 | OD1 | ASN240 | 23.553 | 36.328 | 43.021 | 1.00 | 43.88 |
| ATOM 1615 | ND2 | ASN240 | 25.414 | 35.159 | 42.561 | 1.00 | 46.64 |
| ATOM 1616 | C | ASN240 | 22.861 | 33.393 | 39.550 | 1.00 | 46.58 |

[Table 2] (continued)

| | | | | | | | |
|---|---|---|---|---|---|---|---|
| ATOM 1617 | O | ASN240 | 23.888 | 32.840 | 39.137 | 1.00 | 46.44 |
| ATOM 1618 | N | VAL241 | 21.643 | 32.919 | 39.340 | 1.00 | 44.69 |
| ATOM 1619 | CA | VAL241 | 21.426 | 31.717 | 38.564 | 1.00 | 43.22 |
| ATOM 1620 | CB | VAL241 | 20.103 | 31.056 | 38.948 | 1.00 | 43.93 |
| ATOM 1621 | CG1 | VAL241 | 20.071 | 29.643 | 38.412 | 1.00 | 44.87 |
| ATOM 1622 | CG2 | VAL241 | 19.922 | 31.091 | 40.456 | 1.00 | 40.98 |
| ATOM 1623 | C | VAL241 | 21.358 | 32.182 | 37.126 | 1.00 | 41.83 |
| ATOM 1624 | O | VAL241 | 20.351 | 32.739 | 36.685 | 1.00 | 42.56 |
| ATOM 1625 | N | GLU242 | 22.433 | 31.974 | 36.386 | 1.00 | 39.79 |
| ATOM 1626 | CA | GLU242 | 22.426 | 32.440 | 35.017 | 1.00 | 38.35 |
| ATOM 1627 | CB | GLU242 | 23.841 | 32.438 | 34.435 | 1.00 | 41.38 |
| ATOM 1628 | CG | GLU242 | 24.874 | 33.080 | 35.345 | 1.00 | 43.21 |
| ATOM 1629 | CD | GLU242 | 26.062 | 33.639 | 34.588 | 1.00 | 46.65 |
| ATOM 1630 | OE1 | GLU242 | 26.489 | 33.026 | 33.581 | 1.00 | 46.29 |
| ATOM 1631 | OE2 | GLU242 | 26.581 | 34.694 | 35.014 | 1.00 | 49.23 |
| ATOM 1632 | C | GLU242 | 21.495 | 31.626 | 34.144 | 1.00 | 34.71 |
| ATOM 1633 | O | GLU242 | 21.135 | 32.057 | 33.054 | 1.00 | 33.08 |
| ATOM 1634 | N | LEU243 | 21.085 | 30.456 | 34.612 | 1.00 | 31.90 |
| ATOM 1635 | CA | LEU243 | 20.194 | 29.652 | 33.794 | 1.00 | 30.72 |
| ATOM 1636 | CB | LEU243 | 20.125 | 28.214 | 34.285 | 1.00 | 29.40 |
| ATOM 1637 | CG | LEU243 | 21.244 | 27.279 | 33.833 | 1.00 | 28.38 |
| ATOM 1638 | CD1 | LEU243 | 21.264 | 27.192 | 32.321 | 1.00 | 23.84 |
| ATOM 1639 | CD2 | LEU243 | 22.570 | 27.786 | 34.381 | 1.00 | 31.28 |
| ATOM 1640 | C | LEU243 | 18.799 | 30.222 | 33.763 | 1.00 | 31.18 |
| ATOM 1641 | O | LEU243 | 18.143 | 30.153 | 32.729 | 1.00 | 32.86 |
| ATOM 1642 | N | VAL244 | 18.350 | 30.779 | 34.887 | 1.00 | 30.11 |
| ATOM 1643 | CA | VAL244 | 17.011 | 31.361 | 34.979 | 1.00 | 30.23 |
| ATOM 1644 | CB | VAL244 | 16.549 | 31.527 | 36.432 | 1.00 | 31.77 |
| ATOM 1645 | CG1 | VAL244 | 15.085 | 31.981 | 36.444 | 1.00 | 31.84 |
| ATOM 1646 | CG2 | VAL244 | 16.748 | 30.234 | 37.213 | 1.00 | 31.59 |
| ATOM 1647 | C | VAL244 | 16.955 | 32.746 | 34.361 | 1.00 | 30.94 |
| ATOM 1648 | O | VAL244 | 17.919 | 33.499 | 34.458 | 1.00 | 31.77 |
| ATOM 1649 | N | GLU245 | 15.819 | 33.083 | 33.753 | 1.00 | 32.44 |
| ATOM 1650 | CA | GLU245 | 15.625 | 34.389 | 33.125 | 1.00 | 36.05 |
| ATOM 1651 | CB | GLU245 | 14.384 | 34.384 | 32.237 | 1.00 | 35.98 |
| ATOM 1652 | CG | GLU245 | 14.542 | 35.203 | 30.981 | 1.00 | 38.72 |
| ATOM 1653 | CD | GLU245 | 15.357 | 34.449 | 29.959 | 1.00 | 41.52 |
| ATOM 1654 | OE1 | GLU245 | 15.957 | 33.428 | 30.356 | 1.00 | 40.02 |
| ATOM 1655 | OE2 | GLU245 | 15.402 | 34.859 | 28.776 | 1.00 | 43.26 |
| ATOM 1656 | C | GLU245 | 15.453 | 35.511 | 34.149 | 1.00 | 39.49 |
| ATOM 1657 | O | GLU245 | 15.995 | 36.603 | 33.978 | 1.00 | 39.69 |
| ATOM 1658 | N | GLY246 | 14.676 | 35.239 | 35.197 | 1.00 | 42.62 |
| ATOM 1659 | CA | GLY246 | 14.417 | 36.228 | 36.233 | 1.00 | 44.14 |
| ATOM 1660 | C | GLY246 | 15.642 | 36.762 | 36.953 | 1.00 | 44.54 |
| ATOM 1661 | O | GLY246 | 16.720 | 36.163 | 36.906 | 1.00 | 43.59 |
| ATOM 1662 | N | ASP247 | 15.476 | 37.896 | 37.627 | 1.00 | 44.51 |
| ATOM 1663 | CA | ASP247 | 16.582 | 38.500 | 38.345 | 1.00 | 45.26 |
| ATOM 1664 | CB | ASP247 | 17.179 | 39.654 | 37.540 | 1.00 | 48.06 |
| ATOM 1665 | CG | ASP247 | 18.102 | 39.173 | 36.436 | 1.00 | 52.60 |
| ATOM 1666 | OD1 | ASP247 | 19.016 | 38.376 | 36.744 | 1.00 | 54.76 |
| ATOM 1667 | OD2 | ASP247 | 17.923 | 39.584 | 35.265 | 1.00 | 54.15 |

[Table 2] (continued)

| | | | | | | | |
|---|---|---|---|---|---|---|---|
| ATOM 1668 | C | ASP247 | 16.213 | 38.993 | 39.720 | 1.00 | 44.83 |
| ATOM 1669 | O | ASP247 | 17.087 | 39.306 | 40.518 | 1.00 | 45.80 |
| ATOM 1670 | N | GLU248 | 14.930 | 39.064 | 40.022 | 1.00 | 44.56 |
| ATOM 1671 | CA | GLU248 | 14.561 | 39.546 | 41.336 | 1.00 | 45.70 |
| ATOM 1672 | CB | GLU248 | 13.610 | 40.727 | 41.206 | 1.00 | 50.66 |
| ATOM 1673 | CG | GLU248 | 12.441 | 40.458 | 40.298 | 1.00 | 60.84 |
| ATOM 1674 | CD | GLU248 | 11.394 | 41.556 | 40.355 | 1.00 | 67.29 |
| ATOM 1675 | OE1 | GLU248 | 10.742 | 41.702 | 41.414 | 1.00 | 69.90 |
| ATOM 1676 | OE2 | GLU248 | 11.223 | 42.273 | 39.340 | 1.00 | 71.41 |
| ATOM 1677 | C | GLU248 | 13.952 | 38.482 | 42.224 | 1.00 | 43.15 |
| ATOM 1678 | O | GLU248 | 12.986 | 37.827 | 41.855 | 1.00 | 42.29 |
| ATOM 1679 | N | GLY249 | 14.530 | 38.315 | 43.404 | 1.00 | 42.35 |
| ATOM 1680 | CA | GLY249 | 14.023 | 37.327 | 44.330 | 1.00 | 42.91 |
| ATOM 1681 | C | GLY249 | 15.044 | 36.247 | 44.625 | 1.00 | 43.93 |
| ATOM 1682 | O | GLY249 | 16.177 | 36.294 | 44.145 | 1.00 | 43.62 |
| ATOM 1683 | N | ARG250 | 14.644 | 35.267 | 45.427 | 1.00 | 43.38 |
| ATOM 1684 | CA | ARG250 | 15.526 | 34.160 | 45.781 | 1.00 | 41.04 |
| ATOM 1685 | CB | ARG250 | 15.819 | 34.207 | 47.293 | 1.00 | 42.27 |
| ATOM 1686 | CG | ARG250 | 14.745 | 34.934 | 48.114 | 1.00 | 46.82 |
| ATOM 1687 | CD | ARG250 | 15.139 | 35.142 | 49.584 | 1.00 | 51.21 |
| ATOM 1688 | NE | ARG250 | 16.425 | 35.828 | 49.730 | 1.00 | 55.52 |
| ATOM 1689 | CZ | ARG250 | 16.864 | 36.394 | 50.855 | 1.00 | 55.63 |
| ATOM 1690 | NH1 | ARG250 | 16.121 | 36.375 | 51.956 | 1.00 | 55.05 |
| ATOM 1691 | NH2 | ARG250 | 18.063 | 36.962 | 50.885 | 1.00 | 54.32 |
| ATOM 1692 | C | ARG250 | 14.905 | 32.812 | 45.359 | 1.00 | 38.25 |
| ATOM 1693 | O | ARG250 | 13.681 | 32.640 | 45.394 | 1.00 | 37.44 |
| ATOM 1694 | N | MET251 | 15.760 | 31.880 | 44.932 | 1.00 | 33.58 |
| ATOM 1695 | CA | MET251 | 15.352 | 30.543 | 44.492 | 1.00 | 29.34 |
| ATOM 1696 | CB | MET251 | 15.326 | 30.471 | 42.966 | 1.00 | 24.54 |
| ATOM 1697 | CG | MET251 | 15.180 | 29.069 | 42.379 | 1.00 | 17.89 |
| ATOM 1698 | SD | MET251 | 14.994 | 29.090 | 40.552 | 1.00 | 18.23 |
| ATOM 1699 | CE | MET251 | 16.329 | 28.087 | 40.075 | 1.00 | 12.48 |
| ATOM 1700 | C | MET251 | 16.316 | 29.481 | 45.004 | 1.00 | 30.48 |
| ATOM 1701 | O | MET251 | 17.529 | 29.640 | 44.895 | 1.00 | 31.49 |
| ATOM 1702 | N | CYS252 | 15.775 | 28.392 | 45.546 | 1.00 | 29.56 |
| ATOM 1703 | CA | CYS252 | 16.599 | 27.298 | 46.059 | 1.00 | 26.54 |
| ATOM 1704 | CB | CYS252 | 15.710 | 26.185 | 46.612 | 1.00 | 27.29 |
| ATOM 1705 | SG | CYS252 | 16.613 | 24.659 | 46.927 | 1.00 | 29.14 |
| ATOM 1706 | C | CYS252 | 17.492 | 26.704 | 44.975 | 1.00 | 23.38 |
| ATOM 1707 | O | CYS252 | 17.104 | 26.639 | 43.816 | 1.00 | 22.79 |
| ATOM 1708 | N | VAL253 | 18.688 | 26.268 | 45.349 | 1.00 | 20.80 |
| ATOM 1709 | CA | VAL253 | 19.584 | 25.660 | 44.377 | 1.00 | 20.25 |
| ATOM 1710 | CB | VAL253 | 20.740 | 26.583 | 43.969 | 1.00 | 19.02 |
| ATOM 1711 | CG1 | VAL253 | 21.623 | 25.881 | 42.936 | 1.00 | 15.42 |
| ATOM 1712 | CG2 | VAL253 | 20.198 | 27.866 | 43.411 | 1.00 | 19.77 |
| ATOM 1713 | C | VAL253 | 20.191 | 24.374 | 44.900 | 1.00 | 22.35 |
| ATOM 1714 | O | VAL253 | 20.705 | 24.305 | 46.023 | 1.00 | 22.21 |
| ATOM 1715 | N | ASN254 | 20.127 | 23.352 | 44.060 | 1.00 | 24.23 |
| ATOM 1716 | CA | ASN254 | 20.661 | 22.045 | 44.390 | 1.00 | 22.10 |
| ATOM 1717 | CB | ASN254 | 19.860 | 20.975 | 43.647 | 1.00 | 21.49 |
| ATOM 1718 | CG | ASN254 | 20.479 | 19.604 | 43.747 | 1.00 | 22.93 |

[Table 2] (continued)

| | | | | | | | |
|---|---|---|---|---|---|---|---|
| ATOM 1719 | OD1 | ASN254 | 21.074 | 19.232 | 44.764 | 1.00 | 20.03 |
| ATOM 1720 | ND2 | ASN254 | 20.325 | 18.827 | 42.687 | 1.00 | 26.40 |
| ATOM 1721 | C | ASN254 | 22.124 | 22.046 | 43.975 | 1.00 | 19.26 |
| ATOM 1722 | O | ASN254 | 22.454 | 22.155 | 42.795 | 1.00 | 15.88 |
| ATOM 1723 | N | THR255 | 23.001 | 21.949 | 44.961 | 1.00 | 15.23 |
| ATOM 1724 | CA | THR255 | 24.428 | 21.962 | 44.691 | 1.00 | 15.03 |
| ATOM 1725 | CB | THR255 | 25.193 | 22.217 | 45.944 | 1.00 | 13.56 |
| ATOM 1726 | OG1 | THR255 | 25.035 | 21.087 | 46.808 | 1.00 | 14.56 |
| ATOM 1727 | CG2 | THR255 | 24.670 | 23.458 | 46.617 | 1.00 | 14.18 |
| ATOM 1728 | C | THR255 | 24.957 | 20.665 | 44.127 | 1.00 | 15.21 |
| ATOM 1729 | O | THR255 | 25.675 | 20.647 | 43.126 | 1.00 | 12.07 |
| ATOM 1730 | N | GLU256 | 24.594 | 19.570 | 44.777 | 1.00 | 18.83 |
| ATOM 1731 | CA | GLU256 | 25.076 | 18.268 | 44.355 | 1.00 | 22.28 |
| ATOM 1732 | CB | GLU256 | 24.795 | 18.025 | 42.876 | 1.00 | 25.93 |
| ATOM 1733 | CG | GLU256 | 23.377 | 18.345 | 42.454 | 1.00 | 31.90 |
| ATOM 1734 | CD | GLU256 | 22.500 | 17.121 | 42.336 | 1.00 | 34.74 |
| ATOM 1735 | OE1 | GLU256 | 22.191 | 16.510 | 43.386 | 1.00 | 36.97 |
| ATOM 1736 | OE2 | GLU256 | 22.122 | 16.777 | 41.188 | 1.00 | 35.26 |
| ATOM 1737 | C | GLU256 | 26.562 | 18.402 | 44.559 | 1.00 | 21.32 |
| ATOM 1738 | O | GLU256 | 27.359 | 18.032 | 43.701 | 1.00 | 23.09 |
| ATOM 1739 | N | TRP257 | 26.931 | 18.966 | 45.699 | 1.00 | 17.36 |
| ATOM 1740 | CA | TRP257 | 28.327 | 19.141 | 45.985 | 1.00 | 14.83 |
| ATOM 1741 | CB | TRP257 | 28.514 | 20.074 | 47.176 | 1.00 | 11.59 |
| ATOM 1742 | CG | TRP257 | 28.038 | 19.561 | 48.478 | 1.00 | 8.69 |
| ATOM 1743 | CD2 | TRP257 | 27.830 | 20.332 | 49.676 | 1.00 | 9.05 |
| ATOM 1744 | CE2 | TRP257 | 27.562 | 19.410 | 50.715 | 1.00 | 7.00 |
| ATOM 1745 | CE3 | TRP257 | 27.845 | 21.703 | 49.964 | 1.00 | 7.18 |
| ATOM 1746 | CD1 | TRP257 | 27.881 | 18.265 | 48.827 | 1.00 | 7.58 |
| ATOM 1747 | NE1 | TRP257 | 27.602 | 18.163 | 50.172 | 1.00 | 7.99 |
| ATOM 1748 | CZ2 | TRP257 | 27.325 | 19.818 | 52.038 | 1.00 | 4.73 |
| ATOM 1749 | CZ3 | TRP257 | 27.605 | 22.108 | 51.280 | 1.00 | 7.12 |
| ATOM 1750 | CH2 | TRP257 | 27.346 | 21.164 | 52.300 | 1.00 | 5.47 |
| ATOM 1751 | C | TRP257 | 29.033 | 17.813 | 46.224 | 1.00 | 17.81 |
| ATOM 1752 | O | TRP257 | 30.221 | 17.776 | 46.523 | 1.00 | 19.44 |
| ATOM 1753 | N | GLY258 | 28.318 | 16.708 | 46.099 | 1.00 | 21.88 |
| ATOM 1754 | CA | GLY258 | 28.991 | 15.444 | 46.303 | 1.00 | 23.25 |
| ATOM 1755 | C | GLY258 | 30.137 | 15.303 | 45.316 | 1.00 | 23.01 |
| ATOM 1756 | O | GLY258 | 31.133 | 14.629 | 45.600 | 1.00 | 21.92 |
| ATOM 1757 | N | ALA259 | 29.997 | 15.943 | 44.156 | 1.00 | 23.11 |
| ATOM 1758 | CA | ALA259 | 31.015 | 15.863 | 43.113 | 1.00 | 27.74 |
| ATOM 1759 | CB | ALA259 | 30.400 | 16.139 | 41.766 | 1.00 | 27.03 |
| ATOM 1760 | C | ALA259 | 32.176 | 16.806 | 43.335 | 1.00 | 30.23 |
| ATOM 1761 | O | ALA259 | 33.178 | 16.748 | 42.622 | 1.00 | 32.12 |
| ATOM 1762 | N | PHE260 | 32.041 | 17.680 | 44.320 | 1.00 | 32.43 |
| ATOM 1763 | CA | PHE260 | 33.093 | 18.627 | 44.611 | 1.00 | 36.43 |
| ATOM 1764 | CB | PHE260 | 32.804 | 19.343 | 45.924 | 1.00 | 39.42 |
| ATOM 1765 | CG | PHE260 | 33.932 | 20.206 | 46.411 | 1.00 | 43.92 |
| ATOM 1766 | CD1 | PHE260 | 34.660 | 21.003 | 45.534 | 1.00 | 46.49 |
| ATOM 1767 | CD2 | PHE260 | 34.232 | 20.263 | 47.765 | 1.00 | 45.64 |
| ATOM 1768 | CE1 | PHE260 | 35.672 | 21.835 | 46.002 | 1.00 | 47.73 |
| ATOM 1769 | CE2 | PHE260 | 35.242 | 21.093 | 48.242 | 1.00 | 46.62 |

[Table 2] (continued)

| | | | | | | | |
|---|---|---|---|---|---|---|---|
| ATOM 1770 | CZ | PHE260 | 35.958 | 21.882 | 47.360 | 1.00 | 47.27 |
| ATOM 1771 | C | PHE260 | 34.412 | 17.897 | 44.695 | 1.00 | 39.39 |
| ATOM 1772 | O | PHE260 | 34.495 | 16.800 | 45.243 | 1.00 | 40.20 |
| ATOM 1773 | N | GLY261 | 35.441 | 18.511 | 44.127 | 1.00 | 41.71 |
| ATOM 1774 | CA | GLY261 | 36.753 | 17.911 | 44.152 | 1.00 | 43.62 |
| ATOM 1775 | C | GLY261 | 36.967 | 16.857 | 43.090 | 1.00 | 44.99 |
| ATOM 1776 | O | GLY261 | 38.049 | 16.282 | 43.015 | 1.00 | 47.22 |
| ATOM 1777 | N | ASP262 | 35.961 | 16.578 | 42.270 | 1.00 | 46.06 |
| ATOM 1778 | CA | ASP262 | 36.143 | 15.574 | 41.229 | 1.00 | 47.68 |
| ATOM 1779 | CB | ASP262 | 34.800 | 15.197 | 40.602 | 1.00 | 50.82 |
| ATOM 1780 | CG | ASP262 | 34.024 | 14.187 | 41.445 | 1.00 | 53.64 |
| ATOM 1781 | OD1 | ASP262 | 32.815 | 13.996 | 41.191 | 1.00 | 54.63 |
| ATOM 1782 | OD2 | ASP262 | 34.624 | 13.578 | 42.356 | 1.00 | 54.71 |
| ATOM 1783 | C | ASP262 | 37.089 | 16.129 | 40.177 | 1.00 | 47.19 |
| ATOM 1784 | O | ASP262 | 37.539 | 15.400 | 39.292 | 1.00 | 47.09 |
| ATOM 1785 | N | SER263 | 37.380 | 17.427 | 40.298 | 1.00 | 46.38 |
| ATOM 1786 | CA | SER263 | 38.289 | 18.147 | 39.401 | 1.00 | 44.53 |
| ATOM 1787 | CB | SER263 | 37.651 | 19.445 | 38.903 | 1.00 | 43.57 |
| ATOM 1788 | OG | SER263 | 36.341 | 19.246 | 38.415 | 1.00 | 43.79 |
| ATOM 1789 | C | SER263 | 39.552 | 18.513 | 40.174 | 1.00 | 43.93 |
| ATOM 1790 | O | SER263 | 40.061 | 19.632 | 40.059 | 1.00 | 44.40 |
| ATOM 1791 | N | GLY264 | 40.039 | 17.577 | 40.979 | 1.00 | 43.71 |
| ATOM 1792 | CA | GLY264 | 41.235 | 17.825 | 41.762 | 1.00 | 42.64 |
| ATOM 1793 | C | GLY264 | 41.133 | 18.889 | 42.845 | 1.00 | 40.75 |
| ATOM 1794 | O | GLY264 | 42.052 | 19.012 | 43.648 | 1.00 | 42.90 |
| ATOM 1795 | N | GLU265 | 40.040 | 19.647 | 42.887 | 1.00 | 38.43 |
| ATOM 1796 | CA | GLU265 | 39.881 | 20.700 | 43.893 | 1.00 | 37.42 |
| ATOM 1797 | CB | GLU265 | 38.437 | 21.227 | 43.907 | 1.00 | 39.11 |
| ATOM 1798 | CG | GLU265 | 37.986 | 21.928 | 42.632 | 1.00 | 40.76 |
| ATOM 1799 | CD | GLU265 | 37.198 | 21.023 | 41.701 | 1.00 | 43.56 |
| ATOM 1800 | OE1 | GLU265 | 36.904 | 21.461 | 40.565 | 1.00 | 45.26 |
| ATOM 1801 | OE2 | GLU265 | 36.863 | 19.883 | 42.099 | 1.00 | 42.42 |
| ATOM 1802 | C | GLU265 | 40.266 | 20.299 | 45.321 | 1.00 | 36.38 |
| ATOM 1803 | O | GLU265 | 40.410 | 21.160 | 46.185 | 1.00 | 33.59 |
| ATOM 1804 | N | LEU266 | 40.425 | 19.004 | 45.573 | 1.00 | 37.71 |
| ATOM 1805 | CA | LEU266 | 40.783 | 18.534 | 46.912 | 1.00 | 40.56 |
| ATOM 1806 | CB | LEU266 | 39.597 | 17.831 | 47.567 | 1.00 | 40.03 |
| ATOM 1807 | CG | LEU266 | 38.371 | 18.631 | 48.001 | 1.00 | 40.79 |
| ATOM 1808 | CD1 | LEU266 | 37.234 | 17.673 | 48.259 | 1.00 | 40.27 |
| ATOM 1809 | CD2 | LEU266 | 38.677 | 19.432 | 49.253 | 1.00 | 41.81 |
| ATOM 1810 | C | LEU266 | 41.949 | 17.563 | 46.880 | 1.00 | 43.51 |
| ATOM 1811 | O | LEU266 | 42.363 | 17.045 | 47.919 | 1.00 | 43.63 |
| ATOM 1812 | N | ASP267 | 42.475 | 17.324 | 45.682 | 1.00 | 47.00 |
| ATOM 1813 | CA | ASP267 | 43.584 | 16.393 | 45.480 | 1.00 | 48.18 |
| ATOM 1814 | CB | ASP267 | 44.222 | 16.622 | 44.097 | 1.00 | 50.89 |
| ATOM 1815 | CG | ASP267 | 44.982 | 15.391 | 43.584 | 1.00 | 54.98 |
| ATOM 1816 | OD1 | ASP267 | 45.239 | 15.317 | 42.360 | 1.00 | 56.65 |
| ATOM 1817 | OD2 | ASP267 | 45.328 | 14.499 | 44.398 | 1.00 | 55.43 |
| ATOM 1818 | C | ASP267 | 44.659 | 16.440 | 46.571 | 1.00 | 46.46 |
| ATOM 1819 | O | ASP267 | 45.205 | 15.397 | 46.960 | 1.00 | 45.37 |
| ATOM 1820 | N | GLU268 | 44.957 | 17.630 | 47.084 | 1.00 | 44.63 |

[Table 2] (continued)

| | | | | | | | |
|---|---|---|---|---|---|---|---|
| ATOM 1821 | CA | GLU268 | 45.990 | 17.721 | 48.109 | 1.00 | 44.67 |
| ATOM 1822 | CB | GLU268 | 46.805 | 19.024 | 47.956 | 1.00 | 44.68 |
| ATOM 1823 | CG | GLU268 | 46.508 | 20.163 | 48.934 | 1.00 | 43.60 |
| ATOM 1824 | CD | GLU268 | 45.234 | 20.915 | 48.613 | 1.00 | 43.53 |
| ATOM 1825 | OE1 | GLU268 | 45.020 | 21.258 | 47.423 | 1.00 | 42.43 |
| ATOM 1826 | OE2 | GLU268 | 44.461 | 21.174 | 49.561 | 1.00 | 40.84 |
| ATOM 1827 | C | GLU268 | 45.457 | 17.569 | 49.528 | 1.00 | 43.45 |
| ATOM 1828 | O | GLU268 | 46.102 | 17.961 | 50.499 | 1.00 | 46.29 |
| ATOM 1829 | N | PHE269 | 44.286 | 16.971 | 49.656 | 1.00 | 38.78 |
| ATOM 1830 | CA | PHE269 | 43.729 | 16.785 | 50.974 | 1.00 | 33.75 |
| ATOM 1831 | CB | PHE269 | 42.480 | 17.614 | 51.135 | 1.00 | 33.69 |
| ATOM 1832 | CG | PHE269 | 42.733 | 18.990 | 51.639 | 1.00 | 34.75 |
| ATOM 1833 | CD1 | PHE269 | 43.435 | 19.193 | 52.822 | 1.00 | 36.51 |
| ATOM 1834 | CD2 | PHE269 | 42.161 | 20.079 | 51.001 | 1.00 | 34.78 |
| ATOM 1835 | CE1 | PHE269 | 43.548 | 20.469 | 53.365 | 1.00 | 37.39 |
| ATOM 1836 | CE2 | PHE269 | 42.266 | 21.354 | 51.532 | 1.00 | 35.15 |
| ATOM 1837 | CZ | PHE269 | 42.955 | 21.551 | 52.717 | 1.00 | 37.68 |
| ATOM 1838 | C | PHE269 | 43.405 | 15.343 | 51.225 | 1.00 | 32.83 |
| ATOM 1839 | O | PHE269 | 43.206 | 14.952 | 52.365 | 1.00 | 31.85 |
| ATOM 1840 | N | LEU270 | 43.355 | 14.555 | 50.157 | 1.00 | 33.85 |
| ATOM 1841 | CA | LEU270 | 43.046 | 13.130 | 50.259 | 1.00 | 34.53 |
| ATOM 1842 | CB | LEU270 | 42.712 | 12.553 | 48.884 | 1.00 | 35.63 |
| ATOM 1843 | CG | LEU270 | 41.326 | 12.857 | 48.321 | 1.00 | 37.61 |
| ATOM 1844 | CD1 | LEU270 | 41.323 | 14.293 | 47.842 | 1.00 | 35.85 |
| ATOM 1845 | CD2 | LEU270 | 40.966 | 11.878 | 47.177 | 1.00 | 37.50 |
| ATOM 1846 | C | LEU270 | 44.172 | 12.298 | 50.845 | 1.00 | 33.68 |
| ATOM 1847 | O | LEU270 | 45.334 | 12.640 | 50.695 | 1.00 | 35.64 |
| ATOM 1848 | N | LEU271 | 43.829 | 11.200 | 51.507 | 1.00 | 33.66 |
| ATOM 1849 | CA | LEU271 | 44.850 | 10.324 | 52.059 | 1.00 | 34.55 |
| ATOM 1850 | CB | LEU271 | 44.610 | 10.032 | 53.519 | 1.00 | 30.63 |
| ATOM 1851 | CG | LEU271 | 44.870 | 11.238 | 54.383 | 1.00 | 29.49 |
| ATOM 1852 | CD1 | LEU271 | 43.855 | 12.324 | 54.075 | 1.00 | 27.82 |
| ATOM 1853 | CD2 | LEU271 | 44.783 | 10.798 | 55.824 | 1.00 | 31.04 |
| ATOM 1854 | C | LEU271 | 44.884 | 9.010 | 51.324 | 1.00 | 37.04 |
| ATOM 1855 | O | LEU271 | 44.009 | 8.715 | 50.513 | 1.00 | 36.79 |
| ATOM 1856 | N | GLU272 | 45.890 | 8.209 | 51.638 | 1.00 | 40.66 |
| ATOM 1857 | CA | GLU272 | 46.052 | 6.927 | 50.989 | 1.00 | 44.99 |
| ATOM 1858 | CB | GLU272 | 47.256 | 6.182 | 51.590 | 1.00 | 51.18 |
| ATOM 1859 | CG | GLU272 | 47.124 | 5.781 | 53.075 | 1.00 | 58.46 |
| ATOM 1860 | CD | GLU272 | 48.371 | 5.077 | 53.641 | 1.00 | 62.56 |
| ATOM 1861 | OE1 | GLU272 | 49.393 | 5.772 | 53.876 | 1.00 | 64.96 |
| ATOM 1862 | OE2 | GLU272 | 48.325 | 3.835 | 53.849 | 1.00 | 61.73 |
| ATOM 1863 | C | GLU272 | 44.789 | 6.080 | 51.092 | 1.00 | 44.62 |
| ATOM 1864 | O | GLU272 | 44.377 | 5.452 | 50.116 | 1.00 | 44.50 |
| ATOM 1865 | N | TYR273 | 44.163 | 6.079 | 52.266 | 1.00 | 43.42 |
| ATOM 1866 | CA | TYR273 | 42.955 | 5.284 | 52.486 | 1.00 | 40.23 |
| ATOM 1867 | CB | TYR273 | 42.537 | 5.377 | 53.958 | 1.00 | 38.82 |
| ATOM 1868 | CG | TYR273 | 43.709 | 5.401 | 54.923 | 1.00 | 36.38 |
| ATOM 1869 | CD1 | TYR273 | 44.126 | 6.602 | 55.505 | 1.00 | 35.57 |
| ATOM 1870 | CE1 | TYR273 | 45.210 | 6.647 | 56.380 | 1.00 | 34.95 |
| ATOM 1871 | CD2 | TYR273 | 44.413 | 4.231 | 55.243 | 1.00 | 35.34 |

[Table 2] (continued)

| | | | | | | | |
|---|---|---|---|---|---|---|---|
| ATOM 1872 | CE2 | TYR273 | 45.509 | 4.264 | 56.122 | 1.00 | 34.05 |
| ATOM 1873 | CZ | TYR273 | 45.897 | 5.481 | 56.685 | 1.00 | 34.66 |
| ATOM 1874 | OH | TYR273 | 46.966 | 5.556 | 57.550 | 1.00 | 33.77 |
| ATOM 1875 | C | TYR273 | 41.826 | 5.749 | 51.567 | 1.00 | 38.50 |
| ATOM 1876 | O | TYR273 | 41.264 | 4.967 | 50.804 | 1.00 | 35.21 |
| ATOM 1877 | N | ASP274 | 41.507 | 7.030 | 51.638 | 1.00 | 38.17 |
| ATOM 1878 | CA | ASP274 | 40.473 | 7.579 | 50.796 | 1.00 | 40.03 |
| ATOM 1879 | CB | ASP274 | 40.470 | 9.083 | 50.929 | 1.00 | 41.17 |
| ATOM 1880 | CG | ASP274 | 40.252 | 9.512 | 52.341 | 1.00 | 43.77 |
| ATOM 1881 | OD1 | ASP274 | 39.123 | 9.327 | 52.839 | 1.00 | 46.59 |
| ATOM 1882 | OD2 | ASP274 | 41.212 | 10.010 | 52.958 | 1.00 | 44.41 |
| ATOM 1883 | C | ASP274 | 40.740 | 7.200 | 49.359 | 1.00 | 40.92 |
| ATOM 1884 | O | ASP274 | 39.819 | 6.937 | 48.595 | 1.00 | 41.41 |
| ATOM 1885 | N | ARG275 | 42.007 | 7.160 | 48.984 | 1.00 | 42.93 |
| ATOM 1886 | CA | ARG275 | 42.333 | 6.819 | 47.613 | 1.00 | 45.81 |
| ATOM 1887 | CB | ARG275 | 43.831 | 6.993 | 47.365 | 1.00 | 49.53 |
| ATOM 1888 | CG | ARG275 | 44.191 | 7.563 | 45.995 | 1.00 | 53.24 |
| ATOM 1889 | CD | ARG275 | 45.702 | 7.772 | 45.886 | 1.00 | 58.85 |
| ATOM 1890 | NE | ARG275 | 46.213 | 8.663 | 46.933 | 1.00 | 62.67 |
| ATOM 1891 | CZ | ARG275 | 47.088 | 8.308 | 47.876 | 1.00 | 62.82 |
| ATOM 1892 | NH1 | ARG275 | 47.571 | 7.068 | 47.922 | 1.00 | 61.28 |
| ATOM 1893 | NH2 | ARG275 | 47.476 | 9.201 | 48.777 | 1.00 | 61.64 |
| ATOM 1894 | C | ARG275 | 41.901 | 5.390 | 47.316 | 1.00 | 46.01 |
| ATOM 1895 | O | ARG275 | 41.134 | 5.160 | 46.382 | 1.00 | 45.19 |
| ATOM 1896 | N | LEU276 | 42.382 | 4.437 | 48.113 | 1.00 | 47.51 |
| ATOM 1897 | CA | LEU276 | 42.026 | 3.030 | 47.922 | 1.00 | 48.68 |
| ATOM 1898 | CB | LEU276 | 42.460 | 2.197 | 49.134 | 1.00 | 45.63 |
| ATOM 1899 | CG | LEU276 | 43.971 | 1.999 | 49.287 | 1.00 | 43.28 |
| ATOM 1900 | CD1 | LEU276 | 44.418 | 2.379 | 50.686 | 1.00 | 42.53 |
| ATOM 1901 | CD2 | LEU276 | 44.321 | 0.557 | 48.994 | 1.00 | 42.97 |
| ATOM 1902 | C | LEU276 | 40.520 | 2.915 | 47.718 | 1.00 | 51.24 |
| ATOM 1903 | O | LEU276 | 40.050 | 2.133 | 46.891 | 1.00 | 52.38 |
| ATOM 1904 | N | VAL277 | 39.772 | 3.710 | 48.475 | 1.00 | 53.11 |
| ATOM 1905 | CA | VAL277 | 38.321 | 3.722 | 48.372 | 1.00 | 54.05 |
| ATOM 1906 | CB | VAL277 | 37.703 | 4.640 | 49.423 | 1.00 | 52.84 |
| ATOM 1907 | CG1 | VAL277 | 36.210 | 4.682 | 49.249 | 1.00 | 52.71 |
| ATOM 1908 | CG2 | VAL277 | 38.069 | 4.156 | 50.804 | 1.00 | 54.87 |
| ATOM 1909 | C | VAL277 | 37.906 | 4.231 | 46.999 | 1.00 | 55.80 |
| ATOM 1910 | O | VAL277 | 37.381 | 3.474 | 46.185 | 1.00 | 57.15 |
| ATOM 1911 | N | ASP278 | 38.146 | 5.518 | 46.754 | 1.00 | 56.71 |
| ATOM 1912 | CA | ASP278 | 37.804 | 6.146 | 45.481 | 1.00 | 57.65 |
| ATOM 1913 | CB | ASP278 | 38.479 | 7.514 | 45.353 | 1.00 | 59.73 |
| ATOM 1914 | CG | ASP278 | 38.243 | 8.163 | 43.989 | 1.00 | 61.93 |
| ATOM 1915 | OD1 | ASP278 | 38.990 | 9.110 | 43.642 | 1.00 | 61.47 |
| ATOM 1916 | OD2 | ASP278 | 37.308 | 7.733 | 43.273 | 1.00 | 62.11 |
| ATOM 1917 | C | ASP278 | 38.263 | 5.281 | 44.328 | 1.00 | 58.14 |
| ATOM 1918 | O | ASP278 | 37.645 | 5.271 | 43.266 | 1.00 | 58.75 |
| ATOM 1919 | N | GLU279 | 39.358 | 4.563 | 44.538 | 1.00 | 58.33 |
| ATOM 1920 | CA | GLU279 | 39.900 | 3.710 | 43.498 | 1.00 | 59.14 |
| ATOM 1921 | CB | GLU279 | 41.437 | 3.808 | 43.477 | 1.00 | 60.99 |
| ATOM 1922 | CG | GLU279 | 41.978 | 5.219 | 43.178 | 1.00 | 61.92 |

[Table 2] (continued)

| | | | | | | | |
|---|---|---|---|---|---|---|---|
| ATOM 1923 | CD | GLU279 | 43.497 | 5.276 | 43.014 | 1.00 | 60.92 |
| ATOM 1924 | OE1 | GLU279 | 44.219 | 4.874 | 43.953 | 1.00 | 60.85 |
| ATOM 1925 | OE2 | GLU279 | 43.965 | 5.733 | 41.946 | 1.00 | 58.99 |
| ATOM 1926 | C | GLU279 | 39.467 | 2.261 | 43.664 | 1.00 | 58.04 |
| ATOM 1927 | O | GLU279 | 40.196 | 1.346 | 43.298 | 1.00 | 59.38 |
| ATOM 1928 | N | SER280 | 38.283 | 2.044 | 44.219 | 1.00 | 57.21 |
| ATOM 1929 | CA | SER280 | 37.798 | 0.679 | 44.390 | 1.00 | 56.55 |
| ATOM 1930 | CB | SER280 | 38.283 | 0.091 | 45.719 | 1.00 | 56.66 |
| ATOM 1931 | OG | SER280 | 38.015 | -1.298 | 45.774 | 1.00 | 54.41 |
| ATOM 1932 | C | SER280 | 36.282 | 0.671 | 44.334 | 1.00 | 55.29 |
| ATOM 1933 | O | SER280 | 35.640 | -0.371 | 44.472 | 1.00 | 53.68 |
| ATOM 1934 | N | SER281 | 35.725 | 1.854 | 44.113 | 1.00 | 54.58 |
| ATOM 1935 | CA | SER281 | 34.288 | 2.038 | 44.020 | 1.00 | 55.36 |
| ATOM 1936 | CB | SER281 | 33.919 | 3.451 | 44.464 | 1.00 | 56.89 |
| ATOM 1937 | OG | SER281 | 34.565 | 4.415 | 43.649 | 1.00 | 56.89 |
| ATOM 1938 | C | SER281 | 33.843 | 1.832 | 42.584 | 1.00 | 54.80 |
| ATOM 1939 | O | SER281 | 34.652 | 1.905 | 41.664 | 1.00 | 55.85 |
| ATOM 1940 | N | ALA282 | 32.553 | 1.587 | 42.389 | 1.00 | 53.75 |
| ATOM 1941 | CA | ALA282 | 32.025 | 1.379 | 41.050 | 1.00 | 52.42 |
| ATOM 1942 | CB | ALA282 | 30.626 | 0.809 | 41.133 | 1.00 | 52.26 |
| ATOM 1943 | C | ALA282 | 32.012 | 2.679 | 40.250 | 1.00 | 51.83 |
| ATOM 1944 | O | ALA282 | 31.632 | 2.685 | 39.081 | 1.00 | 52.27 |
| ATOM 1945 | N | ASN283 | 32.441 | 3.772 | 40.879 | 1.00 | 50.19 |
| ATOM 1946 | CA | ASN283 | 32.465 | 5.089 | 40.239 | 1.00 | 47.37 |
| ATOM 1947 | CB | ASN283 | 31.338 | 5.945 | 40.790 | 1.00 | 47.04 |
| ATOM 1948 | CG | ASN283 | 31.482 | 6.191 | 42.276 | 1.00 | 47.38 |
| ATOM 1949 | OD1 | ASN283 | 31.584 | 5.255 | 43.068 | 1.00 | 46.86 |
| ATOM 1950 | ND2 | ASN283 | 31.497 | 7.455 | 42.662 | 1.00 | 49.96 |
| ATOM 1951 | C | ASN283 | 33.777 | 5.806 | 40.513 | 1.00 | 46.64 |
| ATOM 1952 | O | ASN283 | 33.783 | 6.894 | 41.081 | 1.00 | 48.74 |
| ATOM 1953 | N | PRO284 | 34.905 | 5.214 | 40.110 | 1.00 | 45.15 |
| ATOM 1954 | CD | PRO284 | 35.028 | 3.896 | 39.462 | 1.00 | 44.41 |
| ATOM 1955 | CA | PRO284 | 36.227 | 5.814 | 40.327 | 1.00 | 43.24 |
| ATOM 1956 | CB | PRO284 | 37.151 | 4.855 | 39.583 | 1.00 | 44.66 |
| ATOM 1957 | CG | PRO284 | 36.459 | 3.532 | 39.756 | 1.00 | 44.93 |
| ATOM 1958 | C | PRO284 | 36.389 | 7.267 | 39.856 | 1.00 | 41.14 |
| ATOM 1959 | O | PRO284 | 35.978 | 7.624 | 38.755 | 1.00 | 40.17 |
| ATOM 1960 | N | GLY285 | 36.994 | 8.099 | 40.695 | 1.00 | 39.45 |
| ATOM 1961 | CA | GLY285 | 37.208 | 9.484 | 40.321 | 1.00 | 40.34 |
| ATOM 1962 | C | GLY285 | 35.964 | 10.343 | 40.401 | 1.00 | 42.06 |
| ATOM 1963 | O | GLY285 | 36.035 | 11.576 | 40.367 | 1.00 | 43.11 |
| ATOM 1964 | N | GLN286 | 34.811 | 9.699 | 40.510 | 1.00 | 42.34 |
| ATOM 1965 | CA | GLN286 | 33.555 | 10.427 | 40.601 | 1.00 | 41.88 |
| ATOM 1966 | CB | GLN286 | 32.490 | 9.717 | 39.758 | 1.00 | 44.97 |
| ATOM 1967 | CG | GLN286 | 31.973 | 10.544 | 38.588 | 1.00 | 49.89 |
| ATOM 1968 | CD | GLN286 | 31.043 | 11.668 | 39.043 | 1.00 | 54.72 |
| ATOM 1969 | OE1 | GLN286 | 29.911 | 11.419 | 39.483 | 1.00 | 56.09 |
| ATOM 1970 | NE2 | GLN286 | 31.519 | 12.911 | 38.950 | 1.00 | 54.20 |
| ATOM 1971 | C | GLN286 | 33.113 | 10.541 | 42.063 | 1.00 | 40.59 |
| ATOM 1972 | O | GLN286 | 33.396 | 9.660 | 42.879 | 1.00 | 39.39 |
| ATOM 1973 | N | GLN287 | 32.445 | 11.648 | 42.389 | 1.00 | 39.59 |

[Table 2] (continued)

| | | | | | | | |
|---|---|---|---|---|---|---|---|
| ATOM 1974 | CA | GLN287 | 31.939 | 11.913 | 43.741 | 1.00 | 38.06 |
| ATOM 1975 | CB | GLN287 | 30.770 | 10.969 | 44.053 | 1.00 | 37.29 |
| ATOM 1976 | CG | GLN287 | 29.732 | 10.837 | 42.939 | 1.00 | 35.04 |
| ATOM 1977 | CD | GLN287 | 28.912 | 12.100 | 42.736 | 1.00 | 33.74 |
| ATOM 1978 | OE1 | GLN287 | 28.906 | 12.692 | 41.647 | 1.00 | 28.89 |
| ATOM 1979 | NE2 | GLN287 | 28.204 | 12.514 | 43.786 | 1.00 | 31.49 |
| ATOM 1980 | C | GLN287 | 33.015 | 11.744 | 44.820 | 1.00 | 37.30 |
| ATOM 1981 | O | GLN287 | 32.958 | 10.813 | 45.624 | 1.00 | 37.53 |
| ATOM 1982 | N | LEU288 | 33.990 | 12.643 | 44.856 | 1.00 | 34.03 |
| ATOM 1983 | CA | LEU288 | 35.051 | 12.516 | 45.844 | 1.00 | 29.84 |
| ATOM 1984 | CB | LEU288 | 36.351 | 13.071 | 45.293 | 1.00 | 30.50 |
| ATOM 1985 | CG | LEU288 | 37.285 | 11.960 | 44.819 | 1.00 | 32.69 |
| ATOM 1986 | CD1 | LEU288 | 36.645 | 11.102 | 43.728 | 1.00 | 31.90 |
| ATOM 1987 | CD2 | LEU288 | 38.546 | 12.611 | 44.323 | 1.00 | 36.00 |
| ATOM 1988 | C | LEU288 | 34.729 | 13.180 | 47.156 | 1.00 | 26.53 |
| ATOM 1989 | O | LEU288 | 34.991 | 12.627 | 48.218 | 1.00 | 26.76 |
| ATOM 1990 | N | TYR289 | 34.172 | 14.374 | 47.086 | 1.00 | 23.58 |
| ATOM 1991 | CA | TYR289 | 33.809 | 15.074 | 48.292 | 1.00 | 22.36 |
| ATOM 1992 | CB | TYR289 | 33.086 | 16.365 | 47.939 | 1.00 | 20.16 |
| ATOM 1993 | CG | TYR289 | 32.716 | 17.186 | 49.136 | 1.00 | 18.61 |
| ATOM 1994 | CD1 | TYR289 | 33.660 | 17.486 | 50.105 | 1.00 | 18.65 |
| ATOM 1995 | CE1 | TYR289 | 33.347 | 18.269 | 51.195 | 1.00 | 18.34 |
| ATOM 1996 | CD2 | TYR289 | 31.433 | 17.693 | 49.288 | 1.00 | 18.91 |
| ATOM 1997 | CE2 | TYR289 | 31.105 | 18.484 | 50.378 | 1.00 | 18.97 |
| ATOM 1998 | CZ | TYR289 | 32.073 | 18.768 | 51.327 | 1.00 | 20.15 |
| ATOM 1999 | OH | TYR289 | 31.788 | 19.565 | 52.408 | 1.00 | 22.93 |
| ATOM 2000 | C | TYR289 | 32.894 | 14.165 | 49.105 | 1.00 | 25.30 |
| ATOM 2001 | O | TYR289 | 32.991 | 14.106 | 50.337 | 1.00 | 24.21 |
| ATOM 2002 | N | GLU290 | 32.005 | 13.448 | 48.411 | 1.00 | 27.35 |
| ATOM 2003 | CA | GLU290 | 31.071 | 12.532 | 49.084 | 1.00 | 26.68 |
| ATOM 2004 | CB | GLU290 | 30.081 | 11.904 | 48.090 | 1.00 | 26.17 |
| ATOM 2005 | CG | GLU290 | 28.614 | 12.216 | 48.413 | 1.00 | 25.68 |
| ATOM 2006 | CD | GLU290 | 27.617 | 11.404 | 47.591 | 1.00 | 26.93 |
| ATOM 2007 | OE1 | GLU290 | 27.735 | 11.363 | 46.337 | 1.00 | 22.27 |
| ATOM 2008 | OE2 | GLU290 | 26.702 | 10.815 | 48.215 | 1.00 | 27.37 |
| ATOM 2009 | C | GLU290 | 31.838 | 11.425 | 49.781 | 1.00 | 25.75 |
| ATOM 2010 | O | GLU290 | 31.649 | 11.193 | 50.974 | 1.00 | 26.23 |
| ATOM 2011 | N | LYS291 | 32.706 | 10.756 | 49.024 | 1.00 | 24.16 |
| ATOM 2012 | CA | LYS291 | 33.526 | 9.666 | 49.538 | 1.00 | 24.45 |
| ATOM 2013 | CB | LYS291 | 34.342 | 9.063 | 48.408 | 1.00 | 24.19 |
| ATOM 2014 | CG | LYS291 | 33.506 | 8.383 | 47.354 | 1.00 | 28.37 |
| ATOM 2015 | CD | LYS291 | 34.322 | 8.162 | 46.094 | 1.00 | 31.52 |
| ATOM 2016 | CE | LYS291 | 33.533 | 7.434 | 45.030 | 1.00 | 31.16 |
| ATOM 2017 | NZ | LYS291 | 34.367 | 7.299 | 43.813 | 1.00 | 33.55 |
| ATOM 2018 | C | LYS291 | 34.460 | 10.143 | 50.636 | 1.00 | 24.99 |
| ATOM 2019 | O | LYS291 | 35.488 | 9.522 | 50.918 | 1.00 | 25.78 |
| ATOM 2020 | N | LEU292 | 34.095 | 11.254 | 51.255 | 1.00 | 24.20 |
| ATOM 2021 | CA | LEU292 | 34.894 | 11.809 | 52.318 | 1.00 | 25.20 |
| ATOM 2022 | CB | LEU292 | 35.544 | 13.106 | 51.843 | 1.00 | 25.62 |
| ATOM 2023 | CG | LEU292 | 36.904 | 13.450 | 52.464 | 1.00 | 26.59 |
| ATOM 2024 | CD1 | LEU292 | 37.935 | 12.396 | 52.035 | 1.00 | 26.37 |

[Table 2] (continued)

| | | | | | | | |
|---|---|---|---|---|---|---|---|
| ATOM 2025 | CD2 | LEU292 | 37.343 | 14.853 | 52.025 | 1.00 | 24.08 |
| ATOM 2026 | C | LEU292 | 33.999 | 12.063 | 53.528 | 1.00 | 26.58 |
| ATOM 2027 | O | LEU292 | 34.431 | 11.924 | 54.671 | 1.00 | 27.91 |
| ATOM 2028 | N | ILE293 | 32.744 | 12.421 | 53.272 | 1.00 | 27.03 |
| ATOM 2029 | CA | ILE293 | 31.783 | 12.689 | 54.342 | 1.00 | 26.01 |
| ATOM 2030 | CB | ILE293 | 30.948 | 13.956 | 54.019 | 1.00 | 26.42 |
| ATOM 2031 | CG2 | ILE293 | 30.184 | 14.431 | 55.247 | 1.00 | 25.08 |
| ATOM 2032 | CG1 | ILE293 | 31.866 | 15.085 | 53.573 | 1.00 | 24.53 |
| ATOM 2033 | CD1 | ILE293 | 31.131 | 16.366 | 53.336 | 1.00 | 23.77 |
| ATOM 2034 | C | ILE293 | 30.827 | 11.503 | 54.489 | 1.00 | 24.65 |
| ATOM 2035 | O | ILE293 | 30.681 | 10.919 | 55.565 | 1.00 | 23.84 |
| ATOM 2036 | N | GLY294 | 30.197 | 11.159 | 53.374 | 1.00 | 24.02 |
| ATOM 2037 | CA | GLY294 | 29.237 | 10.073 | 53.325 | 1.00 | 25.49 |
| ATOM 2038 | C | GLY294 | 29.454 | 8.815 | 54.142 | 1.00 | 24.75 |
| ATOM 2039 | O | GLY294 | 30.427 | 8.079 | 53.953 | 1.00 | 26.25 |
| ATOM 2040 | N | GLY295 | 28.517 | 8.556 | 55.044 | 1.00 | 22.54 |
| ATOM 2041 | CA | GLY295 | 28.607 | 7.369 | 55.851 | 1.00 | 22.80 |
| ATOM 2042 | C | GLY295 | 28.530 | 6.125 | 54.986 | 1.00 | 25.08 |
| ATOM 2043 | O | GLY295 | 28.252 | 5.047 | 55.497 | 1.00 | 27.80 |
| ATOM 2044 | N | LYS296 | 28.748 | 6.238 | 53.680 | 1.00 | 25.43 |
| ATOM 2045 | CA | LYS296 | 28.696 | 5.039 | 52.849 | 1.00 | 25.87 |
| ATOM 2046 | CB | LYS296 | 28.313 | 5.354 | 51.411 | 1.00 | 27.04 |
| ATOM 2047 | CG | LYS296 | 28.036 | 4.096 | 50.587 | 1.00 | 30.40 |
| ATOM 2048 | CD | LYS296 | 29.249 | 3.562 | 49.842 | 1.00 | 30.20 |
| ATOM 2049 | CE | LYS296 | 28.954 | 2.204 | 49.176 | 1.00 | 32.59 |
| ATOM 2050 | NZ | LYS296 | 29.015 | 1.038 | 50.135 | 1.00 | 32.31 |
| ATOM 2051 | C | LYS296 | 30.044 | 4.364 | 52.828 | 1.00 | 28.34 |
| ATOM 2052 | O | LYS296 | 30.158 | 3.185 | 52.507 | 1.00 | 29.08 |
| ATOM 2053 | N | TYR297 | 31.075 | 5.122 | 53.163 | 1.00 | 29.56 |
| ATOM 2054 | CA | TYR297 | 32.414 | 4.582 | 53.147 | 1.00 | 29.25 |
| ATOM 2055 | CB | TYR297 | 33.208 | 5.230 | 52.022 | 1.00 | 30.07 |
| ATOM 2056 | CG | TYR297 | 32.620 | 5.025 | 50.650 | 1.00 | 30.84 |
| ATOM 2057 | CD1 | TYR297 | 32.023 | 6.082 | 49.960 | 1.00 | 32.45 |
| ATOM 2058 | CE1 | TYR297 | 31.544 | 5.915 | 48.665 | 1.00 | 35.21 |
| ATOM 2059 | CD2 | TYR297 | 32.715 | 3.789 | 50.015 | 1.00 | 30.51 |
| ATOM 2060 | CE2 | TYR297 | 32.244 | 3.604 | 48.724 | 1.00 | 34.82 |
| ATOM 2061 | CZ | TYR297 | 31.661 | 4.673 | 48.049 | 1.00 | 37.82 |
| ATOM 2062 | OH | TYR297 | 31.219 | 4.504 | 46.753 | 1.00 | 41.74 |
| ATOM 2063 | C | TYR297 | 33.097 | 4.842 | 54.465 | 1.00 | 27.53 |
| ATOM 2064 | O | TYR297 | 34.174 | 4.312 | 54.731 | 1.00 | 28.35 |
| ATOM 2065 | N | MET298 | 32.464 | 5.665 | 55.288 | 1.00 | 24.45 |
| ATOM 2066 | CA | MET298 | 33.025 | 6.000 | 56.580 | 1.00 | 23.96 |
| ATOM 2067 | CB | MET298 | 31.959 | 6.652 | 57.454 | 1.00 | 21.69 |
| ATOM 2068 | CG | MET298 | 32.436 | 6.992 | 58.850 | 1.00 | 20.73 |
| ATOM 2069 | SD | MET298 | 31.288 | 8.100 | 59.701 | 1.00 | 20.68 |
| ATOM 2070 | CE | MET298 | 31.435 | 9.523 | 58.620 | 1.00 | 18.32 |
| ATOM 2071 | C | MET298 | 33.579 | 4.750 | 57.254 | 1.00 | 24.25 |
| ATOM 2072 | O | MET298 | 34.776 | 4.656 | 57.529 | 1.00 | 24.74 |
| ATOM 2073 | N | GLY299 | 32.707 | 3.779 | 57.494 | 1.00 | 26.72 |
| ATOM 2074 | CA | GLY299 | 33.135 | 2.552 | 58.135 | 1.00 | 25.77 |
| ATOM 2075 | C | GLY299 | 34.301 | 1.906 | 57.424 | 1.00 | 25.50 |

[Table 2] (continued)

| | | | | | | | |
|---|---|---|---|---|---|---|---|
| ATOM 2076 | O | GLY299 | 35.162 | 1.331 | 58.076 | 1.00 | 26.16 |
| ATOM 2077 | N | GLU300 | 34.325 | 2.004 | 56.095 | 1.00 | 25.37 |
| ATOM 2078 | CA | GLU300 | 35.389 | 1.418 | 55.282 | 1.00 | 24.57 |
| ATOM 2079 | CB | GLU300 | 35.057 | 1.551 | 53.800 | 1.00 | 24.05 |
| ATOM 2080 | CG | GLU300 | 36.066 | 0.859 | 52.905 | 1.00 | 24.66 |
| ATOM 2081 | CD | GLU300 | 36.018 | -0.662 | 53.004 | 1.00 | 24.52 |
| ATOM 2082 | OE1 | GLU300 | 35.581 | -1.195 | 54.054 | 1.00 | 24.02 |
| ATOM 2083 | OE2 | GLU300 | 36.438 | -1.319 | 52.026 | 1.00 | 22.70 |
| ATOM 2084 | C | GLU300 | 36.734 | 2.082 | 55.550 | 1.00 | 25.31 |
| ATOM 2085 | O | GLU300 | 37.769 | 1.408 | 55.663 | 1.00 | 22.71 |
| ATOM 2086 | N | LEU301 | 36.712 | 3.409 | 55.622 | 1.00 | 26.47 |
| ATOM 2087 | CA | LEU301 | 37.919 | 4.174 | 55.900 | 1.00 | 26.65 |
| ATOM 2088 | CB | LEU301 | 37.600 | 5.676 | 55.992 | 1.00 | 26.57 |
| ATOM 2089 | CG | LEU301 | 37.165 | 6.395 | 54.701 | 1.00 | 26.02 |
| ATOM 2090 | CD1 | LEU301 | 36.684 | 7.784 | 55.047 | 1.00 | 27.06 |
| ATOM 2091 | CD2 | LEU301 | 38.312 | 6.474 | 53.701 | 1.00 | 25.38 |
| ATOM 2092 | C | LEU301 | 38.452 | 3.648 | 57.226 | 1.00 | 26.23 |
| ATOM 2093 | O | LEU301 | 39.594 | 3.209 | 57.313 | 1.00 | 26.97 |
| ATOM 2094 | N | VAL302 | 37.623 | 3.670 | 58.259 | 1.00 | 26.05 |
| ATOM 2095 | CA | VAL302 | 38.068 | 3.154 | 59.542 | 1.00 | 27.56 |
| ATOM 2096 | CB | VAL302 | 36.911 | 3.034 | 60.524 | 1.00 | 28.13 |
| ATOM 2097 | CG1 | VAL302 | 37.354 | 2.285 | 61.777 | 1.00 | 26.62 |
| ATOM 2098 | CG2 | VAL302 | 36.433 | 4.424 | 60.882 | 1.00 | 30.95 |
| ATOM 2099 | C | VAL302 | 38.723 | 1.786 | 59.386 | 1.00 | 27.42 |
| ATOM 2100 | O | VAL302 | 39.765 | 1.529 | 59.977 | 1.00 | 29.00 |
| ATOM 2101 | N | ARG303 | 38.127 | 0.906 | 58.593 | 1.00 | 25.04 |
| ATOM 2102 | CA | ARG303 | 38.723 | -0.395 | 58.417 | 1.00 | 25.12 |
| ATOM 2103 | CB | ARG303 | 37.906 | -1.254 | 57.475 | 1.00 | 26.51 |
| ATOM 2104 | CG | ARG303 | 38.587 | -2.558 | 57.126 | 1.00 | 28.11 |
| ATOM 2105 | CD | ARG303 | 37.609 | -3.520 | 56.490 | 1.00 | 31.77 |
| ATOM 2106 | NE | ARG303 | 38.260 | -4.456 | 55.583 | 1.00 | 32.46 |
| ATOM 2107 | CZ | ARG303 | 38.483 | -4.215 | 54.296 | 1.00 | 34.64 |
| ATOM 2108 | NH1 | ARG303 | 38.103 | -3.059 | 53.759 | 1.00 | 33.51 |
| ATOM 2109 | NH2 | ARG303 | 39.082 | -5.136 | 53.546 | 1.00 | 35.80 |
| ATOM 2110 | C | ARG303 | 40.111 | -0.242 | 57.854 | 1.00 | 27.77 |
| ATOM 2111 | O | ARG303 | 41.073 | -0.788 | 58.401 | 1.00 | 30.47 |
| ATOM 2112 | N | LEU304 | 40.236 | 0.495 | 56.754 | 1.00 | 27.67 |
| ATOM 2113 | CA | LEU304 | 41.562 | 0.674 | 56.147 | 1.00 | 24.93 |
| ATOM 2114 | CB | LEU304 | 41.464 | 1.526 | 54.865 | 1.00 | 22.51 |
| ATOM 2115 | CG | LEU304 | 40.640 | 0.902 | 53.718 | 1.00 | 19.14 |
| ATOM 2116 | CD1 | LEU304 | 40.386 | 1.957 | 52.675 | 1.00 | 19.15 |
| ATOM 2117 | CD2 | LEU304 | 41.352 | -0.295 | 53.105 | 1.00 | 14.45 |
| ATOM 2118 | C | LEU304 | 42.523 | 1.290 | 57.168 | 1.00 | 21.35 |
| ATOM 2119 | O | LEU304 | 43.584 | 0.736 | 57.432 | 1.00 | 20.90 |
| ATOM 2120 | N | VAL305 | 42.142 | 2.406 | 57.770 | 1.00 | 17.52 |
| ATOM 2121 | CA | VAL305 | 43.003 | 3.011 | 58.758 | 1.00 | 17.43 |
| ATOM 2122 | CB | VAL305 | 42.316 | 4.162 | 59.423 | 1.00 | 14.40 |
| ATOM 2123 | CG1 | VAL305 | 43.154 | 4.673 | 60.583 | 1.00 | 14.53 |
| ATOM 2124 | CG2 | VAL305 | 42.095 | 5.240 | 58.408 | 1.00 | 14.33 |
| ATOM 2125 | C | VAL305 | 43.400 | 2.010 | 59.829 | 1.00 | 20.92 |
| ATOM 2126 | O | VAL305 | 44.497 | 2.071 | 60.387 | 1.00 | 22.69 |

[Table 2] (continued)

| | | | | | | | |
|---|---|---|---|---|---|---|---|
| ATOM 2127 | N | LEU306 | 42.502 | 1.085 | 60.126 | 1.00 | 24.02 |
| ATOM 2128 | CA | LEU306 | 42.783 | 0.081 | 61.144 | 1.00 | 26.64 |
| ATOM 2129 | CB | LEU306 | 41.481 | -0.585 | 61.594 | 1.00 | 27.02 |
| ATOM 2130 | CG | LEU306 | 41.154 | -0.563 | 63.087 | 1.00 | 27.64 |
| ATOM 2131 | CD1 | LEU306 | 41.094 | 0.873 | 63.592 | 1.00 | 27.51 |
| ATOM 2132 | CD2 | LEU306 | 39.826 | -1.267 | 63.311 | 1.00 | 28.07 |
| ATOM 2133 | C | LEU306 | 43.721 | -0.965 | 60.566 | 1.00 | 27.73 |
| ATOM 2134 | O | LEU306 | 44.745 | -1.303 | 61.157 | 1.00 | 26.86 |
| ATOM 2135 | N | LEU307 | 43.360 | -1.467 | 59.394 | 1.00 | 28.77 |
| ATOM 2136 | CA | LEU307 | 44.156 | -2.478 | 58.733 | 1.00 | 32.47 |
| ATOM 2137 | CB | LEU307 | 43.465 | -2.893 | 57.437 | 1.00 | 29.90 |
| ATOM 2138 | CG | LEU307 | 43.477 | -4.392 | 57.130 | 1.00 | 29.19 |
| ATOM 2139 | CD1 | LEU307 | 43.104 | -5.210 | 58.361 | 1.00 | 28.38 |
| ATOM 2140 | CD2 | LEU307 | 42.495 | -4.648 | 56.015 | 1.00 | 29.88 |
| ATOM 2141 | C | LEU307 | 45.553 | -1.916 | 58.466 | 1.00 | 35.49 |
| ATOM 2142 | O | LEU307 | 46.542 | -2.645 | 58.394 | 1.00 | 36.50 |
| ATOM 2143 | N | ARG308 | 45.622 | -0.602 | 58.332 | 1.00 | 38.03 |
| ATOM 2144 | CA | ARG308 | 46.882 | 0.080 | 58.101 | 1.00 | 41.29 |
| ATOM 2145 | CB | ARG308 | 46.603 | 1.580 | 57.936 | 1.00 | 47.88 |
| ATOM 2146 | CG | ARG308 | 47.706 | 2.544 | 58.368 | 1.00 | 54.88 |
| ATOM 2147 | CD | ARG308 | 48.819 | 2.693 | 57.338 | 1.00 | 60.14 |
| ATOM 2148 | NE | ARG308 | 49.524 | 3.958 | 57.540 | 1.00 | 65.47 |
| ATOM 2149 | CZ | ARG308 | 50.523 | 4.401 | 56.784 | 1.00 | 67.54 |
| ATOM 2150 | NH1 | ARG308 | 50.954 | 3.673 | 55.757 | 1.00 | 68.57 |
| ATOM 2151 | NH2 | ARG308 | 51.074 | 5.584 | 57.046 | 1.00 | 66.83 |
| ATOM 2152 | C | ARG308 | 47.783 | -0.182 | 59.301 | 1.00 | 40.42 |
| ATOM 2153 | O | ARG308 | 48.889 | -0.694 | 59.159 | 1.00 | 40.04 |
| ATOM 2154 | N | LEU309 | 47.287 | 0.152 | 60.487 | 1.00 | 39.27 |
| ATOM 2155 | CA | LEU309 | 48.043 | -0.027 | 61.717 | 1.00 | 38.92 |
| ATOM 2156 | CB | LEU309 | 47.224 | 0.484 | 62.895 | 1.00 | 33.74 |
| ATOM 2157 | CG | LEU309 | 46.852 | 1.958 | 62.854 | 1.00 | 30.26 |
| ATOM 2158 | CD1 | LEU309 | 45.453 | 2.121 | 63.368 | 1.00 | 30.84 |
| ATOM 2159 | CD2 | LEU309 | 47.819 | 2.766 | 63.683 | 1.00 | 27.57 |
| ATOM 2160 | C | LEU309 | 48.461 | -1.473 | 61.984 | 1.00 | 41.92 |
| ATOM 2161 | O | LEU309 | 49.600 | -1.741 | 62.364 | 1.00 | 42.73 |
| ATOM 2162 | N | VAL310 | 47.541 | -2.406 | 61.788 | 1.00 | 44.59 |
| ATOM 2163 | CA | VAL310 | 47.829 | -3.811 | 62.039 | 1.00 | 46.67 |
| ATOM 2164 | CB | VAL310 | 46.606 | -4.651 | 61.798 | 1.00 | 46.95 |
| ATOM 2165 | CG1 | VAL310 | 45.419 | -4.006 | 62.479 | 1.00 | 49.54 |
| ATOM 2166 | CG2 | VAL310 | 46.368 | -4.779 | 60.312 | 1.00 | 47.77 |
| ATOM 2167 | C | VAL310 | 48.929 | -4.321 | 61.139 | 1.00 | 47.55 |
| ATOM 2168 | O | VAL310 | 49.488 | -5.392 | 61.374 | 1.00 | 48.66 |
| ATOM 2169 | N | ASP311 | 49.217 | -3.559 | 60.093 | 1.00 | 48.93 |
| ATOM 2170 | CA | ASP311 | 50.262 | -3.927 | 59.160 | 1.00 | 52.04 |
| ATOM 2171 | CB | ASP311 | 49.993 | -3.298 | 57.793 | 1.00 | 57.14 |
| ATOM 2172 | CG | ASP311 | 48.752 | -3.869 | 57.135 | 1.00 | 61.79 |
| ATOM 2173 | OD1 | ASP311 | 48.348 | -3.377 | 56.054 | 1.00 | 63.59 |
| ATOM 2174 | OD2 | ASP311 | 48.180 | -4.819 | 57.713 | 1.00 | 63.98 |
| ATOM 2175 | C | ASP311 | 51.618 | -3.490 | 59.698 | 1.00 | 51.94 |
| ATOM 2176 | O | ASP311 | 52.580 | -4.256 | 59.653 | 1.00 | 53.89 |
| ATOM 2177 | N | GLU312 | 51.702 | -2.267 | 60.212 | 1.00 | 49.51 |

[Table 2] (continued)

| | | | | | | | |
|---|---|---|---|---|---|---|---|
| ATOM 2178 | CA | GLU312 | 52.961 | -1.785 | 60.762 | 1.00 | 47.68 |
| ATOM 2179 | CB | GLU312 | 53.071 | -0.272 | 60.632 | 1.00 | 48.44 |
| ATOM 2180 | CG | GLU312 | 52.900 | 0.221 | 59.216 | 1.00 | 51.79 |
| ATOM 2181 | CD | GLU312 | 53.122 | 1.713 | 59.084 | 1.00 | 53.56 |
| ATOM 2182 | OE1 | GLU312 | 52.698 | 2.280 | 58.047 | 1.00 | 49.90 |
| ATOM 2183 | OE2 | GLU312 | 53.725 | 2.309 | 60.013 | 1.00 | 56.82 |
| ATOM 2184 | C | GLU312 | 53.075 | -2.172 | 62.222 | 1.00 | 46.11 |
| ATOM 2185 | O | GLU312 | 53.514 | -1.377 | 63.049 | 1.00 | 46.75 |
| ATOM 2186 | N | ASN313 | 52.666 | -3.397 | 62.527 | 1.00 | 45.02 |
| ATOM 2187 | CA | ASN313 | 52.720 | -3.938 | 63.879 | 1.00 | 44.64 |
| ATOM 2188 | CB | ASN313 | 54.100 | -4.550 | 64.119 | 1.00 | 43.84 |
| ATOM 2189 | CG | ASN313 | 54.028 | -5.860 | 64.863 | 1.00 | 45.16 |
| ATOM 2190 | OD1 | ASN313 | 53.377 | -5.965 | 65.906 | 1.00 | 43.79 |
| ATOM 2191 | ND2 | ASN313 | 54.701 | -6.875 | 64.333 | 1.00 | 46.05 |
| ATOM 2192 | C | ASN313 | 52.408 | -2.921 | 64.991 | 1.00 | 44.49 |
| ATOM 2193 | O | ASN313 | 53.303 | -2.509 | 65.728 | 1.00 | 45.19 |
| ATOM 2194 | N | LEU314 | 51.142 | -2.530 | 65.126 | 1.00 | 43.02 |
| ATOM 2195 | CA | LEU314 | 50.743 | -1.563 | 66.159 | 1.00 | 40.80 |
| ATOM 2196 | CB | LEU314 | 50.639 | -0.167 | 65.549 | 1.00 | 34.97 |
| ATOM 2197 | CG | LEU314 | 51.940 | 0.499 | 65.127 | 1.00 | 29.58 |
| ATOM 2198 | CD1 | LEU314 | 51.698 | 1.453 | 63.981 | 1.00 | 28.94 |
| ATOM 2199 | CD2 | LEU314 | 52.516 | 1.212 | 66.311 | 1.00 | 28.16 |
| ATOM 2200 | C | LEU314 | 49.396 | -1.924 | 66.777 | 1.00 | 42.38 |
| ATOM 2201 | O | LEU314 | 49.026 | -1.422 | 67.848 | 1.00 | 39.73 |
| ATOM 2202 | N | LEU315 | 48.689 | -2.812 | 66.078 | 1.00 | 44.49 |
| ATOM 2203 | CA | LEU315 | 47.352 | -3.268 | 66.439 | 1.00 | 45.22 |
| ATOM 2204 | CB | LEU315 | 46.354 | -2.695 | 65.445 | 1.00 | 43.49 |
| ATOM 2205 | CG | LEU315 | 45.121 | -2.063 | 66.045 | 1.00 | 43.28 |
| ATOM 2206 | CD1 | LEU315 | 44.055 | -1.976 | 64.972 | 1.00 | 43.01 |
| ATOM 2207 | CD2 | LEU315 | 44.643 | -2.907 | 67.209 | 1.00 | 46.13 |
| ATOM 2208 | C | LEU315 | 47.214 | -4.781 | 66.407 | 1.00 | 46.34 |
| ATOM 2209 | O | LEU315 | 47.828 | -5.439 | 65.577 | 1.00 | 47.74 |
| ATOM 2210 | N | PHE316 | 46.380 | -5.318 | 67.292 | 1.00 | 48.50 |
| ATOM 2211 | CA | PHE316 | 46.125 | -6.760 | 67.369 | 1.00 | 50.80 |
| ATOM 2212 | CB | PHE316 | 45.054 | -7.186 | 66.347 | 1.00 | 48.89 |
| ATOM 2213 | CG | PHE316 | 43.829 | -6.312 | 66.331 | 1.00 | 46.47 |
| ATOM 2214 | CD1 | PHE316 | 43.163 | -5.999 | 67.508 | 1.00 | 45.93 |
| ATOM 2215 | CD2 | PHE316 | 43.350 | -5.791 | 65.134 | 1.00 | 44.48 |
| ATOM 2216 | CE1 | PHE316 | 42.043 | -5.183 | 67.491 | 1.00 | 44.57 |
| ATOM 2217 | CE2 | PHE316 | 42.229 | -4.974 | 65.109 | 1.00 | 43.59 |
| ATOM 2218 | CZ | PHE316 | 41.577 | -4.669 | 66.290 | 1.00 | 44.05 |
| ATOM 2219 | C | PHE316 | 47.371 | -7.605 | 67.124 | 1.00 | 53.06 |
| ATOM 2220 | O | PHE316 | 47.342 | -8.521 | 66.299 | 1.00 | 54.62 |
| ATOM 2221 | N | HIS317 | 48.456 | -7.304 | 67.835 | 1.00 | 54.60 |
| ATOM 2222 | CA | HIS317 | 49.710 | -8.046 | 67.691 | 1.00 | 55.95 |
| ATOM 2223 | CB | HIS317 | 49.676 | -9.301 | 68.569 | 1.00 | 54.90 |
| ATOM 2224 | CG | HIS317 | 49.708 | -9.004 | 70.034 | 1.00 | 55.21 |
| ATOM 2225 | CD2 | HIS317 | 49.686 | -9.823 | 71.113 | 1.00 | 55.22 |
| ATOM 2226 | ND1 | HIS317 | 49.778 | -7.718 | 70.528 | 1.00 | 54.55 |
| ATOM 2227 | CE1 | HIS317 | 49.798 | -7.756 | 71.848 | 1.00 | 55.21 |
| ATOM 2228 | NE2 | HIS317 | 49.744 | -9.020 | 72.229 | 1.00 | 56.90 |

[Table 2] (continued)

| | | | | | | | |
|---|---|---|---|---|---|---|---|
| ATOM 2229 | C | HIS317 | 50.004 | -8.426 | 66.240 | 1.00 | 58.27 |
| ATOM 2230 | O | HIS317 | 50.521 | -9.514 | 65.950 | 1.00 | 58.90 |
| ATOM 2231 | N | GLY318 | 49.665 | -7.513 | 65.335 | 1.00 | 59.86 |
| ATOM 2232 | CA | GLY318 | 49.881 | -7.734 | 63.921 | 1.00 | 60.72 |
| ATOM 2233 | C | GLY318 | 49.290 | -9.022 | 63.379 | 1.00 | 62.25 |
| ATOM 2234 | O | GLY318 | 50.031 | -9.956 | 63.080 | 1.00 | 63.75 |
| ATOM 2235 | N | GLU319 | 47.962 | -9.087 | 63.277 | 1.00 | 62.86 |
| ATOM 2236 | CA | GLU319 | 47.277 | -10.257 | 62.716 | 1.00 | 62.72 |
| ATOM 2237 | CB | GLU319 | 47.663 | -11.545 | 63.439 | 1.00 | 66.93 |
| ATOM 2238 | CG | GLU319 | 47.437 | -12.784 | 62.575 | 1.00 | 73.23 |
| ATOM 2239 | CD | GLU319 | 47.862 | -14.068 | 63.262 | 1.00 | 78.58 |
| ATOM 2240 | OE1 | GLU319 | 49.020 | -14.129 | 63.745 | 1.00 | 80.57 |
| ATOM 2241 | OE2 | GLU319 | 47.043 | -15.019 | 63.310 | 1.00 | 81.49 |
| ATOM 2242 | C | GLU319 | 45.765 | -10.097 | 62.739 | 1.00 | 59.42 |
| ATOM 2243 | O | GLU319 | 45.098 | -10.287 | 63.735 | 1.00 | 57.03 |
| ATOM 2244 | N | ALA320 | 45.246 | -9.643 | 61.604 | 1.00 | 55.74 |
| ATOM 2245 | CA | ALA320 | 43.828 | -9.394 | 61.414 | 1.00 | 54.02 |
| ATOM 2246 | CB | ALA320 | 43.657 | -8.357 | 60.338 | 1.00 | 52.55 |
| ATOM 2247 | C | ALA320 | 43.052 | -10.650 | 61.043 | 1.00 | 54.49 |
| ATOM 2248 | O | ALA320 | 43.620 | -11.565 | 60.457 | 1.00 | 55.61 |
| ATOM 2249 | N | SER321 | 41.762 | -10.698 | 61.388 | 1.00 | 55.01 |
| ATOM 2250 | CA | SER321 | 40.924 | -11.856 | 61.050 | 1.00 | 55.90 |
| ATOM 2251 | CB | SER321 | 39.649 | -11.911 | 61.895 | 1.00 | 56.08 |
| ATOM 2252 | OG | SER321 | 38.814 | -12.975 | 61.445 | 1.00 | 53.96 |
| ATOM 2253 | C | SER321 | 40.513 | -11.780 | 59.589 | 1.00 | 55.49 |
| ATOM 2254 | O | SER321 | 40.367 | -10.689 | 59.041 | 1.00 | 54.92 |
| ATOM 2255 | N | GLU322 | 40.292 | -12.933 | 58.967 | 1.00 | 54.84 |
| ATOM 2256 | CA | GLU322 | 39.917 | -12.951 | 57.563 | 1.00 | 56.14 |
| ATOM 2257 | CB | GLU322 | 39.646 | -14.382 | 57.092 | 1.00 | 58.38 |
| ATOM 2258 | CG | GLU322 | 40.173 | -14.697 | 55.681 | 1.00 | 63.26 |
| ATOM 2259 | CD | GLU322 | 41.712 | -14.670 | 55.574 | 1.00 | 66.36 |
| ATOM 2260 | OE1 | GLU322 | 42.296 | -13.571 | 55.432 | 1.00 | 66.15 |
| ATOM 2261 | OE2 | GLU322 | 42.339 | -15.754 | 55.637 | 1.00 | 66.78 |
| ATOM 2262 | C | GLU322 | 38.685 | -12.085 | 57.354 | 1.00 | 55.71 |
| ATOM 2263 | O | GLU322 | 38.343 | -11.727 | 56.227 | 1.00 | 54.93 |
| ATOM 2264 | N | GLN323 | 38.027 | -11.740 | 58.454 | 1.00 | 55.82 |
| ATOM 2265 | CA | GLN323 | 36.838 | -10.904 | 58.393 | 1.00 | 55.20 |
| ATOM 2266 | CB | GLN323 | 35.995 | -11.101 | 59.659 | 1.00 | 57.22 |
| ATOM 2267 | CG | GLN323 | 35.737 | -12.571 | 59.983 | 1.00 | 60.42 |
| ATOM 2268 | CD | GLN323 | 34.801 | -12.778 | 61.164 | 1.00 | 62.11 |
| ATOM 2269 | OE1 | GLN323 | 34.596 | -13.909 | 61.612 | 1.00 | 63.58 |
| ATOM 2270 | NE2 | GLN323 | 34.223 | -11.690 | 61.668 | 1.00 | 61.37 |
| ATOM 2271 | C | GLN323 | 37.259 | -9.445 | 58.249 | 1.00 | 53.59 |
| ATOM 2272 | O | GLN323 | 36.963 | -8.800 | 57.242 | 1.00 | 53.27 |
| ATOM 2273 | N | LEU324 | 37.973 | -8.936 | 59.248 | 1.00 | 50.98 |
| ATOM 2274 | CA | LEU324 | 38.430 | -7.553 | 59.224 | 1.00 | 48.40 |
| ATOM 2275 | CB | LEU324 | 39.396 | -7.294 | 60.378 | 1.00 | 46.63 |
| ATOM 2276 | CG | LEU324 | 39.956 | -5.876 | 60.498 | 1.00 | 44.87 |
| ATOM 2277 | CD1 | LEU324 | 38.846 | -4.837 | 60.390 | 1.00 | 44.21 |
| ATOM 2278 | CD2 | LEU324 | 40.671 | -5.758 | 61.827 | 1.00 | 43.22 |
| ATOM 2279 | C | LEU324 | 39.115 | -7.224 | 57.911 | 1.00 | 47.25 |

[Table 2] (continued)

| | | | | | | | |
|---|---|---|---|---|---|---|---|
| ATOM 2280 | O | LEU324 | 39.181 | -6.065 | 57.505 | 1.00 | 44.86 |
| ATOM 2281 | N | ARG325 | 39.627 | -8.253 | 57.252 | 1.00 | 48.35 |
| ATOM 2282 | CA | ARG325 | 40.309 | -8.057 | 55.988 | 1.00 | 50.22 |
| ATOM 2283 | CB | ARG325 | 41.473 | -9.055 | 55.839 | 1.00 | 53.47 |
| ATOM 2284 | CG | ARG325 | 42.580 | -8.896 | 56.894 | 1.00 | 57.97 |
| ATOM 2285 | CD | ARG325 | 43.660 | -9.986 | 56.808 | 1.00 | 61.92 |
| ATOM 2286 | NE | ARG325 | 44.564 | -9.957 | 57.966 | 1.00 | 67.95 |
| ATOM 2287 | CZ | ARG325 | 45.535 | -10,844 | 58.206 | 1.00 | 70.27 |
| ATOM 2288 | NH1 | ARG325 | 45.753 | -11.854 | 57.371 | 1.00 | 69.69 |
| ATOM 2289 | NH2 | ARG325 | 46.290 | -10.725 | 59.293 | 1.00 | 70.39 |
| ATOM 2290 | C | ARG325 | 39.320 | -8.224 | 54.850 | 1.00 | 48.80 |
| ATOM 2291 | O | ARG325 | 39.617 | -8.859 | 53.847 | 1.00 | 50.46 |
| ATOM 2292 | N | THR326 | 38.131 | -7.663 | 54.999 | 1.00 | 46.54 |
| ATOM 2293 | CA | THR326 | 37.162 | -7.783 | 53.929 | 1.00 | 45.13 |
| ATOM 2294 | CB | THR326 | 36.108 | -8.810 | 54.264 | 1.00 | 44.85 |
| ATOM 2295 | OG1 | THR326 | 36.749 | -10.061 | 54.546 | 1.00 | 44.98 |
| ATOM 2296 | CG2 | THR326 | 35.160 | -8.973 | 53.092 | 1.00 | 43.46 |
| ATOM 2297 | C | THR326 | 36.500 | -6.453 | 53.687 | 1.00 | 44.79 |
| ATOM 2298 | O | THR326 | 36.256 | -5.705 | 54.626 | 1.00 | 45.01 |
| ATOM 2299 | N | ARG327 | 36.216 | -6.143 | 52.430 | 1.00 | 45.02 |
| ATOM 2300 | CA | ARG327 | 35.590 | -4.866 | 52.136 | 1.00 | 45.97 |
| ATOM 2301 | CB | ARG327 | 35.476 | -4.655 | 50.623 | 1.00 | 48.63 |
| ATOM 2302 | CG | ARG327 | 34.961 | -3.283 | 50.229 | 1.00 | 53.97 |
| ATOM 2303 | CD | ARG327 | 34.975 | -3.072 | 48.722 | 1.00 | 58.44 |
| ATOM 2304 | NE | ARG327 | 33.747 | -2.410 | 48.282 | 1.00 | 66.14 |
| ATOM 2305 | CZ | ARG327 | 33.387 | -1.178 | 48.648 | 1.00 | 69.53 |
| ATOM 2306 | NH1 | ARG327 | 34.167 | -0.471 | 49.458 | 1.00 | 69.84 |
| ATOM 2307 | NH2 | ARG327 | 32.242 | -0.652 | 48.220 | 1.00 | 68.29 |
| ATOM 2308 | C | ARG327 | 34.217 | -4.790 | 52.794 | 1.00 | 44.69 |
| ATOM 2309 | O | ARG327 | 33.486 | -5.784 | 52.861 | 1.00 | 44.55 |
| ATOM 2310 | N | GLY328 | 33.888 | -3.605 | 53.302 | 1.00 | 42.14 |
| ATOM 2311 | CA | GLY328 | 32.606 | -3.394 | 53.952 | 1.00 | 37.48 |
| ATOM 2312 | C | GLY328 | 32.480 | -4.007 | 55.334 | 1.00 | 33.00 |
| ATOM 2313 | O | GLY328 | 31.693 | -3.532 | 56.148 | 1.00 | 32.88 |
| ATOM 2314 | N | ALA329 | 33.258 | -5.049 | 55.601 | 1.00 | 29.02 |
| ATOM 2315 | CA | ALA329 | 33.227 | -5.743 | 56.885 | 1.00 | 26.22 |
| ATOM 2316 | CB | ALA329 | 34.452 | -6.623 | 57.028 | 1.00 | 28.65 |
| ATOM 2317 | C | ALA329 | 33.092 | -4.861 | 58.115 | 1.00 | 24.38 |
| ATOM 2318 | O | ALA329 | 32.490 | -5.276 | 59.097 | 1.00 | 26.43 |
| ATOM 2319 | N | PHE330 | 33.663 | -3.663 | 58.091 | 1.00 | 21.81 |
| ATOM 2320 | CA | PHE330 | 33.547 | -2.776 | 59.242 | 1.00 | 18.07 |
| ATOM 2321 | CB | PHE330 | 34.887 | -2.137 | 59.558 | 1.00 | 13.90 |
| ATOM 2322 | CG | PHE330 | 34.913 | -1.404 | 60.862 | 1.00 | 12.45 |
| ATOM 2323 | CD1 | PHE330 | 34.460 | -0.096 | 60.961 | 1.00 | 12.64 |
| ATOM 2324 | CD2 | PHE330 | 35.436 | -2.009 | 61.995 | 1.00 | 12.73 |
| ATOM 2325 | CE1 | PHE330 | 34.535 | 0.605 | 62.188 | 1.00 | 12.83 |
| ATOM 2326 | CE2 | PHE330 | 35.515 | -1.315 | 63.221 | 1.00 | 11.49 |
| ATOM 2327 | CZ | PHE330 | 35.066 | -0.008 | 63.315 | 1.00 | 8.96 |
| ATOM 2328 | C | PHE330 | 32.528 | -1.716 | 58.886 | 1.00 | 17.48 |
| ATOM 2329 | O | PHE330 | 32.855 | -0.702 | 58.273 | 1.00 | 17.97 |

[Table 2] (continued)

| | | | | | | | |
|---|---|---|---|---|---|---|---|
| ATOM 2330 | N | GLU331 | 31.288 | -1.976 | 59.275 | 1.00 | 16.36 |
| ATOM 2331 | CA | GLU331 | 30.149 | -1.105 | 58.998 | 1.00 | 18.14 |
| ATOM 2332 | CB | GLU331 | 28.865 | -1.889 | 59.308 | 1.00 | 22.08 |
| ATOM 2333 | CG | GLU331 | 28.790 | -3.226 | 58.546 | 1.00 | 26.82 |
| ATOM 2334 | CD | GLU331 | 28.183 | -4.382 | 59.346 | 1.00 | 28.86 |
| ATOM 2335 | OE1 | GLU331 | 28.381 | -5.552 | 58.931 | 1.00 | 28.12 |
| ATOM 2336 | OE2 | GLU331 | 27.509 | -4.129 | 60.371 | 1.00 | 30.16 |
| ATOM 2337 | C | GLU331 | 30.126 | 0.248 | 59.719 | 1.00 | 16.36 |
| ATOM 2338 | O | GLU331 | 30.596 | 0.380 | 60.849 | 1.00 | 16.97 |
| ATOM 2339 | N | THR332 | 29.583 | 1.263 | 59.060 | 1.00 | 14.04 |
| ATOM 2340 | CA | THR332 | 29.494 | 2.568 | 59.695 | 1.00 | 14.47 |
| ATOM 2341 | CB | THR332 | 28.747 | 3.562 | 58.825 | 1.00 | 10.93 |
| ATOM 2342 | OG1 | THR332 | 29.473 | 3.751 | 57.611 | 1.00 | 6.57 |
| ATOM 2343 | CG2 | THR332 | 28.597 | 4.890 | 59.550 | 1.00 | 6.34 |
| ATOM 2344 | C | THR332 | 28.725 | 2.382 | 60.994 | 1.00 | 18.42 |
| ATOM 2345 | O | THR332 | 29.125 | 2.872 | 62.052 | 1.00 | 17.70 |
| ATOM 2346 | N | ARG333 | 27.609 | 1.671 | 60.892 | 1.00 | 21.79 |
| ATOM 2347 | CA | ARG333 | 26.783 | 1.346 | 62.040 | 1.00 | 24.44 |
| ATOM 2348 | CB | ARG333 | 26.095 | 0.001 | 61.764 | 1.00 | 28.62 |
| ATOM 2349 | CG | ARG333 | 25.291 | -0.590 | 62.910 | 1.00 | 34.65 |
| ATOM 2350 | CD | ARG333 | 24.308 | -1.664 | 62.401 | 1.00 | 39.87 |
| ATOM 2351 | NE | ARG333 | 24.953 | -2.887 | 61.910 | 1.00 | 43.42 |
| ATOM 2352 | CZ | ARG333 | 25.198 | -3.969 | 62.653 | 1.00 | 46.01 |
| ATOM 2353 | NH1 | ARG333 | 24.852 | -3.992 | 63.940 | 1.00 | 45.10 |
| ATOM 2354 | NH2 | ARG333 | 25.791 | -5.030 | 62.104 | 1.00 | 43.75 |
| ATOM 2355 | C | ARG333 | 27.638 | 1.271 | 63.323 | 1.00 | 24.88 |
| ATOM 2356 | O | ARG333 | 27.242 | 1.803 | 64.358 | 1.00 | 24.00 |
| ATOM 2357 | N | PHE334 | 28.818 | 0.635 | 63.232 | 1.00 | 23.97 |
| ATOM 2358 | CA | PHE334 | 29.740 | 0.458 | 64.371 | 1.00 | 19.64 |
| ATOM 2359 | CB | PHE334 | 30.877 | -0.509 | 64.033 | 1.00 | 20.52 |
| ATOM 2360 | CG | PHE334 | 30.420 | -1.813 | 63.468 | 1.00 | 24.74 |
| ATOM 2361 | CD1 | PHE334 | 29.469 | -2.574 | 64.121 | 1.00 | 25.94 |
| ATOM 2362 | CD2 | PHE334 | 30.938 | -2.279 | 62.262 | 1.00 | 26.47 |
| ATOM 2363 | CE1 | PHE334 | 29.039 | -3.780 | 63.575 | 1.00 | 28.43 |
| ATOM 2364 | CE2 | PHE334 | 30.514 | -3.483 | 61.711 | 1.00 | 24.74 |
| ATOM 2365 | CZ | PHE334 | 29.565 | -4.233 | 62.365 | 1.00 | 26.41 |
| ATOM 2366 | C | PHE334 | 30.382 | 1.739 | 64.842 | 1.00 | 16.52 |
| ATOM 2367 | O | PHE334 | 30.434 | 2.020 | 66.039 | 1.00 | 16.16 |
| ATOM 2368 | N | VAL335 | 30.907 | 2.509 | 63.905 | 1.00 | 13.20 |
| ATOM 2369 | CA | VAL335 | 31.546 | 3.752 | 64.284 | 1.00 | 11.36 |
| ATOM 2370 | CB | VAL335 | 31.877 | 4.565 | 63.033 | 1.00 | 8.08 |
| ATOM 2371 | CG1 | VAL335 | 32.113 | 6.003 | 63.402 | 1.00 | 8.71 |
| ATOM 2372 | CG2 | VAL335 | 33.082 | 3.979 | 62.358 | 1.00 | 1.00 |
| ATOM 2373 | C | VAL335 | 30.653 | 4.558 | 65.249 | 1.00 | 13.02 |
| ATOM 2374 | O | VAL335 | 31.126 | 5.066 | 66.264 | 1.00 | 10.40 |
| ATOM 2375 | N | SER336 | 29.359 | 4.640 | 64.934 | 1.00 | 16.23 |
| ATOM 2376 | CA | SER336 | 28.365 | 5.372 | 65.740 | 1.00 | 18.55 |
| ATOM 2377 | CB | SER336 | 27.017 | 5.350 | 65.039 | 1.00 | 19.92 |
| ATOM 2378 | OG | SER336 | 26.611 | 3.999 | 64.866 | 1.00 | 25.40 |
| ATOM 2379 | C | SER336 | 28.162 | 4.766 | 67.118 | 1.00 | 17.99 |
| ATOM 2380 | O | SER336 | 27.896 | 5.465 | 68.100 | 1.00 | 14.64 |

[Table 2] (continued)

| | | | | | | | |
|---|---|---|---|---|---|---|---|
| ATOM 2381 | N | GLN337 | 28.239 | 3.445 | 67.159 | 1.00 | 19.48 |
| ATOM 2382 | CA | GLN337 | 28.061 | 2.719 | 68.394 | 1.00 | 21.39 |
| ATOM 2383 | CB | GLN337 | 27.995 | 1.223 | 68.123 | 1.00 | 21.42 |
| ATOM 2384 | CG | GLN337 | 26.829 | 0.800 | 67.264 | 1.00 | 23.07 |
| ATOM 2385 | CD | GLN337 | 26.920 | -0.654 | 66.895 | 1.00 | 24.96 |
| ATOM 2386 | OE1 | GLN337 | 27.243 | -1.496 | 67.735 | 1.00 | 28.83 |
| ATOM 2387 | NE2 | GLN337 | 26.633 | -0.966 | 65.638 | 1.00 | 24.29 |
| ATOM 2388 | C | GLN337 | 29.260 | 3.011 | 69.240 | 1.00 | 20.91 |
| ATOM 2389 | O | GLN337 | 29.205 | 2.963 | 70.464 | 1.00 | 23.32 |
| ATOM 2390 | N | VAL338 | 30.362 | 3.317 | 68.584 | 1.00 | 20.52 |
| ATOM 2391 | CA | VAL338 | 31.559 | 3.589 | 69.337 | 1.00 | 21.67 |
| ATOM 2392 | CB | VAL338 | 32.812 | 3.470 | 68.443 | 1.00 | 20.93 |
| ATOM 2393 | CG1 | VAL338 | 34.065 | 3.624 | 69.279 | 1.00 | 19.79 |
| ATOM 2394 | CG2 | VAL338 | 32.811 | 2.126 | 67.739 | 1.00 | 16.69 |
| ATOM 2395 | C | VAL338 | 31.480 | 4.973 | 69.977 | 1.00 | 23.61 |
| ATOM 2396 | O | VAL338 | 31.385 | 5.079 | 71.203 | 1.00 | 21.96 |
| ATOM 2397 | N | GLU339 | 31.486 | 6.020 | 69.146 | 1.00 | 25.05 |
| ATOM 2398 | CA | GLU339 | 31.455 | 7.406 | 69.620 | 1.00 | 26.21 |
| ATOM 2399 | CB | GLU339 | 31.460 | 8.402 | 68.440 | 1.00 | 26.37 |
| ATOM 2400 | CG | GLU339 | 30.515 | 8.082 | 67.282 | 1.00 | 31.63 |
| ATOM 2401 | CD | GLU339 | 30.287 | 9.267 | 66.311 | 1.00 | 36.86 |
| ATOM 2402 | OE1 | GLU339 | 29.542 | 10.219 | 66.663 | 1.00 | 37.19 |
| ATOM 2403 | OE2 | GLU339 | 30.850 | 9.243 | 65.187 | 1.00 | 37.90 |
| ATOM 2404 | C | GLU339 | 30.299 | 7.735 | 70.541 | 1.00 | 26.44 |
| ATOM 2405 | O | GLU339 | 30.423 | 8.613 | 71.396 | 1.00 | 27.55 |
| ATOM 2406 | N | SER340 | 29.189 | 7.017 | 70.380 | 1.00 | 26.30 |
| ATOM 2407 | CA | SER340 | 27.987 | 7.246 | 71.181 | 1.00 | 25.08 |
| ATOM 2408 | CB | SER340 | 26.861 | 6.322 | 70.717 | 1.00 | 23.68 |
| ATOM 2409 | OG | SER340 | 27.191 | 4.970 | 70.957 | 1.00 | 23.58 |
| ATOM 2410 | C | SER340 | 28.211 | 7.065 | 72.676 | 1.00 | 26.02 |
| ATOM 2411 | O | SER340 | 27.415 | 7.539 | 73.488 | 1.00 | 26.83 |
| ATOM 2412 | N | ASP341 | 29.294 | 6.380 | 73.033 | 1.00 | 27.41 |
| ATOM 2413 | CA | ASP341 | 29.630 | 6.143 | 74.434 | 1.00 | 27.85 |
| ATOM 2414 | CB | ASP341 | 28.939 | 4.885 | 74.953 | 1.00 | 27.41 |
| ATOM 2415 | CG | ASP341 | 29.253 | 4.621 | 76.410 | 1.00 | 26.49 |
| ATOM 2416 | OD1 | ASP341 | 29.628 | 5.591 | 77.107 | 1.00 | 26.07 |
| ATOM 2417 | OD2 | ASP341 | 29.117 | 3.463 | 76.862 | 1.00 | 25.64 |
| ATOM 2418 | C | ASP341 | 31.128 | 6.008 | 74.672 | 1.00 | 28.59 |
| ATOM 2419 | O | ASP341 | 31.757 | 5.049 | 74.229 | 1.00 | 30.06 |
| ATOM 2420 | N | THR342 | 31.688 | 6.965 | 75.398 | 1.00 | 27.34 |
| ATOM 2421 | CA | THR342 | 33.105 | 6.953 | 75.694 | 1.00 | 26.74 |
| ATOM 2422 | CB | THR342 | 33.681 | 8.348 | 75.553 | 1.00 | 26.75 |
| ATOM 2423 | OG1 | THR342 | 33.072 | 9.217 | 76.511 | 1.00 | 25.10 |
| ATOM 2424 | CG2 | THR342 | 33.387 | 8.881 | 74.171 | 1.00 | 29.29 |
| ATOM 2425 | C | THR342 | 33.292 | 6.477 | 77.114 | 1.00 | 27.84 |
| ATOM 2426 | O | THR342 | 34.365 | 6.625 | 77.692 | 1.00 | 27.29 |
| ATOM 2427 | N | GLY343 | 32.223 | 5.908 | 77.662 | 1.00 | 30.32 |
| ATOM 2428 | CA | GLY343 | 32.234 | 5.398 | 79.020 | 1.00 | 31.31 |
| ATOM 2429 | C | GLY343 | 32.970 | 4.083 | 79.178 | 1.00 | 32.13 |
| ATOM 2430 | O | GLY343 | 33.765 | 3.944 | 80.105 | 1.00 | 34.00 |
| ATOM 2431 | N | ASP344 | 32.712 | 3.114 | 78.304 | 1.00 | 31.93 |

[Table 2] (continued)

| | | | | | | | |
|---|---|---|---|---|---|---|---|
| ATOM 2432 | CA | ASP344 | 33.400 | 1.836 | 78.411 | 1.00 | 34.25 |
| ATOM 2433 | CB | ASP344 | 32.592 | 0.857 | 79.267 | 1.00 | 38.13 |
| ATOM 2434 | CG | ASP344 | 31.205 | 0.646 | 78.744 | 1.00 | 43.49 |
| ATOM 2435 | OD1 | ASP344 | 30.399 | -0.029 | 79.426 | 1.00 | 47.59 |
| ATOM 2436 | OD2 | ASP344 | 30.923 | 1.159 | 77.643 | 1.00 | 46.67 |
| ATOM 2437 | C | ASP344 | 33.744 | 1.196 | 77.075 | 1.00 | 33.85 |
| ATOM 2438 | O | ASP344 | 33.354 | 1.681 | 76.015 | 1.00 | 32.12 |
| ATOM 2439 | N | ARG345 | 34.490 | 0.098 | 77.148 | 1.00 | 34.54 |
| ATOM 2440 | CA | ARG345 | 34.935 | -0.626 | 75.968 | 1.00 | 35.60 |
| ATOM 2441 | CB | ARG345 | 36.297 | -1.278 | 76.233 | 1.00 | 35.33 |
| ATOM 2442 | CG | ARG345 | 37.339 | -0.370 | 76.864 | 1.00 | 35.88 |
| ATOM 2443 | CD | ARG345 | 38.729 | -1.006 | 76.879 | 1.00 | 35.19 |
| ATOM 2444 | NE | ARG345 | 39.507 | -0.597 | 78.054 | 1.00 | 36.95 |
| ATOM 2445 | CZ | ARG345 | 39.984 | 0.629 | 78.275 | 1.00 | 36.97 |
| ATOM 2446 | NH1 | ARG345 | 39.780 | 1.605 | 77.396 | 1.00 | 36.40 |
| ATOM 2447 | NH2 | ARG345 | 40.654 | 0.885 | 79.394 | 1.00 | 36.46 |
| ATOM 2448 | C | ARG345 | 33.961 | -1.716 | 75.551 | 1.00 | 36.31 |
| ATOM 2449 | O | ARG345 | 34.080 | -2.280 | 74.461 | 1.00 | 37.64 |
| ATOM 2450 | N | LYS346 | 33.004 | -2.020 | 76.420 | 1.00 | 35.01 |
| ATOM 2451 | CA | LYS346 | 32.050 | -3.081 | 76.134 | 1.00 | 33.81 |
| ATOM 2452 | CB | LYS346 | 30.824 | -2.975 | 77.041 | 1.00 | 33.64 |
| ATOM 2453 | CG | LYS346 | 29.942 | -4.223 | 76.985 | 1.00 | 33.85 |
| ATOM 2454 | CD | LYS346 | 30.759 | -5.505 | 77.186 | 1.00 | 31.48 |
| ATOM 2455 | CE | LYS346 | 30.061 | -6.699 | 76.542 | 1.00 | 32.39 |
| ATOM 2456 | NZ | LYS346 | 30.855 | -7.968 | 76.542 | 1.00 | 30.01 |
| ATOM 2457 | C | LYS346 | 31.613 | -3.093 | 74.684 | 1.00 | 33.18 |
| ATOM 2458 | O | LYS346 | 31.746 | -4.101 | 73.995 | 1.00 | 31.98 |
| ATOM 2459 | N | GLN347 | 31.101 | -1.967 | 74.214 | 1.00 | 33.36 |
| ATOM 2460 | CA | GLN347 | 30.662 | -1.887 | 72.839 | 1.00 | 34.32 |
| ATOM 2461 | CB | GLN347 | 30.014 | -0.530 | 72.589 | 1.00 | 37.17 |
| ATOM 2462 | CG | GLN347 | 28.510 | -0.578 | 72.703 | 1.00 | 39.97 |
| ATOM 2463 | CD | GLN347 | 27.905 | -1.436 | 71.611 | 1.00 | 43.97 |
| ATOM 2464 | OE1 | GLN347 | 28.219 | -2.626 | 71.491 | 1.00 | 43.88 |
| ATOM 2465 | NE2 | GLN347 | 27.039 | -0.835 | 70.799 | 1.00 | 46.46 |
| ATOM 2466 | C | GLN347 | 31.799 | -2.144 | 71.844 | 1.00 | 34.27 |
| ATOM 2467 | O | GLN347 | 31.630 | -2.922 | 70.902 | 1.00 | 35.29 |
| ATOM 2468 | N | ILE348 | 32.952 | -1.502 | 72.054 | 1.00 | 31.49 |
| ATOM 2469 | CA | ILE348 | 34.109 | -1.679 | 71.165 | 1.00 | 25.43 |
| ATOM 2470 | CB | ILE348 | 35.309 | -0.826 | 71.614 | 1.00 | 21.01 |
| ATOM 2471 | CG2 | ILE348 | 36.369 | -0.826 | 70.540 | 1.00 | 15.50 |
| ATOM 2472 | CG1 | ILE348 | 34.852 | 0.606 | 71.875 | 1.00 | 22.27 |
| ATOM 2473 | CD1 | ILE348 | 35. 914 | 1.509 | 72.462 | 1.00 | 24.55 |
| ATOM 2474 | C | ILE348 | 34.524 | -3.139 | 71.211 | 1.00 | 24.70 |
| ATOM 2475 | O | ILE348 | 34.793 | -3.763 | 70.182 | 1.00 | 23.36 |
| ATOM 2476 | N | TYR349 | 34.560 | -3.681 | 72.421 | 1.00 | 23.30 |
| ATOM 2477 | CA | TYR349 | 34.933 | -5.061 | 72.597 | 1.00 | 23.65 |
| ATOM 2478 | CB | TYR349 | 34.727 | -5.491 | 74.047 | 1.00 | 25.21 |
| ATOM 2479 | CG | TYR349 | 34.779 | -6.989 | 74.221 | 1.00 | 31.27 |
| ATOM 2480 | CD1 | TYR349 | 35.990 | -7.665 | 74.333 | 1.00 | 33.98 |
| ATOM 2481 | CE1 | TYR349 | 36.028 | -9.062 | 74.435 | 1.00 | 36.98 |
| ATOM 2482 | CD2 | TYR349 | 33.607 | -7.740 | 74.216 | 1.00 | 34.38 |

[Table 2] (continued)

| | | | | | | | |
|---|---|---|---|---|---|---|---|
| ATOM 2483 | CE2 | TYR349 | 33.628 | -9.125 | 74.312 | 1.00 | 36.69 |
| ATOM 2484 | CZ | TYR349 | 34.837 | -9.786 | 74.421 | 1.00 | 37.89 |
| ATOM 2485 | OH | TYR349 | 34.834 | -11.165 | 74.512 | 1.00 | 37.12 |
| ATOM 2486 | C | TYR349 | 34.105 | -5.945 | 71.676 | 1.00 | 23.47 |
| ATOM 2487 | O | TYR349 | 34.654 | -6.602 | 70.794 | 1.00 | 21.02 |
| ATOM 2488 | N | ASN350 | 32.783 | -5.934 | 71.872 | 1.00 | 25.29 |
| ATOM 2489 | CA | ASN350 | 31.850 | -6.766 | 71.091 | 1.00 | 25.07 |
| ATOM 2490 | CB | ASN350 | 30.379 | -6.500 | 71.482 | 1.00 | 23.90 |
| ATOM 2491 | CG | ASN350 | 30.069 | -6.844 | 72.941 | 1.00 | 25.09 |
| ATOM 2492 | OD1 | ASN350 | 30.413 | -7.924 | 73.440 | 1.00 | 22.84 |
| ATOM 2493 | ND2 | ASN350 | 29.398 | -5.923 | 73.626 | 1.00 | 25.65 |
| ATOM 2494 | C | ASN350 | 31.982 | -6.620 | 69.580 | 1.00 | 25.25 |
| ATOM 2495 | O | ASN350 | 31.994 | -7.619 | 68.859 | 1.00 | 25.84 |
| ATOM 2496 | N | ILE351 | 32.068 | -5.392 | 69.083 | 1.00 | 25.43 |
| ATOM 2497 | CA | ILE351 | 32.195 | -5.227 | 67.642 | 1.00 | 25.64 |
| ATOM 2498 | CB | ILE351 | 32.388 | -3.745 | 67.248 | 1.00 | 24.60 |
| ATOM 2499 | CG2 | ILE351 | 32.282 | -3.600 | 65.743 | 1.00 | 23.69 |
| ATOM 2500 | CG1 | ILE351 | 31.305 | -2.882 | 67.903 | 1.00 | 22.24 |
| ATOM 2501 | CD1 | ILE351 | 31.357 | -1.431 | 67.509 | 1.00 | 19.88 |
| ATOM 2502 | C | ILE351 | 33.415 | -6.047 | 67.224 | 1.00 | 26.73 |
| ATOM 2503 | O | ILE351 | 33.282 | -7.047 | 66.517 | 1.00 | 25.71 |
| ATOM 2504 | N | LEU352 | 34.592 | -5.629 | 67.695 | 1.00 | 27.08 |
| ATOM 2505 | CA | LEU352 | 35.847 | -6.312 | 67.397 | 1.00 | 27.36 |
| ATOM 2506 | CB | LEU352 | 36.994 | -5.700 | 68.206 | 1.00 | 24.45 |
| ATOM 2507 | CG | LEU352 | 37.295 | -4.208 | 68.090 | 1.00 | 23.84 |
| ATOM 2508 | CD1 | LEU352 | 38.464 | -3.838 | 68.995 | 1.00 | 21.54 |
| ATOM 2509 | CD2 | LEU352 | 37.620 | -3.872 | 66.660 | 1.00 | 23.96 |
| ATOM 2510 | C | LEU352 | 35.746 | -7.798 | 67.737 | 1.00 | 29.42 |
| ATOM 2511 | O | LEU352 | 36.045 | -8.670 | 66.912 | 1.00 | 29.43 |
| ATOM 2512 | N | SER353 | 35.336 | -8.087 | 68.965 | 1.00 | 30.73 |
| ATOM 2513 | CA | SER353 | 35.206 | -9.468 | 69.398 | 1.00 | 32.72 |
| ATOM 2514 | CB | SER353 | 34.408 | -9.531 | 70.711 | 1.00 | 32.86 |
| ATOM 2515 | OG | SER353 | 34.187 | -10.870 | 71.126 | 1.00 | 35.10 |
| ATOM 2516 | C | SER353 | 34.513 | -10.277 | 68.295 | 1.00 | 33.76 |
| ATOM 2517 | O | SER353 | 35.123 | -11.149 | 67.670 | 1.00 | 34.42 |
| ATOM 2518 | N | THR354 | 33.252 | -9.941 | 68.035 | 1.00 | 34.17 |
| ATOM 2519 | CA | THR354 | 32.437 | -10.621 | 67.031 | 1.00 | 32.96 |
| ATOM 2520 | CB | THR354 | 30.999 | -10.073 | 67.076 | 1.00 | 33.01 |
| ATOM 2521 | OG1 | THR354 | 30.120 | -10.980 | 66.408 | 1.00 | 32.52 |
| ATOM 2522 | CG2 | THR354 | 30.922 | -8.702 | 66.411 | 1.00 | 34.65 |
| ATOM 2523 | C | THR354 | 33.007 | -10.503 | 65.608 | 1.00 | 32.28 |
| ATOM 2524 | O | THR354 | 32.444 | -11.038 | 64.646 | 1.00 | 30.58 |
| ATOM 2525 | N | LEU355 | 34.137 | -9.807 | 65.497 | 1.00 | 31.47 |
| ATOM 2526 | CA | LEU355 | 34.832 | -9.612 | 64.227 | 1.00 | 30.67 |
| ATOM 2527 | CB | LEU355 | 35.488 | -8.239 | 64.187 | 1.00 | 28.42 |
| ATOM 2528 | CG | LEU355 | 34.780 | -7.240 | 63.293 | 1.00 | 27.13 |
| ATOM 2529 | CD1 | LEU355 | 35.387 | -5.874 | 63.487 | 1.00 | 26.09 |
| ATOM 2530 | CD2 | LEU355 | 34.898 | -7.698 | 61.859 | 1.00 | 27.39 |
| ATOM 2531 | C | LEU355 | 35.905 | -10.668 | 64.061 | 1.00 | 31.14 |
| ATOM 2532 | O | LEU355 | 36.573 | -10.735 | 63.033 | 1.00 | 30.59 |
| ATOM 2533 | N | GLY356 | 36.074 | -11.484 | 65.091 | 1.00 | 32.64 |

[Table 2] (continued)

| | | | | | | | |
|---|---|---|---|---|---|---|---|
| ATOM 2534 | CA | GLY356 | 37.068 | -12.530 | 65.030 | 1.00 | 35.49 |
| ATOM 2535 | C | GLY356 | 38.435 | -12.074 | 65.493 | 1.00 | 37.44 |
| ATOM 2536 | O | GLY356 | 39.443 | -12.492 | 64.930 | 1.00 | 37.31 |
| ATOM 2537 | N | LEU357 | 38.471 | -11.222 | 66.516 | 1.00 | 39.40 |
| ATOM 2538 | CA | LEU357 | 39.729 | -10.717 | 67.057 | 1.00 | 41.85 |
| ATOM 2539 | CB | LEU357 | 39.898 | -9.239 | 66.705 | 1.00 | 41.35 |
| ATOM 2540 | CG | LEU357 | 39.816 | -8.876 | 65. 218 | 1.00 | 43.17 |
| ATOM 2541 | CD1 | LEU357 | 39.953 | -7.375 | 65.064 | 1.00 | 42.98 |
| ATOM 2542 | CD2 | LEU357 | 40.904 | -9.585 | 64.428 | 1.00 | 43.93 |
| ATOM 2543 | C | LEU357 | 39.759 | -10.888 | 68.571 | 1.00 | 44.59 |
| ATOM 2544 | O | LEU357 | 38.750 | -11.247 | 69.176 | 1.00 | 45.94 |
| ATOM 2545 | N | ARG358 | 40.919 | -10.643 | 69.178 | 1.00 | 46.55 |
| ATOM 2546 | CA | ARG358 | 41.080 | -10.752 | 70.632 | 1.00 | 48.12 |
| ATOM 2547 | CB | ARG358 | 42.113 | -11.819 | 70.994 | 1.00 | 52.19 |
| ATOM 2548 | CG | ARG358 | 41.649 | -13.258 | 70.839 | 1.00 | 61.21 |
| ATOM 2549 | CD | ARG358 | 40.870 | -13.768 | 72.064 | 1.00 | 68.48 |
| ATOM2550 | NE | ARG358 | 39.519 | -13.206 | 72.184 | 1.00 | 74.00 |
| ATOM 2551 | CZ | ARG358 | 38.629 | -13.577 | 73.104 | 1.00 | 75.57 |
| ATOM 2552 | NH1 | ARG358 | 38.935 | -14.517 | 73.998 | 1.00 | 75.58 |
| ATOM 2553 | NH2 | ARG358 | 37.431 | -13.005 | 73.131 | 1.00 | 74.54 |
| ATOM 2554 | C | ARG358 | 41.558 | -9.418 | 71.174 | 1.00 | 46.76 |
| ATOM 2555 | O | ARG358 | 42.702 | -9.284 | 71.580 | 1.00 | 49.52 |
| ATOM 2556 | N | PRO359 | 40.679 | -8.412 | 71.197 | 1.00 | 45.33 |
| ATOM 2557 | CD | PRO359 | 39.271 | -8.532 | 70.791 | 1.00 | 45.90 |
| ATOM 2558 | CA | PRO359 | 40.956 | -7.056 | 71.677 | 1.00 | 44.06 |
| ATOM 2559 | CB | PRO359 | 39.565 | -6.449 | 71.784 | 1.00 | 45.14 |
| ATOM 2560 | CG | PRO359 | 38.865 | -7.086 | 70.643 | 1.00 | 46.70 |
| ATOM 2561 | C | PRO359 | 41.725 | -6.936 | 72.986 | 1.00 | 42.11 |
| ATOM 2562 | O | PRO359 | 41.662 | -7.797 | 73.860 | 1.00 | 42.98 |
| ATOM 2563 | N | SER360 | 42.449 | -5.840 | 73.118 | 1.00 | 38.55 |
| ATOM 2564 | CA | SER360 | 43.209 | -5.608 | 74.321 | 1.00 | 35.42 |
| ATOM 2565 | CB | SER360 | 44.701 | -5.624 | 74.014 | 1.00 | 38.45 |
| ATOM 2566 | OG | SER360 | 45.100 | -4.379 | 73.453 | 1.00 | 37.32 |
| ATOM 2567 | C | SER360 | 42.847 | -4.234 | 74.818 | 1.00 | 33.26 |
| ATOM 2568 | O | SER360 | 42.530 | -3.345 | 74.028 | 1.00 | 30.55 |
| ATOM 2569 | N | THR361 | 42.907 | -4.060 | 76.128 | 1.00 | 31.87 |
| ATOM 2570 | CA | THR361 | 42.625 | -2.771 | 76.721 | 1.00 | 33.02 |
| ATOM 2571 | CB | THR361 | 43.285 | -2.646 | 78.083 | 1.00 | 32.00 |
| ATOM 2572 | OG1 | THR361 | 42.697 | -3.593 | 78.981 | 1.00 | 31.30 |
| ATOM 2573 | CG2 | THR361 | 43.135 | -1.223 | 78.618 | 1.00 | 28.90 |
| ATOM 2574 | C | THR361 | 43.162 | -1.637 | 75.853 | 1.00 | 35.59 |
| ATOM 2575 | O | THR361 | 42.600 | -0.545 | 75.837 | 1.00 | 37.16 |
| ATOM 2576 | N | THR362 | 44.253 | -1.879 | 75.135 | 1.00 | 37.62 |
| ATOM 2577 | CA | THR362 | 44.812 | -0.819 | 74.303 | 1.00 | 37.63 |
| ATOM 2578 | CB | THR362 | 46.341 | -0.949 | 74.156 | 1.00 | 38.04 |
| ATOM 2579 | OG1 | THR362 | 46.950 | -0.981 | 75.453 | 1.00 | 37.77 |
| ATOM 2580 | CG2 | THR362 | 46.890 | 0.242 | 73.395 | 1.00 | 37.49 |
| ATOM 2581 | C | THR362 | 44.183 | -0.839 | 72.928 | 1.00 | 36.67 |
| ATOM 2582 | O | THR362 | 43.758 | 0.194 | 72.416 | 1.00 | 34.48 |
| ATOM 2583 | N | ASP363 | 44.132 | -2.032 | 72.345 | 1.00 | 37.88 |
| ATOM 2584 | CA | ASP363 | 43.555 | -2.246 | 71.024 | 1.00 | 40.18 |

[Table 2] (continued)

| | | | | | | | |
|---|---|---|---|---|---|---|---|
| ATOM 2585 | CB | ASP363 | 43.238 | -3.729 | 70.842 | 1.00 | 42.13 |
| ATOM 2586 | CG | ASP363 | 44.477 | -4.557 | 70.666 | 1.00 | 45.73 |
| ATOM 2587 | OD1 | ASP363 | 44.433 | -5.779 | 70.932 | 1.00 | 49.54 |
| ATOM 2588 | OD2 | ASP363 | 45.500 | -3.976 | 70.247 | 1.00 | 46.04 |
| ATOM 2589 | C | ASP363 | 42.289 | -1.429 | 70.841 | 1.00 | 40.28 |
| ATOM 2590 | O | ASP363 | 42.070 | -0.801 | 69.802 | 1.00 | 38.03 |
| ATOM 2591 | N | CYS364 | 41.455 | -1.449 | 71.871 | 1.00 | 41.60 |
| ATOM 2592 | CA | CYS364 | 40.197 | -0.724 | 71.849 | 1.00 | 41.33 |
| ATOM 2593 | CB | CYS364 | 39.426 | -1.036 | 73.131 | 1.00 | 41.81 |
| ATOM 2594 | SG | CYS364 | 39.078 | -2.818 | 73.225 | 1.00 | 41.98 |
| ATOM 2595 | C | CYS364 | 40.447 | 0.766 | 71.685 | 1.00 | 39.78 |
| ATOM 2596 | O | CYS364 | 39.991 | 1.382 | 70.721 | 1.00 | 37.44 |
| ATOM 2597 | N | ASP365 | 41.194 | 1.333 | 72.622 | 1.00 | 38.65 |
| ATOM 2598 | CA | ASP365 | 41.525 | 2.744 | 72.580 | 1.00 | 37.87 |
| ATOM 2599 | CB | ASP365 | 42.498 | 3.060 | 73.709 | 1.00 | 39.53 |
| ATOM 2600 | CG | ASP365 | 42.073 | 2.424 | 75.014 | 1.00 | 42.28 |
| ATOM 2601 | OD1 | ASP365 | 40.887 | 2.000 | 75.096 | 1.00 | 43.06 |
| ATOM 2602 | OD2 | ASP365 | 42.908 | 2.355 | 75.949 | 1.00 | 41.82 |
| ATOM 2603 | C | ASP365 | 42.123 | 3.092 | 71.220 | 1.00 | 35.70 |
| ATOM 2604 | O | ASP365 | 41.887 | 4.173 | 70.682 | 1.00 | 35.49 |
| ATOM 2605 | N | ILE366 | 42.895 | 2.175 | 70.655 | 1.00 | 32.72 |
| ATOM 2606 | CA | ILE366 | 43.469 | 2.428 | 69.347 | 1.00 | 31.21 |
| ATOM 2607 | CB | ILE366 | 44.345 | 1.241 | 68.891 | 1.00 | 30.98 |
| ATOM 2608 | CG2 | ILE366 | 44.878 | 1.488 | 67.482 | 1.00 | 30.08 |
| ATOM 2609 | CG1 | ILE366 | 45.472 | 1.010 | 69.907 | 1.00 | 30.05 |
| ATOM 2610 | CD1 | ILE366 | 46.426 | 2.165 | 70.071 | 1.00 | 26.19 |
| ATOM 2611 | C | ILE366 | 42.292 | 2.622 | 68.384 | 1.00 | 30.65 |
| ATOM 2612 | O | ILE366 | 42.140 | 3.686 | 67.790 | 1.00 | 29.65 |
| ATOM 2613 | N | VAL367 | 41.451 | 1.598 | 68.255 | 1.00 | 29.81 |
| ATOM 2614 | CA | VAL367 | 40.287 | 1.665 | 67.378 | 1.00 | 27.24 |
| ATOM 2615 | CB | VAL367 | 39.397 | 0.424 | 67.541 | 1.00 | 26.77 |
| ATOM 2616 | CG1 | VAL367 | 38.193 | 0.520 | 66.630 | 1.00 | 25.16 |
| ATOM 2617 | CG2 | VAL367 | 40.190 | -0.817 | 67.220 | 1.00 | 27.90 |
| ATOM 2618 | C | VAL367 | 39.453 | 2.910 | 67.657 | 1.00 | 26.82 |
| ATOM 2619 | O | VAL367 | 39.061 | 3.606 | 66.727 | 1.00 | 27.16 |
| ATOM 2620 | N | ARG368 | 39.171 | 3.191 | 68.927 | 1.00 | 25.49 |
| ATOM 2621 | CA | ARG368 | 38.398 | 4.380 | 69.266 | 1.00 | 24.26 |
| ATOM 2622 | CB | ARG368 | 38.431 | 4.644 | 70.772 | 1.00 | 23.73 |
| ATOM 2623 | CG | ARG368 | 37.765 | 5.951 | 71.217 | 1.00 | 26.32 |
| ATOM 2624 | CD | ARG368 | 36.239 | 5.948 | 71.033 | 1.00 | 32.00 |
| ATOM 2625 | NE | ARG368 | 35.542 | 5.015 | 71.926 | 1.00 | 33.36 |
| ATOM 2626 | CZ | ARG368 | 35.558 | 5.096 | 73.253 | 1.00 | 33.30 |
| ATOM 2627 | NH1 | ARG368 | 36.237 | 6.069 | 73.843 | 1.00 | 36.87 |
| ATOM 2628 | NH2 | ARG368 | 34.904 | 4.209 | 73.990 | 1.00 | 30.08 |
| ATOM 2629 | C | ARG368 | 39.034 | 5.545 | 68.539 | 1.00 | 25.24 |
| ATOM 2630 | O | ARG368 | 38.403 | 6.175 | 67.700 | 1.00 | 26.08 |
| ATOM 2631 | N | ARG369 | 40.299 | 5.808 | 68.844 | 1.00 | 26.69 |
| ATOM 2632 | CA | ARG369 | 41.022 | 6.905 | 68.226 | 1.00 | 28.80 |
| ATOM 2633 | CB | ARG369 | 42.500 | 6.842 | 68.619 | 1.00 | 33.81 |
| ATOM 2634 | CG | ARG369 | 42.992 | 8.041 | 69.421 | 1.00 | 41.54 |
| ATOM 2635 | CD | ARG369 | 44.246 | 8.666 | 68.797 | 1.00 | 47.78 |

[Table 2] (continued)

| | | | | | | | |
|---|---|---|---|---|---|---|---|
| ATOM 2636 | NE | ARG369 | 44.827 | 9.709 | 69.642 | 1.00 | 53.83 |
| ATOM 2637 | CZ | ARG369 | 45.436 | 9.479 | 70.803 | 1.00 | 57.34 |
| ATOM 2638 | NH1 | ARG369 | 45.547 | 8.234 | 71.256 | 1.00 | 57.39 |
| ATOM 2639 | NH2 | ARG369 | 45.925 | 10.492 | 71.517 | 1.00 | 58.51 |
| ATOM 2640 | C | ARG369 | 40.888 | 6.941 | 66.704 | 1.00 | 27.66 |
| ATOM 2641 | O | ARG369 | 40.898 | 8.017 | 66.116 | 1.00 | 27.35 |
| ATOM 2642 | N | ALA370 | 40.760 | 5.778 | 66.071 | 1.00 | 28.23 |
| ATOM 2643 | CA | ALA370 | 40.622 | 5.699 | 64.613 | 1.00 | 29.69 |
| ATOM 2644 | CB | ALA370 | 40.779 | 4.264 | 64.144 | 1.00 | 27.18 |
| ATOM 2645 | C | ALA370 | 39.266 | 6.218 | 64.184 | 1.00 | 32.49 |
| ATOM 2646 | O | ALA370 | 39.155 | 7.084 | 63.313 | 1.00 | 33.37 |
| ATOM 2647 | N | CYS371 | 38.229 | 5.663 | 64.797 | 1.00 | 35.80 |
| ATOM 2648 | CA | CYS371 | 36.860 | 6.053 | 64.500 | 1.00 | 37.09 |
| ATOM 2649 | CB | CYS371 | 35.892 | 5.310 | 65.427 | 1.00 | 37.67 |
| ATOM 2650 | SG | CYS371 | 35.709 | 3.539 | 65.052 | 1.00 | 43.56 |
| ATOM 2651 | C | CYS371 | 36.692 | 7.555 | 64.663 | 1.00 | 36.66 |
| ATOM 2652 | O | CYS371 | 36.237 | 8.231 | 63.746 | 1.00 | 36.14 |
| ATOM 2653 | N | GLU372 | 37.079 | 8.065 | 65.829 | 1.00 | 36.70 |
| ATOM 2654 | CA | GLU372 | 36.962 | 9.482 | 66.140 | 1.00 | 37.83 |
| ATOM 2655 | CB | GLU372 | 37.440 | 9.741 | 67.569 | 1.00 | 41.72 |
| ATOM 2656 | CG | GLU372 | 37.405 | 11.202 | 67.993 | 1.00 | 50.44 |
| ATOM 2657 | CD | GLU372 | 38.615 | 11.981 | 67.504 | 1.00 | 56.78 |
| ATOM 2658 | OE1 | GLU372 | 39.747 | 11.656 | 67.940 | 1.00 | 60.05 |
| ATOM 2659 | OE2 | GLU372 | 38.437 | 12.914 | 66.685 | 1.00 | 59.31 |
| ATOM 2660 | C | GLU372 | 37.736 | 10.344 | 65.163 | 1.00 | 36.14 |
| ATOM 2661 | O | GLU372 | 37.280 | 11.410 | 64.745 | 1.00 | 34.24 |
| ATOM 2662 | N | SER373 | 38.917 | 9.890 | 64.793 | 1.00 | 37.31 |
| ATOM 2663 | CA | SER373 | 39.703 | 10.662 | 63.856 | 1.00 | 39.48 |
| ATOM 2664 | CB | SER373 | 41.095 | 10.040 | 63.694 | 1.00 | 40.54 |
| ATOM 2665 | OG | SER373 | 41.014 | 8.697 | 63.253 | 1.00 | 41.31 |
| ATOM 2666 | C | SER373 | 38.966 | 10.713 | 62.516 | 1.00 | 38.54 |
| ATOM 2667 | O | SER373 | 38.778 | 11.790 | 61.953 | 1.00 | 39.30 |
| ATOM 2668 | N | VAL374 | 38.528 | 9.552 | 62.029 | 1.00 | 35.74 |
| ATOM 2669 | CA | VAL374 | 37.817 | 9.462 | 60.755 | 1.00 | 34.53 |
| ATOM 2670 | CB | VAL374 | 37.519 | 7.987 | 60.388 | 1.00 | 33.30 |
| ATOM 2671 | CG1 | VAL374 | 36.688 | 7.897 | 59.119 | 1.00 | 30.40 |
| ATOM 2672 | CG2 | VAL374 | 38.811 | 7.257 | 60.186 | 1.00 | 34.78 |
| ATOM 2673 | C | VAL374 | 36.512 | 10.250 | 60.736 | 1.00 | 35.17 |
| ATOM 2674 | O | VAL374 | 36.253 | 11.010 | 59.797 | 1.00 | 34.51 |
| ATOM 2675 | N | SER375 | 35.700 | 10.080 | 61.775 | 1.00 | 35.24 |
| ATOM 2676 | CA | SER375 | 34.416 | 10.768 | 61.866 | 1.00 | 34.91 |
| ATOM 2677 | CB | SER375 | 33.641 | 10.312 | 63.103 | 1.00 | 35.91 |
| ATOM 2678 | OG | SER375 | 33.802 | 11.230 | 64.178 | 1.00 | 37.28 |
| ATOM 2679 | C | SER375 | 34.585 | 12.272 | 61.933 | 1.00 | 34.67 |
| ATOM 2680 | O | SER375 | 33.865 | 13.010 | 61.266 | 1.00 | 35.17 |
| ATOM 2681 | N | THR376 | 35.534 | 12.725 | 62.743 | 1.00 | 34.00 |
| ATOM 2682 | CA | THR376 | 35.768 | 14.150 | 62.889 | 1.00 | 35.55 |
| ATOM 2683 | CB | THR376 | 36.827 | 14.421 | 63.954 | 1.00 | 38.06 |
| ATOM 2684 | OG1 | THR376 | 36.461 | 13.739 | 65.158 | 1.00 | 40.51 |
| ATOM 2685 | CG2 | THR376 | 36.926 | 15.923 | 64.239 | 1.00 | 38.22 |
| ATOM 2686 | C | THR376 | 36.208 | 14.788 | 61.583 | 1.00 | 34.80 |

[Table 2] (continued)

| | | | | | | | |
|---|---|---|---|---|---|---|---|
| ATOM 2687 | O | THR376 | 35.794 | 15.901 | 61.241 | 1.00 | 32-23 |
| ATOM 2688 | N | ARG377 | 37.049 | 14.078 | 60.848 | 1.00 | 36.51 |
| ATOM 2689 | CA | ARG377 | 37.523 | 14.601 | 59.581 | 1.00 | 38.20 |
| ATOM 2690 | CB | ARG377 | 38.535 | 13.640 | 58.956 | 1.00 | 41.90 |
| ATOM 2691 | CG | ARG377 | 39.417 | 14.271 | 57.892 | 1.00 | 43.83 |
| ATOM 2692 | CD | ARG377 | 38.735 | 14.280 | 56.551 | 1.00 | 46.24 |
| ATOM 2693 | NE | ARG377 | 38.467 | 12.921 | 56.074 | 1.00 | 50.02 |
| ATOM 2694 | CZ | ARG377 | 39.400 | 12.058 | 55.679 | 1.00 | 48.89 |
| ATOM 2695 | NH1 | ARG377 | 40.681 | 12.405 | 55.700 | 1.00 | 47.77 |
| ATOM 2696 | NH2 | ARG377 | 39.050 | 10.849 | 55.256 | 1.00 | 48.65 |
| ATOM 2697 | C | ARG377 | 36.311 | 14.759 | 58.688 | 1.00 | 37.15 |
| ATOM 2698 | O | ARG377 | 36.163 | 15.780 | 58.016 | 1.00 | 37.23 |
| ATOM 2699 | N | ALA378 | 35.445 | 13.744 | 58.706 | 1.00 | 36.43 |
| ATOM 2700 | CA | ALA378 | 34.212 | 13.732 | 57.920 | 1.00 | 35.58 |
| ATOM 2701 | CB | ALA378 | 33.470 | 12.430 | 58.130 | 1.00 | 35.75 |
| ATOM 2702 | C | ALA378 | 33.314 | 14.897 | 58.304 | 1.00 | 34.75 |
| ATOM 2703 | O | ALA378 | 32.675 | 15.507 | 57.451 | 1.00 | 34.63 |
| ATOM 2704 | N | ALA379 | 33.249 | 15.204 | 59.590 | 1.00 | 34.17 |
| ATOM 2705 | CA | ALA379 | 32.427 | 16.317 | 60.009 | 1.00 | 34.54 |
| ATOM 2706 | CB | ALA379 | 32.281 | 16.340 | 61.515 | 1.00 | 32.43 |
| ATOM 2707 | C | ALA379 | 33.073 | 17.607 | 59.519 | 1.00 | 35.95 |
| ATOM 2708 | O | ALA379 | 32.465 | 18.358 | 58.761 | 1.00 | 38.27 |
| ATOM 2709 | N | HIS380 | 34.314 | 17.856 | 59.925 | 1.00 | 35.13 |
| ATOM 2710 | CA | HIS380 | 34.994 | 19.083 | 59.526 | 1.00 | 34.04 |
| ATOM 2711 | CB | HIS380 | 36.448 | 19.031 | 59.968 | 1.00 | 37.01 |
| ATOM 2712 | CG | HIS380 | 36.628 | 19.284 | 61.430 | 1.00 | 42.02 |
| ATOM 2713 | CD2 | HIS380 | 35.734 | 19.637 | 62.385 | 1.00 | 43.27 |
| ATOM 2714 | ND1 | HIS380 | 37.852 | 19.206 | 62.058 | 1.00 | 44.66 |
| ATOM 2715 | CE1 | HIS380 | 37.704 | 19.500 | 63.339 | 1.00 | 46.06 |
| ATOM 2716 | NE2 | HIS380 | 36.429 | 19.766 | 63.562 | 1.00 | 44.63 |
| ATOM 2717 | C | HIS380 | 34.894 | 19.405 | 58.045 | 1.00 | 32.37 |
| ATOM 2718 | O | HIS380 | 34.581 | 20.536 | 57.671 | 1.00 | 29.98 |
| ATOM 2719 | N | MET381 | 35.154 | 18.417 | 57.197 | 1.00 | 30.55 |
| ATOM 2720 | CA | MET381 | 35.055 | 18.640 | 55.764 | 1.00 | 30.35 |
| ATOM 2721 | CB | MET381 | 35.383 | 17.365 | 54.992 | 1.00 | 28.41 |
| ATOM 2722 | CG | MET381 | 36.852 | 17.181 | 54.767 | 1.00 | 28.31 |
| ATOM 2723 | SD | MET381 | 37.505 | 18.684 | 54.017 | 1.00 | 31.73 |
| ATOM 2724 | CE | MET381 | 38.142 | 18.070 | 52.446 | 1.00 | 30.02 |
| ATOM 2725 | C | MET381 | 33.647 | 19.101 | 55.415 | 1.00 | 32.29 |
| ATOM 2726 | O | MET381 | 33.453 | 19.930 | 54.527 | 1.00 | 32.42 |
| ATOM 2727 | N | CYS382 | 32.660 | 18.566 | 56.124 | 1.00 | 33.02 |
| ATOM 2728 | CA | CYS382 | 31.279 | 18.942 | 55.869 | 1.00 | 33.44 |
| ATOM 2729 | CB | CYS382 | 30.323 | 18.012 | 56.625 | 1.00 | 33.78 |
| ATOM 2730 | SG | CYS382 | 28.582 | 18.152 | 56.124 | 1.00 | 40.21 |
| ATOM 2731 | C | CYS382 | 31.087 | 20.387 | 56.316 | 1.00 | 33.02 |
| ATOM 2732 | O | CYS382 | 30.566 | 21.218 | 55.563 | 1.00 | 32.71 |
| ATOM 2733 | N | SER383 | 31.528 | 20.686 | 57.537 | 1.00 | 33.57 |
| ATOM 2734 | CA | SER383 | 31.418 | 22.037 | 58.097 | 1.00 | 33.39 |
| ATOM 2735 | CB | SER383 | 32.232 | 22.159 | 59.392 | 1.00 | 32.88 |
| ATOM 2736 | OG | SER383 | 33.605 | 21.877 | 59.176 | 1.00 | 31.29 |
| ATOM 2737 | C | SER383 | 31.935 | 23.042 | 57.085 | 1.00 | 32.50 |

[Table 2] (continued)

| | | | | | | | |
|---|---|---|---|---|---|---|---|
| ATOM 2738 | O | SER383 | 31.314 | 24.073 | 56.832 | 1.00 | 32.64 |
| ATOM 2739 | N | ALA384 | 33.082 | 22.729 | 56.501 | 1.00 | 30.75 |
| ATOM 2740 | CA | ALA384 | 33.663 | 23.607 | 55.510 | 1.00 | 29.62 |
| ATOM 2741 | CB | ALA384 | 34.787 | 22.885 | 54.789 | 1.00 | 29.04 |
| ATOM 2742 | C | ALA384 | 32.604 | 24.095 | 54.509 | 1.00 | 29.94 |
| ATOM 2743 | O | ALA384 | 32.211 | 25.259 | 54.544 | 1.00 | 28.35 |
| ATOM 2744 | N | GLY385 | 32.141 | 23.193 | 53.639 | 1.00 | 31.38 |
| ATOM 2745 | CA | GLY385 | 31.149 | 23.525 | 52.621 | 1.00 | 30.00 |
| ATOM 2746 | C | GLY385 | 29.870 | 24.198 | 53.090 | 1.00 | 30.54 |
| ATOM 2747 | O | GLY385 | 29.522 | 25.285 | 52.613 | 1.00 | 28.88 |
| ATOM 2748 | N | LEU386 | 29.151 | 23.559 | 54.010 | 1.00 | 29.58 |
| ATOM 2749 | CA | LEU386 | 27.917 | 24.148 | 54.522 | 1.00 | 28.86 |
| ATOM 2750 | CB | LEU386 | 27.410 | 23.374 | 55.749 | 1.00 | 25.55 |
| ATOM 2751 | CG | LEU386 | 26.141 | 23.824 | 56.493 | 1.00 | 21.28 |
| ATOM 2752 | CD1 | LEU386 | 26.504 | 24.768 | 57.605 | 1.00 | 18.56 |
| ATOM 2753 | CD2 | LEU386 | 25.157 | 24.456 | 55.533 | 1.00 | 17.77 |
| ATOM 2754 | C | LEU386 | 28.199 | 25.595 | 54.898 | 1.00 | 30.29 |
| ATOM 2755 | O | LEU386 | 27.344 | 26.458 | 54.728 | 1.00 | 30.86 |
| ATOM 2756 | N | ALA387 | 29.413 | 25.846 | 55.393 | 1.00 | 32.40 |
| ATOM 2757 | CA | ALA387 | 29.851 | 27.184 | 55.799 | 1.00 | 32.84 |
| ATOM 2758 | CB | ALA387 | 31.181 | 27.101 | 56.536 | 1.00 | 31.99 |
| ATOM 2759 | C | ALA387 | 29.991 | 28.098 | 54.585 | 1.00 | 34.20 |
| ATOM 2760 | O | ALA387 | 29.509 | 29.235 | 54.588 | 1.00 | 34.34 |
| ATOM 2761 | N | GLY388 | 30.663 | 27.597 | 53.553 | 1.00 | 34.88 |
| ATOM 2762 | CA | GLY388 | 30.831 | 28.378 | 52.344 | 1.00 | 35.13 |
| ATOM 2763 | C | GLY388 | 29.467 | 28.833 | 51.867 | 1.00 | 35.42 |
| ATOM 2764 | O | GLY388 | 29.257 | 30.005 | 51.545 | 1.00 | 36.39 |
| ATOM 2765 | N | VAL 389 | 28.524 | 27.898 | 51.839 | 1.00 | 34.42 |
| ATOM 2766 | CA | VAL389 | 27.167 | 28.202 | 51.402 | 1.00 | 32.28 |
| ATOM 2767 | CB | VAL389 | 26.266 | 26.949 | 51.487 | 1.00 | 31.56 |
| ATOM 2768 | CG1 | VAL389 | 24.856 | 27.285 | 51.027 | 1.00 | 28.68 |
| ATOM 2769 | CG2 | VAL389 | 26.853 | 25.836 | 50.638 | 1.00 | 28.05 |
| ATOM 2770 | C | VAL389 | 26.579 | 29.307 | 52.273 | 1.00 | 30.86 |
| ATOM 2771 | O | VAL389 | 26.072 | 30.304 | 51.762 | 1.00 | 26.91 |
| ATOM 2772 | N | ILE390 | 26.665 | 29.115 | 53.586 | 1.00 | 31.06 |
| ATOM 2773 | CA | ILE390 | 26.146 | 30.073 | 54.548 | 1.00 | 34.83 |
| ATOM 2774 | CB | ILE390 | 26.262 | 29.538 | 56.001 | 1.00 | 32.76 |
| ATOM 2775 | CG2 | ILE390 | 25.733 | 30.562 | 56.996 | 1.00 | 31.45 |
| ATOM 2776 | CG1 | ILE390 | 25.425 | 28.274 | 56.154 | 1.00 | 32.06 |
| ATOM 2777 | CD1 | ILE390 | 25.311 | 27.804 | 57.572 | 1.00 | 31.73 |
| ATOM 2778 | C | ILE390 | 26.858 | 31.415 | 54.444 | 1.00 | 39.01 |
| ATOM 2779 | O | ILE390 | 26.209 | 32.465 | 54.370 | 1.00 | 42.11 |
| ATOM 2780 | N | ASN391 | 28.186 | 31.398 | 54.437 | 1.00 | 41.07 |
| ATOM 2781 | CA | ASN391 | 28.921 | 32.652 | 54.326 | 1.00 | 42.97 |
| ATOM 2782 | CB | ASN391 | 30.430 | 32.386 | 54.290 | 1.00 | 47.00 |
| ATOM 2783 | CG | ASN391 | 31.061 | 32.452 | 55.678 | 1.00 | 51.32 |
| ATOM 2784 | OD1 | ASN391 | 32.205 | 32.029 | 55.878 | 1.00 | 51.98 |
| ATOM 2785 | ND2 | ASN391 | 30.312 | 32.996 | 56.646 | 1.00 | 51.87 |
| ATOM 2786 | C | ASN391 | 28.459 | 33.377 | 53.070 | 1.00 | 42.59 |
| ATOM 2787 | O | ASN391 | 27.927 | 34.488 | 53.141 | 1.00 | 40.64 |
| ATOM 2788 | N | ARG392 | 28.638 | 32.723 | 51.928 | 1.00 | 43.30 |

[Table 2] (continued)

| | | | | | | | |
|---|---|---|---|---|---|---|---|
| ATOM 2789 | CA | ARG392 | 28.237 | 33.277 | 50.644 | 1.00 | 45.75 |
| ATOM 2790 | CB | ARG392 | 28.328 | 32.182 | 49.571 | 1.00 | 48.20 |
| ATOM 2791 | CG | ARG392 | 27.020 | 31.811 | 48.892 | 1.00 | 54.22 |
| ATOM 2792 | CD | ARG392 | 26.803 | 32.578 | 47.590 | 1.00 | 59.36 |
| ATOM 2793 | NE | ARG392 | 27.491 | 31.984 | 46.437 | 1.00 | 66.46 |
| ATOM 2794 | CZ | ARG392 | 28.794 | 32.098 | 46.156 | 1.00 | 69.91 |
| ATOM 2795 | NH1 | ARG392 | 29.613 | 32.793 | 46.941 | 1.00 | 69.61 |
| ATOM 2796 | NH2 | ARG392 | 29.279 | 31.524 | 45.063 | 1.00 | 70.50 |
| ATOM 2797 | C | ARG392 | 26.822 | 33.854 | 50.711 | 1.00 | 45.30 |
| ATOM 2798 | O | ARG392 | 26.474 | 34.777 | 49.973 | 1.00 | 44.35 |
| ATOM 2799 | N | MET393 | 26.009 | 33.316 | 51.607 | 1.00 | 47.21 |
| ATOM 2800 | CA | MET393 | 24.640 | 33.785 | 51.739 | 1.00 | 50.87 |
| ATOM 2801 | CB | MET393 | 23.761 | 32.687 | 52.346 | 1.00 | 49.84 |
| ATOM 2802 | CG | MET393 | 23.427 | 31.551 | 51.389 | 1.00 | 45.97 |
| ATOM 2803 | SD | MET393 | 22.244 | 30.416 | 52.096 | 1.00 | 42.67 |
| ATOM 2804 | CE | MET393 | 20.761 | 31.465 | 52.244 | 1.00 | 42.67 |
| ATOM 2805 | C | MET393 | 24.559 | 35.046 | 52.581 | 1.00 | 54.43 |
| ATOM 2806 | O | MET393 | 23.631 | 35.851 | 52.443 | 1.00 | 53.85 |
| ATOM 2807 | N | ARG394 | 25.528 | 35.208 | 53.469 | 1.00 | 59.09 |
| ATOM 2808 | CA | ARG394 | 25.568 | 36.386 | 54.314 | 1.00 | 64.57 |
| ATOM 2809 | CB | ARG394 | 26.624 | 36.224 | 55.404 | 1.00 | 65.91 |
| ATOM 2810 | CG | ARG394 | 26.830 | 37.477 | 56.228 | 1.00 | 67.95 |
| ATOM 2811 | CD | ARG394 | 28.048 | 37.364 | 57.130 | 1.00 | 69.02 |
| ATOM 2812 | NE | ARG394 | 28.499 | 38.673 | 57.600 | 1.00 | 68.97 |
| ATOM 2813 | CZ | ARG394 | 27.776 | 39.494 | 58.357 | 1.00 | 69.21 |
| ATOM 2814 | NH1 | ARG394 | 26.553 | 39.151 | 58.743 | 1.00 | 70.35 |
| ATOM 2815 | NH2 | ARG394 | 28.281 | 40.662 | 58.732 | 1.00 | 68.24 |
| ATOM 2816 | C | ARG394 | 25.952 | 37.537 | 53.404 | 1.00 | 67.94 |
| ATOM 2817 | O | ARG394 | 25.306 | 38.588 | 53.391 | 1.00 | 67.14 |
| ATOM 2818 | N | GLU395 | 27.012 | 37.313 | 52.633 | 1.00 | 72.08 |
| ATOM 2819 | CA | GLU395 | 27.513 | 38.314 | 51.707 | 1.00 | 77.04 |
| ATOM 2820 | CB | GLU395 | 28.578 | 37.691 | 50.784 | 1.00 | 78.09 |
| ATOM 2821 | CG | GLU395 | 29.425 | 38.685 | 49.955 | 1.00 | 81.99 |
| ATOM 2822 | CD | GLU395 | 30.402 | 39.533 | 50.789 | 1.00 | 84.19 |
| ATOM 2823 | OE1 | GLU395 | 29.949 | 40.442 | 51.526 | 1.00 | 83.64 |
| ATOM 2824 | OE2 | GLU395 | 31.631 | 39.290 | 50.702 | 1.00 | 84.22 |
| ATOM 2825 | C | GLU395 | 26.340 | 38.873 | 50.898 | 1.00 | 79.30 |
| ATOM 2826 | O | GLU395 | 26.250 | 40.078 | 50.683 | 1.00 | 81.15 |
| ATOM 2827 | N | SER396 | 25.423 | 38.007 | 50.481 | 1.00 | 81.59 |
| ATOM 2828 | CA | SER396 | 24.276 | 38.451 | 49.696 | 1.00 | 83.40 |
| ATOM 2829 | CB | SER396 | 23.379 | 37.264 | 49.366 | 1.00 | 84.05 |
| ATOM 2830 | OG | SER396 | 24.123 | 36.252 | 48.716 | 1.00 | 86.28 |
| ATOM 2831 | C | SER396 | 23.462 | 39.526 | 50.406 | 1.00 | 84.36 |
| ATOM 2832 | O | SER396 | 23.578 | 40.708 | 50.092 | 1.00 | 84.49 |
| ATOM 2833 | N | ARG397 | 22.639 | 39.118 | 51.362 | 1.00 | 86.41 |
| ATOM 2834 | CA | ARG397 | 21.812 | 40.070 | 52.090 | 1.00 | 88.71 |
| ATOM 2835 | CB | ARG397 | 20.682 | 39.335 | 52.816 | 1.00 | 89.74 |
| ATOM 2836 | CG | ARG397 | 19.579 | 40.241 | 53.346 | 1.00 | 90.87 |
| ATOM 2837 | CD | ARG397 | 19.096 | 39.776 | 54.713 | 1.00 | 91.04 |
| ATOM 2838 | NE | ARG397 | 20.021 | 40.158 | 55.782 | 1.00 | 89.87 |
| ATOM 2839 | CZ | ARG397 | 19.905 | 39.766 | 57.047 | 1.00 | 89.80 |

[Table 2] (continued)

| | | | | | | | |
|---|---|---|---|---|---|---|---|
| ATOM 2840 | NH1 | ARG397 | 18.906 | 38.971 | 57.409 | 1.00 | 91.15 |
| ATOM 2841 | NH2 | ARG397 | 20.779 | 40.174 | 57.955 | 1.00 | 87.97 |
| ATOM 2842 | C | ARG397 | 22.653 | 40.847 | 53.102 | 1.00 | 89.74 |
| ATOM 2843 | O | ARG397 | 22.585 | 40.588 | 54.305 | 1.00 | 90.41 |
| ATOM 2844 | N | SER398 | 23.448 | 41.795 | 52.614 | 1.00 | 90.58 |
| ATOM 2845 | CA | SER398 | 24.288 | 42.602 | 53.492 | 1.00 | 91.09 |
| ATOM 2846 | CB | SER398 | 24.903 | 43.782 | 52.718 | 1.00 | 91.14 |
| ATOM 2847 | OG | SER398 | 25.845 | 43.347 | 51.747 | 1.00 | 89.49 |
| ATOM 2848 | C | SER398 | 23.470 | 43.129 | 54.677 | 1.00 | 91.27 |
| ATOM 2849 | O | SER398 | 22.458 | 43.810 | 54.496 | 1.00 | 91.10 |
| ATOM 2850 | N | GLU399 | 23.904 | 42.786 | 55.887 | 1.00 | 91.43 |
| ATOM 2851 | CA | GLU399 | 23.238 | 43.233 | 57.108 | 1.00 | 90.89 |
| ATOM 2852 | CB | GLU399 | 21.799 | 42.705 | 57.183 | 1.00 | 91.87 |
| ATOM 2853 | CG | GLU399 | 20.969 | 43.349 | 58.298 | 1.00 | 93.31 |
| ATOM 2854 | CD | GLU399 | 20.726 | 44.836 | 58.064 | 1.00 | 94.22 |
| ATOM 2855 | OE1 | GLU399 | 20.270 | 45.533 | 58.999 | 1.00 | 93.53 |
| ATOM 2856 | OE2 | GLU399 | 20.986 | 45.307 | 56.936 | 1.00 | 94.80 |
| ATOM 2857 | C | GLU399 | 24.013 | 42.774 | 58.339 | 1.00 | 89.25 |
| ATOM 2858 | O | GLU399 | 24.987 | 42.029 | 58.236 | 1.00 | 88.96 |
| ATOM 2859 | N | ASP400 | 23.570 | 43.226 | 59.502 | 1.00 | 87.38 |
| ATOM 2860 | CA | ASP400 | 24.214 | 42.883 | 60.754 | 1.00 | 85.70 |
| ATOM 2861 | CB | ASP400 | 23.332 | 43.352 | 61.915 | 1.00 | 88.35 |
| ATOM 2862 | CG | ASP400 | 22.861 | 44.795 | 61.743 | 1.00 | 90.64 |
| ATOM 2863 | OD1 | ASP400 | 22.059 | 45.055 | 60.817 | 1.00 | 91.42 |
| ATOM 2864 | OD2 | ASP400 | 23.297 | 45.671 | 62.524 | 1.00 | 91.68 |
| ATOM 2865 | C | ASP400 | 24.496 | 41.385 | 60.853 | 1.00 | 82.94 |
| ATOM 2866 | O | ASP400 | 25.506 | 40.900 | 60.346 | 1.00 | 82.03 |
| ATOM 2867 | N | VAL401 | 23.593 | 40.658 | 61.502 | 1.00 | 79.90 |
| ATOM 2868 | CA | VAL401 | 23.738 | 39.219 | 61.682 | 1.00 | 75.91 |
| ATOM 2869 | CB | VAL401 | 23.607 | 38.841 | 63.153 | 1.00 | 74.20 |
| ATOM 2870 | CG1 | VAL401 | 24.803 | 39.343 | 63.927 | 1.00 | 73.12 |
| ATOM 2871 | CG2 | VAL401 | 22.314 | 39.430 | 63.710 | 1.00 | 72.79 |
| ATOM 2872 | C | VAL401 | 22.662 | 38.458 | 60.925 | 1.00 | 74.63 |
| ATOM 2873 | O | VAL401 | 21.489 | 38.846 | 60.942 | 1.00 | 75.56 |
| ATOM 2874 | N | MET402 | 23.063 | 37.365 | 60.278 | 1.00 | 70.61 |
| ATOM 2875 | CA | MET402 | 22.130 | 36.539 | 59.521 | 1.00 | 65.65 |
| ATOM 2876 | CB | MET402 | 22.818 | 35.887 | 58.325 | 1.00 | 62.74 |
| ATOM 2877 | CG | MET402 | 21.897 | 34.958 | 57.543 | 1.00 | 56.61 |
| ATOM 2878 | SD | MET402 | 22.543 | 34.551 | 55.906 | 1.00 | 52.49 |
| ATOM 2879 | CE | MET402 | 23.857 | 33.399 | 56.323 | 1.00 | 49.76 |
| ATOM 2880 | C | MET402 | 21.532 | 35.450 | 60.381 | 1.00 | 65.00 |
| ATOM 2881 | O | MET402 | 22.222 | 34.513 | 60.781 | 1.00 | 65.18 |
| ATOM 2882 | N | ARG403 | 20.241 | 35.575 | 60.657 | 1.00 | 63.62 |
| ATOM 2883 | CA | ARG403 | 19.535 | 34.593 | 61.462 | 1.00 | 61.57 |
| ATOM 2884 | CB | ARG403 | 18.418 | 35.275 | 62.262 | 1.00 | 64.83 |
| ATOM 2885 | CG | ARG403 | 18.856 | 36.547 | 62.987 | 1.00 | 70.01 |
| ATOM 2886 | CD | ARG403 | 17.691 | 37.205 | 63.724 | 1.00 | 75.36 |
| ATOM 2887 | NE | ARG403 | 17.412 | 36.582 | 65.018 | 1.00 | 80.50 |
| ATOM 2888 | CZ | ARG403 | 16.305 | 36.788 | 65.731 | 1.00 | 83.41 |
| ATOM 2889 | NH1 | ARG403 | 15.358 | 37.603 | 65.277 | 1.00 | 84.55 |
| ATOM 2890 | NH2 | ARG403 | 16.147 | 36.187 | 66.907 | 1.00 | 83.64 |

[Table 2] (continued)

| | | | | | | | |
|---|---|---|---|---|---|---|---|
| ATOM 2891 | C | ARG403 | 18.946 | 33.560 | 60.504 | 1.00 | 57.99 |
| ATOM 2892 | O | ARG403 | 17.775 | 33.639 | 60.135 | 1.00 | 58.57 |
| ATOM 2893 | N | ILE404 | 19.762 | 32.597 | 60.091 | 1.00 | 52.97 |
| ATOM 2894 | CA | ILE404 | 19.301 | 31.570 | 59.170 | 1.00 | 49.14 |
| ATOM 2895 | CB | ILE404 | 20.293 | 31.412 | 57.999 | 1.00 | 47.44 |
| ATOM 2896 | CG2 | ILE404 | 21.538 | 30.680 | 58.458 | 1.00 | 43.94 |
| ATOM 2897 | CG1 | ILE404 | 19.629 | 30.649 | 56.854 | 1.00 | 48.45 |
| ATOM 2898 | CD1 | ILE404 | 20.477 | 30.559 | 55.598 | 1.00 | 48.95 |
| ATOM 2899 | C | ILE404 | 19.126 | 30.222 | 59.879 | 1.00 | 48.34 |
| ATOM 2900 | O | ILE404 | 19.771 | 29.967 | 60.897 | 1.00 | 48.83 |
| ATOM 2901 | N | THR405 | 18.236 | 29.380 | 59.346 | 1.00 | 46.42 |
| ATOM 2902 | CA | THR405 | 17.956 | 28.043 | 59.892 | 1.00 | 42.37 |
| ATOM 2903 | CB | THR405 | 16.451 | 27.838 | 60.222 | 1.00 | 41.93 |
| ATOM 2904 | OG1 | THR405 | 16.010 | 28.839 | 61.145 | 1.00 | 43.89 |
| ATOM 2905 | CG2 | THR405 | 16.227 | 26.475 | 60.849 | 1.00 | 38.85 |
| ATOM 2906 | C | THR405 | 18.332 | 26.990 | 58.857 | 1.00 | 39.95 |
| ATOM 2907 | O | THR405 | 18.178 | 27.204 | 57.653 | 1.00 | 38.97 |
| ATOM 2908 | N | VAL406 | 18.809 | 25.844 | 59.324 | 1.00 | 38.10 |
| ATOM 2909 | CA | VAL406 | 19.195 | 24.776 | 58.414 | 1.00 | 36-64 |
| ATOM 2910 | CB | VAL406 | 20.686 | 24.442 | 58.563 | 1.00 | 35.12 |
| ATOM 2911 | CG1 | VAL406 | 21.069 | 23.342 | 57.600 | 1.00 | 35.29 |
| ATOM 2912 | CG2 | VAL406 | 21.515 | 25.672 | 58.303 | 1.00 | 35.40 |
| ATOM 2913 | C | VAL406 | 18.390 | 23.499 | 58.635 | 1.00 | 35.83 |
| ATOM 2914 | O | VAL406 | 18.214 | 23.058 | 59.765 | 1.00 | 37.06 |
| ATOM 2915 | N | GLY407 | 17.895 | 22.915 | 57.549 | 1.00 | 34.50 |
| ATOM 2916 | CA | GLY407 | 17.143 | 21.680 | 57.653 | 1.00 | 32.79 |
| ATOM 2917 | C | GLY407 | 18.074 | 20.522 | 57.353 | 1.00 | 32.90 |
| ATOM 2918 | O | GLY407 | 18.704 | 20.467 | 56.294 | 1.00 | 33.85 |
| ATOM 2919 | N | VAL408 | 18.177 | 19.585 | 58.279 | 1.00 | 31.27 |
| ATOM 2920 | CA | VAL408 | 19.064 | 18.466 | 58.054 | 1.00 | 29.57 |
| ATOM 2921 | CB | VAL408 | 20.199 | 18.491 | 59.042 | 1.00 | 29.66 |
| ATOM 2922 | CG1 | VAL408 | 21.390 | 17.767 | 58.468 | 1.00 | 31.77 |
| ATOM 2923 | CG2 | VAL408 | 20.515 | 19.916 | 59.412 | 1.00 | 29.91 |
| ATOM 2924 | C | VAL408 | 18.366 | 17.135 | 58.206 | 1.00 | 29.58 |
| ATOM 2925 | O | VAL408 | 17.392 | 17.015 | 58.942 | 1.00 | 28.54 |
| ATOM 2926 | N | ASP409 | 18.878 | 16.131 | 57.509 | 1.00 | 30.15 |
| ATOM 2927 | CA | ASP409 | 18.324 | 14.789 | 57.598 | 1.00 | 31.95 |
| ATOM 2928 | CB | ASP409 | 17.109 | 14.635 | 56.674 | 1.00 | 35.66 |
| ATOM 2929 | CG | ASP409 | 16.455 | 13.252 | 56.775 | 1.00 | 40.01 |
| ATOM 2930 | OD1 | ASP409 | 15.613 | 12.928 | 55.898 | 1.00 | 40.26 |
| ATOM 2931 | OD2 | ASP409 | 16.773 | 12.499 | 57.728 | 1.00 | 39.33 |
| ATOM 2932 | C | ASP409 | 19.415 | 13.824 | 57.180 | 1.00 | 31.91 |
| ATOM 2933 | O | ASP409 | 20.352 | 14.208 | 56.484 | 1.00 | 32.39 |
| ATOM 2934 | N | GLY410 | 19.300 | 12.574 | 57.607 | 1.00 | 31.09 |
| ATOM 2935 | CA | GLY410 | 20.299 | 11.593 | 57.233 | 1.00 | 29.56 |
| ATOM 2936 | C | GLY410 | 20.703 | 10.704 | 58.385 | 1.00 | 29.32 |
| ATOM 2937 | O | GLY410 | 20.510 | 11.041 | 59.558 | 1.00 | 28.27 |
| ATOM 2938 | N | SER411 | 21.282 | 9.559 | 58.053 | 1.00 | 28.24 |
| ATOM 2939 | CA | SER411 | 21.699 | 8.631 | 59.086 | 1.00 | 27.52 |
| ATOM 2940 | CB | SER411 | 22.018 | 7.253 | 58.481 | 1.00 | 29.46 |
| ATOM 2941 | OG | SER411 | 23.016 | 7.316 | 57.471 | 1.00 | 31.64 |

[Table 2] (continued)

| | | | | | | | |
|---|---|---|---|---|---|---|---|
| ATOM 2942 | C | SER411 | 22.895 | 9.160 | 59.863 | 1.00 | 25.78 |
| ATOM 2943 | O | SER411 | 22.909 | 9.113 | 61.090 | 1.00 | 25.89 |
| ATOM 2944 | N | VAL412 | 23.890 | 9.687 | 59.161 | 1.00 | 23.18 |
| ATOM 2945 | CA | VAL412 | 25.076 | 10.185 | 59.839 | 1.00 | 21.25 |
| ATOM 2946 | CB | VAL412 | 26.099 | 10.669 | 58.841 | 1.00 | 20.56 |
| ATOM 2947 | CG1 | VAL412 | 27.372 | 11.084 | 59.564 | 1.00 | 17.73 |
| ATOM 2948 | CG2 | VAL412 | 26.378 | 9.552 | 57.857 | 1.00 | 20.59 |
| ATOM 2949 | C | VAL412 | 24.769 | 11.300 | 60.818 | 1.00 | 20.44 |
| ATOM 2950 | O | VAL412 | 25.182 | 11.262 | 61.983 | 1.00 | 21.51 |
| ATOM 2951 | N | TYR413 | 24.033 | 12.288 | 60.340 | 1.00 | 16.42 |
| ATOM 2952 | CA | TYR413 | 23.659 | 13.409 | 61.171 | 1.00 | 16.41 |
| ATOM 2953 | CB | TYR413 | 23.095 | 14.526 | 60.288 | 1.00 | 16.40 |
| ATOM 2954 | CG | TYR413 | 22.700 | 15.762 | 61.051 | 1.00 | 14.37 |
| ATOM 2955 | CD1 | TYR413 | 23.645 | 16.707 | 61.434 | 1.00 | 13.43 |
| ATOM 2956 | CE1 | TYR413 | 23.296 | 17.789 | 62.226 | 1.00 | 13.93 |
| ATOM 2957 | CD2 | TYR413 | 21.401 | 15.939 | 61.470 | 1.00 | 13.10 |
| ATOM 2958 | CE2 | TYR413 | 21.049 | 17.007 | 62.256 | 1.00 | 15.82 |
| ATOM 2959 | CZ | TYR413 | 21.994 | 17.927 | 62.638 | 1.00 | 14.65 |
| ATOM 2960 | OH | TYR413 | 21.620 | 18.948 | 63.475 | 1.00 | 16.02 |
| ATOM 2961 | C | TYR413 | 22.626 | 13.007 | 62.233 | 1.00 | 17.41 |
| ATOM 2962 | O | TYR413 | 22.364 | 13.758 | 63.172 | 1.00 | 18.36 |
| ATOM 2963 | N | LYS414 | 22.035 | 11.826 | 62.103 | 1.00 | 18.12 |
| ATOM 2964 | CA | LYS414 | 21.033 | 11.426 | 63.083 | 1.00 | 19.00 |
| ATOM 2965 | CB | LYS414 | 19.706 | 11.130 | 62.384 | 1.00 | 19.22 |
| ATOM 2966 | CG | LYS414 | 18.962 | 12.358 | 61.894 | 1.00 | 18.92 |
| ATOM 2967 | CD | LYS414 | 17.615 | 11.965 | 61.314 | 1.00 | 21.36 |
| ATOM 2968 | CE | LYS414 | 16.829 | 13.181 | 60.855 | 1.00 | 25.08 |
| ATOM 2969 | NZ | LYS414 | 15.567 | 12.829 | 60.132 | 1.00 | 28.46 |
| ATOM 2970 | C | LYS414 | 21.400 | 10.249 | 63.975 | 1.00 | 20.50 |
| ATOM 2971 | O | LYS414 | 20.637 | 9.883 | 64.871 | 1.00 | 21.27 |
| ATOM 2972 | N | LEU415 | 22.565 | 9.655 | 63.753 | 1.00 | 22.22 |
| ATOM 2973 | CA | LEU415 | 22.958 | 8.511 | 64.565 | 1.00 | 23.27 |
| ATOM 2974 | CB | LEU415 | 22.679 | 7.218 | 63.784 | 1.00 | 21.47 |
| ATOM 2975 | CG | LEU415 | 21.234 | 6.978 | 63.313 | 1.00 | 17.45 |
| ATOM 2976 | CD1 | LEU415 | 21.158 | 5.672 | 62.545 | 1.00 | 16.66 |
| ATOM 2977 | CD2 | LEU415 | 20.293 | 6.954 | 64.498 | 1.00 | 14.44 |
| ATOM 2978 | C | LEU415 | 24.418 | 8.566 | 65.033 | 1.00 | 25.05 |
| ATOM 2979 | O | LEU415 | 24.921 | 7.625 | 65.657 | 1.00 | 26.05 |
| ATOM 2980 | N | HIS416 | 25.095 | 9.673 | 64.736 | 1.00 | 24.04 |
| ATOM 2981 | CA | HIS416 | 26.481 | 9.852 | 65.147 | 1.00 | 22.40 |
| ATOM 2982 | CB | HIS416 | 27.365 | 9.997 | 63.922 | 1.00 | 23.29 |
| ATOM 2983 | CG | HIS416 | 27.383 | 8.774 | 63.069 | 1.00 | 25.75 |
| ATOM 2984 | CD2 | HIS416 | 28.392 | 7.937 | 62.729 | 1.00 | 27.82 |
| ATOM 2985 | ND1 | HIS416 | 26.241 | 8.248 | 62.506 | 1.00 | 26.69 |
| ATOM 2986 | CE1 | HIS416 | 26.545 | 7.138 | 61.857 | 1.00 | 28.69 |
| ATOM 2987 | NE2 | HIS416 | 27.844 | 6.926 | 61.977 | 1.00 | 28.20 |
| ATOM 2988 | C | HIS416 | 26.577 | 11.080 | 66.027 | 1.00 | 21.63 |
| ATOM 2989 | O | HIS416 | 26.808 | 12.184 | 65.558 | 1.00 | 22.44 |
| ATOM 2990 | N | PRO417 | 26.386 | 10.898 | 67.331 | 1.00 | 21.25 |
| ATOM 2991 | CD | PRO417 | 26.126 | 9.627 | 68.015 | 1.00 | 22.18 |
| ATOM 2992 | CA | PRO417 | 26.440 | 11.991 | 68.297 | 1.00 | 22.07 |

[Table 2] (continued)

| | | | | | | | |
|---|---|---|---|---|---|---|---|
| ATOM 2993 | CB | PRO417 | 26.447 | 11.258 | 69.627 | 1.00 | 21.52 |
| ATOM 2994 | CG | PRO417 | 25.565 | 10.108 | 69.340 | 1.00 | 23.41 |
| ATOM 2995 | C | PRO417 | 27.655 | 12.874 | 68.113 | 1.00 | 22.46 |
| ATOM 2996 | O | PRO417 | 27.519 | 14.076 | 67.878 | 1.00 | 22.18 |
| ATOM 2997 | N | SER418 | 28.835 | 12.262 | 68.221 | 1.00 | 20.96 |
| ATOM 2998 | CA | SER418 | 30.105 | 12.959 | 68.064 | 1.00 | 18.32 |
| ATOM 2999 | CB | SER418 | 31.264 | 11.962 | 68.076 | 1.00 | 20.88 |
| ATOM 3000 | OG | SER418 | 32.419 | 12.512 | 67.460 | 1.00 | 24.12 |
| ATOM 3001 | C | SER418 | 30.099 | 13.720 | 66.757 | 1.00 | 15.71 |
| ATOM 3002 | O | SER418 | 30.269 | 14.935 | 66.742 | 1.00 | 16.10 |
| ATOM 3003 | N | PHE419 | 29.905 | 13.010 | 65.656 | 1.00 | 11.39 |
| ATOM 3004 | CA | PHE419 | 29.864 | 13.683 | 64.379 | 1.00 | 10.22 |
| ATOM 3005 | CB | PHE419 | 29.243 | 12.789 | 63.335 | 1.00 | 5.53 |
| ATOM 3006 | CG | PHE419 | 29.035 | 13.468 | 62.034 | 1.00 | 1.42 |
| ATOM 3007 | CD1 | PHE419 | 29.814 | 13.137 | 60.942 | 1.00 | 3.13 |
| ATOM 3008 | CD2 | PHE419 | 28.080 | 14.449 | 61.893 | 1.00 | 1.00 |
| ATOM 3009 | CE1 | PHE419 | 29.648 | 13.773 | 59.712 | 1.00 | 1.47 |
| ATOM 3010 | CE2 | PHE419 | 27.909 | 15.088 | 60.670 | 1.00 | 2.68 |
| ATOM 3011 | CZ | PHE419 | 28.699 | 14.746 | 59.575 | 1.00 | 1.00 |
| ATOM 3012 | C | PHE419 | 29.037 | 14.965 | 64.472 | 1.00 | 12.48 |
| ATOM 3013 | O | PHE419 | 29.520 | 16.048 | 64.156 | 1.00 | 12.11 |
| ATOM 3014 | N | LYS420 | 27.785 | 14.838 | 64.900 | 1.00 | 15.88 |
| ATOM 3015 | CA | LYS420 | 26.917 | 16.000 | 64.994 | 1.00 | 20.63 |
| ATOM 3016 | CB | LYS420 | 25.525 | 15.610 | 65.522 | 1.00 | 21.26 |
| ATOM 3017 | CG | LYS420 | 24.470 | 16.730 | 65.361 | 1.00 | 22.35 |
| ATOM 3018 | CD | LYS420 | 23.045 | 16.288 | 65.686 | 1.00 | 22.81 |
| ATOM 3019 | CE | LYS420 | 22.942 | 15.740 | 67.102 | 1.00 | 25.24 |
| ATOM 3020 | NZ | LYS420 | 21.616 | 15.092 | 67.350 | 1.00 | 27.51 |
| ATOM 3021 | C | LYS420 | 27.505 | 17.099 | 65.866 | 1.00 | 24.04 |
| ATOM 3022 | O | LYS420 | 27.533 | 18.260 | 65.465 | 1.00 | 23.74 |
| ATOM 3023 | N | GLU421 | 27.978 | 16.733 | 67.053 | 1.00 | 29.67 |
| ATOM 3024 | CA | GLU421 | 28.550 | 17.701 | 67.999 | 1.00 | 34.96 |
| ATOM 3025 | CB | GLU421 | 29.075 | 16.972 | 69.244 | 1.00 | 36.76 |
| ATOM 3026 | CG | GLU421 | 29.292 | 17.843 | 70.480 | 1.00 | 40.52 |
| ATOM 3027 | CD | GLU421 | 29.895 | 17.047 | 71.638 | 1.00 | 43.55 |
| ATOM 3028 | OE1 | GLU421 | 30.981 | 16.467 | 71.445 | 1.00 | 47.03 |
| ATOM 3029 | OE2 | GLU421 | 29.294 | 16.990 | 72.734 | 1.00 | 43.28 |
| ATOM 3030 | C | GLU421 | 29.680 | 18.512 | 67.369 | 1.00 | 36.40 |
| ATOM 3031 | O | GLU421 | 29.689 | 19.745 | 67.442 | 1.00 | 38.37 |
| ATOM 3032 | N | ARG422 | 30.629 | 17.816 | 66.751 | 1.00 | 35.66 |
| ATOM 3033 | CA | ARG422 | 31.755 | 18.477 | 66.124 | 1.00 | 35.13 |
| ATOM 3034 | CB | ARG422 | 32.801 | 17.449 | 65.684 | 1.00 | 38.76 |
| ATOM 3035 | CG | ARG422 | 33.277 | 16.525 | 66.811 | 1.00 | 46.51 |
| ATOM 3036 | CD | ARG422 | 33.915 | 17.286 | 67.980 | 1.00 | 51.67 |
| ATOM 3037 | NE | ARG422 | 35.322 | 17.578 | 67.732 | 1.00 | 57.41 |
| ATOM 3038 | CZ | ARG422 | 36.269 | 16.649 | 67.625 | 1.00 | 60.70 |
| ATOM 3039 | NH1 | ARG422 | 35.956 | 15.364 | 67.749 | 1.00 | 60.82 |
| ATOM 3040 | NH2 | ARG422 | 37.529 | 17.002 | 67.380 | 1.00 | 61.68 |
| ATOM 3041 | C | ARG422 | 31.256 | 19.278 | 64.942 | 1.00 | 33.47 |
| ATOM 3042 | O | ARG422 | 31.585 | 20.450 | 64.803 | 1.00 | 35.28 |
| ATOM 3043 | N | PHE423 | 30.446 | 18.654 | 64.096 | 1.00 | 32.46 |

[Table 2] (continued)

| | | | | | | | |
|---|---|---|---|---|---|---|---|
| ATOM 3044 | CA | PHE423 | 29.901 | 19.348 | 62.930 | 1.00 | 30.30 |
| ATOM 3045 | CB | PHE423 | 28.949 | 18.423 | 62.165 | 1.00 | 27.32 |
| ATOM 3046 | CG | PHE423 | 28.188 | 19.106 | 61.063 | 1.00 | 23.75 |
| ATOM 3047 | CD1 | PHE423 | 26.891 | 19.552 | 61.270 | 1.00 | 22.33 |
| ATOM 3048 | CD2 | PHE423 | 28.765 | 19.293 | 59.814 | 1.00 | 23.98 |
| ATOM 3049 | CE1 | PHE423 | 26.178 | 20.169 | 60.245 | 1.00 | 22.83 |
| ATOM 3050 | CE2 | PHE423 | 28.061 | 19.909 | 58.784 | 1.00 | 22.46 |
| ATOM 3051 | CZ | PHE423 | 26.769 | 20.347 | 59.001 | 1.00 | 22.73 |
| ATOM 3052 | C | PHE423 | 29.185 | 20.663 | 63.280 | 1.00 | 29.75 |
| ATOM 3053 | O | PHE423 | 29.328 | 21.652 | 62.568 | 1.00 | 27.58 |
| ATOM 3054 | N | HIS424 | 28.415 | 20.694 | 64.363 | 1.00 | 30.19 |
| ATOM 3055 | CA | HIS424 | 27.743 | 21.936 | 64.692 | 1.00 | 32.48 |
| ATOM 3056 | CB | HIS424 | 26.754 | 21.760 | 65.835 | 1.00 | 32.75 |
| ATOM 3057 | CG | HIS424 | 25.412 | 21.279 | 65.387 | 1.00 | 31.94 |
| ATOM 3058 | CD2 | HIS424 | 24.980 | 20.860 | 64.176 | 1.00 | 29.85 |
| ATOM 3059 | ND1 | HIS424 | 24.341 | 21.147 | 66.243 | 1.00 | 32.28 |
| ATOM 3060 | CE1 | HIS424 | 23.308 | 20.661 | 65.580 | 1.00 | 30.67 |
| ATOM 3061 | NE2 | HIS424 | 23.670 | 20.477 | 64.323 | 1.00 | 30.19 |
| ATOM 3062 | C | HIS424 | 28.737 | 23.011 | 65.048 | 1.00 | 35.15 |
| ATOM 3063 | O | HIS424 | 28.689 | 24.102 | 64.487 | 1.00 | 36.91 |
| ATOM 3064 | N | ALA425 | 29.636 | 22.711 | 65.979 | 1.00 | 36.32 |
| ATOM 3065 | CA | ALA425 | 30.652 | 23.675 | 66.395 | 1.00 | 36.74 |
| ATOM 3066 | CB | ALA425 | 31.542 | 23.058 | 67.444 | 1.00 | 35.43 |
| ATOM 3067 | C | ALA425 | 31.492 | 24.149 | 65.201 | 1.00 | 37.82 |
| ATOM 3068 | O | ALA425 | 31.420 | 25.316 | 64.809 | 1.00 | 38.66 |
| ATOM 3069 | N | SER426 | 32.274 | 23.243 | 64.617 | 1.00 | 37.75 |
| ATOM 3070 | CA | SER426 | 33.113 | 23.576 | 63.466 | 1.00 | 37.83 |
| ATOM 3071 | CB | SER426 | 33.602 | 22.289 | 62.782 | 1.00 | 38.67 |
| ATOM 3072 | OG | SER426 | 34.440 | 22.560 | 61.667 | 1.00 | 37.85 |
| ATOM 3073 | C | SER426 | 32.390 | 24.461 | 62.445 | 1.00 | 37.21 |
| ATOM 3074 | O | SER426 | 33.025 | 25.151 | 61.657 | 1.00 | 37.08 |
| ATOM 3075 | N | VAL427 | 31.064 | 24.443 | 62.450 | 1.00 | 37.84 |
| ATOM 3076 | CA | VAL427 | 30.321 | 25.269 | 61.510 | 1.00 | 38.87 |
| ATOM 3077 | CB | VAL427 | 28.935 | 24.667 | 61.194 | 1.00 | 39.38 |
| ATOM 3078 | CG1 | VAL427 | 28.000 | 25.744 | 60.633 | 1.00 | 37.60 |
| ATOM 3079 | CG2 | VAL427 | 29.092 | 23.534 | 60.188 | 1.00 | 36.83 |
| ATOM 3080 | C | VAL427 | 30.138 | 26.655 | 62.090 | 1.00 | 39.54 |
| ATOM 3081 | O | VAL427 | 30.578 | 27.639 | 61.512 | 1.00 | 40.58 |
| ATOM 3082 | N | ARG428 | 29.483 | 26.724 | 63.238 | 1.00 | 40.14 |
| ATOM 3083 | CA | ARG428 | 29.247 | 27.993 | 63.897 | 1.00 | 42.86 |
| ATOM 3084 | CB | ARG428 | 28.603 | 27.739 | 65.258 | 1.00 | 42.72 |
| ATOM 3085 | CG | ARG428 | 27.288 | 26.982 | 65.186 | 1.00 | 43.31 |
| ATOM 3086 | CD | ARG428 | 27.139 | 26.044 | 66.378 | 1.00 | 46.03 |
| ATOM 3087 | NE | ARG428 | 25.802 | 25.461 | 66.485 | 1.00 | 47.83 |
| ATOM 3088 | CZ | ARG428 | 24.690 | 26.173 | 66.648 | 1.00 | 48.39 |
| ATOM 3089 | NH1 | ARG428 | 24.757 | 27.499 | 66.716 | 1.00 | 47.35 |
| ATOM 3090 | NH2 | ARG428 | 23.516 | 25.559 | 66.756 | 1.00 | 47.34 |
| ATOM 3091 | C | ARG428 | 30.561 | 28.768 | 64.064 | 1.00 | 44.67 |
| ATOM 3092 | O | ARG428 | 30.577 | 30.001 | 64.060 | 1.00 | 45.05 |
| ATOM 3093 | N | ARG429 | 31.663 | 28.037 | 64.195 | 1.00 | 45.77 |
| ATOM 3094 | CA | ARG429 | 32.972 | 28.652 | 64.378 | 1.00 | 46.48 |

[Table 2] (continued)

| | | | | | | | |
|---|---|---|---|---|---|---|---|
| ATOM 3095 | CB | ARG429 | 33.849 | 27.738 | 65.244 | 1.00 | 52.63 |
| ATOM 3096 | CG | ARG429 | 33.260 | 27.471 | 66.648 | 1.00 | 59.36 |
| ATOM 3097 | CD | ARG429 | 33.828 | 26.199 | 67.328 | 1.00 | 64.53 |
| ATOM 3098 | NE | ARG429 | 35.247 | 26.286 | 67.677 | 1.00 | 66.23 |
| ATOM 3099 | CZ | ARG429 | 35.963 | 25.274 | 68.159 | 1.00 | 66.80 |
| ATOM 3100 | NH1 | ARG429 | 35.398 | 24.083 | 68.357 | 1.00 | 66.02 |
| ATOM 3101 | NH2 | ARG429 | 37.249 | 25.455 | 68.435 | 1.00 | 68.38 |
| ATOM 3102 | C | ARG429 | 33.657 | 28.954 | 63.049 | 1.00 | 44.06 |
| ATOM 3103 | O | ARG429 | 34.885 | 28.943 | 62.954 | 1.00 | 43.92 |
| ATOM 3104 | N | LEU430 | 32.847 | 29.221 | 62.029 | 1.00 | 41.46 |
| ATOM 3105 | CA | LEU430 | 33.333 | 29.551 | 60.692 | 1.00 | 40.12 |
| ATOM 3106 | CB | LEU430 | 33.495 | 28.300 | 59.830 | 1.00 | 35.57 |
| ATOM 3107 | CG | LEU430 | 34.755 | 27.468 | 60.042 | 1.00 | 34.48 |
| ATOM 3108 | CD1 | LEU430 | 34.764 | 26.279 | 59.101 | 1.00 | 32.77 |
| ATOM 3109 | CD2 | LEU430 | 35.965 | 28.332 | 59.806 | 1.00 | 33.57 |
| ATOM 3110 | C | LEU430 | 32.332 | 30.468 | 60.029 | 1.00 | 42.10 |
| ATOM 3111 | O | LEU430 | 32.503 | 30.868 | 58.880 | 1.00 | 42.67 |
| ATOM 3112 | N | THR431 | 31.280 | 30.797 | 60.763 | 1.00 | 44.70 |
| ATOM 3113 | CA | THR431 | 30.238 | 31.658 | 60.239 | 1.00 | 48.98 |
| ATOM 3114 | CB | THR431 | 28.923 | 30.928 | 60.113 | 1.00 | 49.80 |
| ATOM 3115 | OG1 | THR431 | 28.533 | 30.463 | 61.410 | 1.00 | 50.69 |
| ATOM 3116 | CG2 | THR431 | 29.048 | 29.758 | 59.159 | 1.00 | 51.11 |
| ATOM 3117 | C | THR431 | 29.999 | 32.820 | 61.174 | 1.00 | 51.66 |
| ATOM 3118 | O | THR431 | 28.986 | 32.868 | 61.881 | 1.00 | 52.07 |
| ATOM 3119 | N | PRO432 | 30.935 | 33.774 | 61.190 | 1.00 | 52.95 |
| ATOM 3120 | CD | PRO432 | 32.179 | 33.719 | 60.403 | 1.00 | 51.90 |
| ATOM 3121 | CA | PRO432 | 30.886 | 34.980 | 62.020 | 1.00 | 52.47 |
| ATOM 3122 | CB | PRO432 | 32.135 | 35.733 | 61.587 | 1.00 | 54.48 |
| ATOM 3123 | CG | PRO432 | 33.073 | 34.623 | 61.176 | 1.00 | 54.21 |
| ATOM 3124 | C | PRO432 | 29.620 | 35.783 | 61.739 | 1.00 | 52.15 |
| ATOM 3125 | O | PR0432 | 29.257 | 35.981 | 60.582 | 1.00 | 49.70 |
| ATOM 3126 | N | SER433 | 28.955 | 36.243 | 62.793 | 1.00 | 53.82 |
| ATOM 3127 | CA | SER433 | 27.734 | 37.042 | 62.652 | 1.00 | 57.56 |
| ATOM 3128 | CB | SER433 | 28.055 | 38.372 | 61.952 | 1.00 | 59.89 |
| ATOM 3129 | OG | SER433 | 28.537 | 38.176 | 60.633 | 1.00 | 62.34 |
| ATOM 3130 | C | SER433 | 26.570 | 36.340 | 61.926 | 1.00 | 57.57 |
| ATOM 3131 | O | SER433 | 25.907 | 36.923 | 61.056 | 1.00 | 57.63 |
| ATOM 3132 | N | CYS434 | 26.327 | 35.088 | 62.306 | 1.00 | 56.67 |
| ATOM 3133 | CA | CYS434 | 25.256 | 34.275 | 61.738 | 1.00 | 54.67 |
| ATOM 3134 | CB | CYS434 | 25.805 | 33.375 | 60.634 | 1.00 | 54.21 |
| ATOM 3135 | SG | CYS434 | 26.729 | 34.213 | 59.354 | 1.00 | 55.95 |
| ATOM 3136 | C | CYS434 | 24.657 | 33.390 | 62.832 | 1.00 | 54.71 |
| ATOM 3137 | O | CYS434 | 25.381 | 32.663 | 63.513 | 1.00 | 54.74 |
| ATOM 3138 | N | GLU435 | 23.344 | 33.454 | 63.011 | 1.00 | 54.63 |
| ATOM 3139 | CA | GLU435 | 22.681 | 32.621 | 64.009 | 1.00 | 54.57 |
| ATOM 3140 | CB | GLU435 | 21.529 | 33.383 | 64.681 | 1.00 | 59.73 |
| ATOM 3141 | CG | GLU435 | 21.927 | 34.615 | 65.511 | 1.00 | 64.17 |
| ATOM 3142 | CD | GLU435 | 20.717 | 35.479 | 65.902 | 1.00 | 67.96 |
| ATOM 3143 | OE1 | GLU435 | 20.905 | 36.554 | 66.521 | 1.00 | 68.24 |
| ATOM 3144 | OE2 | GLU435 | 19.574 | 35.079 | 65.584 | 1.00 | 69.98 |
| ATOM 3145 | C | GLU435 | 22.134 | 31.378 | 63.289 | 1.00 | 51.70 |

[Table 2] (continued)

| | | | | | | | |
|---|---|---|---|---|---|---|---|
| ATOM 3146 | O | GLU435 | 21.058 | 31.412 | 62.685 | 1.00 | 51.14 |
| ATOM 3147 | N | ILE436 | 22.889 | 30.288 | 63.350 | 1.00 | 47.61 |
| ATOM 3148 | CA | ILE436 | 22.497 | 29.046 | 62.702 | 1.00 | 43.09 |
| ATOM 3149 | CB | ILE436 | 23.719 | 28.331 | 62.118 | 1.00 | 38.65 |
| ATOM 3150 | CG2 | ILE436 | 23.278 | 27.138 | 61.300 | 1.00 | 38.13 |
| ATOM 3151 | CG1 | ILE436 | 24.502 | 29.286 | 61.234 | 1.00 | 34.79 |
| ATOM 3152 | CD1 | ILE436 | 25.768 | 28.686 | 60.710 | 1.00 | 34.08 |
| ATOM 3153 | C | ILE436 | 21.798 | 28.088 | 63.664 | 1.00 | 42.96 |
| ATOM 3154 | O | ILE436 | 22.403 | 27.608 | 64.621 | 1.00 | 43.46 |
| ATOM 3155 | N | THR437 | 20.521 | 27.821 | 63.402 | 1.00 | 41.73 |
| ATOM 3156 | CA | THR437 | 19.724 | 26.910 | 64.218 | 1.00 | 39.10 |
| ATOM 3157 | CB | THR437 | 18.384 | 27.553 | 64.638 | 1.00 | 37.86 |
| ATOM 3158 | OG1 | THR437 | 18.182 | 28.763 | 63.899 | 1.00 | 37.22 |
| ATOM 3159 | CG2 | THR437 | 18.370 | 27.856 | 66.130 | 1.00 | 37.07 |
| ATOM 3160 | C | THR437 | 19.430 | 25.672 | 63.380 | 1.00 | 38.74 |
| ATOM 3161 | O | THR437 | 18.979 | 25.784 | 62.238 | 1.00 | 39.10 |
| ATOM 3162 | N | PHE438 | 19.696 | 24.494 | 63.936 | 1.00 | 36.24 |
| ATOM 3163 | CA | PHE438 | 19.449 | 23.257 | 63.210 | 1.00 | 33.18 |
| ATOM 3164 | CB | PHE438 | 20.556 | 22.256 | 63.491 | 1.00 | 30.88 |
| ATOM 3165 | CG | PHE438 | 21.905 | 22.742 | 63.093 | 1.00 | 32.48 |
| ATOM 3166 | CD1 | PHE438 | 22.597 | 23.652 | 63.887 | 1.00 | 31.95 |
| ATOM 3167 | CD2 | PHE438 | 22.489 | 22.301 | 61.913 | 1.00 | 32.85 |
| ATOM 3168 | CE1 | PHE438 | 23.857 | 24.118 | 63.507 | 1.00 | 31.30 |
| ATOM 3169 | CE2 | PHE438 | 23.745 | 22.758 | 61.522 | 1.00 | 32.28 |
| ATOM 3170 | CZ | PHE438 | 24.432 | 23.668 | 62.320 | 1.00 | 31.80 |
| ATOM 3171 | C | PHE438 | 18.102 | 22.648 | 63.563 | 1.00 | 33.15 |
| ATOM 3172 | O | PHE438 | 17.662 | 22.729 | 64.705 | 1.00 | 34.90 |
| ATOM 3173 | N | ILE439 | 17.450 | 22.049 | 62.570 | 1.00 | 31.06 |
| ATOM 3174 | CA | ILE439 | 16.150 | 21.412 | 62.738 | 1.00 | 28.59 |
| ATOM 3175 | CB | ILE439 | 15.010 | 22.347 | 62.321 | 1.00 | 26.74 |
| ATOM 3176 | CG2 | ILE439 | 15.268 | 22.879 | 60.937 | 1.00 | 27.91 |
| ATOM 3177 | CG1 | ILE439 | 13.683 | 21.591 | 62.312 | 1.00 | 27.91 |
| ATOM 3178 | CD1 | ILE439 | 12.545 | 22.406 | 61.776 | 1.00 | 26.70 |
| ATOM 3179 | C | ILE439 | 16.113 | 20.190 | 61.837 | 1.00 | 29.76 |
| ATOM 3180 | O | ILE439 | 16.208 | 20.310 | 60.618 | 1.00 | 29.55 |
| ATOM 3181 | N | GLU440 | 15.977 | 19.014 | 62.434 | 1.00 | 30.76 |
| ATOM 3182 | CA | GLU440 | 15.934 | 17.781 | 61.666 | 1.00 | 32.34 |
| ATOM 3183 | CB | GLU440 | 16.028 | 16.592 | 62.609 | 1.00 | 34.09 |
| ATOM 3184 | CG | GLU440 | 17.272 | 16.583 | 63.458 | 1.00 | 38.93 |
| ATOM 3185 | CD | GLU440 | 17.339 | 15.367 | 64.353 | 1.00 | 43.10 |
| ATOM 3186 | OE1 | GLU440 | 16.378 | 15.162 | 65.131 | 1.00 | 44.22 |
| ATOM 3187 | OE2 | GLU440 | 18.346 | 14.623 | 64.277 | 1.00 | 44.99 |
| ATOM 3188 | C | GLU440 | 14.648 | 17.687 | 60.854 | 1.00 | 33.11 |
| ATOM 3189 | O | GLU440 | 13.703 | 18.445 | 61.086 | 1.00 | 31.25 |
| ATOM 3190 | N | SER441 | 14.613 | 16.764 | 59.896 | 1.00 | 34.70 |
| ATOM 3191 | CA | SER441 | 13.416 | 16.587 | 59.086 | 1.00 | 37.07 |
| ATOM 3192 | CB | SER441 | 13.738 | 15.904 | 57.761 | 1.00 | 34.79 |
| ATOM 3193 | OG | SER441 | 14.159 | 14.579 | 57.988 | 1.00 | 34.61 |
| ATOM 3194 | C | SER441 | 12.452 | 15.724 | 59.889 | 1.00 | 40.43 |
| ATOM 3195 | O | SER441 | 12.866 | 14.964 | 60.773 | 1.00 | 38.99 |
| ATOM 3196 | N | GLU442 | 11.168 | 15.855 | 59.571 | 1.00 | 43.31 |

[Table 2] (continued)

| | | | | | | | |
|---|---|---|---|---|---|---|---|
| ATOM 3197 | CA | GLU442 | 10.099 | 15.135 | 60.254 | 1.00 | 45.59 |
| ATOM 3198 | CB | GLU442 | 8.764 | 15.638 | 59.724 | 1.00 | 46.37 |
| ATOM 3199 | CG | GLU442 | 7.575 | 15.244 | 60.549 | 1.00 | 49.47 |
| ATOM 3200 | CD | GLU442 | 6.653 | 16.421 | 60.794 | 1.00 | 52.25 |
| ATOM 3201 | OE1 | GLU442 | 5.425 | 16.199 | 60.895 | 1.00 | 52.44 |
| ATOM 3202 | OE2 | GLU442 | 7.160 | 17.568 | 60.894 | 1.00 | 53.16 |
| ATOM 3203 | C | GLU442 | 10.165 | 13.607 | 60.174 | 1.00 | 47.69 |
| ATOM 3204 | O | GLU442 | 10.828 | 13.035 | 59.314 | 1.00 | 47.21 |
| ATOM 3205 | N | GLU443 | 9.435 | 12.964 | 61.076 | 1.00 | 50.15 |
| ATOM 3206 | CA | GLU443 | 9.382 | 11.508 | 61.210 | 1.00 | 52.79 |
| ATOM 3207 | CB | GLU443 | 8.911 | 11.204 | 62.623 | 1.00 | 55.86 |
| ATOM 3208 | CG | GLU443 | 9.468 | 12.183 | 63.635 | 1.00 | 61.71 |
| ATOM 3209 | CD | GLU443 | 10.948 | 11.962 | 63.877 | 1.00 | 66.19 |
| ATOM 3210 | OE1 | GLU443 | 11.689 | 11.746 | 62.886 | 1.00 | 68.22 |
| ATOM 3211 | OE2 | GLU443 | 11.365 | 12.006 | 65.058 | 1.00 | 67.57 |
| ATOM 3212 | C | GLU443 | 8.521 | 10.711 | 60.218 | 1.00 | 53.03 |
| ATOM 3213 | O | GLU443 | 7.344 | 10.999 | 60.025 | 1.00 | 54.82 |
| ATOM 3214 | N | GLY444 | 9.116 | 9.702 | 59.593 | 1.00 | 52.26 |
| ATOM 3215 | CA | GLY444 | 8.373 | 8.861 | 58.667 | 1.00 | 52.32 |
| ATOM 3216 | C | GLY444 | 7.966 | 9.389 | 57.302 | 1.00 | 53.07 |
| ATOM 3217 | O | GLY444 | 6.767 | 9.460 | 57.003 | 1.00 | 52.97 |
| ATOM 3218 | N | SER445 | 8.961 | 9.750 | 56.483 | 1.00 | 52.71 |
| ATOM 3219 | CA | SER445 | 8.760 | 10.239 | 55.104 | 1.00 | 50.05 |
| ATOM 3220 | CB | SER445 | 7.836 | 11.464 | 55.084 | 1.00 | 51.01 |
| ATOM 3221 | OG | SER445 | 6.487 | 11.084 | 55.318 | 1.00 | 46.84 |
| ATOM 3222 | C | SER445 | 10.076 | 10.545 | 54.356 | 1.00 | 46.51 |
| ATOM 3223 | O | SER445 | 11.123 | 10.710 | 54.976 | 1.00 | 45.30 |
| ATOM 3224 | N | GLY446 | 10.013 | 10.603 | 53.026 | 1.00 | 43.17 |
| ATOM 3225 | CA | GLY446 | 11.207 | 10.842 | 52.235 | 1.00 | 40.08 |
| ATOM 3226 | C | GLY446 | 11.199 | 12.057 | 51.330 | 1.00 | 39.05 |
| ATOM 3227 | O | GLY446 | 11.414 | 13.164 | 51.803 | 1.00 | 42.74 |
| ATOM 3228 | N | ARG447 | 10.940 | 11.873 | 50.039 | 1.00 | 37.60 |
| ATOM 3229 | CA | ARG447 | 10.956 | 13.000 | 49.098 | 1.00 | 37.46 |
| ATOM 3230 | CB | ARG447 | 11.549 | 12.546 | 47.747 | 1.00 | 45.51 |
| ATOM 3231 | CG | ARG447 | 10.793 | 11.401 | 47.014 | 1.00 | 53.91 |
| ATOM 3232 | CD | ARG447 | 11.521 | 10.902 | 45.743 | 1.00 | 58.18 |
| ATOM 3233 | NE | ARG447 | 12.939 | 10.598 | 45.975 | 1.00 | 63.62 |
| ATOM 3234 | CZ | ARG447 | 13.399 | 9.623 | 46.769 | 1.00 | 66.02 |
| ATOM 3235 | NH1 | ARG447 | 12.560 | 8.825 | 47.425 | 1.00 | 67.97 |
| ATOM 3236 | NH2 | ARG447 | 14.711 | 9.447 | 46.924 | 1.00 | 69.08 |
| ATOM 3237 | C | ARG447 | 9.642 | 13.737 | 48.848 | 1.00 | 32.93 |
| ATOM 3238 | O | ARG447 | 9.122 | 14.416 | 49.741 | 1.00 | 29.46 |
| ATOM 3239 | N | GLY448 | 9.150 | 13.625 | 47.607 | 1.00 | 30.52 |
| ATOM 3240 | CA | GLY448 | 7.902 | 14.245 | 47.202 | 1.00 | 26.03 |
| ATOM 3241 | C | GLY448 | 6.845 | 13.828 | 48.200 | 1.00 | 25.25 |
| ATOM 3242 | O | GLY448 | 5.752 | 14.378 | 48.244 | 1.00 | 24.59 |
| ATOM 3243 | N | ALA449 | 7.186 | 12.840 | 49.018 | 1.00 | 23.74 |
| ATOM 3244 | CA | ALA449 | 6.282 | 12.362 | 50.035 | 1.00 | 20.54 |
| ATOM 3245 | CB | ALA449 | 6.611 | 10.917 | 50.410 | 1.00 | 18.12 |
| ATOM 3246 | C | ALA449 | 6.340 | 13.251 | 51.282 | 1.00 | 22.37 |
| ATOM 3247 | O | ALA449 | 5.307 | 13.782 | 51.693 | 1.00 | 22.56 |

[Table 2] (continued)

| | | | | | | | |
|---|---|---|---|---|---|---|---|
| ATOM 3248 | N | ALA450 | 7.524 | 13.443 | 51.881 | 1.00 | 22.28 |
| ATOM 3249 | CA | ALA450 | 7.605 | 14.261 | 53.088 | 1.00 | 20.98 |
| ATOM 3250 | CB | ALA450 | 9.056 | 14.432 | 53.535 | 1.00 | 9.63 |
| ATOM 3251 | C | ALA450 | 6.937 | 15.594 | 52.872 | 1.00 | 20.66 |
| ATOM 3252 | O | ALA450 | 6.417 | 16.168 | 53.826 | 1.00 | 19.81 |
| ATOM 3253 | N | LEU451 | 6.943 | 16.109 | 51.702 | 1.00 | 19.91 |
| ATOM 3254 | CA | LEU451 | 6.279 | 17.379 | 51.602 | 1.00 | 22.22 |
| ATOM 3255 | CB | LEU451 | 6.586 | 18.056 | 50.279 | 1.00 | 26.74 |
| ATOM 3256 | CG | LEU451 | 6.089 | 19.496 | 50.144 | 1.00 | 34.07 |
| ATOM 3257 | CD1 | LEU451 | 6.894 | 20.425 | 51.040 | 1.00 | 37.55 |
| ATOM 3258 | CD2 | LEU451 | 6.160 | 19.955 | 48.696 | 1.00 | 36.19 |
| ATOM 3259 | C | LEU451 | 4.774 | 17.157 | 51.686 | 1.00 | 23.24 |
| ATOM 3260 | O | LEU451 | 4.136 | 17.474 | 52.699 | 1.00 | 21.18 |
| ATOM 3261 | N | VAL452 | 4.212 | 16.613 | 50.608 | 1.00 | 26.44 |
| ATOM 3262 | CA | VAL452 | 2.798 | 16.405 | 50.557 | 1.00 | 26.90 |
| ATOM 3263 | CB | VAL452 | 2.454 | 15.179 | 49.666 | 1.00 | 28.65 |
| ATOM 3264 | CG1 | VAL452 | 3.082 | 15.353 | 48.300 | 1.00 | 26.01 |
| ATOM 3265 | CG2 | VAL452 | 2.933 | 13.886 | 50.313 | 1.00 | 31.22 |
| ATOM 3266 | C | VAL452 | 2.217 | 16.308 | 51.935 | 1.00 | 26.69 |
| ATOM 3267 | O | VAL452 | 1.181 | 16.878 | 52.234 | 1.00 | 24.86 |
| ATOM 3268 | N | SER453 | 2.880 | 15.579 | 52.769 | 1.00 | 26.07 |
| ATOM 3269 | CA | SER453 | 2.377 | 15.346 | 54.125 | 1.00 | 26.79 |
| ATOM 3270 | CB | SER453 | 3.058 | 14.127 | 54.756 | 1.00 | 28.88 |
| ATOM 3271 | OG | SER453 | 2.553 | 13.881 | 56.057 | 1.00 | 37.16 |
| ATOM 3272 | C | SER453 | 2.571 | 16.558 | 55.028 | 1.00 | 26.82 |
| ATOM 3273 | O | SER453 | 1.658 | 17.301 | 55.377 | 1.00 | 26.28 |
| ATOM 3274 | N | ALA454 | 3.831 | 16.701 | 55.389 | 1.00 | 26.83 |
| ATOM 3275 | CA | ALA454 | 4.250 | 17.807 | 56.177 | 1.00 | 23.37 |
| ATOM 3276 | CB | ALA454 | 5.719 | 18.128 | 55.937 | 1.00 | 17.54 |
| ATOM 3277 | C | ALA454 | 3.381 | 19.002 | 55.866 | 1.00 | 21.80 |
| ATOM 3278 | O | ALA454 | 3.194 | 19.883 | 56.714 | 1.00 | 21.02 |
| ATOM 3279 | N | VAL455 | 2.848 | 19.044 | 54.656 | 1.00 | 21.59 |
| ATOM 3280 | CA | VAL455 | 2.020 | 20.156 | 54.281 | 1.00 | 25.32 |
| ATOM 3281 | CB | VAL455 | 2.313 | 20.557 | 52.841 | 1.00 | 28.03 |
| ATOM 3282 | CG1 | VAL455 | 1.676 | 19.583 | 51.871 | 1.00 | 29.06 |
| ATOM 3283 | CG2 | VAL455 | 1.813 | 21.961 | 52.577 | 1.00 | 29.98 |
| ATOM 3284 | C | VAL455 | 0.528 | 19.890 | 54.469 | 1.00 | 27.69 |
| ATOM 3285 | O | VAL455 | -0.202 | 20.783 | 54.911 | 1.00 | 28.10 |
| ATOM 3286 | N | ALA456 | 0.061 | 18.681 | 54.142 | 1.00 | 30.51 |
| ATOM 3287 | CA | ALA456 | -1.367 | 18.349 | 54.318 | 1.00 | 31.54 |
| ATOM 3288 | CB | ALA456 | -1.666 | 16.937 | 53.836 | 1.00 | 25.20 |
| ATOM 3289 | C | ALA456 | -1.702 | 18.505 | 55.797 | 1.00 | 31.77 |
| ATOM 3290 | O | ALA456 | -2.853 | 18.713 | 56.176 | 1.00 | 33.11 |
| ATOM 3291 | N | CYS457 | -0.673 | 18.384 | 56.625 | 1.00 | 31.33 |
| ATOM 3292 | CA | CYS457 | -0.843 | 18.538 | 58.049 | 1.00 | 33.33 |
| ATOM 3293 | CB | CYS457 | 0.262 | 17.815 | 58.811 | 1.00 | 36.53 |
| ATOM 3294 | SG | CYS457 | 1.040 | 16.448 | 57.890 | 1.00 | 44.65 |
| ATOM 3295 | C | CYS457 | -0.903 | 19.990 | 58.438 | 1.00 | 34.59 |
| ATOM 3296 | O | CYS457 | -1.745 | 20.391 | 59.237 | 1.00 | 34.67 |
| ATOM 3297 | N | LYS458 | 0.005 | 20.779 | 57.881 | 1.00 | 37.14 |
| ATOM 3298 | CA | LYS458 | 0.060 | 22.199 | 58.190 | 1.00 | 38.61 |

[Table 2] (continued)

| | | | | | | | |
|---|---|---|---|---|---|---|---|
| ATOM 3299 | CB | LYS458 | 1.363 | 22.799 | 57.669 | 1.00 | 37.21 |
| ATOM 3300 | CG | LYS458 | 2.573 | 22.474 | 58.538 | 1.00 | 37.81 |
| ATOM 3301 | CD | LYS458 | 2.501 | 23.206 | 59.874 | 1.00 | 38.84 |
| ATOM 3302 | CE | LYS458 | 3.820 | 23.143 | 60.639 | 1.00 | 38.18 |
| ATOM 3303 | NZ | LYS458 | 3.812 | 24.023 | 61.851 | 1.00 | 36.51 |
| ATOM 3304 | C | LYS458 | -1.128 | 22.920 | 57.596 | 1.00 | 40.24 |
| ATOM 3305 | O | LYS458 | -1.377 | 24.079 | 57.898 | 1.00 | 39.64 |
| ATOM 3306 | N | LYS459 | -1.869 | 22.223 | 56.752 | 1.00 | 43.69 |
| ATOM 3307 | CA | LYS459 | -3.036 | 22.820 | 56.147 | 1.00 | 50.66 |
| ATOM 3308 | CB | LYS459 | -3.242 | 22.248 | 54.747 | 1.00 | 55.88 |
| ATOM 3309 | CG | LYS459 | -4.657 | 22.405 | 54.183 | 1.00 | 63.64 |
| ATOM 3310 | CD | LYS459 | -5.037 | 23.850 | 53.856 | 1.00 | 66.97 |
| ATOM 3311 | CE | LYS459 | -6.431 | 23.941 | 53.226 | 1.00 | 68.86 |
| ATOM 3312 | NZ | LYS459 | -7.531 | 23.519 | 54.152 | 1.00 | 71.25 |
| ATOM 3313 | C | LYS459 | -4.262 | 22.562 | 57.018 | 1.00 | 52.41 |
| ATOM 3314 | O | LYS459 | -5.132 | 23.425 | 57.132 | 1.00 | 51.90 |
| ATOM 3315 | N | ALA460 | -4.322 | 21.380 | 57.634 | 1.00 | 54.96 |
| ATOM 3316 | CA | ALA460 | -5.449 | 20.997 | 58.495 | 1.00 | 57.72 |
| ATOM 3317 | CB | ALA460 | -5.201 | 19.620 | 59.111 | 1.00 | 54.90 |
| ATOM 3318 | C | ALA460 | -5.736 | 22.018 | 59.596 | 1.00 | 60.41 |
| ATOM 3319 | O | ALA460 | -6.773 | 21.950 | 60.261 | 1.00 | 60.54 |
| ATOM 3320 | N | CYS461 | -4.815 | 22.965 | 59.776 | 1.00 | 63.50 |
| ATOM 3321 | CA | CYS461 | -4.961 | 24.022 | 60.776 | 1.00 | 66.18 |
| ATOM 3322 | CB | CYS461 | -3.580 | 24.489 | 61.252 | 1.00 | 67.98 |
| ATOM 3323 | SG | CYS461 | -3.604 | 26.041 | 62.185 | 1.00 | 75.61 |
| ATOM 3324 | C | CYS461 | -5.727 | 25.217 | 60.200 | 1.00 | 65.92 |
| ATOM 3325 | O | CYS461 | -6.940 | 25.348 | 60.490 | 1.00 | 65.70 |
| ATOM 3326 | OXT | CYS461 | -5.099 | 26.001 | 59.454 | 1.00 | 65.20 |
| ATOM 3327 | S | SO4 600 | 20.241 | 7.477 | 54.655 | 1.00 | 35.04 |
| ATOM 3328 | O1 | SO4 600 | 19.370 | 7.951 | 53.566 | 1.00 | 33.14 |
| ATOM 3329 | O2 | SO4 600 | 20.343 | 8.532 | 55.683 | 1.00 | 32.80 |
| ATOM 3330 | O3 | SO4 600 | 19.690 | 6.249 | 55.260 | 1.00 | 33.32 |
| ATOM 3331 | O4 | SO4 600 | 21.572 | 7.178 | 54.108 | 1.00 | 33.97 |
| ATOM 3332 | S | SO4 601 | 22.953 | 22.471 | 69.199 | 1.00 | 77.32 |
| ATOM 3333 | O1 | SO4 601 | 21.971 | 21.759 | 68.356 | 1.00 | 76.19 |
| ATOM 3334 | O2 | SO4 601 | 22.411 | 23.803 | 69.553 | 1.00 | 77.48 |
| ATOM 3335 | O3 | SO4 601 | 23.205 | 21.698 | 70.433 | 1.00 | 77.23 |
| ATOM 3336 | O4 | SO4 601 | 24.224 | 22.628 | 68.461 | 1.00 | 77.19 |
| ATOM 3337 | NA+1 | NA1 602 | 17.158 | 10.244 | 54.280 | 1.00 | 10.17 |
| ATOM 3338 | OH2 | HOH 603 | 19.770 | 14.543 | 47.159 | 1.00 | 1.00 |
| ATOM 3340 | OH2 | HOH 604 | 20.723 | 24.387 | 67.178 | 1.00 | 17.94 |
| ATOM 3341 | OH2 | HOH 605 | 10.880 | 33.802 | 37.628 | 1.00 | 1.00 |
| ATOM 3342 | OH2 | HOH 606 | 22.743 | 28.762 | 37.147 | 1.00 | 31.78 |
| ATOM 3343 | OH2 | HOH 607 | 38.906 | 1.328 | 74.611 | 1.00 | 37.76 |
| ATOM 3344 | OH2 | HOH 608 | 1.237 | 30.510 | 46.162 | 1.00 | 32.40 |
| ATOM 3345 | OH2 | HOH 609 | 34.702 | -1.731 | 56.455 | 1.00 | 62.03 |
| END | | | | | | | |

Table 2 is made according to a description method of Protein Data Bank conventionally used by those skilled in the art. In Table 2, HOH represents a water molecule.

In the present invention, a crystal of a protein having substantially the same amino acid sequence as that of

SEQ ID No. 5 and/or SEQ ID No. 8 and having a glucokinase activity is within the range of the present invention. Mentioned as such a crystal are, for example, crystals wherein, in three dimensional structure coordinate data obtained by changing at least one datum in three dimensional structure coordinate data described in Table 1 and/or Table 2, the average square deviation between an atom (Cα atom) of a main chain of an amino acid shown by three dimensional structure coordinate data described in Table 1 and/or Table 2 and a Cα atom shown by the above-mentioned changed three dimensional structure coordinate data corresponding to the Cα atom is 0.6 Å or less. Even if the numerical value of a coordinate representing the position of an atom varies, two structure coordinates containing corresponding atoms of which positions can be overlapped represent the same three dimensional structure.

The three dimensional structure coordinates of a GK protein described in Table 1 and/or Table 2 are important information for drug design, and if necessary, stored in a storage medium readable by a computer, and this information is treated by a computer to perform drug design. Therefore, according an another embodiment of the present invention, there is provided a computer-readable storage medium containing a recorded program for allowing a computer to function as a three dimensional coordinate memory means for storing three dimensional coordinates of amino acid residues described in Table 1 and/or Table 2.

According an another embodiment of the present invention, there is provided a computer-readable recording medium containing a recorded program for allowing a computer to function as a three dimensional coordinate memory means storing information regarding three dimensional coordinates of amino acid residues described in Table 1 and/or Table 2, as a binding portion guessing means for guessing a compound binding portion of a protein having an amino acid sequence as depicted in SEQ ID No. 5 and/or SEQ ID No. 8 using three dimensional coordinates of amino acid residues described in Table 1 and/or Table 2 stored in the above-mentioned three dimensional coordinate memory means, as a binding compound memory means memorizing information regarding the kind of a compound connected to a protein and the three dimensional structure of the compound, and as a binding compound candidate selection means for selecting candidates of a compound suitable for a compound binding portion of a protein having an amino acid sequence as depicted in SEQ ID No. 1 of the sequence table, using, at least, information regarding the three dimensional structure of a compound binding portion of a protein having an amino acid sequence as depicted in SEQ ID No. 5 and/or SEQ ID No. 8 guessed by the above-mentioned binding portion guessing means and information regarding the three dimensional structure of a compound stored in the above-mentioned binding compound memory means. Further, according to another embodiment of the present invention, a computer equipped with the above-mentioned means is also provided.

(Crystal of complex of GK protein and compound binding to this)

Next, according to another embodiment of the present invention, there are provided a crystal containing a complex composed of a protein containing an amino acid sequence as depicted in SEQ ID No. 5 or SEQ ID No. 8 or an amino acid sequence which is substantially the same as this amino acid sequence and of a compound capable of binding to this protein, and a method of producing the crystal.

When a compound binding to a GK protein is obtained, a GK protein and its compound are, first, for example, mixed in an aqueous solution to form a complex. A crystal of such a complex is produced by known co-crystallization methods such as a co-crystallization method, soaking method and the like. Regarding crystallization conditions and crystallization methods, the above-mentioned methods are referred to.

The compound binding to a GK protein is selected, for example, from a group of compounds of the above-described formula (I).

Here, as the halogen atom in the above-described formula (I), a fluorine atom, chlorine atom, bromine atom, iodine atom and the like are exemplified, and of them, a chlorine atom is preferable.

As the substituent on A, B in the above-mentioned formula (I) and a heteroaryl group of the formula (II), there are listed an amino group, carbamoyl group, carbamoylamino group, carbamoyloxy group, carboxyl group, cyano group, sulfamoyl group, trifluoromethyl group, halogen atom, hydroxyl group, formyl group, straight-chain $C_1$ to $C_6$ alkyl group, cyclic $C_3$ to $C_6$ hydrocarbon group, aralkyl group, N-aralkylamino group, N,N-diaralkylamino group, aralkyloxy group, aralkylcarbonyl group, N-aralkylcarbamoyl group, aryl group, arylthio group, N-arylamino group, aryloxy group, arylsulfonyl group, arylsulfonyloxy group, N-arylsulfonylamino group, arylsulfamoyl group, N-arylcarbamoyl group, aroyl group, aroxy group, $C_2$ to $C_6$ alkanoyl group, N-$C_2$ to $C_6$ alkanoylamino group, $C_1$ to $C_6$ alkylthio group, N-$C_1$ to $C_6$ alkylsulfamoyl group, N,N-di-$C_1$ to $C_6$ alkylsulfamoyl group, $C_1$ to $C_6$ alkylsulfinyl group, $C_1$ to $C_6$ alkylsulfonyl group, N-$C_1$ to $C_6$ alkylsulfonylamino group, $C_1$ to $C_6$ alkoxy group, $C_1$ to $C_6$ alkoxycarbonyl group, $C_1$ to $C_6$ alkylamino group, and the like. Preferable examples thereof used here include an amino group, carbamoyl group, carbamoylamino group, carbamoyloxy group, carboxyl group, cyano group, sulfamoyl group, trifluoromethyl group, halogen atom, hydroxyl group, formyl group, straight-chain $C_1$ to $C_6$ alkyl group and the like.

Here, "hydrocarbon group" means a straight-chain $C_1$ to $C_6$ alkyl group, or a group in which 1 or 2, preferably 1 of carbon atoms constituting the alkyl group may be substituted by a nitrogen atom, sulfur atom or oxygen atom and/or carbon atoms in the straight-chain $C_1$ to $C_6$ alkyl group may be mutually connected via a double bond or triple bond. The number of the double bond or triple bond is preferably 1 or 2, more preferably 1.

It is preferable that the hydrocarbon group is selected from specifically a methyl group, ethyl group, propyl group, isopropyl group, butyl group and groups of the following formulae.

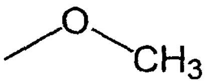, 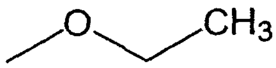, 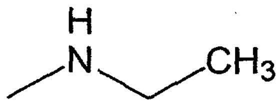, 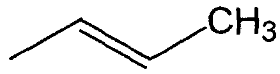,

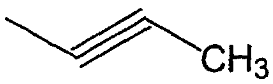, 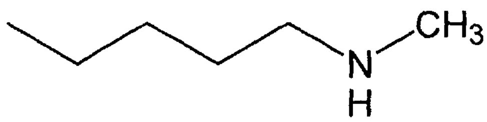,

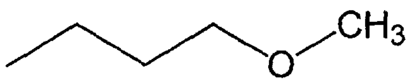, 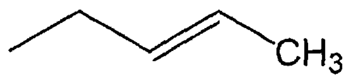,

or

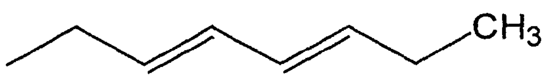

More preferably, the hydrocarbon group is selected from a methyl group, ethyl group, propyl group, isopropyl group and groups of the following formulae.

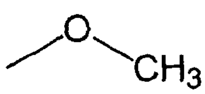, 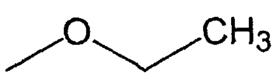, 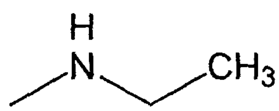,

or

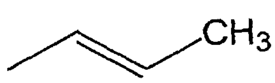

As preferable A (when p = 0), the following groups are exemplified.

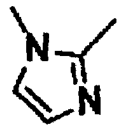 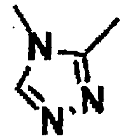 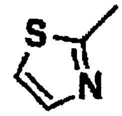

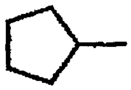 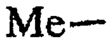 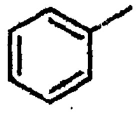 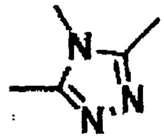 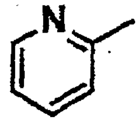

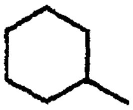 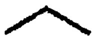

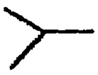 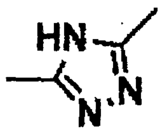 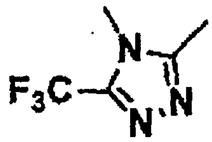 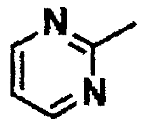

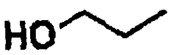 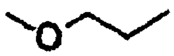

As preferable B, the following groups are exemplified.

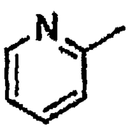 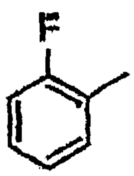 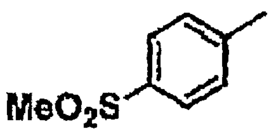 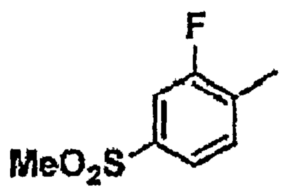

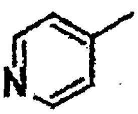 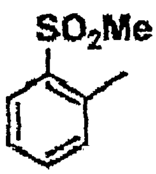 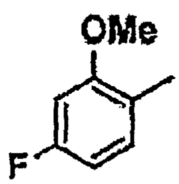 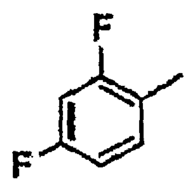

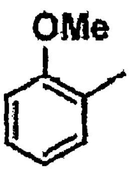 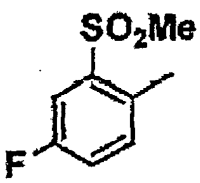

As the heteroaryl group of the formula (II), the following heterocyclic groups are exemplified.

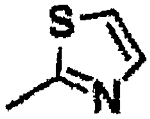
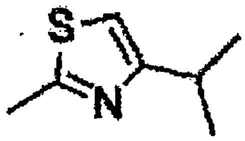
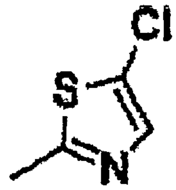
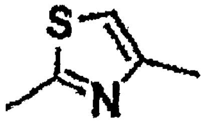
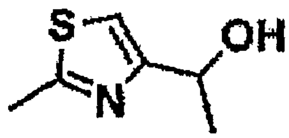
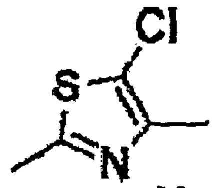
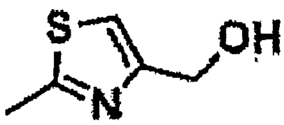
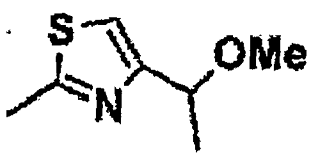
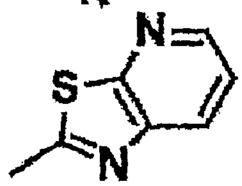
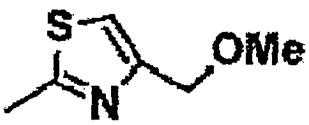
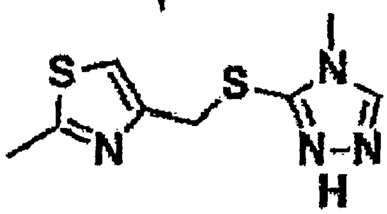
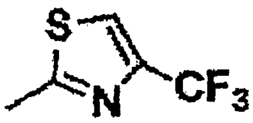
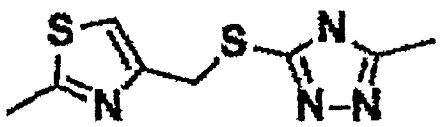
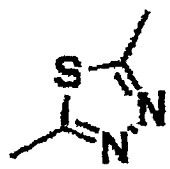

Particularly preferable compounds are compounds of the above-described formulae (IIIa) to (IIIc).

The compound (I) of the present invention can be produced easily using known reaction means or according to known methods. A compound of the general formula (I) of the present invention can be produced not only by usual liquid phase synthesis methods but also by solid phase synthesis methods such as for example combinatorial method, parallel synthesis method and the like showing remarkable development recently. Preferably, the compound (I) can be produced by, for example, the following method.

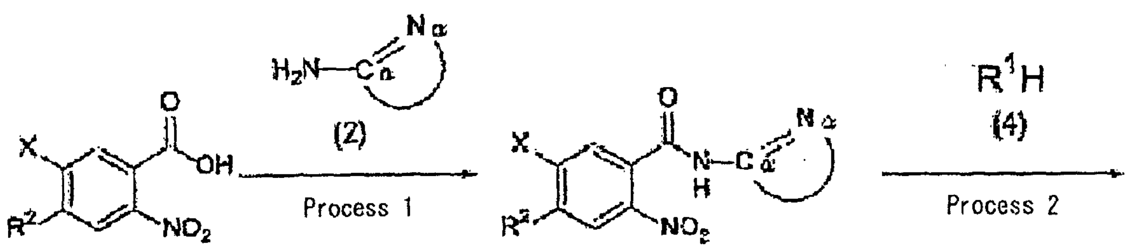


(1) (3)

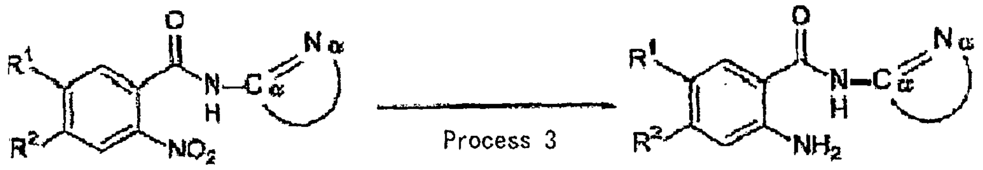


(5) (I-1)

[wherein, marks are as defined above]

(Process 1)

This process is a method in which a carboxylic acid compound (1) or its reactive derivative is reacted with a mono-cyclic or di-cyclic amino compound optionally substituted having a heteroaryl group of the above-mentioned formula (2), to produce a compound (3). In this reaction, a usual amide formation reaction may be advantageously conducted by methods described in literatures (for example, Izumiya Nobuo, et al., Peptide Gosei no Kiso to Jikken, Maruzen, 1983, Comprehensive Organic Synthesis, vol. 6, Pergamon Press, 1991, and the like), methods according to these methods, or combinations of them with ordinary methods, that is, this process can be conducted using a condensing agent well known to those skilled in the art, or by an ester activating method, mixed acid anhydride method, acid chloride method, carbodiimide method and the like utilizable by those skilled in the art. Suitable as such amide forming reagents are, for example, thionyl chloride, N,N-dicyclohexylcarbodiimide, 1-methyl-2-bromopyridinium iodide, N,N'-carbonyl diimidazole, diphenylphosphoryl chloride, diphenylphosphorylazide, N,N'-disuccinimidyl carbonate, N,N'-disuccinimidyl oxalate, 1-ethyl-3-(3-dimethylaminopropyl)carbodiimide hydrochloride, ethyl chloroformate, isobutyl chloroformate, benzotriazo-1-yl-oxy-tris(dimethylamino)phosphonium hexafluorophosphate and the like, and of them, thionyl chloride, N,N-dicyclohexylcarbodiimide, benzotriazo-1-yl-oxy-tris(dimethylamino)phosphonium hexafluorophosphate and the like are preferable. In the amide formation reaction, a base and condensing aid may be used together with the above-mentioned amide formation reagents.

Example of the base used include tertiary aliphatic amines such as trimethylamine, triethylamine, N,N-diisopropylethylamine, N-methylmorpholine, N-methylpyrrolidine, N-methylpiperidine, N,N-dimethylaniline, 1,8-diazabicyclo[5.4.0]undec-7-ene (DBU), 1,5-azabicyclo[4.3.0]nona-5-ene (DBN) and the like; aromatic amines such as pyridine, 4-dimethylaminopyridine, picoline, lutidine, quinoline, isoquinoline and the like, and of them, for example, tertiary aliphatic amines are preferable, and particularly, triethylamine, N,N-diisopropylethylamine and the like are suitable.

As the condensing aid used, for example, N-hydroxybenzotriazole hydrate, N-hydroxysuccinimide, N-hydroxy-5-norbornene-2,3-dicarboxyimide, 3-hydroxy-3,4,-dihydro-4-oxo-1,2,3-benzotriazole and the like are listed, and of them, N-hydroxybenzotriazole and the like are suitable.

The amount of the amino compound (2) used varies depending on the kinds of a compound and a solvent used and other reaction conditions, and usually, is 0.02 to 50 equivalent, preferably 0.2 to 2 equivalent per mol of the carboxylic acid compound (1) or its reactive derivative. Here, mentioned as the reactive derivative are, for example, active ester derivatives, active amide derivatives and the like, usually used in the field of organic chemistry.

The amount of the amide formation reagent used varies depending on the kinds of a compound and a solvent used and other reaction conditions, and usually, is 1 to 50 equivalent, preferably 1 to 5 equivalent per mol of the carboxylic acid compound (1) or its reactive derivative.

The amount of the condensing aid used varies depending on the kinds of a compound and a solvent used and other reaction conditions, and usually, is 1 to 50 equivalent, preferably 1 to 5 equivalent per mol of the carboxylic acid compound (1) or its reactive derivative.

The amount of the base used varies depending on the kinds of a compound and a solvent used and other reaction conditions, and usually, is 1 to 50 equivalent, preferably 3 to 5 equivalent.

The reaction solvent used in this process is, for example, an inert organic solvent and is not particularly restricted providing it causes no disturbance in the reaction, and specific examples thereof include methylene chloride, chloroform, 1,2-dichloroethane, trichloroethane, N,N-dimethylformamide, acetic acid ethyl ester, acetic acid methyl ester, acetonitrile, benzene, xylene, toluene, 1,4-dioxane, tetrahydrofuran, dimethoxyethane or mixed solvents thereof, and from the standpoint of insurance of suitable reaction temperature, for example, methylene chloride, chloroform, 1,2-dichloroethane, acetonitrile, N,N-dimethylformamide and the like are particularly suitable.

The reaction temperature is from -100°C to the boiling point of a solvent, preferably 0 to 30°C.

The reaction time is 0.5 to 96 hours, preferably 3 to 24 hours.

The base, amide formation reagent and condensing aid used in this process 1 can be used singly or in combination of two or more.

When the compound (3) has a protective group, this protective group can be removed appropriately. Removal of this protective group can be conducted by methods described in literatures (Protective Groups in Organic Synthesis, T.W. Green, second ed., John Wiley & Sons, 1991, and the like), methods according to these methods, or combinations of them with ordinary methods.

Thus obtained compound (3) can be isolated and purified by known separation purification means, for example, concentration, concentration under reduced pressure, crystallization, extraction with solvent, re-precipitation, chromatography and the like, or can be used in the subsequent process without isolation and purification.

(Process 2)

This process is a method of reacting an amide compound (3) obtained in the above-mentioned process 1 with a compound (4) to produced a compound (5).

In this reaction, a base may be added into the reaction system, if necessary. As the compound (4) used, phenols derivatives and thiol derivatives are preferable. As the phenols derivatives and thiol derivatives, for example, phenol, thiophenol, thioimidazole, thiotriazole and the like are mentioned. The amount of the compound (4) used varies depending on the kinds of a compound and a solvent used and other reaction conditions, and usually, is 2 to 50 equivalent, preferably 2 to 5 equivalent per mol of an amino derivative (3). Example of the base used include tertiary aliphatic amines such as trimethylamine, triethylamine, N,N-diisopropylethylamine, N-methylmorpholine, N-methylpyrrolidine, N-methylpiperidine, N,N-dimethylaniline, 1,8-diazabicyclo[5.4.0]undec-7-ene (DBU), 1,5-azabicyclo[4.3.0]non-5-ene (DBN) and the like; aromatic amines such as pyridine, 4-dimethylaminopyridine, picoline, lutidine, quinoline, isoquinoline and the like, alkali metal such as metal potassium, metal sodium, metal lithium and the like; alkali metal hydroxide such as sodium hydroxide, potassium hydroxide and the like, alkylated alkali metals such as butyl lithium and the like, alkali metal alkoxides such as potassium-tert-butylate, sodium ethylate, sodium methylate and the like, alkali metal hydroxides such as potassium hydroxide, sodium hydroxide and the like, and alkali metal carbonates such as potassium carbonate and the like, and of them, for example, tertiary aliphatic amines, alkali metal hydroxides and alkali metal carbonates are preferable, and particularly, triethylamine, N,N-diisopropylethylamine, sodium hydroxide and potassium carbonate are suitable.

The amount of the base used varies depending on the kinds of a compound and a solvent used and other reaction conditions, and usually, is 0 to 50 equivalent, preferably 2 to 10 equivalent per mol of an amino derivative (3). One or more bases can be used, if necessary.

The inert organic solvent used is not particularly restricted providing it causes no disturbance for the reaction, and specific examples thereof include methylene chloride, chloroform, 1,2-dichloroethane, trichloroethane, N,N-dimethylformamide, N,N-dimethylacetamide, acetic acid ethyl ester, acetic acid methyl ester, acetonitrile, benzene, xylene, water, toluene, 1,4-dioxane, tetrahydrofuran, or mixed solvents thereof.

Thus obtained compound (5) can be isolated and purified by known separation purification means, for example, concentration, concentration under reduced pressure, crystallization, extraction with solvent, re-precipitation, chromatography and the like.

(Process 3)

This process is a method of reducing a compound (5) to produced a compound (I) used in the present invention. As the reducing reaction used in the present invention, methods well known to those skilled in the art can be used. As the reducing reaction used in this process, specifically mentioned are, for example, (1) a catalytic reduction method using hydrogen, formic acid, ammonium formate, hydrazine hydrate and palladium, platinum, nickel catalyst, (2) a reducing method using hydrochloric acid, ammonium chloride, (3) a reducing method using methanol and tin chloride, and other reducing methods.

The amount of the reducing agent used in the above-mentioned reducing reaction varies depending on the kinds of a compound and a solvent used and other reaction conditions, and usually, is 1 to 50 equivalent, preferably 2 to 20 equivalent per mol of a compound (5).

The reaction solvent used is not particularly restricted providing it causes no disturbance for the reaction, and for example, halogenated hydrocarbons such as dichloromethane, chloroform and the like, ethers such as diethyl ether, tert-butyl methyl ether, tetrahydrofuran and the like, amides such as N,N-dimethylformamide, N,N-dimethylacetamide and the like, sulfoxides such as dimethyl sulfoxide and the like, nitriles such as acetonitrile and the like, alcohols such as methanol, ethanol, propanol and the like, aromatic hydrocarbons such as benzene, toluene, xylene and the like, water or mixed solvents thereof can be used.

The reaction temperature and reaction time are not particularly restricted, and the reaction is conducted at a

reaction temperature of about -10 to 100°C, preferably about 0 to 50°C for 1 to 20 hours, preferably 1 to 5 hours.

Thus obtained compound (I) used in the present invention can be isolated and purified by known separation purification means, for example, concentration, concentration under reduced pressure, crystallization, extraction with solvent, re-precipitation, chromatography and the like, or can be used in the subsequent process without isolation and purification.

The above-mentioned compound in each process may have a protective group on each substituent. The protective group can be removed by known methods and methods according to them, or combinations of them with ordinary methods, appropriately in each process. Removal embodiments can be appropriately selected depending on the compound, the kind of the reaction and other reaction conditions, and when protective groups are removed separately, a case in which protective groups are removed simultaneously, and the like are envisaged, and embodiments can be appropriately selected by those skilled in the art. Listed as the protective group are, for example, protective groups for a hydroxyl group, protective groups for an amino group, protective groups for a carboxyl group, protective groups for an aldehyde, protective groups for a keto group, and the like. The order of removal of these protective groups is not particularly restricted.

The protective group for a hydroxyl group includes lower alkyl silyl groups such as a tert-butyldimethylsilyl group, tert-butyldiphenylsilyl group and the like, lower alkoxymethyl groups such as a methoxymethyl group, 2-methoxyethoxymethyl group and the like, aralkyl groups such as a benzyl group, p-methoxybenzyl group and the like, acyl groups such as a formyl group, acetyl group and the like, and of them, particularly, a tert-butyldimethylsilyl group, acetyl group and the like are preferable.

The protective group for an amino group includes aralkyl groups such as a benzyl group, p-nitrobenzyl group and the like, acyl groups such as a formyl group, acetyl group and the like, lower alkoxycarbonyl groups such as an ethoxycarbonyl group, tert-butoxycarbonyl group and the like, aralkyloxycarbonyl groups such as a benzyloxycarbonyl group, p-nitrobenzyloxycarbonyl group and the like, and of them, particularly, a nitrobenzyl group, tert-butoxycarbonyl group, benzyloxycarbonyl group and the like are preferable.

The protective group for a carboxyl group includes lower alkyl groups such as a methyl group, ethyl group, tert-butyl group and the like, aralkyl groups such as a benzyl group, p-methoxybenzyl group and the like, and of them, particularly, a methyl group, ethyl group, tert-butyl group, benzyl group and the like.

The protective group for a keto group includes a dimethyl ketal group, 1,3-dioxylane group, 1,3-dioxolane group, 1,3-dithiane group, 1,3-dithiolane group and the like, and of them, a dimethyl ketal group, 1,3-dioxolane group are more preferable.

The protective group for an aldehyde group includes a dimethyl acetal group, 1,3-dioxylane group, 1,3-dioxolane group, 1,3-dithiane group, 1,3-dithiolane group and the like, and of them, a dimethyl acetyl group, 1,3-dioxolane group and the like are more preferable.

In producing a compound used in the present invention, a protective group is introduced in some cases in a functional group for efficient progress of the reaction. Introduction of the protective group can be appropriately selected by those skilled in the art, and removal of the protective group can be conducted by methods such as Protective Groups in Organic Synthesis and the like, methods according to them, or combinations of them with ordinary methods. The order of removal of the protective group can also be appropriately selected by those skilled in the art.

Thus obtained compound (I) can be isolated and purified by known separation purification means, for example, concentration, concentration under reduced pressure, crystallization, re-precipitation, extraction with solvent, chromatography and the like, or can be used in the subsequent process without isolation and purification.

The compound (I) used in the present invention can be produced also by the following process.

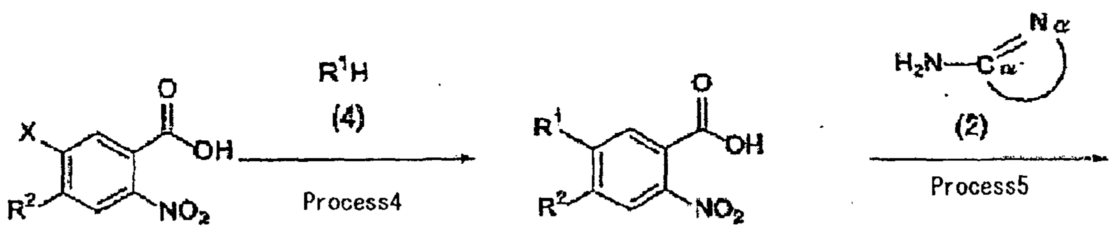


(1)

(6)

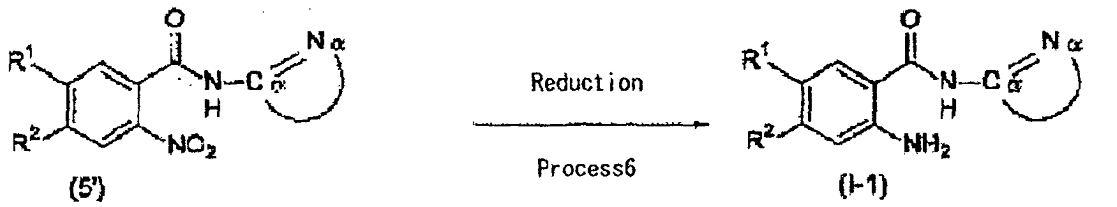


(5') (I-1)

[wherein, marks are as defined above]

In the above-mentioned process 4, process 5 and process 6, the amount of a reagent, reaction solvent, reaction temperature and other reaction conditions can be the same as those in the above-mentioned process 1, process 2 and process 3.

When $R^2$ needs a protective group, the protective group can be appropriately selected by those skilled in the art by the above-mentioned methods such as Protective Groups in Organic Synthesis and the like, methods according to them, or combinations of them with ordinary methods.

Thus obtained compound (6), (5') can be isolated and purified by known separation purification means, for example, concentration, concentration under reduced pressure, crystallization, re-precipitation, extraction with solvent and the like, or can be used in the subsequent process without isolation and purification.

The compound (I) used in the present invention can be isolated and purified by known separation purification means, for example, concentration, concentration under reduced pressure, crystallization, re-precipitation, extraction with solvent and the like.

In the above-mentioned processes 1 to 6, removal of the protective group can be conducted by Protective Groups in Organic Synthesis, T.W. Green, second ed., John Wiley & Sons, 1991, and the like, methods according to this, or combinations of them with ordinary methods, though varying depending on the kind of the protective group and the stability of a compound. For example, the removal can be conducted by solvolysis using an acid or base, chemical reduction using a hydrogenated metal complex and the like, or catalytic reduction using a palladium carbon catalyst, Raney nickel and the like.

The benzamide compound provided by the present invention can exist as a pharmaceutically acceptable salt. This salt can be produced by an ordinary method. Specifically, when the above-mentioned compound (I) has a basic group derived from an amino group, pyridyl group and the like in the molecule, this compound can be treated with an acid to be converted into a corresponding pharmaceutically acceptable salt.

Listed as the acid addition salt are, for example, hydrohalogenates such a hydrochloride, hydrofluoride, hydrobromide, hydroiodide and the like; inorganic acid salts such as nitrate, perchlorate, sulfate, phosphate, carbonate and the like; lower alkylsulfonates such as methanesulfonate, trifluoromethanesulfonate, ethanesulfonate and the like; arylsulfonates such as benzenesulfonate, p-toluenesulfonate and the like; organic acid salts such as fumarate, succinate, citrate, tartarate, oxalate, maleate and the like; acid addition salts which are organic acids such as amino acids such as glutamate, aspartate and the like. When the compound of the present invention has an acidic group in this group, for example, when it has a carboxyl group and the like, it can be converted into a corresponding pharmaceutically acceptable salt also by treating the compound with a base. As this base addition salt, for example, salts of alkali metals such as sodium, potassium and the like, salts of alkali earth metals such as calcium, magnesium and the like, ammonium salt, salts of organic bases such as guanidine, triethylamine, dicyclohexylamine and the like are mentioned. Further, the compound of the present invention may also be present as any hydrate or solvate of a free compound or salt thereof.

In the present invention, as described in examples, a crystal of a complex of a GK protein having an amino acid sequence as depicted in SEQ ID No. 5 with a compound of the above-mentioned formulae (IIIa) to (IIIc) is obtained. By analyzing the three dimensional structure coordinate of the crystal, it is clarified that, in a GK protein as depicted in SEQ ID No. 5, a compound binding portion is constituted of amino acid residues of tyrosine 61 to serine 69, glutamic acid 96 to glutamine 98, isoleucine 159, methionine 210 to tyrosine 215, histidine 218 to glutamic acid 221, methionine 235, arginine 250 and leucine 451 to lysine 459.

According to another embodiment of the present invention, there is provided a method of producing a crystal comprising a complex of a protein with a compound binding to the protein, comprising a protein production step of producing a protein having an amino acid sequence obtained by deletion of given number of amino acid residues at the N terminal side and/or C terminal side from a protein having an amino acid sequence as depicted in SEQ ID No. 2, and a step of reacting a compound binding to a protein obtained in the above-mentioned protein production step with a protein obtained in the above-mentioned protein production step.

The number of proteins produced in the above-mentioned protein production step is not particularly restricted providing there occurs no steric disturbance between an adjacent GK protein in a crystal. Specifically, for example,

amino acid sequences obtained by deletion of 1 to 50, preferably 3 to 30, more preferably 5 to 25, further preferably 8 to 18, particularly preferably 11 to 15 amino acid resides at the N-terminal side, in an amino acid sequence as depicted in SEQ ID No. 2, and the like are listed. Amino acid sequences obtained by deletion of 1 to 8, preferably 1 to 7, more preferably 2 to 6 amino acid resides at the C-terminal side, and the like are listed.

(Drug design method using three dimensional structure coordinate)

The three dimensional structure of a GK protein of the present invention obtained as described above is important information for a drug finding system by CARDD (Computer Aided Rational Drug Design). To clarify the active center and allosteric portion of this GK protein and to search a substance suitable for this portion and interacting with GK protein to disturb or activate the GK protein is an important step for drug development targeting a GK protein.

Namely, according to another embodiment of the present invention, there is provided a drug design method of designing the structure of a compound binding to a protein based on the steric structure information of the protein, wherein the steric structure information of the protein is information obtained by analyzing the crystal obtained as described above. As such a drug design method, there are a method of designing a drug using energy calculation, active prospective value analogous to this, or pharmacophore, and a method of visually designing a drug using a technology of computer graphics.

As the method using energy calculation, active prospective value analogous to this, or pharmacophore, there are exemplified (1) a drug design method comprising a connection portion guessing step of guessing a compound binding portion of the protein, based on steric structure information obtained as described above, and a selection step of selecting a compound adaptable to a compound binding portion guessed in the above-mentioned binding portion guessing step from a compound library, and (2) a binding portion guessing step of guessing a compound binding portion of the protein based on the above-mentioned steric structure information, and a compound structure fabricating step of fabricating the structure of a compound adaptable to a compound binding portion guessed in the above-mentioned binding portion guessing step.

As the method of guessing a compound binding portion of a protein, there is mentioned, for example, a method of estimating and specifying a portion to which a ligand is liable to connect, in a protein crystal structure relaxed without binding of a ligand, in addition to a method of visually confirming a portion of binding of a ligand in a co-crystal with a compound on a computer display. In each method, known methods and commercially available computer soft wares can be used. In the former method, for example, soft wares such as Insight II (Accelrys Inc.), SYBYL (Tripos Inc.), MOE (Chemical Computing Group) and the like can be used. On the other hand, in the latter method, for example, known method such as Cavity search: an algorithm for the isolation and display of cavity-like binding regions. (Journal of Computer-Aided Molecular Design. 4 (4): 337-54, 1990) and the like can be used, and the latter method can be carried out using softwares such as Site ID (Tripos Inc.) and the like.

When a binding portion on a protein with a compound can be guessed, compounds applicable to the guessed binding portion are selected. In this method of selecting compound candidates, structure information of compounds from an existing compound library is obtained, and the structure information of a compounds in the library is compared with the structure information of the binding portion guessed as described above, thus, bindable compound candidates are selected.

More specifically, the compound candidates are selected by judging whether conditions are satisfied or not while searching exhaustively the coordinate and orientation of each compound from a compound library, using, as search conditions, pharmacophores such as hydrogen bonding property or hydrophobicity and the like formed from one or more residues of amino acid residues (tyrosine 61 to serine 69, glutamic acid 96 to glutamine 98, isoleucine 159, methionine 210 to tyrosine 215, histidine 218 to glutamic acid 221, methionine 235, arginine 250 and leucine 451 to lysine 459) of an amino acid sequence as depicted in SEQ ID No. 5 or functional groups of ligands in a complex, and a protein surface made of a protein structure or a structure obtained by modifying the orientation of partial side chains.

In other alternative method, a candidate compound is virtually docked to the structure of a ligand binding portion constituted of amino acid residues (tyrosine 61 to serine 69, glutamic acid 96 to glutamine 98, isoleucine 159, methionine 210 to tyrosine 215, histidine 218 to glutamic acid 221, methionine 235, arginine 250 and leucine 451 to lysine 459) or a structure obtained by modifying the orientation of partial side chains, while searching exhaustively the coordinate and orientation of each compound from a compound library, and those forming a close interaction with one or more residues at a distance of 4 Å or less are selected from amino acid residues (tyrosine 61 to serine 69, glutamic acid 96 to glutamine 98, isoleucine 159, methionine 210 to tyrosine 215, histidine 218 to glutamic acid 221, methionine 235, arginine 250 and leucine 451 to lysine 459).

On the other hand, candidate compounds can also be selected by designing a bindable compound based on the structure information of a binding portion guessed as described above. More specifically, a compound structure is fabricated by variously connecting various atoms and functional groups so as to form an interaction with one or more

residues, of the structure of a compound binding portion constituted of amino acid residues (tyrosine 61 to serine 69, glutamic acid 96 to glutamine 98, isoleucine 159, methionine 210 to tyrosine 215, histidine 218 to glutamic acid 221, methionine 235, arginine 250 and leucine 451 to lysine 459) of an amino acid sequence as depicted in SEQ ID No. 5 or a structure obtained by modifying the orientation of partial side chains. As this method, a method in which chemical groups such methyl, ethyl and the like are arranged at an active portion and suitable compounds are searched, and a method in which atoms are allowed to bind to an active portion using a computer program, are known.

As the method for energy evaluation by a computer, for example, there is a method in which bonding energy of a compound and a GK protein is obtained by molecular force field calculation. This calculation is applied to each compound in a database, and compound candidates capable of stably binding are selected from compounds in a library. Some computer programs such as Ludi of Insight II, and the like automatically select and output bindable compound candidates when the three dimensional structure coordinate of amino acid residues mutually acting in a protein molecule is imparted, and these programs can be suitably used.

Regarding drug design based on the three dimensional structure of a molecule, a lot of literatures including Iyakuhin no Kaihatsu, vol. 7, "Molecule Design", (Hirokawa Shoten) are known. Specifically, first, a library (for example, about 150000 kinds) of lower molecule compounds (molecular weight: 1000 or less) can be screened, using a flexible ligand binding simulation software such as FlexiDock, Flex X and the like. Regarding chemical substances in this library, a three dimensional structure is constituted by a program such as CONCORD and the like, and compounds suitable for an active portion can be selected.

On the other hand, as the method of visually designing a drug, there is a drug design method comprising a binding portion guessing step of guessing a compound binding portion of the protein based on the steric structure information, and a design step of designing visually the structure of a compound so that a compound binding portion guessed in the above-mentioned binding portion guessing step and a compound adaptable to the compound binding portion interact. For example, structure fabrication or structure modification is visually conducted so as to form an interaction with one or more residues, of the structure of a ligand binding portion constituted of amino acid residues (tyrosine 61 to serine 69, glutamic acid 96 to glutamine 98, isoleucine 159, methionine 210 to tyrosine 215, histidine 218 to glutamic acid 221, methionine 235, arginine 250 and leucine 451 to lysine 459) of an amino acid sequence as depicted in SEQ ID No. 5 or a structure obtained by modifying the orientation of partial side chains.

Specifically, in a visual method, first, the structure of a crystal of a complex of a GK protein with a compound capable of binding with this is displayed on a screenof a computer according to the resulted structure coordinate. Bindabilities of compounds in a library with a GK protein are sequentially investigated while considering a chemical interaction on a computer. The chemical interaction to be considered here include an electrostatic interaction, hydrophobic interaction, hydrogen bond, van der Waals interaction and the like. Namely, a synthetic consideration is made whether the structure of the compound in a three dimensional space constitutes a preferable structure for interaction, so that a group liable to be negatively charged such as a carboxyl group, nitro group, halogen group and the like in the group of functional groups interacts with an amino acid residue having positive charge such as lysine, arginine, histidine of GK protein, so that a group liable to be positively charged such as an amino group, imino group, guanidyl group interacts with an amino acid residue having negative charge such as glutamic acid, aspartic acid of a GK protein, so that a hydrophobic functional group such as an aliphatic group and aromatic group interacts with a hydrophobic amino acid residue such as alanine, leucine, isoleucine, valine, proline, phenylalanine, tryptophane and methionine, so that a group correlated with a hydrogen bond such as a hydroxyl group, amide group and the like can form a hydrogen bond with a main chain or side chain portion of a GK protein, further, so that steric hindrance does not occur on binding of the compound with a GK protein, further, so that a void portion is filled to decrease a void portion as small as possible, and van der Waals interaction increases, and the like. Thus, factors such as an electrostatic interaction, hydrophobic interaction, van der Waals interaction, hydrogen bond and the like are synthetically considered visually on a computer screen, and judge whether a candidate compound can finally bind to a GK protein or not is made.

As the program for selecting compound candidates visually, simulation programs such as Insight II, MOE and the like are exemplified. For listing important candidates of compounds interacting with a GK protein, candidates compounds and GK protein are allowed to contact, and the activity of a GK protein is measured. Applicability of important candidate compounds is investigated by mixing important candidate compounds actually with a GK protein and crystallizing the mixture. Further, an applicable complex is modified by organic synthesis to give a more desired structure.

A visual means and a means considering energy can be appropriately combined and used. Such a computer software includes FlexiDock (Tripods Inc.), FlexX (Tripods Inc.), SYBYL (Tripods Inc.), Insight II (Accelrys Inc.), MOE (Chemical Computing Group Inc.) and the like.

In the present invention, compounds selected by the above-mentioned drug design methods are actually synthesized, and a group of these compounds can be provided as a compound array (or compound library). If such a compound array is used, a large amount of candidate compounds can be assayed in one time using a technology such as high throughput screening and the like, therefore, the activating agent or an inhibitor of a glucokinase can efficiently be screened.

(Compound obtained by method of the present invention and therapeutic compound containing this)

Since a compound designed by the above-mentioned drug design methods has an ability of binding to a glucokinase, these compounds can be used as an activating compound for a glucokinase or a glucokinase inhibiting compound. Therapeutic agents or medical compositions containing such compounds can be effectively used as therapeutic agents correlated with a glucokinase activity (for example, diabetes treating agent).

The above-mentioned medical composition contains a pharmaceutically effective amount of a compound binding to a glucokinase of the present invention as an effective component, together with a suitable pharmaceutically acceptable carrier or diluent. As the pharmaceutically acceptable carrier which can be utilized in the above-mentioned medical composition (medical preparation), there are exemplified diluents and excipients such as a filler, extender, binding agent, wetting agent, disintegrator, surfactant, lubricant and the like, usually used corresponding to preparation use forms. These carriers are appropriately selected and used depending on the dose form of the resulted preparation.

Regarding the dose form of the medical composition of the present invention, various forms can be selected depending on treatment objects, and typical examples thereof include solid dose forms such as a tablet, pill, powder, pulvis, granule, capsule and the like, and liquid dose forms such as a solution, suspension, emulsion, syrup, elixir and the like, and these are further classified into an oral drug, parenteral drug, nasal drug, transvaginal drug, suppository, sublingual drug, ointment and the like depending on administration route, and can be blended, molded and prepared according to usual methods, respectively. For example, in molding into a tablet, examples of the above-mentioned preparation carriers capable of being used include excipients such as lactose, white sugar, sodium chloride, glucose, urea, starch, calcium carbonate, kaolin, crystalline cellulose, silic acid, potassium phosphate and the like, binding agents such as water, ethanol, propanol, simple syrup, glucose liquid, starch liquid, gelatin solution, carboxymethyl cellulose, hydroxypropyl cllulose, methyl cellulose, polyvinylpyrrolidone and the like, disintegrators such as carboxymethyl cellulose sodium, carboxymethyl cellulose calcium, low substitution hydroxypropyl cellulose, dry starch, sodium arginate, agar powder, laminaran powder, sodium hydrogen carbonate, calcium carbonate and the like, surfactants such as polyoxyethylenesorbitan fatty acid esters, sodium laurylsulfate, stearic mono-glyceride and the like, disintegration suppressing agents such as white sugar, stearin, cacao butter, hydrogenated oil and the like, absorption promoters such as quaternary ammonium base, sodium laurylsulfate and the like, wetting agents such as glycerin, starch and the like, adsorbents such as starch, lactose, kaolin, bentonite, colloidal silic acid and the like, lubricants such as purified talc, stearate, boric acid powder, polyethylene glycol and the like. Further, the tablet can be, if necessary, a tablet coated generally, for example, a sugar-coated tablet, gelatin-coated table, enteric coated table, film coated tablet, and can also be a double-layer tablet or multi-layer tablet.

In molding into a pill form, examples of preparation carriers capable of being used include excipients such as glucose, lactose, starch, cacao butter, hardened vegetable oil, kaolin, talc and the like, binding agents such as gum Arabic powder, tragacanth powder, gelatin, ethanol and the like, disintegrators such as laminaran, starch and the like.

The capsule is prepared by usually mixing effective components in the present invention with various preparation carries exemplified above according to ordinary methods and filling the mixture in a hard gelatin capsule, soft capsule and the like.

Liquid dose forms for oral administration include conventionally used inert diluents, for example, pharmaceutically acceptable solutions including water, and emulsion, suspension, syrup, elixir and the like, further, auxiliaries such as a wetting agent, emulsifier, suspending agent and the like are allowed to be contained, and these are prepared according to ordinary methods.

In preparing liquid dose forms for parenteral administration, for example, sterile aqueous or non-aqueous solutions, emulsion, suspension and the like, there can be used as a diluent, for example, water, ethyl alcohol, propylene glycol, polyethylene glycol, ethoxylated isostearyl alcohol, polyoxylated isostearyl alcohol, polyoxyethylene sorbitan fatty acid ester, olive oil and the like, and injectable organic esters, for example, ethyl oleate and the like can be compounded. To them, usual dissolution auxiliaries, buffers, wetting agents, emulsifiers, suspending agents, preservatives, dispersing agents and the like can be further added. Sterilization can be performed by a filtration operation through a bacteria holding filter, compounding of a bacteriocide, irradiation treatment and heating treatment and the like. These can also be prepared into a sterile solid composition form which can be dissolved in sterile water or suitable sterilizable medium directly before use.

In molding into a form of suppository and transvaginal preparation, examples of preparation carriers capable of being used include polyethylene glycol, cacao butter, higher alcohol, esters of higher alcohol, gelatin, semi-synthetic glyceride and the like.

In molding into a form of ointment such as paste, cream, gel and the like, examples of diluents capable of being used include white Vaseline, paraffin, glycerin, cellulose derivatives, propylene glycol, polyethylene glycol, silicon, ventonite and vegetable oils such as olive oil and the like.

Compositions for nasal or sublingual administration can be prepared according to ordinary methods using well-known standard excipients.

In the drug of the present invention, coloring agents, preservatives, aromatics, flavors, sweeteners and other medical products and the like are allowed to be contained, if necessary.

The amount and dosage of effective components to be contained in the above-mentioned medical preparations are not particularly restricted, and are appropriately selected widely depending on desired therapeutic affect, administration method, treatment period, age, sex and other conditions of patient, and the like. In general, the dose is usually advantageously about 0.01 mg to 1000 mg, preferably about 1 mg to 100 mg per day per body weight of 60 kg, and the drug can be administered once or in several time a day.

SEQ ID No. in the sequence list in this specification shows the following sequences.

[SEQ ID No.1]

It shows a base sequence of DNA encoding a human-derived liver type glucokinase.

[SEQ ID No. 2]

It shows an amino acid sequence of a human-derived liver type glucokinase.

[SEQ ID No. 3]

It shows an amino acid sequence of a human-derived β-cell glucokinase.

[SEQ ID No. 4]

It shows a base sequence of DNA encoding a protein obtained by deletion of 11 amino acid residues at the N-terminal of a human-derived liver type glucokinase.

[SEQ ID No. 5]

It shows an amino acid sequence of a protein obtained by deletion of 11 amino acid residues at the N-terminal of a human-derived liver type glucokinase.

[SEQ ID No. 6]

It shows a base sequence of primer 1, used in a PCR reaction in the following Example 1.

[SEQ ID No.7]

It shows a base sequence of primer 2, used in a PCR reaction in the following Example 1.

[SEQ ID No. 8]

It shows an amino acid sequence of a protein obtained by deletion of 15 amino acid residues at the N-terminal of a human-derived liver type glucokinase.

[SEQ ID No. 9]

It shows a base sequence of a primer, used in a PCR reaction in the following Example 6.

[SEQ ID No. 10]

It shows a base sequence of a primer, used in a PCR reaction in the following Example 6.

(EXAMPLES)

The present invention will be illustrated specifically using examples.

(Method of purification of variant enzyme)

Human glucokinases are classified into liver type glucokinases and pancreas type glucokinases depending on a difference in promoters, and they differ in 15 residues at the N-terminal. For crystallization intending analysis of a three dimensional structure, variant enzymes in which part or all of this part was deficient was produced in the following method.

Two kind of primer sets and cDNA of Human liver type glucokinase cloned on pCR2.1 (manufactured by INTROGEN)

PCR reactions were conducted using a combination of

5'-gtcacaaggagccagaagcttatggccttgactctggtag-3' (SEQ ID No. 6),

and

5'-gaagccccacgacattgttcccttctgc-3 (SEQ ID No. 7), a

and

5'-ccaggcccagacagccaagcttatggtagagcagatcc-3' (SEQ ID No. 9),

5'-gaagccccacgacattgttcccttctgc-3' (SEQ ID No. 10).

Hind III, ClaI fragment of the resulted PCR product was substituted with Hind III-Cla I region of liver type GK cloned on Hind III, Eco RI portion of pFLAG·CTC vector (Eastman Kodak), to obtain a cDNA coding a variant type GK (Δ1-11) having deletion of 1 to 11 residues of liver type GK and a variant type GK (Δ1-15) having deletion of 1 to 15 residues. The sequence of the resulted cDNA was recognized, then, E. coli DH5 α strain (manufactured by Takara Shuzo Co., Ltd.) was transformed using these vectors as a manifestation vector.

Transformants were cultivated on a LB medium until absorption at 600 nm reached 0.8, then, isopropyl-1-thio-β-D-galaxide (manufactured by Wako Pure Chemical Ltd.) was added to give a final concentration of 0.4 mM, and production of a protein was induced at 25°C for 16 hours.

The cultivated E. coli was collected in a centrifugal separator, and suspended in a buffer containing the following components (50 mM potassium phosphate, pH 7.5, 50 mM NaCl, 2 mM DTT, 0.5 mM Pefabloc SC (manufactured by Kanto Kagaku K.K.), a proteinase inhibitor mixture (manufactured by Roche)).

The collected E. coli was crushed by an ultrasonic crushing method, and soluble fractions were dialyzed into the above-mentioned buffer, then, purified by a HiTrapQ column (manufactured by Amersham). The GK fraction eluted by gradient of potassium chloride by a HiTrapQ column was diluted to a salt concentration of 50 mM.

The diluted GK fraction was purified by a Glucosamin Sepharose column produced by a method shown in a literature (Preparative Biochemistry, 20 (2), 163-178 (1990)). The GK fraction was adsorbed on a Glucosamin Sepharose column, and impurities were removed by 100 mM sodium chloride, then, eluted by 1 M glucose.

The eluted GK fraction was purified by a MonoQ10/10 column. The GK fraction eluted by gradient of sodium chloride by a MonoQ10/10 column (manufactured by Amersham) was purified by a Superdex 200 column (manufactured by Amersham) using 50 mM Tris-Cl pH 7.2, 50 mM NaCl buffer as a mobile phase.

(Crystallization method)

(Crystal of variant type GK (Δ1-11)/glucose/compound complex)

The crystal of variant type GK (Δ1-11)/glucose/compound complex was obtained using a vapor diffusion means described below. The variant type GK (Δ1-11) means a glucokinase having an amino acid sequence as depicted in SEQ ID No. 5.

Namely, the variant type GK purified at high purity was concentrated, to obtain a solution of the variant type GK (25 mM Tris-Cl, 50 mM NaCl, 5 mM TCEP) having a final concentration of about 10 mg/ml. To this was added glucose of a final concentration of 20 mM and the following compound 1 (compound of the formula IIIa) having a final concentration of 0.3 mM activating GK, and used for crystallization. To 1 to 5 μl of a protein solution was added the equal amount of 28 to 30% PEG 1500, 0.1 M Hepes-NaOH (pH 6.6) as a crystallization solution and mixed to give a solution which was accommodated in a closed vessel containing 0.5 to 1 ml of a crystallization solution so that both solutions did not come into contact, and allowed to stand still at 20°C. After standing still for about 3 days to 1 month, a crystal having a maximum size of about 0.4 mm × 0.4 mm × 0.7 mm was obtained in the sample solution (Example 1).

Further, the crystal obtained by the above-mentioned method was immersed in 28 to 30% PEG 1500, 0.1 M Hepes-NaOH (pH 6.6) for about 3 to 7 days so that the following compound 2 (compound of the formula IIIb) was contained in a concentration of 0.3 mM, to obtain a complex crystal of the following compound 2 and the above-mentioned variant type GK (Example 2).

Compound 1

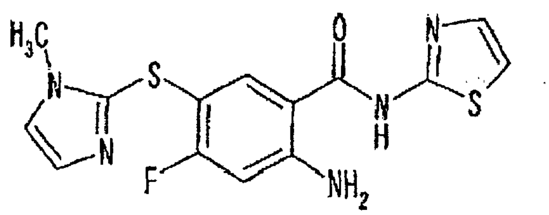

(IIIa)

Compound 2

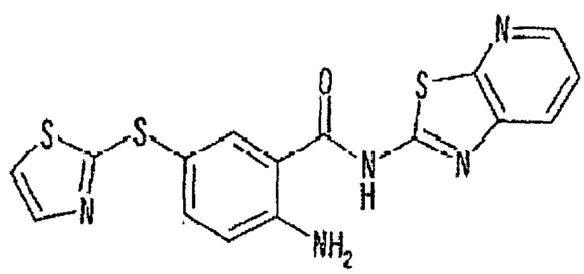

(IIIb)

Crystallization was tried in the same manner as in Example 1 except that a compound 3 (compound of the formula IIIc) was used instead of the above-mentioned compound 1, as a result, the same crystal as in Example 1 was obtained (Example 3).

Compound 3

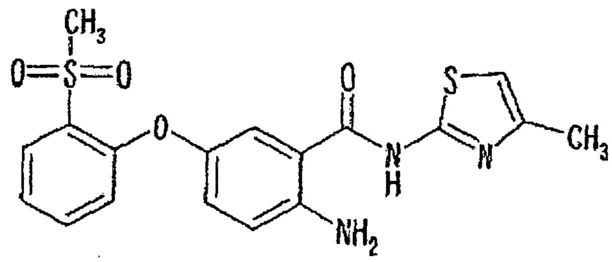

(IIIc)

The resulted crystal was immersed in a crystallization solution containing 10% glycerol, subsequently, frozen quickly in liquid nitrogen. The X-ray diffraction data of the frozen crystal was collected in 100 K nitrogen flow by a vibration method in a synchrotron equipment KEK-PF, BL6B. From the resulted diffraction image, diffraction intensity was converted into numerical value using DENZO/SCALEPACK (manufactured by HKL), and a crystal structure factor was obtained. In this stage, the crystal was hexagonal and the space group had $P6_522$ or $P6_122$, and the unit lattice of the crystal was found to be, a = b = 79.9 Å, c = 322.2 Å, $\alpha = \beta = 90°$, $\gamma = 120°$.

The structure was analyzed by conducting a molecule substitution method using the resulted structure factor and the three dimensional structure coordinate of Human hexakinase type 1. The calculation was conducted by an Amore program of CCP4 (Council for the Central laboratory of the Research Councils) using a data having a resolution ability of 8 Å to 4 Å. The R factor of the structure obtained by calculation was 53.7%, and the space group of the crystal was $P6_522$ and it was found that one variant type GK molecule was contained at an asymmetric unit. An electron density map was obtained from this structure and structure factor, and the structure of the variant type glucokinase was determined using Program 0 (manufactured by Dat-ONO).

Next, preciseness of a position of an amino acid was increased using CNX (Accelrys Inc.), and identification of an amino acid residue was conducted using Program 0. This operation was repeated, and the structure coordinate of 448 amino acid residues from threonine 14 to cysteine 461 of the variant type glucokinase, one molecule of glucose, one molecule of compound A, one sodium ion, and 149 water molecules were identified, to determine the structure coordinate. The R factor which is an indicator of correctness of the finally determined structure was 23.2% for data of resolution ability from 30 Å to 2.3 Å, and the R factor (Rfree) for data not used in calculation in the stage of refining the structure was 27.4%. When confirmed by Ramachandran plot, an amino acid residue having an unacceptable structure was not found.

The determined structure of the variant type glucokinase was analogous to the structure of an isozyme, hexakinase, however, the structure of a portion to which a compound 1 (compound of the formula IIIa) that activates glucokinase binds was different. This difference in the structure cannot be prospected by capability of current computational chemistry, and this structure analysis clarified, for the first time, that this portion is an activator binding portion and, its detail steric structure. Fig. 1a is a ribbon view showing the three dimensional structure of glucokinase analyzed here. As shown in Fig. 1a, the newly found activator binding portion situated between a large domain and a small domain, and was away by about 20 Å from the active center of glucokinase to which a substrate glucokinase was bound. Amino acid residues of glucokinase constituting the activator binding portion were as described below. Tyrosine 61 to serine 69, glutamic acid 96 to glutamine 98, isoleucine 159, methionine 210 to tyrosine 215, histidine 218 to glutamic acid 221, methionine 235, arginine 250 and leucine 451 to lysine 459.

Binding mode of a compound 1 (compound of the formula IIIa) to this binding portion is shown in Fig. 2, and the structure of the binding portion of glucokinase is shown in Fig. 3. A thiazole ring was in van der Walls contact with molecules at amino acid side chains of valine 62, valine 452 and valine 455, and a nitrogen atom on a thiazole ring had a hydrogen bond with a nitrogen atom of a main chain of arginine 63. A nitrogen atom of an amide on the compound 1 had a hydrogen bond with an oxygen atom of a main chain of arginine 63. A benzene ring portion of the compound 1 was in van der Walls contact with isoleucine 211, and a fluorine atom substituted on a benzene ring was in van der Walls contact with a side chain of tyrosine 214. The aniline structure of the compound 1 formed a hydrogen bond with an oxygen atom of a side chain of tyrosine 215. An imidazole ring portion connected to a benzene ring via a sulfur was in van der Walls contact with amino acid side chain portions of methionine 210, methionine 235 and tyrosine 214. A portion of serine 64 to serine 69 connecting a large domain and a small domain projected into a solution, and the compound 1 was connected to a lower part of an arch structure formed by this portion (Fig. 3).

(Example 4: example of drug design)

Library compounds were screened using, as research conditions, pharmacophore of a hydrogen bond acceptor and hydrogen bond donor generated respectively from NH and CO of a main chain of Arg63, hydrophobic pharmacophore formed in a space corresponding to a phenyl group of an aniline part of a ligand forming a complex, and the protein surface made based on the structure of a protein, by using a software UNITY (manufactured by Tripos), and the following compound 4 and compound 5 were obtained and assayed, to confirm activities of 780% and 560%. An activity of 780% means that when the activity of glucokinase is 100% in the case of control, the activity is enhanced up to 780% by these compound (using glucose 2.5 M, and ligand 10 μM).

Compound 4

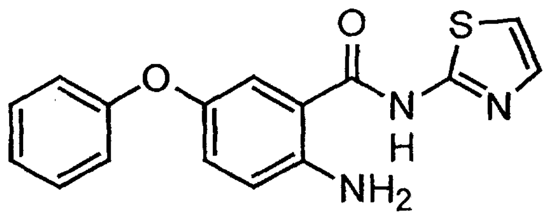

activity: 780%

Compound 5

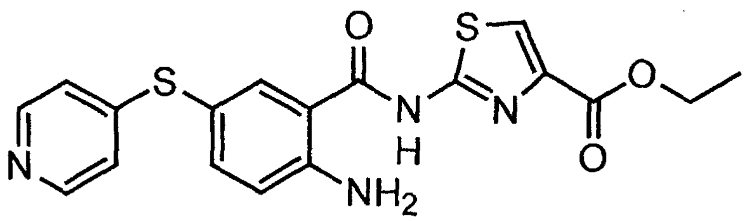

activity: 560%

(Example 5)

(Crystal of variant type GK (Δ1-15))

A crystal of a single entity of a variant type GK (Δ1-15)(glucokinase having an amino acid sequence as depicted in SEQ ID No. 8) was obtained by using a vapor diffusion means described below.

Namely, the variant type GK purified at high purity was concentrated, to obtain a solution of the variant type GK (25 mM Tris-Cl, pH 7.2, 50 mM NaCl, 5 mM TCEP) having a final concentration of about 10 mg/ml. To 1 to 5 μl of a protein solution was added the equal amount of a crystallization solution (1.5 to 1.6 M ammonium sulfate, 50 mM NaCl, 0.1 M Bicine NaOH (pH 8.7)) and mixed to give a solution which was accommodated in a sealed vessel containing 0.5 to 1 ml of a crystallization solution so that both solutions did not come into contact, and allowed to stand still at 20°C. After standing still for about 3 days to 1 month, a crystal having a maximum size of about 0.07 mm × 0.07 mm × 0.5 mm was obtained in the sample solution.

The resulted crystal was immersed in a crystallization solution containing 20% glycerol, subsequently, frozen quickly in liquid nitrogen. The X-ray diffraction data of the frozen crystal was collected in 100 K nitrogen flow by a vibration method in a synchrotron equipment Spring-8, BL32B2. From the resulted diffraction image, diffraction intensity was converted into numerical value using Mosflm, and a crystal structure factor was obtained. In this stage, the crystal was hexagonal and the space group had $P6_522$ or $P6_122$, and the unit lattice of the crystal was found to be, a = b = 103.2 Å, c = 281.0 Å, $\alpha = \beta = 90°$, $\gamma = 120°$.

Next, the structure was analyzed by conducting a molecule substitution method using the resulted structure factor. As the steric structure model, three dimensional structure coordinates of respective domains of glucokinase determined by a crystal of variant type GK (Δ1-11)/glucose/compound complex were used separately. The calculation was conducted by an Amore program of CCP4 (Council for the Central laboratory of the Research Councils) using a data having a resolution of 8 Å to 4 Å. The space group of the crystal was $P6_522$ and it was found that one variant type GK molecule GK (Δ1-15) was contained at an asymmetric unit. An electron density map was obtained from this structure and structure factor, and the structure of the variant type GK (Δ1-15) single entity was determined using Program 0 (manufactured by Dat-ONO).

Next, preciseness of a position of an amino acid was increased using CNX (manufactured by Molecular Simulation), and identification of an amino acid residue was conducted using Program 0. This operation was repeated, and the structure coordinate of 424 amino acid residues from methionine 15 to histidine 156 and from asparagine 180 to cysteine 461 of the variant type glucokinase, two sulfate ions, one sodium ion and 7 water molecules were identified, to determine the structure coordinate. The R factor which is an indicator of correctness of the finally determined structure was 23.8% for data of resolution from 50 Å to 3.4 Å, and the R factor (Rfree) for data not used in calculation in the stage of increase in preciseness of the structure was 30.6%. When confirmed by Ramachandran plot, an amino acid residue having an unacceptable structure was not found.

Figs 1a and 1b show, respectively, a ribbon view showing the structures of glucokinase (Δ1-11)/glucose/compound 1, and a ribbon view showing the structures of glucokinase (Δ1-15) single entity. In the determined structure of the variant type GK (Δ1-15), the structures of main parts of a large domain and a small domain were analogous to respective structures in the glucokinase determined by a crystal of variant type GK (Δ1-11)/glucose/compound complex, however, the relative positions of the two domains were different significantly. The main portion of a small domain in the structure of the variant type GK (Δ1-15) single entity was rotated by about 99° from the position of a small domain in the structure of variant type GK (Δ1-11)/glucose/compound complex. An α-13 helix constituting a small domain in the structure of variant type GK (Δ1-11)/glucose/compound complex situated at the C-terminal region of glucokinase did not constitute a small domain already in the structure of variant type GK (Δ1-15) single entity, and situated between both the domains. Further, since both of the substrate glucose binding portion and activating agent binding portion in the structure of variant type GK (Δ1-11)/glucose/compound complex were present between the two domains, the structures of these portions were changed significantly in newly determined structures. In the structure of variant type GK (Δ1-15) single entity, an amino acid residue performing an important role in enzymatic activity did not form an active portion, and the structure of variant type GK (Δ1-15) single entity was a structure of glucokinase inactivated condition. In the structure of variant type GK (Δ1-15) single entity, the activating agent binding portion disappeared completely. Change in the structure of glucokinase (rotation of domain by about 99°) observed by the structure of variant type GK (Δ1-11)/glucose/compound complex and the structure of variant type GK (Δ1-15) single entity was by far larger as compared with change in the structure (rotation of domain by about 12°) of hexokinase known to date, and cannot be prospected by the capacity of current computational chemistry , and it was clarified for the first time in this structure analysis.

It was also clarified that the activating agent for glucokinase can be designed by designing a compound that binds to a compound binding portion shown in the structure of variant type GK (Δ1-11)/glucose/compound complex, with an intention to inhibit structure change into the structure of variant type GK (Δ1-15) single entity which is of inactive type.

Industrial Applicability

As described above, according to the present invention, a crystal of a glucokinase protein which could not be easily crystallized conventionally can be obtained. A three dimensional structure coordinate obtained by analyzing the structure of this crystal can be used suitably for designing a compound that binds to glucokinase. Thus designed compound binds to glucokinase, therefore, it can be used as a therapeutic agent (for example, diabetes remedy) for patients correlated with glucokinase activity, as a glucokinase activating agent or inhibitor.

## Claims

1. A glucokinase protein, used for crystallization.

2. The protein according to Claim 1, consisting of an amino acid sequence as depicted in SEQ ID No. 5.

3. A crystal of a protein, consisting of an amino acid sequence as depicted in SEQ ID No. 5 or an amino acid sequence which is substantially the same as the amino acid sequence.

4. The crystal according to Claim 3, wherein said protein is a glucokinase protein.

5. The crystal according to Claim 3, which is a crystal of a protein having an amino acid sequence as depicted in SEQ ID No. 5.

6. The crystal according to Claim 3, wherein the lattice constant satisfies the following formulae (1) to (4):

$$a = b = 79.9 \pm 4 \text{ Å} \qquad (1)$$

$$c = 322.2 \pm 15 \text{ Å} \qquad (2)$$

$$\alpha = \beta = 90° \qquad (3)$$

$$\gamma = 120° \qquad (4)$$

7. The crystal according to Claim 6, wherein the space group is $P6_522$.

8. A crystal of a protein specified by three dimensional structure coordinate data described in Table 1.

9. A crystal wherein, in three dimensional structure coordinate data obtained by changing at least one datum in three dimensional structure coordinate data described in Table 1, the average square deviation between an atom (Cα atom) of a main chain of an amino acid shown by three dimensional structure coordinate data described in Table 1 and a Cα atom shown by said changed three dimensional structure coordinate data corresponding to the Cα atom is 0.6 Å or less.

10. The crystal according to any of Claims 3 to 9, wherein the compound binding portion is constituted of at least one of amino acid residues of tyrosine 61 to serine 69, glutamic acid 96 to glutamine 98, isoleucine 159, methionine 210 to tyrosine 215, histidine 218 to glutamic acid 221, methionine 235, arginine 250 and leucine 451 to lysine 459, in an amino acid sequence as depicted in SEQ ID No. 5.

11. A crystal comprising a complex of a protein consisting of an amino acid sequence as depicted in SEQ ID No. 5 or

an amino acid sequence which is substantially the same as the amino acid sequence with a compound capable of binding to the protein.

**12.** The crystal according to Claim 11, wherein said compound is represented by the formula (I):

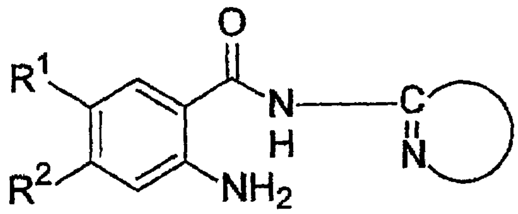

(I)

[wherein, $R^1$ represents a halogen atom, -S-$(O)_p$-A, -S-$(O)_q$-B or -O-B (wherein, p and q are the same or different and represent an integer of 0 to 2, A represents a straight chain $C_1$ to $C_6$ alkyl group optionally substituted and B represents a 5-membered cyclic or 6-membered cyclic aryl group or heteroaryl group optionally substituted), $R^2$ represents a hydrogen atom or halogen atom, and

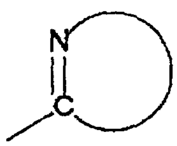

(II)

represents a mono-cyclic or di-cyclic heteroaryl group optionally substituted, having a nitrogen atom adjacent to a carbon atom connected to an amide group.].

**13.** The crystal according to Claim 12, wherein said compound is any of compounds of the formulae (IIIa) to (IIIc):

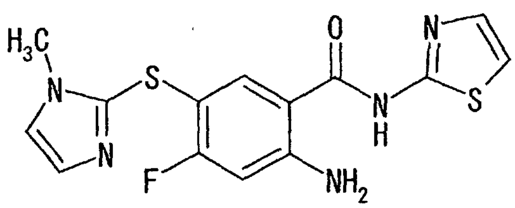

(IIIa)

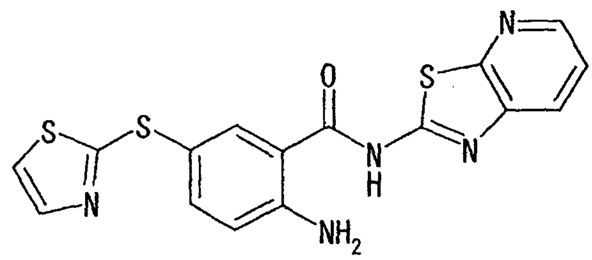

(IIIb)

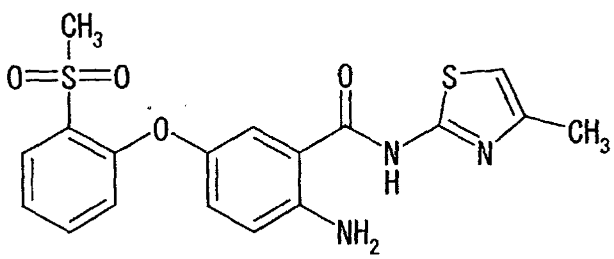

(IIIc)

**14.** The protein according to Claim 1, consisting of an amino acid sequence as depicted in SEQ ID No. 8.

**15.** A crystal of a protein, consisting of an amino acid sequence as depicted in SEQ ID No. 8 or an amino acid sequence which is substantially the same as the amino acid sequence.

**16.** The crystal according to Claim 15, wherein said protein is a glucokinase protein.

**17.** The crystal according to Claim 15, which is a crystal of a protein having an amino acid sequence as depicted in SEQ ID No. 8.

**18.** The crystal according to Claim 15, wherein the lattice constant satisfies the following formulae:

$$a = b = 103.2 \pm 5\ \text{Å} \quad (5)$$

$$c = 281.0 \pm 7\ \text{Å} \quad (6)$$

$$\alpha = \beta = 90° \quad (7)$$

$$\gamma = 120° \quad (8)$$

**19.** The crystal according to Claim 18, wherein the space group is $P6_522$.

**20.** A crystal of a protein specified by three dimensional structure coordinate data described in Table 2.

**21.** A crystal wherein, in three dimensional structure coordinate data obtained by changing at least one datum in three dimensional structure coordinate data described in Table 2, the average square deviation between an atom ($C\alpha$ atom) of a main chain of an amino acid shown by three dimensional structure coordinate data described in Table 2 and a $C\alpha$ atom shown by said changed three dimensional structure coordinate data corresponding to the $C\alpha$

atom is 0.6 Å or less.

**22.** A method of producing a crystal comprising a complex of a protein with a compound binding to the protein, comprising
a protein production step of producing a protein having an amino acid sequence obtained by deleting 1 to 50 amino acid residues from one or both of the N terminal and C terminal of a protein having an amino acid sequence as depicted in SEQ ID No. 2, and
a protein reaction step of reacting a compound binding to a protein obtained in said protein production step with a protein obtained in said protein production step.

**23.** A method of producing a crystal of a protein, using a protein containing an amino acid sequence as depicted in SEQ ID No. 5 or an amino acid sequence which is substantially the same as the amino acid sequence, and having a glucokinase activity, and a compound capable of binding to said protein.

**24.** The method of producing a crystal of a protein according to Claim 23, wherein said compound capable of binding to the protein is a compound of the formula (I):

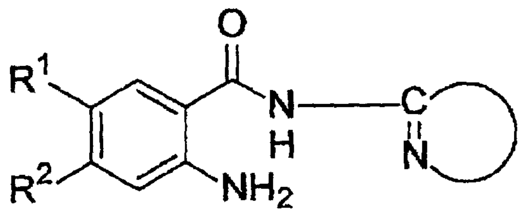

(I)

[wherein, $R^1$ represents a halogen atom, -S-(O)$_p$-A, -S-(O)$_q$-B or -O-B (wherein, p and q are the same or different and represent an integer of 0 to 2, A represents a straight chain $C_1$ to $C_6$ alkyl group optionally substituted and B represents a 5-membered cyclic or 6-membered cyclic aryl group or heteroaryl group optionally substituted), $R^2$ represents a hydrogen atom or halogen atom, and

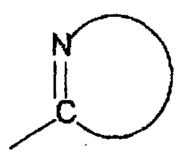

(II)

represents a mono-cyclic or di-cyclic heteroaryl group optionally substituted, having a nitrogen atom adjacent to a carbon atom connected to an amide group.].

**25.** The method of producing a crystal according to Claim 23 or 24, according to a co-crystallization method or soaking method.

**26.** A drug design method of designing the structure of a compound binding to a protein based on the steric structure information of the protein, wherein the steric structure information of the protein is information obtained by analyzing the crystal according to any one of Claims 3 to 13, 15 to 21.

**27.** The drug design method according to Claim 26, comprising
a binding portion guessing step of guessing a compound binding portion of said protein based on said steric

structure information, and

a selection step of selecting a compound adaptable to a compound binding portion guessed in said binding portion guessing step from a compound library.

**28.** The drug design method according to Claim 26, comprising

a binding portion guessing step of guessing a compound binding portion of said protein based on said steric structure information, and

a compound structure fabricating step of fabricating the structure of a compound adaptable to a compound binding portion guessed in said binding portion guessing step.

**29.** The drug design method according to Claim 26, comprising

a binding portion guessing step of guessing a compound binding portion of said protein based on said steric structure information, and

a design step of designing visually the structure of a compound so that a compound binding portion guessed in said binding portion guessing step and a compound adaptable to the compound binding portion interact.

**30.** The drug design method according to any one of Claims 26 to 29, wherein said compound binding portion is constituted of at least one of amino acid residues of tyrosine 61 to serine 69, glutamic acid 96 to glutamine 98, isoleucine 159, methionine 210 to tyrosine 215, histidine 218 to glutamic acid 221, methionine 235, arginine 250 and leucine 451 to lysine 459, in an amino acid sequence as depicted in SEQ ID No. 5.

**31.** The drug design method according to any one of Claims 26 to 30, further comprising a step of measuring the physiological activity of a candidate compound guessed to be adaptable to said compound binding portion.

**32.** The drug design method according to any one of Claims 26 to 30, further comprising a binding judgment step of contacting a candidate compound guessed to be adaptable to said compound binding portion with a protein containing an amino acid sequence as depicted in SEQ ID No. 5 or an amino acid sequence which is substantially the same as the amino acid sequence, and judging if the candidate compound binds to the protein or not.

**33.** A method of producing a compound array, comprising combining compounds in a group selected by the drug design method according to any one of Claims 26 to 30, as a compound array.

Fig. 1A

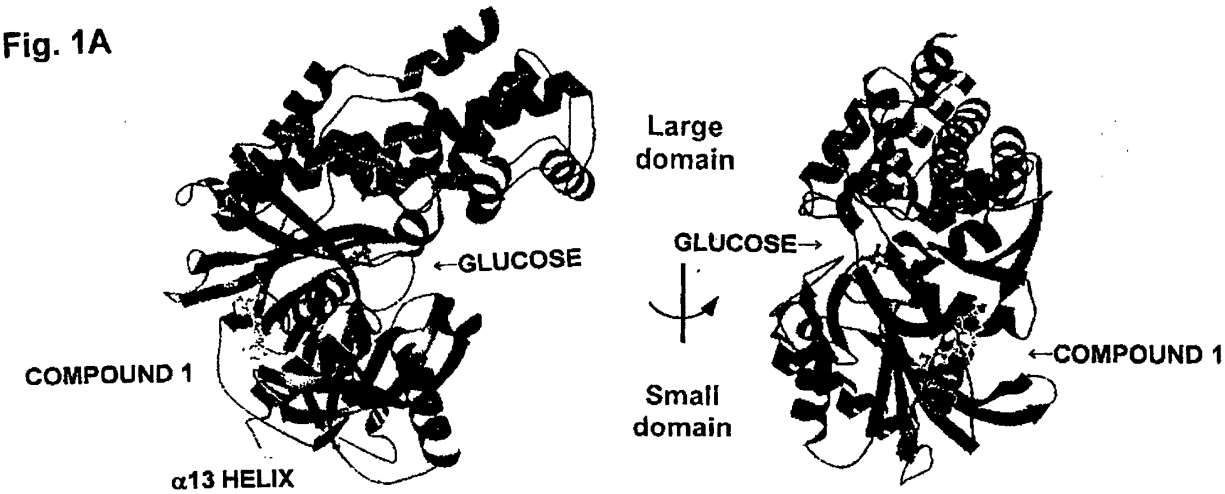
Large domain
←GLUCOSE
GLUCOSE→
COMPOUND 1
←COMPOUND 1
Small domain
α13 HELIX

FIG. 1B

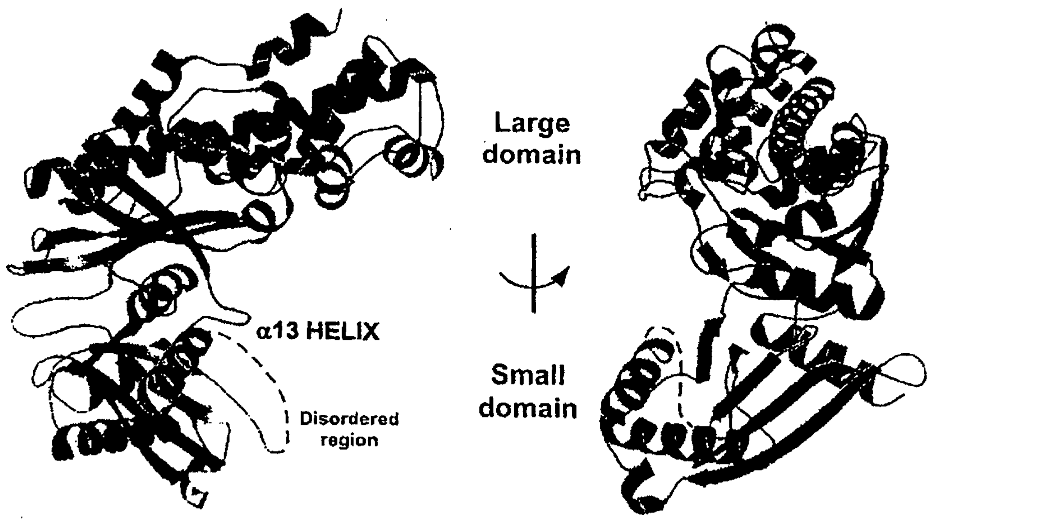
Large domain
α13 HELIX
Disordered region
Small domain

Fig. 2

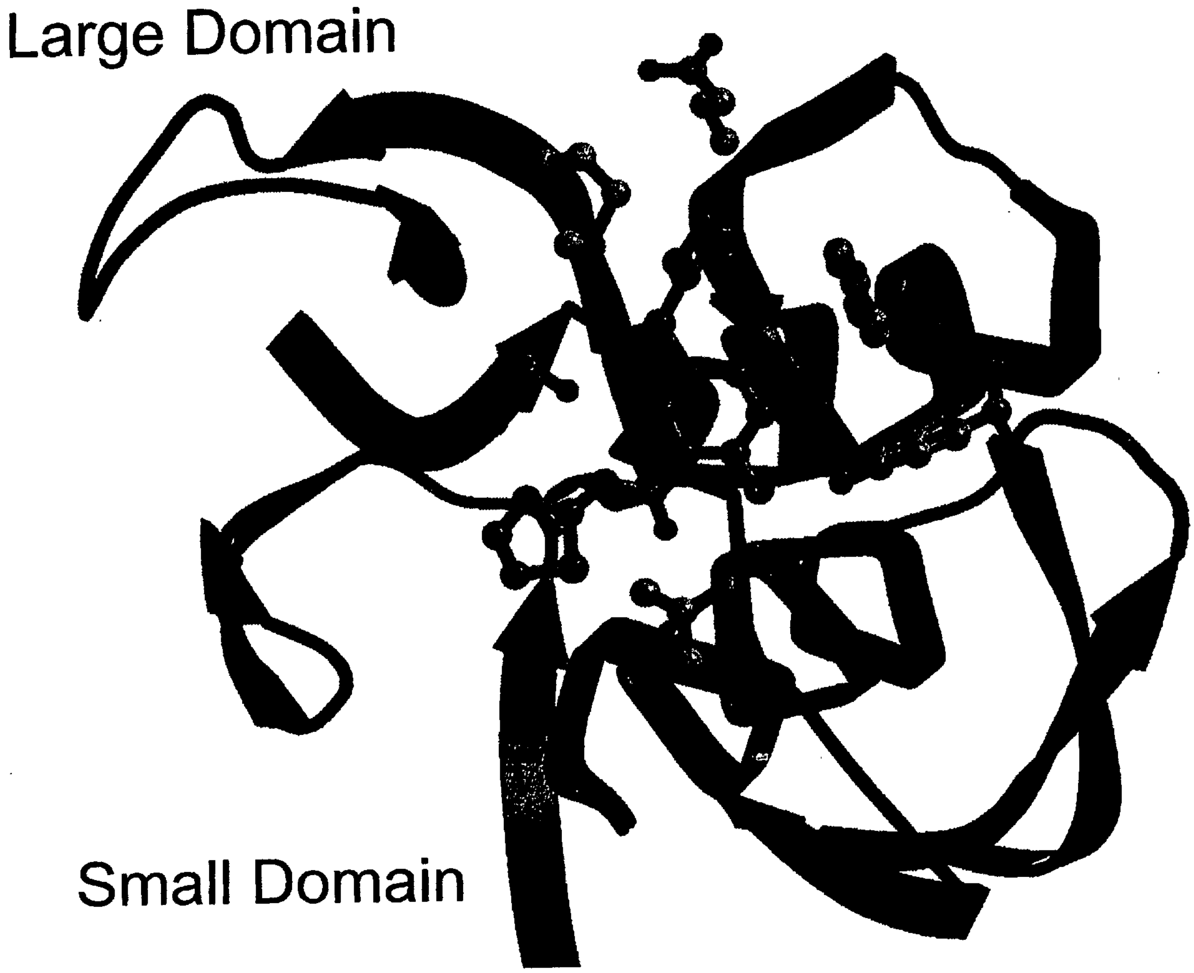

Fig. 3

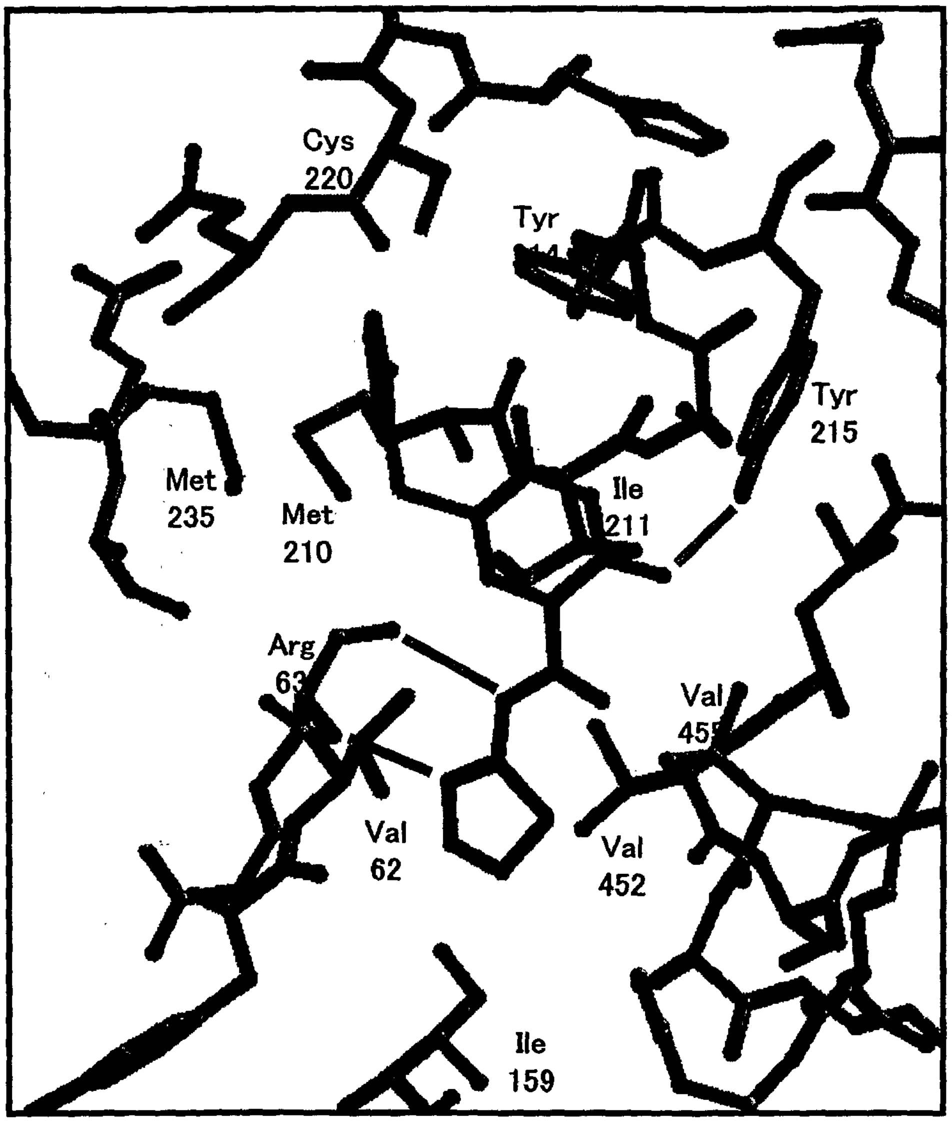
Cys
220
Tyr
Tyr
215
Met
235
Met
210
Ile
211
Arg
Val
Val
62
Val
452
Ile
159

**INTERNATIONAL SEARCH REPORT**

International application No.
PCT/JP03/06054

A. CLASSIFICATION OF SUBJECT MATTER
Int.Cl[7] C12N9/12, C12Q1/48

According to International Patent Classification (IPC) or to both national classification and IPC

B. FIELDS SEARCHED

Minimum documentation searched (classification system followed by classification symbols)
Int.Cl[7] C12N9/12, C12Q1/48

Documentation searched other than minimum documentation to the extent that such documents are included in the fields searched

Electronic data base consulted during the international search (name of data base and, where practicable, search terms used)
CA(STN), BIOSIS(DIALOG), WPI(DIALOG),
SwissProt/PIR/Genbank/EMBL/DDBJ/GeneSeq

C. DOCUMENTS CONSIDERED TO BE RELEVANT

| Category* | Citation of document, with indication, where appropriate, of the relevant passages | Relevant to claim No. |
|---|---|---|
| X<br>A | TANIZAWA, Y. et al., Human Liver Glucokinase Gene: Cloning and Sequence Determination of Two Alternatively Spliced cDNAs, Proc.Natl.Acad.Sci. USA., 1991, Vol.88, pages 7294 to 7297 | 1<br>2-25 |
| A | MAHALINGAM B. et al., Structural model of human glucokinase in complex with glucose and ATP., Diabetes, 1999, Vol.48, pages 1698 to 1705 | 1-25 |
| A | WILLSON M. et al., Yeast hexokinase inhibitors designed from the 3-D enzyme structure reboilding. J. Enzyme Inhib., 1997, Vol.12, No.2, pages 101 to 121 | 1-25 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 12 June, 2003 (12.06.03) | 24 June, 2003 (24.06.03) |
| Name and mailing address of the ISA/<br>Japanese Patent Office | Authorized officer |
| Facsimile No. | Telephone No. |

Form PCT/ISA/210 (second sheet) (July 1998)

**INTERNATIONAL SEARCH REPORT**

International application No.
PCT/JP03/06054

**Box I Observations where certain claims were found unsearchable (Continuation of item 2 of first sheet)**

This international search report has not been established in respect of certain claims under Article 17(2)(a) for the following reasons:

1. [X] Claims Nos.: 26 to 33
because they relate to subject matter not required to be searched by this Authority, namely:
Inventions according to said claims relate to subject matters not required to be searched by this Authority in accordance with PCT Article 17 (2) (a) and PCT Rule 39.1. (see extra sheet for details)

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box II Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.

2. [ ] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** [ ] The additional search fees were accompanied by the applicant's protest.
[ ] No protest accompanied the payment of additional search fees.

Form PCT/ISA/210 (continuation of first sheet (1)) (July 1998)

**INTERNATIONAL SEARCH REPORT**

International application No.
PCT/JP03/06054

Continuation of Box No.I-1 of continuation of first sheet(1)

"Method for drug design" according to the present invention relates to the design of a compound to be bonded to a protein, on the basis of the information on the three-dimensional structure of the protein. The design according to the present invention involves the work of the inventor to estimate a suitable compound by his mental acts, and such work is considered to correspond to the performance of purely mental acts.

Form PCT/ISA/210 (extra sheet) (July 1998)